# EUROPEAN PATENT SPECIFICATION

(11) **EP 1 812 590 B1**
(45) Date of publication and mention of the grant of the patent: **15.04.2009**
(21) Application number: 05791715.5
(22) Date of filing: 24.06.2005
(51) Int. Cl.: C12Q 1/68

(54) **METHODS AND REAGENTS FOR THE DETECTION OF MELANOMA**
VERFAHREN UND REAGENZIEN ZUR ERKENNUNG VON MELANOMEN
PROCEDES ET REACTIFS POUR LA DETECTION DE MELANOME

(30) Priority: 25.06.2004 US 582906 P
(43) Date of publication of application: 01.08.2007
(73) Proprietor: Veridex, LLC, Warren, NJ 07059 (US)
(72) Inventor: WANG, Yixin, San Diego, CA 92139 (US); TALANTOV, Dimitri, San Diego, CA 92130 (US); MAZUMDER, Abhijit, Basking Ridge, NJ 07920 (US)
(74) Representative: Mercer, Christopher Paul
(86) International application number: PCT/US2005/022846
(87) International publication number: WO 2006/002433

(56) References cited:
- WO-A-01/27295
- WO-A-01/94629
- WO-A-02/27028
- WO-A-20/04019886
- WO-A-20/05044990
- FOGEL MINA ET AL: "L1 adhesion molecule (CD 171) in development and progression of human malignant melanoma." CANCER LETTERS, vol. 189, no. 2, 28 January 2003 (2003-01-28), pages 237-247, XP002377433 ISSN: 0304-3835
- XU XIAOWEI ET AL: "Expression of neurotrophin receptor Trk-C in nevi and melanomas." JOURNAL OF CUTANEOUS PATHOLOGY, vol. 30, no. 5, May 2003 (2003-05), pages 318-322, XP002377434 ISSN: 0303-6987
- VIHINEN PIA P ET AL: "New prognostic factors and developing therapy of cutaneous melanoma." ANNALS OF MEDICINE, vol. 35, no. 2, 2003, pages 66-78, XP009065071 ISSN: 0785-3890
- THIES ANKA ET AL: "Overexpression of the cell adhesion molecule L1 is associated with metastasis in cutaneous malignant melanoma." EUROPEAN JOURNAL OF CANCER (OXFORD, ENGLAND : 1990) SEP 2002, vol. 38, no. 13, September 2002 (2002-09), pages 1708-1716, XP002377435 ISSN: 0959-8049
- BITTNER M ET AL: "Molecular classification of cutaneous malignant melanoma by gene expression profiling" NATURE (LONDON), vol. 406, no. 6795, 3 August 2000 (2000-08-03), pages 536-540, XP000990000 ISSN: 0028-0836
- TALANTOV DMITRI ET AL: "Novel genes associated with malignant melanoma but not benign melanocytic lesions." CLINICAL CANCER RESEARCH : AN OFFICIAL JOURNAL OF THE AMERICAN ASSOCIATION FOR CANCER RESEARCH. 15 OCT 2005, vol. 11, no. 20, 15 October 2005 (2005-10-15), pages 7234-7242, XP002377436 ISSN: 1078-0432

## Description

Cutaneous malignant melanoma is a common, aggressive cancer with growing incidence. It is a serious healthcare problem with over 55,100 new cases anticipated in 2004 in US, and a mortality rate of about 14.5%. Cancer Facts and Figures 2003. American Cancer Society, 2003. The incidence of melanoma continues to rise faster than that of any other malignancy. De Braud et al. (2003). While prognosis of early local melanoma is favorable with 5-year overall survival over 90%, regional lymph node involvement decreases the overall survival rate to 10-46%. Balch et al. (2001). Therefore regional lymph node (LN) status becomes the most significant prognostic factor in a melanoma patient's survival. Introduction of the sentinel lymph nodes (SLN) technique (Morton (1992)) has increased the sensitivity of melanoma micrometastasis detection compared to H&E staining alone. Yu et al. (1999); and Messina et al. (1999). Nevertheless, even when enhanced by IHC, histological analysis is limited by the ability of light microscopy to recognize the tumor cells. Reverse transcription-polymerase chain reaction (RT-PCR) analysis has recently been proposed for a more sensitive detection of melanoma cells in LN. Many studies, when using well-characterized melanocyte specific markers, such as tyrosinase and MART-1, have demonstrated the presence of these gene transcripts in LNs otherwise found to be negative by routine histology and IHC. Shivers et al. (1998); and Kuo et al. (2003). However, these genes are not specific to tumor cells and cannot be used to discriminate between benign and malignant tissue. In fact, they caused false-positive results in the presence of benign capsular nevi. Takeuchi et al. (2004); Starz et al. (2003); and Gutzmer et al. (2002). Considering that benign nevi are not rare events in the melanoma SLN, the current RT-PCR assays are not useful clinically for diagnostic of melanoma micrometastasis. A recent study, proposed a multi-marker panel, including cancer specific markers for RT-PCR assay in order to increase assay specificity. Hoon et al. (2004). Identification of novel melanoma specific markers remains one of the key questions of melanoma research.

Certain proteins have been shown to be associated with melanoma and its metastases. These proteins or their activities have been used in IHC to identify metastases and include L1CAM (Thies et al. (2002); Fogel et al. (2003)); and S-100 (Diego et al. (2003)).

Nucleic acid tests have been proposed to increase the sensitivity of detection of metastatic melanoma. US Patent Publication Nos. 2002/0110820; and 2003/0232356. Studies have used markers that include MAGE3, tyrosinase, MART-1, MITF-M or IL-1, R1, endothelin-2, ephrin-A5, IGF Binding protein 7, HLA-A0202 heavy chain, Activin A (βA subunit), TNF RII, SPC4, CNTF Rα, or gp100 (HMB45) genes. Bostick et al. (1999); Hoon et al. (2001); Palmieri et al. (2001); Wrightson et al. (2001); Gutzmer et al. (2002); Davids et al. (2003); Starz et al. (2003); Rimboldi et al. (2003); Cook et al (2003); Reintgen et al. (2004); US Patent Publication Nos. 2002/0098535; 2003/0049701; US Patent Nos. 5,512,437; 5,512,444; 5,612,201; 5,759,783; 5,844,075; 6,025,474; 6,057,105; 6,235,525; 6,291,430; 6,338,947; 6,369,211; 6,426,217; 6,475,727; 6,500,919; 6,527,560; 6,599,699; WO 96/29430. Where determined, these markers have not been found adequate for sole use in melanoma diagnosis. Riccioni et al. (2002); Gutzmer et al. (2002); Davids et al. (2003); Goydos et al. (2003); and Prichard et al. (2003).

A number of these markers have also been shown to be indicative of other neoplasias such as ME20M (GP 100) for clear cell sarcoma, biliary tract carcinoma and gastric carcinoma. Hiraga et al. (1997); Okada et al. (2001); Okami et al. (2001); Antonescu et al. (2002); Segal et al. (2003). MAGE3 is also indicative of a number of neoplasias including breast, hepatocellular, renal, neural, lung and esophageal. Yamanaka et al. (1999); Ooka et al. (2000); Suzuki et al. (2000); Cheung et al. (2001); and Weiser et al. (2001). Several melanoma antigen-encoding genes are also expressed in lung cancer. Yoshimatsu et al. (1998).

These markers proved to be sensitive but non-specific since they showed positive expression in other cancers and benign melanocytes. Additionally, tyrosinase is expressed in Schwann cells which are present in normal lymph nodes. The lack of specificity alone calls for the development of assays with new or additional markers. H&E histology and IHC remain the "gold standard for the identification of melanoma and nevus cells in SLNs." Starz et al. (2003). Detection issues in the intra-operative setting make this need even more acute.

Lymph node involvement is the strongest prognostic factor in many solid tumors, and detection of lymph node micrometastases is of great interest to pathologists and surgeons. Current lymph node evaluation involves microscopic examination of H&E-stained tissue sections and IHC and suffers from three major limitations: (a) small foci of cells, are easily missed; (b) the result is not rapidly available, meaning that any positive result in a SLN procedure requires a second surgery for removal of axcillary lymph nodes; and (c) only one or two tissue sections are studied, and thus the vast majority of each node is left unexamined. Serial sectioning can help overcome sampling error, and IHC can help identify small foci of cells; this combination, however, is costly and time consuming for routine analysis.

Surgical decisions of regional lymph node dissection can be based on intra-operative frozen section analysis of lymph nodes; however, the sensitivity of these methods is relatively poor, ranging from 50-70% relative to standard H&E pathology, leading to a high rate of second surgeries. Thus, pathologists are not routinely performing intra-operative frozen section analysis or touch print cytology analysis for melanoma patients. Improvements in the sensitivity and specificity of intra-operative assays for melanoma would significantly benefit oncology.

High-density microarrays have been applied to simultaneously monitor expression, in biological samples, of thousands of genes. Studies have resulted in the identification of genes differentially expressed in benign and malignant lesions, as well as genes that might be of prognostic value. Luo et al. (2001); and Wang et al. (2004). Gene expression profiling of malignant melanoma has been accomplished using a microarray containing probes for 8,150 cDNAs. Bittner et al. (2000). These researchers identified several genes that might be associated with aggressive tumor behavior. In recent work, comparison of gene expression profiles of a few melanoma and normal melanocyte cell lines led to the identification of differentially expressed genes and pathways modulated in melanoma. Takeuchi et al. (2004).

### SUMMARY OF PRESENT APPLICATION

Gene expression profiling of an extensive set of clinically relevant tissue samples is provided in the present invention. Total RNA from forty-five primary malignant melanomas, 18 benign skin nevi and 7 normal skin tissues were hybridized on an Affymetrix Hu133A microarray containing 22,000 probe sets. Differentially expressed genes in malignant melanoma as compared to benign tissue were identified. Pathway analysis of the differentially expressed genes revealed an over-representation of genes associated with neural tissue development and activation of amyloid processing signaling pathway. A one-step quantitative RT-PCR assay was used to test a combination of two melanoma specific genes, PLAB and L1CAM in a panel of clinically relevant samples that included primary malignant melanoma, benign nevi, melanoma LN metastasis and melanoma-free lymph node samples.

The present invention provides a method of identifying a melanoma by obtaining a tissue sample; and assaying and measuring the expression levels in the sample of genes encoding mRNA corresponding to prostate differentiation factor (PLAB, MIC1) (SEQ ID NO: 1) and L1 cell adhesion molecule (L1CAM) (SEQ ID NO: 2); or PLAB, L1CAM and neurotrophic tyrosine kinase receptor, type 3 (NTRK3) (SEQ ID NO: 3) where the gene expression levels above pre-determined cut-off levels are indicative of the presence of a melanoma in the sample and wherein said predetermined cut-off levels are the highest score in: primary normal samples; and/or benign nevi samples. The invention further provides a method of identifying a melanoma by obtaining a tissue sample; and assaying and measuring the expression levels in the sample of genes encoding mRNA recognized by the primer/probe sets SEQ ID NOs: 4-6 or SEQ ID NOs: 7-9 and SEQ ID NOs: 10-12 or SEQ ID NOs: 13-15; or SEQ ID NOs: 4-6 or SEQ ID NOs: 7-9 and SEQ ID NOs: 10-12 or SEQ ID NOs: 13-15 and SEQ ID NOs: 16-18 where the gene expression levels above pre-determined cut-off levels are indicative of the presence of a melanoma in the sample.

The application also provides a method of distinguishing a malignant melanocyte from a benign melanocyte by obtaining a tissue sample; and assaying and measuring the expression levels in the sample of genes encoding PLAB and L1CAM; or PLAB, L1CAM and NTRK3 where the gene expression levels above pre-determined cut-off levels are indicative of the presence of a melanoma in the sample.

The application also provides a method of distinguishing a malignant melanocyte from a benign melanocyte by obtaining a tissue sample; and assaying and measuring the expression levels in the sample of genes recognized by the primer/probe sets SEQ ID NOs: 4-6 or SEQ ID NOs: 7-9 and SEQ ID NOs: 10-12 or SEQ ID NOs: 13-15; or SEQ ID NOs: 4-6 or SEQ ID NOs: 7-9 and SEQ ID NOs: 10-12 or SEQ ID NOs: 13-15 and SEQ ID NOs: 16-18 where the gene expression levels above pre-determined cut-off levels are indicative of the presence of a melanoma in the sample.

The application further provides a method of determining patient treatment protocol by obtaining a tissue sample from the patient; and assaying and measuring the expression levels in the sample of genes encoding PLAB and L1CAM; or PLAB, L1 CAM and NTRK3 where the gene expression levels above pre-determined cut-off levels are indicative of the presence of a melanoma in the sample.

The application further provides a method of determining patient treatment protocol by obtaining a tissue sample from the patient; and assaying and measuring the expression levels in the sample of genes recognized by the primer/probe sets SEQ ID NOs: 4-6 or SEQ ID NOs: 7-9 and SEQ ID NOs: 10-12 or SEQ ID NOs: 13-15; or SEQ ID NOs: 4-6 or SEQ ID NOs: 7-9 and SEQ ID NOs: 10-12 or SEQ ID NOs: 13-15 and SEQ ID NOs: 16-18 where the gene expression levels above pre-determined cut-off levels are indicative of the presence of a melanoma in the sample.

The application further provides additional Marker and control genes, the expression of which aid in the claimed methods. These additional genes include up-regulated SEQ ID NOs: 29-467 and down-regulated SEQ ID NOs: 468-978.

The primary Marker can be PLAB and is defined herein as the gene encoding any variant, allele etc. including SEQ ID NO: 1. PLAB is also described by Paralkar et al. (1998) and represented by Accession No. AF003934. PLAB is also defined as the gene encoding mRNA recognized by the primer/probe sets SEQ ID NOs: 4-9.

The secondary Marker can be L1CAM and is defined herein as the gene encoding any variant, allele etc. including SEQ ID NO: 2. L1CAM is also described by Haspel et al (2003); and US Patent No. 6,107,476 and is represented by Accession No. NM_000425. L1CAM is also defined as the gene encoding mRNA recognized by the primer/probe sets SEQ ID NOs: 10-15.

The application further provides a kit for conducting an assay to determine the presence of melanoma in a cell sample comprising: nucleic acid amplification and detection reagents.

The application further provides primer/probe sets for amplification and detection of PCR products obtained in the inventive methods. These sets include the following:
SEQ ID NO:4 (PLAB forward primer) ggcagaatcttcgtccgca
SEQ ID NO:5(PLAB reverse primer) ggacagtggtccccgttg
SEQ ID NO:6 (PLAB probe) cccagctggagttgcacttgcggcc
SEQ ID NO:7 (PLAB upper primer) gaacaccgacctcgtccc
SEQ ID NO:8 (PLAB lower primer) ggcggcccgagagata
SEQ ID NO:9 (PLAB probe) cgccagaagtgcggctgggattt
SEQ ID NO:10 (L1CAM forward) gctgggactgggaacagaact
SEQ ID NO:11 (L1CAM Reverse) ggagcagagatggcaaagaaa
SEQ ID NO:12 (L1CAM probe) ttccccaccatctgctgt
SEQ ID NO:13 (L1CAM upper) ccacagatgacatcagcctcaa
SEQ ID NO:14 (L1CAM lower) ggtcacacccagctcttcctt
SEQ ID NO:15 (L1CAM probe) tggcaagcccgaagtgcagttcctt
SEQ ID NO:16 (NTRK3 primer) gccccggcacccttta
SEQ ID NO:17 (NTRK3 primer) aaccctgccagtggtggat
SEQ ID NO:18 (NTRK3 probe) cagatgggtgttttc
SEQ ID NO:19 (Tyr upper) actcagcccagcatcattcttc
SEQ ID NO:20 (Tyr lower) atggctgttgtactcctccaatc
SEQ ID NO:21 (Tyr probe) cttctcctcttggcagattgtctgtagctt
SEQ ID NO:22 (PBGD upper) ccacacacagcctactttccaa
SEQ ID NO:23 (PBGD lower) tacccacgcgaatcactctca
SEQ ID NO:24 (PBGD probe) aacggcaatgcggctgcaacggcggaatt

The application further provides amplicons obtained by PCR methods utilized in the methods. These amplicons include the following: SEQ ID NO:27 (tyrosinase Amplicon)
actcagcccagcatcattcttctcctcttggcagattgtctgtagccgattggaggagtacaacagccat

Other genes described herein include up-regulated Markers (SEQ ID NOs: 29-467), down-regulated Markers (SEQ ID NOs: 468-978), PBGD (SEQ ID NO: 979), MART1 (SEQ ID NO: 980), ME20M (GP100; SEQ ID NO: 981) and MAGE-3 (SEQ ID NO: 982) and various primers and probes (SEQ ID NOs: 983-1011) used in detecting their expression.

### BRIEF DESCRIPTION OF THE DRAWINGS

Fig. 1. Flowchart of data analysis.
Fig. 2. Hierarchical clustering on the 15,795 genes that have at least two "present" calls in all samples. Each column is a sample and each row is a gene. Red is up-regulation and green is down-regulation. Purple: melanoma samples; yellow: benign nevi; and blue: normal skin.
Fig. 3. Microarray expression (A) and real time RT-PCR validation data (B) of the selected genes. First fourteen samples from the left are the melanoma tissue samples (red); next seven are benign nevi samples (yellow) and last five are normal skin (blue). For microarray plots x-axis shows intensity values; for PCR plots, x -axis is 2^{ΔCT}, where ΔCt is Ct (Target Gene) - Ct PBGD.
Fig. 4. Amyloid processing pathway. Adopted from Ingenuity^{™} Pathway Analysis Software Application. Genes up-regulated in melanoma are red and down-regulated in melanoma are green. Each gene symbol is followed by the fold-change of expression level between melanoma and benign/normal samples.

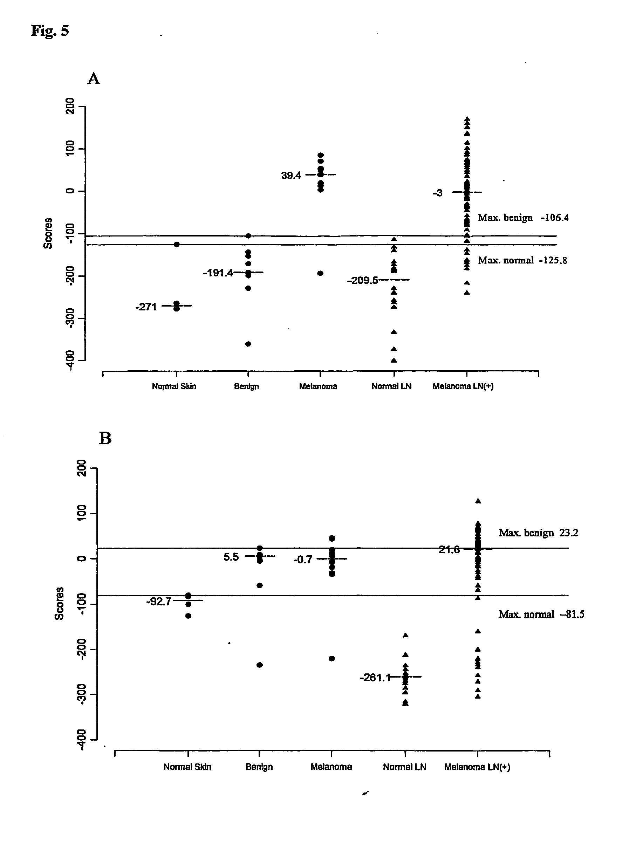

Fig. 5. One-step quantitative RT-PCR assay of PLAB and L1CAM (A) and conventional melanoma markers, gp100, tyrosinase (SEQ ID NO: 999) and MART1 (B). For each plot x-axis represents score for the new markers or the conventional markers. Median scores for each samples category are labeled. Two cut-off levels based on normal (green) and benign (red) samples are labeled on each plot.

### DETAILED DESCRIPTION

The present application provides methods of qualitatively and quantitatively identifying a melanoma; distinguishing a malignant melanocyte from a benign melanocyte; diagnosing melanocytic lesions with uncertain pathological features; and determining a melanoma patient treatment protocol. The methods further provide aids in patient prognosis, patient monitoring and drug development. The methods rely on assaying and measuring expression levels of various Marker genes encoding mRNAs provided herein where gene expression over a pre-determined cut-off level is indicative of the presence of a malignant melanocyte in the sample assayed.

Cutaneous melanoma is a common, aggressive cancer with growing incidence. Identification of melanoma-specific deregulated genes could provide molecular markers for LN staging assays and further insight on melanoma tumorigenesis. Total RNA isolated from 45 primary melanoma, 18 benign skin nevi, and 7 normal skin tissue specimens were analyzed on an Affymetrix U133A microarray containing 22,000 probe sets. Hierarchical clustering revealed a distinct separation of the melanoma samples from benign and normal specimens. Novel genes associated with malignant melanoma were identified. Differential gene expression of two melanoma specific genes, PLAB and L1CAM, were tested by a one-step quantitative RT-PCR assay on primary malignant melanoma, benign nevi and normal skin samples and also on malignant melanoma LN metastasis and melanoma-free lymph nodes. The performance of the markers was compared to conventional melanoma markers such as tyrosinase, gp100, and MART1. The results demonstrated the ability of using a combination of PLAB and L1CAM in a RT-PCR assay to differentiate clinically relevant tissue samples containing benign or malignant melanocytes.

High-density cDNA and oligonucleotide microarrays allow simultaneous monitoring of the expression of thousands of genes. Microarray technology provides a quantitative measurement of mRNA abundance and has gained acceptance as a tool for marker discovery based on gene expression. In the context of cancer research, microarray analysis has identified genes differentially expressed in benign and malignant lesions, different cancer types or that have prognostic significance. Luo et al. (2001); Su et al. (2001); Henshall et al. (2003); and Wang et al. (2004). The first gene expression profiling of malignant melanoma used a microarray containing probes for 8,150 cDNAs and identified genes that might be associated with aggressive tumor behavior. Bittner et al. (2000). Since the samples analyzed in their study did not include tissues containing normal or benign melanocytes, differentially expressed genes in malignant melanoma were not identified. In contrast to normal skin, melanocyte content in benign nevi is close to that in melanoma.

In another study, two pooled samples derived from either melanoma or benign nevi tissues were hybridized to a cDNA array and genes preferentially expressed in melanoma- or nevi-derived samples were found. Seykora et al. (2003). Other researchers used subtractive hybridization or analysis of SAGE libraries generated on melanoma cell lines, for monitoring gene expression in melanoma. Hipfel et al. (2000); and Weeraratna (2004). Recently, comparison of gene expression profiles of a few melanoma and melanocyte cell lines led to the identification of differentially expressed genes and pathways modulated in melanoma. Hoek et al. (2004). While these studies provide a solid foundation for melanoma genetics, there is no marker that can clearly differentiate melanoma from benign tissue. Several markers currently used such as tyrosinase and Mart-1 cannot discriminate between benign and malignant tissue. Takeuchi et al. (2004). Consequently, these markers have limited use in applications such as intra-operative, lymph-node-based staging of disease.

Difficulties in obtaining sufficient RNA samples from malignant melanoma and benign melanocytic lesions, tissue heterogeneity, and the presence of melanin in purified RNA remain the major challenges in these studies. In the study presented herein, total RNA isolated from 45 primary malignant melanomas, 18 benign skin nevi, and 7 normal skin tissues were hybridized on an Affymetrix Hu133A microarray containing 22,000 probe sets. A modified RNA extraction method was developed to produce melanin-free RNA samples that increased the micorarray hybridization signals. Hierarchical clustering revealed distinct separation of the melanoma samples from benign and normal specimens. Significance Analysis of Microarray (SAM) method, *t*-test and percentile analysis identified 439 up-regulated (SEQ ID NOs: 29-467) and 511 down-regulated (SEQ ID NOs: 468-978) genes in the melanoma samples. Besides well-characterized genes such as me20m (gp100), melanocortin receptor 1, and L1CAM, many novel genes previously unassociated with melanoma were identified including NTKR3 and PLAB. Pathway analysis of the differentially expressed genes revealed an over-representation of genes associated with neural tissue development and function, activation of amyloid processing and integrin signalling pathways. RT-PCR assays were performed to confirm the differential expression of the selected genes.

The methods provided have sufficient specificity and sensitivity to detect metastasis of melanoma. A comparison of the current methods available indicates that tradition methods of H&E and IHC are clinically acceptable whereas, prior to the current invention, PCR methods were unacceptable. Table 1 shows the drawbacks and advantages of current methods prior to the invention claimed herein.

**Table 1**

| Method | Sensitivity | Specificity |
|---|---|---|
| H&E | Low | 100% |
| IHC | Low | 100% |
| PCR | High | Low |

In the present invention, specificity is preferably at least 95%, more preferably, specificity is at least 97% and most preferably, specificity is at least 99% based on a comparison of H&E and IHC negative nodes. Preferably, sensitivity is at least at least 80%, more preferably sensitivity is at least 85% and most preferably, sensitivity is at least 90% based on a comparison of H&E and IHC positive nodes. Preferably, specificity and sensitivity are at least 97% based on a comparison of H&E and IHC negative nodes and at least 85% based on a comparison of H&E and IHC positive nodes, respectively.

Preferably, the pre-determined cut-off levels are at least two-fold over-expression in tissue having metastatic melanoma relative to benign melanocyte or normal tissue.

The preferred methods of the application employ a rapid technique for extracting nucleic acids from a tissue sample and a method of amplifying and detecting nucleic acid fragments indicative of metastasis. The nucleic acid fragments qualitatively and quantitatively measure mRNA encoded by the Marker genes. Tissue samples include lymph node, both regional and sentinel, skin lesions and other biopsy material.

The methods provided herein allow for intra-operative detection of micrometastases allowing a physician to determine whether to excise additional lymph nodes and to immediately implement an appropriate treatment protocol. As shown in Table 2, if a LN is found to be positive for melanoma, regional LNs are excised and interferon therapy could be suggested. Standard biopsy methods can take over one week and a positive result requires additional surgery to remove LNs and there is a concomitant delay in interferon therapy.

**Table 2**

| Clinical Stage | 1° Tumor (T) | LN | Metastasis | Treatment |
|---|---|---|---|---|
| Stage I | T1: ≤ 1 mm | Negative | Absent | Excision 1 cm |
| | T2: 1.01-2.00 mm | Negative | Absent | Excision 1-2 cm |
| Stage II | T3: 2.01-4.00 mm | Negative | Absent | Excision 2 cm |
| | T4: > 4.01 mm | Negative | Absent | Excision 2 cm |
| Stage III | Any thickness | Positive | Absent | Excision + complete LN dissection + interferon clinical trial |
| Stage IV | Any thickness | Positive | Present | Interferon clinical trial, symptomatic therapy |

It is important to adequately sample the tissue used to conduct the assay. This includes proper excision and processing of the tissue sample as well as extraction of RNA. Once obtained, it is important to process the tissue samples properly so that any cancerous cells present are detected.

In the most preferred embodiment, node sampling is also given attention both intra- and extra-operatively. Since the distribution of cancer cells in nodes is non-uniform, it is preferable that multiple sections of the node be sampled. Every identified SLN should be submitted for pathological evaluation. SLN material is ordinarily be fixed in formalin and examined as formalin fixed, paraffin embedded tissue sample. Equally representative parts of SLN are processed for molecular analysis (fresh tissue) and histology (fixed tissue). General LN sampling procedures are described in Cochran et al. (2001); and Cochran et al. (2004). One method for accomplishing both a molecular based test and an examination of the same node sample by pathology is to bisect the node through the longest diameter. Each half is then divided into at least four full-faced sections with at least one outer and inner section for pathology as fixed material, and at least one outer and inner section for molecular testing. As the distribution of metastases and micrometastases is not uniform in nodes or other tissues, a sufficiently large sample should be obtained so that metastases will not be missed. One approach to this sampling issue in the present method is to homogenize a large tissue sample, and subsequently perform a dilution of the well-mixed homogenized sample to be used in subsequent molecular testing.

In the case of LN tissue samples, it is preferable to remove any adipose tissue prior to cellular disruption. Manual cell and tissue disruption can be by any means known in the art such as a disposable tissue grinder described in US Patent 4,715,545 or a commercial homogenizer such as Omni GLH115 with disposable probes (Omni International, Warrenton, VA). Homogenization time is within 1 to 2 minutes and is more preferably 30-45 sec. The sample can then be processed to purify the RNA prior to assaying and measuring Marker expression levels. Suitable RNA purification methods include columns such as (e.g., RNeasy mini column, QIAshredder, QIAGEN Inc., Valencia, CA, or a suitable substitute).

A variety of techniques are available for extracting nucleic acids from tissue samples. Typical commercially available nucleic acid extraction kits take at least 15 minutes to extract the nucleic acid. In the preferred intra-operative methods, nucleic acid is extracted in less than 8 minutes and preferably less than 6 minutes.

The successful isolation of intact RNA generally involves four steps: effective disruption of cells or tissue, denaturation of nucleoprotein complexes, inactivation of endogenous ribonuclease (RNase) and removal of contaminating DNA and protein. The disruptive and protective properties of guanidinium isothiocyanate (GITC) and β-mercaptoethanol (β-me) to inactivate the ribonucleases present in cell extracts make them preferred reagents for the first step. When used in conjunction with a surfactant such as sodium dodecylsulfate (SDS), disruption of nucleoprotein complexes is achieved allowing the RNA to be released into solution and isolated free of protein. Tissues are homogenized in the GITC-containing lysis buffer, addition of ethanol creates the appropriate conditions for RNA to bind to the silica membrane. Centrifugation can clear the lysate of precipitated proteins and cellular DNA and is preferably performed through a column. RNA purification is preferably conducted on a spin column containing silica or other material.

RNA is precipitated via the spin column as described above and centrifugation times are preferably no greater than 30 sec. Typically, the sample is diluted with an equal volume of 70% ethanol and thoroughly mixed prior to applying to the column. After washing, the column is dried by centrifugation, and RNA is eluted in RNase free water and collected by centrifugation. The total time of this rapid protocol is less than 8 minutes and preferably less than 6 min.

In summary the rapid RNA extraction method involves the following steps:
obtaining a tissue sample;
homogenizing the tissue to produce a homogenate;
contacting the homogenate with a substrate containing, or to which is affixed,
an RNA-binding material;
allowing the RNA to bind to the RNA binding material;
washing the substrate under conditions sufficient to remove any contaminants,
interferents and un-bound RNA; and
eluting bound RNA from the substrate.

The reagents involved in this rapid extraction process can be those provided by the manufacturer or can be, for instance:
LysisBinding buffer (preferably, 4.5M GITC, 100mM NaPO₄),
Wash buffer I (preferably, 37% ethanol in 5M GITC, 20mM Tris-HCl),
Wash buffer II (preferably, 80% ethanol in 20mM NaCl, 2mM Tris-HCl), and
Nuclease-free sterile double distilled water for elution.

In one method, prior to the process for isolating nucleic acids described above, tissue samples are weighed and put into 8 or 14 ml polypropylene culture tubes and pre-cooled on dry ice. The frozen tissue samples are then divided into pieces of about 50 mg or less without being thawed. All buffers are those provided by QIAGEN in the RNeasy mini kit. A volume of homogenization (lysis) buffer is added to the tissue based on Table 3.

**Table 3**

| Tissue Weight (mg) | Homogenization buffer (ml) |
|---|---|
| ≤ 100 | 2 |
| 100-149 | 2 |
| 150-199 | 3 |
| 200-249 | 4 |
| 250-299 | 5 |
| 300-349 | 6 |
| 350-399 | 7 |
| 400-449 | 8 |
| 450-400 | 9 |
| 500-550 | 10 |
| >550 | * |

| | |
|---|---|
| * Tissue above 550 mg is divided into equivalent parts and processed as individual samples. An alternative method to calculate lysis buffer volume for tissues over 100 mg is to add 1 ml per 50mg tissue; using 2 ml for tissues less than 100 mg. | |

The tissue sample is then homogenized for instance by the Omni GLH115 at a power setting to grade 6, Adaptor A1000 and disposable probes. The homogenate is then mixed with an equal volume of 70% ethanol and thoroughly mixed for instance by vortexing on a VWR Model G560 set at 10 speed (maximum) about 10 seconds or by pipetting 4-5 times. The homogenate/ethanol mixture is then applied to an RNeasy mini column mounted on a vacuum manifold in a volume in accordance with Table 4 so that a consistent amount of the original tissue (approximately 5 mg/column) is loaded thus producing comparable RNA yields for each tissue sample.

**Table 4**

| Tissue weight (mg) | Volume homogenate/ethanol mix (µl) (recommended) |
|---|---|
| 30-39 | 700 |
| 40-49 | 500 |
| 50-59 | 400 |
| 60-69 | 350 |
| 70-79 | 300 |
| 80-89 | 250 |
| 90-99 | 225 |
| > 100 | 200 |

A vacuum is then applied to the column to remove the liquid. The vacuum is stopped and two washes of 700 ml are applied, first with RWI buffer and second with RPE buffer each removed by filtration. Vacuum is at 800-1200 mBar in each case. The column is then placed into a 1.5 ml collection tube and centrifuged in an Eppendorf 5415D centrifuge at 13,200 rpm for 30 seconds to dry. The column is transferred to a new 1.5 ml collection tube. Fifty µl RNase-free water is directly added to the membrane and the column is centrifuged in an Eppendorf 5415D centrifuge for 30 seconds at 13,200 rpm to elute the RNA. The RNA quality is determined with an Agilent Bioanalyzer and the RNA is stored at -70°C.

Melanin can negatively impact the efficiency of reversed transcription and amplification reactions. Accordingly, a melanin removal process is undertaken when the sample is suspected of containing a significant amount of melanin (as in the case of samples of a primary melanoma or benign skin nevi) and is less of a concern when performing the assay on a SLN since melanocyte content is low. If necessary, melanin is removed to enhance reverse transcription and/or nucleic acid amplification.

Typically, melanin is removed during the filtration steps provided above. In the case of tissue with high melanin concentration, less tissue should be used, approximately 5 mg per Qiagen RNeasy mini column.

If another method is used that results in residual melanin in the sample, removal involves the use of a matrix employing a polymer bead system such as Bio-Gel P-60 (Bio-Rad Laboratories, Hercules, CA). Such a method is described by Satyamoorthy et al. (2002). Essentially, this method involves preparing a 50% (w/v) mixture of the Bio-Gel material in 10 mM sodium acetate (pH 4.2). About 300 µl of the mixture are placed in a micro-centrifuge tube and centrifuged at 1000rpm for 1 min. The supernatant is discarded and the beads are placed in a mini-column or similar vessel. Homogenate is then passed through the vessel containing the beads (after first incubating them in the vessel). The supernatant is collected. Further washing of the beads with additional 100 µl aliquots of 10 mM sodium acetate can be used to capture additional volumes of melanin-free sample if necessary for adequate assay volume. The dark melanin will be clearly visible on the beads retained in the vessel. Other silica-based filters can also be used to remove the melanin pigment as described by Wang et al. (2001).

An important aspect of the intra-operative methods is rapid Marker detection. Provided that such methods can be conducted within a period acceptable for an intra-operative assay (i.e., no more than about 35 minutes), any reliable, sensitive, and specific method can be used.

In the case of measuring mRNA levels to determine gene expression, assays can be by any means known in the art and include methods such as PCR, Rolling Circle Amplification (RCA), Ligase Chain Reaction (LCR), Strand Displacement Amplification (SDA), Nucleic Acid Sequence Based Amplification (NASBA), and others. The rapid molecular diagnostics involved are most preferably quantitative PCR methods, including QRT-PCR. Detection can be by any method known in the art including microarrays, gene chips and fluorescence.

A typical PCR includes multiple amplification steps, or cycles that selectively amplify target nucleic acid species. A typical PCR includes three steps: a denaturing step in which a target nucleic acid is denatured; an annealing step in which a set of PCR primers (forward and backward primers) anneal to complementary DNA strands; and an elongation step in which a thermostable DNA polymerase elongates the primers. By repeating this step multiple times, a DNA fragment is amplified to produce an amplicon, corresponding to the target DNA sequence. Typical PCR includes 20 or more cycles of denaturation, annealing and elongation. Often, the annealing and elongation steps can be performed concurrently, in which case the cycle contains only two steps.

In the preferred method, employing RT-PCR, the RT-PCR amplification reaction is conducted in a time suitable for intra-operative diagnosis, the lengths of each of these steps can be in the seconds range, rather than minutes. Specifically, with certain new thermal cyclers being capable of generating a thermal ramp rate of at least about 5C° per second, RT-PCR amplifications in 30 minutes or less are used. More preferably, amplifications are conducted in less than 25 minutes. With this in mind, the following times provided for each step of the PCR cycle do not include ramp times. The denaturation step may be conducted for times of 10 seconds or less. In fact, some thermal cyclers have settings for "0 seconds" which may be the optimal duration of the denaturation step. That is, it is enough that the thermal cycler reaches the denaturation temperature. The annealing and elongation steps are most preferably less than 10 seconds each, and when conducted at the same temperature, the combination annealing/elongation step may be less than 10 seconds. Some homogeneous probe detection methods, may require a separate step for elongation to maximize rapid assay performance. In order to minimize both the total amplification time and the formation of non-specific side reactions, annealing temperatures are typically above 50°C. More preferably annealing temperatures are above 55°C.

A single combined reaction for RT-PCR, with no experimenter intervention, is desirable for several reasons: (1) decreased risk of experimenter error; (2) decreased risk of target or product contamination; and (3) increased assay speed. The reaction can consist of either one or two polymerases. In the case of two polymerases, one of these enzymes is typically an RNA-based DNA polymerase (reverse transcriptase) and one is a thermostable DNA-based DNA polymerase. To maximize assay performance, it is preferable to employ a form of "hot start" technology for both of these enzymatic functions. US Patents 5,411,876 and 5,985,619 provide examples of different "hot start" approaches. Preferred methods include the use of one or more thermoactivation methods that sequester one or more of the components required for efficient DNA polymerization. US Patents 5,550,044 and 5,413,924 describe methods for preparing reagents for use in such methods. US Patent 6,403,341 describes a sequestering approach that involves chemical alteration of one of the PCR reagent components. In the most preferred embodiment, both RNA- and DNA-dependent polymerase activities reside in a single enzyme. Other components that are required for efficient amplification include nucleoside triphosphates, divalent salts and buffer components. In some instances, non-specific nucleic acid and enzyme stabilizers may be beneficial.

In the preferred RT-PCR, the amounts of certain reverse transcriptase and the PCR components are atypical in order to take advantage of the faster ramp times of some thermal cyclers. Specifically, the primer concentrations are very high.

Typical gene-specific primer concentrations for reverse transcriptase reactions are less than about 20 nM. To achieve a rapid reverse transcriptase reaction on the order of one to two minutes, the reverse transcriptase primer concentration is raised to greater than 20 nM, preferably at least about 50 nM, and typically about 100 nM. Standard PCR primer concentrations range from 100 nM to 300 nM. Higher concentrations may be used in standard PCR to compensate for Tm variations. However, for the purposes herein, the referenced primer concentrations are for circumstances where no Tm compensation is needed. Proportionately higher concentrations of primers may be empirically determined and used if Tm compensation is necessary or desired. To achieve rapid PCR, the PCR primer concentrations typically are greater than 250 nM, preferably greater than about 300 nM and typically about 500 nM.

Commercially used diagnostics also preferably employ one or more internal positive control that confirms the operation of a particular amplification reaction in case of a negative result. Potential causes of false negative results that must be controlled in an RT-PCR include: inadequate RNA quantity, degradation of RNA, inhibition of RT and/or PCR and experimenter error.

In the case of measuring protein levels to determine gene expression, any method known in the art is suitable provided it results in adequate specificity and sensitivity. For example, protein levels can be measured by binding to an antibody or antibody fragment specific for the protein and measuring the amount of antibody-bound protein. Antibodies can be labeled by radioactive, fluorescent or other detectable reagents to facilitate detection. Methods of detection include, without limitation, enzyme-linked immunosorbent assay (ELISA) and immunoblot techniques.

The invention provides specificity and sensitivity sufficient to identify a malignant melanocyte in a tissue sample. The methods determine expression of particularly Marker genes by measuring mRNA encoded by the Markers. The preferred Markers of the invention display at least a two-fold over-expression in tissue having malignant melanocytes relative to benign melanocyte or normal tissue. The results presented herein show that a primary Marker is insufficient to provide clinically relevant information but, when combined with one or more secondary Markers, the information obtained compares to the "gold standard" of H&E and IHC upon which clinicians currently rely. Tertiary Markers and control genes can augment the primary and secondary Markers to further increase specificity and/or sensitivity.

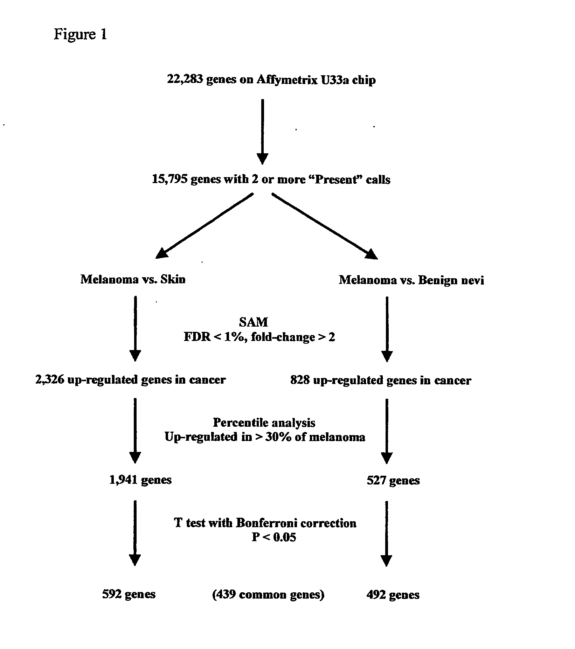

As described in the following Examples, the Markers were identified by the protocol depicted in Figure 1. Thus, the invention provides a method for identifying melanoma-specific Markers by following the protocol in Figure 1 and the Examples provided herein.

The primary Marker can be PLAB and is defined herein as the gene encoding any variant, allele etc. including SEQ ID NO: 1. PLAB is also described by Paralkar et al. (1998) and represented by Accession No. AF003934. PLAB is linked to the pathogenesis of prostate cancer (Liu et al (2003); Karan et al. (2003); and Nakamura et al. (2003); US Patent Nos. 5,994,102; 6,107,476; 6,465,181; 6,500,638; 6,521,227; US Patent Publication Nos. 2002/0048784; 2003/0013097; and 2003/0059431) and colorectal cancer (Brown et al. (2003); Buckhaults et al. (2001); and US Patent Publication No. 2002/0160382).

The secondary Marker is L1CAM and is defmed herein as the gene encoding any variant, allele etc. including SEQ ID NO: 2. L1CAM is also described by Haspel et al (2003); and US Patent Nos. 5,872,225; and 5,969,124 and is represented by Accession No. NM_000425.

The invention further provides tertiary markers that fall into several functional categories. Thus, additional Markers can be used that are found in these functional categories. As described in more detail in the Examples, melanoma-specific up-regulated genes fall into the functional categories of neural tissue development and cell cycle control and melanoma-specific down-regulated genes fall into the functional categories of tissue development and cell differentiation.

The tertiary Markers include SEQ ID NOs: 3, 29-978 and 999. A number of tertiary markers are described in Table 5 and all are summarized in Table 15.

NTRK3 is described by Strausberg et al. (2002); Marchetti et al. (2003); Hisaoka et al. (2002); McGregor et al. (1999); Ryden et al. (1996); US Patent Nos. 5,348,856; 5,844,092; 5,910,574; and US Patent Publication Nos. 2002/0155480; and 2003/014283 and is represented by Accession No. BC013693 or S76476.1. NTRK3 is also defined as the gene encoding mRNA recognized by the primer/probe sets SEQ ID NOs: 16-18.

Tyrosinase is described by Mandelcorn-Monson et al. (2003); and US Patent No. 6,153,388 and is represented by Accession No. NM_000372. Tyrosinase is also defined as the gene encoding mRNA recognized by the primer/probe sets SEQ ID NOs: 19-21.

**Table 5**

| Gene | Reference | Accession # |
|---|---|---|
| PBGD | Raich et al. (1986) | NM_000190 |
| CITED1 | Fenner et al. (1998) | NM_004143 |
| PEX6 | Raas-Rothschild et al. (2002) | NM_000287 |
| CAPG | Van Impe et al. (2003) | NM_001747 |
| DUSP4 | Smith et al. (1997) | NM_001394 |
| GDF1 | Ducy et al. (2000) | NM_001492 |
| E2-EpF | Liu et al. (1992) | NM_014501 |
| me20m | Maresh et al. (1994) | U01874 |
| CDH3 | Patel et al. (2003) | NM_001793 |
| SMARCD3 | Ring et al. (1998) | NM_003078 |
| PKM2 | Luftner et al. (2003) | NM_002654 |
| GPI | Tsutsumi et al. (2003) | NM_000175 |
| Pig10 | Polyak et al. (1997) | AF010413 |
| CPEB1 | Welk et al. (2001) | NM_030594 |
| HOXHB9 | Catala et al. (2002) | AI738662 |
| Truncated calcium binding protein | The Washington University-Merck EST Project Hillier et al. (1995) | N30649 |
| SAAS | Kikuchi et al. (2003) | NM_013271 |
| HS1-2 | Edgar et al. (2002) | NM_007011 |
| HCN2 | Stieber et al. (2003) | NM_001194 |
| MBP | Kamholz et al. (1996) | M13577 |
| AD3LPAD5 | Li et al. (1995) | U34349 |
| PLOD3 | Wang et al. (2002) | NM_001084 |
| MC1R | Salazar-Onfray et al. (2002) | |
| MIF | Shimizu et al. (1999) | NM_002415 |
| HOXB7 | Care et al. (1996) | NM_004502 |
| AIM1 | Ray et al. (1997) | XM_166300 |
| EpHB6 | Hafner et al. (2003) | NM_004445 |
| AKT1 | Majumder et al. (2004) | NM_005163 |
| AKT2 | Gosmanov et al. (2004) | NM_001626 |
| AKT3 | Xu et al. (2003) | NM 005465 |
| APH-1A | Xu et al. (2003) | NM_016022 |
| APP | Masters et al. (1985) | NM_201414 |
| BACE | Pastorino et al. (2004) | NM_138973 |
| BACE2 | Pastorino et al. (2004) | NM_012104 |
| CAPN1 | Altznauer et al. (2004) | NM_005186 |
| CAPN2 | Alexa et al. (2004) | NM_001748 |
| CDK5 | Qi et al. (2004) | NM_004935 |
| CDK5R1 | Kam et al. (2004) | NM_003885 |
| CSNK1A1 | Burzio et al. (2002) | NM_001892 |
| CSNK1D | Li et al. (2004) | NM_139062 |
| CSNK1E | Swiatek et al. (2004) | NM_152221 |
| CSNK2A1 | Hilgard et al. (2004) | NM_001895 |
| CSNK2A2 | Szebeni et al. (2003) | NM_001896 |
| CSN2K2B | Lee et al. (2004) | NM_001320 |
| GSK3B | Chen et al. (2003) | NM_182946 |
| MAPK1 | Nishihara et al. (2004) | NM_138957 |
| MAPK14 | Bendotti et al. (2004) | NM_139014 |
| MAPK3 | Nishihara et al. (2004) | NM_002746 |
| MAPT | Yu et al. (2004) | NM_016841 |
| NCSTN | Shirotani et al. (2004) | NM_015331 |
| PEN2 | Marlow et al. (2003) | NM_172341 |
| PRKACA | Sakwe et al. (2004) | NM_207518 |
| PRKACB | Dwivedi et al. (2004) | NM_002731 |
| PRKACG | Zhang et al. (2004) | NM_002732 |
| PRKAR1A | Gronholm et al. (2003) | NM_212472 |
| PRKAR2A | MacDougall et al. (2003) | NM_004157 |
| PRKAR2B | Dwivedi et al. (2004) | NM_002736 |
| PRKCE | Schechtman et al. (2004) | NM_005400 |
| PSEN1 | Pitsi et al. (2004) | NM_000021 |
| PSEN2 | Zatti et al. (2004) | NM_012486 |
| PSFL | Clark et al. (2003) | NM_031301 |
| ABL1 | Gustafson et al. (2004) | NM_007313 |
| ACK1 | Ahmed et al. (2004) | NM_005781 |
| ACTN4 | Menez et al. (2004) | NM_004924 |
| ARF1 | Kadaja et al. (2004) | NM_001658 |
| ARPC1B | Kaneda et al. (2002) | |
| BCAR3 | Clark et al. (2003) | NM_003567 |
| BRAF | Sasaki et al. (2004) | NM_004333 |
| CDC42 | Chen et al. (2004) | NM_044472 |
| CRK | Stoletov et al. (2004) | NM_016823 |
| CRKL | Zhang et al. (2003) | NM_005207 |
| DDEF1 | Oda et al. (2003) | NM_018482 |
| DOCK1 | Grimsley et al. (2004) | NM_001380 |
| FYN | Lee et al. (2004) | NM_153048 |
| GIT1 | Haendeler et al. (2003) | NM_014030 |
| GRB2 | Zhou et al. (2004) | NM_203506 |
| GRF2 | Arozarena et al. (2004) | NM_006909 |
| HRAS | Nomura et al. (2004) | NM_005343 |
| JUN | Schmuth et al. (2004) | NM_002228 |
| KRAS2 | Qi et al. (2004) | NM_033360 |
| MAP2K1 | Rhee et al. (2004) | NM_002755 |
| MAP2K2 | Chen et al. (2004) | NM_030662 |
| MAP2K4 | Woo et al. (2004) | NM_003010 |
| MAP3K11 | Zhang et al. (2004a) | NM_002419 |
| MAPK8 | Fujii et al. (2004) | NM_139049 |
| MYLK | Oury et al. (2004) | NM_053032 |
| NRAS | Reifenberger et al. (2004) | NM_002524 |
| PAK1 | Sells et al. (1997) | HSU24152 |
| PAK2 | Kirchhoff et al. (2004) | NM_002577 |
| PAK3 | Kitano et al. (2003) | NM_002578 |
| PAK4 | Barac et al. (2004) | NM_005884 |
| PAK6 | Ching et al. (2003) | NM_020168 |
| PAK7 | Jaffer et al. (2002) | NM_020341 |
| PTK2 | Golubovskaya et al. (2004) | NM_005607 |
| PXN | Saito et al. (2004) | NM_002859 |
| RAC1 | Pontow et al. (2004) | NM_198829 |
| RAF1 | Akula et al. (2004) | NM_002880 |
| RAP1A | Nomura et al. (2004) | NM_002884 |
| RAP2B | Evellin et al. (2002) | NM_002886 |
| SHC1 | Yannoni et al. (2004) | NM_183001 |
| SOS1 | Buchs et al. (2004) | NM_005633 |
| SRC | Encinas et al. (2004) | NM_198291 |
| TLN1 | Tremuth et al. (2004) | NM_006289 |
| VASP | Tokuo et al. (2004) | NM_003370 |
| VCL | Izard et al. (2004) | NM_003373 |
| WASPIP | Luthi et al. (2003) | NM_003387 |
| ZYX | Li et al. (2004) | NM_003461 |

Tertiary Markers are able to replace and/or supplement primary or secondary Markers provided that the resulting assays have adequate sensitivity and specificity.

The specificity of any given amplification-based molecular diagnostic relies heavily, but not exclusively, on the identity of the primer sets. The primer sets are pairs of forward and reverse oligonucleotide primers that anneal to a target DNA sequence to permit amplification of the target sequence, thereby producing a target sequence-specific amplicon. The primers must be capable of amplifying Markers of the disease state of interest. In the case of the instant invention, these Markers are directed to melanoma.

The reaction must also contain some means of detection of a specific signal. This is preferably accomplished through the use of a reagent that detects a region of DNA sequence derived from polymerization of the target sequence of interest. Preferred reagents for detection give a measurable signal differential when bound to a specific nucleic acid sequence of interest. Often, these methods involve nucleic acid probes that give increased fluorescence when bound to the sequence of interest. Typically, the progress of the reactions of the inventive methods are monitored by analyzing the relative rates of amplicon production for each PCR primer set.

The application further includes primer/probe sets and their use in the claimed methods. The sequences are:
SEQ ID NO:4 (PLAB forward primer) ggcagaatcttcgtccgca
SEQ ID NO:5(PLAB reverse primer) ggacagtggtccccgttg
SEQ ID NO:6 (PLAB probe) cccagctggagttgcacttgcggcc
SEQ ID NO:7 (PLAB upper primer) gaacaccgacctcgtccc
SEQ ID NO:8 (PLAB lower primer) ggcggcccgagagata
SEQ ID NO:9 (PLAB probe) cgccagaagtgcggctgggattt
SEQ ID NO:10 (L1CAM forward) gctgggactgggaacagaact
SEQ ID NO:11 (L1CAM Reverse) ggagcagagatggcaaagaaa
SEQ ID NO:12 (L1CAM probe) ttccccaccatctgctgt
SEQ ID NO:13 (L1CAM upper) ccacagatgacatcagcctcaa
SEQ ID NO:14 (L1CAM lower) ggtcacacccagctcttcctt
SEQ ID NO:15 (L1CAM probe) tggcaagcccgaagtgcagttcctt
SEQ ID NO:16 (NTRK3 primer) gccccggcacccttta
SEQ ID NO:17 (NTRK3 primer) aaccctgccagtggtggat
SEQ ID NO:18 (NTRK3 probe) cagatgggtgttttc
SEQ ID NO:19 (Tyr upper) actcagcccagcatcattcttc
SEQ ID NO:20 (Tyr lower) atggctgttgtactcctccaatc
SEQ ID NO:21 (Tyr probe) cttctcctcttggcagattgtctgtagctt
SEQ ID NO:22 (PBGD upper) ccacacacagcctactttccaa
SEQ ID NO:23 (PBGD lower) tacccacgcgaatcactctca
SEQ ID NO:24 (PBGD probe) aacggcaatgcggctgcaacggcggaatt

Monitoring amplicon production may be achieved by a number of detection reagents and methods, including without limitation, fluorescent primers, and fluorogenic probes and fluorescent dyes that bind double-stranded DNA. Molecular beacons, Scorpions, and other detection schemes may also be used. A common method of monitoring a PCR employs a fluorescent hydrolysis probe assay. This method exploits the 5' nuclease activity of certain thermostable DNA polymerases (such as Taq or Tfl DNA polymerases) to cleave an oligomeric probe during the PCR process.

The further provides amplicons obtained by PCR methods utilized in the inventive methods. These amplicons include the following: SEQ ID NO:27 (tyrosinase Amplicon)
actcagcccagcatcattcttctcctcttggcagattgtctgtagccgattggaggagtacaacagccat

The oligomer is selected to anneal to the amplified target sequence under elongation conditions. The probe typically has a fluorescent reporter on its 5' end and a fluorescent quencher of the reporter at the 3' end. So long as the oligomer is intact, the fluorescent signal from the reporter is quenched. However, when the oligomer is digested during the elongation process, the fluorescent reporter is no longer in proximity to the quencher. The relative accumulation of free fluorescent reporter for a given amplicon may be compared to the accumulation of the same amplicons for a control sample and/or to that of a control gene, such as, without limitation, β-Actin or PBGD to determine the relative abundance of a given cDNA product of a given RNA in a RNA population. Products and reagents for the fluorescent hydrolysis probe assay are readily available commercially, for instance from Applied Biosystems.

Suitable detection reagents are commonly referred to as "Scorpions" and are described in US Patents 6,326,145 and 5,525,494. These reagents include one or more molecules comprising a tailed primer and an integrated signaling system. The primer has a template binding region and a tail comprising a linker and a target binding region. The target binding region in the tail hybridizes to complementary sequence in an extension product of the primer. This target specific hybridization event is coupled to a signaling system wherein hybridization leads to a detectable change. In PCR the target binding region and the tail region are advantageously arranged such that the tail region remains single stranded, i.e. uncopied. Thus the tail region is non-amplifiable in the PCR amplification products. The linker comprises a blocking moiety that prevents polymerase mediated chain extension on the primer template.

The most preferred detection reagents are TaqMan® probes (Roche Diagnostics, Branchburg, NJ) and they are described in US Patents 5,210,015; 5,487,972; and 5,804,375. Essentially, these probes involve nucleic acid detection by virtue of the separation of a fluor-quencher combination on a probe through the 5'-3' exonuclease activity of the polymerase used in the PCR. Any suitable fluorophore can be used for any of the Markers or controls. Such fluorophores include, without limitation, Texas Red, Cal Red, Fam, Cy3 and Cy5. In one embodiment, the following fluorophores correspond to the noted Markers: PLAB: Fam; L1CAM: Texas Red or Cal Red, tyrosinase: C1; PBGD: Cy5.

Equipment and software also are readily available for controlling and monitoring amplicon accumulation in PCR and QRT-PCR including the Smart Cycler thermocylcer commercially available from Cepheid of Sunnyvale, California, and the ABI Prism 7700 Sequence Detection System, commercially available from Applied Biosystems.

In the case of gene expression assays, it is preferable to use a gene constitutively expressed in the tissue of interest. PBGD is commonly used as an internal control due to several factors: it contains no known pseudogenes in humans, it is constitutively expressed in human tissues and it is expressed at a relatively low level and therefore is less likely to cause inhibition of the amplification of target sequences of interest. Use of PBGD as a control minimizes or eliminates reporting erroneous results arising from all potential sources of false negative results.

In the commercialization of the described methods for QRT-PCR certain kits for detection of specific nucleic acids are particularly useful. In one embodiment, the kit includes reagents for amplifying and detecting Markers. Optionally, the kit includes sample preparation reagents and or articles (e.g., tubes) to extract nucleic acids from lymph node tissue. The kits may also include articles to minimize the risk of sample contamination (e.g., disposable scalpel and surface for lymph node dissection and preparation).

In a preferred kit, reagents necessary for the one-tube QRT-PCR process described above are included such as reverse transcriptase, a reverse transcriptase primer, a corresponding PCR primer set (preferably for Markers and controls), a thermostable DNA polymerase, such as Taq polymerase, and a suitable detection reagent(s), such as, without limitation, a scorpion probe, a probe for a fluorescent hydrolysis probe assay, a molecular beacon probe, a single dye primer or a fluorescent dye specific to double-stranded DNA, such as ethidium bromide. The primers are preferably in quantities that yield the high concentrations described above. Thermostable DNA polymerases are commonly and commercially available from a variety of manufacturers. Additional materials in the kit may include: suitable reaction tubes or vials, a barrier composition, typically a wax bead, optionally including magnesium; reaction mixtures (typically 10X) for the reverse transcriptase and the PCR stages, including necessary buffers and reagents such as dNTPs; nuclease-or RNase-free water; RNase inhibitor; control nucleic acid(s) and/or any additional buffers, compounds, co-factors, ionic constituents, proteins and enzymes, polymers, and the like that may be used in reverse transcriptase and/or PCR stages of QRT-PCR. Optionally, the kits include nucleic acid extraction reagents and materials. Instructions are also preferably included in the kits.

The following examples are provided to illustrate but not limit the claimed invention.

### Example 1

### Tissue Preparation

Fresh frozen malignant melanoma, benign skin nevi, normal skin, melanoma lymph node metastasis and melanoma-free lymph node samples were obtained from Genomics Collaborative, Inc. (Cambridge, MA), Asterand (Detroit, MI), Clinomics (Pittsfield, MA) and Proteogenex (Los Angeles, CA), Ardais (Lexington, MA) and Impath (Westborough, MA). All tissue vendors declared that tissue specimens used in the study were collected according to an Institutional Review Board approved protocol of corresponding hospitals and principles of bioethics. Patients' demographic and pathology information was also collected. The histopathological features of each sample were reviewed to confirm diagnosis, and to estimate sample preservation and tumor content.

Melanoma and benign nevi primary tissues chosen for microarray analysis had melanocyte content greater than 50% with no mixed histology. Melanoma positive lymph nodes were collected from malignant melanoma patients; diagnosis of melanoma was confirmed by H&E in combination with IHC (S100 and HMB45). Melanoma free lymph nodes derived from patients that did not have melanoma in their clinical history and absence of melanoma was confirmed by H&E and IHC using antibodies for S100 and HMB45.

RNA from a total of 70 primary tissue samples was used for gene expression profiling and melanoma specific gene identification. Samples included 45 primary malignant melanoma, 18 benign skin nevi, and 7 normal skin tissues. The majority of primary melanomas included in the study represent early stage of disease and have thickness less than 4 mm, which is consistent with the standard melanoma patient population. Aitken et al. (2004). Patient demographic, clinical and pathology characteristics are presented in Table 6 and summarized in Table 7.

In addition, 77 malignant melanoma LN metastasis and 18 melanoma-free LN tissue samples were used for one-step quantitative PCR assay. Melanoma positive lymph nodes included axillary, cervical and inguinal lymph nodes with metastasis derived from epithelioid and spindle cell primary melanomas. Out of 18 melanoma free LN, 10 were collected from other cancer patients but no cancer cells were found in these nodes by pathologists and 8 LN were from non-malignant lesions.

**Table 6**

| Sample ID | Age | Gender | Race | Diagnosis | Location | T Stage | N&M Stage | Clark level |
|---|---|---|---|---|---|---|---|---|
| 430MM | n/a | F | Cau | normal skin | trunk | | | |
| 431 MM | n/a | F | Cau | normal skin | trunk | | | |
| 432MM | n/a | F | Cau | normal skin | trunk | | | |
| 433MM | n/a | F | Cau | normal skin | trunk | | | |
| 435MM | n/a | F | Cau | normal skin | trunk | | | |
| 437MM | n/a | F | Cau | normal skin | trunk | | | |
| 485MM | 37 | M | Cau | normal skin | skin, NOS | | | |
| 487MM | 35 | F | Cau | atypical nevus, mild | face | | | |
| 489MM | 56 | F | Cau | compound nevus | face | | | |
| 490MM | 16 | F | Cau | compound nevus | scalp & neck | | | |
| 491 MM | 15 | M | Cau | compound nevus | trunk | | | |
| 493MM | 35 | F | Cau | compound nevus | trunk | | | |
| 495MM | 18 | F | Cau | benign nevus, NOS | trunk | | | |
| 496MM | 21 | F | Cau | intradermal nevus | lower limb & hip | | | |
| 497MM | 12 | M | Cau | intradermal nevus | lower limb & hip | | | |
| 498MM | 44 | F | Cau | benign nevus, NOS | trunk | | | |
| 499MM | 48 | F | Cau | benign nevus, NOS | face | | | |
| 500MM | 65 | M | Cau | intradermal nevus | trunk | | | |
| 501 MM | 30 | M | Cau | compound nevus | lower limb & hip | | | |
| 502MM | 20 | F | Cau | compound nevus | trunk | | | |
| 503MM | 35 | M | Cau | intradermal nevus | lower limb & hip | | | |
| 504MM | 23 | M | Cau | compound nevus | trunk | | | |
| 507MM | 53 | M | Cau | atypical nevus, moderate | trunk | | | |
| 508MM | 28 | M | Cau | compound nevus | trunk | | | |
| 509MM | 43 | M | Cau | intradermal nevus | trunk | | | |
| 392MM | 58 | F | Cau | epithelioid melanoma | trunk | T3 | N0M0 | 4 |
| 397MM | 51 | F | Cau | epithelioid melanoma | lower limb & hip | T2 | N0M0 | 3 |
| 405MM | 46 | M | Cau | epithelioid melanoma | upper limb & shoulder | T2 | N0M0 | 3 |
| 407MM | 64 | F | Cau | epithelioid melanoma | trunk | T1 | N0M0 | 2 |
| 409MM | 54 | F | Cau | epithelioid melanoma | scalp & neck | T2 | N0M0 | 3 |
| 440MM | 61 | M | Cau | malignant melanoma, NOS | lower limb & hip | T1 | N0M0 | 2 |
| 441 MM | 78 | M | Cau | spindle cell melanoma | face | T4 | N0M0 | 5 |
| 442MM | 52 | M | Cau | malignant melanoma, NOS | upper limb & shoulder | T2 | N0M0 | 3 |
| 443MM | 51 | F | Cau | spindle cell melanoma | trunk | T2 | N0M0 | 3 |
| 444MM | 49 | F | Cau | spindle cell melanoma | lower limb & hip | T3 | N0M0 | 4 |
| 445MM | 76 | F | Cau | malignant melanoma, NOS | upper limb & shoulder | T3 | N0M0 | 4 |
| 446MM | 86 | M | Cau | malignant melanoma, NOS | scalp & neck | T1 | N0M0 | 2 |
| 447MM | 48 | M | Cau | epithelioid melanoma | skin, NOS | T3 | N0M0 | 4 |
| 448MM | 72 | F | Cau | epithelioid melanoma | upper limb & shoulder | T2 | N0M0 | 3 |
| 449MM | 62 | M | Asian | epithelioid melanoma | lower limb & hip | T3 | N1M0 | n/a |
| 450MM | 90 | F | Cau | epithelioid melanoma | upper limb & shoulder | T4 | N1M1 | n/a |
| 452MM | 43 | M | Cau | epithelioid melanoma | skin, NOS | T3 | N0M0 | n/a |
| 453MM | 48 | F | Cau | epithelioid melanoma | trunk | T3 | N0M0 | n/a |
| 454MM | 69 | M | Cau | epithelioid melanoma | upper limb & shoulder | T3 | N0M0 | n/a |
| 455MM | 55 | M | Cau | malignant melanoma, NOS | skin, NOS | T2 | N0M0 | n/a |
| 456MM | 63 | M | Cau | malignant melanoma, NOS | lower limb & hip | T2 | N0M0 | 3 |
| 457M | 69 | M | Cau | spindle cell melanoma | trunk | T1 | N0M0 | 2 |
| 459MM | 86 | F | Cau | malignant melanoma, NOS | lower limb & hip | T2 | N0M0 | 3 |
| 460MM | 64 | M | Cau | malignant melanoma, NOS | upper limb & shoulder | T3 | N0M0 | 4 |
| 461 MM | 66 | M | Cau | epithelioid melanoma | trunk | T1 | N0M0 | 2 |
| 463MM | 58 | M | Cau | malignant melanoma, NOS | trunk | T1 | N0M0 | 2 |
| 464MM | 53 | M | Cau | epithelioid melanoma | face | T2 | N0M0 | 3 |
| 465MM | 77 | F | Cau | epithelioid melanoma | upper limb & shoulder | T3 | N0M0 | 4 |
| 466MM | 79 | F | Cau | malignant melanoma, NOS | upper limb & shoulder | T1 | N0M0 | 2 |
| 468MM | 86 | F | Cau | spindle cell melanoma | upper limb & shoulder | T2 | N0M0 | 3 |
| 469MM | 43 | F | Cau | malignant melanoma, NOS | scalp & neck | T1 | N0M0 | 2 |
| 470MM | 81 | M | Cau | malignant melanoma, NOS | upper limb & shoulder | T2 | N0M0 | 3 |
| 472MM | 38 | F | Cau | spindle cell melanoma | upper limb & shoulder | T1 | N0M0 | 2 |
| 473MM | 69 | F | Cau | malignant melanoma, NOS | upper limb & shoulder | T1 | N0M0 | 3 |
| 475MM | 77 | F | Cau | malignant melanoma, NOS | face | T3 | N0M0 | 4 |
| 476MM | 87 | F | Cau | spindle cell melanoma | upper limb & shoulder | T3 | N0M0 | 4 |
| 477MM | 82 | M | Cau | malignant melanoma, NOS | scalp & neck | T2 | N0M0 | 3 |
| 478MM | 78 | F | Cau | epithelioid melanoma | face . | T3 | N0M0 | 4 |
| 480MM | 59 | M | Cau | malignant melanoma, NOS | upper limb & shoulder | T2 | N0M0 | 3 |
| 481 MM | 85 | M | Cau | malignant melanoma, NOS | upper limb & shoulder | T3 | N0M0 | 4 |
| 482MM | 66 | M | Cau | epithelioid melanoma | face | T3 | N0M0 | 4 |
| 483MM | 85 | F | Cau | epithelioid melanoma | trunk | T4 | N0M0 | 5 3 |
| 484MM | 70 | F | Cau | malignant melanoma, NOS | upper limb & shoulder | T1 | N0M0 | |
| 511 MM | 69 | M | Cau | epithelioid melanoma | skin, NOS | T3 | N1M0 | 4 |
| 512MM | 45 | M | Cau | epithelioid melanoma | trunk | T4 | N0M0 | 3 |

**Table 7**

| Characteristics | Melanoma (%) | Nevi (%) | Normal skin (%) |
|---|---|---|---|
| Mean Age | 65.51 ± 14.55 | 33.17 ± 15.60 | n/a |
| Gender | | | |
| Female | 22 (48.9) | 9 (50) | 6 |
| Male | 23 (51.1) | 0 (50) | 1 |
| Anatomical location | | | |
| Face | 5 (11.1) | 3 (17) | |
| Scalp and neck | 4 (8.() | 1 (6) | |
| Trunk | 9 (20) | 10 (55) | 6 (86) |
| Upper limb and shoulder | 17 (37.8) | | |
| Lower limb and hip | 6 (13.3) | 4 (22) | |
| Skin, NOS | 4 (8.9) | | 1 (14) |
| Histological diagnosis | | | |
| Epitheloid cell | 20 (44.4) | | |
| Spindle cell | 7 (15.6) | | |
| Malignant melanoma NOS | 18 (40) | | |
| Compound nevus | | 8 (44) | |
| Intradermal nevus | | 5 (28) | |
| Atypical nevus | | 2 (11) | |
| Benign nevus, NOS | | 3 (17) | |
| Normal skin | | | 7 (100) |
| T stage (thickness) | | | |
| T1 | 11 (24.4) | | |
| T2 | 14 (31.1) | | |
| T3 | 16 (35.6) | | |
| T4 | 4 (8.9) | | |
| N stage | | | |
| N0 | 42 (93.3) | | |
| N1 | 3 (6.7) | | |
| M stage | | | |
| M0 | 44 (97.8) | | |
| M1 | 1 (2.2) | | |
| Clark level | | | |
| 2 | 9 (20) | | |
| 3 | 16 (35/6) | | |
| 4 | 12 (26.7) | | |
| 5 | 2 (4.4) | | |
| n/a | 6 (13.3) | | |

### Example 2

### RNA Isolation from malignant melanoma and benign skin nevi samples

Qiagen RNeasy™ Mini Kit (QIAGEN Inc., Valencia, CA) was used, with a modified protocol to minimize the residual melanin in the RNA sample. For melanocyte containing tissues, four replicate tissue samples derived from individual patient each weighed approximately 5 mg and were used and processed separately. Tissue samples were homogenized in 1.0 ml RLT buffer (QIAGEN) containing 10 µl β-mercaptoethanol (Sigma Chemical Co., St. Louis, MO) by a mechanical homogenizer (UltraTurrex T8, IKA-Werke, Staufen, Germany). After homogenization, samples were loaded onto QIAGEN RNeasy™ columns and followed by centrifugation. After discarding the flow-through, 700 ml of RW1 buffer was added; the column was kept for 5 min at room temperature and then centrifuged. This step was repeated 3 times. Then the standard QIAGEN RNeasy™ Mini Kit protocol was followed. To remove RNA from the silica gel membrane, a two-step elution was performed. The total RNA derived from the same individual patient tissue was pooled and used for further analysis.

Standard Trizol protocol was used for RNA isolation from tissues that do not contain a significant proportion of melanocytes. Tissue was homogenized in Trizol reagent (Invitrogen, Carlsbad, CA). After centrifugation the top liquid phase was collected and total RNA was precipitated with isopropyl alcohol at -20°C. RNA pellets were washed with 75% ethanol, resolved in water and stored at -80°C until use. RNA quality was examined with an Agilent 2100 Bioanalyzer RNA 6000 Nano Assay (Agilent Technologies, Palo Alto, CA).

Labeled cRNA was prepared and hybridized with the high-density oligonucleotide array Hu133A Gene Chip (Affymetrix, Santa Clara, CA) containing a total of 22,000 probe sets according to the standard manufacturer protocol. Arrays were scanned using Affymetrix protocols and scanners. For subsequent analysis, each probe set was considered as a separate gene. Expression values for each gene were calculated by using Affymetrix Gene Chip analysis software MAS 5.0. All chips met three quality control standards: "present" call was greater than 35%, scale factor was smaller than 12 when scaled to a target intensity of 600, and background level was less than 150. Lower than usual percent of "present" calls cut-off was chosen because it is difficult to isolate RNA from skin cells (Hipfel et al. (1998)) resulting in lower overall gene expression levels.

### Example 3

### Data Analysis

Gene expression data were filtered to include only genes called "present" in 2 or more samples. This filter was used to remove genes that did not change expression in the samples. Of the 22,000 genes presented on the array, 15,795 passed this filter and were used for hierarchical clustering. Prior to clustering, each gene expression signal was divided by the median expression in al samples in the data set. This standardization step minimized the effect of the magnitude of gene expression and group together genes with similar expression patterns in the clustering analysis. Average linkage hierarchical clustering using Pearson correlation was performed on both the genes and the samples using GeneSpring 6.1.

In order to identify differentially expressed genes, we compared the melanoma samples to the benign nevi and the normal skin samples separately. The first analysis consisted of the 45 melanoma and 7 normal skin samples; the second analysis consisted of 45 melanoma and 18 nevi samples. These two datasets were analyzed separately in following procedures as shown in Fig. 1. Significance analysis of microarray (SAM; Tusher et al. (2001)) and Student T-test were used in gene selection. Parameters for SAM were set as Δ=2.5 and fold change = 2.0 with 1,000 permutations. FDR was 1%. There were no missing data and the default random number was used. Next percentile analysis was conducted. For up-regulated genes the 30%ile in melanoma samples was compared to the maximum of the normal samples, or that of nevi samples. Student T-test with Bonferroni correction was also performed with cut-off p<0.05 in order to ensure that the selected genes had significant differential expression between the two groups of the samples. As a final step, we identified common genes between the melanoma/benign and melanoma/normal gene lists resulting in the single list of genes upregulated in melanoma shown in Figure 1 where the 439 common genes correspond to SEQ ID NOs: 29-467 as described in Table 15 with the results shown in Table 8.

| Table 8 | | | |
|---|---|---|---|
| PSID | Median Expression in Melanoma | Fold Change (Cancer vs Benign) | Fold Change (Cancer vs skin) |
| 200078_s_at | 3954 | 2 | 3 |
| 200601_at | 9254 | 2 | 7 |
| 200612_s_at | 2396 | 2 | 5 |
| 200644_at | 7240 | 3 | 6 |
| 200660_at | 14659 | 3 | 4 |
| 200707_at | 3153 | 2 | 51 |
| 200736_s_at | 7305 | 3 | 3 |
| 200737_at | 2423 | 2 | 2 |
| 200783_s_at | 1028 | 2 | 2 |
| 200825_s_at | 3746 | 3 | 3 |
| 200827_at | 1593 | 2 | 2 |
| 200837_at | 5817 | 2 | 5 |
| 200838_at | 19225 | 8 | 17 |
| 200839_s_at | 28353 | 5 | 7 |
| 200859_x_at | 9665 | 3 | 7 |
| 200910_at | 7780 | 2 | 4 |
| 200950_at | 8419 | 3 | 7 |
| 200954_at | 3132 | 2 | 6 |
| 200966_x_at | 27388 | 2 | 3 |
| 200967_at | 6154 | 2 | 4 |
| 200968_s_at | 5587 | 2 | 7 |
| 200972_at | 8943 | 3 | 3 |
| 201038_s_at | 1480 | 2 | 2 |
| 201051_at | 5439 | 2 | 5 |
| 201105_at | 33285 | 4 | 5 |
| 201106_at | 7546 | 2 | 4 |
| 201188_s_at | 1730 | 2 | 8 |
| 201189_s_at | 3870 | 3 | 3 |
| 201195_s_at | 6005 | 4 | 18 |
| 201202_at | 1860 | 3 | 2 |
| 201251_at | 23965 | 5 | 11 |
| 201252_at | 901 | 2 | 3 |
| 201271_s_at | 1648 | 2 | 5 |
| 201291_s_at | 601 | 3 | 42 |
| 201313_at | 2414 | 5 | 6 |
| 201346_at | 3359 | 2 | 2 |
| 201393_s_at | 2166 | 2 | 5 |
| 201416_at | 4845 | 4 | 4 |
| 201417_at | 2905 | 2 | 4 |
| 201470_at | 18525 | 4 | 4 |
| 201474_s_at | 2471 | 5 | 22 |
| 201485_s_at | 1398 | 2 | 10 |
| 201486_at | 1105 | 2 | 3 |
| 201536_at | 2441 | 2 | 4 |
| 201614_s_at | 651 | 2 | 4 |
| 201660_at | 4713 | 7 | 3 |
| 201661_s_at | 3336 | 6 | 5 |
| 201662_s_at | 2693 | 5 | 3 |
| 201670_s_at | 3047 | 2 | 12 |
| 201714_at | 993 | 2 | 3 |
| 201765_s_at | 5475 | 4 | 5 |
| 201792_at | 3897 | 3 | 4 |
| 201804_x_at | 5609 | 3 | 3 |
| 201819_at | 2575 | 5 | 3 |
| 201850_at | 11103 | 10 | 20 |
| 201880_at | 1833 | 2 | 3 |
| 201910_at | 3854 | 4 | 4 |
| 201911_s_at | 2154 | 3 | 4 |
| 201931_at | 2578 | 3 | 2 |
| 201954_at | 25901 | 9 | 15 |
| 201976_s_at | 7697 | 2 | 6 |
| 202069_s_at | 855 | 2 | 3 |
| 202070_s_at | 2490 | 4 | 5 |
| 202111_at | 1380 | 3 | 9 |
| 202154_x_at | 10260 | 3 | 4 |
| 202185_at | 8493 | 4 | 6 |
| 202188_at | 1269 | 3 | 9 |
| 202219_at | 5630 | 3 | 7 |
| 202224_at | 2650 | 3 | 4 |
| 202225_at | 1534 | 2 | 2 |
| 202260_s_at | 7877 | 6 | 6 |
| 202295_s_at | 15129 | 6 | 4 |
| 202329_at | 3438 | 4 | 4 |
| 202367_at | 898 | 2 | 2 |
| 202370_s_at | 4669 | 3 | 2 |
| 202478_at | 7922 | 3 | 11 |
| 202503_s_at | 2424 | 3 | 5 |
| 202589_at | 3494 | 2 | 6 |
| 202603_at | 3772 | 3 | 2 |
| 202705_at | 1058 | 4 | 4 |
| 202737_s_at | 2865 | 3 | 4 |
| 202779_s_at | 3400 | 9 | 55 |
| 202785_at | 1134 | 2 | 6 |
| 202862_at | 2540 | 6 | 4 |
| 202898_at | 4736 | 4 | 63 |
| 202954_at | 1357 | 2 | 2 |
| 202958_at | 2401 | 4 | 3 |
| 202961_s_at | 19648 | 4 | 5 |
| 202986_at | 2052 | 3 | 28 |
| 203011_at | 1346 | 2 | 2 |
| 203022_at | 1068 | 2 | 2 |
| 203069_at | 560 | 15 | 13 |
| 203071_at | 958 | 19 | 3 |
| 203094_at | 968 | 2 | 2 |
| 203145_at | 513 | 2 | 2 |
| 203167_at | 2523 | 3 | 5 |
| 203217_s_at | 6416 | 3 | 5 |
| 203234_at | 725 | 3 | 3 |
| 203256_at | 7799 | 5 | 31 |
| 203262_s_at | 2482 | 2 | 2 |
| 203300_x_at | 5278 | 3 | 16 |
| 203315_at | 3273 | 2 | 2 |
| 203366_at | 847 | 2 | 2 |
| 203396_at | 3946 | 2 | 3 |
| 203452_at | 579 | 4 | 12 |
| 203456_at | 1132 | 2 | 3 |
| 203502_at | 657 | 3 | 3 |
| 203518_at | 2943 | 3 | 7 |
| 203554_x_at | 5056 | 3 | 4 |
| 203557_s_at | 839 | 2 | 2 |
| 203570_at | 2553 | 4 | 15 |
| 203590_at | 3093 | 3 | 7 |
| 203643_at | 1380 | 3 | 8 |
| 203663_s_at | 9720 | 5 | 3 |
| 203668_at | 1963 | 2 | 3 |
| 203693_s_at | 588 | 3 | 3 |
| 203695_s_at | 1548 | 6 | 3 |
| 203723_at | 4087 | 5 | 9 |
| 203729_at | 9154 | 2 | 14 |
| 203730_s_at | 517 | 3 | 3 |
| 203731_s_at | 691 | 3 | 2 |
| 203775_at | 1251 | 4 | 3 |
| 203827_at | 3380 | 11 | 8 |
| 203878_s_at | 2036 | 8 | 5 |
| 204014_at | 7184 | 10 | 46 |
| 204015_s_at | 2207 | 5 | 15 |
| 204033_at | 2154 | 7 | 7 |
| 204092_s_at | 664 | 3 | 3 |
| 204099_at | 2496 | 6 | 6 |
| 204170_s_at | 1362 | 3 | 4 |
| 204197_s_at | 2889 | 3 | 4 |
| 204198_s_at | 4024 | 3 | 3 |
| 204202_at | 875 | 3 | 3 |
| 204228_at | 1299 | 2 | 3 |
| 204244_s_at | 538 | 4 | 4 |
| 204247_s_at | 689 | 3 | 27 |
| 204252_at | 5294 | 5 | 7 |
| 204262_s_at | 1003 | 4 | 5 |
| 204423_at | 689 | 4 | 3 |
| 204436_at | 3113 | 3 | 3 |
| 204458_at | 908 | 3 | 3 |
| 204467_s_at | 1910 | 3 | 6 |
| 204584_at | 9677 | 21 | 15 |
| 204585_s_at | 805 | 13 | 17 |
| 204647_at | 1693 | 3 | 3 |
| 204654_s_at | 3894 | 4 | 25 |
| 204709_s_at | 312 | 24 | 16 |
| 204778_x_at | 537 | 2 | 4 |
| 204779_s_at | 1641 | 4 | 4 |
| 204857_at | 2736 | 3 | 297 |
| 204932_at | 241 | 3 | 3 |
| 204973_at | 1478 | 3 | 7 |
| 204995_at | 496 | 4 | 6 |
| 205051_s_at | 3875 | 4 | 3 |
| 205142_x_at | 937 | 2 | 3 |
| 205169_at | 267 | 2 | 7 |
| 205373_at | 579 | 10 | 8 |
| 205376_at | 815 | 2 | 3 |
| 205405_at | 2302 | 4 | 11 |
| 205447_s_at | 567 | 13 | 5 |
| 205458_at | 3288 | 6 | 8 |
| 205566_at | 2684 | 7 | 8 |
| 205591_at | 1190 | 5 | 3 |
| 205681_at | 1316 | 8 | 12 |
| 205690_s_at | 9179 | 8 | 9 |
| 205691_at | 226 | 7 | 5 |
| 205717_x_at | 8127 | 3 | 3 |
| 205813_s_at | 430 | 11 | 9 |
| 205937_at | 301 | 7 | 5 |
| 205945_at | 772 | 2 | 4 |
| 205996_s_at | 909 | 2 | 3 |
| 206128_at | 364 | 3 | 3 |
| 206307_s_at | 534 | 8 | 4 |
| 206332_s_at | 4671 | 2 | 2 |
| 206397_x_at | 1436 | 6 | 43 |
| 206441_s_at | 3681 | 6 | 31 |
| 206462_s_at | 9953 | 53 | 24 |
| 206503_x_at | 419 | 8 | 9 |
| 206617_s_at | 898 | 4 | 19 |
| 206630_at | 23194 | 3 | 46 |
| 206688_s_at | 2989 | 3 | 2 |
| 206696_at | 6446 | 7 | 191 |
| 206777_s_at | 683 | 4 | 7 |
| 206864_s_at | 421 | 5 | 5 |
| 206976_s_at | 3375 | 4 | 3 |
| 207038_at | 1986 | 9 | 47 |
| 207060_at | 497 | 4 | 5 |
| 207144_s_at | 593 | 17 | 24 |
| 207163_s_at | 3217 | 3 | 10 |
| 207183_at | 230 | 6 | 6 |
| 207592_s_at | 350 | 5 | 14 |
| 207614_s_at | 2139 | 2 | 6 |
| 207622_s_at | 882 | 2 | 16 |
| 207828_s_at | 997 | 3 | 3 |
| 208002_s_at | 3142 | 3 | 7 |
| 208089_s_at | 1374 | 3 | 6 |
| 208308_s_at | 12282 | 4 | 9 |
| 208540_x_at | 5257 | 2 | 2 |
| 208644_at | 2242 | 2 | 3 |
| 208657_s_at | 1547 | 2 | 4 |
| 208677_s_at | 5414 | 3 | 14 |
| 208696_at | 7351 | 4 | 3 |
| 208710_s_at | 1112 | 3 | 44 |
| 208723_at | 4402 | 3 | 6 |
| 208744_x_at | 1673 | 4 | 49 |
| 208837_at | 3997 | 2 | 3 |
| 208916_at | 1630 | 3 | 5 |
| 208928_at | 1439 | 4 | 7 |
| 208956_x_at | 7772 | 3 | 2 |
| 208974_x_at | 6025 | 2 | 6 |
| 208975_s_at | 1085 | 2 | 3 |
| 209015_s_at | 1739 | 4 | 4 |
| 209036_s_at | 8944 | 3 | 3 |
| 209053_s_at | 269 | 7 | 10 |
| 209072_at | 6299 | 4 | 18 |
| 209079_x_at | 12870 | 3 | 3 |
| 209081_s_at | 3160 | 3 | 2 |
| 209123_at | 4686 | 4 | 3 |
| 209132_s_at | 4385 | 5 | 12 |
| 209172_s_at | 268 | 3 | 3 |
| 209197_at | 820 | 3 | 3 |
| 209198_s_at | 491 | 3 | 3 |
| 209247_s_at | 1486 | 2 | 2 |
| 209254_at | 1384 | 4 | 7 |
| 209255_at | 4283 | 6 | 8 |
| 209256_s_at | 4949 | 8 | 6 |
| 209283_at | 12529 | 5 | 3 |
| 209345_s_at | 1678 | 2 | 2 |
| 209407_s_at | 1461 | 2 | 6 |
| 209515_s_at | 5827 | 5 | 15 |
| 209773_s_at | 1243 | 3 | 5 |
| 209825_s_at | 765 | 2 | 3 |
| 209827_s_at | 4884 | 7 | 7 |
| 209828_s_at | 1146 | 4 | 5 |
| 209848_s_at | 32959 | 7 | 74 |
| 209875_s_at | 3038 | 21 | 12 |
| 209932_s_at | 7126 | 3 | 5 |
| 210052_s_at | 1085 | 3 | 6 |
| 210073_at | 337 | 4 | 6 |
| 210111_s_at | 7841 | 5 | 3 |
| 210127_at | 391 | 2 | 9 |
| 210854_x_at | 2100 | 2 | 6 |
| 210926_at | 574 | 2 | 3 |
| 210948_s_at | 396 | 2 | 4 |
| 210951_x_at | 2501 | 2 | 13 |
| 211013_x_at | 498 | 8 | 13 |
| 211052_s_at | 1399 | 3 | 2 |
| 211066_x_at | 12431 | 2 | 2 |
| 211373_s_at | 2063 | 5 | 6 |
| 211564_s_at | 1992 | 3 | 2 |
| 211752_s_at | 2183 | 2 | 2 |
| 211759_x_at | 5674 | 2 | 3 |
| 211833_s_at | 502 | 2 | 28 |
| 212000_at | 339 | 3 | 14 |
| 212070_at | 13437 | 2 | 4 |
| 212081 x_at | 1457 | 2 | 4 |
| 212119_at | 4415 | 2 | 5 |
| 212178_s_at | 2976 | 3 | 14 |
| 212193_s_at | 3646 | 3 | 10 |
| 212247_at | 1716 | 3 | 3 |
| 212285_s_at | 4252 | 2 | 4 |
| 212312_at | 1234 | 4 | 2 |
| 212338_at | 1598 | 3 | 4 |
| 212402_at | 3019 | 3 | 4 |
| 212472_at | 1987 | 5 | 5 |
| 212473_s_at | 3747 | 5 | 4 |
| 212512_s_at | 1441 | 3 | 2 |
| 212520_s_at | 2188 | 2 | 4 |
| 212552_at | 2611 | 2 | 2 |
| 212715_s_at | 1085 | 3 | 4 |
| 212739_s_at | 2736 | 2 | 3 |
| 212744_at | 959 | 3 | 5 |
| 212745_s_at | 376 | 2 | 15 |
| 212793_at | 3123 | 4 | 5 |
| 212796_s_at | 2511 | 2 | 2 |
| 213002_at | 1439 | 2 | 3 |
| 213007_at | 968 | 3 | 4 |
| 213008_at | 1086 | 6 | 10 |
| 213096_at | 924 | 3 | 3 |
| 213131_at | 1392 | 3 | 3 |
| 213169_at | 4028 | 5 | 4 |
| 213215_at | 1926 | 4 | 3 |
| 213217_at | 5848 | 10 | 6 |
| 213241_at | 9479 | 4 | 23 |
| 213274_s_at | 18263 | 12 | 26 |
| 213275_x_at | 17604 | 7 | 3 |
| 213330_s_at | 1233 | 2 | 7 |
| 213333_at | 1845 | 2 | 3 |
| 213338_at | 932 | 6 | 4 |
| 213392_at | 1022 | 2 | 2 |
| 213474_at | 723 | 2 | 3 |
| 213496_at | 2322 | 3 | 7 |
| 213573_at | 1643 | 2 | 3 |
| 213587_s_at | 10416 | 18 | 9 |
| 213638_at | 1827 | 26 | 102 |
| 213670_x_at | 1959 | 2 | 4 |
| 213720_s_at | 2248 | 2 | 3 |
| 213746_s_at | 4187 | 3 | 14 |
| 213836_s_at | 2605 | 8 | 5 |
| 213895_at | 1279 | 3 | 4 |
| 213960_at | 11768 | 80 | 29 |
| 214023_x_at | 1602 | 7 | 9 |
| 214068_at | 2148 | 9 | 10 |
| 214104_at | 814 | 2 | 3 |
| 214201_x_at | 746 | 2 | 3 |
| 214581_x_at | 510 | 5 | 6 |
| 214614_at | 542 | 10 | 9 |
| 214632_at | 358 | 2 | 2 |
| 214656_x_at | 2977 | 2 | 2 |
| 214687_x_at | 26310 | 2 | 3 |
| 214708_at | 249 | 2 | 3 |
| 214710_s_at | 575 | 2 | 3 |
| 214714_at | 2366 | 4 | 9 |
| 214717_at | 471 | 4 | 4 |
| 214752_x_at | 6462 | 3 | 4 |
| 214778_at | 311 | 3 | 27 |
| 214841_at | 913 | 9 | 11 |
| 214893_x_at | 214 | 10 | 9 |
| 214896_at | 3071 | 8 | 11 |
| 215025_at | 2365 | 149 | 93 |
| 215115_x_at | 12421 | 34 | 15 |
| 215126_at | 4940 | 8 | 19 |
| 215155_at | 505 | 6 | 4 |
| 215311_at | 10093 | 86 | 30 |
| 215812_s_at | 1176 | 3 | 13 |
| 215836_s_at | 9406 | 3 | 3 |
| 216194_s_at | 5011 | 3 | 3 |
| 216973_s_at | 1732 | 6 | 4 |
| 217033_x_at | 10961 | 21 | 19 |
| 217104_at | 317 | 6 | 3 |
| 217226_s_at | 2191 | 3 | 3 |
| 217297_s_at | 838 | 3 | 21 |
| 217377_x_at | 12402 | 27 | 18 |
| 217419_x_at | 2742 | 3 | 5 |
| 217624_at | 349 | 21 | 20 |
| 217799_x_at | 1724 | 2 | 11 |
| 217827_s_at | 4762 | 2 | 2 |
| 217867_x_at | 9024 | 3 | 9 |
| 217871_s_at | 19519 | 3 | 11 |
| 217891_at | 1271 | 2 | 3 |
| 218009_s_at | 1557 | 4 | 3 |
| 218030_at | 1316 | 2 | 3 |
| 218074_at | 3594 | 2 | 4 |
| 218143_s_at | 4007 | 3 | 5 |
| 218151_x_at | 1384 | 2 | 2 |
| 218152_at | 1440 | 2 | 3 |
| 218161_s_at | 941 | 5 | 5 |
| 218175_at | 3563 | 3 | 2 |
| 218330_s_at | 3853 | 7 | 4 |
| 218349_s_at | 588 | 3 | 14 |
| 218359_at | 796 | 3 | 5 |
| 218376_s_at | 1931 | 4 | 4 |
| 218447_at | 2209 | 2 | 3 |
| 218542_at | 409 | 3 | 6 |
| 218564_at | 433 | 2 | 4 |
| 218608_at | 627 | 3 | 4 |
| 218678_at | 11356 | 14 | 20 |
| 218774_at | 1061 | 2 | 5 |
| 218786_at | 732 | 3 | 2 |
| 218824_at | 1351 | 3 | 5 |
| 218839_at | 1996 | 38 | 7 |
| 218856_at | 3199 | 5 | 4 |
| 218888_s_at | 906 | 6 | 5 |
| 218931_at | 911 | 3 | 2 |
| 218952_at | 2661 | 7 | 7 |
| 218956_s_at | 1840 | 4 | 3 |
| 218980_at | 1627 | 3 | 8 |
| 218996_at | 1859 | 4 | 6 |
| 219011_at | 113 | 4 | 3 |
| 219039_at | 1852 | 2 | 5 |
| 219040_at | 480 | 3 | 10 |
| 219041_s_at | 3435 | 4 | 2 |
| 219051_x_at | 1127 | 3 | 8 |
| 219066_at | 621 | 4 | 2 |
| 219143_s_at | 3618 | 9 | 13 |
| 219148_at | 426 | 2 | 3 |
| 219152_at | 365 | 13 | 10 |
| 219219_at | 859 | 2 | 3 |
| 219361_s_at | 1033 | 3 | 7 |
| 219372_at | 376 | 2 | 2 |
| 219408_at | 421 | 3 | 36 |
| 219478_at | 5485 | 80 | 18 |
| 219491_at | 547 | 3 | 12 |
| 219522_at | 822 | 4 | 3 |
| 219537_x_at | 411 | 3 | 6 |
| 219555_s_at | 402 | 12 | 21 |
| 219578_s_at | 1419 | 17 | 26 |
| 219634_at | 686 | 3 | 15 |
| 219637_at | 355 | 3 | 3 |
| 219703_at | 378 | 3 | 3 |
| 219742_at | 409 | 3 | 9 |
| 219895_at | 528 | 6 | 3 |
| 219933_at | 1399 | 2 | 2 |
| 220116_at | 748 | 5 | 8 |
| 220155_s_at | 5010 | 5 | 6 |
| 220178_at | 5915 | 9 | 15 |
| 220454_s_at | 581 | 2 | 2 |
| 220864_s_at | 8416 | 3 | 3 |
| 220948_s_at | 11794 | 2 | 3 |
| 220973_s_at | 497 | 4 | 3 |
| 220974_x_at | 2540 | 2 | 2 |
| 220980_s_at | 3598 | 3 | 2 |
| 221059_s_at | 2438 | 5 | 3 |
| 221483_s_at | 9194 | 3 | 3 |
| 221484_at | 3834 | 3 | 3 |
| 221538_s_at | 3971 | 3 | 3 |
| 221558_s_at | 2356 | 3 | 5 |
| 221577_x_at | 4897 | 28 | 38 |
| 221641_s_at | 1199 | 2 | 4 |
| 221688_s_at | 3740 | 2 | 4 |
| 221710_x_at | 728 | 2 | 2 |
| 221732_at | 931 | 3 | 2 |
| 221759_at | 1261 | 4 | 21 |
| 221797_at | 430 | 2 | 7 |
| 221799_at | 1601 | 5 | 3 |
| 221815_at | 3293 | 17 | 144 |
| 221882_s_at | 1144 | 5 | 6 |
| 221902_at | 2491 | 4 | 4 |
| 221909_at | 243 | 22 | 17 |
| 221962_s_at | 1132 | 2 | 6 |
| 222116_s_at | 4208 | 2 | 4 |
| 222153_at | 445 | 3 | 8 |
| 222155_s_at | 1264 | 3 | 10 |
| 222175_s_at | 2415 | 3 | 6 |
| 222196_at | 224 | 3 | 6 |
| 222199_s_at | 2152 | 2 | 3 |
| 222206_s_at | 383 | 4 | 12 |
| 222212_s_at | 3715 | 3 | 4 |
| 222231_s_at | 2724 | 3 | 2 |
| 222234_s_at | 754 | 4 | 12 |
| 222240_s_at | 1331 | 3 | 3 |
| 222294_s_at | 2193 | 3 | 5 |
| 32811_at | 4194 | 2 | 3 |
| 40560_at | 1629 | 3 | 5 |
| 44783_s_at | 8503 | 14 | 6 |
| 46665_at | 7835 | 4 | 3 |
| 55093_at | 3032 | 4 | 4 |
| 63825_at | 10096 | 14 | 74 |
| 87100_at | 737 | 6 | 70 |

We selected a short list of genes with at least 10-fold over-expression in melanoma as compared to the benign specimens. The complete array dataset has been submitted to the NCBI/Genbank GEO database (series entry pending).

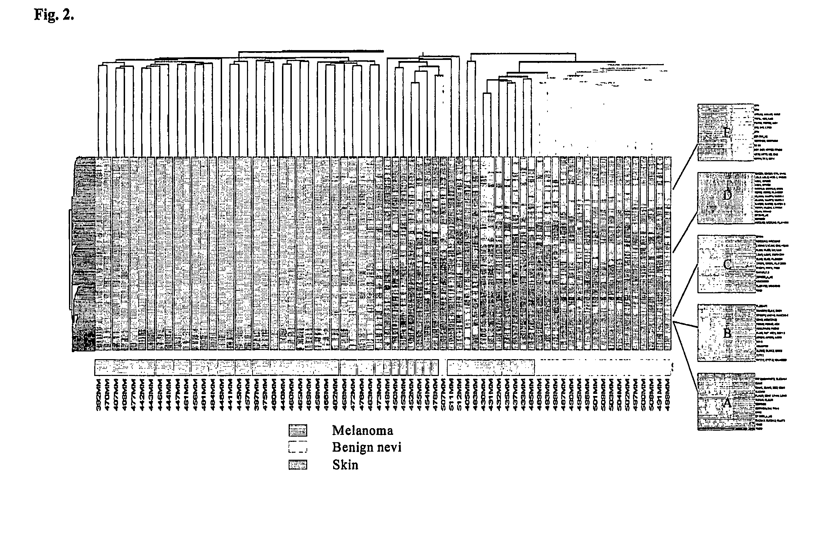

Hierarchical clustering revealed four distinct clusters (Fig. 2). Two clusters consisted of majority of the melanoma samples (43 out of 45); the third cluster included the majority of benign nevi samples (15 out of 18) and the fourth contained all 7 normal skin specimens. Melanoma samples themselves formed two clusters with 35 samples in one cluster and 10 samples in the other. Samples that formed the small cluster represented epithelioid melanoma only, visually contained less melanin and demonstrated higher expression of PRAME and MIA genes (p<0.05). The few stage III and IV tumors were all grouped in the small cluster. The large cluster showed higher expression of NTRK3 and nestin (NES) (p<0.05). All melanoma and benign nevi samples demonstrated equally high expression of known melanocyte markers such as tyrosinase and MART-1, confirming that there is comparable melanocyte content in these samples. Our data indicate that melanoma, benign nevi and normal skin samples have distinct gene expression profiles and can be separated on molecular basis. Selected genes that were highly expressed in melanoma and their associated functional categories are summarized in Table 9.

**Table 9**

| psid | Name | Median expression in melanoma | Median expression in benign/normal | Fold change |
|---|---|---|---|---|
| **Neural system development and function** | | | | |
| 215025_at | NTRK3 | 2365.1 | 19.9 | 118.8 |
| 204709_s_at | KNSL5 | 311.8 | 13.5 | 23.1 |
| 204585_s_at | LICAM var1 | 805.2 | 49 | 16.4 |
| 218678_at | NES | 11355.5 | 703.6 | 16.1 |
| 202260_s_at | STXBP1 | 7877.1 | 1312 | 6.0 |
| 204995_at | p35 | 496.3 | 89.2 | 5.6 |
| 208308_s_at | GP1 | 12281.7 | 2238.2 | 5.5 |
| 201340_s_at | ENC1 | 390.8 | 74.9 | 5.2 |
| 209072_at | MBP | 6299.1 | 1215.9 | 5.2 |

| **Cell movements** | | | | |
|---|---|---|---|---|
| 214614_at | HOXB9 | 541.6 | 54.4 | 10.0 |
| 205447_s_at | MAP3K12 | 566.5 | 76.5 | 7.4 |

| **Tissue morphology** | | | | |
|---|---|---|---|---|
| 206397_x_at | GDF1 | 1436.1 | 130.1 | 11.0 |
| 205458_at | MC1R | 3287.6 | 458.1 | 7.2 |

| **Cancer cell invasion** | | | | |
|---|---|---|---|---|
| 213274_s_at | CSTTB | 18262.9 | 1261.4 | 14.5 |
| 208677_s_at | BSG | 5413.8 | 1088 | 5.0 |
| **Cell cycle control** | | | | |
| 219578 s_at | CPEB1 | 1418.5 | 75.6 | 18.8 |
| 207144_s_at | CITED1 | 593.4 | 33.5 | 17.7 |
| 204252_at | CDK2 | 5293.7 | 869.3 | 6.1 |
| 211373_s_at | PSEN2 | 2063.3 | 403.2 | 5.1 |

| **Cell death** | | | | |
|---|---|---|---|---|
| 221577_x_at | PLAB | 4896.9 | 173.9 | 28.2 |
| 205681_at | BCL2A1 | 1316.4 | 135.1 | 9.7 |

| **Unknown** | | | | |
|---|---|---|---|---|
| 204545_at | PEX6 | 379.1 | 23.9 | 15.9 |
| 201850 at | CAPG | 11103.2 | 725.6 | 15.3 |
| 204014_at | DUSP4 | 7183.6 | 601.3 | 11.9 |
| 202779_s_at | E2-EPF | 3400 | 323.3 | 10.5 |
| 201954_at | ARPC1B | 25900.7 | 2470 | 10.5 |
| 2098481_s_at | me20m | 32958.9 | 3778.4 | 8.7 |
| 213112_s_at | SQSTM1 | 260.4 | 33.9 | 7.7 |
| 218952_at | SAAS | 2660.7 | 368.7 | 7.2 |
| 204099_at | SMARCD3 | 2496.2 | 428.2 | 5.8 |
| 206999_at | IL12RB2 | 354 | 61.3 | 5.8 |
| 201251_at | PKM2 | 23964.7 | 4228.2 | 5.7 |
| 202185_at | PLOD3 | 8493.2 | 1541.7 | 5.5 |

### Example 4

### Identification of genes differentially expressed in melanoma

A total of 70 gene expression profiles were used for analysis. The he median percentages of "present calls" for melanoma, benign and normal sample groups were 43.8%, 46.9% and 41.7%. Sixty microarrays (86%) had scaling factors within 3-fold range of the minimum value. Ten chips with the scaling factors more than 3 were equally distributed between the sample categories, melanoma, benign and normal.

Unsupervised hierarchical clustering result revealed a distinct separation of the melanoma, benign nevi and normal skin samples (Fig. 2). We observed four clusters, including two clusters consisting of majority of the melanoma samples (43 out of 45), the third cluster contained all 7 normal skin, 3 benign nevi and 2 melanoma specimens and the fourth cluster, that included 14 of the 18 benign nevi samples. Source of the samples did not affect clustering. Specimens originated from different sources were clustered together according the sample type (melanoma, benign or normal). To further test the stability of the clustering patterns, we used an alternative cut-off on gene filtering prior to the cluster analysis. Specifically, we retained genes that have at least 10% "present" calls in each of the melanoma, benign nevi and skin samples. With this cut-off, we obtained 15, 306 genes and repeated hierarchical clustering. The cluster pattern on the patient samples was the same as the one from the 15,795 from the 2 "present" calls, confirming clustering stability.

The single nevi sample that clustered with the melanoma samples is an atypical nevi (moderate degree) sample with no melanoma *in-situ* present. All three nevi samples that clustered with normal skin are compound nevi samples and one of them has melanocyte content lower than the other nevi specimens. The melanoma samples themselves formed two clusters with 34 samples in the large and 9 samples in the smaller cluster. Samples that formed the small cluster represented epithelioid melanoma only and visually contained less melanin. The few stage III and IV tumors, used in our study, were all grouped in the small cluster. The large cluster was composed from epithelioid, spindle cell and melanoma of mixed histology specimens with more significant presence of melanin. The large cluster included Stage I and Stage II specimens only.

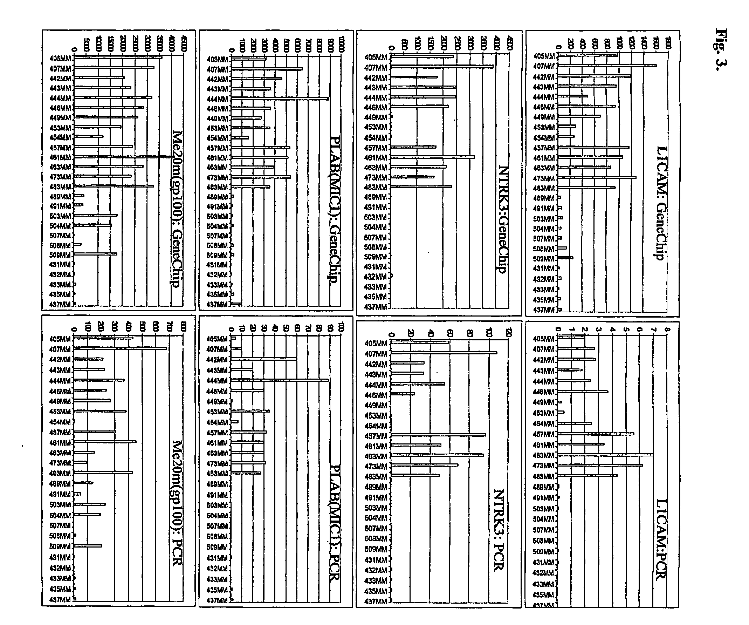

Distinct gene clusters were found in association to melanoma. This can be characterized by up-regulated (Fig. 2, A, B, C) and down-regulated (Fig. 2, E) genes in the melanoma samples. At the same time, melanoma and benign nevi samples demonstrated high expression of known melanocyte markers, such as MART-1 (Fig. 3, D) confirming a comparable content of melanocyte in these samples and inability of melanocyte specific markers to differentiate them. Our data indicate that melanoma, benign nevi and normal skin samples have distinct gene expression profiles and can be separated on their molecular basis.

In order to identify genes upregulated in malignant melanoma, we applied SAM in combination with t-test with Bonferroni correction and percentile analysis (Fig. 1). Bonferroni-adjusted t-test and percentile analyses were used to address the multiple testing issue and the heterogeneity of the tumor samples, respectively. As the result of these analyses, 439 genes were selected and are summarized in Table 15 as SEQ ID NOs: 29-467. Out of 439 genes up-regulated in melanoma, we selected a short list of 33 genes that had more than 10-fold over-expression in the melanoma samples than that of the benign specimens. These include many genes with known association with malignant melanoma such as NTRK3 (Xu et al. (2003)), L1CAM (Fogel et al. (2003); and Thies et al. (2002)), me20m (Adema et al. (1994)), as well as novel genes. Genes with more than 10-fold overexpression in melanoma are presented in Table 10.

**Table 10**

| Psid | Description | Median Exp Melanoma | Fold change (Can v Benign) | Fold change (Can v skin) |
|---|---|---|---|---|
| 215025_at | NTRK3 | 2365 | 149 | 93 |
| 215311_at | EUROIMAGE 21920 | 10093 | 86 | 30 |
| 213960_at | EUROIMAGE 51358 | 11768 | 80 | 29 |
| 219478_at | WFDC1 | 5485 | 80 | 18 |
| 218839_at | HEY1 | 1996 | 38 | 7 |
| 215115_x_at | TEL oncogene | 12421 | 34 | 15 |
| 221577_x_at | PLAB | 4897 | 28 | 38 |
| 217377_x_at | ETV6-NTRK3 fusion | 12402 | 27 | 18 |
| 213638_at | PHACTR1 | 1827 | 26 | 102 |
| 204709_s_at | KNSL5 | 312 | 24 | 16 |
| 221909_at | Hyp protein FLJ14627 | 243 | 22 | 17 |
| 204584_at | L1CAM | 9677 | 21 | 15 |
| 209875_s_at | SPP1 | 3038 | 21 | 12 |
| 217624_at | PDAP1 | 349 | 21 | 20 |
| 203071_at | SEMA3B | 958 | 19 | 3 |
| 213587_s_at | C70RF32 | 10416 | 18 | 9 |
| 221815_at | ABHD2 | 3293 | 17 | 144 |
| 219578_s_at | CPEB1 | 1419 | 17 | 26 |
| 207144_s_at | CITED1 | 593 | 17 | 24 |
| 203069_at | SV2A | 560 | 15 | 13 |
| 218678_at | NES | 11356 | 14 | 20 |
| 219152_at | PODXL2 | 365 | 13 | 10 |
| 205447_s_at | MAP3L12 | 567 | 13 | 5 |
| 213274_s_at | CTSB | 18263 | 12 | 26 |
| 219555_s_at | BM039 | 402 | 12 | 21 |
| 203827_at | WIPI49 | 3380 | 11 | 8 |
| 205813_s_at | MAT1A | 430 | 11 | 9 |
| 201850_at | CAPG | 11103 | 10 | 20 |
| 205373_at | CTNNA2 | 579 | 10 | 8 |
| 214614_at | HLXB9 | 542 | 10 | 9 |
| 213217 | ADCY2 | 5848 | 10 | 6 |
| 204014_at | DUSP4 | 7184 | 10 | 46 |
| 214893 | HCN2 | 214 | 10 | 9 |

We further selected three genes over-expressed in melanoma, including NTRK3, PLAB, L1CAM, for quantitative real-time RT-PCR validation of the microarray results (Fig. 3). PLAB is a novel gene, whose differential expression in melanoma was not reported before at our best knowledge. For L1CAM and NTRK3, differential expression in melanoma was demonstrated at protein level only. Xu et al. (2003); Fogel et al. (2003); and Thies et al. (2002). Moreover, we identified PLAB and L1CAM as the best combination, on complementary basis, to separate melanoma from benign/normal tissues in our study. GP100 is known as a melanoma specific marker and was selected as positive control. For the RT-PCR assay we used a panel of 14 primary melanoma, 7 benign nevi and 5 normal skin samples, isolated from the same tissues as used for the microarray study. The expression value of each gene was normalized to the housekeeping control gene PBGD. The correlation coefficients between the RT-PCR and the microarray results for L1CAM, NTRK3, PLAB and gp100 are 0.79, 0.86, 0.87 and 0.88, respectively. This result indicates that the RT-PCR results are highly consistent with the microarray data.

### Example 5

### Pathway Analysis of Differentially Expressed Genes

Functional analysis of genes differentially expressed in melanoma was performed using Ingenuity^{™} Pathway Analysis Software Application (Ingenuity, Mountain View, CA). Functional categories or canonical pathways that have p-value of less than 0.05 were selected. Specificity of canonical pathways identification was tested using randomly selected genes.

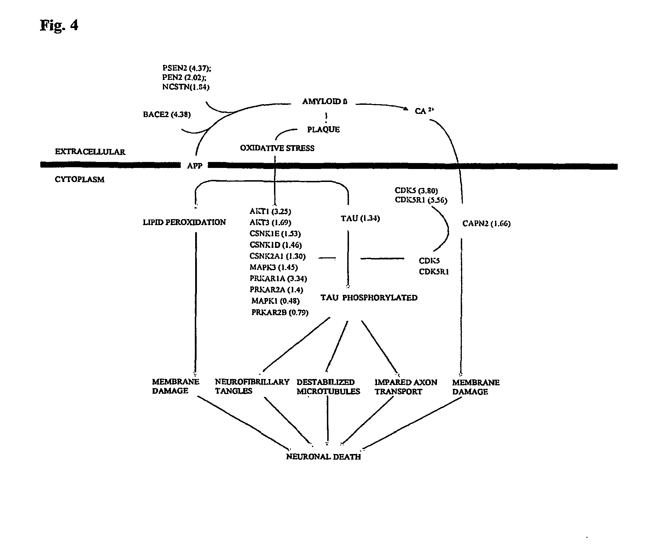

In order to gain further insight into a potential mechanism that differentiates melanoma from benign and normal tissue, we used Ingenuity pathway analysis software to identify canonical pathways associated with melanoma. The results analysis revealed that many of the genes in amyloid processing were up-regulated in the melanoma samples. To verify specificity of our observation, we selected three random lists of genes from Affymetrix Hu133A microarray and subjected them to Ingenuity pathway analysis. None of these lists produced a significant association to amyloid processing or any other canonical pathways. To confirm the activation of this canonical pathway in melanoma, gene expression data for all the genes in the pathway were retrieved. Fold-change and p-value of differential expression between melanoma and benign/normal tissues were calculated. Out of the 34 genes included in the amyloid processing pathway (Esler et al. (2001); and Giancotti et al. (1999)), 25 demonstrated up-regulation trend and for 19 of them (56%), differential expression was statistically significant (p-value< 0.05; Fig 4). As the additional control, we randomly selected two metabolic pathways with a similar number of genes. Out of the 63 genes in alanine synthesis pathway, 8 of them (13%) showed significant up-regulation with p-value less than 0.05. Out of the 47 genes in histidine synthesis pathway, only 2 genes (4%) were found using the same criteria. For the first time, our data strongly indicated that activation of the amyloid processing pathway is involved in malignant melanoma.

### Example 6

### RT-PCR Validation of Microarray Results

Ten microgram total RNA from each sample was treated with DNase I and reverse-transcribed with oligo (dT) primer using Superscript II reverse transcriptase according to the manufacturer's instructions (Invitrogen, Carlsbad, CA). A control gene PBGD was previously tested and reported as a housekeeping gene. Vandesompele et al. (2003). Primers and MGB-probes for me20m (gp100), L1CAM, NTRK3, and the control gene PBGD were designed using Primer Express software (Applied Biosystems, Foster City, CA). The PLAB (MIC1) gene probe was FAM-TAMRA based since sequences were inadequate to design MGB based probes. Primer/probe sequences were as follows:

**Table 11**

| Description | Sequence | SEQ ID NO: |
|---|---|---|
| me20m forward | TGTGTCTCTGGCTGATACCAACA | 983 |
| me20m reverse | TTCTTGACCAGGCATGATAAGCT | 984 |
| me20m probe | (6-FAM) CTGGCAGTGGTCAGC | 985 |
| L1CAM forward | GCTGGGACTGGGAACAGAACT | 10 |
| L1CAM reverse | GGAGCAGAGATGGCAAAGAAA | 11 |
| L1CAM probe | (6-FAM) TCCCCACCATCTGCTGT | 12 |
| NTRK3 forward | GCCCCGGCACCCTTTA | 16 |
| NTRK3 reverse | AACCCTGCCAGTGGTGGAT | 17 |
| NTRK3 probe | (6-FAM) CAGATGGGTGTTTTC | 18 |
| PLAB forward | GGCAGAATCTTCGTCCGCA | 4 |
| PLAB reverse | GGACAGTGGTCCCCGTTG | 5 |
| PLAB probe | (6-FAM) CCCAGCTGGAGTTGCACTTGCGGCC(TAMRA) | 6 |
| PBGD forward | CTGCTTCGCTGCATCGCTGAAA | 986 |
| PBGD reverse | CAGACTCCTCCAGTCAGGTACA | 987 |
| PBGD probe | (6-FAM) CCTGAGGCACCTGGAAGGAGGCTGCAGTGT(TAMRA) | 988 |

All primers and probes were tested for optimal amplification efficiency above 90%. The standard curve was composed of six 10-fold dilutions of target gene PCR product with copy numbers ranging from 10 to 10⁶. RT-PCR amplification was carried out in a 20µl reaction mix containing 50ng template cDNA, 2 x TaqMan^{®} universal PCR master mix (12.5µl) (Applied Biosystems, Foster City, CA), 500nM forward and reverse primers, and 250nM probe. Reactions were run on an ABI PRISM 7900HT Sequence Detection System (Applied Biosystems, Foster City, CA). The cycling conditions were: 2 min of AmpErase UNG activation at 50°C, 10 min of polymerase activation at 95°C and 50 cycles at 95°C for 15 sec and annealing temperature (60°C) for 60 sec. In each assay, a standard curve and a no-template control along with template cDNA were included in duplicate for both the gene of interest and the control gene. The relative quantity of each target gene was represented as ΔCt, which is equal to Ct of the target gene subtracted by Ct of the control gene.

To confirm the melanoma specific genes identified by the microarray analysis, four genes (L1CAM, NTRK3, PLAB and gp100) were selected for quantitative real-time RT-PCR validation (Fig. 4). The expression value of each gene was normalized to housekeeping control PBGD. The correlation coefficient between the RT-PCR and the microarray results for L1CAM, NTRK3, PLAB and gp100 are 0.79, 0.86, 0.87 and 0.88, respectively, indicating that the RT-PCR results are highly consistent with the microarray data.

### Example 7

### One-step qRTPCR Assays Using RNA-specific Primers and Cutoff Establishment

Evaluation of expression of selected genes was carried out with one-step RT-PCR with RNA from primary melanoma, benign nevi, normal skin, melanoma LN metastasis and melanoma-free lymph nodes. Beta-actin was used as a housekeeping gene to control for the input quantity and quality of RNA in the reactions. DNase treatment was not used. Instead, primers or probes were designed to span an intron so they would not report on genomic DNA. Eight ng of total RNA was used for the RT-PCR. The Total RNA was reverse transcribed using 40X Multiscribe and RNase inhibitor mix contained in the TaqMan® One Step PCR Master Mix Reagents Kit (Applied Biosystems, Foster City, CA). The cDNA was then subjected to the 2x Master Mix without UNG and PCR amplification was carried out on the ABI 7900HT Sequence Detection System (Applied Biosystems, Foster City, CA) in the 384-well block format using a 10 µl reaction size. The primer and probe concentrations were 4 µM and 2.5 µM, respectively. The reaction mixture was incubated at 48°C for 30 min for the reverse transcription, followed by a Amplitaq activation step of 95°C for 10 min and finally 40 cycles of 95°C for 15 sec denaturing and 60°C for 1 min anneal and extension. On each plate a standard curve is generated from 8 pg to 80 ng and when the R2 value was greater than 0.99 the Cycle Threshold (Ct) values were accepted.

Sequences used in the reactions were as follows, each written in the 5' to 3' direction.

**Table 12**

| Description | Sequence | SEQ ID NO: |
|---|---|---|
| L1CAM Forward | CCACAGATGACATCAGCCTCAA | 13 |
| L1CAM Reverse | GGTCACACCCAGCTCTTCCTT | 14 |
| L1CAM probe | TGGCAAGCCCGAAGTGCAGTTCC | 15 |
| Tyrosinase Forward | CTTTAGAAATACACTGGAAGGATTTGCTA | 1000 |
| Tyrosinase Reverse | CATTGTGCATGCTGCTTTGA | 1001 |
| Tyrosinase probe | TCCACTTACTGGGATAGCGGATGCCTC | 1002 |
| MART1 Forward | ACTTCATCTATGGTTACCCCAAGAA | 1003 |
| MART1 Reverse | TCCCAGCGGCCTCTTCA | 1004 |
| MART1 Probe | CACGGCCACTCTTACACCACGGC | 1005 |
| HMB45 Forward | CTTAAGGCTGGTGAAGAGACAAGTC | 1006 |
| gp100 Reverse | CAGGATCTCGGCACTTTCAATAC | 1007 |
| gp100 Probe | TCGATATGGTTCCTTTTCCGTCACCCTG | 1008 |
| PLAB Forward | ATTCGAACACCGACCTCGTC | 1009 |
| PLAB Reverse | CGCAGGTGCAGGTGGC | 1010 |
| PLAB Probe | GATACTCACGCCAGAAGTGCGGCT | 1011 |

For each sample ΔCt=Ct (Target Gene) - Ct β-actin was calculated. ΔCt has been widely used in clinical RT-PCR assays and was chosen as a straightforward method. Cronin et al. (2004). T-test was performed on ΔCt between the melanoma and non-melanoma samples including both primary and LN samples. We then used ΔCt to construct two scores for each patient. One score was derived from a combination of 2 melanoma specific genes, PLAB and L1CAM; and the other score was derived from a combination of 3 conventional melanoma markers, tyrosinase, gp100 and MART1. The score was defined as the weighted sum of ΔCt values of the tested genes with the corresponding t statistics as the weight. The two scores were normalized to have the same mean in order to compare them on the same scale.

We examined a combination of two highly overexpressed in melanoma genes, PLAB and L1CAM, in a variety of clinical tissue samples containing malignant melanocytes (primary melanoma and melanoma LN metastasis), benign melanocytes (benign skin nevi) and normal samples (normal skin and melanoma-free LN) by RT-PCR. The primary tissues were the same as those used for the microarray study while all the LN specimens were derived from independent patients. Conventional melanoma markers, such as tyrosinase, gp100 and MART1, were also tested on the same samples as the controls because they are the most commonly used markers for the melanoma molecular assays in current clinical studies. Rimboldi et al. (2003); Abrahamsen et al. (2005); and Kammula et al. (2004). Calculated scores were presented on Fig. 4A for PLAB and L1CAM and on Fig. 4B for tyrosinase, gp100 and MART1. The results demonstrated significant difference in expression of PLAB and L1CAM between malignant melanoma samples (primary and LN metastasis) and benign nevi and normal LN. In contrast, three conventional markers showed similar expression levels in benign and melanoma samples. To further demonstrate the ability of gene markers to separate benign and malignant tissues, we tested two cutoffs; first was set up as the highest score in primary normal samples and the second as the highest score in benign nevi samples. For each cut-off we estimated sensitivity and of the assay in the LN samples. With the cut-off determined on the normal samples, the new markers and the conventional markers gave sensitivity of 90% and 83%, respectively. Using the cut-off determined on the benign samples, the sensitivity for the new and conventional markers were 88% and 42%. The results indicated that the new markers potentially have better abilities to differentiate tissues containing benign and malignant melanocytes.

### Example 8

### Multiplex Assay

### Materials and methods

Each reaction was set up in a final volume of 25 µl containing the following:

| | |
|---|---|
| forward primer | 400 nM |
| reverse primer | 500 nM |
| PLAB probe | 150 nM |
| Tyrosinase probe | 300 nM |
| L1CAM probe | 200 nM |
| PBGD probe | 200 nM |
| Tth | 5 U |
| Ab TP 6-25 | 1 µg |
| Glycerol | 10% |
| Tris-HCl | 3.7 mM |
| NaCl | 4 mM |
| EDTA | 0.004 mM |
| Tween-20 | 0.22% |
| NP-40 | 0.02% |
| DTT | 0.04 mM |
| Potassium Hydroxide | 20.5 mM |
| Bicine | 50 mM |
| Potassium Acetate | 115 mM |
| Albumin, bovine | 5 µg |
| Trehalose | 0.15 M |
| dNTP | 0.2 mM ea |
| MgCl₂ | 0.5 mM |
| MnSO₄ | 3.5 mM |
| Primers | 300 nM ea |
| Probes | 200 nM ea |

The primer and probe sequences are provided in Table 13.

**Table 13**

| SEQ ID NO | Sequence 5'-3' | Function |
|---|---|---|
| 43 | gaacaccgacctcgtccc | PLAB Upper Primer |
| 44 | ggcggcccgagagata | PLAB Lower Primer |
| 45 | Fam-cgccagaagtgcggctgggat-BHQ1-tt | PLAB Probe |
| 55 | actcagcccagcatcattcttc | Tyr Upper Primer |
| 56 | atggctgttgtactcctccaatc | Tyr Lower Primer |
| 57 | Q570-cttctcctcttggcagattgtctgtagc BHQ2-tt | Tyr Probe |
| 49 | ccacagatgacatcagcctcaa | L1CAM Upper Primer |
| 50 | ggtcacacccagctcttcctt | L1CAM Lower Primer |
| 51 | CalRed-tggcaagcccgaagtgcagttcc-BHQ2-tt | L1CAM Probe |
| 58 | ccacacacagcctactttccaa | PBGD Upper Primer |
| 59 | tacccacgcgaatcactctca | PBGD Lower Primer |
| 60 | Q670-aacggcaatgcggctgcaacggcggaa-BHQ2-tt | PBGD Probe |

The reactions are run with PLAB in Fam, Tyrosinase in Cy3, L1CAM in Texas Red, and PBGD in Cy5 channels. The cycling protocol used is described below and takes 30 min to complete.
95°C x 15 sec
65°C x 420 sec
40°cycles of:
95°C for 5 sec
62°C for 15 sec - fluor read

The thresholds used are 30 in Fam, 20 in Cy3, 20 in Texas Red, and 20 in Cy5 channels. The thresholds employed in the Cy3 and Texas red channels can be lowered. The results obtained are summarized in Table 14.

**Table 14**

| Best Marker Combinations | | |
|---|---|---|
| Markers | % Sensitivity (95% CI) | % Specificity (95% CI) |
| L1CAM + PLAB | 82 (73-89) | 96 (87-100) |
| Tyrosinase + ME20M (GP100) | 63 (52-72) | 100 (94-100) |
| L1CAM + PLAB + Tyrosinase | 87 (79-93) | 96 (87-100) |

### Ct cutoffs:

| | |
|---|---|
| L1CAM | 27 |
| PLAB | 29 |
| Tyrosinase | 23 |
| ME20M (GP100) | 23.5 |

| | |
|---|---|
| Note: these data are benchmarked against H&E pathology only. The amplification efficiency in each of the 4 reactions is high and the reaction is also linear over 5 logs (as judged by the R2 value which is >0.99 in all cases). Therefore, these data demonstrate a working 4 plex, rapid assay. These data suggest that PLAB is the primary marker and complementation, achieved with L1CAM, further increases sensitivity. If required, addition of tyrosinase as a third marker further complements L1CAM and PLAB and increases sensitivity. Tyrosinase can be dropped from the assay, if needed, without affecting the performance of the remaining markers. | |

### Discussion

We performed gene expression profiling analysis of primary melanoma, benign nevi and normal skin tissue specimens in order to find melanoma specific gene markers for potential use in the LN molecular staging assay. Novel genes that are highly and differentially expressed in malignant melanoma samples were identified. Inclusion of benign nevi in the experimental design was key to our study. In contrast to normal skin, melanocyte content in benign nevi is close to that in melanoma. This was confirmed, in addition to histological assessment, by equally high expression level of conventional melanoma markers such as tyrosinase and MART1 in both melanoma and nevi tissue specimens. Similar cellular composition allowed us to monitor gene expression changes specifically associated with melanocyte malignant transformation, not just with melanocyte lineage differentiation. As the result, we identified novel genes specifically overexpressed in melanoma. One of the novel highly overexpressed in melanoma genes, prostate differentiation factor (PLAB, MIC 1), is a member of transforming growth factor-beta superfamily and also known to be associated with other malignancies. Bae et al. (2003); and Welsh et al. (2003). PLAB reduces cell adhesion (Yamauchi et al. (2003)), implicating its potential role in melanoma progression. Pathway analysis of the overexpressed genes in melanoma indicated that many of these genes belong to neural tissue functioning and development, suggesting that dedifferentiation of melanocytes and activation of the processes related to a pluripotent progenitor cell might be important for melanoma development and progression. Moreover, the analysis of canonical pathways showed that neural tissue associated amyloid processing is significantly modulated in melanoma. Amyloid processing (APP) pathway itself has not been associated with melanoma development and progression before. Many genes in the APP pathway, such as members of the β- and Υ-secretase family (BACE2, PSEN2) also participate in the Notch pathway and play a role of cleavage of integral membrane proteins in both Notch and APP. Esler et al. (2001). Notch suppresses differentiation and helps maintain neural crest stem cells in undifferentiated state (Gangemi et al. (2004)) and Notch's involvement in melanoma and, particularly, the role of Υ-secretases is the focus of many studies. Hoek et al. (2004); Baldi et al. (2003); and Wilson et al. (2000).

We have compared our results to the recent study of Haqq et al (2005). In their work, cDNA microarray containing 20,862 probes was used to profile benign nevi, primary melanoma and metastatic melanoma specimens. The sample set included metastatic and primary melanoma and benign nevi. Similar clustering results that separated the benign nevi and the primary malignant melanoma tissues were found in their study. Common genes were reported in both studies that can discriminate melanoma from benign nevi including kinesin-like 5 (KNSL5), prostate differentiation factor (PLAB), CITED1, osteopontin (SPP1), cathepsin B (CSTB), cadherin 3 (CDH3), presenilin 2 (PSEN2).

Our results of the one-step RT-PCR assay demonstrated that novel melanoma specific gene PLAB and L1CAM expressed not only in primary melanoma tissues but also in melanoma LN metastasis. Moreover, the ability to differentiate malignant melanoma from benign nevi made them better candidates than the conventional markers for the molecular test of melanoma diagnostics. With further validation in clinical studies, these genes could be developed as specific markers for a molecular staging assay to detect melanoma micrometastasis during sentinel lymph node (SLN) biopsy procedure. Another potential application of the genes is for diagnosis of melanocyte lesions with uncertain pathological features.

Although the foregoing invention has been described in some detail by way of illustration and example for purposes of clarity of understanding, the descriptions and examples should not be construed as limiting the scope of the invention.

**Table 15 Sequence Descriptions, names and SEQ ID NOs:**

| | | | | |
|---|---|---|---|---|
| 1 | | | PLAB | |
| 2 | | | L1 CAM | |
| 3 | | | NTRK | |
| 4 | | | | PLAB forward primer |
| 5 | | | | PLAB reverse primer |
| 6 | | | | PLAB probe |
| 7 | | | | PLAB upper primer |
| 8 | | | | PLAB lower primer |
| 9 | | | | PLAB probe |
| 10 | | | | L1CAM forward primer |
| 11 | | | | L1CAM reverse primer |
| 12 | | | | L1CAM probe |
| 13 | | | | L1CAM upper primer |
| 14 | | | | L1CAM lower primer |
| 15 | | | | L1CAM probe |
| 16 | | | | NTRK primer |
| 17 | | | | NTRK primer |
| 18 | | | | NTRK probe |
| 19 | | | | Tyrosinase upper primer |
| 20 | | | | Tyrosinase lower primer |
| 21 | | | | Tyrosinase probe |
| 22 | | | | PBGD upper primer |
| 23 | | | | PBGD lower primer |
| 24 | | | | PBGD probe |
| 25 | | | | PLAB amplicon |
| 26 | | | | L1CAM amplicon |
| 27 | | | | Tyrosinase amplicon |
| 28 | | | | PBGD amplicon |
| 29 | 200078_s_at | BC005876 | | ATPase, H+ transporting, lysosomal |
| 30 | 200601_at | U48734 | | non-muscle alpha-actinin |
| 31 | 200612_s_at | NM 001282 | AP2B1 | adaptor-related protein complex 2, β 1 |
| 32 | 200644_at | NM_023009 | MACMARCKS | macrophage myristoylated alanine-rich C kinase substrate |
| 33 | 200660_at | NM 005620 | S100A11 | S100 calcium-binding protein A11 |
| 34 | 200707_at | NM 002743 | PRKCSH | protein kinase C substrate 80K-H |
| 35 | 200736_s_at | NM 000581 | GPX1 | glutathione peroxidase 1 |
| 36 | 200737_at | NM 000291 | PGK1 | phosphoglycerate kinase 1 |
| 37 | 200783_s_at | NM 005563 | LAP18 | leukemia-assoc phosphoprotein p18 |
| 38 | 200825_s_at | NM 006389 | ORP150 | oxygen regulated protein (150kD) |
| 39 | 200827_at | NM_000302 | PLOD | procollagen-lysine, 2-oxoglutarate 5-dioxygenase |
| 40 | 200837_at | NM 005745 | DXS1357E | accessory proteins BAP31BAP29 |
| 41 | 200838_at | NM 001908 | CTSB | cathepsin B |
| 42 | 200839_s_at | NM 001908 | CTSB | |
| 43 | 200859_x_at | NM 001456 | FLNA | filamin A, alpha |
| 44 | 200910_at | NM 005998 | CCT3 | chaperonin containing TCP1, sub 3(γ) |
| 45 | 200950_at | NM_006409 | ARPC1A | actin related protein 23 complex, sub 1A |
| 46 | 200950_at | NM 006409 | ARPC1A | |
| 47 | 200966_x_at | NM 000034 | ALDOA | aldolase A, fructose-bisphosphate |
| 48 | 200967_at | NM 000942 | PPIB | peptidylprolyl isomerase B |
| 49 | 200968_s_at | NM 000942 | PPIB | |
| 50 | 200972_at | BC000704 | | tetraspan 3 |
| 51 | 201038_s_at | BE560202 | | putative HLA class II assoc protein I |
| 52 | 201051_at | BE560202 | | putative HLA class II assoc protein I |
| 53 | 201105_at | NM_002305 | LGALS1 | lectin, galactoside-binding, soluble, 1 |
| 54 | 201106_at | NM_002085 | GPX4 | glutathione peroxidase 4 |
| 55 | 201188_s_at | D26351 | ITPR3 | type 3 inositol 1,4,5-trisphosphate receptor |
| 56 | 201189_s_at | NM_002224 | ITPR3 | |
| 57 | 201195_s_at | AB018009 | | L-type amino acid transporter 1 |
| 58 | 201202_at | NM_002592 | PCNA | proliferating cell nuclear antigen |
| 59 | 201251_at | NM_002654 | PKM2 | pyruvate kinase, muscle |
| 60 | 201252_at | NM_006503 | PSMC4 | proteasome 26S subunit, ATPase, 4 |
| 61 | 201271_s_at | NM_016732 | RALY | RNA-binding protein transcript var 1 |
| 62 | 201291_s_at | NM_001067 | | topoisomerase (DNA) II alpha |
| 63 | 201313_at | NM_001975 | ENO2 | enolase 2 |
| 64 | 201346_at | NM_024551 | FLJ21432 | hypothetical protein FLJ21432 |
| 65 | 201393_s_at | NM_000876 | IGF2R | insulin-like growth factor 2 receptor |
| 66 | 201416_at | NM_003107 | | SRY-box 4 |
| 67 | 201417_at | NM_003107 | | SRY-box 4 |
| 68 | 201470_at | NM_004832 | GSTTLp28 | glutathione-S-transferase like; glutathione transferase omega |
| 69 | 201474_s_at | NM_002204 | ITGA3 | integrin, alpha 3 transcript variant a |
| 70 | 201485_s_at | BC004892 | RCN2 | reticulocalbin 2, EF-hand calcium binding domain |
| 71 | 201486_at | NM_002902 | RCN2 | |
| 72 | 201536_at | AL048503 | | DKFZp586M1524 |
| 73 | 201614_s_at | NM_003707 | RUVBL1 | RuvB (E coli homolog)-like 1 |
| 74 | 201660_at | | FACL3 | fatty-acid-Coenzyme A ligase, long-chain 3 |
| 75 | 201661_s_at | NM_004457 | FACL3 | |
| 76 | 201662_s_at | D89053 | | Acyl-CoA synthetase 3 |
| 77 | 201670_s_at | M68956 | MARCKS 80K-L | myristoylated alanine-rich C-kinase substrate |
| 78 | 201714_at | NM_001070 | TUBG1 | tubulin, gamma 1 |
| 79 | 201765_s_at | AL523158 | | hexosaminidase A |
| 80 | 201792_at | NM_001129 | AEBP1 | AE-binding protein 1 |
| 81 | 201804_x_at | NM_001281 | CKAP1 | cytoskeleton-associated protein 1 |
| 82 | 201819_at | NM_005505 | CD36L1 | CD36 antigen-like 1 |
| 83 | 201850_at | NM_001747 | CAPG | capping protein gelsolin-like |
| 84 | 201880_at | NM_005744 | | ariadne (Drosophila) homolog, ubiquitin-conjugating enzyme E2-binding protein, 1 |
| 85 | 201910_at | BF213279 | FARP1 | RhoGEF & pleckstrin domain 1 |
| 86 | 201911_s_at | NM_005766 | FARP1 | |
| 87 | 201931_at | NM_000126 | ETFA | electron-transfer-flavoprotein, α polypeptide |
| 88 | 201954_at | NM_005720 | ARPC1B | actin related protein 23 com, sub 1A |
| 89 | 201976_s_at | NM_012334 | MYO10 | myosin X |
| 90 | 202069_s_at | AI826060 | IDH3A | isocitrate dehydrogenase 3 alpha |
| 91 | 202070_s_at | NM_005530 | IDH3A | |
| 92 | 202111_at | NM_003040 | SLC4A2 | solute carrier fam 4 anion exchanger mem 2 |
| 93 | 202154_x_at | NM_006086 | TUBB4 | tubulin, beta, 4 |
| 94 | 202185_at | NM_001084 | PLOD3 | procollagen-lysine, 2-oxoglutarate 5-dioxygenase |
| 95 | 202188_at | NM_014669 | KIAA0095 | KIAA0095 gene product |
| 96 | 202219_at | NM_005629 | SLC6A8 | solute carrier family 6, member 8 |
| 97 | 202224_at | NM_016823 | | v-crk avian sarcoma virus CT10 oncogene homolog |
| 98 | 202225_at | NM_016823 | | v-crk avian sarcoma virus CT10 oncogene homolog |
| 99 | 202260_s_at | NM_003165 | STXBP1 | syntaxin binding protein 1 |
| 100 | 202295_s_at | NM_004390 | CTSH | cathepsin H |
| 101 | 202329_at | NM_004383 | CSK | c-src tyrosine kinase |
| 102 | 202367_at | NM_001913 | CUTL1 | cut (Drosophila)-like 1 |
| 103 | 202370_s_at | NM_001755 | CBFB | core-binding factor, β sub trans var 2 |
| 104 | 202478_at | NM_021643 | GS3955 | GS3955 protein |
| 105 | 202503_s_at | NM_014736 | KIAA0101 | KIAA0101 gene product |
| 106 | 202589_at | NM_001071 | TYMS | thymidylate synthetase |
| 107 | 202603_at | N51370 | | disintegrin and metalloproteinase domain 10 |
| 108 | 202705_at | NM_004701 | CCNB2 | cyclin B2 |
| 109 | 202737_s_at | NM_012321 | LSM4 | U6 snRNA-associated Sm-like protein |
| 110 | 202779_s_at | NM_014501 | E2-EPF | ubiquitin carrier protein |
| 111 | 202785_at | NM_005001 | NDUFA7 | NADH dehydrogenase 1 α subcomplex, 7 |
| 112 | 202862_at | NM_000137 | FAH | fumarylacetoacetate |
| 113 | 202898_at | NM_014654 | KIAA0468 | KIAA0468 gene product |
| 114 | 202954_at | NM_007019 | UBCH10 | ubiquitin carrier protein E2-C |
| 115 | 202958_at | NM_002833 | PTPN9 | protein tyrosine phosphatase, non-receptor type 9 |
| 116 | 202961_s_at | NM_004889 | ATP5J2 | ATP synthase, H+ transporting, mitochondrial F0 complex, subunit f, isoform 2 |
| 117 | 202986_at | NM_014862 | KIAA0307 | KIAA0307 gene product |
| 118 | 203011_at | NM_005536 | IMPA1 | inositol(myo)-1(or 4)-monophosphatase 1 |
| 119 | 203022_at | NM_006397 | RNASEHI | ribonuclease HI, large subunit |
| 120 | 203069_at | NM_014849 | KIAA0736 | KIAA0736 gene product |
| 121 | 203071_at | NM_004636 | SEMA3B | sema domain, Ig domain, short basic domain, secreted, 3B |
| 122 | 203094_at | NM_014628 | KIAA0110 | gene predicted from cDNA |
| 123 | 203145_at | NM_006461 | DEEPEST | mitotic spindle coiled-coil related |
| 124 | 203167_at | NM_003255 | TIMP2 | tissue inhibitor of metalloproteinase 2 |
| 125 | 203217_s_at | NM_003896 | SIAT9 | sialyltransferase 9 |
| 126 | 203234_at | NM_003364 | UP | uridine phosphorylase |
| 127 | 203256_at | NM_001793 | CDH3 | cadherin 3, type 1, P-cadherin (placental) |
| 128 | 203262_s_at | NM_004699 | DXS9928E | chromosome X 9928 expressed seq |
| 129 | 203300_x_at | NM_003916 | AP1S2 | adaptor-related protein complex 1, sigma 2 subunit |
| 130 | 203315_at | BC000103 | | NCK adaptor protein 2, |
| 131 | 203366_at | NM_002693 | POLG | polymerase (DNA directed), gamma |
| 132 | 203396_at | NM_002789 | PSMA4 | proteasome subunit, α type, 4 |
| 133 | 203452_at | NM_012200 | B3GAT3 | beta-1,3-glucuronyltransferase 3 |
| 134 | 203456_at | NM_007213 | JM4 | JM4 protein |
| 135 | 203502_at | NM_001724 | BPGM | 2,3-bisphosphoglycerate mutase |
| 136 | 203518_at | NM_000081 | CHS1 | Chediak-Higashi syndrome 1 |
| 137 | 203554_x_at | NM_004219 | PTTG1 | pituitary tumor-transforming 1 |
| 138 | 203557_s_at | NM_000281 | PCBD | 6-pyruvoyl-tetrahydropterin synthasedimerization cofactor of hepatocyte nuclear factor 1 alpha |
| 139 | 203570_at | NM_005576 | LOXL1 | lysyl oxidase-like 1 |
| 140 | 203590_a_t | NM_006141 | DNCLI2 | dynein, cytoplasmic, light intermediate polypeptide 2 |
| 141 | 203643_at | NM_006494 | ERF | Ets2 repressor factor |
| 142 | 203663_s_at | NM_004255 | COX5A | cytochrome c oxidase subunit Va |
| 143 | 203668_at | NM_006715 | MAN2C1 | mannosidase, α, class 2C, mem 1 |
| 144 | 203693_s_at | NM_001949 | E2F3 | E2F transcription factor 3 |
| 145 | 203695_s_at | NM_004403 | DFNA5 | deafness, autosomal dominant 5 |
| 146 | 203723_at | NM_002221 | ITPKB | inositol 1,4,5-trisphosphate 3-kinase B |
| 147 | 203729_at | NM_001425 | EMP3 | epithelial membrane protein 3 |
| 148 | 203730_s_at | BF196931 | ZFP95 | zinc finger protein homologous to Zfp95 in mouse |
| 149 | 203731_s_at | NM_014569 | ZFP95 | |
| 150 | 203775_at | NM_014251 | SLC25A13 | solute carrier family 25, member 13 |
| 151 | 203827_at | NM_017983 | FLJ10055 | hypothetical protein FLJ10055 |
| 152 | 203878_s_at | NM_005940 | MMP11 | matrix metalloproteinase 11 |
| 153 | 204014_at | NM_001394 | DUSP4 | dual specificity phosphatase 4 |
| 154 | 204015_s_at | BC002671 | DUSP4 | |
| 155 | 204033_at | NM_004237 | TRIP13 | thyroid hormone receptor interactor 13 |
| 156 | 204092_s_at | NM_003600 | STK15 | serinethreonine kinase 15 |
| 157 | 204099_at | NM_003078 | SMARCD3 | SWISNF related, matrix associated, actin dependent regulator of chromatin, subfamily d, member 3 |
| 158 | 204170_s_at | NM_001827 | CKS2 | CDC28 protein kinase 2 |
| 159 | 204197_s_at | NM_004350 | RUNX3 | runt-related transcription factor 3 |
| 160 | 204198_s_at | AA541630 | RUNX3 | |
| 161 | 204202_at | NM_017604 | KIAA1023 | KIAA1023 protein |
| 162 | 204228_at | NM_006347 | USA-CYP | cyclophilin |
| 163 | 204244_s_at | NM_006716 | ASK | activator of S phase kinase |
| 164 | 204247_s_at | NM_004935 | CDK5 | cyclin-dependent kinase 5 |
| 165 | 204252_at | M68520 | | cdc2-related protein kinase |
| 166 | 204262_s_at | NM_000447 | PSEN2 | presenilin 2 transcript variant 1 |
| 167 | 204423_at | NM_013255 | MKLN1 | muskelin 1, intracellular mediator containing kelch motifs |
| 168 | 204436_at | NM_025201 | PP1628 | hypothetical protein PP1628 |
| 169 | 204458_at | AL110209 | | DKFZp564A0122 |
| 170 | 204467_sat | NM_000345 | SNCA | synuclein, α transcript var NACP140 |
| 171 | 204584_at | AI653981 | L1CAM | L1 cell adhesion molecule, MASA transcript var 1 |
| 172 | 204585_s_at | NM_000425 | L1CAM | |
| 173 | 204647_at | NM_004838 | HOMER-3 | Homer, neuronal imm early gene, 3 |
| 174 | 204654_s_at | NM_003220 | TFAP2A | transcription factor AP-2 alpha |
| 175 | 204709_s_at | NM_004856 | KNSL5 | kinesin-like 5 |
| 176 | 204778_x_at | AW102783 | HOXB7 | homeo box B7 |
| 177 | 204779_s_at | NM_004502 | HOXB7 | |
| 178 | 204857_at | NM_003550 | MAD1L1 | MAD1-like 1 |
| 179 | 204932_at | BF433902 | | TNF receptor superfam, mem 11b |
| 180 | 204973_at | NM_000166 | GJB1 | gap junction protein, beta 1, 32kD |
| 181 | 204995_at | AL567411 | | cyclin-dependent kinase 5, regulatory sub 1 (p35) |
| 182 | 205051_s_at | NM_000222 | KIT | v-kit Hardy-Zuckerman 4 feline sarcoma viral oncogene homolog |
| 183 | 205142_x_at | NM_000033 | ABCD1 | ATP-binding cassette, sub-family D (ALD), mem 1 |
| 184 | 205169_at | NM_005057 | RBBP5 | retinoblastoma-binding protein 5 |
| 185 | 205373_at | NM_004389 | CTNNA2 | catenin alpha 2 |
| 186 | 205376_at | NM_003866 | INPP4B | inositol polyphosphate-4-phosphatase, type II, 105kD |
| 187 | 205405_at | NM_003966 | SEMA5A | sema domain, seven thrombospondin repeats, transmembrane domain and short cytoplasmic domain 5A |
| 188 | 205447_s_at | BE222201 | | mitogen-activated protein kinase kinase kinase 12 |
| 189 | 205458_at | BG034972 | | melanocortin 1 receptor |
| 190 | 205566_at | NM_007011 | HS1-2 | putative transmembrane protein |
| 191 | 205591_at | NM_006334 | AMY | neuroblastoma (nerve tissue) protein |
| 192 | 205681_at | NM_004049 | BCL2A1 | BCL2-related protein A1 |
| 193 | 205690_s_at | NM_003910 | G10 | maternal G10 transcript |
| 194 | 205691_at | NM_004209 | SYNGR3 | synaptogyrin 3 |
| 195 | 205717_x_at | NM_002588 | PCDHGC3 | protocadherin gamma subfamily C, 3 |
| 196 | 205813_s_at | NM_000429 | MAT1A | methionine adenosyltransferase I, α |
| 197 | 205937_at | NM_006569 | CGR11 | cell growth regulatory with EF-hand domain |
| 198 | 205945_at | NM_000565 | IL6R | interleukin 6 receptor |
| 199 | 205996_s_at | NM_013411 | AK2 B | adenylate kinase 2 |
| 200 | 206128_at | AI264306 | | adrenergic, alpha-2C-, receptor |
| 201 | 206307_s_at | NM_004472 | FOXD1 | forkhead box D1 |
| 202 | 206332_s_at | NM_005531 | IFI16 | interferon, gamma-inducible 16 |
| 203 | 206397_x_at | NM_001492 | GDF1 | growth differentiation factor 1 |
| 204 | 206441_s_at | NM_017828 | FLJ20452 | hypothetical protein FLJ20452 |
| 205 | 206462_s_at | NM_002530 | NTRK3 | neurotrophic tyrosine kinase, receptor, type 3 |
| 206 | 206503_x_at | NM_002675 | PML | promyelocytic leukemia |
| 207 | 206617_s_at | NM_002910 | RENBP | renin-binding protein |
| 208 | 206630_at | NM_000372 | TYR | tyrosinase |
| 209 | 206688_s_at | NM_006693 | CPSF4 | cleavage and polyadenylation specific factor 4, 30kD subunit |
| 210 | 206696_at | NM_000273 | OA1 | ocular albinism 1 |
| 211 | 206777_s_at | NM_000496 | CRYBB2 | crystallin, beta B2 |
| 212 | 206864_s_at | NM_003806 | HRK | harakiri, BCL2-interacting protein |
| 213 | 206976_s_at | NM_006644 | HSP105B | heat shock 105kD |
| 214 | 207038_at | NM_004694 | SLC16A6 | solute carrier family 16 member 6 |
| 215 | 207060_at | NM_001427 | EN2 | engrailed homolog 2 |
| 216 | 207144_s_at | NM_004143 | CITED1 | Cbpp300-interacting transactivator, with GluAsp-rich carboxy-terminal domain, 1 |
| 217 | 207163_s_at | NM_005163 | AKT1 | v-akt murine thymoma viral oncogene homolog 1 |
| 218 | 207183_at | NM_006143 | GPR19 | G protein-coupled receptor 19 |
| 219 | 207592_s_at | NM_001194 | HCN2 | hyperpolarization activated cyclic nucleotide-gated potassium channel 2 |
| 220 | 207614_s_at | NM_003592 | CUL1 | cullin 1 |
| 221 | 207622_s_at | NM_005692 | ABCF2 | ATP-binding cassette, sub-fam F mem 2 |
| 222 | 207828_s_at | NM_005196 | CENPF | centromere protein F |
| 223 | 208002_s_at | NM_007274 | HBACH | cytosolic acyl coenzyme A thioester hydrolase |
| 224 | 208089_s_at | NM_030794 | FLJ21007 | hypothetical protein FLJ21007 |
| 225 | 208308_s_at | NM_000175 | GPI | glucose phosphate isomerase |
| 226 | 208540_x_at | NM_021039 | S100A14 | S100 calcium-binding protein A14 |
| 227 | 208644_at | M32721 | | poly(ADP-ribose) polymerase |
| 228 | 208657_s_at | AF142408 | | cell division control protein septin D1 |
| 229 | 208677_s_at | AL550657 | | Basigin |
| 230 | 208696_at | AF275798 | | PNAS-102 |
| 231 | 208710_s_at | AI424923 | | adaptor-related protein complex 3, delta 1 subunit |
| 232 | 208723_at | BC000350 | | ubiquitin specific protease 11 |
| 233 | 208744_x_at | BG403660 | | heat shock 105kD |
| 234 | 208837_at | BC000027 | | integral type I protein |
| 235 | 208916_at | AF105230 | SLC1A5 | neutral amino acid transporter |
| 236 | 208928_at | AF258341 | | NADPH-cytochrome P450 reductase |
| 237 | 208956_x_at | U62891 | DUT | deoxyuridine triphosphatase |
| 238 | 208974_x_at | BC003572 | | karyopherin (importin) beta 1 |
| 239 | 208975_s_at | L38951 | | importin beta subunit |
| 240 | 209015_s_at | BC002446 | | MRJ gene for a member of DNAJ fam |
| 241 | 209036_s_at | BC001917 | | malate dehydrogenase 2, NAD |
| 242 | 209053_s_at | AF083389 | | Wolf-Hirschhorn syn candidate 1 |
| 243 | 209072_at | M13577 | MBP | myelin basic protein |
| 244 | 209079_x_at | AF152318 | PCDH-gamma-A1 | protocadherin gamma A1 |
| 245 | 209081_s_at | NM_030582 | COL18A1 | collagen, type XVIII, alpha 1 |
| 246 | 209123_at | BC000576 | | quinoid dihydropteridine reductase |
| 247 | 209132_s_at | BE313890 | | hypothetical protein FLJ20452 |
| 248 | 209172_s_at | U30872 | | mitosin |
| 249 | 209197_at | AA626780 | | KIAA0080 protein |
| 250 | 209198_s_at | BC004291 | | Similar to synaptotagmin 11 |
| 251 | 209247_s_at | BC001661 | | ATP-binding cassette, sub-fam F mem 2 |
| 252 | 209254_at | AF277177 | | KIAA0265 protein |
| 253 | 209255_at | AF277177 | | KIAA0265 protein |
| 254 | 209256_s_at | AF277177 | | PNAS-119 |
| 255 | 209283_at | AF007162 | | unknown mRNA |
| 256 | 209345_s_at | AL561930 | | phosphatidylinositol 4-kinase type II |
| 257 | 209407_s_at | AF068892 | | Dukes type B colon adenocarcinoma truncated suppressin |
| 258 | 209515_s_at | U38654 | | Rab27a |
| 259 | 209773_s_at | BC001886 | | ribonucleotide reductase M2 polypep |
| 260 | 209825_s_at | BC002906 | | Sim to uridine monophosphate kinase |
| 261 | 209827_s_at | NM 004513 | IL16 | interleukin 16 |
| 262 | 209828_s_at | M90391 | | putative IL-16 protein precursor |
| 263 | 209848_s_at | U01874 | | me20m |
| 264 | 209875_s_at | M83248 | | nephropontin |
| 265 | 209932_s_at | U90223 | | deoxyuridine triphosphate nucleotidohydrolase precursor |
| 266 | 210052_s_at | AF098158 | | restricted expressed proliferation associated protein 100 |
| 267 | 210073_at | L32867 | | alpha 2,8-sialyltransferase |
| 268 | 210111_s_at | AF277175 | | PNAS-138 |
| 269 | 210127_at | BC002510 | | small GTPase RAB6B |
| 270 | 210854_x_at | U17986 | | GABAnoradrenaline transporter |
| 271 | 210926_at | AY014272 | FKSG30 | FKSG30 |
| 272 | 210948_s_at | AF294627 | LEF1 | lymphoid enhancer factor 1 isoform |
| 273 | 210951_x_at | AF125393 | | Rab27 isoform |
| 274 | 211013_x_at | AF230411 | | tripartite motif protein TRIM19 lambda |
| 275 | 211052_s_at | BC006364 | | clone MGC:12705, |
| 276 | 211066_x_at | BC006439 | | Similar to protocadherin gamma subfamily A, 5 |
| 277 | 211373_s_at | U34349 | AD3LPAD5 | seven trans-membrane domain |
| 278 | 211564_s_at | BC003096 | | Sim to LIM domain protein |
| 279 | 211752_s_at | BC005954 | | clone MGC:14592 |
| 280 | 211759_x_at | BC005969 | | clone MGC:14625 |
| 281 | 211833_s_at | U19599 | | BAX delta |
| 282 | 212000_at | AB002363 | | KIAA0365 gene product |
| 283 | 212070_at | AL554008 | | G protein-coupled receptor 56 |
| 284 | 212081_x_at | AF129756 | MSH55 | MSH55 |
| 285 | 212119_at | BF348067 | | phosphatidylinositol glycan, class F |
| 286 | 212178_s_at | AK022555 | | FLJ12493 fis |
| 287 | 212193_s_at | BE881529 | | KIAA0731 protein |
| 288 | 212247_at | AW008531 | | KIAA0225 protein |
| 289 | 212285_s_at | AF016903 | | IMAGE:3506210 |
| 290 | 212312_at | AL117381 | | clone RP5-857M17 on chrom 20 |
| 291 | 212338_at | AA621962 | | KIAA0727 protein |
| 292 | 212402_at | BE895685 | | KIAA0853 protein |
| 293 | 212472_at | BE965029 | | FLJ22463 fis |
| 294 | 212473_s_at | BE965029 | | FLJ22463 fis |
| 295 | 212512_s_at | AA551784 | | coactivator-associated arginine methyltransferase-1 |
| 296 | 212520_s_at | AI684141 | | SWISNF related |
| 297 | 212552_at | BE617588 | | hippocalcin-like |
| 298 | 212715_s_at | AB020626 | | KIAA0819 protein |
| 299 | 212739_s_at | AL523860 | | non-metastatic cells 4 |
| 300 | 212744_at | AI813772 | | clone HQ0692 |
| 301 | 212745_s_at | AI813772 | | clone HQ0692 |
| 302 | 212793_at | BF513244 | | KIAA0381 protein |
| 303 | 212796_s_at | BF195608 | | KIAA1055 protein |
| 304 | 213002_at | BF347326 | MARCKS, 80K-L | myristoylated alanine-rich protein kinase C substrate |
| 305 | 213007_at | BG478677 | | polymerase (DNA directed), gamma |
| 306 | 213008_at | BG478677 | | polymerase (DNA directed), gamma |
| 307 | 213096_at | T51252 | | KIAA0481 gene product |
| 308 | 213131_at | R38389 | | olfactomedin related ER localized |
| 309 | 213169_at | BG109855 | | clone TUA8 Cri-du-chat region |
| 310 | 213215_at | AI910895 | | clone EUROIMAGE 42138 |
| 311 | 213217_at | AU149572 | | adenylate cyclase 2(brain) |
| 312 | 213241_at | AF035307 | | clone 23785 |
| 313 | 213274_s_at | BE875786 | | cathepsin B |
| 314 | 213275_x_at | BE875786 | | cathepsin B |
| 315 | 213330_s_at | BE886580 | | stress-induced-phosphoprotein 1 |
| 316 | 213333_at | AL520774 | | malate dehydrogenase 2, NAD |
| 317 | 213338_at | BF062629 | | DKFZP586E1621 protein |
| 318 | 213392_at | AW070229 | | G protein-coupled receptor, fam C, group 5, mem B |
| 319 | 213474_at | AI890903 | | ESTs |
| 320 | 213496_at | AW592563 | | KIAA0455 gene product |
| 321 | 213573_at | AA861608 | | karyopherin (importin) beta 1 |
| 322 | 213587_s_at | AI884867 | | ribosomal protein L26 |
| 323 | 213638_at | AW054711 | | PAC 257A7 on chromosome 6p24 |
| 324 | 213670_x_at | AI768378 | | KIAA0618 gene product |
| 325 | 213720_s_at | AI831675 | | SWISNF related, matrix associated, actin dependent regulator of chromatin, subfam a, member 4 |
| 326 | 213746_s_at | AW051856 | | filamin A, alpha |
| 327 | 213836_s_at | AW052084 | | KIAA1001 protein |
| 328 | 213895_at | BF445047 | | epithelial membrane protein 1 |
| 329 | 213960_at | T87225 | | CLONE=IMAGE:22392 |
| 330 | 214023_x_at | AL533838 | | tubulin, beta polypeptide |
| 331 | 214068_at | AF070610 | | clone 24505 |
| 332 | 214104_at | AI703188 | | G-protein coupled receptor |
| 333 | 214201_x_at | AA742237 | | HLA-B associated transcript-2 |
| 334 | 214581_x_at | BE568134 | | death receptor 6 |
| 335 | 214614_at | AI738662 | | homeo box HB9 |
| 336 | 214632_at | AA295257 | | neuropilin 2 |
| 337 | 214656_x_at | BE790157 | | myosin IB |
| 338 | 214687_x_at | AK026577 | | FLJ22924 fis |
| 339 | 214708_at | BG484314 | | syntrophin, beta 1 |
| 340 | 214710_s_at | BE407516 | | cyclin B1 |
| 341 | 214714_at | AK022360 | | FLJ12298 fis |
| 342 | 214717_at | AL137534 | | DKFZp434H1419 |
| 343 | 214752_x_at | A1625550 | | filamin A, alpha |
| 344 | 214778_at | AB011541 | | MEGF8 |
| 345 | 214841_at | AF070524 | | clone 24453 |
| 346 | 214893_x_at | AI421964 | | hyperpolarization activated cyclic nucleotide-gated potassium channel 2 |
| 347 | 214896_at | AL109671 | | EUROIMAGE 29222 |
| 348 | 215025_at | S76476 | | trkC {alternatively spliced} |
| 349 | 215115_x_at | AI613045 | | ets variant gene 6 (TEL oncogene) |
| 350 | 215126_at | AL109716 | | EUROIMAGE 208948 |
| 351 | 215155_at | J04178 | HEXA | abnormal β-hexosaminidase α chain |
| 352 | 215311_at | AL109696 | | EUROIMAGE 21920 |
| 353 | 215812_s_at | U41163 | SLC6A10 | creatine transporter |
| 354 | 215836_s_at | AK026188 | | FLJ22535 fis |
| 355 | 216194_s_at | AD001527 | | DNA from chrom 19-cosmid f24590 containing CAPNS and POL2RI |
| 356 | 216973_s_at | S49765 | | homeo box B7 |
| 357 | 217033_x_at | S76475 | trkC | neurotrophic tyrosine kinase, receptor, type 3 |
| 358 | 217104_at | AL109714 | | EUROIMAGE 327506 |
| 359 | 217226_s_at | M95929 | PHOX1 | Paired mesoderm homeo box 1 |
| 360 | 217297_s_at | AF143684 | MYO9b | unconventional myosin IXb |
| 361 | 217377_x_at | AF041811 | ETV6-NTRK3 fusion | ETS related protein-growth factor receptor tyrosine kinase fusion proteins |
| 362 | 217419_x_at | AK021586 | | FLJ11524fis |
| 363 | 217624_at | AA464753 | | ESTs |
| 364 | 217799_x_at | NM 003344 | UBE2H | ubiquitin-conjugating enzyme E2H |
| 365 | 217827_s_at | NM 016630 | ACP33 | acid cluster protein 33 |
| 366 | 217867_x_at | NM 012105 | BACE2 | beta-site APP-cleaving enzyme 2 |
| 367 | 217874_at | NM_003849 | SUCLG1 | succinate-CoA ligase, GDP-forming, alpha subunit |
| 368 | 217891_at | NM_022744 | FLJ13868 | hypothetical protein FLJ13868 |
| 369 | 218009_s_at | NM_003981 | PRC1 | protein regulator of cytokinesis 1 |
| 370 | 218030_at | NM_014030 | GIT1 | G protein-coupled receptor kinase-interactor 1 |
| 371 | 218074_at | NM_016062 | LOC51647 | CGI-128 protein |
| 372 | 218143_s_at | NM_005697 | SCAMP2 | secretory carrier membrane protein 2 |
| 373 | 218151_x_at | NM_024531 | FLJ11856 | hypothetical protein FLJ11856 |
| 374 | 218152_at | NM_018200 | HMG20A | high-mobility group 20A |
| 375 | 218161_s_at | NM_017882 | FLJ20561 | hypothetical protein FLJ20561 |
| 376 | 218175_at | NM_025140 | FLJ22471 | hypothetical protein FLJ22471 |
| 377 | 218330_s_at | NM_018162 | FLJ10633 | hypothetical protein FLJ10633 |
| 378 | 218349_s_at | NM_017975 | FLJ10036 | hypothetical protein FLJ10036 |
| 379 | 218359_at | NM_024958 | FLJ23329 | hypothetical protein FLJ23329 |
| 380 | 218376_s_at | NM_022765 | FLJ11937 | hypothetical protein FLJ11937 |
| 381 | 218447_at | NM_020188 | DC13 | DC13 protein |
| 382 | 218542_at | NM_018131 | FLJ10540 | hypothetical protein FLJ10540 |
| 383 | 218564_at | BC002574 | FLJ10520 | hypothetical protein FLJ10520 |
| 384 | 218618_s_at | NM_022763 | FLJ23399 | hypothetical protein FLJ23399 |
| 385 | 218678_at | NM_024609 | FLJ21841 | hypothetical protein FLJ21841 |
| 386 | 218774_at | NM_014026 | HSPC015 | HSPC015 protein |
| 387 | 218786_at | NM_016575 | TU12B1-TY | TU12B1-TY protein |
| 388 | 218824_at | NM_018215 | FLJ10781 | hypothetical protein FLJ10781 |
| 389 | 218839_at | NM_012258 | HEY1 | hairyenhancer-of-split related with YRPW motif 1 |
| 390 | 218856_at | NM_016629 | LOC51323 | hypothetical protein LOC51323 |
| 391 | 218888_s_at | NM_018092 | FLJ10430 | hypothetical protein FLJ10430 |
| 392 | 218931_at | NM_022449 | FLJ12538 | hypothetical protein FLJ12538 |
| 393 | 218952_at | NM_013271 | SAAS | granin-like neuroendocrine peptide precursor |
| 394 | 218956_s_at | NM_015545 | KIAA0632 | KIAA0632 protein |
| 395 | 218980_at | NM_025135 | KIAA1695 | hypothetical protein FLJ22297 |
| 396 | 218996_at | NM_013342 | TFPT | TCF3 (E2A) fusion partner |
| 397 | 219011_at | NM_020904 | PLEKHA4 | pleckstrin homology domain-containing, family A member 4 |
| 398 | 219039_at | NM_017789 | FLJ20369 | hypothetical protein FLJ20369 |
| 399 | 219040_at | NM_024535 | FLJ22021 | hypothetical protein FLJ22021 |
| 400 | 219041_s_at | NM_014374 | AP4 | zinc finger protein |
| 401 | 219051_x_at | NM_024042 | MGC2601 | hypothetical protein MGC2601 |
| 402 | 219066_at | NM_021823 | MDS018 | hypothetical protein MDS018 |
| 403 | 219066_at | NM_021823 | MDS018 | hypothetical protein MDS018 |
| 404 | 219148_at | NM_018492 | TOPK | PDZ-binding kinase; T-cell originated protein kinase |
| 405 | 219152_at | NM_015720 | PODLX2 | endoglycan |
| 406 | 219219_at | NM_017854 | FLJ20512 | hypothetical protein FLJ20512 |
| 407 | 219361_s_at | NM_022767 | FLJ12484 | hypothetical protein FLJ12484 |
| 408 | 219372_at | NM_014055 | CDV-1 | CDV-1 protein |
| 409 | 219408_at | NM_019023 | FLJ10640 | hypothetical protein FLJ10640 |
| 410 | 219478_at | NM_021197 | WFDC1 | WAP four-disulfide core domain 1 |
| 411 | 219491_at | NM_024036 | MGC3103 | hypothetical protein MGC3103 |
| 412 | 219522_at | NM_014344 | FJX1 | putative secreted ligand homologous to fjx1 |
| 413 | 219537_x_at | NM_016941 | DLL3 | Delta (Drosophila)-like 3 |
| 414 | 219555_s_at | NM_018455 | BM039 | uncharacterized bone marrow protein BM039 |
| 415 | 219578_s_at | NM_030594 | FLJ13203 | hypothetical protein FLJ13203 |
| 416 | 219634_at | NM_018413 | C4ST | chondroitin 4-sulfotransferase |
| 417 | 219637_at | NM_025139 | FLJ12584 | hypothetical protein FLJ12584 |
| 418 | 219703_at | NM_018365 | FLJ11222 | hypothetical protein FLJ11222 |
| 419 | 219742_at | NM_030567 | MGC10772 | hypothetical protein MGC10772 |
| 420 | 219895_at | NM_017938 | FLJ20716 | hypothetical protein FLJ20716 |
| 421 | 219933_at | NM_016066 | LOC51022 | CGI-133 protein |
| 422 | 220116_at | MM_021614 | KCNN2 | potassium intermediatesmall conductance calcium-activated channel, subfamily N, member 2 |
| 423 | 220155_s_at | NM_023924 | FLJ13441 | hypothetical protein FLJ13441 |
| 424 | 220178_at | NM_021731 | PP3501 | hypothetical protein PP3501 |
| 425 | 220454_s_at | NM_020796 | SEMA6A | sema domain, transmembrane domain and cytoplasmic domain, 6A |
| 426 | 220864_s_at | NM_015965 | LOC51079 | CGI-39 protein; cell death-regulatory protein GRIM19 |
| 427 | 220948_s_at | NM_000701 | ATP1A1 | ATPase, Na+K+ transporting, alpha 1 polypeptide |
| 428 | 220973_s_at | NM_030974 | | hypothetical protein DKFZp434N1923 |
| 429 | 220974_x_at | NM_030971 | BA108L7.2 | similar to rat tricarboxylate carrier-like |
| 430 | 220980_s_at | NM_031284 | | hypothetical protein DKFZp434B195 |
| 431 | 221059_s_at | NM_021615 | CHST6 | carbohydrate sulfotransferase 6 |
| 432 | 221483_s_at | AF084555 | ARPP-19 | okadaic acid-inducible and cAMP-regulated phosphoprotein 19 |
| 433 | 221484_at | NM_004776 | | UDP-Gal:betaGlcNAc beta 1,4-galactosyltransferase, polypeptide 5 |
| 434 | 221538_s_at | AL136663 | | DKFZp564A176 |
| 435 | 221558_s_at | AF288571 | LEF1 | lymphoid enhancer factor-1 |
| 436 | 221577_x_at | AF003934 | | prostate differentiation factor |
| 437 | 221641_s_at | AF241787 | | CGI16-iso |
| 438 | 221688_s_at | AL136913 | | DKFZp586L0118 |
| 439 | 221710_x_at | BC006241 | | hypothetical protein FLJ10647 |
| 440 | 221732_at | AK026161 | | FLJ22508 fis |
| 441 | 221759_at | AL583123 | | CLONE=CSODL009YN09 |
| 442 | 221797_at | AY007126 | | clone CDABP0028 |
| 443 | 221799_at | AB037823 | | KIAA1402 protein, |
| 444 | 221815_at | BE671816 | | ESTs |
| 445 | 221882_s_at | AI636233 | | five-span transmembrane protein M83 |
| 446 | 221902_at | AL567940 | | CLONE=CS0DF036YK19 |
| 447 | 221909_at | BF984207 | | ESTs |
| 448 | 221962_s_at | AI829920 | | ubiquitin-conjugating enzyme E2H |
| 449 | 222116_s_at | AL157485 | | DKFZp762O207 |
| 450 | 222153_at | AK023133 | | FLJ13071 fis |
| 451 | 222155_s_at | AK021918 | . | FLJ11856 fis |
| 452 | 222175_s_at | AK000003 | | FLJ00003 protein |
| 453 | 222196_at | AK000470 | | FLJ20463 fis |
| 454 | 222199_s_at | AK001289 | | FLJ10427 fis |
| 455 | 222206_s_at | AA781143 | | EUROIMAGE 2021883 |
| 456 | 222212_s_at | AK001105 | | FLJ10243 fis |
| 457 | 222231_s_at | AK025328 | | FLJ21675 fis |
| 458 | 222234_s_at | AK022644 | | FLJ12582 fis |
| 459 | 222240_s_at | AL137749 | | DKFZp434A0612 |
| 460 | 222294_s_at | AW971415 | | ESTs |
| 461 | 32811_at | X98507 | | myosin-I beta |
| 462 | 40560_at | U28049 | TXB2 | TBX2 |
| 463 | 44783_s_at | R61374 | | IMAGE-37665 |
| 464 | 46665_at | AI949392 | | IMAGE-2470926 |
| 465 | 55093_at | AA534198 | | IMAGE-993116 |
| 466 | 63825_at | AI557319 | | |
| 467 | 87100_at | AI832249 | | |
| 468 | 200017_at | NM_002954 | RPS27A | ribosomal protein S27a |
| 469 | 200606_at | NM_004415 | DSP | desmoplakin (DPI, DPII) |
| 470 | 200632_s_at | NM_006096 | NDRG1 | N-myc downstream regulated |
| 471 | 200636_s_at | NM_002840 | PTPRF | protein tyrosine phosphatase, receptor type, F |
| 472 | 200795_at | NM_004684 | SPARCL1 | SPARC-like 1 |
| 473 | 200810_s_at | NM_001280 | CIRBP | cold inducible RNA-binding protein |
| 474 | 200897_s_at | NM_016081 | KIAA0992 | Palladin |
| 475 | 200953_s_at | NM_001759 | CCND2 | cyclin D2 |
| 476 | 200965_s_at | NM_006720 | ABLIM-s | actin binding LIM protein 1 transcript variant |
| 477 | 201012_at | NM_000700 | ANXA1 | annexin A1 |
| 478 | 201041_s_at | NM_004417 | DUSP1 | dual specificity phosphatase 1 |
| 479 | 201125_s_at | NM_002213 | ITGB5 | integrin, beta 5 |
| 480 | 201200_at | NM_003851 | CREG | cellular repressor of E1A-stimulated genes |
| 481 | 201286_at | Z48199 | syndecan 1 | syndecan-1 gene (exons 2-5) |
| 482 | 201328_at | AL575509 | | v-ets avian erythroblastosis virus E26 oncogene homolog 2 |
| 483 | 201425_at | NM_000690 | ALDH2 | aldehyde dehydrogenase 2, mitochondrial |
| 484 | 201427_s_at | NM_005410 | SEPP1 | selenoprotein P, plasma, 1 |
| 485 | 201432_at | NM_001752 | CAT | Catalase |
| 486 | 201540_at | NM_001449 | FHL1 | four and a half LIM domains 1 |
| 487 | 201667_at | NM_000165 | GJA1 | gap junction protein, alpha 1, 43kD |
| 488 | 201681_s_at | AB011155 | KIAA0583 | KIAA0583 |
| 489 | 201798_s_at | NM_013451 | FER1L3 | fer-1 (C.elegans)-like 3 (myoferlin) |
| 490 | 201820_at | NM_000424 | KRT5 | keratin 5 |
| 491 | 201829_at | AW263232 | NET1 | neuroepithelial cell transf gene 1 |
| 492 | 201830_s_at | NM_005863 | NET1 | neuroepithelial cell transf gene 1 |
| 493 | 201839_s_at | NM_002354 | TACSTD1 | tumor-associated calcium signal transducer 1 |
| 494 | 201842_s_at | AI826799 | | EGF-containing fibulin-like extracellular matrix protein 1 |
| 495 | 201843_s_at | NM_004105 | EFEMP1 | EGF-containing fibulin-like extracellular matrix protein 1 transcript variant 1 |
| 496 | 201983_s_at | AW157070 | | epidermal growth factor receptor |
| 497 | 201984_s_at | NM_005228 | EGFR | epidermal growth factor receptor |
| 498 | 202054_s_at | NM_000382 | ALDH3A2 | aldehyde dehydrogenase 3 family, member A2 |
| 499 | 202085_at | NM_004817 | TJP2 | tight junction protein 2 |
| 500 | 202193_s_at | NM_001144 | AMFR | autocrine motility factor receptor |
| 501 | 202196_s_at | NM_013253 | DKK3 | dickkopf (Xenopus laevis) homolog 3 |
| 502 | 202242_at | NM_004615 | TM4SF2 | transmembrane 4 superfamily mem 2 |
| 503 | 202267_at | NM_005562 | LAMC2 | laminin, gamma 2, transcript variant 1 |
| 504 | 202286_s_at | J04152 | GA733-1 | gastrointestinal tumor-assoc antigen |
| 505 | 202289_s_at | NM_006997 | TACC2 | transforming, acidic coiled-coil containing protein 2 |
| 506 | 202350_s_at | NM_002380 | MATN2 | matrilin 2 precursor, transcript var 1 |
| 507 | 202387_at | NM_004323 | BAG1 | BCL2-associated athanogene |
| 508 | 202489_s_at | BC005238 | | FXYD domain-containing ion transport regulator 3 |
| 509 | 202525_at | NM_002773 | PRSS8 | protease, serine, 8 (prostasin) |
| 510 | 202552_s_at | NM_016441 | CRIM1 | cysteine-rich motor neuron 1 |
| 511 | 202565_s_at | NM_003174 | SVIL | supervillin transcript variant 1 |
| 512 | 202575_at | NM_001878 | CRABP2 | cellular retinoic acid-binding protein 2 |
| 513 | 202597_at | AU144284 | | interferon regulatory factor 6 |
| 514 | 202668_at | BF001670 | | ephrin-B2 |
| 515 | 202712_s_at | NM_020990 | CKMT1 | creatine kinase, mitochondrial 1 nuclear gene mitochondrial protein |
| 516 | 202746_at | AL021786 | | PAC 696H22 on chrom Xq21.1-21.2 |
| 517 | 202826_at | NM_003710 | SPINT1 | serine protease inhibitor, Kunitz t 1 |
| 518 | 202890_at | T62571 | | microtubule-associated protein 7 |
| 519 | 202936_s_at | NM_000346 | SOX9 | SRY-box 9 |
| 520 | 202994_s_at | Z95331 | | clone CTA-941 F9 on chrom 22q13 |
| 521 | 203037_s_at | NM_014751 | KIAA0429 | KIAA0429 gene product |
| 522 | 203074_at | NM_001630 | ANXA8 | annexin A8 |
| 523 | 203081_at | NM_020248 | LOC56998 | beta-catenin-interacting protein ICAT |
| 524 | 203126_at | NM_014214 | IMPA2 | inositol(myo)-1(or 4)-monophosphatase 2 |
| 525 | 203178_at | NM_001482 | GATM | glycine amidinotransferase |
| 526 | 203240_at | NM_003890 | FC(γ)BP | IgG Fc binding protein |
| 527 | 203327_at | N22903 | | insulin-degrading enzyme |
| 528 | 203355_s_at | NM_015310 | KIAA0942 | KIAA0942 protein |
| 529 | 203407_at | NM_002705 | PPL | Periplakin |
| 530 | 203408_s_at | NM_002971 | SATB1 | special AT-rich sequence binding protein 1 |
| 531 | 203430_at | NM_014320 | SOUL | putative heme-binding protein |
| 532 | 203453_at | NM_001038 | SCNN1A | Na channel, nonvoltage-gated 1 α |
| 533 | 203485_at | NM_021136 | RTN1 | reticulon 1 |
| 534 | 203549_s_at | NM_000237 | LPL | lipoprotein lipase |
| 535 | 203571_s_at | NM_006829 | APM2 | adipose specific 2 |
| 536 | 203585_at | NM_007150 | ZNF185 | zinc finger protein 185 (LIM domain) |
| 537 | 203636_at | BE967532 | MID1 | midline 1 (OpitzBBB syndrome) |
| 538 | 203637_s_at | NM_000381 | MID1 | |
| 539 | 203638_s_at | NM_022969 | FGFR2 | FGF receptor 2 transcript var 2 |
| 540 | 203678_at | NM_014967 | KIAA1018 | KIAA1018 protein |
| 541 | 203687_at | NM_002996 | SCYD1 | small inducible cytokine subfam D (Cys-X3-Cys) mem 1 |
| 542 | 203726_s_at | NM_000227 | LAMA3 | laminin, alpha 3 |
| 543 | 203786_s_at | NM_003287 | TPD52L1 | tumor protein D52-like 1 |
| 544 | 203797_at | AF039555 | VSNL1 | visinin-like protein 1 |
| 545 | 203799_at | NM_014880 | KIAA0022 | KIAA0022 gene product |
| 546 | 203812_at | AB011538 | | MEGF5 |
| 547 | 203881_s_at | NM_004010 | | dystrophin transcript variant Dp427p2 |
| 548 | 203910_at | NM_004815 | PARG1 | PTPL1-associated RhoGAP 1 |
| 549 | 203917_at | NM_001338 | CXADR | coxsackie virus and adenovirus receptor |
| 550 | 203961_at | AL157398 | NEBL | nebulette protein |
| 551 | 203962_s_at | NM_006393 | NEBL | |
| 552 | 203963_at | NM_001218 | CA12 | carbonic anhydrase XII |
| 553 | 203992_s_at | AF000992 | UTX | ubiquitous TPR motif, X isoform alternative transcript 1 |
| 554 | 203997_at | NM_002829 | PTPN3 | protein tyrosine phosphatase, non-receptor type 3 |
| 555 | 204005_s_at | NM_002583 | PAWR | PRKC, apoptosis, WT1, regulator |
| 556 | 204019_s_at | NM_015677 | | hypothetical protein DKFZP586F1318 |
| 557 | 204036_at | AW269335 | | endothelial differentiation, lysophosphatidic acid G-protein-coupled receptor, 2 |
| 558 | 204037_at | AW269335 | | endothelial differentiation, lysophosphatidic acid G-protein-coupled receptor, 2 |
| 559 | 204042_at | AB020707 | | KIAA0900 protein |
| 560 | 204058_at | AL049699 | | clone 747H23 on chrom 6q13-15 |
| 561 | 204059_s_at | NM_002395 | ME1 | malic enzyme 1, NADP(+)-dependent, cytosolic |
| 562 | 204072_s_at | NM_023037 | 13CDNA73 | putative gene product |
| 563 | 204112_s_at | NM_006895 | HNMT | histamine N-methyltransferase |
| 564 | 204135_at | NM_014890 | DOC1 | downregulated in ovarian cancer 1 |
| 565 | 204136_at | NM_000094 | COL7A1 | collagen, type VII, alpha 1 |
| 566 | 204151_x_at | NM_001353 | AKR1C1 | aldo-keto reductase family 1, mem C1 |
| 567 | 204154_at | NM_001801 | CDO1 | cysteine dioxygenase, type I |
| 568 | 204168_at | NM_002413 | MGST2 | microsomal glutathione S-transferase 2 |
| 569 | 204201_s_at | NM_006264 | PTPN13 | protein tyrosine phosphatase, non-receptor type 13 |
| 570 | 204204_at | NM_001860 | SLC31A2 | solute carrier family 31 member 2 |
| 571 | 204224_s_at | NM_000161 | GCH1 | GTP cyclohydrolase 1 |
| 572 | 204254_s_at | NM_000376 | VDR | vitamin D receptor |
| 573 | 204345_at | NM_001856 | COL16A1 | collagen, type XVI, alpha 1 |
| 574 | 204351_at | NM_005980 | S100P | S100 calcium-binding protein P |
| 575 | 204359_at | NM_013231 | FLRT2 | fibronectin leucine rich transmembrane protein 2 |
| 576 | 204363_at | NM_001993 | F3 | coagulation factor III |
| 577 | 204379_s_at | NM_000142 | FGFR3 | fibroblast growth factor receptor 3 |
| 578 | 204388_s_at | NM_000240 | MAOA | monoamine oxidase A |
| 579 | 204389_at | NM_000240 | MAOA | monoamine oxidase A |
| 580 | 204400_at | NM_005864 | EFS2 | signal transduction protein |
| 581 | 204421_s_at | M27968 | FGF | basic fibroblast growth factor |
| 582 | 204422_s_at | NM_002006 | FGF2 | fibroblast growth factor 2 (basic) |
| 583 | 204424_s_at | AL050152 | | neuronal specific transcription factor DAT1 |
| 584 | 204455_at | NM_001723 | BPAG1 | bullous pemphigoid antigen 1 |
| 585 | 204503_at | NM_001988 | EVPL | envoplakin |
| 586 | 204517_at | BE962749 | cyclophilin C | peptidylprolyl isomerase C |
| 587 | 204519_s_at | NM_015993 | LOC51090 | plasmolipin |
| 588 | 204537_s_at | NM_004961 | GABRE | gamma-aminobutyric acid A receptor, epsilon transcript variant 1 |
| 589 | 204591_at | NM_006614 | CHL1 | cell adhesion molecule with homology to L1CAM |
| 590 | 204600_at | NM_004443 | EPHB3 | EphB3 |
| 591 | 204671_s_at | BE677131 | | KIAA0957 protein |
| 592 | 204675_at | NM_001047 | SRD5A1 | steroid-5-α-reductase, α polypeptide 1 |
| 593 | 204718_at | NM_004445 | EPHB6 | EphB6 |
| 594 | 204719_at | NM_007168 | ABCA8 | ATP-binding cassette, sub-fam A mem 8 |
| 595 | 204734_at | NM_002275 | KRT15 | keratin 15 |
| 596 | 204749_at | NM_004538 | NAP1L3 | nucleosome assembly protein 1-like 3 |
| 597 | 204753_s_at | AI810712 | | hepatic leukemia factor |
| 598 | 204754_at | AI810712 | | hepatic leukemia factor |
| 599 | 204755_x_at | M95585 | HLF | leukemia factor |
| 600 | 204765_at | NM_005435 | ARHGEF5 | Rho guanine nucleotide exchange factor 5 |
| 601 | 204773_at | NM_004512 | IL11RA | interleukin 11 receptor, alpha |
| 602 | 204773_at | NM_004512 | IL11RA | |
| 603 | 204855_at | NM_002639 | SERPINB5 | serine (or cysteine) proteinase inhibitor, clade B, member 5 |
| 604 | 204872_at | NM_007005 | BCE-1 | BCE-1 protein |
| 605 | 204937_s_at | NM_016325 | ZNF274 | zinc finger protein 274 |
| 606 | 204942_s_at | NM_000695 | ALDH3B2 | aldehyde dehydrogenase 3 fam mem B2 |
| 607 | 204952_at | NM_014400 | C4.4A | GPI-anchored metastasis-associated protein homolog |
| 608 | 204971_at | NM_005213 | CSTA | cystatin A (stefin A) |
| 609 | 204975_at | NM_001424 | EMP2 | epithelial membrane protein 2 |
| 610 | 204990_s_at | NM_000213 | ITGB4 | integrin, beta 4 |
| 611 | 205014_at | NM_005130 | HBP17 | heparin-binding growth factor binding |
| 612 | 205019_s_at | NM_004624 | VIPR1 | vasoactive intestinal pep receptor 1 |
| 613 | 205081_at | NM_001311 | CRIP1 | cysteine-rich protein 1 (intestinal) |
| 614 | 205109_s_at | NM_015320 | ARHGEF4 | Rho guanine nucleotide exchange factor (GEF) 4 |
| 615 | 205128_x_at | NM_000962 | PTGS1 | prostaglandin-endoperoxide synthase 1 |
| 616 | 205185_at | NM_006846 | SPINK5 | serine protease inhibitor, Kazal t, 5 |
| 617 | 205200_at | NM_003278 | TNA | tetranectin |
| 618 | 205206_at | NM_000216 | KAL1 | Kallmann syndrome 1 sequence |
| 619 | 205236_x_at | NM_003102 | SOD3 | superoxide dismutase 3, extracellular |
| 620 | 205251_at | NM_022817 | PER2 | period homolog 2 transcript variant 1 |
| 621 | 205259_at | NM_000901 | NR3C2 | nuclear receptor subfamily 3, group C, member 2 |
| 622 | 205286_at | U85658 | | transcription factor ERF-1 |
| 623 | 205349_at | NM_002068 | GNA15 | guanine nucleotide binding protein, α 15 |
| 624 | 205363 _at | NM_003986 | BBOX1 | butyrobetaine (γ), 2-oxoglutarate dioxygenase 1 |
| 625 | 205382_s_at | NM_001928 | DF | D component of complement (adipsin) |
| 626 | 205384_at | NM_005031 | FXYD1 | FXYD domain-containing ion transport regulator 1 variant a |
| 627 | 205403_at | NM_004633 | IL1R2 | interleukin 1 receptor, type II |
| 628 | 205404_at | NM_005525 | HSD11B1 | hydroxysteroid dehydrogenase 1 |
| 629 | 205407_at | NM_021111 | RECK | reversion-inducing-cysteine-rich protein with kazal motifs |
| 630 | 205440_s_at | NM_000909 | NPY1R | neuropeptide Y receptor Y1 |
| 631 | 205455_at | NM_002447 | MST1R | macrophage stimulating 1 receptor |
| 632 | 205464_at | NM_000336 | SCNN1B | Na channel, nonvoltage-gated 1, β |
| 633 | 205470_s_at | NM_006853 | KLK11 | kallikrein 11 |
| 634 | 205490_x_at | BF060667 | connexin 31 | gap junction protein, beta 3, 31 kD |
| 635 | 205498_at | NM_000163 | GHR | growth hormone receptor |
| 636 | 205559_s_at | NM_006200 | PCSK5 | proprotein convertase subtilisinkexin type 5 |
| 637 | 205560_at | NM_006200 | PCSK5 | |
| 638 | 205569_at | NM_014398 | TSC403 | similar to lysosome-associated membrane glycoprotein |
| 639 | 205613_at | NM_016524 | LOC51760 | BK protein |
| 640 | 205668_at | NM_002349 | LY75 | lymphocyte antigen 75 |
| 641 | 205672_at | NM_000380 | XPA | xeroderma pigmentosum, complementation group A |
| 642 | 205709_s_at | NM_001263 | CDS1 | CDP-diacylglycerol synthase 1 |
| 643 | 205730_s_at | NM_014945 | KIAA0843 | KIAA0843 protein |
| 644 | 205765_at | NM_000777 | CYP3A5 | cyt P450, subfam IIIA, polypep 5 |
| 645 | 205807_s_at | NM_020127 | TUFT1 | tuftelin 1 |
| 646 | 205857_at | AI269290 | | solute carrier family 18, member 2 |
| 647 | 205883_at | NM_006006 | ZNF145 | zinc finger protein 145 |
| 648 | 205900_at | NM_006121 | KRT1 | keratin 1 |
| 649 | 205933_at | NM_015559 | KIAA0437 | KIAA0437 protein |
| 650 | 205977_s_at | NM_005232 | EPHA1 | EphA1 |
| 651 | 206032_at | AI797281 | | est:we86g02.x1 |
| 652 | 206033_s_at | NM_001941 | DSC3 | desmocollin 3 transcript variant Dsc3a |
| 653 | 206068_s_at | AI367275 | | acyl-Coenzyme A dehydrogenase, long chain |
| 654 | 206093_x_at | NM_007116 | TNXA | tenascin XA |
| 655 | 206122_at | NM_006942 | SOX20 | SRY-box 20 |
| 656 | 206149_at | MM_022097 | LOC63928 | hepatocellular carcinoma antigen gene 520 |
| 657 | 206170_at | NM_000024 | ADRB2 | adrenergic, beta-2-, receptor, surface |
| 658 | 206192_at | L20815 | | S protein |
| 659 | 206201_s_at | NM_005924 | MEOX2 | mesenchyme homeo box 2 |
| 660 | 206276_at | NM_003695 | E48 | lymphocyte antigen 6 comp locus D |
| 661 | 206315_at | NM_004750 | CRLF1 | cytokine receptor-like factor 1 |
| 662 | 206363_at | NM_005360 | MAF | v-maf musculoaponeurotic fibrosarcoma oncogene homolog |
| 663 | 206385_s_at | NM_020987 | ANK3 | ankyrin 3, node of Ranvier, tran var 1 |
| 664 | 206400_at | NM_002307 | LGALS7 | lectin, galactoside-binding, soluble, 7 |
| 665 | 206453_s_at | NM_016250 | NDRG2 | N-myc downstream-regulated gene 2 |
| 666 | 206481_s_at | NM_001290 | LDB2 | LIM domain binding 2 |
| 667 | 206482_at | NM_005975 | PTK6 | PTK6 protein tyrosine kinase 6 |
| 668 | 206515_at | NM_000896 | CYP4F3 | cyt P450, subfam IVF, polypeptide 3 |
| 669 | 206539_s_at | NM_023944 | CYP4F12 | cytochrome P450 isoform 4F12 |
| 670 | 206581_at | NM_001717 | BNC | basonuclin |
| 671 | 206637_at | NM_014879 | KIAA0001 | KIAA0001 gene product |
| 672 | 206655_s_at | NM_000407 | GP1BB | glycoprotein Ib (platelet), β polypep |
| 673 | 206693_at | NM_000880 | IL7 | interleukin 7 |
| 674 | 206884_s_at | NM_003843 | SCEL | sciellin |
| 675 | 207002_s_at | NM_002656 | PLAGL1 | pleiomorphic adenoma gene-like 1 |
| 676 | 207023_x_at | NM_000421 | KRT10 | keratin 10 |
| 677 | 207076_s_at | NM_000050 | ASS | argininosuccinate synthetase |
| 678 | 207121_s_at | NM_002748 | MAPK6 | mitogen-activated protein kinase 6 |
| 679 | 207655_s_at | NM_013314 | SLP65 | B cell linker protein |
| 680 | 207720_at | NM_000427 | LOR | loricrin |
| 681 | 207761_s_at | NM_014033 | | DKFZP586A0522 protein |
| 682 | 207843_x_at | NM_001914 | CYB5 | cytochrome b-5 |
| 683 | 207908_at | NM_000423 | KRT2A | keratin 2A |
| 684 | 207943_x_at | NM_006718 | PLAGL1 | pleiomorphic adenoma gene-like transcript variant 2 |
| 685 | 207955_at | NM_006664 | SCYA27 | small inducible cytokine subfamily A (Cys-Cys), member 27 |
| 686 | 207996_s_at | NM 004338 | C18ORF1 | chrom 18 open reading frame 1 |
| 687 | 208096_s_at | NM 030820 | | hypothetical protein DKFZp564B052 |
| 688 | 208146_s_at | NM_031311 | LOC54504 | serine carboxypeptidase vitellogenic-like |
| 689 | 208161_s_at | NM 020037 | ABCC3 | ATP-binding cassette sub-fam C mem 3 |
| 690 | 208190_s_at | NM_015925 | LISCH7 | liver-specific bHLH-Zip transcription factor |
| 691 | 208228_s_at | M87771 | K-sam-III | secreted FGF receptor |
| 692 | 208609_s_at | NM 019105 | TNXB | tenascin XB |
| 693 | 208614_s_at | M62994 | | thyroid autoantigen |
| 694 | 208651_x_at | M58664 | | CD24 signal transducer |
| 695 | 208690_s_at | BC000915 | | Similar to LIM protein, |
| 696 | 208798_x_at | AF204231 | GM88 | 88-kDa Golgi protein |
| 697 | 209047_at | AL518391 | | aquaporin 1 |
| 698 | 209159_s_at | AV724216 | | NDRG family, member 4 |
| 699 | 209160_at | AB018580 | hluPGFS | aldo-keto reductase family 1, mem C3 |
| 700 | 209211_at | AF132818 | CKLF | colon Kruppel-like factor |
| 701 | 209212_s_at | AB030824 | | transcription factor BTEB2 |
| 702 | 209289_at | AI700518 | | nuclear factor IB |
| 703 | 209290_s_at | BC001283 | | Similar to nuclear factor IB, |
| 704 | 209309_at | D90427 | | zinc-alpha2-glycoprotein |
| 705 | 209318_x_at | BG547855 | | pleiomorphic adenoma gene-like 1 |
| 706 | 209335_at | AI281593 | | decorin |
| 707 | 209348_s_at | AF055376 | c-maf | short form transcription factor C-MAF |
| 708 | 209351_at | BC002690 | | keratin 14 |
| 709 | 209357_at | AF109161 | MRG1 | p35srj |
| 710 | 209366_x_at | M22865 | | cytochrome b5 |
| 711 | 209368_at | AF233336 | EPHX2 | soluble epoxide hydrolase |
| 712 | 209386_at | A1346835 | | transmembrane 4 superfam mem 1 |
| 713 | 209392_at | L35594 | autotaxin | ectonucleotide pyrophosphatasephosphodiesterase 2 |
| 714 | 209465_x_at | AL565812 | | pleiotrophin |
| 715 | 209493_at | AF338650 | AIPC | PDZ domain-containing protein AIPC |
| 716 | 209540_at | NM 000618 | somatomedin C | insulin-like growth factor 1 |
| 717 | 209550_at | U35139 | | NECDIN related protein |
| 718 | 209558_s_at | AB013384 | HIP1R | huntingtin interacting protein-1-related |
| 719 | 209590_at | AL157414 | | clone RP11-560A15 on chrom 20 |
| 720 | 209602_s_at | AI796169 | | GATA-binding protein 3 |
| 721 | 209603_at | AI796169 | | GATA-binding protein 3 |
| 722 | 209604_s_at | BC003070 | | GATA-binding protein 3, clone MGC:2346 |
| 723 | 209605_at | D87292 | rhodanese | thiosulfate sulfurtransferase |
| 724 | 209656_s_at | AL136550 | | DKFZp761J17121 |
| 725 | 209679_s_at | BC003379 | | hyp protein from clone 643, clone MGC:5115 |
| 726 | 209684_at | AL136924 | | DKFZp586G2120 |
| 727 | 209687_at | U19495 | hIRH | intercrine-alpha |
| 728 | 209691_s_at | BC003541 | FLJ10488 | hypothetical protein FLJ10488 |
| 729 | 209699_x_at | U05598 | | dihydrodiol dehydrogenase |
| 730 | 209732_at | NM 005127 | | Sim to C-type lectin, superfam mem 2 |
| 731 | 209763_at | AL049176 | | clone 141 H5 on chrom Xq22.1-23 |
| 732 | 209771_x_at | AA761181 | | CD24 antigen |
| 733 | 209863_s_at | AF091627 | | CUSP |
| 734 | 209866_s_at | R50822 | | KIAA0768 protein |
| 735 | 209975_at | AF182276 | CYP2E1 | cytochrome P450-2E1 |
| 736 | 210059_s_at | BC000433 | | mitogen-activated protein kinase 13 |
| 737 | 210096_at | J02871 | | lung cytochrome P450 BI |
| 738 | 210128_s_at | U41070 | | P2 purinergic receptor |
| 739 | 210298_x_at | AF098518 | FHL1 | 4 and ½ LIM domains 1 protein isoform B |
| 740 | 210347_s_at | AF080216 | | C2H2-type zinc-finger protein |
| 741 | 210372_s_at | AF208012 | TPD52L2 | tumor protein D52-like 2 |
| 742 | 210397_at | U73945 | | beta-defensin-1 |
| 743 | 210619_s_at | AF173154 | HYAL1 | hyaluronoglucosaminidase 1 isof 2 |
| 744 | 210633_x_at | M19156 | KRT10 | acidic keratin-10 |
| 745 | 210715_s_at | AF027205 | kop | Kunitz-type protease inhibitor |
| 746 | 210880_s_at | AB001467 | Efs2 | |
| 747 | 210958_s_at | BC003646 | | clone MGC:4693 |
| 748 | 211043_s_at | BC006332 | Lcb | clathrin, light polypeptide |
| 749 | 211105_s_at | U80918 | NF-ATcC | transcription factor |
| 750 | 211382_s_at | AF220152 | TACC2 | transforming, acidic coiled-coil containing protein 2 |
| 751 | 211458_s_at | AF180519 | | GABA-A receptor-associated |
| 752 | 211596_s_at | AB050468 | | membrane glycoprotein LIG-1 |
| 753 | 211597_s_at | AB059408 | SMAP31-12 | |
| 754 | 211653_x_at | M33376 | | pseudo-chlordecone reductase |
| 755 | 211712_s_at | BC005830 | | clone MGC:1925 |
| 756 | 211734_s_at | BC005912 | | Fc fragment IgE, high affinity I, rec for α polypep |
| 757 | 211841_s_at | U94510 | | lymphocyte associated receptor of death 9, alternatively spliced |
| 758 | 211986_at | BG287862 | desmoyokin | AHNAK nucleoprotein |
| 759 | 212148_at | BF967998 | | FLJ12900 fis, |
| 760 | 212204_at | AF132733 | | DKFZP564G2022 protein |
| 761 | 212242_at | AL565074 | | tubulin, alpha 1 (testis specific) |
| 762 | 212327_at | AK027231 | | FLJ23578 fis, KIAA1102 protein |
| 763 | 212328_at | AK027231 | | FLJ23578 fis, KIAA1102 protein |
| 764 | 212390_at | AB007923 | | KIAA0477 gene product |
| 765 | 212538_at | AL576253 | | KIAA1058 protein |
| 766 | 212543_at | U83115.1 | | non-lens β gamma-crystallin like |
| 767 | 212589_at | BG168858 | | oncogene TC21 |
| 768 | 212593_s_at | N92498 | | FLJ22071 fis, clone HEP11691 |
| 769 | 212724_at | BG054844 | | ras homolog gene family, member E |
| 770 | 212741_at | AA923354 | | monoamine oxidase A |
| 771 | 212823_s_at | AU147160 | | KIAA0599 protein |
| 772 | 212841_s_at | AI692180 | | PTPRF interacting protein, binding protein 2 |
| 773 | 212850_s_at | AA584297 | | low density lipoprotein receptor-related protein 4 |
| 774 | 212875_s_at | AP001745 | | chrom 21 open reading frame 25 |
| 775 | 212992_at | AI935123 | | ESTs |
| 776 | 213029_at | AL110126 | | DKFZp564H1916 |
| 777 | 213032_at | AL110126 | | DKFZp564H1916 |
| 778 | 213050_at | AA594937 | | KIAA0633 protein |
| 779 | 213068_at | AI146848 | | dermatopontin |
| 780 | 213071_at | AI146848 | | dermatopontin |
| 781 | 213106_at | AI769688 | | 23664 and 23905 mRNA sequence |
| 782 | 213110_s_at | AW052179 | | collagen, type IV, alpha 5 |
| 783 | 213122_at | AI096375 | | KIAA1750 protein, partial cds |
| 784 | 213135_at | U90902 | | clone 23612 |
| 785 | 213194_at | BF059159 | | Hs.301198 roundabout homolog |
| 786 | 213227_at | BE879873 | | progesterone membrane binding |
| 787 | 213280_at | AK000478 | | FLJ20471 fis |
| 788 | 213285_at | AV691491 | | DKFZp564D1462 |
| 789 | 213287_s_at | X14487 | | acidic (type I) cytokeratin 10 |
| 790 | 213353_at | BF693921 | | ATP-binding cassette, sub-family A, member 5 |
| 791 | 213359_at | W74620 | | heterogeneous nuclear ribonucleoprotein D |
| 792 | 213369_at | AI825832 | | DKFZp434A132 |
| 793 | 213375_s_at | N80918 | | Novel gene mapping to chomo 13 |
| 794 | 213397_x_at | AI761728 | | DnaJ homolog, subfam C, mem 8 |
| 795 | 213451_x_at | BE044614 | | tenascin XB |
| 796 | 213456_at | AI927000 | | DKFZP564D206 |
| 797 | 213506_at | BE965369 | | proteinase activated receptor-2 |
| 798 | 213556_at | BE673445 | | chromosome 19, cosmid R28379 |
| 799 | 213618_at | AB011152 | | KIAA0580 |
| 800 | 213695_at | L48516 | PON3 | paraoxonase 3 |
| 801 | 213707_s_at | NM_005221 | DLX5 | distal-less homeo box 5 |
| 802 | 213725_x_at | AI693140 | | DKFZp586F071 |
| 803 | 213737_x_at | AI620911 | | |
| 804 | 213800_at | X04697 | | complement factor H 38-kDa N-term |
| 805 | 213817_at | AL049435 | | DKFZp586B0220 |
| 806 | 213820_s_at | T54159 | | hypothetical protein MGC10327 |
| 807 | 213844_at | NM_019102 | HOXA5 | homeo box A5 |
| 808 | 213848_at | AI655015 | | DKFZp586F2224 |
| 809 | 213891_s_at | AI927067 | | FLJ11918 fis |
| 810 | 213924_at | BF476502 | | hypothetical protein FLJ11585 |
| 811 | 213929_at | AL050204 | | DKFZp586F1223 |
| 812 | 213933_at | AW242315 | | DKFZp586M0723 |
| 813 | 213935_at | AF007132 | | clone 23551 |
| 814 | 213942_at | AL134303 | | DKFZp547K034 r1 |
| 815 | 213992_at | AI889941 | | collagen, type IV, alpha 6 |
| 816 | 213994_s_at | AI885290 | | spondin 1, extracellular matrix |
| 817 | 214058_at | M19720 | | L-myc protein |
| 818 | 214132_at | BG232034 | | ATP synthase, H+ transporting, mitochondrial F1 complex, gamma polypeptide 1 |
| 819 | 214164_x_at | BF752277 | | adaptor-related protein complex 1, gamma 1 subunit |
| 820 | 214234_s_at | X90579 | | cyp related pseudogene |
| 821 | 214235_at | X90579 | | cyp related pseudogene |
| 822 | 214247_s_at | AU148057 | | regulated in glioma |
| 823 | 214598_at | AL049977 | | DKFZp564C122 |
| 824 | 214696_at | AF070569 | | clone 24659 |
| 825 | 214753_at | AW084068 | | BRCA2 region |
| 826 | 214823_at | AF033199 | | C2H2 zinc finger protein pseudogene |
| 827 | 215034_s_at | AI189753 | | FLJ13302 fis |
| 828 | 215062_at | AL390143 | | DKFZp547N074 |
| 829 | 215129_at | AJ000008 | | C2 domain containing PI3-kinase |
| 830 | 215239_x_at | AU132789 | | zinc finger protein 273 |
| 831 | 215243_s_at | AF099730 | GJB3 | connexin 31 |
| 832 | 215388_s_at | X56210 | FHR-1 | complement Factor H-related 1 |
| 833 | 215513_at | AF241534 | HYMAI | hydatidiform mole assoc & imprinted |
| 834 | 215516_at | AC005048 | | BAC clone CTB-15P3 fr 7q22-q31.2 |
| 835 | 215536_at | | | DMA, DMB, HLA-Z1, IPP2, LMP2, TAP1, LMP7, TAP2, DOB, DQB2 and RING8, 9, 13 and 14 genes |
| 836 | 215659_at | AK025174 | | FLJ21521 |
| 837 | 215704_at | AL356504 | | clone RP1-14N1 chrom 1q21.1-21.3 |
| 838 | 215726_s_at | M22976 | | cytochrome b5 |
| 839 | 215867_x_at | AL050025 | | DKFZp564D066 |
| 840 | 216199_s_at | AL109942 | | clone RP3-473J16 chrom 6q25.3-26 |
| 841 | 216268_s_at | U77914 | | soluble protein Jagged |
| 842 | 216333_x_at | M25813 | | unidentified gene complementary to P450c21 |
| 843 | 216379_x_at | AK000168 | | CD24 signal transducer |
| 844 | 216594_x_at | S68290 | | chlordecone reductase homolog |
| 845 | 216699_s_at | L10038 | | pre-pro-protein for kallikrein |
| 846 | 217087_at | AF005081 | xp32 | skin-specific protein |
| 847 | 217528_at | BF003134 | | ESTs |
| 848 | 217707_x_at | AI535683 | | ESTs |
| 849 | 217901_at | BF031829 | | desmoglein 2 |
| 850 | 217961_at | NM_017875 | FLJ20551 | hypothetical protein FLJ20551 |
| 851 | 218002_s_at | NM_004887 | SCYB14 | small inducible cytokine subfamily B (Cys-X-Cys), member 14 |
| 852 | 218170_at | NM_016048 | LOC51015 | CGI-111 protein |
| 853 | 218180_s_at | NM_022772 | FLJ21935 | hypothetical protein FLJ21935 |
| 854 | 218186_at | NM_020387 | CATX-8 | CATX-8 protein |
| 855 | 218237_s_at | NM_030674 | ATA1 | amino acid transporter system A1 |
| 856 | 218326_s_at | NM_018490 | GPR48 | G protein-coupled receptor 48 |
| 857 | 218434_s_at | NM_023928 | | hypothetical protein FLJ12389 |
| 858 | 218451_at | NM_022842 | | hypothetical protein FLJ22969 |
| 859 | 218499_at | NM_016542 | LOC51765 | serinethreonine protein kinase MASK |
| 860 | 218546_at | NM_024709 | | hypothetical protein FLJ14146 |
| 861 | 218552_at | NM_018281 | | hypothetical protein FLJ10948 |
| 862 | 218603_at | NM_016217 | LOC51696 | hHDC for homolog of Dros headcase |
| 863 | 218644_at | NM_016445 | PLEK2 | pleckstrin 2 (mouse) homolog |
| 864 | 218651_s_at | NM_018357 | | hypothetical protein FLJ11196 |
| 865 | 218657_at | NM_016339 | LOC51195 | Link guanine nucleotide exchange factor II |
| 866 | 218675_at | NM_020372 | LOC57100 | organic cation transporter |
| 867 | 218677_at | NM_020672 | LOC57402 | S100-type calcium binding protein A14 |
| 868 | 218692_at | NM_017786 | | hypothetical protein FLJ20366 |
| 869 | 218704_at | NM_017763 | | hypothetical protein FLJ20315 |
| 870 | 218718_at | NM_016205 | PDGFC | platelet derived growth factor C |
| 871 | 218736_s_at | NM_017734 | | hypothetical protein FLJ20271 |
| 872 | 218751_s_at | NM_018315 | | hypothetical protein FLJ11071 |
| 873 | 218764_at | NM_024064 | | hypothetical protein MGC5363 |
| 874 | 218792_s_at | NM_017688 | | hypothetical protein FLJ20150 |
| 875 | 218796_at | NM_017671 | | hypothetical protein FLJ20116 |
| 876 | 218804_at | NM_018043 | | hypothetical protein FLJ10261 |
| 877 | 218806_s_at | AF118887 | VAV-3 | VAV-3 protein |
| 878 | 218807_at | NM_006113 | VAV3 | vav 3 oncogene |
| 879 | 218816_at | NM_018214 | | hypothetical protein FLJ10775 |
| 880 | 218820_at | NM_020215 | | hypothetical protein DKFZp761F2014 |
| 881 | 218849_s_at | NM_006663 | RAI | ReIA-associated inhibitor |
| 882 | 218854_at | NM_013352 | SART-2 | squamous cell carcinoma antigen recog by T cell |
| 883 | 218901_at | NM_020353 | LOC57088 | phospholipid scramblase 4 |
| 884 | 218919_at | NM_024699 | | hypothetical protein FLJ14007 |
| 885 | 218963_s_at | NM_015515 | | DKFZP434G032 protein |
| 886 | 219010_at | NM_018265 | | hypothetical protein FLJ10901 |
| 887 | 219054_at | NM_024563 | | hypothetical protein FLJ14054 |
| 888 | 219064_at | NM_030569 | | hypothetical protein MGC10848 |
| 889 | 219073_s_at | NM_017784 | | hypothetical protein FLJ20363 |
| 890 | 219090_at | NM_020689 | NCKX3 | sodium calcium exchanger |
| 891 | 219093_at | NM_017933 | | hypothetical protein FLJ20701 |
| 892 | 219095_at | NM_005090 | PLA2G4B | phospholipase A2, group IVB |
| 893 | 219109_at | NM_024532 | | hypothetical protein FLJ22724 |
| 894 | 219115_s_at | NM_014432 | IL20RA | interleukin 20 receptor, alpha |
| 895 | 219229_at | NM_013272 | SLC21A11 | solute carrier family 21, member 11 |
| 896 | 219232_s_at | NM_022073 | | hypothetical protein FLJ21620 |
| 897 | 219263_at | NM_024539 | | hypothetical protein FLJ23516 |
| 898 | 219298_at | NM_024693 | | hypothetical protein FLJ20909 |
| 899 | 219313_at | NM_017577 | | hypothetical protein DKFZp434C0328 |
| 900 | 219368_at | NM_021963 | NAP1L2 | nucleosome assembly protein 1-like 2 |
| 901 | 219388_at | NM_024915 | | hypothetical protein FLJ13782 |
| 902 | 219395_at | NM_024939 | | hypothetical protein FLJ21918 |
| 903 | 219410_at | NM_018004 | | hypothetical protein FLJ10134 |
| 904 | 219411_at | NM_024712 | | hypothetical protein FLJ13824 |
| 905 | 219423_x_at | NM_003790 | TNFRSF12 | TNF receptor superfamily, member 12 |
| 906 | 219436_s_at | NM_016242 | LOC51705 | endomucin-2 |
| 907 | 219461_at | AJ236915 | | pak5 protein |
| 908 | 219476_at | NM_024115 | | hypothetical protein MGC4309 |
| 909 | 219489_s_at | NM_017821 | | hypothetical protein FLJ20435 |
| 910 | 219497_s_at | NM_022893 | BCL11A | B-cell CLLlymphoma 11A |
| 911 | 219518_s_at | NM_025165 | | hypothetical protein FLJ22637 |
| 912 | 219528_s_at | NM_022898 | BCL11B | B-cell lymphomaleukaemia 11B |
| 913 | 219532_at | NM_022726 | ELOVL4 | Stargardt disease 3 |
| 914 | 219597_s_at | NM_017434 | DUOX1 | dual oxidase 1 |
| 915 | 219689_at | NM_020163 | LOC56920 | semaphorin sem2 |
| 916 | 219729_at | NM_016307 | PRX2 | paired related homeobox protein |
| 917 | 219764_at | NM_007197 | FZD10 | frizzled (Drosophila) homolog 10 |
| 918 | 219806_s_at | NM_020179 | FN5 | FN5 protein |
| 919 | 219825_at | NM_019885 | P450RAI-2 | cyt P450 retinoid metabolizing |
| 920 | 219908_at | NM_014421 | DKK2 | dickkopf homolog 2 |
| 921 | 219936_s_at | NM_023915 | GPR87 | G protein-coupled receptor 87 |
| 922 | 219938_s_at | NM_024430 | PSTPIP2 | proline-serine-threonine phosphatase interacting protein 2 |
| 923 | 219970_at | NM_017655 | | hypothetical protein FLJ20075 |
| 924 | 219976_at | NM_015888 | HOOK1 | hook1 protein |
| 925 | 219995_s_at | NM_024702 | | hypothetical protein FLJ13841 |
| 926 | 219998_at | NM_014181 | | HSPC159 protein |
| 927 | 220016_at | NM_024060 | | hypothetical protein MGC5395 |
| 928 | 220056_at | NM_021258 | IL22R | interleukin 22 receptor |
| 929 | 220066_at | NM_022162 | NOD2 | NOD2 protein |
| 930 | 220076_at | NM_019847 | ANKH | ankylosis, progressive homolog |
| 931 | 220161_s_at | NM_019114 | EHM2 | EHM2 gene |
| 932 | 220225_at | NM_016358 | IRX4 | iroquois homeobox protein 4 |
| 933 | 220230_s_at | NM_016229 | LOC51700 | cytochrome b5 reductase b5R.2 |
| 934 | 220262_s_at | NM_023932 | | hypothetical protein MGC2487 |
| 935 | 22026_s_at | NM_004235 | KLF4 | Kruppel-like factor 4 (gut) |
| 936 | 220289_s_at | NM_017977 | | hypothetical protein FLJ10040 |
| 937 | 220318_at | NM_017957 | FLJ20778 | epsin 3 |
| 938 | 220413_at | NM_014579 | ZIP2 | zinc transporter |
| 939 | 220414_at | NM_017422 | CLSP | calmodulin-like skin protein |
| 940 | 220428_at | NM_015717 | LANGERIN | Langerhans cell specific c-type lectin |
| 941 | 220432_s_at | NM_016593 | CYP39A1 | oxysterol 7alpha-hydroxylase |
| 942 | 220518_at | NM_024801 | | hypothetical protein FLJ21551 |
| 943 | 220625_s_at | AF115403 | | Ets transcription factor ESE-2b |
| 944 | 220723_s_at | NM_025087 | | hypothetical protein FLJ21511 |
| 945 | 220724_at | NM_025087 | | hypothetical protein FLJ21511 |
| 946 | 220911_s_at | NM_025081 | | KIAA1305 protein |
| 947 | 220945_x_at | NM_018050 | | hypothetical protein FLJ10298 |
| 948 | 221127_s_at | NM_006394 | RIG | regulated in glioma |
| 949 | 221215_s_at | NM_020639 | ANKRD3 | ankyrin repeat domain 3 |
| 950 | 221541_at | AL136861 | | DKFZp434B044 |
| 951 | 221667_s_at | AF133207 | | protein kinase H11 |
| 952 | 221747_at | AL046979 | | DKFZp586K0617 |
| 953 | 221748_s_at | AL046979 | | DKFZp586K0617 |
| 954 | 221760_at | BG287153 | | mannosidase, a, class 1A, member 1 |
| 955 | 221796_at | AA707199 | | Similar to hyp protein FLJ20093 |
| 956 | 221841_s_at | BF514079 | | Kruppel-like factor 4 (gut) |
| 957 | 221854_at | AI378979 | | ESTs |
| 958 | 221922_at | AW195581 | | KIAA0761 |
| 959 | 221950_at | AI478455 | | empty spiracles homolog 2 |
| 960 | 222043_at | AI982754 | | clusterin |
| 961 | 222102_at | NM_000847 | GSTA3 | glutathione S-transferase A3 |
| 962 | 222236_s_at | AK000253 | | FLJ20246 fis |
| 963 | 222256_s_at | AK000550 | | FLJ20543 fis |
| 964 | 222288_at | AI004009 | | ESTs |
| 965 | 222290_at | AA731709 | | ESTs |
| 966 | 222303_at | AV700891 | | ESTs |
| 967 | 266_s_at | L33930 | | CD24 signal transducer |
| 968 | 33322_i_at | X57348 | | clone 9112 |
| 969 | 33323_r_at | X57348 | | clone 9112 |
| 970 | 35666_at | U38276 | | semaphorin III family homolog |
| 971 | 38340_at | AB014555 | | KIAA0655 protein |
| 972 | 39248_at | N74607 | | za55a01.s1 |
| 973 | 40016_g_at | AB002301 | | KIAA0303 gene |
| 974 | 40093_at | X83425 | | LU gene Lutheran blood group glycoprotein |
| 975 | 40472_at | AF007155 | | clone 23763 unknown mRNA |
| 976 | 57588_at | R62432 | | yg52e11.s1 |
| 977 | 60474_at | AA469071 | | ne17f11.s1 |
| 978 | 91826_at | AI219073 | | qg16e08.x1 |
| 979 | | | PBGD | |
| 980 | | | MART1 | |
| 981 | | | Me20m | |
| 982 | | | MAGE-3 | |
| 983 | | | | Me20m forward primer |
| 984 | | | | Me20m reverse primer |
| 985 | | | | Me20m probe |
| 986 | | | | PBGD forward primer |
| 987 | | | | PBGD reverse primer |
| 988 | | | | PBGD probe |
| 999 | | | | Tyrosinase |
| 1000 | | | | Tyrosinase Forward |
| 1001 | | | | Tyrosinase Reverse |
| 1002 | | | | Tyrosinase probe |
| 1003 | | | | MART1 Forward |
| 1004 | | | | MART1 Reverse |
| 1005 | | | | MART1 Probe |
| 1006 | | | | HMB45 Forward |
| 1007 | | | | gp100 Reverse |
| 1008 | | | | gp100 Probe |
| 1009 | | | | PLAB Forward |
| 1010 | | | | PLAB Reverse |
| 1011 | | | | PLAB Probe |

### References Cited

WO 96/29430
2003/0232356
2002/0160382
2002/0155480
2002/0110820
2002/0098535
2003/0059431
2003/0049701
2002/0048784
2003/014283
2003/0013097
6,500,919
4,715,545
5,210,015
5,348,856
5,411,876
5,413,924
5,487,972
5,512,437
5,512,444
5,525,494
5,550,044
5,612,201
5,759,783
5,804,375
5,844,075
5,844,092
5,872,225
5,910,574
5,969,124
5,985,619
5,994,102
6,025,474
6,057,105
6,107,476
6,153,388
6,235,525
6,291,430
6,326,145
6,338,947
6,369,211
6,403,341
6,426,217
6,465,181
6,475,727
6,500,638
6,521,227
6,527,560
6,599,699

Abrahamsen et al. (2004) Quantification of melanoma mRNA markers in sentinel nodes: pre-clinical evaluation of a single-step real-time reverse transcriptase-polymerase chain reaction assay J Molec Diag 6:253-259
Abrahamsen et al. (2005) Pathologic assessment of melanoma sentinel nodes: a role for molecular analysis using quantitative real-time reverse transcription-PCR for MART1 and tyrosinase messenger RNA Clin Cancer Res 11:1425-1433
Adema et al. (1994) Molecular characterization of the melanocyte lineage specific antigen gp100 J Biol Chem 69:20126-20133
Ahmed et al. (2004) Cdc42-dependent nuclear translocation of non-receptor tyrosine kinase, ACK Biochem. Biophys. Res. Commun. 314:571-579
Aitken et al. (2004) Population screening for melanoma: current evidence and a community based randomized trial. In "Textbook of Melanoma" Ed. by JF Thompson, DL Morton and BBR Kroon. Martin and Dunitz
Akula et al. (2004) Raf promotes human herpesvirus-8 (HHV-8/KSHV) infection Oncogene 23:5227-5241
Alexa et al. (2004) Contribution of distinct structural elements to activation of calpain by Ca2+ ions J. Biol. Chem. 279:20118-20126
Altznauer et al. (2004) Calpain-1 regulates Bax and subsequent Smac-dependent caspase-3 activation in neutrophil apoptosis J. Biol. Chem. 279:5947-5957
Antonescu et al. (2002) Molecular diagnosis of clear cell sarcoma: detection of EWS-ATF1 and MITF-M transcripts and histopathological and ultrastructural analysis of 12 cases J. Mol. Diag. 4:44-52
Arozarena et al. (2004) Activation of H-Ras in the endoplasmic reticulum by the RasGRF family guanine nucleotide exchange factors Mol. Cell. Biol. 24:1516-1530
Bae et al. (2003) Gene expression patterns as potential molecular biomarkers for malignant transformation in human keratinocytes treated with MNNG, arsenic or a metal mixture Toxicol Sci 74:32-42
Balch et al. (2001) Final version of the American Joint Committee on cancer staging system for cutaneous melanoma J Clin Oncol 19:3635-3648
Baldi et al. (2003) Identification of genes down-regulated during melanoma progression: a cDNA array study Exp Dermatol 12:213-218
Barac et al. (2004) Direct interaction of p21-activated kinase 4 with PDZ-RhoGEF, a G protein-linked Rho guanine exchange factor J. Biol. Chem. 279:6182-6189
Bendotti et al. (2004) Activated p38MAPK is a novel component of the intracellular inclusions found in human amyotrophic lateral sclerosis and mutant SOD1 transgenic mice J. Neuropathol. Exp. Neurol. 63:113-119
Bittner et al. (2000) Molecular classification of cutaneous malignant melanoma by gene expression profiling Nature 406:536-540
Bostick et al. (1999) Prognostic significance of occult metastases detected by sentinel lymphadenectomy and reverse transcriptase-polymerase chain reaction in early-stage melanoma patients J. Clin. Oncol. 17:3238-3244
Brown et al. (2003) MIC-1 Serum Level and Genotype: Associations with Progress and Prognosis of Colorectal Carcinoma Clin. Cancer Res. 9:2642-2650
Buckhaults et al. (2001) Secreted and cell surface genes expressed in benign and malignant colorectal tumors Cancer Res. 61:6996-7001
Burzio et al. (2002) Biochemical and cellular characteristics of the four splice variants of protein kinase CK1alpha from zebrafish (Danio rerio) J. Cell. Biochem. 86:805-814
Buchs et al. (2004) Normal p21Ras/MAP kinase pathway expression and function in PBMC from patients with polycystic ovary disease Int. J. Mol. Med. 13:595-599
Cancer Facts and Figures 2003. American Cancer Society, 2003.
Care et al. (1996) HOXB7 constitutively activates basic fibroblast growth factor in melanomas Mol. Cell. Biol. 16:4842-4851
Carlson et al. (2003) Biomarkers in melanoma: staging, prognosis and detection of early metastasis Expert Rev Mol Diagn 3:89-116
Catala et al. (2002) Genetic control of caudal development Clin. Genet. 61:89-96
Chen et al. (2003) Molecular characterization of human ninein protein: two distinct subdomains required for centrosomal targeting and regulating signals in cell cycle Biochem. Biophys. Res. Commun. 308:975-983
Karan et al. (2003) Dysregulated expression of MIC-1/PDF in human prostate tumor cells Biochim. Biophys. Res. Commun. 305:598-604
Chen et al. (2004) MEK1,2 response element mediates angiotensin II-stimulated plasminogen activator inhibitor-1 promoter activation Blood 103:2636-2644
Chen et al. (2004) Cdc42 and the actin-related protein/neural Wiskott-Aldrich syndrome protein network mediate cellular invasion by Cryptosporidium parvum Infect. Immun. 72:3011-3021
Cheung et al. (2001) Detection of microscopic disease: comparing histology, immunocytology, and RT-PCR of tyrosine hydroxylase, GAGE, and MAGE Med. Ped. Oncol. 36:210-212
Ching et al. (2003) Identification of an autoinhibitory domain of p21-activated protein kinase 5 J. Biol. Chem. 278:33621-33624
Clark et al. (2003) The secreted protein discovery initiative (SPDI), a large-scale effort to identify novel human secreted and transmembrane proteins: a bioinformatics assessment Genome Res. 13:2265-2270
Cochran et al. (2001) Current practice and future directions in pathology and laboratory evaluation of the sentinel node Ann Surg Oncol. 8(9 Suppl):13S-17S
Cochran et al. (2004) Optimized assessment of sentinel lymph nodes for metastatic melanoma: implications for regional surgery and overall treatment planning Ann. Surg. Oncol. 11:156S-161S
Cook et al (2003) The development of optimal pathological assessment of sentinel lymph nodes for melanoma J. Pathol. 200:314-319
Cronin et al. (2004) Measurement of gene expression in archival paraffin-embedded tissues: development and performance of a 92-gene reverse transcriptase-polymerase chain reaction assay Am J Pathol 164:35-42
Davids et al. (2003) Accurate molecular detection of melanoma nodal metastases: an assessment of multimarker assay specificity, sensitivity, and detection rate Mol. Pathol. 56:43-51
de Braud et al. (2003) Malignant melanoma Critical Review In Oncology/Hematology 47:35-63
Diego et al. (2003) Prognostic Significance of Reverse Transcriptase-Polymerase Chain Reaction-Negative Sentinel Nodes in Malignant Melanoma Ann. Surg. Oncol. 10:396-402
Ducy et al. (2000) The family of bone morphogenetic proteins Kidney Int. 57:2207-2214
Dwivedi et al. (2004) Protein kinase A in postmortem brain of depressed suicide victims: altered expression of specific regulatory and catalytic subunits Biol. Psychiatry 55:234-243
Edgar et al. (2002) Cloning and tissue distribution of three murine alpha/beta hydrolase fold protein cDNAs Biophys. Biochim. Res. Comm. 292:617-625
Encinas et al. (2004) Tyrosine 981, a novel ret autophosphorylation site, binds c-Src to mediate neuronal survival J. Biol. Chem. 279:18262-18269
Esler et al. (2001) A portrait of Alzheimer secretases - new features and familiar faces Science 293:1449-1454
Evellin et al. (2002) Stimulation of phospholipase C-epsilon by the M3 muscarinic acetylcholine receptor mediated by cyclic AMP and the GTPase Rap2B J. Biol. Chem. 277:16805-16813
Fenner et al. (1998) MSG1 (melanocyte-specific gene 1): mapping to chromosome Xq13.1, genomic organization, and promoter analysis Genomics 51:401-407
Fogel et al. (2003) L1 adhesion molecule (CD171) in development and progression of human malignant melanoma Cancer Letters 189:237-247
Fujii et al. (2004) Am. J. Physiol. Cell Physiol. 287:C200-C208
Gangemi et al. (2004) Regulatory genes controlling cell fate choice in embryonic and adult neural stem cells J Neurochem 89:286-306
Giancotti et al. (1999) Integrin signaling Science 285:1028-1032
Golubovskaya et al. (2004) Cloning and characterization of the promoter region of human focal adhesion kinase gene: nuclear factor kappa B and p53 binding sites Biochim. Biophys. Acta 1678:111-125
Gosmanov et al. (2004) Impaired expression and insulin-stimulated phosphorylation of Akt-2 in muscle of obese patients with atypical diabetes Am. J. Physiol. Endocrinol. Metab. 287:E8-E15
Goydos et al. (2003) Patterns of recurrence in patients with melanoma and histologically negative but RT-PCR-positive sentinel lymph nodes J. Am. Coll. Surg. 196:196-204
Grimsley et al. (2004) Dock180 and ELMO1 proteins cooperate to promote evolutionarily conserved Rac-dependent cell migration J. Biol. Chem. 279:6087-6097
Gronholm et al. (2003) Merlin links to the cAMP neuronal signaling pathway by anchoring the RIbeta subunit of protein kinase A J. Biol. Chem. 278:41167-41172
Guan et al. (2003) ASC/TMS 1, a caspase-1 activating adaptor, is down regulated by aberrant methylation in human melanoma Int J Cancer 107:202-208
Gustafson et al. (2004) Bcr-Abl Regulates Protein Kinase Ci (PKCi) Transcription via an Elk1 Site in the PKCi Promoter J. Biol. Chem. 279:9400-9408
Gutzmer et al. (2002) Specificity of tyrosinase and HMB45 PCR in the detection of melanoma metastases in sentinel lymph node biopsies Histopathol 41:510-518
Haendeler et al. (2003) GIT1 mediates Src-dependent activation of phospholipase Cgamma by angiotensin II and epidermal growth factor J. Biol. Chem. 278:49936-49944
Hafner et al. (2003) Loss of EphB6 expression in metastatic melanoma Int. J. Oncol. 23:1553-1559
Haqq et al. (2005) The gene expression signature of melanoma progression Proc Natl Acad Sci USA 102:6092-6097
Haspel et al (2003) Front. Biosci. 8:S1210-S1225
Hendrix et al. (2002) Cancer Res 62:665-668
Henshall et al. (2003) Cancer Res. 63:4196-4203
Hilgard et al. (2004) Am. J. Physiol. Gastrointest. Liver Physiol. 287:G192-G201
Hipfel et al. (1998) RNA isolation from human skin tissues for colorimetric differential display J Biochem Biophys Method 37:131-135
Hipfel et al. (2000) Br. J. Cancer 82:1149-1157
Hiraga et al. (1997) Virchows Archiv. 431:45-51
Hisaoka et al. (2002) J. Pathol. 197:661-667
Hoek et al. (2004) Expression profiling reveals novel pathways in the transformation of melanocytes to melanomas Cancer Res 64:5270-5282
Hoon et al. (2001) J. Invest. Dermatol. 117:375-378
Hoon et al. (2004) Profiling epigenetic inactivation of tumor suppressor genes in tumor and plasma from cutaneous melanoma patients Oncogene 23:4014-4022
Izard et al. (2004) Nature 427:171-175
Jaffer et al. (2002) Int. J. Biochem. Cell Biol. 34:713-717
Kadaja et al. (2004) Oncogene 23:2523-2530
Kam et al. (2004) Neuropathol. Appl. Neurobiol. 30:225-232
Kamholz et al. (1996) Proc. Natl. Acad. Sci. USA 83:4962-4966
Kammula et al. (2004) Serial follow-up and prognostic significance of reverse transcriptase-polymerase chain reaction-staged sentinel lymph nodes from melanoma patients J Clin Oncol 22:3989-3996
Kaneda et al. (2002) Int. J. Cancer 100:57-62
Kikuchi et al. (2003) An N-terminal fragment of ProSAAS (a granin-like neuroendocrine peptide precursor) is associated with tau inclusions in Pick's disease Biochem. Biophys. Res. Commun. 308:646-654
Kirchhoff et al. (2004) J. Virol. 78:6864-6874
Kitano et al. (2003) Mol. Biol. Evol. 20:1281-1289
Kuo et al. (2003) Prediction of disease outcome in melanoma patients by molecular analysis of paraffin- embedded sentinel lymph nodes J Clin Oncol 21:3566-3572
Lee et al. (2004) J. Biol. Chem. 279:6834-6839
Lee et al. (2004) J. Neurosci. 24:2304-2312
Li et al. (1995) Proc. Natl. Acad. Sci. USA 92:121180-12184
Li et al. (2004) J. Biol. Chem. 279:15938-15945
Li et al. (2004) J. Biol. Chem. 279:20401-20410
Liu et al (2003) Cancer Res. 63:5034-5040
Liu et al. (1992) J. Biol. Chem. 267:15829-15835
Liu et al. (2003) Macrophage inhibitory cytokine 1 reduces cell adhesion and induces apoptosis in prostate cancer cells Cancer Res 63:5034-40
Luftner et al. (2003) Plasma levels of HER-2/neu, tumor type M2 pyruvate kinase and its tyrosine-phosphorylated metabolite in advanced breast cancer Anticancer Res. 23(2A):991-997
Luo et al. (2001) Human prostate cancer and benign prostatic hyperplasia: molecular dissection by gene expression profiling Cancer Res 61:4683-4688
Luthi et al. (2003) Exp. Hematol. 31:150-158
MacDougall et al. (2003) J. Clin. Endocrinol. Metab. 88:2194-2205
Majumder et al. (2004) Nat. Med. 10:594-601
Mandelcorn-Monson et al. (2003) J. Invest. Dermatol. 121:550-556
Marchetti et al. (2003) J. Cell. Biochem. 88:865-872
Maresh et al. (1994) DNA Cell. Biol. 13:87-95
Marlow et al. (2003) Biochem. Biophys. Res. Commun. 305:502-509
McGregor et al. (1999) Proc. Natl. Acad. Sci. USA 96:4540-4545
McMasters (2003) Molecular staging of melanoma: sensitivity, specificity and the search of clinical significance Ann Surg Oncol 10:336-337
Menez et al. (2004) Oncogene 23:2630-2639
Messina et al. (1999) Pathologic examination of the sentinel lymph node in malignant melanoma Am J Surg Pathol 23:686-690
Monaco (2004) Evidence regarding a role fro CDK5 dysregulation in Alzheimer's disease Curr Alzheimer Res 1:33-38
Morton et al. (1992) Technical details of intraoperative lymphatic mapping for early stage melanoma Arch Surg 127:392-399
Nakamura et al. (2003) Quantitative analysis of macrophage inhibitory cytokine-1 (MIC-1) gene expression in human prostatic tissues Br. J. Cancer 88:1101-1104
Nishihara et al. (2004) Cyclic AMP promotes cAMP-responsive element-binding protein-dependent induction of cellular inhibitor of apoptosis protein-2 and suppresses apoptosis of colon cancer cells through ERK1/2 and p38 MAPK J. Biol. Chem. 279:26176-26183
Nomura et al. (2004) Identification of a novel domain of Ras and Rap1 that directs their differential subcellular localizationsJ. Biol. Chem. 279:22664-22673
Oda et al. (2003) CrkL directs ASAP1 to peripheral focal adhesions J. Biol. Chem. 278:6456-6460
Okada et al. (2001) Genetic detection of lymph node micrometastases in patients with gastric carcinoma by multiple-marker reverse transcriptase-polymerase chain reaction assay Cancer 92:2056-2064
Okami et al. (2001) Genetic detection for micrometastasis in lymph node of biliary tract carcinoma Clin. Cancer Res. 6:2326-2332
Ooka et al. (2000) Selection of mRNA markers for detection of lymph node micrometastases in breast cancer patients Oncol. Rep. 7:561-566
Oury et al. (2004) ATP augments von Willebrand factor-dependent shear-induced platelet aggregation through Ca2+-calmodulin and myosin light chain kinase activation J. Biol. Chem. 279:26266-26273
Palmieri et al. (2001) Detection of occult melanoma cells in paraffin-embedded histologically negative sentinel lymph nodes using a reverse transcriptase polymerase chain reaction assay J. Clin. Oncol. 19:1437-1443
Paralkar et al. (1998) Cloning and characterization of a novel member of the transforming growth factor-beta/bone morphogenetic protein family J. Biol. Chem. 273:13760-13767
Pastorino et al. (2004) BACE (beta-secretase) modulates the processing of APLP2 in vivo Mol. Cell. Neurosci. 25:642-649
Patel et al. (2003) Cadherin switching in ovarian cancer progression Int. J. Cancer 106:172-177
Pitsi et al. (2004) Presenilin 1 stabilizes the C-terminal fragment of the amyloid precursor protein independently of gamma-secretase activity J. Biol. Chem. 279:25333-25338
Masters et al. (1985) Amyloid plaque core protein in Alzheimer disease and Down syndrome Proc. Natl. Acad. Sci. U.S.A. 82:4245-4249
Polyak et al. (1997) A model for p53-induced apoptosis Nature 389:300-305
Pontow et al. (2004) Actin cytoskeletal reorganizations and coreceptor-mediated activation of rac during human immunodeficiency virus-induced cell fusion J. Virol. 78:7138-7147
Prichard et al. (2003) The role of molecular staging in malignant melanoma Eur. J. Surg. Oncol. 29:306-314
Qi et al. (2004) α-Chimaerin exists in a functional complex with the Cdk5 kinase in brain FEBS Lett. 561:177-180
Qi et al. (2004) Essential Role of p38{gamma} in K-Ras Transformation Independent of Phosphorylation J. Biol. Chem. 279:22138-22144
Qin et al. (2004) p53-independent NOXA induction overcomes apoptotic resistance of malignant melanomas Mol Cancer Therap 3:895-902
Raas-Rothschild et al. (2002) A PEX6-defective peroxisomal biogenesis disorder with severe phenotype in an infant, versus mild phenotype resembling Usher syndrome in the affected parents Am. J. Hum. Genet. 70:1062-1068
Raich et al. (1986) Molecular cloning and complete primary sequence of human erythrocyte porphobilinogen deaminase Nucl. Acids Res. 14:5955-5968
Ray et al. (1997) AIM1, a novel non-lens member of the betagamma-crystallin superfamily, is associated with the control of tumorigenicity in human malignant melanoma Proc. Natl. Acad. Sci. USA 94:3229-3234
Reifenberger et al. (2004) Frequent alterations of Ras signaling pathway genes in sporadic malignant melanomas Int. J. Cancer 109:377-384
Reintgen et al. (2004) The staging of malignant melanoma and the Florida Melanoma Trial Ann. Surg. Oncol. 11(3Supp1):186S-191S
Rhee et al. (2004) MEK is a key modulator for TLR5-induced interleukin-8 and MIP3alpha gene expression in non-transformed human colonic epithelial cells J. Biol. Chem. 279:25179-25188
Riccioni et al. (2002) The sentinel lymph node in melanoma: utilization of molecular biology (RT-PCR) to detect occult metastases Pathologica 94:190-195
Rimboldi et al. (2003) Detection of micrometastasis in sentinel lymph nodes from melanoma patients: direct comparison of multimarker molecular and immunopathological methods Melanoma Res 13:511-520
Ring et al. (1998) Five SWI/SNF-related, matrix-associated, actin-dependent regulator of chromatin (SMARC) genes are dispersed in the human genome Genomics 51:140-143
Ryden et al. (1996) Expression of mRNA for the neurotrophin receptor trkC in neuroblastomas with favourable tumour stage and good prognosis Br. J. Cancer 74:773-779
Saito et al. (2004) Immunohistochemical diagnosis of a rare case of epithelioid malignant peripheral nerve sheath tumor with multiple metastases Jpn. J. Ophthalmol. 48:199-207
Sakwe et al. (2004) Involvement of protein kinase C-alpha and -epsilon in extracellular Ca(2+) signalling mediated by the calcium sensing receptor Exp. Cell Res. 297:560-573
Salazar-Onfray et al. (2002) Tissue distribution and differential expression of melanocortin 1 receptor, a malignant melanoma marker Br. J. Cancer 87:414-422
Salazar-Onfray et al. (2002) Tissue distribution and differential expression of melanocortin 1 receptor, a malignant melanoma marker Br J Cancer 87:414-422
Sasaki et al. (2004) BRAF point mutations in primary melanoma show different prevalences by subtype J. Invest. Dermatol. 123:177-183
Satyamoorthy et al. (2002) A versatile method for the removal of melanin from ribonucleic acids in melanocytic cells Mel. Res. 12:449-452
Schechtman et al. (2004) A critical intramolecular interaction for protein kinase Cepsilon translocation J. Biol. Chem. 279:15831-15840
Schmuth et al. (2004) The effect of LXR activators on AP-1 proteins in keratinocytes J. Invest. Dermatol. 123:41-48
Segal et al. (2003) Classification of clear-cell sarcoma as a subtype of melanoma by genomic profiling J. Clin. Oncol. 21:1775-1781
Sells et al. (1997) Human p21-activated kinase (Pak1) regulates actin organization in mammalian cells Curr. Biol. 7:202-210
Seykora et al. (2003) Gene expression profiling of melanocytic lesions Am J Dermatopathol 25 :6-11
Shimizu et al. (1999) High expression pf macrophage migration inhibitory factor in human melanoma cells and is role in tumor cell growth and angiogenesis Biochem Biophys Res Commun 264:751-758
Shirotani et al. (2004) Immature nicastrin stabilizes APH-1 independent of PEN-2 and presenilin: identification of nicastrin mutants that selectively interact with APH-1 J. Neurochem. 89:1520-1527
Shivers et al. (1998) Molecular staging of malignant melanoma: correlation with clinical outcome JAMA 280:1410-1415
Smith et al. (1997) Chromosomal localization of three human dual specificity phosphatase genes (DUSP4, DUSP6, and DUSP7) Genomics 42:524-7
Starz et al. (2003) Tyrosinase RT-PCR as a supplement to histology for detecting melanoma and nevus cells in paraffin sections of sentinel lymph nodes Mod Pathol 16:920-929
Stieber et al. (2003) Molecular basis for the different activation kinetics of the pacemaker channels HCN2 and HCN4 J. Biol. Chem. 278:33672-33680
Stoletov et al. (2004) Nck and Crk mediate distinct VEGF-induced signaling pathways that serve overlapping functions in focal adhesion turnover and integrin activation Exp. Cell Res. 295:258-268
Strausberg et al. (2002) Generation and initial analysis of more than 15,000 full-length human and mouse cDNA sequences Proc. Natl. Acad. Sci. USA 99:16899-16903
Su et al. (2001) Evaluation of acute hepatic failure treated at the Department of Medicine III, Kyushu University Hospital: indications for living-donor liver transplantation Cancer Res. 61:7388-7993
Suzuki et al. (2000) Power spectral analysis of R-R interval variability before and during the sinusoidal heart rate pattern in fetal lambs Int. J. Oncol. 15:1227-1232
Swiatek et al. (2004) Regulation of casein kinase I epsilon activity by Wnt signaling J. Biol. Chem. 279:13011-13017
Szebeni et al. (2003) Role of protein kinase CK2 phosphorylation in the molecular chaperone activity of nucleolar protein b23 J. Biol. Chem. 278:9107-9115
Taback et al. (2000) The clinical utility of multimarker RT-PCR in the detection of occult metastasis in patients with melanoma Recent Results Cancer Res 158:78-92
Takeuchi et al. (2004) Prognostic significance of molecular upstaging of paraffin-embedded sentinel lymph nodes in melanoma patients J Clin Oncol 22:2671-2680
The Washington University-Merck EST Project Hillier et al. (1995)
Thies et al. (2002) Overexpression of the cell adhesion molecule L1 is associated with metastasis in cutaneous malignant melanoma Eur J Cancer 38:1708-1716
Tokuo et al. (2004) Myosin X transports Mena/VASP to the tip of filopodia Biochem. Biophys. Res. Commun. 319:214-220
Tremuth et al. (2004) A fluorescence cell biology approach to map the second integrin-binding site of talin to a 130-amino acid sequence within the rod domain J. Biol. Chem. 279:22258-22266
Tsutsumi et al. (2003) Regulation of cell proliferation by autocrine motility factor/phosphoglucose isomerase signaling J. Biol. Chem. 278:32165-32172
Tusher et al. (2001) Significance analysis of microarrays applied to the ionizing radiation response Proc Natl Acad Sci USA 98:5116-5121
Van Impe et al. (2003) The Nucleo-cytoplasmic actin-binding protein CapG lacks a nuclear export sequence present in structurally related proteins J. Biol. Chem. 278:17945-17952
Vandesompele et al. (2002) Accurate normalization of real-time quantitative RT-PCR data by geometric averaging of multiple internal control genes Genome Biol 3:1-11
Wang et al. (2001) Optimal procedure for extracting RNA from human ocular tissues and expression profiling of the congenital glaucoma gene FOXCI using quantitative RT-PCR Mol. Vision 7:89-94
Wang et al. (2002) The third activity for lysyl hydroxylase 3: galactosylation of hydroxylysyl residues in collagens in vitro Matrix Biol. 21:559-566
Wang et al. (2004) Gene expression profiles and molecular markers to predict recurrence of Dukes' B colon cancer J. Clin Oncol 22:1564-1571
Weeraratna (2004) Generation and analysis of melanoma SAGE libraries: SAGE advice on the melanoma transcriptome Oncogene 23:2264-2274
Weiser et al. (2001) Induction of MAGE-3 expression in lung and esophageal cancer cells Ann. Thorac. Surg. 71:295-301
Welk et al. (2001) Identification and characterization of the gene encoding human cytoplasmic polyadenylation element binding protein Gene 263:113-20.
Welsh et al. (2003) Large-scale delineation of secreted protein biomarkers overexpressed in cancer tissue and serum Proc Natl Acad Sci USA 100:3410-3415
Wilson et al. (2000) An early requirement for FGF signaling in the acquisition of neural cell fate in the chick embryo Curr Bio 10:421-429
Woo et al. (2004) Resveratrol inhibits phorbol myristate acetate-induced matrix metalloproteinase-9 expression by inhibiting JNK and PKC delta signal transduction Oncogene 23:1845-1853
Wrightson et al. (2001) Reverse transcriptase-polymerase chain reaction (RT-PCR) analysis of nonsentinel nodes following completion lymphadenectomy for melanoma J. Surg. Res. 98:47-51
Xu et al. (2003) Transforming growth factor-betal stimulated protein kinase B serine-473 and focal adhesion kinase tyrosine phosphorylation dependent on cell adhesion in human hepatocellular carcinoma SMMC-7721 cells Biochem. Biophys. Res. Commun. 312:388-396
Xu et al. (2003) Expression of neurotrophin receptor Trk-C in nevi and melanomas J Cutan Pathol 30:318-322
Yamanaka et al. (1999) Expression of MAGE genes in renal cell carcinoma Int. J. Mol. Med. 2:57-60
Yamauchi et al. (2003) Neurotrophin 3 activation of TrkC induces Schwann cell migration through the c-Jun N-terminal kinase pathway Proc Natl Acad Sci USA 100:14421-14426
Yang et al. (2003) Macrophage inhibitory cytokine-1: a novel biomarker for p53 pathway activation Mol Cancer Therapy 2:1023-1029
Yannoni et al. (2004) P66ShcA interacts with MAPKAP kinase 2 and regulates its activity FEBS Lett. 564:205-211
Yoshimatsu et al. (1998) Expression of the melanoma antigen-encoding gene in human lung cancer J. Surg. Oncol. 67:126-129
Yu et al. (1999) Detection of microscopic melanoma metastases in sentinel lymph nodes Cancer 86:617-627
Yu et al. (2004) A minimal length between tau exon 10 and 11 is required for correct splicing of exon 10 J. Neurochem. 90:164-172
Zatti et al. (2004) The presenilin 2 M239I mutation associated with familial Alzheimer's disease reduces Ca2+ release from intracellular stores Neurobiol. Dis. 15:269-278
Zhang et al. (2003) Negative regulation of T cell antigen receptor-mediated Crk-L-C3G signaling and cell adhesion by Cbl-b J. Biol. Chem. 278:23978-23983
Zhang et al. (2004) Characterization of the cAMP-dependent protein kinase catalytic subunit Cgamma expressed and purified from sf9 cells Protein Expr. Purif. 35:156-169
Zhang et al. (2004a) Hsp90/p50cdc37 is required for mixed-lineage kinase (MLK) 3 signaling J. Biol. Chem. 279:19457-19463
Zhou et al. (2004) A novel crosstalk mechanism between nuclear receptor-mediated and growth factor/Ras-mediated pathways through PNRC-Grb2 interaction Oncogene 23:5394-5404

### SEQUENCE LISTING

<110> VERIDEX, LLC
   Wang, Yixin
   Talantov, Dimitri
   Mazumder, Abhij it
<120> METHODS AND REAGENT FOR THE DETECTION OF MELANOMA
<130> VDX5006WOPCT
<140> US 60/582,906
<141> 2004-06-25
<150> US 60/582,906
<151> 2004-06-25
<160> 1001
<170> PatentIn version 3.2
<210> 1
   <211> 1204
   <212> DNA
   <213> Homo sapiens
   <400> 1
   cggaacgagg gcaacctgca cagccatgcc cgggcaagaa ctcaggacgg tgaatggctc 60
   tcagatgctc ctggtgttgc tggtgctctc gtggctgccg catgggggcg ccctgtctct 120
   ggccgaggcg agccgcgcaa gtttcccggg accctcagag ttgcactccg aagactccag 180
   attccgagag ttgcggaaac gctacgagga cctgctaacc aggctgcggg ccaaccagag 240
   ctgggaagat tcgaacaccg acctcgtccc ggcccctgca gtccggatac tcacgccaga 300
   agtgcggctg ggatccggcg gccacctgca cctgcgtatc tctcgggccg cccttcccga 360
   ggggctcccc gaggcctccc gccttcaccg ggctctgttc cggctgtccc cgacggcgtc 420
   aaggtcgtgg gacgtgacac gaccgctgcg gcgtcagctc agccttgcaa gaccccaagc 480
   gcccgcgctg cacctgcgac tgtcgccgcc gccgtcgcag tcggaccaac tgctggcaga 540
   atcttcgtcc gcacggcccc agctggagtt gcacttgcgg ccgcaagccg ccagggggcg 600
   ccgcagagcg cgtgcgcgca acggggacga ctgtccgctc gggcccgggc gttgctgccg 660
   tctgcacacg gtccgcgcgt cgctggaaga cctgggctgg gccgattggg tgctgtcgcc 720
   acgggaggtg caagtgacca tgtgcatcgg cgcgtgcccg agccagttcc gggcggcaaa 780
   catgcacgcg cagatcaaga cgagcctgca ccgcctgaag cccgacacgg agccagcgcc 840
   ctgctgcgtg cccgccagct acaatcccat ggtgctcatt caaaagaccg acaccggggt 900
   gtcgctccag acctatgatg acttgttagc caaagactgc cactgcatat gagcagtcct 960
   ggtccttcca ctgtgcacct gcgcggggga ggcgacctca gttgtcctgc cctgtggaat 1020
   gggctcaagg ttcctgagac acccgattcc tgcccaaaca gctgtattta tataagtctg 1080
   ttatttatta ttaatttatt ggggtgacct tcttggggac tcgggggctg gtctgatgga 1140
   actgtgtatt tatttaaaac tctggtgata aaaataaagc tgtctgaact gttaaaaaaa 1200
   aaaa 1204
<210> 2
   <211> 4513
   <212> DNA
   <213> Homo sapiens
   <400> 2
   gcgcggtgcc gccgggaaag atggtcgtgg cgctgcggta cgtgtggcct ctcctcctct 60
   gcagcccctg cctgcttatc cagatccccg aggaatatga aggacaccat gtgatggagc 120
   cacctgtcat cacggaacag tctccacggc gcctggttgt cttccccaca gatgacatca 180
   gcctcaagtg tgaggccagt ggcaagcccg aagtgcagtt ccgctggacg agggatggtg 240
   tccacttcaa acccaaggaa gagctgggtg tgaccgtgta ccagtcgccc cactctggct 300
   ccttcaccat cacgggcaac aacagcaact ttgctcagag gttccagggc atctaccgct 360
   gctttgccag caataagctg ggcaccgcca tgtcccatga gatccggctc atggccgagg 420
   gtgcccccaa gtggccaaag gagacagtga agcccgtgga ggtggaggaa ggggagtcag 480
   tggttctgcc ttgcaaccct cccccaagtg cagagcctct ccggatctac tggatgaaca 540
   gcaagatctt gcacatcaag caggacgagc gggtgacgat gggccagaac ggcaacctct 600
   actttgccaa tgtgctcacc tccgacaacc actcagacta catctgccac gcccacttcc 660
   caggcaccag gaccatcatt cagaaggaac ccattgacct ccgggtcaag gccaccaaca 720
   gcatgattga caggaagccg cgcctgctct tccccaccaa ctccagcagc cacctggtgg 780
   ccttgcaggg gcagccattg gtcctggagt gcatcgccga gggctttccc acgcccacca 840
   tcaaatggct gcgccccagt ggccccatgc cagccgaccg tgtcacctac cagaaccaca 900
   acaagaccct gcagctgctg aaagtgggcg aggaggatga tggcgagtac cgctgcctgg 960
   ccgagaactc actgggcagt gcccggcatg cgtactatgt caccgtggag gctgccccgt 1020
   actggctgca caagccccag agccatctat atgggccagg agagactgcc cgcctggact 1080
   gccaagtcca gggcaggccc caaccagagg tcacctggag aatcaacggg atccctgtgg 1140
   aggagctggc caaagaccag aagtaccgga ttcagcgtgg cgccctgatc ctgagcaacg 1200
   tgcagcccag tgacacaatg gtgacccaat gtgaggcccg caaccggcac gggctcttgc 1260
   tggccaatgc ctacatctac gttgtccagc tgccagccaa gatcctgact gcggacaatc 1320
   agacgtacat ggctgtccag ggcagcactg cctaccttct gtgcaaggcc ttcggagcgc 1380
   ctgtgcccag tgttcagtgg ctggacgagg atgggacaac agtgcttcag gacgaacgct 1440
   tcttccccta tgccaatggg accctgggca ttcgagacct ccaggccaat gacaccggac 1500
   gctacttctg cctggctgcc aatgaccaaa acaatgttac catcatggct aacctgaagg 1560
   ttaaagatgc aactcagatc actcaggggc cccgcagcac aatcgagaag aaaggttcca 1620
   gggtgacctt cacgtgccag gcctcctttg acccctcctt gcagcccagc atcacctggc 1680
   gtggggacgg tcgagacctc caggagcttg gggacagtga caagtacttc atagaggatg 1740
   ggcgcctggt catccacagc ctggactaca gcgaccaggg caactacagc tgcgtggcca 1800
   gtaccgaact ggatgtggtg gagagtaggg cacagctctt ggtggtgggg agccctgggc 1860
   cggtgccacg gctggtgctg tccgacctgc acctgctgac gcagagccag gtgcgcgtgt 1920
   cctggagtcc tgcagaagac cacaatgccc ccattgagaa atatgacatt gaatttgagg 1980
   acaaggaaat ggcgcctgaa aaatggtaca gtctgggcaa ggttccaggg aaccagacct 2040
   ctaccaccct caagctgtcg ccctatgtcc actacacctt tagggttact gccataaaca 2100
   aatatggccc cggggagccc agcccggtct ctgagactgt ggtcacacct gaggcagccc 2160
   cagagaagaa ccctgtggat gtgaaggggg aaggaaatga gaccaccaat atggtcatca 2220
   cgtggaagcc gctccggtgg atggactgga acgcccccca ggttcagtac cgcgtgcagt 2280
   ggcgccctca ggggacacga gggccctggc aggagcagat tgtcagcgac cccttcctgg 2340
   tggtgtccaa cacgtccacc ttcgtgccct atgagatcaa agtccaggcc gtcaacagcc 2400
   agggcaaggg accagagccc caggtcacta tcggctactc tggagaggac tacccccagg 2460
   caatccctga gctggaaggc attgaaatcc tcaactcaag tgccgtgctg gtcaagtggc 2520
   ggccggtgga cctggcccag gtcaagggcc acctccgcgg atacaatgtg acgtactgga 2580
   gggagggcag tcagaggaag cacagcaaga gacatatcca caaagaccat gtggtggtgc 2640
   ccgccaacac caccagtgtc atcctcagtg gcttgcggcc ctatagctcc taccacctgg 2700
   aggtgcaggc ctttaacggg cgaggatcgg ggcccgccag cgagttcacc ttcagcaccc 2760
   cagagggagt gcctggccac cccgaggcgt tgcacctgga gtgccagtcg aacaccagcc 2820
   tgctgctgcg ctggcagccc ccactcagcc acaacggcgt gctcaccggc tacgtgctct 2880
   cctaccaccc cctggatgag gggggcaagg ggcaactgtc cttcaacctt cgggaccccg 2940
   aacttcggac acacaacctg accgatctca gcccccacct gcggtaccgc ttccagcttc 3000
   aggccaccac caaagagggc cctggtgaag ccatcgtacg ggaaggaggc actatggcct 3060
   tgtctgggat ctcagatttt ggcaacatct cagccacagc gggtgaaaac tacagtgtcg 3120
   tctcctgggt ccccaaggag ggccagtgca acttcaggtt ccatatcttg ttcaaagcct 3180
   tgggagaaga gaagggtggg gcttcccttt cgccacagta tgtcagctac aaccagagct 3240
   cctacacgca gtgggacctg cagcctgaca ctgactacga gatccacttg tttaaggaga 3300
   ggatgttccg gcaccaaatg gctgtgaaga ccaatggcac aggccgcgtg aggctccctc 3360
   ctgctggctt cgccactgag ggctggttca tcggctttgt gagtgccatc atcctcctgc 3420
   tcctcgtcct gctcatcctc tgcttcatca agcgcagcaa gggcggcaaa tactcagtga 3480
   aggataagga ggacacccag gtggactctg aggcccgacc gatgaaagat gagaccttcg 3540
   gcgagtacag tgacaacgag gagaaggcct ttggcagcag ccagccatcg ctcaacgggg 3600
   acatcaagcc cctgggcagt gacgacagcc tggccgatta tgggggcagc gtggatgttc 3660
   agttcaacga ggatggttcg ttcattggcc agtacagtgg caagaaggag aaggaggcgg 3720
   cagggggcaa tgacagctca ggggccactt cccccatcaa ccctgccgtg gccctagaat 3780
   agtggagtcc aggacaggag atgctgtgcc cctggccttg ggatccaggc ccctccctct 3840
   ccagcaggcc catgggaggc tggagttggg gcagaggaga acttgctgcc tcggatcccc 3900
   ttcctaccac ccggtcccca ctttattgcc aaaacccagc tgcacccctt cctgggcaca 3960
   cgctgctctg ccccagcttg ggcagatctc ccacatgcca ggggcctttg ggtgctgttt 4020
   tgccagccca tttgggcaga gaggctgtgg tttgggggag aagaagtagg ggtggcccga 4080
   aagggtctcc gaaatgctgt ctttcttgct ccctgactgg gggcagacat ggtggggtct 4140
   cctcaggacc agggttggca ccttccccct cccccagcca ctccccagcc agcctggctg 4200
   ggactgggaa cagaactcgg tgtccccacc atctgctgtc ttttctttgc catctctgct 4260
   ccaaccggga tgggagccgg gcaaactggc cgcgggggca ggggaggcca tctggagagc 4320
   ccagagtccc cccactccca gcatcgcact ctggcagcac cgcctcttcc cgccgcccag 4380
   cccaccccat ggccggcttt caggagctcc atacacacgc tgccttcggt acccaccaca 4440
   caacatccaa gtggcctccg tcactacctg gctgcggggc gggcacacct cctcccactg 4500
   cccactggcc ggc 4513
<210> 3
   <211> 2146
   <212> DNA
   <213> Homo sapiens
   <400> 3
   cggagatgga tgtctctctt tgcccagcca agtgtagttt ctggcggatt ttcttgctgg 60
   gaagcgtctg gctggactat gtgggctccg tgctggcttg ccctgcaaat tgtgtctgca 120
   gcaagactga gatcaattgc cggcggccgg acgatgggaa cctcttcccc ctcctggaag 180
   ggcaggattc agggaacagc aatgggaacg ccagtatcaa catcacggac atctcaagga 240
   atatcacttc catacacata gagaactggc gcagtcttca cacgctcaac gccgtggaca 300
   tggagctcta caccggactt caaaagctga ccatcaagaa ctcaggactt cggagcattc 360
   agcccagagc ctttgccaag aacccccatt tgcgttatat aaacctgtca agtaaccggc 420
   tcaccacact ctcgtggcag ctcttccaga cgctgagtct tcgggaattg cagttggagc 480
   agaacttttt caactgcagc tgtgacatcc gctggatgca gctctggcag gagcaggggg 540
   aggccaagct caacagccag aacctctact gcatcaacgc tgatggctcc cagcttcctc 600
   tcttccgcat gaacatcagt cagtgtgacc ttcctgagat cagcgtgagc cacgtcaacc 660
   tgaccgtacg agagggtgac aatgctgtta tcacttgcaa tggctctgga tcaccccttc 720
   ctgatgtgga ctggatagtc actgggctgc agtccatcaa cactcaccag accaatctga 780
   actggaccaa tgttcatgcc atcaacttga cgctggtgaa tgtgacgagt gaggacaatg 840
   gcttcaccct gacgtgcatt gcagagaacg tggtgggcat gagcaatgcc agtgttgccc 900
   tcactgtcta ctatccccca cgtgtggtga gcctggagga gcctgagctg cgcctggagc 960
   actgcatcga gtttgtggtg cgtggcaacc ccccaccaac gctgcactgg ctgcacaatg 1020
   ggcagcctct gcgggagtcc aagatcatcc atgtggaata ctaccaagag ggagagattt 1080
   ccgagggctg cctgctcttc aacaagccca cccactacaa caatggcaac tataccctca 1140
   ttgccaaaaa cccactgggc acagccaacc agaccatcaa tggccacttc ctcaaggagc 1200
   cctttccaga gagcacggat aactttatct tgtttgacga agtgagtccc acacctccta 1260
   tcactgtgac ccacaaacca gaagaagaca cttttggggt atccatagca gttggacttg 1320
   ctgcttttgc ctgtgtcctg ttggtggttc tcttcgtcat gatcaacaaa tatggtcgac 1380
   ggtccaaatt tggaatgaag ggtcccgtgg ctgtcatcag tggtgaggag gactcagcca 1440
   gcccactgca ccacatcaac cacggcatca ccacgccctc gtcactggat gcggggcccg 1500
   acactgtggt cattggcatg actcgcatcc ctgtcattga gaacccccag tacttccgtc 1560
   agggacacaa ctgccacaag ccggacacgt gggtcttttc aaacatagac aatcatggga 1620
   tattaaactt gaaggacaat agagatcatc tagtcccatc aactcactat atatatgagg 1680
   aacctgaggt ccagagtggg gaagtgtctt acccaaggtc acatggtttc agagaaatta 1740
   tgttgaatcc aataagcctt cccggacatt ccaagcctct taaccatggc atctatgttg 1800
   aggatgtcaa tgtttatttc agcaaaggac gtcatggctt ttaaaaactc cttttaagcc 1860
   tccttgtttt gatgtcacct tggtaggctg ggccctctga gaggttggaa gctctaggca 1920
   ttgttctctt tggatccagg gatgctaagt agaaactgca tgagccacca gtgccccggc 1980
   accctttaac accaccagat gggtgttttc ccccatccac cactggcagg gttgcccctt 2040
   ccctccaatc atcactgtgc tccttttttc ccggcctacg aggcagctcc tgccactatc 2100
   tttagagcca ataaagagaa ttaaaaacct gaaaaaaaaa aaaaaa 2146
<210> 4
   <211> 19
   <212> DNA
   <213> Homo sapiens
   <400> 4
   ggcagaatct tcgtccgca 19
<210> 5
   <211> 18
   <212> DNA
   <213> Homo sapiens
   <400> 5
   ggacagtggt ccccgttg 18
<210> 6
   <211> 25
   <212> DNA
   <213> Homo sapiens
   <400> 6
   cccagctgga gttgcacttg cggcc 25
<210> 7
   <211> 18
   <212> DNA
   <213> Homo sapiens
   <400> 7
   gaacaccgac ctcgtccc 18
<210> 8
   <211> 16
   <212> DNA
   <213> Homo sapiens
   <400> 8
   ggcggcccga gagata 16
<210> 9
   <211> 23
   <212> DNA
   <213> Homo sapiens
   <400> 9
   cgccagaagt gcggctggga ttt 23
<210> 10
   <211> 21
   <212> DNA
   <213> Homo sapiens
   <400> 10
   gctgggactg ggaacagaac t 21
<210> 11
   <211> 21
   <212> DNA
   <213> Homo sapiens
   <400> 11
   ggagcagaga tggcaaagaa a 21
<210> 12
   <211> 17
   <212> DNA
   <213> Homo sapiens
   <400> 12
   tccccaccat ctgctgt 17
<210> 13
   <211> 22
   <212> DNA
   <213> Homo sapiens
   <400> 13
   ccacagatga catcagcctc aa 22
<210> 14
   <211> 21
   <212> DNA
   <213> Homo sapiens
   <400> 14
   ggtcacaccc agctcttcct t 21
<210> 15
   <211> 25
   <212> DNA
   <213> Homo sapiens
   <400> 15
   tggcaagccc gaagtgcagt tcctt 25
<210> 16
   <211> 16
   <212> DNA
   <213> Homo sapiens
   <400> 16
   gccccggcac ccttta 16
<210> 17
   <211> 19
   <212> DNA
   <213> Homo sapiens
   <400> 17
   aaccctgcca gtggtggat 19
<210> 18
   <211> 15
   <212> DNA
   <213> Homo sapiens
   <400> 18
   cagatgggtg ttttc 15
<210> 19
   <211> 22
   <212> DNA
   <213> Homo sapiens
   <400> 19
   actcagccca gcatcattct tc 22
<210> 20
   <211> 23
   <212> DNA
   <213> Homo sapiens
   <400> 20
   atggctgttg tactcctcca atc 23
<210> 21
   <211> 30
   <212> DNA
   <213> Homo sapiens
   <400> 21
   cttctcctct tggcagattg tctgtagctt 30
<210> 22
   <211> 22
   <212> DNA
   <213> Homo sapiens
   <400> 22
   ccacacacag cctactttcc aa 22
<210> 23
   <211> 21
   <212> DNA
   <213> Homo sapiens
   <400> 23
   tacccacgcg aatcactctc a 21
<210> 24
   <211> 29
   <212> DNA
   <213> Homo sapiens
   <400> 24
   aacggcaatg cggctgcaac ggcggaatt 29
<210> 25
   <211> 100
   <212> DNA
   <213> Homo sapiens
   <400> 25
   gaacaccgac ctcgtcccgg cccctgcagt ccggatactc acgccagaag tgcggctggg 60
   atccggcggc cacctgcacc tgcgtatctc tcgggccgcc 100
<210> 26
   <211> 110
   <212> DNA
   <213> Homo sapiens
   <400> 26
   ccacagatga catcagcctc aagtgtgagg ccagtggcaa gcccgaagtg cagttccgct 60
   ggacgaggga tggtgtccac ttcaaaccca aggaagagct gggtgtgacc 110

<210> 27
   <211> 70
   <212> DNA
   <213> Homo sapiens
   <400> 27
   actcagccca gcatcattct tctcctcttg gcagattgtc tgtagccgat tggaggagta 60
   caacagccat 70
<210> 28
   <211> 103
   <212> DNA
   <213> Homo sapiens
   <400> 28
   ccacacacag cctactttcc aagcagagcc atgtctggta acggcaatgc ggctgcaacg 60
   gcggaagaaa acagcccaaa gatgagagtg attcgcgtgg gta 103
<210> 29
   <211> 512
   <212> DNA
   <213> Homo sapiens
   <400> 29
   ccaaggccat cggccatcgg aactaccatg caggctactc catgtttggg gctggcctca 60
   ccgtaggcct gtctaacctc ttctgtggag tctgcgtggg catcgtgggc agtggggctg 120
   ccctggccga tgctcagaac cccagcctct ttgtaaagat tctcatcgtg gagatctttg 180
   gcagcgccat tggcctcttt ggggtcatcg tcgcaattct tcagacctcc agagtgaaga 240
   tgggtgacta gatgatatgt gtgggtgggg ccgtgcctca cttttattta ttgctggttt 300
   tcctgggaca gctggagctg tgtcccttag cctttcagag gcttggtgtt cagggccctc 360
   cctgcactcc cctcttgctg cgtgttgatt tggaggcact gcagtccagg ccgagtcctc 420
   agtgcgggga gcaggctgct gctgctgact ctgtgcagct gcgcacctgt gtcccccacc 480
   tccaccctca acccatcttc ctagtgtttg tg 512
<210> 30
   <211> 419
   <212> DNA
   <213> Homo sapiens
   <400> 30
   tctctctttg tgggttggcc aggaggttcc cccgaccagg ttggggagac ttggggccag 60
   cgcttctggt ctggtaaata tgtatgatgt gttgtgcttt tttaaccaag gaggggccag 120
   tggattccca cagcacaacc ggtcccttcc atgccctggg atgcctcacc acacccaggt 180
   ctcttccttt gctctgaggt cccttcaagg cctccccaat ccaggccaaa gccccatgtg 240
   ccttgtccag ggaactgcct gggccatgcg aggggccagc agagggcgcc accacctgac 300
   ggctgggacc cacccagccc ctctcccctc tctgctccag actcacttgc cattgccagg 360
   agatggcccc aacaagcacc ccgcttttgc agcagaggag ctgagttggc agaccgggc 419
<210> 31
   <211> 505
   <212> DNA
   <213> Homo sapiens
   <400> 31
   cctatcagaa tatgtccctc aacccccgaa acaaggcttc tctcagcctc cccaccagtg    60
   atggataaca gctcctattc tcagctgacc tgactgagcc aacccatgaa ctcttcactc 120
   cttggggaag ccacctccca tcacacccct gagcagagtt agggaggaat tctacttccc 180
   ataaaaggac ctctcctgag aggcaaaacc tgttgcctcc accacggctt ccctcttggc 240
   tcattccaag cttggccaaa ttggggaagt gggatggagg ttgccctgca tcccccctcc 300
   tctgcctgag tgtgtctttg taatgtcagc tggcatcata caaagagcag gagaagcaaa 360
   cacccagaac tcttttgctg gtcagagatt ccctgagtgt ctgtcctcac ccaagcctgc 420
   tctgtgtctg tgttgtgaag cttgagactc tggaaagaaa tggggagggg gggcagggga 480
   aatgttgccc taagaatgct tctca 505
<210> 32
   <211> 475
   <212> DNA
   <213> Homo sapiens
   <400> 32
   agttgaagat ggtcccttac agcttcccaa gttaggttag tgatgtgaaa tgctcctgtc 60
   cctggcccta cctccttccc tgtccccacc cctgcataag gcagttgttg gttttcttcc 120
   ccaattcttt tccaagtagg ttttgtttac cctactcccc aaatccctga gccagaagtg 180
   gggtgcttat actcccaaac cttgagtgtc cagccttccc ctgttgtttt tagtctcttg 240
   tgctgtgcct agtggcacct gggctgggga ggacactgcc ccgtctaggt ttttataaat 300
   gtcttactca agttcaaacc tccagcctgt gaatcaactg tgtctctttt ttgacttggt 360
   aagcaagtat taggctttgg ggtgggggga ggtctgtaat gtgaaacaac ttcttgtctt 420
   tttttctccc actgttgtaa ataactttta atggccaaac cccagatttg tactt 475
<210> 33
   <211> 441
   <212> DNA
   <213> Homo sapiens
   <400> 33
   caaggctggg ccgggaaggg cgtgggttga ggagaggctc cagacccgca cgccgcgcgc 60
   acagagctct cagcgccgct cccagccaca gcctcccgcg cctcgctcag ctccaacatg 120
   gcaaaaatct ccagccctac agagactgag cggtgcatcg agtccctgat tgctgtcttc 180
   cagaagtatg ctggaaagga tggttataac tacactctct ccaagacaga gttcctaagc 240
   ttcatgaata cagaactagc tgccttcaca aagaaccaga aggaccctgg tgtccttgac 300
   cgcatgatga agaaactgga caccaacagt gatggtcagc tagatttctc agaatttctt 360
   aatctgattg gtggcctagc tatggcttgc catgactcct tcctcaaggc tgtcccttcc 420
   cagaagcgga cctgaggacc c 441
<210> 34
   <211> 276
   <212> DNA
   <213> Homo sapiens
   <400> 34
   ggcacctggg gctcatggat tggccccgac cacgacaagt tcagtgccat gaagtatgag 60
   caaggcacgg gctgctggca gggccccaac cgctccacca ccgtgcgcct cctgtgcggg 120
   aaagagacca tggtgaccag caccacagag cccagtcgct gcgagtacct catggagctg 180
   atgacgccag ccgcctgccc ggagccaccg cctgaagcac ccaccgaaga cgaccatgac 240
   gagctctagc tggatgggcg cagagaacct caagaa 276
<210> 35
   <211> 567
   <212> DNA
   <213> Homo sapiens
   <400> 35
   ttcccgtgca accagtttgg gcatcaggag aacgccaaga acgaagagat tctgaattcc 60
   ctcaagtacg tccggcctgg tggtgggttc gagcccaact tcatgctctt cgagaagtgc 120
   gaggtgaacg gtgcgggggc gcaccctctc ttcgccttcc tgcgggaggc cctgccagct 180
   cccagcgacg acgccaccgc gcttatgacc gaccccaagc tcatcacctg gtctccggtg 240
   tgtcgcaacg atgttgcctg gaactttgag aagttcctgg tgggccctga cggtgtgccc 300
   ctacgcaggt acagccgccg cttccagacc attgacatcg agcctgacat cgaagccctg 360
   ctgtctcaag ggcccagctg tgcctagggc gcccctccta ccccggctgc ttggcagttg 420
   cagtgctgct gtctcggggg ggttttcatc tatgagggtg tttcctctaa acctacgagg 480
   gaggaacacc ttgatcttac agaaaatacc acctcgagat gggtgctggt cctgttgatc 540
   ccagtctctg ccagaccaag gcgagtt 567
<210> 36
   <211> 165
   <212> DNA
   <213> Homo sapiens
   <400> 36
   gggctgcatc accatcatag gtggtggaga cactgccact tgctgtgcca aatggaacac 60
   ggaggataaa gtcagccatg tgagcactgg gggtggtgcc agtttggagc tcctggaagg 120
   taaagtcctt cctggggtgg atgctctcag caatatttag tactt 165
<210> 37
   <211> 481
   <212> DNA
   <213> Homo sapiens
   <400> 37
   gagtatgtag tggcttcttt tgaactgtta gatgctgaat atctgttcac ttttcaatcc 60
   caattctgtc ccaatcttac cagatgctac tggacttgaa tggttaataa aactgcacag 120
   tgctgttggt ggcagtgact tcttttgagt taggttaata aatcaagcca tagagcccct 180
   cctggttgat acttgttcca gatggggcct ttggggctgg tagaaatacc caacgcacaa 240
   atgaccgcac gttctctgcc ccgtttcttg ccccagtgtg gtttgcattg tctccttcca 300
   caatgactgc tttgtttgga tgcctcagcc caggtcagct gttactttct ttcagatgtt 360
   tatttgcaaa caaccatttt ttgttctgtg tcccttttaa aaggcagatt aaaagcacaa 420
   gcgtgtttct agagaacagt tgagagagaa tctcaagatt ctacttggtg gtttgcttgc 480
   t 481
<210> 38
   <211> 461
   <212> DNA
   <213> Homo sapiens
   <400> 38
   ctgggctgac caaaatgtgc tttctactgt gagtccctat cccaagatcc tggggaaagg 60
   agagaccatg gtgtgaatgt agagatgcca cctccctctc tctgaggcag gcctgtggat 120
   gaaggaggag ggtcagggct ggccttcctc tgtgcatcac tctgctaggt tgggggcccc 180
   cgacccacca tacctacgcc tagggagccc gtcctccagt attccgtctg tagcaggagc 240
   tagggctgct gcctcagctc caagacaaga atgaacctgg ctgtgtcagt cattttgtct 300
   tttccttttt ttttttttgc cacattggca gagatgggac ctaagggtcc cacccctcac 360
   cccaccccca cctcttctgt atgtttgaat tctttcagta gctgttgatg ctggttggac 420
   aggtttgagt caaattgtac tttgctccat tgttaattga g 461
<210> 39
   <211> 479
   <212> DNA
   <213> Homo sapiens
   <400> 39
   gattcaaaga gattcctgca ggccagaggc cggaacacac ctttatggct ggggctctcc 60
   gtggtgttct ggacccagcc cctggagaca ccattcactt ttactgcttt gtagtgactc 120
   gtgctctcca acctgtcttc ctgaaaaacc aaggccccct tcccccacct cttccatggg 180
   gtgagacttg agcagaacag gggcttcccc aagttgccca gaaagactgt ctgggtgaga 240
   agccatggcc agagcttctc ccaggcacag gtgttgcacc agggacttct gcttcaagtt 300
   ttggggtaaa gacacctgga tcagactcca agggctgccc tgagtctggg acttctgcct 360
   ccatggctgg tcatgagagc aaaccgtagt cccctggaga cagccactcc agagaacctc 420
   ttgggagaca gaagaggcat ctgtgcacag ctcgatcttc tacttgcctg tggggaggg 479
<210> 40
   <211> 529
   <212> DNA
   <213> Homo sapiens
   <400> 40
   gagctggcca gcactaagca aaaactagag aaagctgaaa accaggttct ggccatgcgg 60
   aagcagtctg agggcctcac caaggagtac gaccgcttgc tggaggagca cgcaaagctg 120
   caggctgcag tagatggtcc catggacaag aaggaagagt aagggcctcc ttcctcccct 180
   gcctgcagct ggcttccacc tggcacgtgc ctgctgcttc ctgagagccc ggcctctccc 240
   tccagtactt ctgtttgtgc ccttctgctt cccccattcc cttccacagc tcatagctcg 300
   tcatctcggc ccttgtccac actctccaag cacattacag gggacctgat tgctacacgt 360
   tcagaatgcg tttgctgtca tcctgcttgg cctggccagg cctggcacag ccttggcttc 420
   cacgcctgag cgtggagagc acgagttagt tgtagtccgg cttgcggtgg ggctgacttc 480
   ctgttggttt gagccccttt ttgttttgcc ctctgggtgt tttctttgg 529
<210> 41
   <211> 195
   <212> DNA
   <213> Homo sapiens
   <400> 41
   tccccctgta gactagtgcc gtgggagtac ctgctgccca gctgctgtgg ccccctccgt 60
   gatccatcca tctccaggga gcaagacaga gacgcaggat ggaaagcgga gttcctaaca 120
   ggatgaaagt tcccccatca gttcccccag tacctccaag caagtagctt tccacatttg 180
   tcacagaaat cagag 195
<210> 42
   <211> 301
   <212> DNA
   <213> Homo sapiens
   <400> 42
   tggtgttggg agccctttgg agaacgccag tctccaggtc cccctgcatc tatcgagttt 60
   gcaatgtcac aacctctctg atcttgtgct cagcatgatt ctttaataga agttttattt 120
   ttcgtgcact ctgctaatca tgtgggtgag ccagtggaac agcgggagcc tgtgctggtt 180
   tgcagattgc ctcctaatga cgcggctcaa aaggaaacca agtggtcagg agttgtttct 240
   gacccactga tctctactac cacaaggaaa atagtttagg agaaaccagc ttttactgtt 300
   t 301
<210> 43
   <211> 562
   <212> DNA
   <213> Homo sapiens
   <400> 43
   gtttgtagac tctctgacca aggccacctg tgccccccag catggggccc cgggtcctgg 60
   gcctgctgac gccagcaagg tggtggccaa gggcctgggg ctgagcaagg cctacgtagg 120
   ccagaagagc agcttcacag tagactgcag caaagcaggc aacaacatgc tgctggtggg 180
   ggttcatggc ccaaggaccc cctgcgagga gatcctggtg aagcacgtgg gcagccggct 240
   ctacagcgtg tcctacctgc tcaaggacaa gggggagtac acactggtgg tcaaatgggg 300
   gcacgagcac atcccaggca gcccctaccg cgttgtggtg ccctgagtct ggggcccgtg 360
   ccagccggca gcccccaagc ctgccccgct acccaagcag ccccgccctc ttcccctcaa 420
   ccccggccca ggccgccctg gccgcccgcc tgtcactgca gctgcccctg ccctgtgccg 480
   tgctgcgctc acctgcctcc ccagccagcc gctgacctct cggctttcac ttgggcagag 540
   ggagccattt ggtggcgctg ct 562
<210> 44
   <211> 333
   <212> DNA
   <213> Homo sapiens
   <400> 44
   gccaagcaca cccaggagaa ctgtgagacc tggggtgtaa atggtgagac gggtactttg 60
   gtggacatga aggaactggg catatgggag ccattggctg tgaagctgca gacttataag 120
   acagcagtgg agacggcagt tctgctactg cgaattgatg acatcgtttc aggccacaaa 180
   aagaaaggcg atgaccagag ccggcaaggc ggggctcctg atgctggcca ggagtgagtg 240
   ctaggcaagg ctacttcaat gcacagaacc agcagagtct ccccttttcc tgagccagag 300
   tgccaggaac actgtggacg tctttgttca gaa 333
<210> 45
   <211> 411
   <212> DNA
   <213> Homo sapiens
   <400> 45
   gtgtctgttg ctgatgcctc aaaaagtgtg caggtctcga ctctgaagac agagttcctg 60
   ccgctcctaa gtgtgtcatt tgtctcagag aacagcgtcg tggctgctgg ccatgactgc 120
   tgcccaatgc tctttatcta cgatgaccgc ggctgcctga ccttcgtctc caagttagat 180
   attccaaaac agagcatcca acgcaacatg tctgccatgg aacgcttccg caacatggac 240
   aagagagcca caactgagga ccgcaacacg gccttggaga cgctgcacca gaatagcatc 300
   actcaagtct ctatttatga ggtggacaag caagattgtc gcaaattttg cactactggc 360
   atcgatggag ccatgacaat ttgggatttc aagaccctcg agtcttccat c 411
<210> 46
   <211> 411
   <212> DNA
   <213> Homo sapiens
   <400> 46
   gtgtctgttg ctgatgcctc aaaaagtgtg caggtctcga ctctgaagac agagttcctg 60
   ccgctcctaa gtgtgtcatt tgtctcagag aacagcgtcg tggctgctgg ccatgactgc 120
   tgcccaatgc tctttatcta cgatgaccgc ggctgcctga ccttcgtctc caagttagat 180
   attccaaaac agagcatcca acgcaacatg tctgccatgg aacgcttccg caacatggac 240
   aagagagcca caactgagga ccgcaacacg gccttggaga cgctgcacca gaatagcatc 300
   actcaagtct ctatttatga ggtggacaag caagattgtc gcaaattttg cactactggc 360
   atcgatggag ccatgacaat ttgggatttc aagaccctcg agtcttccat c 411

<210> 47
   <211> 555
   <212> DNA
   <213> Homo sapiens
   <400> 47
   caggccatgc ttgcactcag aagttttctc atgaggagat tgccatggcg accgtcacag 60
   cgctgcgccg cacagtgccc cccgctgtca ctgggatcac cttcctgtct ggaggccaga 120
   gtgaggagga ggcgtccatc aacctcaatg ccattaacaa gtgccccctg ctgaagccct 180
   gggccctgac cttctcctac ggccgagccc tgcaggcctc tgccctgaag gcctggggcg 240
   ggaagaagga gaacctgaag gctgcgcagg aggagtatgt caagcgagcc ctggccaaca 300
   gccttgcctg tcaaggaaag tacactccga gcggtcaggc tggggctgct gccagcgagt 360
   ccctcttcgt ctctaaccac gcctattaag cggaggtgtt cccaggctgc ccccaacaac 420
   tccaggccct gccccctccc actcttgaag aggaggccgc ctcctcgggg ctccaggctg 480
   gcttgcccgc gctctttctt ccctcgtgac agtggtgtgt ggtgtcgtct gtgaatgcta 540
   agtccatcac ccttt 555
<210> 48
   <211> 550
   <212> DNA
   <213> Homo sapiens
   <400> 48
   gcaaattcca tcgtgtaatc aaggacttca tgatccaggg cggagacttc accaggggag 60
   atggcacagg aggaaagagc atctacggtg agcgcttccc cgatgagaac ttcaaactga 120
   agcactacgg gcctggctgg gtgagcatgg ccaacgcagg caaagacacc aacggctccc 180
   agttcttcat cacgacagtc aagacagcct ggctagatgg caagcatgtg gtgtttggca 240
   aagttctaga gggcatggag gtggtgcgga aggtggagag caccaagaca gacagccggg 300
   ataaacccct gaaggatgtg atcatcgcag actgcggcaa gatcgaggtg gagaagccct 360
   ttgccatcgc caaggagtag ggcacaggga catctttctt tgagtgaccg tctgtgcagg 420
   ccctgtagtc cgccacaggg ctctgagctg cactggcccc ggtgctggca tctggtggag 480
   cggacccact cccctcacat tccacaggcc catggactca cttttgtaac aaactcctac 540
   caacactgac 550
<210> 49
   <211> 198
   <212> DNA
   <213> Homo sapiens
   <400> 49
   gacttcatga tccagggcgg agacttcacc aggggagatg gcacaggagg aaagagcatc 60
   tacggtgagc gcttccccga tgagaacttc aaactgaagc actacgggcc tggctgggtg 120
   agcatggcca acgcaggcaa agacaccaac ggctcccagt tcttcatcac gacagtcaag 180
   acagcctggc tagatggc 198
<210> 50
   <211> 493
   <212> DNA
   <213> Homo sapiens
   <400> 50
   gaaccaattg cgagtcatgt agtgtggtag aattaaagga ggacacgagc ctgcttctgt 60
   tacctccaag tggtaacagg actgatgccg aaatgtcacc aggtcctttc agtcttcaca 120
   gtggagaact cttggccaaa ggtttttggg gggaggagga ggaaaccagc tttctggtta 180
   aggttaacac cagatggtgc ccctcattgg tgtcctttta aaaaatattt actgtagtcc 240
   aataagatag cagctgtaca aaatgactaa aatagattgt aggatcatat ggcgtatatc 300
   ttggttcatc ttcaaaatca gagactgagc tttgaaacta gtggttttta atcaaagttg 360
   gctttatagg aggagtataa tgtatgcact actgttttaa aagaattagt gtgagtgtgt 420
   ttttgtatga atgagcccat tcatggtaag tcttaagctt gttggaaata atgtacccat 480
   gtagactagc aaa 493
<210> 51
   <211> 509
   <212> DNA
   <213> Homo sapiens
   <220>
   <221> misc_feature
   <222> (210)..(210)
   <223> n is a, c, g, or t
   <220>
   <221> misc_feature
   <222> (212)..(213)
   <223> n is a, c, g, or t
   <220>
   <221> misc_feature
   <222> (226)..(226)
   <223> n is a, c, g, or t
   <220>
   <221> misc_feature
   <222> (228)..(231)
   <223> n is a, c, g, or t
   <220>
   <221> misc_feature
   <222> (233)..(234)
   <223> n is a, c, g, or t
   <220>
   <221> misc_feature
   <222> (236)..(240)
   <223> n is a, c, g, or t
   <220>
   <221> misc_feature
   <222> (243)..(243)
   <223> n is a, c, g, or t
   <220>
   <221> misc_feature
   <222> (245)..(246)
   <223> n is a, c, g, or t
   <220>
   <221> misc_feature
   <222> (248)..(249)
   <223> n is a, c, g, or t
   <400> 51
   gaaatgactg caaattccta gtgaatgtac aggtttgctt tcgtgtccct cttctggttg 60
   ctttagaagt gacgtgtaat ttctgaaccc atgtttcatc tgtataaaag aacatctgca 120
   ccagtttttc tcctgcccct cagaagagcc aaactttgag ttttatgtct gtttgtcatt 180
   gataaatttc aataaatctt tttatacaan tnnaaaaaaa aaaaanannn nannannnnn 240
   aananncnna ttgatctttt caagatgcat tccagatgaa ctgctaggtg agggggaagc 300
   ttcatttttg ttacctgata gaatagcttt tcttatgaga tatatataat gtgatactat 360
   gtttggatat ttttggtctt aaagcaagac tcagtggtgt atcttcatta aaagcttcct 420
   ttaaaaaagt tacagagtta ctaaaaaaac aagtacccaa acaatcaagt tgggccaacc 480
   ttggaacctt gttttgaata tctttcatt 509
<210> 52
   <211> 453
   <212> DNA
   <213> Homo sapiens
   <400> 52
   gtgagcattt gttcctgact ctcaaagagg atggtttgga gttctcttac gtttcctggt 60
   atttcccaag tctcttgggt tggttggaag gctgtggctg gtctcagttt ggttactcaa 120
   tgcccaggag gggctgagca ccagccatat cttttgcttt ggttcacatg atgatacctg 180
   cttttctcag gcctgctaga ggcatccaac gccctggttt gtaaatagca acctaaaggc 240
   gtattttggc actggtctgg ggacattccc catctctcat cccttttccc ccttcacaga 300
   tggtggtggg cttcgctcta caaagaggac tctgatgtta ctcttgagct tatgagccag 360
   agagctgaaa accgcaggct tgttgtgtta agttacaagg aaaatggatt tggtaattaa 420
   aattagaaga aacacacctt caaacttcaa ctt 453
<210> 53
   <211> 398
   <212> DNA
   <213> Homo sapiens
   <400> 53
   ctcctggact caatcatggc ttgtggtctg gtcgccagca acctgaatct caaacctgga 60
   gagtgccttc gagtgcgagg cgaggtggct cctgacgcta agagcttcgt gctgaacctg 120
   ggcaaagaca gcaacaacct gtgcctgcac ttcaaccctc gcttcaacgc ccacggcgac 180
   gccaacacca tcgtgtgcaa cagcaaggac ggcggggcct gggggaccga gcagcgggag 240
   gctgtctttc ccttccagcc tggaagtgtt gcagaggtgt gcatcacctt cgaccaggcc 300
   aacctgaccg tcaagctgcc agatggatac gaattcaagt tccccaaccg cctcaacctg 360
   gaggccatca actacatggc agctgacggt gacttcaa 398
<210> 54
   <211> 446
   <212> DNA
   <213> Homo sapiens
   <400> 54
   acgcccgata cgctgagtgt ggtttgcgga tcctggcctt cccgtgtaac cagttcggga 60
   agcaggagcc agggagtaac gaagagatca aagagttcgc cgcgggctac aacgtcaaat 120
   tcgatatgtt cagcaagatc tgcgtgaacg gggacgacgc ccacccgctg tggaagtgga 180
   tgaagatcca acccaagggc aagggcatcc tgggaaatgc catcaagtgg aacttcacca 240
   agttcctcat cgacaagaac ggctgcgtgg tgaagcgcta cggacccatg gaggagcccc 300
   tggtgataga gaaggacctg ccccactatt tctagctcca caagtgtgtg gccccgcccg 360
   agcccctgcc cacgcccttg gagccttcca ccggcactca tgacggcctg cctgcaaacc 420
   tgctggtggg gcagacccga aaatcc 446
<210> 55
   <211> 456
   <212> DNA
   <213> Homo sapiens
   <400> 55
   aagacgacat gcttcatctg tggtctggag agggacaagt ttgataacaa gacagtgtca 60
   tttgaggaac acatcaagct ggagcacaac atgtggaact acttgtactt cattgtgctg 120
   gtccgcgtga agaacaagac cgactacacg ggccctgaga gctacgtggc ccagatgatc 180
   aagaacaaga acctggactg gttcccccgg atgcgggcca tgtcccttgt cagcaatgag 240
   ggcgaggggg agcagaatga gattcggatt ctccaggaca agctcaactc caccatgaag 300
   ctggtgtccc acctcactgc ccagctcaac gagctcaagg agcagatgac ggagcagcgg 360
   aaacgcaggc aacgcctagg ctttgtggat gtccagaact gcattagccg ctgaggagag 420
   ccaccgaagg ccccaacagg ggatgctcat cactgg 456
<210> 56
   <211> 510
   <212> DNA
   <213> Homo sapiens
   <400> 56
   acagtcctgc ttagagccct taaaaagact tgaaagttca ctgggactca gtttacctta 60
   atgccttagc agaagataaa tcctacctag agacctttgt tccttaaagc aataactgac 120
   aactctttgt agtcctcctt gtgggtagtt aagagtgggg tcaccccttt aactccaagc 180
   actacatttt ggcggctgcg gcctctgggg gaggtggcag ttatgctgtt actagtgatt 240
   ttagggcttt gttatttaac ttatttcaag ggtgctgtgc tcagccctgc ccatggctgt 300
   gcagctccct ccgtgcctca gatctgctgt agccagtgca gacctcactg tcgtgtccat 360
   gccacccccg gcatggctcc aggtggcctg gtgactccat gatggacgat cttgctccca 420
   ggacctgcct cttcccaggc ttcctgggga agagttgtac gcccaggcaa caagggctga 480
   gctgcgcttg cgtggctgtt tcatgaccgc 510
<210> 57
   <211> 522
   <212> DNA
   <213> Homo sapiens
   <400> 57
   tcagaaggta ggggccgtgt cccgcggtgc tgactgaggc ctgcttcccc ctccccctcc 60
   tgctgtgctg gaattccaca gggaccaggg ccaccgcagg ggactgtctc agaagacttg 120
   atttttccgt ccctttttct ccacactcca ctgacaaacg tccccagcgg tttccacttg 180
   tgggcttcag gtgttttcaa gcacaaccca ccacaacaag caagtgcatt ttcagtcgtt 240
   gtgctttttt gttttgtgct aacgtcttac taatttaaag atgctgtcgg caccatgttt 300
   atttatttcc agtggtcatg ctcagccttg ctgctctgcg tggcgcaggt gccatgcctg 360
   ctccctgtct gtgtcccagc cacgcagggc catccactgt gacgtcggcc gaccaggctg 420
   gacaccctct gccgagtaat gacgtgtgtg gctgggacct tctttattct gtgttaatgg 480
   ctaacctgtt acactgggct gggttgggta gggtgttctg gc 522
<210> 58
   <211> 356
   <212> DNA
   <213> Homo sapiens
   <400> 58
   ctctcttcaa cggtgacact cagtatgtct gcagatgtac cccttgttgt agagtataaa 60
   attgcggata tgggacactt aaaatactac ttggctccca agatcgagga tgaagaagga 120
   tcttaggcat tcttaaaatt caagaaaata aaactaagct ctttgagaac tgcttctaag 180
   atgccagcat atactgaagt cttttctgtc accaaatttg tacctctaag tacatatgta 240
   gatattgttt tctgtaaata acctattttt tttctctatt ctctccaatt tgtttaaaga 300
   ataaagtcca aagtctgatc tggtctagtt aacctagaag tatttttgtc tcttag 356
<210> 59
   <211> 381
   <212> DNA
   <213> Homo sapiens
   <400> 59
   catccattag gccagcaacg cttgtagaac tcactctggg ctgtaacgtg gcactggtag 60
   gttgggacac cagggaagaa gatcaacgcc tcactgaaac atggctgtgt ttgcagcctg 120
   ctctagtggg acagcccaga gcctggctgc cccatcatgt ggccccaccc aatcaaggga 180
   agaaggagga atgctggact ggaggcccct ggagccagat ggcaagaggg tgacagcttc 240
   ctttcctgtg tgtactctgt ccagttcctt tagaaaaaat ggatgcccag aggactccca 300
   accctggctt ggggtcaaga aacagccagc aagagttagg ggccttaggg cactgggctg 360
   ttgttccatt gaagccgact c 381

<210> 60
   <211> 441
   <212> DNA
   <213> Homo sapiens
   <400> 60
   ttcgatgctc agacaggggc cgacagggag gttcagagga tcctgctgga gctgctgaat 60
   cagatggatg gatttgatca gaatgtcaat gtcaaggtaa tcatggccac aaacagagca 120
   gacaccctgg atccggccct gctacggcca ggacggctgg accgtaaaat tgaatttcca 180
   cttcctgacc gccgccagaa gagattgatt ttctccacta tcactagcaa gatgaacctc 240
   tctgaggagg ttgacttgga agactatgtg gcccggccag ataagatttc aggagctgat 300
   atcaactcca tctgtcagga gagtggaatg ttggctgtcc gtgaaaaccg ctacattgtc 360
   ctggccaagg acttcgagaa agcatacaag actgtcatca agaaggacga gcaggagcat 420
   gagttttaca agtgaccctt c 441
<210> 61
   <211> 442
   <212> DNA
   <213> Homo sapiens
   <400> 61
   aacacaactt ctgaggcagg cctgccccag ggggaagcac ggacccgaga cgacggcgat 60
   gaggaagggc tcctgacaca cagcgaggaa gagctggaac acagccagga cacagacgcg 120
   gatgatgggg ccttgcagta agcagcctga caggagcaat ggccaccagc aggtgaaggg 180
   catcgctgcc ccaggcctca agccgggcac ccaaccctgg atgccacccc ccagcgggta 240
   ccagaggaaa gctggcagca ggcgcctcct cccccaacgc atcccagcca gtgccatgtc 300
   ctctgcaggt ggagttactg gcctactcct tccccatgag ccctccctgt ctgcactgcc 360
   caggccagag ggtagagcac aggggtttcc ccatactacc tcccctcccc aggacactcc 420
   caggcttggg ttttttctat ag 442
<210> 62
   <211> 524
   <212> DNA
   <213> Homo sapiens
   <400> 62
   gagacttttt tgaactcaga cttaaatatt atggattaag aaaagaatgg ctcctaggaa 60
   tgcttggtgc tgaatctgct aaactgaata atcaggctcg ctttatctta gagaaaatag 120
   atggcaaaat aatcattgaa aataagccta agaaagaatt aattaaagtt ctgattcaga 180
   ggggatatga ttcggatcct gtgaaggcct ggaaagaagc ccagcaaaag gttccagatg 240
   aagaagaaaa tgaagagagt gacaacgaaa aggaaactga aaagagtgac tccgtaacag 300
   attctggacc aaccttcaac tatcttcttg atatgcccct ttggtattta accaaggaaa 360
   agaaagatga actctgcagg ctaagaaatg aaaaagaaca agagctggac acattaaaaa 420
   gaaagagtcc atcagatttg tggaaagaag acttggctac atttattgaa gaattggagg 480
   ctgttgaagc caaggaaaaa caagatgaac aagtcggact tcct 524
<210> 63
   <211> 416
   <212> DNA
   <213> Homo sapiens
   <400> 63
   gagggaccat gtgtcacttg tgctttgctc ttgtcccacg tgtcttccac tttgcatatg 60
   agccgtgaac tgtgcatagt gctgggatgg aggggagtgt tgggcatgtg atcacgcctg 120
   gctaataagg ctttagtgta tttatttatt tatttatttt atttgttttt cattcatccc 180
   attaatcatt tccccataac tcaatggcct aaaactggcc tgacttgggg gaacgatgtg 240
   tctgtatttc atgtggctgt agatcccaag atgactgggg tgggaggtct tgctagaatg 300
   ggaagggtca tagaaagggc cttgacatca gttcctttgt gtgtactcac tgaagcctgc 360
   gttggtccag agcggaggct gtgtgcctgg gggagttttc ctctatacat ctctcc 416
<210> 64
   <211> 556
   <212> DNA
   <213> Homo sapiens
   <400> 64
   tacagcgtat aggtgcagcc ctgtcacaac accaacagaa gtagcagcct ctgggtgcag 60
   tcacccacac cccaaagctg gaaggatctg gttcaacata gcacaaaccc ttaggaaaaa 120
   tgaaattaac atcactgatg tgtaatccag taaaatctcc ctttttcggg tgtgtatgtg 180
   ggcatgtgcc catttctatg tgtgtgtcta cgtgcagctc actaccaaca gcctcatgtg 240
   cacttgacct gacagtgctc gctgagaact ctcaccaggt tggcgcctga atgccttact 300
   ctcagcagtc agaggcttgc ttgctctgtg cagattttta attttctttt ttggccctag 360
   gctggttggg acctctacag cttcattctt tcacattaaa tagtgacctt tttcagtatt 420
   ttccctcttc ccctttataa attatgctaa agccacaaag cacatttttg gggatcatag 480
   aaggttgggg ttccagaaag gcatctgtgt gatggttcca ttgatgtggg atttccctac 540
   ttgctgtatt ctcagt 556
<210> 65
   <211> 453
   <212> DNA
   <213> Homo sapiens
   <400> 65
   ttggggtata ggtctcatct cttcaggttc tcatgatacc acctttactg tgcttatttt 60
   tttaagaaaa aagtgttgat caaccattcg acctataaga agccttaatt tgcacagtgt 120
   gtgacttaca gaaactgcat gaaaaatcat gggccagagc ctcggcccta gcattgcact 180
   tggcctcatg ctggagggag gctgggcggg tacagcgcgg aggaggaggg aggccaggcg 240
   ggcatggcgt ggaggaggag ggaggccggg cggtcacagc atggaggagg agggaggcgc 300
   tgctggtgtt cttattctgg cggcagcgcc tttcctgcca tgtttagtga atgacttttc 360
   tcgcattgta gaattgtata tagactctgg tgttctattg ctgagaagca aaccgccctg 420
   cagcatccct cagcctgtac cggtttggct ggc 453
<210> 66
   <211> 533
   <212> DNA
   <213> Homo sapiens
   <220>
   <221> misc_feature
   <222> (360)..(361)
   <223> n is a, c, g, or t
   <400> 66
   gaggtcagat ttggagcttc tcattgcacg cggagattat tattgcatcg ggttccaagc 60
   caatgggaag cccgggggag gggtttggca tgaggaagcg ttggttacag cagctgattg 120
   gctgcagcca agactgtgaa aggataaaga ggcgcgaggc ggaattgggg tctgctctaa 180
   gctgcagcaa gagaaactgt gtgtgagggg aagaggcctg tttcgctgtc gggtctctag 240
   ttcttgcacg ctctttaaga gtctgcactg gaggaactcc tgccattacc agctcccttc 300
   ttgcagaagg gagggggaaa catacattta ttcatgccag tctgttgcat gcaggctttn 360
   nggcttccta ccttgcaaca aaataattgc accaactcct tagtgccgat tccgcccaca 420
   gagagtcctg gagccacagt cttttttgct ttgcattgta ggagagggac taagtgctag 480
   agactatgtc gctttcctga gctaccgaga gcgctcgtga actggaatca act 533
<210> 67
   <211> 408
   <212> DNA
   <213> Homo sapiens
   <400> 67
   gtaaaccaca tcttttttgc acttttttta taagcaaaaa cgtgccgttt aaaccactgg 60
   atctatctaa atgccgattt gagttcgcga cactatgtac tgcgtttttc attcttgtat 120
   ttgactattt aatcctttct acttgtcgct aaatataatt gttttagtct tatggcatga 180
   tgatagcata tgtgttcagg tttatagctg ttgtgtttaa aaattgaaaa aagtggaaaa 240
   catctttgta catttaagtc tgtattataa taagcaaaaa gattgtgtgt atgtatgttt 300
   aatataacat gacaggcact aggacgtctg cctttttaag gcagttccgt taagggtttt 360
   tgtttttaaa cttttttttg ccatccatcc tgtgcaatat gccgtgta 408
<210> 68
   <211> 526
   <212> DNA
   <213> Homo sapiens
   <400> 68
   ccctttggtc tggtgccagt tctggaaaac agtcagggtc agctgatcta cgagtctgcc 60
   atcacctgtg agtacctgga tgaagcatac ccagggaaga agctgttgcc ggatgacccc 120
   tatgagaaag cttgccagaa gatgatctta gagttgtttt ctaaggtgcc atccttggta 180
   ggaagcttta ttagaagcca aaataaagaa gactatgctg gcctaaaaga agaatttcgt 240
   aaagaattta ccaagctaga ggaggttctg actaataaga agacgacctt ctttggtggc 300
   aattctatct ctatgattga ttacctcatc tggccctggt ttgaacggct ggaagcaatg 360
   aagttaaatg agtgtgtaga ccacactcca aaactgaaac tgtggatggc agccatgaag 420
   gaagatccca cagtctcagc cctgcttact agtgagaaag actggcaagg tttcctagag 480
   ctctacttac agaacagccc tgaggcctgt gactatgggc tctgaa 526
<210> 69
   <211> 432
   <212> DNA
   <213> Homo sapiens
   <400> 69
   gccacagact gaactcgcag ggagtgcagc aggaaggaac aaagacaggc aaacggcaac 60
   gtagcctggg ctcactgtgc tggggcatgg cgggatcctc cacagagagg aggggaccaa 120
   ttctggacag acagatgttg ggaggataca gaggagatgc cacttctcac tcaccactac 180
   cagccagcct ccagaaggcc ccagagagac cctgcaagac cacggaggga gccgacactt 240
   gaatgtagta ataggcaggg ggccctgcca ccccatccag ccagacccca gctgaaccat 300
   gcgtcagggg cctagaggtg gagttcttag ctatccttgg ctttctgtgc cagcctggct 360
   ctgcccctcc cccatgggct gtgtcctaag gcccatttga gaagctgagg ctagttccaa 420
   aaacctctcc tg 432
<210> 70
   <211> 450
   <212> DNA
   <213> Homo sapiens
   <400> 70
   gaatttcttg gtgattacag gtgggatcca actgcaaatg aagatccaga atggatactt 60
   gttgagaaag acagattcgt gaatgattat gacaaagata acgatggcag gcttgatccc 120
   caagagctgt taccttgggt agtacctaat aatcagggca ttgcacaaga ggaggcactt 180
   catctaattg atgaaatgga tttgaatggt gacaaaaagc tctctgaaga agagattctg 240
   gaaaacccgg acttgtttct caccagtgaa gccacagatt atggcagaca gctccatgat 300
   gactatttct atcatgatga gctttaatct ccgagcctgt ctcagtagag tactggctcc 360
   ttttataatt tgttaccagc tttacttttg tgataaaata ttgatgttgt attttacact 420
   cttaagtctt aaccacagtc agaattatct 450
<210> 71
   <211> 477
   <212> DNA
   <213> Homo sapiens
   <400> 71
   gatatttttc caaacgtatt gagcaacaaa atattaatat tgtgccatat gacaacaaag 60
   tctttcctaa atactccatc tgtttagtac tgtattgtgg aatatttgag ttctatttcc 120
   agacttgaaa acatggagga ttttagagat gcctgaacaa tattatttaa gtagtatgtg 180
   accgagctat aaattttttg tttttgttct aagtagattt aatttgggaa ctgacaggac 240
   aatgttttta ggtttagcat tttgtttaaa aacctttaaa gaaaccttta gaaggactta 300
   gacctcacat attaatgttg agaagttctg cttaatttta aaatggtttc tataaagggt 360
   tttattgtat gaaatagaac tttatatttt tgcatatgta tagaggataa ttatatttaa 420
   tgtataacta tagcattatg gtgagtggaa tttgacattg tccaaacctt tttcatt 477
<210> 72
   <211> 497
   <212> DNA
   <213> Homo sapiens
   <220>
   <221> misc_feature
   <222> (432)..(432)
   <223> n is a, c, g, or t
   <400> 72
   gatttagctc ttagttcttc aagtaaaatt aaagtctctt gtgtaagagc caacacatgc 60
   ccagctgcgg atgggagctg ttcctggaca gccttctact gcctgggaag tgatggaaca 120
   ggaactcagg gtgcccttac cccctcccca gacctgttcc ctttctttga ctgacagagc 180
   accatccagg caaaattaga gcgccaaatg gttttcttct caatcttaaa gcagtatacc 240
   tttccacagg ctcgtctgtg tccctgccac tctgagttat ccagaaacca ccacctacaa 300
   atgaggggac tcatctagaa gacctctaag gtcccctttt ggctctgagg ggtctctaat 360
   aatccccact tggaattcag caccgcaagg aaattatggg tatgtgagcc ataatatgat 420
   ggccagcagg tngcgctgcc ttccacccat ggtgatggat ggtttggaaa gggaatgttg 480
   gtgccttttg tgccaca 497
<210> 73
   <211> 481
   <212> DNA
   <213> Homo sapiens
   <400> 73
   gatgataatc cggaccatgc tgtatactcc acaggaaatg aaacagatca ttaaaatccg 60
   tgcccagacg gaaggaatca acatcagtga ggaggcactg aaccacctgg gggagattgg 120
   caccaagacc acactgaggt actcagtgca gctgctgacc ccggccaact tgcttgctaa 180
   aatcaacggg aaggacagca ttgagaaaga gcatgtcgaa gagatcagtg aacttttcta 240
   tgatgccaag tcctccgcca aaatcctggc tgaccagcag gataagtaca tgaagtgaga 300
   tggctgaggt tttcagcagc aagagactcc ccaggtgtgc ctggcctggg tccagcctgt 360
   gggcgcttgc ccctgggctt ggggctgccg tccccactca ggcgtgggct gcagcgctgt 420
   cagttcagtg tggaaagcat ttctttttaa gttatcgtaa ctgttcctgt ggttgctttg 480
   a 481
<210> 74
   <211> 469
   <212> DNA
   <213> Homo sapiens
<220>
   <221> misc_feature
   <222> (40)..(40)
   <223> n is a, c, g, or t
   <400> 74
   gacatccttc ttagcagaaa cttcatgaaa aagtttttgn ctgacacaga acaaacctga 60
   aagtagtatc tactttctaa atactacttt gcttttcagt agtggatttg atatttataa 120
   tgttctctaa agcttgcaac tttttcagca acgtttaaaa atagattaac ctggaataac 180
   ttacttgttt gctgctaaaa tactcaagat tttgccattt ttaaacaacc agtccctgtg 240
   atacaacttt gaaaaaactt ttaaaaatct ctgatgtatg ggctcttttt ttcccataag 300
   aattatgtac atctgtgatg ttttacaggg ggatccgctt ttaaacagtg tacatattgg 360
   accacactga aatgtcatat atcctttctc tacttaaaat tggttattta ctgtgagttc 420
   atttccgatg tgttcttggt tgttgctgtt ttctgcctga agacgtgta 469
<210> 75
   <211> 455
   <212> DNA
   <213> Homo sapiens
   <400> 75
   caaagtctcc ttttagtcta gataatcatt atttcatttt aaaattagtg tttttcatag 60
   tttgcactga tgcgtgtatg gatgtgtgtg agtcagtggt agcttattta aaaagcacct 120
   tatcctttct cccataacct ttgtacacta aaaaatgaaa gaatttagaa tgtatttgat 180
   gatagcattc tcactaagac acatgagaat ttaactttat aaccgcgtga gttaagattt 240
   aattcatagg ttttgatgtc attgttgaag ttatttgtaa ttcagaaacc ttgcttgtgt 300
   gatacatagt aagtctcttc atttattact gcttgcctgt tgttatatct ggattatcaa 360
   aagcaatagt gcaccaatta agatgtgctc aaatcaggac ttaaatcata ggcaccacat 420
   ttttcatgtc agactagtta ctttgttgat tctca 455
<210> 76
   <211> 525
   <212> DNA
   <213> Homo sapiens
   <400> 76
   tctggcatca gtttgctaca gtgagctcac atcaaatagg aaaatacttg aaatgcatgt 60
   ctcaagctgc aaggcaaact ccattcctca tattaaacta ttacttctca tgacgtcacc 120
   atttttaact gacaggatta gtaaaacatt aagacagcaa acttgtgtct gtctcttctt 180
   tcattttccc cgccaccaac ttactttacc acctatgact gtacttgtca gtatgagaat 240
   ttttctgaat catattgggg aagcagtgat tttaaaacct caagttttta aacatgattt 300
   atatgttctg tataatgttc agtttgtaac tttttaaaag tttggatgta tagagggata 360
   aataggaaat ataagaattg gttatttggg ggctttttta cttactgtat ttaaaaatac 420
   aagggtattg atatgaaatt atgtaaattt caaatgctta tgaatcaaat cattgttgaa 480
   caaaagattt gttgctgtgt aattattgtc ttgtatgcat ttgag 525
<210> 77
   <211> 397
   <212> DNA
   <213> Homo sapiens
   <400> 77
   ggagaacttg tctacaacca gggattgatt ttaaagatgt ctttttttat tttacttttt 60
   tttaagcacc aaattttgtt gttttttttt tctcccctcc ccacagatcc catctcaaat 120
   cattctgtta accaccattc caacaggtcg aggagagctt aaacaccttc ttcctctggc 180
   cttgtttctc ttttattttt tattttttcg catcagtatt aatgtttttg catactttgc 240
   atctttattc aaaagtgtaa actttctttg tcaatctatg gacatgccca tatatgaagg 300
   agatgggtgg gtcaaaaagg gatatcaaat gaagtgatag gggtcacaat ggggaaattg 360
   aagtggtgca taacattgcc aaaatagtgt gccacta 397
<210> 78
   <211> 329
   <212> DNA
   <213> Homo sapiens
   <400> 78
   ctcttcgaga gaacctgtcg ccagtatgac aagctgcgta agcgggaggc cttcctggag 60
   cagttccgca aggaggacat gttcaaggac aactttgatg agatggacac atccagggag 120
   attgtgcagc agctcatcga tgagtaccat gcggccacac ggccagacta catctcctgg 180
   ggcacccagg agcagtgagt cccccaggac aggggaccct catctgcctt actggttggc 240
   ccaagccctg cctgactgac caccccctca gagcacagat cagggacctc acgcatctct 300
   ttctcatata catggactct ctgttggcc 329
<210> 79
   <211> 535
   <212> DNA
   <213> Homo sapiens
   <400> 79
   ggagctggaa ctggtcacca aggccggctt ccgggccctt ctctctgccc cctggtacct 60
   gaaccgtata tcctatggcc ctgactggaa ggatttctac gtagtggaac ccctggcatt 120
   tgaaggtacc cctgagcaga aggctctggt gattggtgga gaggcttgta tgtggggaga 180
   atatgtggac aacacaaacc tggtccccag gctctggccc agagcagggg ctgttgccga 240
   aaggctgtgg agcaacaagt tgacatctga cctgacattt gcctatgaac gtttgtcaca 300
   cttccgctgt gagttgctga ggcgaggtgt ccaggcccaa cccctcaatg taggcttctg 360
   tgagcaggag tttgaacaga cctgagcccc aggcaccgag gagggtgctg gctgtaggtg 420
   aatggtagtg gagccaggct tccactgcat cctggccagg ggacggagcc ccttgccttc 480
   gtgccccttg cctgcgtgcc cctgtgcttg gagagaaagg ggccggtgct ggcgc 535
<210> 80
   <211> 537
   <212> DNA
   <213> Homo sapiens
   <400> 80
   ccaccgctgg ctgggaggag tcggagactg agacctacac agaggtggtg acagagtttg 60
   ggaccgaggt ggagcccgag tttgggacca aggtggagcc cgagtttgag acccagttgg 120
   agcctgagtt cgagacccag ctggaacccg agtttgagga agaggaggag gaggagaaag 180
   aggaggagat agccactggc caggcattcc ccttcacaac agtagagacc tacacagtga 240
   actttgggga cttctgagat cagcgtccta ccaagacccc agcccaactc aagctacagc 300
   agcagcactt cccaagcctg ctgaccacag tcacatcacc catcagcaca tggaaggccc 360
   ctggtatgga cactgaaagg aagggctggt cctgcccctt tgagggggtg caaacatgac 420
   tgggacctaa gagccagagg ctgtgtagag gctcctgctc cacctgccag tctcgtaaga 480
   gatggggttg ctgcagtgtt ggagtagggg cagagggagg gagccaaggt cactcca 537
<210> 81
   <211> 483
   <212> DNA
   <213> Homo sapiens
   <400> 81
   ctgaagcgca gaaagctcgg ccggtacaac gaggaggagc gggctcagca ggaggccgag 60
   gccgcccagc gcctggccga ggagaaggcc caggccagct ccatccccgt gggcagccgc 120
   tgtgaggtgc gggcggcggg acaatcccct cgccggggca ccgtcatgta tgtaggtctc 180
   acagatttca agcctggcta ctggattggt gtccgctatg atgagccact ggggaaaaat 240
   gatggcagtg tgaatgggaa acgctacttc gaatgccagg ccaagtatgg cgcctttgtc 300
   aagccagcag tcgtgacggt gggggacttc ccggaggagg actacgggtt ggacgagata 360
   tgacacctaa ggaattcccc tgcttcagct cctagctcag ccactgactg cccctcctgt 420
   gtgtgcccat ggcccttttc tcctgacccc attttaattt tattcatttt ttcctttgcc 480
   att 483
<210> 82
   <211> 505
   <212> DNA
   <213> Homo sapiens
   <400> 82
   caaggtgaaa cactgcagtc ccggtgtggt ggctccccat gcaggacggg ccaggctggg 60
   agtgccgcct tcctgtgcca aattcagtgg ggactcagtg cccaggccct ggcacgagct 120
   ttggccttgg tctacctgcc aggccaggca aagcgccttt acacaggcct cggaaaacaa 180
   tggagtgagc acaagatgcc ctgtgcagct gcccgagggt ctccgcccac cccggccgga 240
   ctttgatccc cccgaagtct tcacaggcac tgcatcgggt tgtctggcgc ccttttcctc 300
   cagcctaaac tgacatcatc ctatggactg agccggccac tctctggccg aagtggcgca 360
   ggctgtgccc ccgagctgcc cccaccccct cacagggtcc ctcagattat aggtgcccag 420
   gctgaggtga agaggcctgg gggccctgcc ttccgggcgc tcctggaccc tggggcaaac 480
   ctgtgaccct tttctactgg aatag 505
<210> 83
   <211> 299
   <212> DNA
   <213> Homo sapiens
   <400> 83
   tggccatccg ggacagtgag cgacagggca aggcccaggt ggagattgtc actgatgggg 60
   aggagcctgc tgagatgatc caggtcctgg gccccaagcc tgctctgaag gagggcaacc 120
   ctgaggaaga cctcacagct gacaaggcaa atgcccaggc cgcagctctg tataaggtct 180
   ctgatgccac tggacagatg aacctgacca aggtggctga ctccagcccc tttgcccttg 240
   aactgctgat atctgatgac tgctttgtgc tggacaacgg gctctgtggc aagatctat 299
<210> 84
   <211> 533
   <212> DNA
   <213> Homo sapiens
<220>
   <221> misc_feature
   <222> (136)..(136)
   <223> n is a, c, g, or t
   <400> 84
   gaaaagtgca tgcttcattt gaacaattca ttcagcagca gatggacttt cagtgattta 60
   aaataaaatt ttgatccaaa gctcaggaca caaaccacag tggtaaaatt gagtagcata 120
   taatatcaga ctaaanttat ctgtaatttt ccacaaccca gattgtatgt gttttatgtg 180
   tgtttaaata aatatgttag atacacgtgt atacatacac ccatatacaa cagatccaag 240
   actggctgac ttcatttgaa atggttgaat ctgctgtgta ataaagtggt tcaaccatga 300
   ttaggaactg aaatttagta gaagagggaa aaggagttaa tgtaacaaat tattttagct 360
   acaaaccccg gtaatagagc acttggggga tgggatgggg tgggttggtg agacaatcag 420
   aatggtaaat tgattaaatg ctcctaaccc tgtaattttg tgcatagagc accctatgct 480
   gtggaaataa ctgttcttag atttcattgt aactggactg ttcaggttgc cca 533
<210> 85
   <211> 403
   <212> DNA
   <213> Homo sapiens
<220>
   <221> misc_feature
   <222> (117)..(117)
   <223> n is a, c, g, or t
   <220>
   <221> misc_feature
   <222> (119)..(119)
   <223> n is a, c, g, or t
   <220>
   <221> misc_feature
   <222> (339)..(339)
   <223> n is a, c, g, or t
   <400> 85
   gaaactgcgc attctctagt agtatatatc gtgcctgtct tcaaaaacat ttcccttttt 60
   atactcattc cccccaggca tggggtagtg tcagtcggac tgcacaggga acacggntnc 120
   cagtggcttt ggcccctact cgggaaacgt ctgcctgttc tcgatggtga tggggtggct 180
   gccattccct tggttttcct aagccctttc taacgagagt ctcaaacaag cggaggcgag 240
   ggccaattca accccattct ttccagcgcc ccgcaccata gcacctgccc acctgagaac 300
   caggaacgca ccctctctgt ggagctctga ctggtgtanc tggaaacaaa cagcaacttg 360
   caaacggacg aagagcctgc cgtgtgttaa tcatttgcct tac 403
<210> 86
   <211> 441
   <212> DNA
   <213> Homo sapiens
   <400> 86
   gttgtctgga aacctgctga ggaaattcaa aaacagcaac gggtggcaga agctgtgggt 60
   ggtgttcaca aacttctgcc tgttcttcta caaatcacac caggacaatc atccccttgc 120
   cagcctgcct ctgctcggct actcgctcac catcccctct gagtccgaga acatccagaa 180
   agactacgtg ttcaagctgc acttcaagtc ccacgtctac tacttcaggg cggaaagcga 240
   gtacacgttc gaaaggtgga tggaagtgat ccgcagtgcc accagctctg cctcgcgacc 300
   ccacgtgttg agccacaaag agtctcttgt gtattgatgg ccggacacac tcgtttccgc 360
   agtggctgct ttcctggaag acgtttcctt tcttctgtat taatgaagcc tggtaaaatt 420
   aacacctgtc tgaaaatcaa a 441
<210> 87
   <211> 467
   <212> DNA
   <213> Homo sapiens
   <400> 87
   tatatgactt ggcagatcaa ctacatgctg cagttggtgc ttcccgtgct gctgttgatg 60
   ctggctttgt tcccaatgac atgcaagttg gacagacggg aaaaatagta gcaccagaac 120
   tttatattgc tgttggaata tctggagcca tccaacattt agctgggatg aaagacagca 180
   agacaattgt ggcaattaat aaagacccag aagctccaat tttccaagtg gcagattatg 240
   gaatagttgc agatttattt aaggtagttc ctgaaatgac tgagatattg aagaaaaaat 300
   gaatcaggat catgccttaa aaagaaaact tttgttaaag tattccactg aaatcacaga 360
   tatttgtggg tattataaca atcattggaa agcatggaga gctacatttc ataatttgag 420
   ggaaaatttc taacagatgc cagaatgctt gtttatggga ttgctgt 467

<210> 88
   <211> 527
   <212> DNA
   <213> Homo sapiens
   <400> 88
   cagacaacag cctggtggca gcgggccacg actgcttccc ggtgctgttc acctatgacg 60
   ccgccgcggg gatgctgagc ttcggcgggc ggctggacgt tcctaagcag agctcgcagc 120
   gtggcttgac ggcccgcgag cgcttccaga acctggacaa gaaggcgagc tccgagggtg 180
   gcacggctgc gggcgcgggc ctagactcgc tgcacaagaa cagcgtcagc cagatctcgg 240
   tgctcagcgg cggcaaggcc aagtgctcgc agttctgcac cactggcatg gatggcggca 300
   tgagtatctg ggatgtgaag agcttggagt cagccttgaa ggacctcaag atcaaatgac 360
   ctgtgaggaa tatgttgcct tcatcctaac tgctggggaa gcggggagag gggtcaggga 420
   ggctaatggt tgctttgctg aatgtttctg gggtaccaat acgagttccc ataggggctg 480
   ctccctcaaa aagggagggg acagatgggg agcttttctt acctatt 527
<210> 89
   <211> 546
   <212> DNA
   <213> Homo sapiens
   <400> 89
   acacgtgttg actccattgt tttacatgta gcaaagtctg ccatctgtgt ctgctgtatt 60
   ataaacagat aagcagccta caagataact gtatttataa accactcttc aacagctggc 120
   tccagtgctg gttttagaac aagaatgaag tcattttgga gtctttcatg tctaaaagat 180
   ttaagttaaa aacaaagtgt tacttggaag gttagcttct atcattctgg atagattaca 240
   gatataataa ccatgttgac tatgggggag agacgctgca ttccagaaac gtcttaacac 300
   ttgagtgaat cttcaaagga ccctgacatt aaatgctgag gctttaatac acacatattt 360
   tatcccaagt ttataatggt ggtctgaaca aggcacctgt aaataaatca gcatttatga 420
   ccagaagaaa aataatctgg tcttggactt tttattttta tatggaaaag ttttaaggac 480
   ttgggccaac taagtctacc cacacgaaaa aagaaatttg ccttgtccct ttgtgtacaa 540
   ccatgc 546
<210> 90
   <211> 464
   <212> DNA
   <213> Homo sapiens
   <400> 90
   cagtcactct aaatggacac cacatgaacc tctgtttaga atacctacgt atgtatgcat 60
   tggtttgctt gtttcttgac agtacatttt tagatctggc cttttcttaa caaaatctgt 120
   gcaaaagatg caggtggatg tccctaggtc tgttttcaaa gaactttttc caagtgcttg 180
   ttttatttat taagtgtcta cctggtaaat gttttttttg taaactctga gtggactgta 240
   tcatttgcta ttctaaacca ttttacactt aagttaaaat agtttctctt cagctgtaaa 300
   taacaggata cagaattaac aagagaaaat gtctaacttt ttaagaaaaa ccttattttc 360
   ttcggttttt gaaaaacata atggaaataa aacaggatat tgacataata gcacaaaatg 420
   acactcttct aaaactaaat gggcacaaga gaattttcct ggga 464
<210> 91
   <211> 409
   <212> DNA
   <213> Homo sapiens
   <400> 91
   atcccaaagc accaattact gccctctgcc tcagcagtac cagtataaga tgacattcca 60
   aagactggag gcaactcagc ctgagttaat tcacaaaatt atgccatgct ggggcttgag 120
   cttgagcttg ggcttaggct tgggctcagc ttttgaccct caggcatctc cttttccttc 180
   ctgtcttcct ctcccttctc ctctgctgca gcatgatttt cttaatcttc agacactcac 240
   tattttcatg aacagttacc ctctgtcccc acaaccaaag acaactcatg gcctcctttg 300
   gcccttgtgt aacattgcaa acctgtggct ttgcaaaatg tacccaggtc acaaggggat 360
   tttttttttt ttagcaatga tatccctgtc tgggtcactt tttaagctt 409
<210> 92
   <211> 481
   <212> DNA
   <213> Homo sapiens
   <400> 92
   ggcctctcca tagttatcgg ggatctgctc cggcagatcc ccctggccgt gctctttgga 60
   attttcctgt acatgggagt cacctcccct aacgggatcc agttctatga gcggctgcat 120
   ctgctgctca tgccgcccaa acaccaccca gatgtcactt acgtcaagaa ggtccggacc 180
   ctccgtatgc acctgttcac ggccctgcag ctgctctgcc tggccctgct ctgggccgtc 240
   atgtccacag ctgcctccct ggccttcccc ttcatcctca tcctcacagt gccgctccgc 300
   atggtggtgc tcacccgtat cttcaccgac cgagagatga aatgtctgga tgctaacgag 360
   gcagagccgg tgtttgatga gcgggagggt gtggacgagt acaatgagat gcccatgcct 420
   gtgtagccgc caccgaggga cagccgaggg accgatggac gaggggacag gctggtggga 480
   t 481
<210> 93
   <211> 393
   <212> DNA
   <213> Homo sapiens
   <400> 93
   acagcacggc catccaggag ctgttcaagc gcatctccga gcagttcacg gccatgttcc 60
   ggcgcaaggc cttcctgcac tggtacacgg gcgagggcat ggacgagatg gagttcaccg 120
   aggccgagag caacatgaac gacctggtgt ccgagtacca gcagtaccag gacgccacgg 180
   ccgaggaaga gggcgagatg tacgaagacg acgaggagga gtcggaggcc cagggcccca 240
   agtgaaactg ctcgcagctg gagtgagagg caggtggcgg ccggggccga agccagcagt 300
   gtctaaaccc ccggagccat cttgctgccg acaccctgct ttccccatcg ccctagggct 360
   cccttgccgc cctcctgcag tatttatggc ctc 393
<210> 94
   <211> 564
   <212> DNA
   <213> Homo sapiens
   <400> 94
   accaaggcgc gggcggtgat gaactttgtg gttcgctacc ggccagacga gcagccgtct 60
   ctgcggccac accacgactc atccaccttc accctcaacg ttgccctcaa ccacaagggc 120
   ctggactatg agggaggtgg ctgccgcttc ctgcgctacg actgtgtgat ctcctccccg 180
   aggaagggct gggcactcct gcaccccggc cgcctcaccc actaccacga ggggctgcca 240
   acgacctggg gcacacgcta catcatggtg tcctttgtcg acccctgaca ctcaaccact 300
   ctgccaaacc tgccctgcca ttgtgccttt ttagggggcc tggcccccgt cctgggagtt 360
   gggggatggg tctctctgtc tccccacttc ctgagttcat gttccgcgtg cctgaactga 420
   atatgtcacc ttgctcccaa gacacggccc tctcaggaag ctcccggagt ccccgcctct 480
   ctcctccgcc cacaggggtt cgtgggcaca gggcttctgg ggactccccg cgtgataaat 540
   tattaatgtt ccgcagtctc actc 564
<210> 95
   <211> 474
   <212> DNA
   <213> Homo sapiens
   <400> 95
   tttgtggact ccacgttcta tcttcttttg gacttgatca ccttttttga cgagtatcat 60
   agtggtcata ttgatagagc ttttgatatc attgagcgct tgaagctggt gcccctgaat 120
   caggaaagtg tggaagagag agtggctgct ttcagaaatt tcagtgatga aatcaggcac 180
   aacctctcag aagtgcttct tgccaccatg aacatcttgt tcacacagtt taagaggctc 240
   aaggggacaa gtccatcctc gtcatccagg ccccagcgag tcatcgagga ccgcgactct 300
   caactccgaa gtcaagcccg cactctgatt acctttgctg gaatgatacc ataccgaacg 360
   tctggggaca ccaatgcgag gctggtgcag atggaggtcc tcatgaatta agtgccatgc 420
   tttgtgggag tctgggtcgg cacactgtca gtacatcagg cacatgggcc cact 474
<210> 96
   <211> 448
   <212> DNA
   <213> Homo sapiens
   <400> 96
   aagcttcgag ctgttgcgtg tgtgagtctg ttgtgtggat gtgcgtgtgt ggtccccagc 60
   cccagactgg attggaaaag tgcatggtgg gggcctcggg gctgtcccca cgctgtccct 120
   ttgccacaag tctgtggggc aagaggctgc aatattccgt cctgggtgtc tgggctgcta 180
   acctggcctg ctcaggcttc ccaccctgtg cggggcacac ccccaggaag ggaccctgga 240
   cacggctccc acgtccaggc ttaaggtgga tgcacttccc gcacctccag tcttctgtgt 300
   agcagcttta acccacgttt gtctgtcacg tccagtcccg agacggctga gtgaccccaa 360
   gaaaggcttc cccgacaccc agacagaggc tgcagggctg gggctgggtg agggtggcgg 420
   gcctgcgggg acattctact gtgctaaa 448
<210> 97
   <211> 271
   <212> DNA
   <213> Homo sapiens
   <400> 97
   tcacccttct acagcagcta actagagtcc taactaatgg gatccagcag ggccatttct 60
   ccagagggcc agtatcctat taggagactc ttggaattct taggttctac tcaagagtgg 120
   aaggaccaat cacctctgat attctgtgga aggttttggg gtcaaattct gccctctgca 180
   ttctgtgcaa cttgtataaa agtcaagtta gtattacatg aatttggggt agggttagtg 240
   ctttgaaaaa atgttgaacc ggctgggcgc g 271
<210> 98
   <211> 344
   <212> DNA
   <213> Homo sapiens
<220>
   <221> misc_feature
   <222> (106)..(106)
   <223> n is a, c, g, or t
   <220>
   <221> misc_feature
   <222> (108)..(113)
   <223> n is a, c, g, or t
   <400> 98
   gatactgtaa agtccacaca cacattaaat cttgttttcc tgaaagtatg gcatcaaaaa 60
   tacttgtaga aaaaccttgt cacaactgat ttgaatgttc ctattntnnn nnnctttgac 120
   tttgatattg gcttgtaatg tctcttttca tcatatgtaa tatcagtgga acaggcagcg 180
   ctactcaagt cctaaggatt cctcagtgat cagtgatcca gggccgttca tgaaccactg 240
   ggctggattt gactgttgag tgtggcagtt aatgcccctc aagaaatcaa aggatgtctt 300
   ataagtgtct tccaaaaaaa agcaaatgct gaaatcctat tggc 344
<210> 99
   <211> 497
   <212> DNA
   <213> Homo sapiens
   <400> 99
   ctcctgcagg ccatgtgtgt attacttgtc tagtgatgtc ctctcaaagt gctgtacgcg 60
   agctcggcgc cacctccgcc tccctttcag agcctgctcc ccgccctctc tgctcgctgc 120
   attgtggtgt tctcttctca aggctttgaa atctcccctt gcactgagat tagtcgtcag 180
   atctctcccc gtctccctcc caacttatac gacctgattt ccttaggacg gaaccgcagg 240
   cacctgcgcc gggcgtctta ctcccgctgc ttgttctgtc ccctccctcg gaccaaacag 300
   tgctcatgct tcaggacctt gtttgtcgaa gatgttggtt tccctttctc tgttatttat 360
   ataaaaataa tttatcaaaa ggatatttta aaaaagctag tctgtcttga aacttgttta 420
   ccttaaaatt atcagaatct cagtgtttga aagtactgaa gcacaaacat atatcatctc 480
   tgtaccattc tgtacta 497
<210> 100
   <211> 540
   <212> DNA
   <213> Homo sapiens
   <400> 100
   tagaacgggc atctactcca gtacttcctg ccataaaact ccagataaag taaaccatgc 60
   agtactggct gttgggtatg gagaaaaaaa tgggatccct tactggatcg tgaaaaactc 120
   ttggggtccc cagtggggaa tgaacgggta cttcctcatc gagcgcggaa agaacatgtg 180
   tggcctggct gcctgcgcct cctaccccat ccctctggtg tgagccgtgg cagccgcagc 240
   gcagactggc ggagaaggag aggaacgggc agcctgggcc tgggtggaaa tcctgccctg 300
   gaggaagttg tggggagatc cactgggacc cccaacattc tgccctcacc tctgtgccca 360
   gcctggaaac ctacagacaa ggaggagttc caccatgagc tcacccgtgt ctatgacgca 420
   aagatcacca gccatgtgcc ttagtgtcct tcttaacaga ctcaaaccac atggaccacg 480
   aatattcttt ctgtccagaa gggctacttt ccacatatag agctccaggg actgtctttt 540
<210> 101
   <211> 329
   <212> DNA
   <213> Homo sapiens
   <400> 101
   gccactcgcc ttcttagagt tttattcctt tccttttttg agattttttt tccgtgtgtt 60
   tattttttat tatttttcaa gataaggaga aagaaagtac ccagcaaatg ggcattttac 120
   aagaagtacg aatcttattt ttcctgtcct gcccgtgagg tgggggggac cgggcccctc 180
   tctagggacc cctcgcccca gcctcattcc ccattctgtg tcccatgtcc cgtgtctcct 240
   cggtcgcccc gtgtttgcgc ttgaccatgt tgcactgttt gcatgcgccc gaggcagacg 300
   tctgtcaggg gcttggattt cgtgtgccg   329
<210> 102
   <211> 540
   <212> DNA
   <213> Homo sapiens
   <400> 102
   cccggccagg ctaagccgca gagaccctct cagcccccac ctcaggttag ggctctgccc 60
   gcagcctgac ctctagccct ggtggcagag gtccctcagc tgcgaggcta attgggtgac 120
   caccgattcc agctgcggtt aatccagctt gggcctgtct gcactgcgat cctcttgggc 180
   tctcctagga tccccccatg ccccgtaaga ggtggaagac gcttccttcc aggacagcag 240
   gctttggagt ccgacacccc cagcctgcct ttgccaccag ccccaaccct gcagagatat 300
   gaggcttgac agagtctgcc ccctccccca ctgcacccca agagagagag ccccagccag 360
   cggaacagtt tctattaccc cctccctgcc cccagaccca tgtgatttct gctttcttct 420
   ttagcaagat attctggttt ctagataagg aagagtctct aatgagcccc cgagccccag 480
   tctcttcaga ctcatggatt ggtctgaggg gtctgaacgt ctcctagcca atcagaactg 540
<210> 103
   <211> 513
   <212> DNA
   <213> Homo sapiens
   <400> 103
   ggtgttgtac agctcacatg tttacacact cagtgcccta atttcccctg agggaatcgc 60
   tttttaagtg atccttacag tggtgtttta tgttacttta ttacagagct ccttggtttt 120
   ttacttctgc acttaaattt ttttaaataa catgatgatg gtacattttc ctctattgtc 180
   tagctaaggg ctttcggtcc accagtaaat aagatcaaat gctcttaaat gttcctgtta 240
   ccatcctaat gtaaatactg gatttttctg tcatttagca ccatgctgct tctgtctgtc 300
   ttaatgctgg cattaagatc atgagccctt tttctccagt agtacaggct ttgaaaacta 360
   cttctattaa gttattgatg caatttgata ttttttcata atctatattt aaacaaaatt 420
   acatcattgc atcatctttt ctaaattcat ctccattaaa acttgcctta agctaccaga 480
   ttgcttttgc caccattggc catactgtgt gtt 513
<210> 104
   <211> 529
   <212> DNA
   <213> Homo sapiens
   <400> 104
   attacggctt ttctattgct gtatgataca gaactctttt ggcataaata tttgtgttcc 60
   cagtacctca cttgttcgga tttgactgcc tgtatatgtt ttgtgaaatg gtcctgtttt 120
   tgggtaggtg acacgtggac tctagtatgt aaatgttact tgaatctgtg cttcataata 180
   gtgtgtggca tgtatgtgca gactcttgga tgctttatgc ctgcgcacca ggagccctgt 240
   cctcacgttc ccaggagggc ggcttcaccc ttcgtaacca ggagacaagg cggccatgga 300
   tttgcccttg attctatttt gctaatggaa gatagaaagg agagaaggtt tttttttttt 360
   tttaacattc tgaagatggt gctgtgtcaa gaaggacctt ttttttcccc tctcccctat 420
   tttttaagta ccttggagga ggagaggttg gtgacatgca tggtggggat ctatggcctc 480
   tggtgctttg tcctgtattt ggtttaatgt ttttgtccta atctcttca 529
<210> 105
   <211> 524
   <212> DNA
   <213> Homo sapiens
   <400> 105
   tggagaattc tttaggttgt cccctaaaga ttctgaaaaa gagaatcaga ttcctgaaga 60
   ggcaggaagc agtggcttag gaaaagcaaa gagaaaagca tgtcctttgc aacctgatca 120
   cacaaatgat gaaaaagaat agaactttct cattcatctt tgaataacgt ctccttgttt 180
   accctggtat tctagaatgt aaatttacat aaatgtgttt gttccaatta gctttgttga 240
   acaggcattt aattaaaaaa tttaggttta aatttagatg ttcaaaagta gttgtgaaat 300
   ttgagaattt gtaagactaa ttatggtaac ttagcttagt attcaatata atgcattgtt 360
   tggtttcttt taccaaatta agtgtctagt tcttgctaaa atcaagtcat tgcattgtgt 420
   tctaattaca agtatgttgt atttgagatt tgcttagatt gttgtactgc tgccattttt 480
   attggtgttt gattattgga atggtgccat attgtcactc cttc 524
<210> 106
   <211> 532
   <212> DNA
   <213> Homo sapiens
   <400> 106
   aaagctcagg attcttcgaa aagttgagaa aattgatgac ttcaaagctg aagactttca 60
   gattgaaggg tacaatccgc atccaactat taaaatggaa atggctgttt agggtgcttt 120
   caaaggagct tgaaggatat tgtcagtctt taggggttgg gctggatgcc gaggtaaaag 180
   ttctttttgc tctaaaagaa aaaggaacta ggtcaaaaat ctgtccgtga cctatcagtt 240
   attaattttt aaggatgttg ccactggcaa atgtaactgt gccagttctt tccataataa 300
   aaggctttga gttaactcac tgagggtatc tgacaatgct gaggttatga acaaagtgag 360
   gagaatgaaa tgtatgtgct cttagcaaaa acatgtatgt gcatttcaat cccacgtact 420
   tataaagaag gttggtgaat ttcacaagct atttttggaa tatttttaga atattttaag 480
   aatttcacaa gctattccct caaatctgag ggagctgagt aacaccatcg at 532
<210> 107
   <211> 402
   <212> DNA
   <213> Homo sapiens
   <400> 107
   gtacatgaaa ccccagatag actataaata attctaaaca aacaagtagg tagatatgta 60
   tgtaattgct tttaaatcat ttaaatgcct ttgtttttgg actgtgcaaa ggttggaagt 120
   gggtttgcat ttctaaaatg gtgactttta ttctgcaaga gttcttagta acttcttgag 180
   tgtggtagac tttggaacat gtaaattttt tgcttgtaat gttatcctgt ggtaggattt 240
   tggcaggtac acacactgcc ctattttatt ttgagtctaa gttaaatgtt ttctgaaaag 300
   agatacatgc actgaactct ttccactgcg aatcaagatg tggtaatata aaaggatcaa 360
   gacaaatgag atctaatact actgtcagtt ttaatgtcca ct 402
<210> 108
   <211> 504
   <212> DNA
   <213> Homo sapiens
   <400> 108
   gccactacac ttcttaaggc gagcatcaaa agccggggag gttgatgttg aacagcacac 60
   tttagccaag tatttgatgg agctgactct catcgactat gatatggtgc attatcatcc 120
   ttctaaggta gcagcagctg cttcctgctt gtctcagaag gttctaggac aaggaaaatg 180
   gaacttaaag cagcagtatt acacaggata cacagagaat gaagtattgg aagtcatgca 240
   gcacatggcc aagaatgtgg tgaaagtaaa tgaaaactta actaaattca tcgccatcaa 300
   gaataagtat gcaagcagca aactcctgaa gatcagcatg atccctcagc tgaactcaaa 360
   agccgtcaaa gaccttgcct ccccactgat aggaaggtcc taggctgccg tgggccctgg 420
   ggatgtgtgc ttcattgtgc cctttttctt attggtttag aactcttgat tttgtacata 480
   gtcctctggt ctatctcatg aaac 504
<210> 109
   <211> 512
   <212> DNA
   <213> Homo sapiens
   <400> 109
   gaagaagcct ggcagacagg cgggcaaaca gtgagcgccc acccagaccg gctgctgcgc 60
   cccctcctgc cagggtggcg attccgctcc acagtctcgg acggatctgc tcagaaagga 120
   agaggcaggc gccaggggga acccccttcg tgttttgtga ccctcccttt taggtgaagc 180
   ccctttttct tgctaaaacc ggcaattctc cggttagaaa tgttacttgg tgttttttgg 240
   ttttgtgaaa cggccgtccc aaagctggct ggattcctag aagagtctgt gttgaaggca 300
   tctttcaacc cctcgctctg gttctcaggg cagcattttc caggcgggtt tgttttgcat 360
   ttcttggagc ctctccgagc agcaaccaga cgggagattt ttattttaag ctgttcatgc 420
   tgggactgac agcctgcagg gtttccttgg gcgcggcccc aaaattgcct tcaaaacaaa 480
   cccgggacgg ttgaaagcct tcgaaccgtg ca 512
<210> 110
   <211> 212
   <212> DNA
   <213> Homo sapiens
   <400> 110
   ccgaacgtgg gcgccaatgg cgagatctgc gtcaacgtgc tcaagaggga ctggacggct 60
   gagctgggca tccgacacgt actgctgacc atcaagtgcc tgctgatcca ccctaacccc 120
   gagtctgcac tcaacgagga ggcgggccgc ctgctcttgg agaactacga ggagtatgcg 180
   gctcgggccc gtctgctcac agagatccac gg 212

<210> 111
   <211> 337
   <212> DNA
   <213> Homo sapiens
   <400> 111
   cggacggaag atggcgtccg ccacccgtct catccagcgg ctgcggaact gggcgtccgg 60
   gcatgacctg caggggaagc tgcagctacg ctaccaggag atctccaagc gaactcagcc 120
   tcctcccaag ctccctgtgg gtcctagcca caagctctcc aacaattact attgcactcg 180
   cgatggccgc cgggaatctg tgcccccttc catcatcatg tcgtcgcaga aggcgctggt 240
   gtcaggcaag ccagcagaga gctctgctgt agctgccact gagaagaagg cggtgactcc 300
   agctcctccc ataaagaggt gggagctgtc ctcggac 337
<210> 112
   <211> 330
   <212> DNA
   <213> Homo sapiens
   <400> 112
   agccctacac atttgacatc aacctctctg ttaacctgaa aggagaagga atgagccagg 60
   cggctaccat atgcaagtcc aattttaagt acatgtactg gacgatgctg cagcagctca 120
   ctcaccactc tgtcaacggc tgcaacctgc ggccggggga cctcctggct tctgggacca 180
   tcagcgggcc ggagccagaa aacttcggct ccatgttgga actgtcgtgg aagggaacga 240
   agcccataga cctggggaat ggtcagacca ggaagtttct gctggacggg gatgaagtca 300
   tcataacagg gtactgccag ggggatggtt 330
<210> 113
   <211> 454
   <212> DNA
   <213> Homo sapiens
   <400> 113
   ggcctcttgc ctgtaaatag aagcccgcaa actgtacaga tttacagagg catcgagact 60
   gggccctggg agttgccatc tgagagccga tggccccagc atcccccagg tgcctgcctg 120
   gcaccacagt gaccctggcc tcagcgtggc aaatgcatgt aaatattttt cgtaggcagc 180
   gtggctccag agagccccct gaagacagtg tccctccctc ctgtgagtcc tttctcctgt 240
   acagaacctg cctggggtgg gtgggggtct gccattccct cccccaggcc ttccctgccc 300
   cttctctccc ctgtaacctg tttattaacc atacctgtcc tgagttcatg gccaaaacct 360
   taaataagaa aaacaaaaga aaaagacagt ggaaaaaaga gaccaaggcg cctgccccac 420
   tgcgggtact ctcctgttcc agccttgtga agga 454
<210> 114
   <211> 459
   <212> DNA
   <213> Homo sapiens
   <400> 114
   gccttccctg aatcagacaa ccttttcaaa tgggtaggga ccatccatgg agcagctgga 60
   acagtatatg aagacctgag gtataagctc tcgctagagt tccccagtgg ctacccttac 120
   aatgcgccca cagtgaagtt cctcacgccc tgctatcacc ccaacgtgga cacccagggt 180
   aacatatgcc tggacatcct gaaggaaaag tggtctgccc tgtatgatgt caggaccatt 240
   ctgctctcca tccagagcct tctaggagaa cccaacattg atagtccctt gaacacacat 300
   gctgccgagc tctggaaaaa ccccacagct tttaagaagt acctgcaaga aacctactca 360
   aagcaggtca ccagccagga gccctgaccc aggctgccca gcctgtcctt gtgtcgtctt 420
   tttaattttt ccttagatgg tctgtccttt ttgtgattt 459
<210> 115
   <211> 371
   <212> DNA
   <213> Homo sapiens
   <400> 115
   cactaagaaa atacctccct gggaggatga gctggggccc tttttctttt gctggatggt 60
   tcctttatgc agcttggccc tgtctaccga gatgcccatc tcttcctgcc tgctagcctg 120
   ctagaccctc aaactgggtg ggttctgtgt caataaaaag cttcaccccc tggctgagtg 180
   aggtggtccc ctgcaatcac tgtttgtccc ctacccaccc aacctgtccc tgcctgctcc 240
   cagcccactc atccttatgt gctagggata aatcaagagt cctcagcact ccacattccc 300
   aaaaaatccc aggaactcct aaaccttccc ctgtgacaga agatgaggtt ggcagctgat 360
   cagacctcaa t 371
<210> 116
   <211> 319
   <212> DNA
   <213> Homo sapiens
   <400> 116
   tggaggtcaa actgggggag ctgccaagct ggatcttgat gcgggacttc agtcctagtg 60
   gcattttcgg agcgtttcaa agaggttact accggtacta caacaagtac atcaatgtga 120
   agaaggggag catctcgggg attaccatgg tgctggcatg ctacgtgctc tttagctact 180
   ccttttccta caagcatctc aagcacgagc ggctccgcaa ataccactga agaggacaca 240
   ctctgcaccc ccccacccca cgaccttggc ccgagcccct ccgtgaggaa cacaatctca 300
   atcgttgctg aatcctttc 319
<210> 117
   <211> 352
   <212> DNA
   <213> Homo sapiens
   <400> 117
   gaagtgtcct ttatattacc agaaaatatg ggcttggcct aagtcgctgt ctcctaacct 60
   gccggggtca ttccccacca aacaccccat actaaggagc catgagccac ctggacattc 120
   accttttctt tgaccatctg gagtctgggg caacttaagg aggcaccaca cagtggtgca 180
   ggcacatttc caagcgtagg tgtccctggc ttttgtggcc aaagctagtg ttatggtcaa 240
   caacaggcca gggtctgtgg ggcactgacc ttgaaagtgg caaaatggag gtttcacagg 300
   ctgtgcggga gcaggacggc ttgcttcatc taacaatctc agtttccttt aa 352
<210> 118
   <211> 487
   <212> DNA
   <213> Homo sapiens
   <400> 118
   aaaagcactc tcatcagata tctgacataa ttagatacaa tataacattt tactaagttc 60
   agtattcatg ttttaaaggt gtttatactg atttgattgt gctggcaaat atactgtatt 120
   gttaatattg aactgtttat ttttctctta gtcttcttat ttaattaact tcattgccgc 180
   tggattctgt tcagccttta aaaatatttc ttagtggtca ttgctctgca gaactcaaaa 240
   agaaaattgt acttgttcat agacattttt aaagggttaa tttattgttc agccttatcc 300
   cttggcacgt aaacagacta ctagacttat tgtaggttcg tttgagcttt gtgttgtaaa 360
   attaaaaatg cttctataaa gttttcaagg tagggagtga ttttattatt gtgtatatct 420
   aatatattaa gtatgtgtga tactaaggtt tgactgctat aattatttgt actgttgatc 480
   acatgta 487
<210> 119
   <211> 476
   <212> DNA
   <213> Homo sapiens
   <400> 119
   cgtgaacgtc acccaggtat tcgtggacac cgtagggatg ccagagacat accaggcgca 60
   gctgcagcaa agttttcccg ggattgaggt gacggtcaag gccaaagcag atgccctcta 120
   cccggtggtt agtgctgcca gcatctgtgc caaggtggcc cgggaccagg ccgtgaagaa 180
   atggcagttc gtggagaaac tgcaggactt ggatactgat tatggctcag gctaccccaa 240
   tgatcccaag acaaaagcgt ggttgaagga gcacgtggag cctgtgttcg gcttccccca 300
   gtttgtccgg ttcagctggc gcacggccca gaccatcctg gagaaagagg cggaagatgt 360
   tatatgggag gactcagcat ccgagaatca ggagggactc aggaagatca catcctactt 420
   cctcaatgaa gggtcccaag cccgtccccg ttcttcccac cgatatttcc tggaac 476
<210> 120
   <211> 419
   <212> DNA
   <213> Homo sapiens
   <400> 120
   ctggcagctc ctctgagtgg ggagaggttg ggcagtgagt gagggacccc taatgcaggg 60
   actagaagcc tcagtttccc cattttaccc ttccacacaa tagcctctgt aggttaggct 120
   gccccatccc accctactct gtgtggctgc tttctttggt gccctcccct caccccactg 180
   tagctgtgac gtgttgtagt ttttagatgt ttgtaaaatg tttaaaaaaa tgttaaaagg 240
   aaaaaagtga aaataacaaa aaagaaaatc aaaattcacc ttcgtcatgc tgcgtccagt 300
   gccccaaccc tgtggtcact ctccccattt tgtaacactg taccaggtgg tgactgttta 360
   actctttggt gtctgtgctc aaaagactgc cttctccagt gcccagtgta tgagtgtgt 419
<210> 121
   <211> 438
   <212> DNA
   <213> Homo sapiens
   <400> 121
   gcccctggag tcgcggagaa agggccgtaa ccggaggacc cacgcccctg agcctcgcgc 60
   tgagcggggg ccgcgcagcg caacgcactg gtgaccagac tgtccccacg ccgggaacca 120
   agcaggagac gacaggcgag agaggagcca gacagaccct gaaaagaagg acgggttggg 180
   gccgggcaca ttgggggtca ccggccgatg gagacaccaa ccgacaggcc ctggctgagg 240
   gcagctgcgc gggcttattt attaacagga taacccttga atgtagcagc cccgggaggg 300
   cggcacaggt cgggcgcagg attcagccgg agggaaggga cggggaagcc gagctccaga 360
   gcaacgacca gggccgagga ggtgcctgga gtgcccaccc tgggagacag accccacctc 420
   cttgggtagt gagcagtg 438
<210> 122
   <211> 471
   <212> DNA
   <213> Homo sapiens
   <400> 122
   gattggtttc gacccaagct caactatcga gtgcccagcc ggggccataa actgactgtg 60
   accctgtcat gtggcagacc ttccatccga accacggctt gggaagacta catttggttc 120
   caggcaccag tgacatttaa aggcttccgc gagtgaatga gtgcttctta atcctaaaaa 180
   cacaatggct gaattatctt tctccatgtg gcgctgaatc acccatctgg tttggagcta 240
   gagttgcttc ctggtgagag aggaagcaac tctccttctg gttgtctgcc tcccctcaga 300
   tttcctgata ggctgatggc atgtggctgt gactgtgact gtaatcattg ctgaacaaca 360
   tctctttgaa tcaaaggttg attttcccag agggtgctgg gtcaggcatt tctattagga 420
   gttggaaagc aaaaatgggt ccatagacac tctatggagg tgtccctttc t 471
<210> 123
   <211> 475
   <212> DNA
   <213> Homo sapiens
   <400> 123
   gagtggcgag ctcataagcc ttagagagga ggtgacccac cttacccgct cacttcggcg 60
   tgcggagaca gagaccaaag tgctccagga ggcctggcag gccagctgga ctccaactgc 120
   cagcctatgg ccaccaattg gatccaggag aaagtgtggc tctctcagga ggtggacaaa 180
   ctgagagtga tgttcctgga gatgaaaaat gagaaggaaa actcctgatc aagttccaga 240
   gcccatagaa atatcctaga ggagaacctt cggcgctctg acaaggagtt agaaaaacta 300
   gatgacattg ttcagcatat ttataagacc ctgctctcta ttccagaggt ggtgagggga 360
   tgcaaagaac tacagggatt gctggaattt ctgagctaag aaactgaaag ccagaatttg 420
   tttcacctct ttttacctgc aataccccct taccccaata ccaagaccaa ctggc 475
<210> 124
   <211> 482
   <212> DNA
   <213> Homo sapiens
   <400> 124
   tatagagttt atctacacgg ccccctcctc ggcagtgtgt ggggtctcgc tggacgttgg 60
   aggaaagaag gaatatctca ttgcaggaaa ggccgagggg gacggcaaga tgcacatcac 120
   cctctgtgac ttcatcgtgc cctgggacac cctgagcacc acccagaaga agagcctgaa 180
   ccacaggtac cagatgggct gcgagtgcaa gatcacgcgc tgccccatga tcccgtgcta 240
   catctcctcc ccggacgagt gcctctggat ggactgggtc acagagaaga acatcaacgg 300
   gcaccaggcc aagttcttcg cctgcatcaa gagaagtgac ggctcctgtg cgtggtaccg 360
   cggcgcggcg ccccccaagc aggagtttct cgacatcgag gacccataag caggcctcca 420
   acgcccctgt ggccaactgc aaaaaaagcc tccaagggtt tcgactggtc cagctctgac 480
   at 482
<210> 125
   <211> 530
   <212> DNA
   <213> Homo sapiens
   <400> 125
   tgcttggtgt gacccacgga ggatccactc ccaggatgac gtgctccgta gctctgctgc 60
   tgatactggg tctgcgatgc agcggcgtga ggcctgggct ggttggagaa ggtcacaacc 120
   cttctctgtt ggtctgcctt ctgctgaaag actcgagaac caaccaggga agctgtcctg 180
   gaggtccctg gtcggagagg gacatagaat ctgtgacctc tgacaactgt gaagccaccc 240
   tgggctacag aaaccacagt cttcccagca attattacaa ttcttgaatt ccttggggat 300
   tttttactgc cctttcaaag cacttaagtg ttagatctaa cgtgttccag tgtctgtctg 360
   aggtgactta aaaaatcaga acaaaacttc tattatccag agtcatggga gagtacaccc 420
   tttccaggaa taatgttttg ggaaacactg aaatgaaatc ttcccagtat tataaattgt 480
   gtatttaaaa aaaagaaact tttctgaatg cctacctggc ggtgtatacc 530
<210> 126
   <211> 504
   <212> DNA
   <213> Homo sapiens
   <400> 126
   tccgcattgg cacttctggt gggataggtc tggagcccgg cactgtggtc ataacagagc 60
   aggcagtgga tacctgcttc aaggcagagt ttgagcagat tgtcctgggg aagcgggtca 120
   tccggaaaac ggaccttaac aagaagctgg tgcaggagct gttgctgtgt tctgcagagc 180
   tgagcgagtt caccacagtg gtggggaaca ccatgtgcac cttggacttc tatgaagggc 240
   aaggccgtct ggatggggct ctctgctcct acacggagaa ggacaagcag gcgtatctgg 300
   aggcagccta tgcagccggc gtccgcaata tcgagatgga gtcctcggtg tttgccgcca 360
   tgtgcagcgc ctgcggcctc caagcggccg tggtgtgtgt caccctcctg aaccgcctgg 420
   aaggggacca gatcagcagc cctcgcaatg tgctcagcga gtaccagcag aggccgcagc 480
   ggctggtgag ctacttcatc aaga 504
<210> 127
   <211> 477
   <212> DNA
   <213> Homo sapiens
   <400> 127
   gtggccgtag caacttggcg gagacaggct atgagtctga cgttagagtg gttgcttcct 60
   tagcctttca ggatggagga atgtgggcag tttgacttca gcactgaaaa cctctccacc 120
   tgggccaggg ttgcctcaga ggccaagttt ccagaagcct cttacctgcc gtaaaatgct 180
   caaccctgtg tcctgggcct gggcctgctg tgactgacct acagtggact ttctctctgg 240
   aatggaacct tcttaggcct cctggtgcaa cttaattttt ttttttaatg ctatcttcaa 300
   aacgttagag aaagttcttc aaaagtgcag cccagagctg ctgggcccac tggccgtcct 360
   gcatttctgg tttccagacc ccaatgcctc ccattcggat ggatctctgc gtttttatac 420
   tgagtgtgcc taggttgccc cttatttttt attttccctg ttgcgttgct atagatg 477
<210> 128
   <211> 460
   <212> DNA
   <213> Homo sapiens
   <400> 128
   gttcctgcag aaggcgctcg agatccttcg gaaagacttc agtgagctga ggtccgcagg 60
   ggtggagcag ctcatgtaca tcaaggagga cttgatcatc cctcaccatc acagcttcta 120
   cgacttcatc gtcaccaagg cacgggggaa gagtggacca ctcttcaact ttgatgttca 180
   tgacgatgtg cggttgctca gtgacgccac tgtggagaag gatgagtccc atgcaggcaa 240
   ggtggtgctg aggagctggt acgagaagaa caagcacatc tttcccgcca gccgctggga 300
   accctacgac cctgaaaaga agtgggacaa gtacacgatc cgctgagcat ccaggaggct 360
   gcgcggcccc ggctcctcag ctccctcagt gtgccccgtg gtgtcaccgg gactccaggc 420
   acccgctccc ctgcgaccat gccaggcacg ctgggaggag 460
<210> 129
   <211> 526
   <212> DNA
   <213> Homo sapiens
   <400> 129
   gaacttgttc agaccgtttt agcacggaaa cctaaaatgt gcagcttcct tgagtggcga 60
   gatctgaaga ttgtttacaa aagatatgct agtctgtatt tttgctgtgc tattgaggat 120
   caggacaatg aactaattac cctggaaata attcatcgtt atgtggaatt acttgacaag 180
   tatttcggca gtgtctgtga actagatatc atctttaatt ttgagaaggc ttattttatt 240
   ttggatgagt ttcttttggg aggggaagtt caggaaacat ccaagaaaaa tgtccttaaa 300
   gcaattgagc aggctgatct actgcaggag gaagctgaaa ccccacgtag tgttcttgaa 360
   gaaattggac tgacataact ctcctccctt gttgatgact tcttgtggca tttcacacac 420
   tgtagatggt cactcccttc atgtccatgt tagctcatgg tgtaagatga tgtcttgtca 480
   gtattactgt tttgctaagc cgcttcattc atgcctacac aatttt 526
<210> 130
   <211> 463
   <212> DNA
   <213> Homo sapiens
   <400> 130
   gggaaccggt gactcagaaa gacagatgtt ttggtaattt accccaaatg tgccatccac 60
   atagtgcttt ttcctcttgc ccttcggctt gtttgaatct cacaattatg tatttaattc 120
   tcaaagaaat atgtatctgt agccgtttgt tgacactaat acagatgatt aaggaaaaca 180
   gctgatcttt ggggaaggga gctaccaaca ctttatacac acacacacgt gcacacacac 240
   acacacacta tatatatata ttatttacag ggaaattttt cagggtttac aaaagagtat 300
   gtgattggta gtaagagaca cacagaatgt ttatgaagaa attgcatttt ctttttcctt 360
   tacatttgaa cttctttata gtttaaatat aacgtcttga gatggcacat tcctacgatt 420
   gaagaagggg tcttgagatc ccctaaactt gcatacccag ttt 463
<210> 131
   <211> 255
   <212> DNA
   <213> Homo sapiens
   <400> 131
   ccgtggagct tcatcggggt ggtgcaggct cccaaactca ggctttcagc tgtgcttttt 60
   gcaaaagggc ttgcctaagg ccagccattt ttcagtagca ggacctgcca agaagattcc 120
   ttctaactga aggtgcagtt gaattcagtg ggttcagaac caagatgcca acatcggtgt 180
   ggactacagg acaaggggca ttgttgcttg ttgggtaaaa atgaagcaga agccccaaag 240
   ttcacattaa ctcag 255
<210> 132
   <211> 560
   <212> DNA
   <213> Homo sapiens
   <400> 132
   ggctttcagc tctatcagag tgaccctagt ggaaattacg ggggatggaa ggccacatgc 60
   attggaaata atagcgctgc agctgtgtca atgttgaaac aagactataa agaaggagaa 120
   atgaccttga agtcagcact tgctttagct atcaaagtac taaataagac catggatgtt 180
   agtaaactct ctgctgaaaa agtggaaatt gcaacactaa caagagagaa tggaaagaca 240
   gtaatcagag ttctcaaaca aaaagaagtg gagcagttga tcaaaaaaca tgaggaagaa 300
   gaagccaaag ctgagcgtga gaagaaagaa aaagaacaga aagaaaagga taaatagaat 360
   cagagatttt attactcatt tggggcacca tttcagtgta aaagcagtcc tactcttcca 420
   cactaggaag gctttacttt ttttaactgg tgcagtggga aaataggaca ttacatactg 480
   aattgggtcc ttgtcatttc tgtccaattg aatactttat tgtaacgatg atggttaccc 540
   ttcatggacg tcttaatctt 560
<210> 133
   <211> 470
   <212> DNA
   <213> Homo sapiens
   <400> 133
   ttctgagcca ccttgtggat cccaaggacc tggagccacg ggctgccaac tgcactcggg 60
   tactggtgtg gcatactcgg acagagaagc ccaagatgaa gcaggaggag cagctgcagc 120
   ggcagggccg gggctcagac ccagcaattg aggtgtgatg gcggccccac cccaactacc 180
   acctcttttc aggcacagac cttgtgggac tgggccccag gcctgcccag gatgtggttt 240
   tccaagtcct gacccttgga gccagaagtg gcccctctgc ccctccaggc ccagggcatg 300
   gtcctgctgc ttcacccctc ccctagcctg ccgtgtggca ctgcccacag gctggggaca 360
   agcagccctt gtgttgagtc aggttggccc tgtctagggt ggaacagaag gacagatgga 420
   cccaggaggg agggcagctg agtaactggg taacttattg gggctgggca 470

<210> 134
   <211> 541
   <212> DNA
   <213> Homo sapiens
   <400> 134
   aaaacaggac atctgtgacc gccctacccc cacgccagcc ccaaactaag atatccctca 60
   cacccagccc ccattaccta gggacaagag tcttccccag ccttgaacct aggaccaaga 120
   gccacctaca tccagcccca aaactggggc ttcaggccag agcatccatg gccaatttca 180
   aattgtgaac ccagagacac tcccatccac ccttctccat gctcatcccc aaactggggc 240
   ctggagcaag gcactctcaa atcttgaacc ctggaccaaa gcttttccag accccaccct 300
   accttccaac ccaggtcaag acattgccaa atcttgaact cagaacccaa gtgttccatg 360
   cccctgtgtg gatggagtcg ggtatcctga ctgttggacc cctggtccag gtgatcccga 420
   ccctcaccag tcccatttgc ctccctccag ctctgcttag gcattttgcc cctcacccca 480
   atgttccaca ccatcgacaa ccaaggggtg aggtggggac aggcctcagc agggaatggg 540
   g 541
<210> 135
   <211> 501
   <212> DNA
   <213> Homo sapiens
   <400> 135
   tatgagttag ctttcttgct agccccctag tcggtcacca aactagtaac tagtggggct 60
   taatgaaggt cataagtttc tgagatggga gagcaacaag tagagatgaa gttaaaggta 120
   tttatcattc aagaaatcat tattgagtca ccaattgaca ggcactattc taatcagtag 180
   ttcactttaa tatttaataa gattttctgg gataacagta agggatatta gataatatac 240
   cgtatgtatt tattactagt cttttcctct aggaaaaggg atactttgat aattaaggcc 300
   agaggcccat tagttgagaa agtcacagat atatttctcc aagaaagcca acaaccacca 360
   ccacaatgac agaaatgaca acaaggccct ttaacttgtc ttctagttta gagacatcct 420
   tcatttgaca tttagtagaa ttcctctttg gccacaagaa taagcagcaa ataaacaact 480
   atggctgttg aggttctcat t 501
<210> 136
   <211> 533
   <212> DNA
   <213> Homo sapiens
   <400> 136
   ttccaaagtc tctgctgtca agatagattc gagagaaagc acgtggccat gtatgcttta 60
   accttaaact gcatacacat gtagtgatac ctaggctgca tttagatcac cgtgtgctca 120
   ggccaggtgt gaatcctgag gtccatggag gtgcagagat gagattactc ctattcacgt 180
   tgaagtgatt tgctttgtta acaaaaaatt gcagctattg tctagctttc atttttttac 240
   tgagaacttt aaattagtcc cctattagaa tagggttgct actcatcttt ttttaaaaac 300
   cgaatttcat catttatcta aagagaaaat atgcagaata actggtcttg ttaagagtgc 360
   aatattatat ttttatgtaa aaataaaaat taatttgggg ggattattta ttcagcatga 420
   aacctaatat gtatatgttt gaaatacttc ataatgtgca tgttgtagca aacatttctg 480
   taaattatca caagctctgt tacctttata tacgctgcct cttcaatttg gaa 533
<210> 137
   <211> 351
   <212> DNA
   <213> Homo sapiens
   <400> 137
   aaaacagcca agcttttctg ccaaaaagat gactgagaag actgttaaag caaaaagctc 60
   tgttcctgcc tcagatgatg cctatccaga aatagaaaaa ttctttccct tcaatcctct 120
   agactttgag agttttgacc tgcctgaaga gcaccagatt gcgcacctcc ccttgagtgg 180
   agtgcctctc atgatccttg acgaggagag agagcttgaa aagctgtttc agctgggccc 240
   cccttcacct gtgaagatgc cctctccacc atgggaatcc aatctgttgc agtctccttc 300
   aagcattctg tcgaccctgg atgttgaatt gccacctgtt tgctgtgaca t 351
<210> 138
   <211> 542
   <212> DNA
   <213> Homo sapiens
   <400> 138
   ggcaaagcac acaggctgag cgctgaggag agggaccagc tgctgccaaa cctgagggct 60
   gtggggtgga atgagctgga aggccgtgat gccatcttca agcagtttca tttcaaagac 120
   ttcaacaggg cctttgggtt catgacaaga gtggccctgc aggctgagaa actggaccac 180
   catcctgaat ggtttaacgt gtacaacaag gtccacatca cgctgagcac ccatgagtgt 240
   gccggccttt cagaacggga cataaacctg gccagcttca tcgaacaagt agcagtgtcc 300
   atgacataga ccctgccctt cctctttgaa ttcttccggg ggaaggggtg actgaactgg 360
   gagtccaggg agggagctga ggagccctta ccctcccacc actcccctcc caagacccag 420
   ccgccgccgt tgagggctga gtccttgctg tgggatgtgc cagtgtcccc accaacacca 480
   ggaatttaga ccttttccct gcaccactct cttcatcctg ggggctctgt tacactaatt 540
   tg 542
<210> 139
   <211> 549
   <212> DNA
   <213> Homo sapiens
   <400> 139
   ctggaggaca gcacctgtga cttcggcaac ctcaagcgct atgcatgcac ctctcatacc 60
   cagggcctga gcccaggctg ctatgacacc tacaatgcgg acatcgactg ccagtggatc 120
   gacataaccg acgtgcagcc tgggaactac atcctcaagg tgcacgtgaa cccaaagtat 180
   attgttttgg agtctgactt caccaacaac gtggtgagat gcaacattca ctacacaggt 240
   cgctacgttt ctgcaacaaa ctgcaaaatt gtccaatcct gatctccggg agggacagat 300
   ggccaatctc tccccttcca aagcaggccc tgctccccgg gcagcctccc gccgaggggc 360
   ccagccccca acccacaggc agggaggggc atccctccct gccggcctca gggagcgaac 420
   gtggatgaaa accacaggga ttccggatgc cagaccccat tttatacttc acttttctct 480
   acagtgttgt tttgttgttg ttggttttta ttttttatac tttggccata ccacagagct 540
   agattgccc 549
<210> 140
   <211> 558
   <212> DNA
   <213> Homo sapiens
   <400> 140
   acctcccgtg agaaagctgg tccacgacaa agagttggca gcagaagatg agcaggtgtt 60
   cctaatgaag caacagtcac tccttgccaa gcaaccagcc actcccacga gagcttctga 120
   atctcctgca agaggaccct ctggctctcc aaggacccag ggtcggggag ggccagccag 180
   tgtgcctagc tcctccccag gcacgtcagt aaaaaagccg gacccaaaca tcaaaaataa 240
   tgcagcaagt gaaggggtgt tggccagctt cttcaacagt ctgttgagta aaaagacagg 300
   ctctcctgga agtcctggtg ctggtggggt gcagagcaca gccaagaagt caggacaaaa 360
   gactgtgttg tcaaatgttc aggaagaact ggatagaatg actcgaaagc cagactctat 420
   ggtaacaaac tcttcaacag aaaatgaagc ctgaacctcc ttaaaaagtg catatgtcga 480
   atgaccaaat aactatgtat attgatctgc taagaccagg atttttctga tatggcacat 540
   gctatcagtt ttttgggg 558
<210> 141
   <211> 518
   <212> DNA
   <213> Homo sapiens
   <400> 141
   tgaggctttg gccttaacac ccaggaactt ttctattaca atcgcttagg aagtaaagcc 60
   ttgtctccct ccctgttctc tgcctcttgt acccctctga cccacccgct ctgccccact 120
   cccagccctc ctcagcccca gccctgcctg ccctgcccct ccagggggcc atgagtgcct 180
   aggtttctca taccccacaa ggtcacagca ggggagggag ggacaatttt ataatgaacc 240
   aaaaattcca tgtgttgggg ggtggggggc ggaggagggt gaggggtgcc gcccatgggc 300
   cacaaatctc tacaagtgcc tgctatccct ctcccactcc ccaccccagc accggtccaa 360
   ccccttcatc cccagctgct cctaggactg gcccatgggc aggcgggtgg ggggatggga 420
   agggggtgcc ctgaaaccaa actggaagcc ccctctgcct cccagctggg gcctctgggg 480
   tggggtgggg ggctgtggtc aagccttatt ctgtattg 518
<210> 142
   <211> 433
   <212> DNA
   <213> Homo sapiens
   <400> 142
   gtttgatgct cgctgggtaa catacttcaa caagccagat atagatgcct gggaattgcg 60
   taaagggata aacacacttg ttacctatga tatggttcca gagcccaaaa tcattgatgc 120
   tgctttgcgg gcatgcagac ggttaaatga ttttgctagt ctagttcgaa tcctagaggt 180
   tgttaaggac aaagcaggac ctcataagga aatctacccc tatgtcatcc aggaacttag 240
   accaacttta aatgaactgg gaatctccac tccggaggaa ctgggccttg acaaagtgta 300
   aaccgcatgg atgggcttcc ccaaggattt attgacattg ctacttgagt gtgaacagtt 360
   acctggaaat actgatgata acatattacc ttattttgaa caagtttccc tttattgagt 420
   accaagccat gta 433
<210> 143
   <211> 512
   <212> DNA
   <213> Homo sapiens
   <400> 143
   ccacgagttc acctatgcac tgatgccgca caagggctct ttccaggatg ctggcgttat 60
   ccaagctgcc tacagcctaa acttccccct gttggctctg ccagccccca gcccagcgcc 120
   cgccacctcc tggagtgcgt tttccgtgtc ttcacccgcg gtcgtattgg agaccgtcaa 180
   gcaggcggag agcagccccc agcgccgctc gctggtcctg aggctgtatg aggcccacgg 240
   cagccacgtg gactgctggc tgcacttgtc gctgccggtt caggaggcca tcctctgcga 300
   tctcttggag cgaccagacc ctgctggcca cttgacttcg ggacaaccgc ctgaagctca 360
   ccttttctcc cttccaagtg ctgtccctgt tgctcgtgct tcagcctccg ccacactgag 420
   tccctggggc tggggttttg tttgtagaag gctctgggga ctcctaattt ctgcttcccc 480
   agcctaaagc agggatcagt cttttcttgt gg 512
<210> 144
   <211> 500
   <212> DNA
   <213> Homo sapiens
   <400> 144
   aacactgcca gaatactttc tagctgcttt gtaatttttt aagagtgtta ttttgttttt 60
   gtttttctgt tctttgttgt ggctcttgtt ttcatttttg ttgtacgtgt agatctgtaa 120
   ataaaattgc agtatttaaa gcttaagctt tcaggaaaaa gaaaataaga attcagtgtg 180
   tgcatgacaa ctcgtgtgta tgagaaggag ggatatgaag gaagatggct tgcagagtaa 240
   gtcgggtggc aattgtcagg gtgtgggaat ttcttttcct acggggtacg tgattttgta 300
   aaaaggaagt atttctccca aaattgggag taggcaaact actaatcagt ttagctttgt 360
   gttgtatgct agtttaaaaa agaaaatatg taatataatg taaaaaaaaa caaaaaaaag 420
   cttttatgat ggattttgta aatagatttg ttacagggtg acctgttctc tagctgtgat 480
   cttaccactt caaatgggtg 500
<210> 145
   <211> 512
   <212> DNA
   <213> Homo sapiens
   <400> 145
   tgaatgacct gacttttagc caccaggtac tctttaaaca gttttcctta tcagaggccc 60
   tcctgtgctg gtgacccagc atctgagtta ggttccagca tgtaaagagc tgggagggcg 120
   gagaattctt agcatacatt cagacgtttt ttctgcacaa taataagtcc atctgtcact 180
   tgcattccac tttttgttac atagaaagag tctgaccctt taatccaaaa ggtcttttta 240
   cattgtgaat gctgtgggaa ggcaatttct ctgcacacaa gaggctacgt tttggaagtg 300
   atgtatgtta tttgatgact gaaaatgaac tgtaaatgct cctagagtat attcctctgc 360
   tgaacaaaat taaacttcaa aaaaatctaa cagtaacaca cccctgcttg ggaccctagc 420
   tatatgcatt ttatgtgacc ttgccatgct tcagtgaaca tactaattct atgtctagca 480
   catgttgatt tcctatgtat tctgggtatt ct 512
<210> 146
   <211> 562
   <212> DNA
   <213> Homo sapiens
   <400> 146
   aggacaaact ctgtgtacct gtgcccaggt gaatgggcgc agggtcctct tgccctgtcc 60
   tgcggggggc cccacgagtt cctggcattc agcactgctt agcattctcg gaaggtttct 120
   tcaactgctt gcttttccca ggcttgcctt tagtgtcatg taagacattt ttaagttata 180
   tttattttgt tgggttttaa aattgcacag aacactaaga ccgaaaggct ggactcttgt 240
   ttctccttga aagctttgcc tttgttttga acttcctttc ccacttggta gaaagagccc 300
   agaagcagcc ctggccctgt aagatggact ctttcatcct tcagttgtat ttagctttga 360
   gtttctctgc atctgtccac cccatgtgta tataacccag cccctggctc tggggtggtc 420
   acctcgtcag tgccttttgt tctggaggag aggacccccc cgcctgccga gaggctctct 480
   tcctgttctg cacccctctc cccatgggac cttggagaaa actgaactgt tacaaacccc 540
   tgcacagtgc ctgtcaaaca ga 562
<210> 147
   <211> 465
   <212> DNA
   <213> Homo sapiens
   <400> 147
   atcctcattc ttatactgct tttcgtggcc actttggaca agtcctggtg gactctccct 60
   gggaaagagt ccctgaatct ctggtacgac tgcacgtgga acaacgacac caaaacatgg 120
   gcctgcagta atgtcagcga gaatggctgg ctgaaggcgg tgcaggtcct catggtgctc 180
   tccctcattc tctgctgtct ctccttcatc ctgttcatgt tccagctcta caccatgcga 240
   cgaggaggtc tcttctatgc caccggcctc tgccagcttt gcaccagcgt ggcggtgttt 300
   actggcgcct tgatctatgc cattcacgcc gaggagatcc tggagaagca cccgcgaggg 360
   ggcagcttcg gatactgctt cgccctggcc tgggtggcct tccccctcgc cctggtcagc 420
   ggcatcatct acatccacct acggaagcgg gagtgagcgc cccgc 465
<210> 148
   <211> 493
   <212> DNA
   <213> Homo sapiens
   <400> 148
   ggagttgtag cctctttaaa cacctgagaa gccatgagag gacagatccc ataaatacct 60
   taagtgtaga ggggtctctg ttgtagaata gctcttaatt ttagagaaac cttcctggag 120
   ggaaaccata ctcctataat gagcaaagta acaacttcaa gcatttttcc agcgttacca 180
   tcaaactcac aaataggttg aaatccttta gttataactc agcctttagg aacaccggag 240
   aacccacaat aatagaaatc ttttcgtgtt ccccattgag aaatgcttta gttagcatct 300
   tcatgcttgg aaatctagac aagaagagaa tccatggatg gacatggtcg aggaattcgg 360
   aaagcctgca gttgacattc agtcttcact tgaaactcaa aactgacact aggaacagct 420
   tcatgagttc agtagaagta agctttattt gtagcttctg ccttgtttga cggcgtatct 480
   attcagggaa gcg 493
<210> 149
   <211> 480
   <212> DNA
   <213> Homo sapiens
   <400> 149
   caggcaggag gtcctgttag ccctgccttc caggaaggtt ggggtgggag ttttgagtgg 60
   gaaagaggat gacatgtgtg agagagttct gagcctgttt gctagggaga gtgagtgagt 120
   gctcttgggc actgctcagg ccgtttctgc tgacttgcct ggcttacaat aaatgcccaa 180
   taaatatttg ttgaccatat gtgttgtaca ctgtggtgcc ctgtccagtc ccctctacca 240
   agctgagacc cccatcccca gctgctctga gtttgggctg caagtgctca cagctcttgt 300
   tctccagaaa ctggagaatt gccctcagga gatgagagcc atctcacctc acccaggagt 360
   cacttcctct ctacacccca acacctggtt catttgatta aagcggagaa aactccaggg 420
   tgctatgact gctctggcac ccttggatca ggccaagcta gactttttct gagccttcat 480
<210> 150
   <211> 483
   <212> DNA
   <213> Homo sapiens
   <400> 150
   attcagcctg gcttcaaatt gtaagcatgc acaaattctg tctctggatt atattatgaa 60
   gcttttatgt gaaacatgtt tctttgtaat gaaaaccaca ttggagatgt ttagtaatca 120
   tattgttact ggtaccaaga ctactaggga aatgcctttg tactttaggg aagtactttt 180
   ggcattttac tgtacagaca gaaaaaactg agatgtagcc cctctcctgg aagtgctaat 240
   tttgaaaaac tgctcatatg atgtacatgt actgattact gcctatttta ataaacactc 300
   ttgaaaaatg catgttgccc tgttgctgcc tgccctattc tcctcatctc cccatcattg 360
   gtacccactt gcttttaaaa tccactttat cttgaataat gtaagacaaa tatgttctga 420
   cataagtatt taattcatgt tgccttgcat aatggtcaga ggcgcatgaa tttgtgaagg 480
   tgg 483
<210> 151
   <211> 145
   <212> DNA
   <213> Homo sapiens
   <400> 151
   ttcctgaaca tgagtttgcg acgggaccag tgtgtcttga tgatgagaat gagtttcctc 60
   ctataatctt gtgccgtgga aatcagaagg gcaaaacgaa gcagtcatga tgagaagcac 120
   acctcagaaa tcaggacatc ccccc 145
<210> 152
   <211> 539
   <212> DNA
   <213> Homo sapiens
   <400> 152
   tgccagcgac tgtctcagac tgggcaggga ggctttggca tgacttaaga ggaagggcag 60
   tcttgggacc cgctatgcag gtcctggcaa acctggctgc cctgtctcat ccctgtccct 120
   cagggtagca ccatggcagg actgggggaa ctggagtgtc cttgctgtat ccctgttgtg 180
   aggttccttc caggggctgg cactgaagca agggtgctgg ggccccatgg ccttcagccc 240
   tggctgagca actgggctgt agggcagggc cacttcctga ggtcaggtct tggtaggtgc 300
   ctgcatctgt ctgccttctg gctgacaatc ctggaaatct gttctccaga atccaggcca 360
   aaaagttcac agtcaaatgg ggaggggtat tcttcatgca ggagacccca ggccctggag 420
   gctgcaacat acctcaatcc tgtcccaggc cggatcctcc tgaagccctt ttcgcagcac 480
   tgctatcctc caaagccatt gtaaatgtgt gtacagtgtg tataaacctt cttcttctt 539
<210> 153
   <211> 390
   <212> DNA
   <213> Homo sapiens
   <400> 153
   gaaggtgtgg ttttcatttc tcagtcacca acagatgaat aattatgctt aataataaag 60
   tatttattaa gactttcttc agagtatgaa agtacaaaaa gtctagttac agtggattta 120
   gaatatattt atgttgatgt caaacagctg agcaccgtag catgcagatg tcaaggcagt 180
   taggaagtaa atggtgtctt gtagatatgt gcaaggtagc atgatgagca acttgagttt 240
   gttgccactg agaagcaggc gggttgggtg ggaggaggaa gaaagggaag aattaggttt 300
   gaattgcttt ttaaaaaaaa aagaaaagaa aaagacagca tctcactatg ttgccaaggc 360
   tcatcttgag aagcaggcgg gttgggtggg 390
<210> 154
   <211> 398
   <212> DNA
   <213> Homo sapiens
   <400> 154
   ggctcccagc aagggtagga cgggccgcat gcgggcagaa agttgggact gagcagctgg 60
   gagcaggcga ccgagctcct tccccatcat ttctccttgg ccaacgacga ggccagccag 120
   aatggcaata aggactccga atacataata aaagcaaaca gaacactcca acttagagca 180
   ataacggctg ccgcagcagc cagggaagac cttggtttgg tttatgtgtc agtttcactt 240
   ttccgataga aatttcttac ctcatttttt taagcagtaa ggcttgaagt gatgaaaccc 300
   acagatccta gcaaatgtgc ccaaccagct ttactaaagg gggaggaagg gagggcaaag 360
   ggatgagaag acaagtttcc cagaagtgcc tggttctg 398
<210> 155
   <211> 562
   <212> DNA
   <213> Homo sapiens
   <400> 155
   gaagaaccat cgaaacctgt ttgttcccag cccaccccca gtggatggga tgcataatgc 60
   cagcaagttt tgtttaacag caaaaaagga agattaatgc aggtgttata gaagccagaa 120
   gagaaactgt gtcaccctaa agaagcatat aatcatagca ttaaaaatgc acacattact 180
   ccaggtggaa ggtggcaatt gctttctgat atcagctcgt ttgatttagt gcaaaaatgt 240
   tttcaagact atttaatgga tgtaaaaaag cctatttcta cattatacca actgagaaaa 300
   aaatggtcgg taaagtgttc tttcataata aataatcaag acatggtccc atttgcagga 360
   aaagtgcaga ctctgagtgt tccagggaaa cacatgctgg acatcccttg taacccggta 420
   tgggcgcccc tgcattgctg ggatgtttct gcccacggtt ttgtttgtgc aataacgtta 480
   tcacatttct aatgaggatt cacattaata taatataaaa taaataggtc agttactggt 540
   ctctttctgc cgaatgttat gt 562

<210> 156
   <211> 268
   <212> DNA
   <213> Homo sapiens
   <400> 156
   tgccctgacc ccgatcagtt aaggagctgt gcaataacct tcctagtacc tgagtgagtg 60
   tgtaacttat tgggttggcg aagcctggta aagctgttgg aatgagtatg tgattctttt 120
   taagtatgaa aataaagata tatgtacaga cttgtatttt ttctctggtg gcattccttt 180
   aggaatgctg tgtgtctgtc cggcaccccg gtaggcctga ttgggtttct agtcctcctt 240
   aaccacttat ctcccatatg agagtgtg 268
<210> 157
   <211> 490
   <212> DNA
   <213> Homo sapiens
   <400> 157
   ccctgaccca attgtcatca accatgtcat cagcgtggac ccttcagacc agaagaagac 60
   agcgtgctat gacattgacg tggaggtgga ggagccatta aaggggcaga tgagcagctt 120
   cctcctatcc acggccaacc agcaggagat cagtcctctg gacagtaaga tccatgagac 180
   gattgagtcc ataaaccagc tcaagatcca gagggacttc atgctaagct tctccagaga 240
   ccccaaaggc tatgtccaag acctgctccg ctcccagagc cgggacctca aggtgatgac 300
   agatgtagcc ggcaaccctg aagaggagcg ccgggctgag ttctaccacc agccctggtc 360
   ccaggaggcc gtcagtcgct acttctactg caagatccag cagcgcaggc aggagctgga 420
   gcagtcgctg gttgtgcgca acacctagga gcccaaaaac aagcagcacg acggaacttt 480
   cagccgtgtc 490
<210> 158
   <211> 496
   <212> DNA
   <213> Homo sapiens
   <400> 158
   cgctctcgtt tcattttctg cagcgcgcca cgaggatggc ccacaagcag atctactact 60
   cggacaagta cttcgacgaa cactacgagt accggcatgt tatgttaccc agagaacttt 120
   ccaaacaagt acctaaaact catctgatgt ctgaagagga gtggaggaga cttggtgtcc 180
   aacagagtct aggctgggtt cattacatga ttcatgagcc agaaccacat attcttctct 240
   ttagacgacc tcttccaaaa gatcaacaaa aatgaagttt atctggggat cgtcaaatct 300
   ttttcaaatt taatgtatat gtgtatataa ggtagtattc agtgaatact tgagaaatgt 360
   acaaatcttt catccatacc tgtgcatgag ctgtattctt cacagcaaca gagctcagtt 420
   aaatgcaact gcaagtaggt tactgtaaga tgtttaagat aaaagttctt ccagtcagtt 480
   tttctcttaa gtgcct 496
<210> 159
   <211> 508
   <212> DNA
   <213> Homo sapiens
   <400> 159
   atccattgtc cttgtagttt cttccctcct gttctctggt tatagctggt cccaggtcag 60
   cgtgggaggc acctttgggt tcccagtgcc cagcactttg tagtctcatc ccagattact 120
   aacccttcct gatcctggag aggcagggat agtaaataaa ttgctcttcc taccccatcc 180
   cccatcccct gacaaaaagt gacggcagcc gtactgagtc tgtaaggccc aaagtgggta 240
   cagacagcct gggctggtaa aagtaggtcc ttatttacaa ggctgcgtta aagttgtact 300
   aggcaaacac actgatgtag gaagcacgag gaaaggaaga cgttttgata tagtgttact 360
   gtgagcctgt cagtagtggg taccaatctt ttgtgacata ttgtcatgct gaggtgtgac 420
   acctgctgca ctcatctgat gtaaaaccat cccagagctg gcgagaggat ggagctgggt 480
   ggaaactgct ttgcactatc gtttgctt 508
<210> 160
   <211> 370
   <212> DNA
   <213> Homo sapiens
<220>
   <221> misc_feature
   <222> (251)..(251)
   <223> n is a, c, g, or t
   <400> 160
   gaagatgagt ctatggcatc aggttcttaa acccaggaaa gcacctacag accggctcct 60
   ccatgcactt taccagctca acgcatccac tctctgttct cttggcaggg cgggggaggg 120
   gggataggag gtcccctttc ccctaggtgg tctcataatt ccatttgtgg agagaacagg 180
   agggccagat agataggtcc tagcagaagg cattgaggtg agggatcatt ttgggtcaga 240
   catcaatgtc nctgtccccc ctgggtccag ccaagctgtg ccccatcccc caagcctcct 300
   gggaggatcc agccaaatct tgcgactcct ggcacacacc tgtctgtaac ctgttttgtg 360
   ctctgaaagc 370
<210> 161
   <211> 544
   <212> DNA
   <213> Homo sapiens
   <400> 161
   aagatagccc aacctagctc agatccacca agataagcac agcaaaagct tggctgcatt 60
   tttgaggaat aaaaacctgc agaaagcacc gataaccttc aagatctgaa tgagattcta 120
   ttataacccg tctaaacgat tgcaaaattc ctcctttggt tttggaagca gcgtttgctc 180
   tcccgtggct cggattctct gaggaccagg gagttgacac acaaaccccg ccatgggtcc 240
   gagccagcta tttctcaagg ctcccacctc gccaagctcc caagggcctg ctggcagtgc 300
   ctacgctgtg ccaactaccc tgtctggtac agaccacggc tgggtaagca cccttaaaag 360
   caacagaaat gacgtctgga agctgaaatg tgaaactgtc aagatggctt aggagaggaa 420
   ggagtggacc cgctggtctt tggcattttg tatttagaat tattctaact ttatacataa 480
   tgtataggcc gatcttttgg aagggataag gttttcattc ttgtgcaact cattattctc 540
   atta 544
<210> 162
   <211> 412
   <212> DNA
   <213> Homo sapiens
   <400> 162
   atggagatgg tactggagtc gccagtattt accgggggcc atttgcagat gaaaatttta 60
   aacttagaca ctcagctcca ggcctgcttt ccatggcgaa cagtggtcca agtacaaatg 120
   gctgtcagtt ctttatcacc tgctctaagt gcgattggct ggatgggaag catgtggtgt 180
   ttggaaaaat catcgatgga cttctagtga tgagaaagat tgagaatgtt cccacaggcc 240
   ccaacaataa gcccaagcta cctgtggtga tctcgcagtg tggggagatg tagtccagac 300
   aaagactgaa tcaggccttc ccttcttctt ggtggtgttc ttgagtaaga taatctggac 360
   tggcccccgt ctttgcttcc ctgcctgctg ctgccccatt tgatcaagag ac 412
<210> 163
   <211> 569
   <212> DNA
   <213> Homo sapiens
   <400> 163
   tgaggaaccc aatgaatgtg acttcaagaa tatggatagt ttaccttctg gtaaaataca 60
   tcgaaaagtg aaaataatat taggacgaaa tagaaaagaa aatctggaac caaatgctga 120
   atttgataaa agaactgaat ttattacaca agaagaaaac agaatttgta gttcaccggt 180
   acagtcttta ctagacttgt ttcagactag tgaagagaaa tcagaatttt tgggtttcac 240
   aagctacaca gaaaagagtg gtatatgcaa tgttttagat atttgggaag aggaaaattc 300
   agataatctg ttaacagcgt ttttctcgtc cccttcaact tctacattta ctggctttta 360
   gaatttaaaa aatgcatact tttcagaagt gataaggatc atattcttga aatttttata 420
   aatatgtatg gaaattctta ggattttttt accagctttg tttacagacc caaatgtaaa 480
   tattaaaaat aaatatttgc aattttctac agaattgaat acctgttaaa gaaaaattac 540
   agaataaact tgtgactggt cttgtttta 569
<210> 164
   <211> 375
   <212> DNA
   <213> Homo sapiens
   <400> 164
   ccgtccgctg ttactcagct gaggtggtca cactgtggta ccgcccaccg gatgtcctct 60
   ttggggccaa gctgtactcc acgtccatcg acatgtggtc agccggctgc atctttgcag 120
   agctggccaa tgctgggcgg cctctttttc ccggcaatga tgtcgatgac cagttgaaga 180
   ggatcttccg actgctgggg acgcccaccg aggagcagtg gccctctatg accaagctgc 240
   cagactataa gccctatccg atgtacccgg ccacaacatc cctggtgaac gtcgtgccca 300
   aactcaatgc cacagggagg gatctgctgc agaaccttct gaagtgtaac cctgtccagc 360
   gtatctcagc agaag 375
<210> 165
   <211> 549
   <212> DNA
   <213> Homo sapiens
   <400> 165
   gttttgcctc acctaatagg ctgggagact gaagactcag cccgggtggc tgcagaaaaa 60
   tgattggccc cagtcccctt gtttgtccct tctacaggca tgaggaatct gggaggccct 120
   gagacaggga ttgtgcttca ttccaatcta ttgcttcacc atggccttat gaggcaggtg 180
   agagatgttt gaatttttct cttcctttta gtattcttag ttgttcagtt gccaaggatc 240
   cctgatccca ttttcctctg acgtccacct cctaccccat aggagttaga agttagggtt 300
   taggcatcat tttgagaatg ctgacacttt ttcagggctg tgattgagtg agggcatggg 360
   taaaaatatt tctttaaaag aaggatgaac aattatattt atatttcagg ttatatccaa 420
   tagtagagtt ggcttttttt tttttttttt ggtcatagtg ggtggatttg ttgccatgtg 480
   caccttgggg ttttgtaatg acagtgctaa aaaaaaagca tttttttttt atgatttgtc 540
   tctgtcacc 549
<210> 166
   <211> 230
   <212> DNA
   <213> Homo sapiens
   <400> 166
   cctcccatca gctctacatc tgagggacat ggtgtgccac aggctgcaag ctgcagggaa 60
   ttttcattgg atgcagttgt atagttttac actctagtgc catatatttt taagactttt 120
   ctttccttaa aaaataaagt acgtgtttac ttggtgagga ggaggcagaa ccagctcttt 180
   ggtgccagct gtttcatcac cagactttgg ctcccgcttt ggggagcgcc 230
<210> 167
   <211> 329
   <212> DNA
   <213> Homo sapiens
   <400> 167
   atccccttag tgctctgaaa tatttacaaa atgatcttta tataactgtg gatcattcag 60
   acccagaaga gacaaaagag tttcagctcc tggcatcagc tctattcaaa tctggttcag 120
   attttacagc tctgggcttt tctgatgtgg atcacaccta tgctcaaaga actcagctct 180
   ttgacacctt agtaaatttc tttcctgaca gcatgactcc tcctaaaggc aacctcgtag 240
   acctgatcac actgtaactg aagagtcact ggacacagaa atggaaaaca ggagtcgatt 300
   ttccgtcttt tggattgcag ctccactga 329
<210> 168
   <211> 437
   <212> DNA
   <213> Homo sapiens
   <400> 168
   tccatctgcc ccaggacaag agcaagaagg acatcagttg cccagtcatg tgatcccctg 60
   ccatcttgcc ttaggaacag ccttccccca ccagcagcca tggctggctg gggcgttagc 120
   caagccacct actgccagga attggagcct cagttccctc ctgtgtcaag tagctaactg 180
   cagcagctgg actgagggca gagtctgtgg gtgcagagac cctgcatgta ggtcacaggt 240
   tgaggcccag ccactctccc tggggcctgg tgggtaggca agtagctctg gggccacctc 300
   aagtgaccaa atgctattaa tttccatcct ttagcaggct gggccctagg caggaagctg 360
   gcttctggga gaggagtgag aacgtgcagg gcctgcctag cttgcgtgct tgaggaaggt 420
   ggcattccgt gcttgcc 437
<210> 169
   <211> 554
   <212> DNA
   <213> Homo sapiens
<220>
   <221> misc_feature
   <222> (52)..(52)
   <223> n is a, c, g, or t
   <220>
   <221> misc_feature
   <222> (59)..(59)
   <223> n is a, c, g, or t
   <220>
   <221> misc_feature
   <222> (252)..(252)
   <223> n is a, c, g, or t
   <220>
   <221> misc_feature
   <222> (513)..(513)
   <223> n is a, c, g, or t
   <400> 169
   gccttctggg aacctatgga gaaagggaat ccaaggaagc agccaaggct gntcgcagnt 60
   tccctgagct gcacctcttg ctaaccccac catcacactg ccaccctgcc ctagggtctc 120
   actagtacca agtgggtcag cacagggctg aggatggggc tcctatccac cctggccagc 180
   acccagctta gtgctgggac tagcccagaa acttgaatgg gaccctgaga gagccagggg 240
   tcccctgagg cncccctagg ggctttctgt ctgccccagg gtgctccatg gatctccctg 300
   tggcagcagg catggagagt cagggctgcc ttcatggcag taggctctaa gtgggtgact 360
   ggccacaggc cgagaaaagg gtacagcctc taggtggggt tcccaaagac gccttcaggc 420
   tggactgagc tgctctccca cagggtttct gtgcagctgg attttctctg ttgcatacat 480
   gcctggcatc tgtctcccct tgttcctgag tgnccccaca tggggctctg agcaggctgt 540
   atctggattc tggc 554
<210> 170
   <211> 309
   <212> DNA
   <213> Homo sapiens
   <400> 170
   ctcggaattc cctgaagcaa cactgccaga agtgtgtttt ggtatgcact ggttccttaa 60
   gtggctgtga ttaattattg aaagtggggt gttgaagacc ccaactacta ttgtagagtg 120
   gtctatttct cccttcaatc ctgtcaatgt ttgctttatg tattttgggg aactgttgtt 180
   tgatgtgtat gtgtttataa ttgttataca tttttaattg agccttttat taacatatat 240
   tgttattttt gtctcgaaat aattttttag ttaaaatcta ttttgtctga tattggtgtg 300
   aatgctgta 309
<210> 171
   <211> 302
   <212> DNA
   <213> Homo sapiens
   <400> 171
   cctccctatc gtctgaacag ttgtcttcct cagcctcctc ccgcccccac cttgggaatg 60
   taaatacacc gtgactttga aagtttgtac ccctgtcctt ccctttacgc cactagtgtg 120
   taggcagatg tctgagtccc taggtggttt ctaggattga tagcaattag ctttgatgaa 180
   cccatcccag gaaaaataaa aacagacaaa aaaaaaggaa agattggttc tcccagcact 240
   gctcagcagc cacagcctcc ctgtatgcct gtgcttggtc tactgataag ccctctacaa 300
   aa 302
<210> 172
   <211> 491
   <212> DNA
   <213> Homo sapiens
   <400> 172
   tgctctgccc cagcttgggc agatctccca catgccaggg gcctttgggt gctgttttgc 60
   cagcccattt gggcagagag gctgtggttt gggggagaag aagtaggggt ggcccgaaag 120
   ggtctccgaa atgctgtctt tcttgctccc tgactggggg cagacatggt ggggtctcct 180
   caggaccagg gttggcacct tccccctccc ccagccactc cccagccagc ctggctggga 240
   ctgggaacag aactcggtgt ccccaccatc tgctgtcttt tctttgccat ctctgctcca 300
   accgggatgg gagccgggca aactggccgc gggggcaggg gaggccatct ggagagccca 360
   gagtcccccc actcccagca tcgcactctg gcagcaccgc ctcttcccgc cgcccagccc 420
   accccatggc cggctttcag gagctccata cacacgctgc cttcggtacc caccacacaa 480
   catccaagtg g 491
<210> 173
   <211> 122
   <212> DNA
   <213> Homo sapiens
   <400> 173
   ccggggctgg ttttctatga acgattccgg cctgggatgc gggccaggct gcaggcggca 60
   tagttgggcc cattcgtcct ggaaagggac tggggggtcc caacttagcc ctgggtgggc 120
   cg 122
<210> 174
   <211> 536
   <212> DNA
   <213> Homo sapiens
   <400> 174
   attccgatcc caatgagcaa gtgacaagaa aaaacatgct cctggctaca aaacagatat 60
   gcaaagagtt caccgacctg ctggctcagg accgatctcc cctggggaac tcacggccca 120
   accccatcct ggagcccggc atccagagct gcttgaccca cttcaacctc atctcccacg 180
   gcttcggcag ccccgcggtg tgtgccgcgg tcacggccct gcagaactat ctcaccgagg 240
   ccctcaaggc catggacaaa atgtacctca gcaacaaccc caacagccac acggacaaca 300
   acgccaaaag cagtgacaaa gaggagaagc acagaaagtg aggctctcct cccgccccgc 360
   ccctcccacg cctcaccagc cccccgcgcg cccaccctcc ggcgggtgac agctccggga 420
   tcagcaaccc ttcctgctgc tgctactgct gctgctgctg ccgccgccgc cgccgccgct 480
   gcccttgggt ccccccgagt ctccgggact gccctctcga ctgtcagtgg ggcagc 536
<210> 175
   <211> 487
   <212> DNA
   <213> Homo sapiens
   <400> 175
   gatgatttct cgaaagccat gccagaagca gtcttccagg tcatcttgta gaactccagc 60
   tttgttgaaa atcacggacc tcagctacat catacactga cccagagcaa agctttccct 120
   atggttcaaa gacaactagt attcaacaaa ccttgtatag tgtatgtttt gccatattta 180
   atattaatag cagaggaaga ctcctttttt catcactgta tgaatttttt ataatgtttt 240
   tttaaaatat atttcatgta tacttataaa ctaattcaca caagtgtttg tcttagatga 300
   ttaaggaaga ctatatctag atcatgtctg attttttatt gtgacttctc cagccctggt 360
   ctgaatttct taaggtttta taaacaaatg ctgctattta ttagctgcaa gaatgcactt 420
   tagaactatt tgacaattca gactttcaaa ataaagatgt aaatgactgg ccaataataa 480
   ccatttt 487
<210> 176
   <211> 504
   <212> DNA
   <213> Homo sapiens
   <400> 176
   ccggctatgg gctcgagccg agttccttca acatgcactg cgcgcccttt gagcagaacc 60
   tctccggggt gtgtcccggc gactccgcca aggcggcggg cgccaaggag cagagggact 120
   cggacttggc ggccgagagt aacttccgga tctacccctg gatgcgaagc tcaggaactg 180
   accgcaaacg aggccgccag acctacaccc gctaccagac cctggagctg gagaaagaat 240
   ttcactacaa tcgctacctg acgcggcggc ggcgcatcga gatcgcgcac acgctctgcc 300
   tcacggaaag acagatcaag atttggtttc agaaccggcg catgaagtgg aaaaaggaga 360
   acaagaccgc gggcccgggg accaccggcc aagacagggc tgaagcagag gaggaagagg 420
   aagagtgagg gatggagaaa gggcagagga agagacatga gaaagggaga ggaagagaag 480
   cccagctctg ggaactgaat cagg 504
<210> 177
   <211> 356
   <212> DNA
   <213> Homo sapiens
   <400> 177
   gaatcaggaa actcaaatcg aatagggaag taaaaaaaca aaacaaaaaa caaaaaaaaa 60
   caaaaaaaaa ccctatttaa atgaaaggag tttaaaaaca ttttttaagg agggagaaag 120
   gagaaatttt ggtttttcaa cactgaaaaa atagtaccta taggaaagtc tgtcaggttt 180
   ggtttttttg tacaatatga aaaggacatt atctacctgt tctgtagctt tctggaattt 240
   acctcccctt ttctatgttg ctattgtaag gtctttgtaa aatcttgcag ttttgtaagc 300
   cctctttaat gctgtctttg tggactgtgg gtctggacta accctgtggt tgcctg 356

<210> 178
   <211> 225
   <212> DNA
   <213> Homo sapiens
   <400> 178
   ccgagctgaa gaaccagcgg ctcaaggagg ttttccagac caagatccag gagttccgca 60
   aggcctgcta cacgctcacc ggctaccaga tcgacatcac cacggagaac cagtaccggc 120
   tgacctcgct gtacgccgag cacccaggcg actgctcatc ttcaaggcca ccagcccctc 180
   gggttccaag atgcagctac tggagacaga gttctcacac accgt 225
<210> 179
   <211> 380
   <212> DNA
   <213> Homo sapiens
   <400> 179
   tactgcttgc agtaattcaa ctggaaatta aaaaaaaaaa actagactcc attgtgcctt 60
   actaaatatg ggaatgtcta acttaaatag ctttgagatt tcagctatgc tagaggcttt 120
   tattagaaag ccatattttt ttctgtaaaa gttactaata tatctgtaac actattacag 180
   tattgctatt tatattcatt cagatataag atttgtacat attatcatcc tataaagaaa 240
   cggtatgact taattttaga aagaaaatta tattctgttt attatgacaa atgaaagaga 300
   aaatatatat ttttaatgga aagtttgtag catttttcta ataggtactg ccatattttt 360
   ctgtgtggag tatttttata 380
<210> 180
   <211> 440
   <212> DNA
   <213> Homo sapiens
   <400> 180
   tgcctgctgg ggattactcg atcaaaacct tccttccctg gctacttccc ttcctcccgg 60
   ggccttcctt ttaggtgctg gagctggagg ggtggggagc tagaggccac ctatgccagt 120
   gctcaaggtt actgggagtg tgggctgccc ttgttgcctg cacccttccc tcttccctct 180
   ccctctctct gggaccactg ggtacaagag atgggatgct ccgacagcgt ctccaattat 240
   gaaactaatc ttaaccctgt gctgtcagat accctggttt tctggagtca cagtcagtga 300
   ggaggatgtg gtaagaggag gcagagggca ggggtgctgt ggacatgtgg gtggagaagg 360
   gagggtggcc agcactagta aaggaggaat agtgcttgct ggccacaagg aaaaggagga 420
   ggtgtctggg gtgagggagt 440
<210> 181
   <211> 518
   <212> DNA
   <213> Homo sapiens
<220>
   <221> misc_feature
   <222> (41)..(41)
   <223> n is a, c, g, or t
   <400> 181
   gcttttacgg tgatattgtg catgcaaacc aggagcattt ngtgtcttaa gaaaaataat 60
   cttagaacag atggctgtga aaattacacc catgcacaga acaagccaca ggaataatag 120
   ttcaggattt ggtttttctc tttttcttgt aaacctggag ggttgatata ttctttccat 180
   gcagttatta gaacttagtt ttgttccaac agttaaactt gcaatgaaaa gaaaatgtgc 240
   catttttttc actcagaatt attcatagct gtatatttga aactgctaat tacacacgtg 300
   tgatgtatgt tggtttttta gtgcaatttc ttctgtagct attctttgac caaactgtgg 360
   gtattgttaa tattaattta tatttgtctc attttgtatg tatgtgtagt gtgtttgtga 420
   gtatgtgtgg tttataatct gacaaagtca tgaagctcag tttggctgta atttaattcc 480
   ccttccctta tttttattta tttttgtact gtgctgat 518
<210> 182
   <211> 538
   <212> DNA
   <213> Homo sapiens
   <400> 182
   caggtatgtt gcctttatgg tttccccctt ctacatttct tagactacat ttagagaact 60
   gtggccgtta tctggaagta accatttgca ctggagttct atgctctcgc acctttccaa 120
   agttaacaga ttttggggtt gtgttgtcac ccaagagatt gttgtttgcc atactttgtc 180
   tgaaaaattc ctttgtgttt ctattgactt caatgatagt aagaaaagtg gttgttagtt 240
   atagatgtct aggtacttca ggggcacttc attgagagtt ttgtcttgcc atactttgtc 300
   tgaaaaattc ctttgtgttt ctattgactt caatgatagt aagaaaagtg gttgttagtt 360
   atagatgtct aggtacttca ggggcacttc attgagagtt ttgtcaatgt cttttgaata 420
   ttcccaagcc catgagtcct tgaaaatatt ttttatatat acagtaactt tatgtgtaaa 480
   tacataagcg gcgtaagttt aaaggatgtt ggtgttccac gtgttttatt cctgtatg 538
<210> 183
   <211> 498
   <212> DNA
   <213> Homo sapiens
   <400> 183
   tcagtctctc aaagacccca tggtccatcc cctgagggtg gtcagccaag gctcccgttc 60
   cgtgggatgc cataaaagcc gcccagtggg acccacagtc acacagagcg cctcacctgc 120
   atcctctccc ccacaagagc cccaaagatc ccacgggaga ggggagaggg acgcacagca 180
   ctgcctgcca agcgagaatg caggccccgc cccctcggcc cctcaccacc tctttctaca 240
   gcctaattta ttggattccc tattcgtagc catctccgtg gccaatgtga ctaccgtgcc 300
   agcagcgggg gcggcccagc ctctgagtcc cgtggggccc cggctcccac cggtgccaaa 360
   cccagcccct gcggccgtca ccccgccagc ctacactgcc agccgccacc ggggcacacg 420
   ggcctctgct tgccagccag gagtgcggac accatgttcc cagctcagtg ccaaagaggg 480
   gtcaccaggg ggagctgt 498
<210> 184
   <211> 421
   <212> DNA
   <213> Homo sapiens
   <400> 184
   cttactgtgt tttcagtcac ttcctttctg tgaaaggttg cttagctttt tttttgacat 60
   ttgttgttct ttatataaaa ataacagatt ggatagatgt gtacatttgg tgtttgaaat 120
   tctctgaaaa tcccattagg aaaccaggtg tgaaaagggc tcagtagctt ctctgagtgg 180
   cgtttttagc tgactggaag tgcttaatct ggatcgtctt tttttttttt ttttttttca 240
   atattttaaa aggagaattt aaatactgtg cttactgtga aatatatcag ttggtgagcc 300
   gggcgtggtg ggtcacgcct gtaatcccag cactttggga ggccaaggcg ggtggatcac 360
   ccgaggtcag gagttcaaga ccagcctggc caacgtggtg aaagcctgta tctattaaaa 420
   g 421
<210> 185
   <211> 498
   <212> DNA
   <213> Homo sapiens
   <400> 185
   gtcctttgca acattctcat aaaattgggc acagagttcg cattggcgca atatttatgg 60
   gagtgggagg gatggggaaa ataaacttaa ctctacaaaa gcaaactcta atgcatgcaa 120
   gaatcattag gttggcaggt atatgcataa gtgaaaaatc tggaagtgta atggtagaac 180
   ataaaacttg tattgcttct gtttcagtgc aaaaatgtac tagccaatac gcttaagtgt 240
   gtggcccatg aattgaacaa tttaaccttg aagtctatat ccgtgatatt atgtcgattt 300
   ttaactgagg ggaaattaac tagtccagcc taaaatgctt cttttaatct gcattctgtt 360
   tcctcttcta gttgtgccat tactagtgat catgtttttt tccccccttt aatgaaaaca 420
   ataaacatct atttgagaca attaaaatcc ttctgggggc actggaagca caatacggtg 480
   accaatcttg ctttcatt 498
<210> 186
   <211> 426
   <212> DNA
   <213> Homo sapiens
   <400> 186
   gatgcctcct gattatattt cacattttca ggaacaaaat gatttaaaag cattgctaga 60
   aaatctcctt caaaatatcc aatccaaaaa aagaaagaat gtagaaatta tgtggctggc 120
   tgcaacgatt tgccgcaaac tgaatggtat tcgtttcacc tgttgtaaaa gtgccaaaga 180
   caggacatcg atgtcagtga cacttgaaca atgctcaatc ttgagagatg agcaccagtt 240
   acacaaggac ttctttatcc gagcgctgga ttgcatgaga agagaaggat gccgcataga 300
   gaatgtactg aagaatatca aatgcagaaa gtatgctttc aacatgctac agctgatggc 360
   tttccccaag tactacagac ctccagaggg gacttatgga aaagctgaca cctaagttta 420
   ccaaca 426
<210> 187
   <211> 419
   <212> DNA
   <213> Homo sapiens
   <400> 187
   tgaaaggcag gacctggtca ccccagcaag tgctatggac agttcccgga aacggttgcc 60
   cacttcacag gtccatgggt ctgacccttg gactctgcca ggatcaactg cccagagtgc 120
   cagagtttta gccaaaggtg tacttacttc cttatttatc tccaaaagga tggaaactgt 180
   gggagtcaaa gcctattttg ctgagtgttc ccactggatc ctctgtagaa ttagcaggtc 240
   atgctgtcaa aatcatggac aaaggctggg tgcagtggct catgcctata atcccagcac 300
   tttgggaggc caaggtgggc ggatcacctg agctcaggag tttaagacca gcctgggcaa 360
   catggggaaa ctccatctct acaaaatata caaaatatta gccagccatc gtggtgcgt 419
<210> 188
   <211> 481
   <212> DNA
   <213> Homo sapiens
   <400> 188
   gccgtcaccc gaagtcagaa acgtggcatc tcatcggaag aggaggaagg agaggtagac 60
   agtgaagtag agctgacatc aagccagagg tggcctcaga gcctgaacat gcgccagtca 120
   ctatctacct tcagctcaga gaatccatca gatggggagg aaggcacagc tagtgaacct 180
   tcccccagtg gcacacctga agttggcagc accaacactg atgagcggcc agatgagcgg 240
   tctgatgaca tgtgctccca gggctcagaa atcccactgg acccacctcc ttcagaggtc 300
   atccctggcc ctgaacccag ctccctgccc attccacacc aggaacttct cagagagcgg 360
   ggccctccca attctgagga ctcagactgt gacagcactg aattggacaa ctccaacagc 420
   gttgatgcct tgcggccccc agcttccctc cctccatgaa agccactcgt attccttgta 480
   c 481
<210> 189
   <211> 424
   <212> DNA
   <213> Homo sapiens
   <400> 189
   acttctcacc agcagtcgtg gggaacggag gaggacatgg ggaggttgtg gggcctcagg 60
   ctccgggcac caggggccaa cctcaggctc ctaaagagac attttccgcc cactcctggg 120
   acactccgtc tgctccaatg actgagcagc atccacccca ccccatcttt gctgccagct 180
   ctcaggaccg tgccctcgtc agctgggatg tgaagtctct gggtggaagt gtgtgccaag 240
   agctactccc acagcagccc caggagaagg ggctttgtga ccagaaagct tcatccacag 300
   ccttgcagcg gctcctgcaa aaggaggtga aatccctgcc tcaggccaag ggaccaggtt 360
   tgcaggagcc cccctagtgg tatggggctg agccctcctg agggccggtt ctaaggctca 420
   gact 424
<210> 190
   <211> 515
   <212> DNA
   <213> Homo sapiens
   <400> 190
   aatgcagctg acgatccgtt ggtgcatgaa agtcttctaa ccattccaaa atctctttca 60
   gagaaacgag agaacgtcat gtttgtgctg cctctgcatg ggggccactt gggcttcttt 120
   gagggctctg tgctgttccc cgagcccctg acatggatgg ataagctggt ggtggagtac 180
   gccaacgcca tttgccaatg ggagcgtaac aagttgcagt gctctgacac ggagcaggtg 240
   gaggccgacc tggagtgagg cctccggact ctggcacgct ccagcagccc tcctctggaa 300
   gctgcgtccc ctcaccccct gtttcaggtc tcccatctcc ctcagtgacc tggatctgac 360
   ctcacaccat cagcaggggg cacccaccat gcacacctgt ctcggagtag gcagctcttc 420
   ctgggagctc caggctattt ttgtgcttag ttactggttt tctccattgc attgttaggc 480
   atggtgacaa gtgacagagt tcttgccctc tgtcc 515
<210> 191
   <211> 434
   <212> DNA
   <213> Homo sapiens
   <400> 191
   caggtgtatc tgcacagtgg tcgccccaca gcagaccatg tgttcacggg atgcccgcac 60
   aaaacagctg aggcagctac tggagaaggt gcagaacatg tctcaatcca tagaggtctt 120
   ggacaggcgg acccagagag acttgcagta cgtggagaag atggagaacc aaatgaaagg 180
   actggagtcc aagttcaaac aggtggagga gagtcataag caacacctgg ccaggcagtt 240
   taagggctaa cttaaaagag ttttttcaat gctgcagtga ctgaagaagc agtccactcc 300
   catgtaacca tgaaagagag ccagagagct ttttgcacca tgcattttta ctattatttt 360
   ccaatactta gcaccatttc actaaggaac cttgaataca accaggatcc tcctttgcat 420
   gcgactgtag ctgc 434
<210> 192
   <211> 403
   <212> DNA
   <213> Homo sapiens
   <400> 192
   aaaatgttgc gttctcagtc caaaaagaag tggaaaagaa tctgaagtca tgcttggaca 60
   atgttaatgt tgtgtccgta gacactgcca gaacactatt caaccaagtg atggaaaagg 120
   agtttgaaga cggcatcatt aactggggaa gaattgtaac catatttgca tttgaaggta 180
   ttctcatcaa gaaacttcta cgacagcaaa ttgccccgga tgtggatacc tataaggaga 240
   tttcatattt tgttgcggag ttcataatga ataacacagg agaatggata aggcaaaacg 300
   gaggctggga aaatggcttt gtaaagaagt ttgaacctaa atctggctgg atgacttttc 360
   tagaagttac aggaaagatc tgtgaaatgc tatctctcct gaa 403
<210> 193
   <211> 355
   <212> DNA
   <213> Homo sapiens
   <400> 193
   ggctgggagt tgattgagcc aacactggat caattagatc aaaagatgag agaagctgaa 60
   acagaaccgc atgagggaaa gaggaaagtg gaatctctgt ggcccatctt caggatccac 120
   caccagaaaa cccgctacat cttcgacctc ttttacaagc ggaaagccta cagcagagaa 180
   ctcttagata tatgttataa agaaggctta gcagacaaaa acctgttggc aaaatggaaa 240
   aagcaaggta taggaaactt gtgctgcctg cggtgcattc agacacggga caccaacttc 300
   gggacgaact gcatctgccg cgtgcccaaa agcaagctgg aagtgggccg catca 355
<210> 194
   <211> 527
   <212> DNA
   <213> Homo sapiens
   <400> 194
   gggtggttct ggccaggaag gcacaaggta gctgtgggcc aagacaccag ccctgtccta 60
   gcccttcagt aagaccttgc caggagagga gaaggatgcc tgggtgccag gcaagacaag 120
   cccctcagca ggagagaggc ccagaggctc cagctggcca ccgtgcccca caagatggcc 180
   cctgtgtggt tccctttacc ttggcttcct ggcccagtcc ctgcctctcc acctgcaccc 240
   tgcttcctgg cccagtccca ggttggagtc cctctgcata gctgactact catgcattgc 300
   tcaaagctgg cttttcacat taagtcaaca ccaaacgtgg ttgccacatt tcatcagaca 360
   gacacctccc tctggagatg cagttgagtg acaaccttgt tacattgtag cctagaccaa 420
   ttctgtgtgg atatttaagt gaacatgttt acaatttttg tatatatcac tctctccctc 480
   tcctgaaaga ccagagattg tgtattttca gtgtcccatg ttccgac 527
<210> 195
   <211> 531
   <212> DNA
   <213> Homo sapiens
   <400> 195
   aacagaaagt ctcagcccag gatggggctt cttcaacagg cccctgccct cctgaagcct 60
   cagtccttca ccttgccagg tgccgtttct cttccgtgaa ggccactgcc caggtcccca 120
   gtgcgccccc tagtggccat agcctggtta aagttcccca gtgcctcctt gtgatagacc 180
   ttcttctccc acccccttct gcccctgggt ccccggccat ccagcggggc tgccagagaa 240
   ccccagacct gcccttacag tagtgtagcg ccccctccct ctttcggctg gtgtagaata 300
   gccagtagtg tagtgcggtg tgcttttacg tgatggcggg tgggcagcgg gcggcggcgt 360
   ccgcgcagcc gtctgtcctt gatctgcccg cggcggcccg tgttgtgttt tgtgctgtgt 420
   ccagcgctaa ggcgaccccc tcccccgtac tgacttctcc tataagcgct tctcttcgca 480
   tagtcacgta gctcccaccc caccctcttc ctgtgtctca cgcaagtttt a 531
<210> 196
   <211> 441
   <212> DNA
   <213> Homo sapiens
   <400> 196
   cttggcctgc taaggtcttg gaacttgcct gcctttccat ccatggccag cagcacctgc 60
   cctacctgcc ccacttgtcc ttagcctgga cctctgacag cagcatctct accttctccc 120
   cagctcccag gaccacaggc tcaggcaggg cctccatggg ccccagggga acactgggga 180
   cttggcctct ctctagggta catggtgctg ggagaggcag cccaggaagt ctcatctggg 240
   gagcaggcag ccagcatctg ggccttggcc tggagcacaa agaccctggc tttcattttc 300
   tctcaggtga aaggaaatta aggcaacaaa agaagcccgg ctcctggtca cctaggaagc 360
   ctcagattcc ttcccatgga gggagggagt ggtttgcagg tggccaagtt cctctaactt 420
   ggctcacact cgacatgaaa a 441
<210> 197
   <211> 552
   <212> DNA
   <213> Homo sapiens
   <400> 197
   gcagtcccta ttagctaaaa gcccattaag acaagaaaca caggaagccc ctggtcccag 60
   agaagaagca aagggccagg tagaggccag aagggagtct ttggatcctg tccaggagcc 120
   tgggggccag gcagaggctg atggagatgt tccagggccc agaggggaag ctgagggcca 180
   ggcagaggct aaaggagatg cccctgggcc cagaggggaa gctgagggcc aggcagaggc 240
   taaaggagat gcccctgggc ccagagggga agctgggggc caggcagagg ccagggagaa 300
   tggagaggag gccaaggaac ttccagggga aacactggag tctaagaaca cccaaaatga 360
   ctttgaggtg cacattgttc aagtggagaa tgatgagatc tagatcttaa gatacaggta 420
   cccacagaag tctcagtgcc agaacataag ccctgaagtg ggcaggggaa atgtacgctg 480
   ggacaaggac catctctgtg ccccctgtct ggtcccagta ggtatcaggt ctttctatgc 540
   agctcaggga ga 552
<210> 198
   <211> 467
   <212> DNA
   <213> Homo sapiens
   <400> 198
   agcagggagg gcttctgcca tttctgagat caaaacggtt ttactgcagc tttgtttgtt 60
   gtcagctgaa cctgggtaac tagggaagat aatattaagg aagacaatgt gaaaagaaaa 120
   atgagcctgg caagaatgcg tttaaacttg gtttttaaaa aactgctgac tgttttctct 180
   tgagagggtg gaatatccaa tattcgctgt gtcagcatag aagtaactta cttaggtgtg 240
   ggggaagcac cataactttg tttagcccaa aaccaagtca agtgaaaaag gaggaagaga 300
   aaaaatattt tcctgccagg catggaggcc cacgcacttc gggaggtcga ggcaggagga 360
   tcacttgagt ccagaagttt gagatcagcc tgggcaatgt gataaaaccc catctctaca 420
   aaaagcataa aaattagcca agtgtggtag agtgtgcctg aagtccc 467
<210> 199
   <211> 562
   <212> DNA
   <213> Homo sapiens
   <400> 199
   tcactcaaca gcactgtgat gtattatttt caatgaggtg cctttcttaa ctgaccaaat 60
   gctgccttgt ttggccccta aatcaataaa atatgttaaa atttgtatcc cctgttgtgg 120
   catttttttt agataatcta agctagaaaa atgacattga attctggacc tggctggaag 180
   gaaaagaagc cctttcttgt cgctggcagc tgtgtggtag gaggtccaag tatgtgcata 240
   tgagataagc ctgcaacctc ttgaccttca gctcctatgc aggcttctct tgagcccaga 300
   gacaaggcag cttggtctag tggagatagc actgtgcttg gagttcaggg gacctaggac 360
   aaatcccagc cagttagtta ttcactgtgc tcctgtttcc tcagctgaaa aaggaagttg 420
   gttatgccac cttcttggcc ttaatggcat taaatgaaat ttataggaag aaggtttttg 480
   ctcagtacct ggcatgcaac agacattgga taaatgttag ttggatccag atatacacag 540
   aaagatatct gcttcctgcc ag 562
<210> 200
   <211> 432
   <212> DNA
   <213> Homo sapiens
<220>
   <221> misc_feature
   <222> (34)..(34)
   <223> n is a, c, g, or t
   <220>
   <221> misc_feature
   <222> (46)..(46)
   <223> n is a, c, g, or t
   <220>
   <221> misc_feature
   <222> (104)..(104)
   <223> n is a, c, g, or t
   <220>
   <221> misc_feature
   <222> (108)..(108)
   <223> n is a, c, g, or t
   <220>
   <221> mise_feature
   <222> (274)..(274)
   <223> n is a, c, g, or t
   <400> 200
   ctttcccaga gacccgggga tggattggcc tccngggcgc aggggngggt gcggcagggc 60
   aggagcttgg cagagagata gccgggctcc agggagtggg gagnaganag ggggagaccc 120
   ctttgccttc ccccctcagc aaggggctgc ttctggggct ccctgcctgg atccagctct 180
   gggagccctg ccgaggtgtg gctgtgaggt cagggtttta gagagcagtg gcagaggtag 240
   ccccctaaat gggcaagcaa ggagcccccc aaanacacta ccactcccca tccccgtctg 300
   accaagggct gacttctcca ggacctagtc ggggggtggc tgccaggggg caaggagaaa 360
   gcaccgacaa tctttgatta ctgaaagtat ttaaatgttt gccaaaaaca acagccaaaa 420
   caaccaaact at 432
<210> 201
   <211> 353
   <212> DNA
   <213> Homo sapiens
   <400> 201
   cgccgctgcg aattctcgga caaaactgtc aacagcccgg gcgcgccttt tggctctgcg 60
   ggtccctcta tttatgcaaa gccgacctat gctacagccc cccaaccccc gacctggggt 120
   agggaggaag agggtgccgg ggaagggagt ccgccctgtc caggcactag aggctccctt 180
   gacgtttggc agatgaaaaa caactaagcc tttttgaggt gtagagattc tcaggtccag 240
   gcgttaaaaa ataatggtca aaagaataat acaaaaatag taaaggtctt gaagaatgcc 300
   agcgaagcaa ttctttttta tttgaggaca cttgtctggt gtactttttc atg 353
<210> 202
   <211> 546
   <212> DNA
   <213> Homo sapiens
   <400> 202
   atcaatcagc tttgctcaca aactaaagga agttttgtga atggggtgtt tgaggtacat 60
   aagaaaaatg taaggggtga attcacttat tatgaaatac aagataatac agggaagatg 120
   gaagtggtgg tgcatggacg actgaacaca atcaactgtg aggaaggaga taaactgaaa 180
   ctcaccagct ttgaattggc accgaaaagt gggaataccg gggagttgag atctgtaatt 240
   catagtcaca tcaaggtcat caagaccagg aaaaacaaga aagacatact caatcctgat 300
   tcaagtatgg aaacttcacc agactttttc ttctaaaatc tggatgtcat tgacgataat 360
   gtttatggag ataaggtcta agtccctaaa aaaatgtaca tatacctggt tgaaatacaa 420
   cactatacat acacaccacc atatatacta gctgttaatc ctatggaatg ggggtattgg 480
   gagtgctttt ttaatttttc atagtttttt tttaataaaa tggcatattt tgcatctaca 540
   acttct 546

<210> 203
   <211> 246
   <212> DNA
   <213> Homo sapiens
   <400> 203
   ggcttcctgg ccaactactg ccagggtcag tgcgcgctgc ccgtcgcgct gtcggggtcc 60
   ggggggccgc cggcgctcaa ccacgctgtg ctgcgcgcgc tcatgcacgc ggccgccccg 120
   ggagccgccg acctgccctg ctgcgtgccc gcgcgcctgt cgcccatctc cgtgctcttc 180
   tttgacaaca gcgacaacgt ggtgctgcgg cagtatgagg acatggtggt ggacgagtgc 240
   ggctgc 246
<210> 204
   <211> 470
   <212> DNA
   <213> Homo sapiens
   <400> 204
   ggagctgctg ggacagggga ttgattatga gaagatcctg aagctcacgg ctgacgccaa 60
   gtttgagtca ggcgatgtga aggccacagt ggcagtgctg agtttcatcc tctccagtgc 120
   ggccaagcac agtgtcgatg gcgaatcctt gtccagtgaa ctgcagcagc tggggctgcc 180
   caaagagcac gcggccagcc tgtgccgctg ttatgaggag aagcaaagcc ccttgcagaa 240
   gcacttgcgg gtctgcagcc tacgcaaact gaagcaggcc cagaccctga tgagctccct 300
   gggctgagga gaagggtgtt ccaggcctgt gtggagccgc cctgcccgta tggagtcacg 360
   ccctctgaac tgctcttcgg gaggcagccc tggttctagg atgctgaggc cctggcccgg 420
   actctggcct cccagatccc cagctgcctc acttctctct tgagaacttg 470
<210> 205
   <211> 469
   <212> DNA
   <213> Homo sapiens
   <400> 205
   gaactgcctg gttggagcga atctgctagt gaagattggg gacttcggca tgtccagaga 60
   tgtctacagc acggattatt acagggtggg aggacacacc atgctcccca ttcgctggat 120
   gcctcctgaa agcatcatgt accggaagtt cactacagag agtgatgtat ggagcttcgg 180
   ggtgatcctc tgggagatct tcacctatgg aaagcagcca tggttccaac tctcaaacac 240
   ggaggtcatt gagtgcatta cccaaggtcg tgttttggag cggccccgag tctgccccaa 300
   agaggtgtac gatgtcatgc tggggtgctg gcagagggaa ccacagcagc ggttgaacat 360
   caaggagatc tacaaaatcc tccatgcttt ggggaaggcc accccaatct acctggacat 420
   tcttggctag tggtggctgg tggtcatgaa ttcatactct gttgcctcc 469
<210> 206
   <211> 512
   <212> DNA
   <213> Homo sapiens
   <400> 206
   aggaggcaag gttggctcgg agctccccgg agcagcccag gcccagcacc tccaaggcag 60
   tctcaccacc ccacctggat ggaccgccta gccccaggag ccccgtcata ggaagtgagg 120
   tcttcctgcc caacagcaac cacgtggcca gtggcgccgg ggaggcagag gaacgcgttg 180
   tggtgatcag cagctcggaa gactcagatg ccgaaaactc gtcctcccga gagctggatg 240
   acagcagcag tgagtccagt gacctccagc tggaaggccc cagcaccctc agggtcctgg 300
   acgagaacct tgctgacccc caagcagaag acagacctct ggttttcttt gacctcaaga 360
   ttgacaatga aagtgggttc tcctggggct acccccaccc ctttctaatt tagtctctga 420
   gtcccaaaaa gaagtgcagg cagagcatct gccaggccca ggagagctct gagctctggc 480
   caacaactgc agccaggctg ggcagagcac tc 512
<210> 207
   <211> 488
   <212> DNA
   <213> Homo sapiens
   <400> 207
   gagggtggca aggaacttcc tggctgcctg gggagacagc agaacccagg ccacacgctg 60
   gaagccggct ggtttctgct ccgtcattgc attcggaaag gcgaccccga acttcgagcc 120
   cacgtgattg acaagttcct attgttgccc ttccactccg gatgggaccc tgaccacgga 180
   ggcctctttt acttccagga tgctgataac ttctgcccca cccagctgga gtgggccatg 240
   aagctctggt ggccacacag tgaagccatg attgccttcc tcatgggtta cagtgacagt 300
   ggggaccctg tgctgctgcg cctcttctac caagtggctg agtacacctt ccgccagttt 360
   cgcgatcccg agtacgggga atggtttggc tacctgagcc gagagggcaa ggtggccctc 420
   tccatcaagg gaggtccttt caaaggctgc ttccacgtgc cgcggtgcct agccatgtgc 480
   gaggagat 488
<210> 208
   <211> 459
   <212> DNA
   <213> Homo sapiens
   <400> 208
   ttcagaccca gactcttttc aagactacat taagtcctat ttggaacaag cgagtcggat 60
   ctggtcatgg ctccttgggg cggcgatggt aggggccgtc ctcactgccc tgctggcagg 120
   gcttgtgagc ttgctgtgtc gtcacaagag aaagcagctt cctgaagaaa agcagccact 180
   cctcatggag aaagaggatt accacagctt gtatcagagc catttataaa aggcttaggc 240
   aatagagtag ggccaaaaag cctgacctca ctctaactca aagtaatgtc caggttccca 300
   gagaatatct gctggtattt ttctgtaaag accatttgca aaattgtaac ctaatacaaa 360
   gtgtagcctt cttccaactc aggtagaaca cacctgtctt tgtcttgctg ttttcactca 420
   gcccttttaa cattttcccc taagcccata tgtctaagg 459
<210> 209
   <211> 533
   <212> DNA
   <213> Homo sapiens
   <400> 209
   gggaggggct tggctaggta gttctgtgtg gcggtggtca ttcccctcat taaacaccag 60
   ttcttggtga cgccaggggc tggtaggtca ttcaaagctg tggccagctc acgcctgctt 120
   cctccctccc tgccctgctg aatcctaaag ctgtgcctat atctgtgatt tgaatgaggg 180
   agccctttgg ggcaaattca ggtgccccca ttgcctcagg ctggccctgg tcccaggtgg 240
   cagcggttga ggaggggtac agggctctca agcctgaggt tttcttctct gggcttaatt 300
   ttctcttggg gtacgtgcct gacagtgttt aaggtgtccg ttgaactgga gttgcagact 360
   tttaaataga tgaccccttc agatcatctg tgcctacctc ctgcccatca ggcgtctaca 420
   ctgtcactca gacacctgtg gcatgtggag gagactgccc tgtcctgagc ctggaaaatg 480
   tgaaactgtc tcctgcaacc tgctgggcat gtgggcctgg ctgtgttcaa ttg 533
<210> 210
   <211> 438
   <212> DNA
   <213> Homo sapiens
   <400> 210
   gcttccggga aggtgtctca agtgggtggg cagacttctg acgaagccct gagcatgctg 60
   tctgaaggtt ctgatgccag cacaattgaa attcacactg caagtgaatc ctgcaacaaa 120
   aatgagggtg accctgctct cccaacccat ggagacctat gaaggggatg tgctgggggt 180
   ccagacccca tattcctcag actcaacaat tcttgttctt tagaactgtg ttctcacctt 240
   cccaacactg cactgccgaa gtgtagcggc ccccaaacct tgctctcatc accagctaga 300
   gcttcttccc gaagggcctt taggatagga gaaagggttc atgcacacac gtgtgagaat 360
   ggaagagccc cctccagacc actctacagc tgctctagcc ttagttgcca ctaggaagtt 420
   ttctgaggct ggctgtaa 438
<210> 211
   <211> 135
   <212> DNA
   <213> Homo sapiens
   <400> 211
   cctgaggccc atcaaagtgg acagccaaga gcacaagatc atcctctatg aaaaccccaa 60
   cttcaccggg aagaagatgg aaatcataga tgacgatgta cccagcttcc acgcccatgg 120
   ctaccaggag aaggt 135
<210> 212
   <211> 440
   <212> DNA
   <213> Homo sapiens
   <400> 212
   tcaaggcgct aggcgacgag ctgcaccagc gcaccatgtg gcggcgccgc gcgcggagcc 60
   ggagggcgcc ggcgcccggc gcgctcccca cctactggcc ttggctgtgc gcggccgcgc 120
   aggtggcggc gctggcggcc tggctgctcg gcaggcggaa cttgtaggaa cgcggggctt 180
   cttggtgggg ccggagccga gacccagccg gagcgagcaa caggttggtg aaaaccctgt 240
   gtccttggag aaagctggtt cccgttttcc agagggggag cccagagctt gaaaggccgc 300
   ggttggcact tcgagaagga agtggagagt aaagacagcg cctggagcga tcgtagaaac 360
   acagaatggg actggggaag ccctttggaa atccagctgc agaaacagac accccaatgc 420
   tatttacata cagctctata 440
<210> 213
   <211> 489
   <212> DNA
   <213> Homo sapiens
   <400> 213
   aagtctgtag tctttatgat cctaaaaggg aaaattgcct tggtaacttt cagattcctg 60
   tggaattgtg aattcatact aagctttctg tgcagtctca ccatttgcat cactgaggat 120
   gaaactgact tttgtctttt ggagaaaaaa aactgtactg ttgttcaaga gggctgtgat 180
   taaaatcttt aagcatttgt tcctgccaag gtagttttct tgcattttgc tctccattca 240
   gcatgtgtgt gggtgtggat gtttataaac aagactaagt ctgacttcat aagggctttc 300
   taaaaccatt tctgtccaag agaaaatgac tttttgcttt gatattaaaa attcaatgag 360
   taaaacaaaa gctagtcaaa tgtgttagca gcatgcagaa caaaaacttt aaactttctc 420
   tctcactata cagtatattg tcaatgtgaa agtgtggaat ggaagaaatg tcgatcctgt 480
   tgtaactga 489
<210> 214
   <211> 514
   <212> DNA
   <213> Homo sapiens
   <400> 214
   gagccatcgt gggaagactt tacaggacat acctgaagac tttctggaaa tggatcttgc 60
   aaaaaatgag cacagagttc acgtgcaaat ggagccggta tgacacactt tcttacaaca 120
   acagccactg tgttggctgg agagggatgg ggtgggccca acggggacac aaggaggcag 180
   aggagctaac ccctctactc cactttcaaa actacatttt aaagggaatg tgtatgtgaa 240
   gagcactacc aacatcgctt ttgttttgtt ttgttttgtt ttaagctttt tttttttgct 300
   tgtttttaaa gccaaaacaa aaaacaacca agcactcttc catatataaa tctggctgta 360
   ttcagtagca atacaagaga tatgtagaaa gactctttgg ttcacattcc gatattaaaa 420
   tagtgacatg aactggcaaa gtggttttaa aagctttcac gtgggataaa tgattttctt 480
   tttttctttt ctttcttcct atggtcttgt ctga 514
<210> 215
   <211> 543
   <212> DNA
   <213> Homo sapiens
   <400> 215
   aatatatttc ccaccaagta cctatatatg tatataaaca aacacattat ctatatataa 60
   cgccacactg tcttctgttt agtgtatggg gaaagaccaa tccaactgtc catctgtggc 120
   tgggacagcc agggggtgtg cccacggctg acccaggggt gtgcacacgg ctgagctggg 180
   agtcccgctg gtctccctga ggactgaggg tgaacttcgc tctttgcctt aaacctcttt 240
   atttcattgc agtaatagtt ttacgttgta cataatagtg taaacctttt taaaaaggaa 300
   agtataaaaa caaaagttgt aatttaaaag tctgaataac catctgctgc ttaggaaact 360
   caatgaaatg acatgccttt ttagcaggaa gcaaagttgg tttctgtttt ttgttttctt 420
   tgttgtttta gtttataaaa catgtgcatt ttacagttca gtatcaaata tttataatct 480
   tatgagaaat gaatgaatgt ttctatttac aactgtgctt atcaaaattg tgaacacccc 540
   cac 543
<210> 216
   <211> 518
   <212> DNA
   <213> Homo sapiens
   <400> 216
   ccaagagatg agctccgtgg cctactccaa ccttgcggtg aaagatcgca aagcagtggc 60
   cattctgcac taccctgggg tagcctcaaa tggaaccaag gccagtgggg ctcccactag 120
   ttcctcggga tctccaatag gctctcctac aaccacccct cccactaaac ccccatcctt 180
   caacctgcac cccgcccctc acttgctggc tagtatgcag ctgcagaaac ttaatagcca 240
   gtatcagggg atggctgctg ccactccagg ccaacccggg gaggcaggac ccctgcaaaa 300
   ctgggacttt ggggcccagg cgggaggggc agaatcactc tctccttctg ctggtgccca 360
   gagccctgct atcatcgatt cggacccagt ggatgaggaa gtgctgatgt cgctggtggt 420
   ggaactgggg ttggaccgag ccaatgagct tccggagctg tggctggggc agaatgagtt 480
   tgacttcact gcggactttc catctagctg ctaatgcc 518
<210> 217
   <211> 480
   <212> DNA
   <213> Homo sapiens
   <400> 217
   gcaccagatg caacctcact atggtatgct ggccagcacc ctctcctggg ggtggcaggc 60
   acacagcagc cccccagcac taaggccgtg tctctgagga cgtcatcgga ggctgggccc 120
   ctgggatggg accagggatg ggggatgggc cagggtttac ccagtgggac agaggagcaa 180
   ggtttaaatt tgttattgtg tattatgttg ttcaaatgca ttttgggggt ttttaatctt 240
   tgtgacagga aagccctccc ccttcccctt ctgtgtcaca gttcttggtg actgtcccac 300
   cggagcctcc ccctcagatg atctctccac ggtagcactt gaccttttcg acgcttaacc 360
   tttccgctgt cgccccaggc cctccctgac tccctgtggg ggtggccatc cctgggcccc 420
   tccacgcctc ctggccagac gctgccgctg ccgctgcacc acggcgtttt tttacaacat 480
<210> 218
   <211> 472
   <212> DNA
   <213> Homo sapiens
   <400> 218
   tcatgtagct cagctatggc acccccatga acaagactat aagaaaagtt cccttgtttt 60
   cacagctatc acatggatat cctttagttc ttcagcctct aaacctactc tgtattcaat 120
   ttataatgcc aattttcgga gagggatgaa agagactttt tgcatgtcct ctatgaaatg 180
   ttaccgaagc aatgcctata ctatcacaac aagttcaagg atggccaaaa aaaactacgt 240
   tggcatttca gaaatccctt ccatggccaa aactattacc aaagactcga tctatgactc 300
   atttgacaga gaagccaagg aaaaaaagct tgcttggccc attaactcaa atccaccaaa 360
   tacttttgtc taagttctca ttctttcaat tgttatgcac cagagattaa aaagctttaa 420
   ctataaaaac agaagctatt tacatatttg ttttcactca actttccaag gg 472
<210> 219
   <211> 309
   <212> DNA
   <213> Homo sapiens
   <400> 219
   gtccgcccag aagccataga cgagacgtag gtagccgtag ttggacggac gggcagggcc 60
   ggcggggcag ccccctccgc gcccccggcc gtcccccctc atcgccccgc gcccaccccc 120
   atcgcccctg cccccggcgg cggcctcgcg tgcgaggggg ctcccttcac ctcggtgcct 180
   cagttccccc agctgtaaga cagggacggg gcggcccagt ggctgagagg agccggctgt 240
   ggagccccgc ccgcccccca ccctctaggt ggcccccgtc cgaggaggat cgttttctaa 300
   gtgcaatac 309
<210> 220
   <211> 560
   <212> DNA
   <213> Homo sapiens
   <400> 220
   ctgtgcagca gctgaccgac agcactcaaa ttaaaatgga cattttggcg caagttttac 60
   agattttatt aaagtcgaag ctattggtct tggaagatga aaatgcaaat gttgatgagg 120
   tggaattgaa gccagatacc ttaataaaat tatatcttgg ttataaaaat aagaaattaa 180
   gggttaacat caatgtgcca atgaaaaccg aacagaagca ggaacaagaa accacacaca 240
   aaaacatcga ggaagaccgc aaactactga ttcaggcggc catcgtgaga atcatgaaga 300
   tgaggaaggt tctgaaacac cagcagttac ttggcgaggt cctcactcag ctgtcctcca 360
   ggttcaaacc tcgagtccct gtgatcaaga aatgcattga cattctaatt gagaaagaat 420
   atttggagcg agtggatggt gaaaaggaca cctacagtta cttggcttaa cccttctgga 480
   agggtctgac tgtgtgaccc gcagcaaata gttcatgttg gaaagaatga aaacaacttc 540
   aagttcatag gcagccagcc 560
<210> 221
   <211> 280
   <212> DNA
   <213> Homo sapiens
   <400> 221
   gtcagacggg cagaagtgcc gagtgtgtct ggcctggctg gcctggcaga acccccacat 60
   gctcttcctg gatgaaccca ccaatcacct ggatatcgag accatcgacg ccctggcaga 120
   tgccatcaat gagtttgagg gtggtatgat gctggtcagc catgacttca gactcattca 180
   gcaggttgca caggaaattt gggtctgtga gaagcagaca atcaccaagt ggcctggaga 240
   catcctggct tacaaggagc acctcaagtc caagctggtg 280
<210> 222
   <211> 524
   <212> DNA
   <213> Homo sapiens
   <400> 222
   tgcacagaag ttagcgctat ccccactgag tctcggcaaa gaaaatcttg cagagtcctc 60
   caaaccaaca gctggtggca gcagatcaca aaaggtcaaa gttgctcagc ggagcccagt 120
   agattcaggc accatcctcc gagaacccac cacgaaatcc gtcccagtca ataatcttcc 180
   tgagagaagt ccgactgaca gccccagaga gggcctgagg gtcaagcgag gccgacttgt 240
   ccccagcccc aaagctggac tggagtccaa gggcagtgag aactgtaagg tccagtgaag 300
   gcactttgtg tgtcagtacc cctgggaggt gccagtcatt gaatagataa ggctgtgcct 360
   acaggacttc tctttagtca gggcatgctt tattagtgag gagaaaacaa ttccttagaa 420
   gtcttaaata tattgtactc tttagatctc ccatgtgtag gtattgaaaa agtttggaag 480
   cactgatcac ctgttagcat tgccattcct ctactgcaat gtaa 524
<210> 223
   <211> 550
   <212> DNA
   <213> Homo sapiens
   <400> 223
   tctcgggacg catgaccttc acgagcaata agtccatgga gatcgaggtg ttggtggacg 60
   ccgaccctgt tgtggacagc tctcagaagc gctaccgggc cgccagtgcc ttcttcacct 120
   acgtgtcgct gagccaggaa ggcaggtcgc tgcctgtgcc ccagctggtg cccgagaccg 180
   aggacgagaa gaagcgcttt gaggaaggca aagggcggta cctgcagatg aaggcgacga 240
   tcagggccac gcggacgctc agccctagac tccctcctcc tgccactggt gcctcgagta 300
   gccatggcaa cgggcccagt gtccagtcac ttagaagttc cccccttggc caaaaaccca 360
   attcacattg agagctggtg ttgtctgaag ttttcgtatc acagtgttaa cctgtactct 420
   ctcctgcaaa cctacacacc aaagctttat ttatatcatt ccagtatcaa tgctacacag 480
   tgttgtcccg agcgccggga ggcgttgggc agaaaccctc gggaatgctt ccgagcacgc 540
   tgtagggtat 550
<210> 224
   <211> 233
   <212> DNA
   <213> Homo sapiens
   <400> 224
   gatgaatgtt ttgcacttta ttgggaagac aacaagtttt accgggcaga agttgaagcc 60
   ctccattctt cgggtatgac agcagttgtt aaattcattg actacggaaa ctatgaagag 120
   gtgctactga gcaatatcaa gcccattcaa acagaggcat gggaggaaga aggcacctac 180
   gatcaaactc tggagttccg taggggaggt gatggccagc caagacgatc cac 233
<210> 225
   <211> 419
   <212> DNA
   <213> Homo sapiens
   <400> 225
   ctgctgccac ataaggtctt tgaaggaaat cgcccaacca actctattgt gttcaccaag 60
   ctcacaccat tcatgcttgg agccttggtc gccatgtatg agcacaagat cttcgttcag 120
   ggcatcatct gggacatcaa cagctttgac cagtggggag tggagctggg aaagcagctg 180
   gctaagaaaa tagagcctga gcttgatggc agtgctcaag tgacctctca cgacgcttct 240
   accaatgggc tcatcaactt catcaagcag cagcgcgagg ccagagtcca ataaactcgt 300
   gctcatctgc agcctcctct gtgactcccc tttctcttct cgtccctcct ccccggagcc 360
   ggcactgcat gttcctggac accacccaga gcaccctctg gttgtgggct tggaccacg 419
<210> 226
   <211> 265
   <212> DNA
   <213> Homo sapiens
   <400> 226
   atggcaaaaa tctccagccc tacagagact gagcggtgca ttgagtccct gattgctgtt 60
   ttccagaagt atgctggaaa ggatggttac aaccgcaatc tctccaagac ggagttccta 120
   agcttcatga atacagagct ggctgccttt acaaagaacc agaaggaccc cggtgtcctt 180
   gaccacatga agaaactgga tgtcagcagt gatgggcagt tagatttccc aaaatttctt 240
   aatctgattg gtggcctagc tgtgg 265
<210> 227
   <211> 467
   <212> DNA
   <213> Homo sapiens
   <400> 227
   gggaccggga tttcatctgg tgtgatagac acctctctac tatataacga gtacattgtc 60
   tatgatattg ctcaggtaaa tctgaagtat ctgctgaaac tgaaattcaa ttttaagacc 120
   tccctgtggt aattgggaga ggtagccgag tcacacccgg tggctgtggt atgaattcac 180
   ccgaagcgct tctgcaccaa ctcacctggc cgctaagttg ctgatgggta gtacctgtac 240
   taaaccacct cagaaaggat tttacagaaa cgtgttaaag gttttctcta acttctcaag 300
   tcccttgttt tgtgttgtgt ctgtggggag gggttgtttt ggggttgttt ttgttttttc 360
   ttgccaggta gataaaactg acatagagaa aaggctggag agagattctg ttgcatagac 420
   tagtcctatg gaaaaaacca aagcttcgtt agaatgtctg ccttact 467
<210> 228
   <211> 277
   <212> DNA
   <213> Homo sapiens
   <400> 228
   aagaggggcc tgatgagact ccactcaggt gcacacatca ccaggtgcat ctgcaggcac 60
   cgggctggct gcttgcagcc aggagaaggt cagcgagaag gagtgtatga gtgtgagtgt 120
   gtgtgcatgg aagttggggc actgggcgtc tgactccctc cccacccaag agaggaagga 180
   cccctcacca cccccactgg cgagacagtt tactttgccg acttgccatg tttttgccaa 240
   aaccaagatt ttgaaggaaa tgagtggcca gcgccag 277

<210> 229
   <211> 506
   <212> DNA
   <213> Homo sapiens
<220>
   <221> misc_feature
   <222> (198)..(198)
   <223> n is a, c, g, or t
   <220>
   <221> misc_feature
   <222> (201)..(201)
   <223> n is a, c, g, or t
   <220>
   <221> misc_feature
   <222> (429)..(429)
   <223> n is a, c, g, or t
   <220>
   <221> misc_feature
   <222> (439)..(439)
   <223> n is a, c, g, or t
   <400> 229
   gactgggcct ggtacaagat cactgactct gaggacaagg ccctcatgaa cggctccgag 60
   agcaggttct tcgtgagttc ctcgcagggc cggtcagagc tacacattga gaacctgaac 120
   atggaggccg accccggcca gtaccggtgc aacggcacca gctccaaggg ctccgaccag 180
   gccatcatca cgctccgncg ntgcgcagcc acctggccgc cctctggccc ttcctgggca 240
   tcgtggctga ggtgctggtg ctggtcacca tcatcttcat ctacgagaag cgccggaagc 300
   ccgaggacgt cctggatgat gacgacgccg gctctgcacc cctgaagagc agcgggcagc 360
   accagaatga caaaggcaag aacgtccgcc agaggaactc ttcctgaggc aggtggcccg 420
   aggacgctnc cctgctccng cgtctgcgcc gccgccggag tccactccca gtgcttgcaa 480
   gattccaagt tctcacctct taaaga 506
<210> 230
   <211> 536
   <212> DNA
   <213> Homo sapiens
   <400> 230
   cctgtgccct ggcagttagc caagaggcgg ataagtgccc caccttagaa cagtatgcca 60
   tgagagcgtt tgccgacgca ctggaggtca tccccatggc cctctctgaa aacagtggca 120
   tgaatcccat ccagactatg accgaagtcc gagccagaca ggtgaaggag atgaaccctg 180
   ctcttggcat cgactgtttg cacaagggga caaatgatat gaagcaacag catgtcatag 240
   aaaccttgat tggcaaaaag caacagatat ctcttgcaac acaaatggtt agaatgattt 300
   tgaagattga tgacattcgt aagcctggag aatctgaaga atgaagacat tgagaaaact 360
   atgtagcaag atccacttct gtgattaagt aaatggatgt ctcgtgatgc gtctacagtt 420
   atttattgtt acatcctttt ccagacactg tagatgctat aataaaaata gctgtttggt 480
   aaccatagtt tcacttgttc aaagccgtgt aatcgtgggg gtactatctc aactgc 536
<210> 231
   <211> 389
   <212> DNA
   <213> Homo sapiens
   <400> 231
   ccatcgccac agaagcggta ccaggacacc ccgggcgtgg agcacattcc cgtggtgcag 60
   attgacctct ccgtcccctt gaaggttcca gggctgccta tgtcagatca gtatgtgaag 120
   ctggaggagg agcggcggca ccggcagaag ctggagaagg acaagaggag gaaaaagagg 180
   aaggagaagg agaagaaggg caagcgccgc cacagctcgc tgcccacgga gagcgacgag 240
   gacatcgccc ctgcccagca ggtggacatc gtcacagagg agatgcctga gaatgctctg 300
   cccagcgacg aggatgacaa agaccccaac gacccctaca gggctctgga tattgacctg 360
   gataagccct tagccgacag cgagaaact 389
<210> 232
   <211> 525
   <212> DNA
   <213> Homo sapiens
   <400> 232
   ctcttcacca ctgtggagac cctggagaag gaaaacccct ggtactgccc ttcctgcaag 60
   cagcaccagc tggcaaccaa gaagctggac ctgtggatgc tgccggagat tctcatcatc 120
   cacctgaaac gcttttccta caccaagttc tcccgagaga agctggacac cctcgtggag 180
   tttcctatcc gggacctgga cttctctgag tttgtcatcc agccacagaa tgagtcgaat 240
   ccggagctgt acaaatatga cctcatcgcg gtttccaacc attatggggg catgcgtgat 300
   ggacactaca caacatttgc ctgcaacaag gacagcggcc agtggcacta ctttgatgac 360
   aacagcgtct cccctgtcaa tgagaatcag atcgagtcca aggcagccta tgtcctcttc 420
   taccaacgcc aggacgtggc gcgacgcctg ctgtccccgg ccggctcatc tggcgcccca 480
   gcctcccctg cctgcagctc cccacccagc tctgagttca tggat 525
<210> 233
   <211> 501
   <212> DNA
   <213> Homo sapiens
   <400> 233
   gaagggggcc ttttgagcta gaagctttct attctgatcc ccaaggagtt ccatatccag 60
   aagcaaaaat aggccgcttt gtagttcaga atgtttctgc acagaaagat ggagaaaaat 120
   ctagagtaaa agtcaaagtg cgagtcaaca cccatggcat tttcaccatc tctacggcat 180
   ctatggtgga gaaagtccca actgaggaga atgaaatgtc ttctgaagct gacatggagt 240
   gtctgaatca gagaccacca gaaaacccag acactgataa aaatgtccag caagacaaca 300
   gtgaagctgg aacacagccc caggtacaaa ctgatgctca acaaacctca cagtctcccc 360
   cttcacctga acttacctca gaagaaaaca aaatcccaga tgctgacaaa gcaaatgaaa 420
   aaaaagttga ccagcctcca gaagctaaaa agcccaaaat aaaggtggtg aatgttgagc 480
   tgcctattga agccaacttg g 501
<210> 234
   <211> 432
   <212> DNA
   <213> Homo sapiens
   <400> 234
   tgctgggctg ggtcgcgtag cccagggtgg aggcagaacg atgctgctgt ggtagccctt 60
   tgcctttcat gcccatgctt gattcttgca cctcagcagc tgaaggtctc agagaccagt 120
   aatcagaagg catccgactg cattaagtgt gcagcgctga aaagacattt acaactaggc 180
   cagggattag ccactgtggg agggtggaca ggcaatggtt cagtggcctg gctgttggca 240
   ggaactccaa gtgcccaggc ctcttgggca gcttagggcc ctgcctctgt ttcatgatgc 300
   atgggtcatt tgtcttgggt gtcctatccc atatggagaa gaaaggggct ctaagttctg 360
   gctcttcttt ctttggggtt ctctgtacct gaggaaacca ggccctgggt gactttgcag 420
   atctgctcac cc 432
<210> 235
   <211> 454
   <212> DNA
   <213> Homo sapiens
   <400> 235
   tgtagaaggt gacgctctgg gggcaggact cctccaaaat tatgtggacc gtacggagtc 60
   gagaagcaca gagcctgagt tgatacaagt gaagagtgag ctgcccctgg atccgctgcc 120
   agtccccact gaggaaggaa cccccctcct caaacactat cgggggcccg caggggatgc 180
   cacggtcgcc tctgagaagg aatcagtcat gtaaaccccg ggagggacct tccctgccct 240
   gctgggggtg ctctttggac actggattat gaggaatgga taaatggatg agctagggct 300
   ctgggggtct gcctgcacac tctggggagc caggggcccc agcaccctcc aggacaggag 360
   atctgggatg cctggctgct ggagtacatg tgttcacaag ggttactcct caaaaccccc 420
   agttctcact catgtcccca actcaaggct agaa 454
<210> 236
   <211> 475
   <212> DNA
   <213> Homo sapiens
   <400> 236
   gcaagaccga gagcacctgt ggaagttgat cgaaggcggt gcccacatct acgtctgtgg 60
   ggatgcacgg aacatggcca gggatgtgca gaacaccttc tacgacatcg tggctgagct 120
   cggggccatg gagcacgcgc aggcggtgga ctacatcaag aaactgatga ccaagggccg 180
   ctactccctg gacgtgtgga gctaggggcc tgcctgcccc acccacccca cagactccgg 240
   cctgtaatca gctctcctgg ctccctcccg tagtctcctg ggtgtgtttg gcttggcctt 300
   ggcatgggcg caggcccagt gacaaagact cctctgggcc tggggtgcat cctcctcagc 360
   ccccaggcca ggtgaggtcc accggcccct ggcagcacag cccagggcct gcatgggggc 420
   accgggctcc atgcctctgg agcctctggc cctcggtggc tgcacagaag ggctc 475
<210> 237
   <211> 531
   <212> DNA
   <213> Homo sapiens
   <400> 237
   ggtcaggctt ggctgcaaaa cactttattg atgtaggagc tggtgtcata gatgaagatt 60
   atagaggaaa tgttggtgtt gtactgttta attttggcaa agaaaagttt gaagtcaaaa 120
   aaggtgatcg aattgcacag ctcatttgcg aacggatttt ttatccagaa atagaagaag 180
   ttcaagcctt ggatgacacc gaaaggggtt caggaggttt tggttccact ggaaagaatt 240
   aaaatttatg ccaagaacag aaaacaagaa gtcatacctt tttcttaaaa aaaaaaaaaa 300
   aagtttttgc ttcaagtgtt ttggtgtttt gcacttctgt aaacttacta gctttacctt 360
   ctaaaagtac tgcatttttt actttttttt atgatcaagg aaaagatcat taaaaaaaaa 420
   cacaaagaag tttttctttg tgtttggatc aaaaagaaac tttgtttttc cgcaattgaa 480
   ggttgtatgt aaatctgctt tgtggtgacc tgatgtaaac agtgtcttct t 531
<210> 238
   <211> 543
   <212> DNA
   <213> Homo sapiens
   <400> 238
   ggatcaggag aacgtacacc cggatgtgat gctggtacaa cccagagtag aatttattct 60
   gtctttcatt gaccacattg ctggagatga ggatcacaca gatggagtag tagcttgtgc 120
   tgctggacta ataggggact tatgtacagc atttgggaag gatgtactga aattagtaga 180
   agctaggcca atgatccatg aattgttaac tgaagggcgg agatcgaaga ctaacaaagc 240
   aaaaaccctt gctacatggg caacaaaaga actgaggaaa ctgaagaacc aagcttgatc 300
   tgttaccatt gggatgataa cctgaggacc cccactggaa atctcccatc ttttgaaaaa 360
   cctggaagtg aggagtgtgc acggatgctg aatgtttggg aatgagagga tgagtgagtg 420
   aggcttgaaa acacaccaca ttgaaaatcc tgccacagca gcagccgcag ccgccaacag 480
   cagcgctgtt agtgagctaa gtaagcactg acttcgtaga aaaccataac atcggccatc 540
   ttg 543
<210> 239
   <211> 460
   <212> DNA
   <213> Homo sapiens
<220>
   <221> misc_feature
   <222> (173)..(174)
   <223> n is a, c, g, or t
   <400> 239
   gaggaaagac gctctttagg ttttgttttg tttttttttt tttggttttg ttttttgttt 60
   ttttttttac tctagggaaa acactgacga atggtcagag ctcctatcct gatcttttca 120
   tcaaggcgcc tttcctaata atatggttca actgtgaatg tagaagtggg ggnnaggggg 180
   gagaaaaaga aaactctggc gttagaggat ataaaaaaat ataagtacaa ttgttacaaa 240
   taacgcagac ttcaaaaaca aaaaaatcac aacccaaaca aaccaaaatt taaatgatca 300
   gaattggcag cacaaagaaa acgccctctc ctgacttgta ttgtggcagt ctgaacgccc 360
   ccagaaaatt gtgccaaaga gtttagaaaa ataaatatac aataaaagta aacacataca 420
   cacaaaacag caaacttcag gtaactattt tggattgcaa 460
<210> 240
   <211> 498
   <212> DNA
   <213> Homo sapiens
   <400> 240
   gttgaactca tgtttcagtt cgcgaacatt gactccttac gaaagtcact tcattctaac 60
   tagatgcgcc cacttccggt cattatttcg tttgcatgat gtattgcttc ttcacgtttt 120
   gtttttattg agcacggagt agaattccag ggctgccttg acttcttccc tgcatgctcc 180
   ctcccagtga ctttccttcc ctttcacatg aggatctgcc gttcatgttg ctttctcctt 240
   tgtcctcttg gacttgaggg cattgtgaaa agctttgctg tgatttaaaa atgccagcaa 300
   ttttaatcta gcagtgttga agctgggaat tttttggcgc aatccatgta gcagtgaccc 360
   aggcttggga gccagaaaca agtgtgacct gggattttat ttaacacaac tgttgccaaa 420
   gagttggctt tgtttatttg gttttggcgg ggagaggagt ggtatttgat gctttctgtg 480
   gacaatgtaa ccctaaac 498
<210> 241
   <211> 378
   <212> DNA
   <213> Homo sapiens
   <400> 241
   ggtcaaggct aaagccggag caggctctgc caccctctcc atggcgtatg ccggcgcccg 60
   ctttgtcttc tcccttgtgg atgcaatgaa tggaaaggaa ggtgttgtgg aatgttcctt 120
   cgttaagtca caggaaacgg aatgtaccta cttctccaca ccgctgctgc ttgggaaaaa 180
   gggcatcgag aagaacctgg gcatcggcaa agtctcctct tttgaggaga agatgatctc 240
   ggatgccatc cccgagctga aggcctccat caagaagggg gaagatttcg tgaagaccct 300
   gaagtgagcc gctgtgacgg gtggccagtt tccttaattt atgaaggcat catgtcactg 360
   caaagccgtt gcagataa 378
<210> 242
   <211> 428
   <212> DNA
   <213> Homo sapiens
   <400> 242
   tgtgtagcgt aggcttttcc caagggtcgc tagaaactcg tcttcgcgtt gccccctttc 60
   tggctctcag cgccgtcgcc actcgggaga ggctgggtga ggcccgtgtg aggactgacc 120
   ctggattcct cgaaactgcc attgtgatca ttactctgct ctttggaaat ggctgtatca 180
   tttttttgta ctaatgtgaa ttgttcctca gaaacgcttc ttttccatcc tagtgagaag 240
   ctggccctgc aggtggtggc agcaatggtg ttgtaagatt tcctcccgta gttttttctc 300
   ctcatggatt tgaatgaaat gccaataaca cgtccacttt caacgtgtag tttacgcgga 360
   gcactttcga ggcctggccg ggttgggcct acttctcacc tgggcctatc ttctgaactc 420
   gctaggtt 428
<210> 243
   <211> 534
   <212> DNA
   <213> Homo sapiens
   <400> 243
   gaagataacc ggctcattca cttcctccca gaagacgcgt ggtagcgagt aggcacaggc 60
   gtgcacctgc tcccgaatta ctcaccgaga cacacgggct gagcagacgg cccctgtgat 120
   ggagacaaag agctcttctg accatatcct tcttaacacc cgctggcatc tcctttcgcg 180
   cctccctccc taacctactg acccaccttt tgattttagc gcacctgtga ttgataggcc 240
   ttccaaagag tcccacgctg gcatcaccct ccccgaggac ggagatgagg agtagtcagc 300
   gtgatgccaa aacgcgtctt cttaatccaa ttctaattct gaatgtttcg tgtgggctta 360
   ataccatgtc tattaatata tagcctcgat gatgagagag ttacaaagaa caaaactcca 420
   gacacaaacc tccaaatttt tcagcagaag cactctgcgt cgctgagctg aggtcggctc 480
   tgcgatccat acgtggccgc acccacacag cacgtgctgt gacgatggct gaac 534
<210> 244
   <211> 532
   <212> DNA
   <213> Homo sapiens
   <400> 244
   cagaaagtct cagcccagga tggggcttct tcaacagggc ccctgccctc ctgaagcctc 60
   agtccttcac cttgccaggt gccgtttctc ttccgtgaag gccactgccc aggtccccag 120
   tgcgccccct agtggccata gcctggttaa agttccccag tgcctccttg tgcatagacc 180
   ttcttctccc acccccttct gcccctgggt ccccggccat ccagcggggc tgccagagaa 240
   ccccagacct gcccttacag tagtgtagcg ccccctccct ctttcggctg gtgtagaata 300
   gccagtagtg tagtgcggtg tgcttttacg tgatggcggg tgggcagcgg gcggcgggct 360
   ccgcgcagcc gtctgtcctt gatctgcccg cggcggcccg tgttgtgttt tgtgctgtgt 420
   ccacgcgcta aggcgacccc ctcccccgta ctgacttctc ctataagcgc ttctcttcgc 480
   atagtcacgt agctcccacc ccaccctctt cctgtgtctc acgcaagttt ta 532
<210> 245
   <211> 477
   <212> DNA
   <213> Homo sapiens
<220>
   <221> misc_feature
   <222> (363)..(363)
   <223> n is a, c, g, or t
   <220>
   <221> misc_feature
   <222> (418)..(418)
   <223> n is a, c, g, or t
   <400> 245
   tgcccatcgt caacctcaag gacgagctgc tgtttcccag ctgggaggct ctgttctcag 60
   gctctgaggg tccgctgaag cccggggcac gcatcttctc ctttgacggc aaggacgtcc 120
   tgaggcaccc cacctggccc cagaagagcg tgtggcatgg ctcggacccc aacgggcgca 180
   ggctgaccga gagctactgt gagacgtggc ggacggaggc tccctcggcc acgggccagg 240
   cctcctcgct gctggggggc aggctcctgg ggcagagtgc cgcgagctgc catcacgcct 300
   acatcgtgct ctgcattgag aacagcttca tgactgcctc caagtagcca ccgcctggat 360
   gcngatggcc ggagaggacc ggcggctcgg aggaagcccc caccgtgggc agggagcngc 420
   cggccagccc ctggccccag gacctggctg ccatactttc ctgtatagtt cacgttt 477
<210> 246
   <211> 445
   <212> DNA
   <213> Homo sapiens
   <400> 246
   gtcactaacc tgtctcagtg tggccttgtc cagccttgtg ttttctgtaa cccctgtttg 60
   tggtacgaga taatgagtcc tatttttctc tcacataata tgcatttgct ctcctaggac 120
   agtgtaatac atttatgtga agtaaagaca tgcgagactg gtggcctgca aatagcatcc 180
   gtcaatctgt gttaactgca tagggagggc tctgcatagc acctgctata gcggtgtcat 240
   gttggatcgc ttttgtgact gttcatctgt ccttgacagt ggctgtcatc ttgactactt 300
   tgttgatttg ttggtattgg ggacatttta aaggctgagt tatttttgaa tgtcatgttt 360
   atgtcataga cgtagttttc gcatccttga attaaactgc cttaactcct tttgtggtat 420
   aagcaaaact ccatggactc tgtcc 445
<210> 247
   <211> 182
   <212> DNA
   <213> Homo sapiens
   <400> 247
   tctgcagcct acgcatgaat aggttggcag gtgtgggctg gcgggtggac tacaccctga 60
   gctccagcct gctgcaatcc gtggaagagc ccatggtgca cctgcggctg gaggtggcag 120
   ctgccccagg gaccccagcc cagcctgttg ccatgtccct ctcagcagac aagttccagg 180
   tc 182
<210> 248
   <211> 403
   <212> DNA
   <213> Homo sapiens
   <400> 248
   ttattcttct aattaacagc tcctaggaaa atgtagactt ttgctttatg atattctatc 60
   tgtagtatga ggcatggaat agttttgtat cgggaatttc tcagagctga gtaaaatgaa 120
   ggaaaagcat gttatgtgtt tttaaggaaa atgtgcacac atatacatgt aggagtgttt 180
   atctttctct tacaatctgt tttagacatc tttgcttatg aaacctgtac atatgtgtgt 240
   gtgggtatgt gtttatttcc agtgagggct gcaggcttcc tagaggtgtg ctataccatg 300
   cgtctgtcgt tgtgcttttt tctgttttta gaccaatttt ttacagttct ttggtaagca 360
   ttgtcgtatc tggtgatgga ttaacatata gcctttgttt tct 403
<210> 249
   <211> 487
   <212> DNA
   <213> Homo sapiens
   <400> 249
   gccgtctcaa agtttcttag ctgactttgg ctttcacatt tgttctttcc agagctaact 60
   gataagagtg gaggaggaat gccttctcct aagagtcagt tgaaagaaag acaagagagt 120
   cacatcttag cttttgcaca aggcattcgt ggtcaggaat aggttaggga atggtcactt 180
   ctgattttcc aacagttgct ccttctctga agagatcttg attcctttgg gaagacaaga 240
   atttttctta ataacaaagg tccctttatg agttattcct tctttcagtt catctcactg 300
   gagcacagcc aagatggaca tgtttatgga cagtgctcta gatgtgaaaa cagatagaac 360
   tggtttgtgg gacaggggca gcttgctcag gagagggaat aacgcaggtc ccttttcttg 420
   gaaggcttgt actatggcca tgacagtgac attgccctca ccatgatccc tctccaaagt 480
   ggttgtc 487
<210> 250
   <211> 471
   <212> DNA
   <213> Homo sapiens
   <400> 250
   tttgctatca gctcttctgc tatgaagtag taaaaggcag tctataatta actgacagac 60
   ctaactgaag cacagagaat acatcagact tatgcatcca agacatcaga acttggattt 120
   tatcaaactt gatgacttct ctaaaaggag ctttggaaac ttcaaattca gctataggat 180
   agtaccaatg aacacatcca gctgatccca aaagctgttt tcaggtataa ggacaaggag 240
   aggagacaag tgacgacagc cattcccctt tgcagctatc tactgtagtg acagccattt 300
   cttggttgat gggttggaag tcatcagagg tttgaagaat tacactggcc tttgtttttc 360
   tggaaatgcc gaccatggag atgctttaga gtcttctcaa atagcttaga tgttgtaatg 420
   aggttagctt tgcttcataa aacaggggcc ctcagaagtt ctccttaaat t 471
<210> 251
   <211> 529
   <212> DNA
   <213> Homo sapiens
   <400> 251
   cctctacctg ggttcgggtc aggagctcca tctgggaact aacagctgct aacctgacca 60
   gccgctcagg acaggaccct ggggctacac tcctgcattg ctgcaatact gctcccccag 120
   cctctcccct gcccctcaac ctgccttagc tgcactctct tacctacagc tggacagtac 180
   ctgtctgttt cctgtcctcc ttccagttac atctgtccat gtctggactc ggctggccgt 240
   tccctccagc cccttgctgg ttatcttact ctgagtgtga tgcagtcaga ggcacctgcg 300
   ggttagccca ggggcccaag ccctggattt ggcctgcgga ggagcttagg atcctcgttt 360
   tctgggtttt ggtgatgttg gaggagtacc ccccagccca ccgccccgat tcctttttgc 420
   ttctggtttg gagctccgga ccaggacctt cgtcctggtc agtttttaaa taattattta 480
   gcagtgtaac ttttaaacct gcgtgacatc tacaaagcgc ccaataaag 529
<210> 252
   <211> 419
   <212> DNA
   <213> Homo sapiens
<220>
   <221> misc_feature
   <222> (160)..(160)
   <223> n is a, c, g, or t
   <400> 252
   gggtcattgt ttaagatctg gctggtggta cctagcctgc tggaactggc atgggagaag 60
   ctgcttgcgg ccttccctaa ccttgcaaac ctctcccgaa cacaacttct gcaccttgga 120
   ctcacacagg gactcatcga acgcttgaaa tgaggatttn tggactgttc attgatactg 180
   gaaatgttaa tttaaagaga ctcctttatt tatgggcagt gtagaatgtg ctacaaagag 240
   gattggttac cctgatcaag gccttattta gaaaatacat cagatgcctt tctgtaaatt 300
   ggtttttcag tttatggaca tctcactttc ccacgtgctt ccttctttgc ttctgttcct 360
   cctgacccat tacatgcaca tgtactcaca tactccctct tccttctcga tggagttaa 419
<210> 253
   <211> 358
   <212> DNA
   <213> Homo sapiens
<220>
   <221> misc_feature
   <222> (134)..(135)
   <223> n is a, c, g, or t
   <400> 253
   ttgcttttcc tctaatcttg caagagctat ggctcttcta ttttccaatc acacagcttg 60
   gcatgtagga aaggttgaat gatcctctaa gactgtgttg gtcttcgtat tctgtaaaac 120
   ccattttttt tttnngtggt cttacagatg tttagaaagt ggcacaggtt actgaattgt 180
   ctacctgcca gcattctgat atagcacaaa aagctatttt cctttatttt ttgtattatt 240
   ttttattttt ctggcattga gctctagggt ggatgagggt ttatggtcct ctgatcataa 300
   gctccattct aaaaactggt cactgttagc tgaaattgct ttggttcccc aaatgcct 358
<210> 254
   <211> 516
   <212> DNA
   <213> Homo sapiens
   <400> 254
   ggcctttccc ttctaaggtc attagattca gccaaaagcg acctcttctc tagtccggtg 60
   ttacgaacag aagttctgag ttgtgctaca aaagtagttc catctttttg gtgtaatttt 120
   cacgttttta atttgaaaaa aaaaaaaaaa acaacttttt ataagttttt taagggccct 180
   gcttagtcag tgtacagggt ggagtcagag gcagttttca gaaaaaaaac aaaaaacaaa 240
   aaacaatttc accaagcggt agtaattgtt gttttactag ttatacattt agaatataaa 300
   ggaggcatca gaaaacacac tctctaaagc cacttccttg tgcacagagt ctgcacaggg 360
   agagcacagg catctccctg gaaaagcacc tgccaatgac gaatttcatg gaagaaccta 420
   ggcaagaaag gaagcctctt tctgagacac agtctctgag aggtgagcct agctttgctc 480
   ttcctacagg gtatgcttgg gccatacaca atgctc 516

<210> 255
   <211> 514
   <212> DNA
   <213> Homo sapiens
   <400> 255
   gaccagttct tcggagagca cctgttggag tctgatcttt tcccgacgtc tacttccctg 60
   agtcccttct accttcggcc accctccttc ctgcgggcac ccagctggtt tgacactgga 120
   ctctcagaga tgcgcctgga gaaggacagg ttctctgtca acctggatgt gaagcacttc 180
   tccccagagg aactcaaagt taaggtgttg ggagatgtga ttgaggtgca tggaaaacat 240
   gaagagcgcc aggatgaaca tggtttcatc tccagggagt tccacaggaa ataccggatc 300
   ccagctgatg tagaccctct caccattact tcatccctgt catctgatgg ggtcctcact 360
   gtgaatggac caaggaaaca ggtctctggc cctgagcgca ccattcccat cacccgtgaa 420
   aagaagcctg ctgtcaccgc agcccccaag aaaaagatgc cctttcttga attgcatttt 480
   ttaaaacaag aaagtttccc caccagtgaa tgaa 514
<210> 256
   <211> 500
   <212> DNA
   <213> Homo sapiens
<220>
   <221> misc_feature
   <222> (267)..(267)
   <223> n is a, c, g, or t
   <220>
   <221> misc_feature
   <222> (409)..(409)
   <223> n is a, c, g, or t
   <400> 256
   tcggacactg gccttgggaa caatgttcga gagaacactt gccccttgac tgtaggagcc 60
   agaaggggac ccaggtgtgc atagctctct gtagacattt ttacccaaac ctgttggtaa 120
   agtgcccatc tggtgctcaa gagagcctgg gggtctaaca gggagcccgg ctgcctcacc 180
   tggccacagc ctccacacca gatctccaca ttgtcttgat ccagaccagc tctgtgatca 240
   gaaggaaatt gggtccagtg taggagngag ctggtcctgg gcctggcagg caagagtgtg 300
   ggcatccttt cctggccttt ctccactctc cctcaagcct gtgctcaggt tgccttgaat 360
   gtggactctg gaagagccag gggcccagaa tgccggggga ggcttctgng tggcactcat 420
   ggaacaccgt ccctctgcca gccataggcc ctgcctccag tgtcagggaa tggaggctgg 480
   gctgcgagag tgttgctgcc 500
<210> 257
   <211> 500
   <212> DNA
   <213> Homo sapiens
   <400> 257
   atcgcaccgt ttccagaagc tgcgttgcca acgtcacatc ccaaaatagt gttgacatcc 60
   ctgcctgcgc tggcggtccc acccccgact cccaccaaag cggcacctcc cgcgtaggtc 120
   aatgggctgg agctgtcaga gccgcggagc tggctgtacc tagaagagat ggtcaactcc 180
   ttgctcaaca cagcgcagca gctgaagacg ctgtttgagc aagccaagca tgccagcacc 240
   taccgagaag ctgccacaaa ccaggccaag atccacgctg acgcagagcg gaaggagcag 300
   tcctgcgtta actgcggccg ggaggctatg agcgagtgca ccggttgcca caaggtcaac 360
   tactgctcca acttctgcca acgcaaggac tggaaggatc accagcacat atgcggccag 420
   tcagcagctg tcaccgtcca ggcagacgaa gtccacgtgg ctgaaagcgt gatggagaag 480
   gtgaccgtgt gaggctccat 500
<210> 258
   <211> 516
   <212> DNA
   <213> Homo sapiens
   <400> 258
   agatgcctgt ttgctatttg gtggaagata gatgttcata ttgaagcagt cacatttgta 60
   ctgtagttca ataaaagaaa aatgaagtat tctgtagcct atatttttca tagagctcat 120
   gagcatttac tgtacttgct gggtcttgcc aagatcattt attccgctgc attgccaaag 180
   tgtcttcata ccaaattaaa ggtggtttta atatatgttt catggaagtt gtttataaaa 240
   ttcaaaggta tttcatttag gtgaaaagtc ttatttatta aagtggtttg aataaagtag 300
   atcaaaactt ccagagatct taatggctat ataggaagaa atatcactca ccataattta 360
   aataaagaat aaaaatacat gtattttatg gtggcaaatg tttggtagaa ctgtaattag 420
   aaaaatacaa gtatatttgc gtgatggtta cactagaagc ccagacttta cgactacaca 480
   atatattcat gtatctaaac tgtacttgta ccccct 516
<210> 259
   <211> 375
   <212> DNA
   <213> Homo sapiens
   <400> 259
   ttttaccttg gatgctgact tctaaatgaa ctgaagatgt gcccttactt ggctgatttt 60
   ttttttccat ctcataagaa aaatcagctg aagtgttacc aactagccac accatgaatt 120
   gtccgtaatg ttcattaaca gcatctttaa aactgtgtag ctacctcaca accagtcctg 180
   tctgtttata gtgctggtag tatcaccttt tgccagaagg cctggctggc tgtgacttac 240
   catagcagtg acaatggcag tcttggcttt aaagtgaggg gtgacccttt agtgagctta 300
   gcacagcggg attaaacagt cctttaacca gcacagccag ttaaaagatg cagcctcact 360
   gcttcaacgc agatt 375
<210> 260
   <211> 427
   <212> DNA
   <213> Homo sapiens
   <400> 260
   gtacgagacc tgttccagat gaagcttttt gtggatacag atgcggacac ccggctctca 60
   cgcagagtat taagggacat cagcgagaga ggcagggatc ttgagcagat tttatctcag 120
   tacattacgt tcgtcaagcc tgcctttgag gaattctgct tgccaacaaa gaagtatgct 180
   gatgtgatca tccctagagg tgcagataat ctggtggcca tcaacctcat cgtgcagcac 240
   atccaggaca tcctgaatgg agggccctcc aaacggcaga ccaatggctg tctcaacggc 300
   tacacccctt cacgcaagag gcaggcatcg gagtccagca gcaggccgca ttgacccgtc 360
   tccatcggac cccagcccct atctccaaga gacagaggag gggtcaggag gcactgctca 420
   tctgtac 427
<210> 261
   <211> 463
   <212> DNA
   <213> Homo sapiens
<220>
   <221> misc_feature
   <222> (435)..(435)
   <223> n is a, c, g, or t
   <400> 261
   gaagatgtcg gcagggctgg gcttcagcct ggaaggaggg aagggctccc tacacggaga 60
   caagcctctc accattaaca ggattttcaa aggagcagcc tcagaacaaa gtgagacagt 120
   ccagcctgga gatgaaatct tgcagctggg tggcactgcc atgcagggcc tcacacggtt 180
   tgaagcctgg aacatcatca aggcactgcc tgatggacct gtcacgattg tcatcaggag 240
   aaaaagcctc cagtccaagg aaaccacagc tgctggagac tcctaggcag gacatgctga 300
   agccaaagcc aataacacac agctaacaca cagctcccat aaccgctgat tctcagggtc 360
   tctgctgccg ccccacccag atgggggaaa gcacaggtgg gcttcccagt ggctgctgcc 420
   caggcccaga ccttntagga cgccacccag caaaaggttg ttc 463
<210> 262
   <211> 531
   <212> DNA
   <213> Homo sapiens
   <400> 262
   ttggaatggg cagctcatct ctgtcccact tggcatcagc tggcgtcatg caaagtcatg 60
   caaaggctgg gaccacctga gatcattcac tcatacatct ggccgttgat gttggctggg 120
   aactcacctg gggctgctgg cctgaatgct tataggtggc ctctccttgt ggcctgggct 180
   cctcacaaca tggtgtctgg attcccagga tgagcatccc aggatcgcaa gagccacgta 240
   gaagctgcat cttgtttata cctttgcctt ggaagttgca tggcatcacc tccaccatac 300
   tccatcagtt agagctgaca caaacctgcc tgggtttaag gggagaggaa atattgctgg 360
   ggtcatttat gaaaaataca gtttgtcaca tgaaacattt gcaaaattgt ttttggttgg 420
   attggagaag taatcctagg gaagggtggt ggagccagta aatagaggag tacaggtgaa 480
   gcaccaagct caaagcgtgg acaggtgtgc cgacagaagg aaccagcgtg t 531
<210> 263
   <211> 528
   <212> DNA
   <213> Homo sapiens
   <400> 263
   gtatcgatat ggttcctttt ccgtcaccct ggacattgtc cagggtattg aaagtgccga 60
   gatcctgcag gctgtgccgt ccggtgaggg ggatgcattt gagctgactg tgtcctgcca 120
   aggcgggctg cccaaggaag cctgcatgga gatctcatcg ccagggtgcc agccccctgc 180
   ccagcggctg tgccagcctg tgctacccag cccagcctgc cagctggttc tgcaccagat 240
   actgaagggt ggctcgggga catactgcct caatgtgtct ctggctgata ccaacagcct 300
   ggcagtggtc agcacccagc ttatcatgcc tggtcaagaa gcagggggcc ttgggcaggt 360
   tccgctgatc gtgggcatct tgctggtgtt gatggctgtg gtccttgcat ctctgatata 420
   taggcgcaga cttatgaagc aagacttctc cgtaccccag ttgccacata gcagcagtca 480
   ctggctgcgt ctaccccgca tcttctgctc ttgtcccatt ggtgagaa 528
<210> 264
   <211> 529
   <212> DNA
   <213> Homo sapiens
   <400> 264
   gaatggtgca tacaaggcca tccccgttgc ccaggacctg aacgcgcctt ctgattggga 60
   cagccgtggg aaggacagtt atgaaacgag tcagctggat gaccagagtg ctgaaaccca 120
   cagccacaag cagtccagat tatataagcg gaaagctaat gatgagagca atgagcattc 180
   cgatgtgatt gatagtcagg aactttccaa agtcagccgt gaattccaca gccatgaatt 240
   tcacagccat gaagatatgc tggttgtaga ccccaaaagt aaggaagaag ataaacacct 300
   gaaatttcgt atttctcatg aattagatag tgcatcttct gaggtcaatt aaaaggagaa 360
   aaaatacaat ttctcacttt gcatttagtc aaaagaaaaa atgctttata gcaaaatgaa 420
   agagaacatg aaatgcttct ttctcagttt attggttgaa tgtgtatcta tttgagtctg 480
   gaaataactg atgtgtttga taattagttt agtttgtggc ttcatggaa 529
<210> 265
   <211> 372
   <212> DNA
   <213> Homo sapiens
   <400> 265
   cctgcggagg tgggcggcat gcagctccgc tttgcccggc tctccgagca cgccacggcc 60
   cccacccggg gctccgcgcg cgccgcgggc tacgacctgt acagtgccta tgattacaca 120
   ataccaccta tggagaaagc tgttgtgaaa acggacattc agatagcgct cccttctggg 180
   tgttatggaa gagtggctcc acggtcaggc ttggctgcaa aacactttat tgatgtagga 240
   gctggtgtca tagatgaaga ttatagagga aatgttggtg ttgtactgtt taattttggc 300
   aaagaaaagt ttgaagtcaa aaaaggtgat cgaattgcac agctcatttg cgaacggatt 360
   ttttatccag aa 372
<210> 266
   <211> 409
   <212> DNA
   <213> Homo sapiens
   <400> 266
   agtcaagtga ccagcctctg actgtgcctg tatctcccaa attctccact cgattccact 60
   gctaaactca gctgtgagct gcggataccg cccggcaatg ggacctgctc ttaacctcaa 120
   acctaggacc gtcttgcttt gtcattgggc atggagagaa cccatttctc cagactttta 180
   cctacccgtg cctgagaaag catacttgac aactgtggac tccagttttg ttgagaattg 240
   ttttcttaca ttactaaggc taataatgag atgtaactca tgaatgtctc gattagactc 300
   catgtagtta cttcctttaa accatcagcc ggccttttat atgggtcttc actctgacta 360
   gaatttagtc tctgtgtcag cacagtgtaa tctctattgc tattgcccc 409
<210> 267
   <211> 523
   <212> DNA
   <213> Homo sapiens
   <400> 267
   ggtatcttca taaaatcggt gcactgagaa tgcagctgga cccatgtgaa gatacctcac 60
   tccagcccac ttcctaggaa caatggaaga agaaaggact gaaccagggt atttttgtta 120
   ggttttctat gtgactccaa gagggaatgg tcaagttgtt tcatgagttt gcatgggccc 180
   ttggaaaaac aggaaaggag caatgaagat ccaagcaaaa ctttactttc agcgttggct 240
   tggaggacaa ataagaaatg aaacatccta tgaaatactt tatagcacat ggcagatttg 300
   caactagtaa aatgctggtg aaatgctgtt ggtaaagcac atggtccaaa tctagaagat 360
   gcagttcaaa aacaagacag actcgagttg ttagggctga ggaaccaatc aaggtagaac 420
   aaagaaaatg ttggggtaaa agtgttgctg attgtcaaca caaactggct taataatatt 480
   aataagaacc tgtcttatta agactggctt tagaaccgta ggt 523
<210> 268
   <211> 161
   <212> DNA
   <213> Homo sapiens
   <400> 268
   gtgcatgcca tatgatcagg acagcttttc cactttactc ggtttcctac aagcaagtag 60
   gaaatacagt gaatttaccc taaaatgtcc aatctgtatt tatgtacctt gtcagtgttt 120
   tgctgttggt tttctaaaac aatctgatca ataaatctta t 161
<210> 269
   <211> 445
   <212> DNA
   <213> Homo sapiens
   <400> 269
   caacaagacg gacctggctg ataagaggca gataaccatc gaggaggggg agcagcgcgc 60
   caaagaactg agcgtcatgt tcattgagac cagtgcgaag actggctaca acgtgaagca 120
   gctttttcga cgtgtggcgt cggctctacc cggaatggag aatgtccagg agaaaagcaa 180
   agaagggatg attgacatca agctggacaa accccaggag cccccggcca gcgagggcgg 240
   ctgctcctgc taatgcagag ccgacctgtg gcttcccatg acactccttg cttgttgtgt 300
   tgcttcctat tggctagctt cctaaggggg gagggaaccg agttatcaag atgggaggat 360
   ttttcttttc tctctgtctt taggagtagg gtgggatggg gagggaggct gggcatcagg 420
   gatcacatca ctcttaacgg ctgtt 445
<210> 270
   <211> 503
   <212> DNA
   <213> Homo sapiens
   <400> 270
   gacattgcct gtatgatcgg gtaccgacct tgcccctgga tgaaatggtg ctggtccttc 60
   ttcaccccgc tggtctgcat gggcatcttc atcttcaacg ttgtgtacta cgagccgctg 120
   gtctacaaca acacctacgt gtacccgtgg tggggtgagg ccatgggctg ggccttcgcc 180
   ctgtcctcca tgctgtgcgt gccgctgcac ctcctgggct gcctcctcag ggcaaagggc 240
   accatggctg agcgctggca gcacctgacc cagcccatct ggggcctcca ccacttggag 300
   taccgagctc aggacgcaga tgtcaggggc ctgaccaccc tgaccccagt gtccgagagc 360
   agcaaggtcg tcgtggtgga gagtgtcatg tgacaactca gctcacatca ccagctcacc 420
   tctggtagcc atagcagccc ctgcttcagc cccaccgcac ccctccaggg ggcctgcctt 480
   tccctgacac ttttggggtc tgc 503
<210> 271
   <211> 508
   <212> DNA
   <213> Homo sapiens
   <400> 271
   tcaactccat agtgaagtct gatgtggaca tccgcaaaga cctgtacacc aacacagtgc 60
   tgtctggcgg caccaccatg taccctggca tcgcccacag gatgcagaag gagatcactg 120
   ccctggcgcc tagcattatg aagatcaaga tcattgctcc tcccaagcgc aagtactccg 180
   tgtgggtcgg tggctccatc ctggcctcgc tgtccacctt ccagcagatg tggatcagca 240
   agcaggagta tgatgagtca ggcccctcca ttgtccaccg caaatgcttc taggtggact 300
   ctgacttagt tgcgttacac cctttcttga caaaaccaaa cttctcagaa aacaacatga 360
   gattggcgtg gctttatttg ttttcttgtt tcattttttg ttttgttttt tattggcttg 420
   actcaggatt tgaaaaccgg aacggcgaag gtgatagtag tcggttggag cgagcttccc 480
   ccaaagttct acaatgtggc caaggact 508
<210> 272
   <211> 502
   <212> DNA
   <213> Homo sapiens
   <400> 272
   tcactgtcag tcgacacttc catgtccagg ttttcccatc atatgattcc cggtcctcct 60
   ggtccccaca caactggcat ccctcatcca gctattgtaa cacctcaggt caaacaggaa 120
   catccccaca ctgacagtga cctaatgcac gtgtgctctg cttttctcct cccccatccc 180
   ttcctcattc cttcaacccc ttcccctaac caccaccacc accacctttt aggaagcctc 240
   agcatgaaca gagaaaggag caggagccaa aaagacctca cattaagaag cctctgaatg 300
   cttttatgtt atacatgaaa gaaatgagag cgaatgtcgt tgctgagtgt actctaaaag 360
   aaagtgcagc tatcaaccag attcttggca gaaggtggca tgccctctcc cgtgaagagc 420
   aggctaaata ttatgaatta gcacggaaag aaagacagct acatatgcag ctttatccag 480
   gctggtctgc aagagacaat ta 502
<210> 273
   <211> 552
   <212> DNA
   <213> Homo sapiens
   <400> 273
   aagccagcta cagatgcatg catattgtga aaacccagat atagtgctgt gtggaaacaa 60
   gagtgatctg gaggaccaga gagtagtgaa agagaaatat ggaatcccct actttgaaac 120
   tagtgctgcc aatgggacaa acataagcca agcaattgag atgcttctgg acctgataat 180
   gaagcgaatg gaacggtgtg tggacaagtc ctggattcct gaaggagtgg tgcgatcaaa 240
   tggtcatgcc tctacggatc agttaagtga agaaaaggag aaaggggcat gtggctgttg 300
   agaagtcaag taagcgacat agtagttcag gtggcccatg cctgggatct tctctatgat 360
   tgatacatgg cacagtgaga gattaatggg cattgtgtac aaattgcttc tcaccatccc 420
   cattagacct acgaataaag catccggttc taaaattaat ttgttgcagc tttgtaaata 480
   tttctttaag attcagcctg agagttagga gaaatatttc agagccaaaa gtgccttata 540
   caaccttagc ct 552
<210> 274
   <211> 417
   <212> DNA
   <213> Homo sapiens
   <400> 274
   ggagccccgt cataggaagt gaggtcttcc tgcccaacag caaccacgtg gccagtggcg 60
   ccggggaggc agaggaacgc gttgtggtga tcagcagctc ggaagactca gatgccgaaa 120
   actcgtcctc ccgagagctg gatgacagca gcagtgagtc cagtgacctc cagctggaag 180
   gccccagcac cctcagggtc ctggacgaga accttgctga cccccaagca gaagacagac 240
   ctctggtttt ctttgacctc aagattgaca atgaaagtgg gttctcctgg ggctaccccc 300
   acccctttct aatttagtct ctgagtccca aaaagaagtg caggcagagc catctgccag 360
   gcccaggaga gctctgagct ctggccaaca actgcagcca ggctgggcag agcactc 417
<210> 275
   <211> 510
   <212> DNA
   <213> Homo sapiens
   <400> 275
   gttctgcggg atggtgcagt tccccggcga cgtgaggagg caggccctcc tgcagctgtg 60
   tctgctcctc tgccaccgtt tcccgctgat ccggaagacc acggccagcc aggtgtacga 120
   gacattgctc acctacagtg acgtcgtggg cgcggatgtg ctggacgagg tggtgactgt 180
   gctcagtgac actgcgtggg acgcggagct tgcagtggtg agagagcagc gcaaccgtct 240
   gtgtgacctt ctgggcgtac ccaggcccca gctggtgccc cagcctggtg cctgctgaag 300
   ccagtcctgg agcccatacc tcacccctgc ctggtgagga tgtcttgttc ctgagggagg 360
   ccggtgtgga aagcctcgca cagtggtgcc tccagctgtt gaagggtagc gctggccctt 420
   ggaggctggc actagctgac agcttttcct ctctgcacct gcgctctggt gacttggggt 480
   ggacgcctct gccttcactt gaacacaaat 510
<210> 276
   <211> 551
   <212> DNA
   <213> Homo sapiens
   <400> 276
   ggatggggct tcttcaacag ggcccctgcc ctcctgaagc ctcagtcctt caccttgcca 60
   ggtgccgttt ctcttccgtg aaggccactg cccaggtccc cagtgcgccc cctagtggcc 120
   atagcctggt taaagttccc cagtgcctcc ttgtgcatag accttcttct cccaccccct 180
   tctgcccctg ggtccccggc catccagcgg ggctgccaga gaaccccaga cctgccctta 240
   cagtagtgta gcgccccctc cctctttcgg ctggtgtaga atagccagta gtgtagtgcg 300
   gtgtgctttt acgtgatggc gggtgggcag cgggcggcgg gctccgcgca gccgtctgtc 360
   cttgatctgc ccgcggcggc ccgtgttgtg ttttgtgctg tgtccacgcg ctaaggcgac 420
   cccctccccc gtactgactt ctcctataag cgcttctctt cgcatagtca cgtagctccc 480
   accccaccct cttcctgtgt ctcacgcaag ttttatactc taatatttat atggcttttt 540
   ttcttcgaca a 551
<210> 277
   <211> 533
   <212> DNA
   <213> Homo sapiens
   <400> 277
   ccttgactgg ctacccaggg gaggagctgg aggaagagga ggaaagtcaa gggggcgtga 60
   agcttggcct cggggacttc atcttctaca gtgtgctggt gggcaaggcg gctgccacgg 120
   gcagcgggga ctggaatacc acgctggcct gcttcgtggc catcctcatt ggcttgtgtc 180
   tgaccctcct gctgcttgct gtgttcaaga aggcgctgcc cgccctcccc atctccatca 240
   cgttcgggct catcttttac ttctccacgg acaggaagca cagcaggttt atccagatga 300
   actgagaagg tcagattagg gcggggagaa gagcatccgg catgagggct gagatgcgca 360
   aagagtgtgc tcgggagtgg cccctggcac ctgggtgctc tggctggaga ggaaaaacca 420
   gttccctacg aggagtgttc ccaatgcttt gtccatgatg tccttgttat tttattgcct 480
   ttagaaactg agtcctgttc ttgttacggc agtcacactg ctgggaagtg gct 533

<210> 278
   <211> 238
   <212> DNA
   <213> Homo sapiens
   <400> 278
   ctgggctccg aggtgtacag gatgctgcgg gagccggccg agcccgtggc cgcggagccc 60
   aagcagtcag gctccttccg ctacttgcag ggcatgctag aggccggcga gggcggggca 120
   ccatcgtcaa ggcacgggac aagctctacc atcccgagtg cttcatgtgc agtgactgcg 180
   gcctgaacct caagcagcgt ggttacttct ttctggacga gcggctctac tgtgagag 238
<210> 279
   <211> 491
   <212> DNA
   <213> Homo sapiens
   <400> 279
   gctctttcct gaagcgcagc aagctcggcc ggtacaacga ggaggagcgg gctcagcagg 60
   aggccgaggc cgcccagcgc ctggccgagg agaaggccca ggccagctcc atccccgtgg 120
   gcagccgctg tgaggtgcgg gcggcgggac aatcccctcg ccggggcacc gtcatgtatg 180
   taggtctcac agatttcaag cctggctact ggattggtgt ccgctatgat gagccactgg 240
   ggaaaaatga tggcagtgtg aatgggaaac gctacttcga atgccaggcc aagtatggcg 300
   cctttgtcaa gccagcagtc gtgacggtgg gggacttccc ggaggaggac tacgggttgg 360
   acgagatatg acacctaagg aattcccctg cttcagctcc tagctcagcc actgactgcc 420
   cctcctgtgt gtgcccatgg cccttttctc ctgaccccat tttaatttta ttcatttttt 480
   cctttgccat t 491
<210> 280
   <211> 268
   <212> DNA
   <213> Homo sapiens
   <400> 280
   agcagatcat gaagacaggg gcccttttgc ttcaggggat gattgccgcc gtggacacag 60
   actccccccg agaggtcttt ttccgagtgg cagctgacat gttttctgac ggcaacttca 120
   actggggccg ggttgtcgcc cttttctact ttgccagcaa actggtgctc aaggccctgt 180
   gcaccaaggt gccggaactg atcagaacca tcatgggctg gacattggac ttcctccggg 240
   agcggctgtt gggctggatc caagacca 268
<210> 281
   <211> 261
   <212> DNA
   <213> Homo sapiens
   <400> 281
   gctctatttc caggcatgtg atcgcccccg ctctccagat tccccagcac tctgctgcgt 60
   gtaactccac tcaattctcc actcatcctt ccttgtgaag caggatcgtt gaagttttaa 120
   gtatgggcaa aaatctggaa aacttaggat ccctctgaca ccccaggatt aggggacaca 180
   gcagtggcta gggcatcagc cacagaactg agcgggaaat gccacttgta ttggctgtaa 240
   agaaatcctg gctttgggcc a 261
<210> 282
   <211> 372
   <212> DNA
   <213> Homo sapiens
<220>
   <221> misc_feature
   <222> (43)..(43)
   <223> n is a, c, g, or t
   <400> 282
   tccaaggact gagactgacc tcctctggtg acactggcct agngcctgac actctcctaa 60
   gaggttctct ccaagccccc aaatagctcc aggcgccctc ggccgcccat catggttaat 120
   tctgtccaac aaacacacac gggtagattg ctggcctgtt gtaggtggta gggacacaga 180
   tgaccgacct ggtcactcct cctgccaaca ttcagtctgg tatgtgaggc gtgcgtgaag 240
   caagaactcc tggagctaca gggacaggga gccatcattc ctgcctggga atcctggaag 300
   acttcctgca ggagtcagcg ttcaatcttg accttgaaga tgggaaggat gttcttttta 360
   cgtaccaatt ct 372
<210> 283
   <211> 398
   <212> DNA
   <213> Homo sapiens
<220>
   <221> misc_feature
   <222> (335)..(336)
   <223> n is a, c, g, or t
   <220>
   <221> misc_feature
   <222> (338)..(338)
   <223> n is a, c, g, or t
   <400> 283
   tccccgctag cttggggcga gcagagctgc atccagtgga actaaagccg ttccaggatt 60
   atcaaaaact gagcagcaac cttgggggac ctggatcatc acggactccc ccaactggaa 120
   ggtccttctc tggcctcaat tcccgtctca aggccacgcc ttccacctac agtggagtct 180
   tccgcaccca gcgcgtcgac ctttaccagc aggcctcccc accagatgcc ctgcgctgga 240
   tacctaagcc ttgggagcgg acagggccgc cacctcgaga agggccctcc cgacgggcag 300
   aggagcctgg gtcccgaggg gacaaggagc ctggnntngc ccccaccccg ctgagggagt 360
   tcctcttgcc ccctaccccc ggggcttgta tatagatt 398
<210> 284
   <211> 478
   <212> DNA
   <213> Homo sapiens
   <400> 284
   tgtagattta gtttgacgct ccccaaagtg catgagacac atgctaaaat tacaaattaa 60
   aattttgggt cagactttgc cataatgata gactcaattt agctctctga actagttggt 120
   aatttttttt ttttaattcc cactttggct gtgtacatca aatgaaatga gaagtgtgta 180
   tgctgaccaa accacaagaa actttcttta agttgtgtta aagaggaaag acctagaatc 240
   caagcgtgtt acatgaaaat tgtaacagag cagctgcttc cacctttcag atatagatgt 300
   tggaaccaca gcagaagtta tagagcgaca acttatatac acacctagaa tgtaagttaa 360
   acaaaatacc ggcttccaga gacccctttt ctccagccat attacatcag gctagaagta 420
   attaatgttg atttatttca tctacaagca gttggtccct aagtgaaagg ctctgctt 478
<210> 285
   <211> 336
   <212> DNA
   <213> Homo sapiens
<220>
   <221> misc_feature
   <222> (299)..(299)
   <223> n is a, c, g, or t
   <400> 285
   gtctgcctct ccaggattgt atgtttcaag ccttgtcctg tgttcctttg tctgacgctc 60
   tgtgtattgc tctttgaatc gagtttggag gaagagttga gttgtatgag tggcggcatg 120
   ttggtagtgc cggacttcct gtttcaagtt ttctggggcc tcgctaattg aatgtggaaa 180
   gtagcaccac ttgacggcta caagtgccga ctcctgaatt ttcccatggt gttctgactt 240
   caagggctgg cagccaggga gaatgggccc aggggaagca aagacctctt ccctctgcng 300
   tttctgtccc acttaactga cctcactgga ggctac 336
<210> 286
   <211> 262
   <212> DNA
   <213> Homo sapiens
<220>
   <221> misc_feature
   <222> (47)..(47)
   <223> n is a, c, g, or t
   <220>
   <221> misc_feature
   <222> (81)..(81)
   <223> n is a, c, g, or t
   <400> 286
   tcttgacatc ctagcttctt ctaagggggg agggaaaggg gggaganttt ttatatatat 60
   atacatatat atatatcaag ntttaaatta ttgatagttc atctggatta ccaaaatcac 120
   tctgcagccc tgcccgaggc tagtaggctg caaccctggt ccccacccct aacctcctgc 180
   tccccctcaa gccaactatg cagcccacaa gaaggccctg cgggcccccc cattgcccag 240
   cactgtctca tagaaggctc tg 262
<210> 287
   <211> 388
   <212> DNA
   <213> Homo sapiens
<220>
   <221> misc_feature
   <222> (70)..(70)
   <223> n is a, c, g, or t
   <220>
   <221> misc_feature
   <222> (72)..(78)
   <223> n is a, c, g, or t
   <220>
   <221> misc_feature
   <222> (80)..(80)
   <223> n is a, c, g, or t
   <220>
   <221> misc_feature
   <222> (82)..(82)
   <223> n is a, c, g, or t
   <400> 287
   tttccctttg ttcttttatc atagagacct gcctatttat tctttggcgc catctggagt 60
   actacttgtn annnnnnnan gnccacggat tctcaagatt ccttatttgc ctcgagaacc 120
   ttgtttaaaa gcagaagact gcaagattcc ttcgcctcag aaaccaatct agattttaga 180
   agtgggctgg ctatagtgag ccaacatgat ttagaccagc ttcaggctga tgcaatcaac 240
   gcttttggag aatcactaca aaagaaactt ctggacattg aaggattata ttcaaaagtt 300
   cgatctcgat atagtttcat acaggctctt gtcagacgta tccgtggcct cttgaggata 360
   tcaaggaact gagagcccgt gcttatgc 388
<210> 288
   <211> 438
   <212> DNA
   <213> Homo sapiens
<220>
   <221> misc_feature
   <222> (300)..(300)
   <223> n is a, c, g, or t
   <220>
   <221> misc_feature
   <222> (303)..(303)
   <223> n is a, c, g, or t
   <400> 288
   gagctcactg tgggatgggg ttgacctctg ccgcctgcct gggtatctgg gcctggccat 60
   ggctgtgttc ttcatgtgtt gattttattt gacccctgga gtggtgggtc tcatctttcc 120
   catctcgcct gagagcggct gagggctgcc tcactgcaaa tcctccccac agcgtcagtg 180
   aaagtcgtcc ttgtctcaga atgaccaggg gccagccagt gtctgaccaa ggtcaagggg 240
   caggtgcaga ggtggcaggg atggctccga agccagaaat gccttaaact gcaacgtccn 300
   gtnccttccc cacccccatc ccatccccac ccccagcccc agcccagtcc tcctaggagc 360
   aggacccgat gaagcgggcg gcggtggggc tgggtgccgt gttactaact ctagtatgtt 420
   tctgtgtcaa tcgctgtg 438
<210> 289
   <211> 509
   <212> DNA
   <213> Homo sapiens
<220>
   <221> misc_feature
   <222> (440)..(440)
   <223> n is a, c, g, or t
   <220>
   <221> misc_feature
   <222> (448)..(448)
   <223> n is a, c, g, or t
   <400> 289
   gtcttcccta cctcaggcag gaagggcagg aaggagagcc tgctgcatgg ggtggggtag 60
   ggctgactag aagggccagt cctgcctggc caggcagatc tgtgccccat gcctgtccag 120
   cctgggcagc caggctgcca aggccagagt ggcctggcca ggagctcttc aggcctccct 180
   ctctcttctg ctccaccctt ggcctgtctc atccccaggg gtcccagcca ccccgggctc 240
   tctgctgtac atatttgaga ctagttttta ttccttgtga agatgatata ctatttttgt 300
   taagcgtgtc tgtatttatg tgtgaggagc tgctggcttg cagtgcgcgt gcacgtggag 360
   agctggtgcc cggagattgg acggcctgat gctccctccc ctgccctggt ccagggaagc 420
   tggccgaggg tcctggctcn ctgagggnca tctgcccctc ccccaacccc caccccacac 480
   ttgttccagc tctttgaaat agtctgtgt 509
<210> 290
   <211> 442
   <212> DNA
   <213> Homo sapiens
<220>
   <221> misc_feature
   <222> (286)..(286)
   <223> n is a, c, g, or t
   <400> 290
   ttagcaacac tcatagtttt gccaattacc agtagacact agtggaacca tctaactgga 60
   acttcctctc tccttccact tatttcctca aacttgttgc tttacactag acacatgcaa 120
   atgtatgttt taaacacacc aaaacagatc atgccaaatg agttgcctgt caaaggctgg 180
   agggcaggag gagggcctgg gtttgggttc tttcctccca gcctttggat ggtgccttgg 240
   gccccttagc cccagcgcca gggcctccca gctgaggcca cagganaagc acttttttat 300
   gatgtactaa aagccacagt atgtggcaac tgcaaaagga tcaggaattt agggtatgat 360
   ctcggtcacg tgtcccgggc gctgagggga aaggaagcgg gcatgattgt agacaatgag 420
   ggggttctct tgatgtaatg aa 442
<210> 291
   <211> 467
   <212> DNA
   <213> Homo sapiens
   <400> 291
   gagacactag ttttggccaa cttaagattt tacgttaatt tttacatagt atttgacact 60
   catgcaaaat aatgtgaaaa catctagatt tagtagttta ttctgcgcct tttgttaaaa 120
   ctgaagattt tggaaaatgg ttgtcactgc tcttccagcc tatgaatatt tttgtgaaat 180
   ggaaccatgg atttatgtct ggatcatcca tacagaacca acaattttat tcaaaaacaa 240
   tgtgttcatc aaagtaattg ctcacattgt gcagtactat gttgtacaga ccacgtgaaa 300
   gggaatgctg gtctagctgg cgtggtatgt ttataggcga atttcagcag aaggaagcca 360
   aaatagtttt ttccttttga aagtttttta aaaattattt catgggtctt ttttttaatt 420
   aatatgtgtg cattgttaca atgtatgttg gatgtctttt gacccta 467
<210> 292
   <211> 356
   <212> DNA
   <213> Homo sapiens
   <400> 292
   ttagagccat catcatccca ggcagggata tctttgagaa atgactcagt tcagccccag 60
   gcccctgtga ctctgcttaa agcacacatt tctgctgact cttgtacctg gggcagcagg 120
   ataatcacca acacactctt aacgagaaac aacacaccaa gcacagtgga gctgtcctag 180
   gcaacactcg cggtctcagg ctgcggtggg cgtctgtcct gcatgtggcc cagaccaccc 240
   tgacccccgg gcctgcctgc ctggccctgc atgctgcacg ctcactgtat ttgtgcagat 300
   cctggccagt acaaagtcgt tgctcttgtc ttatcttctc ttacagagtc tccctc 356
<210> 293
   <211> 203
   <212> DNA
   <213> Homo sapiens
   <400> 293
   gtctccctcc ctttatagaa tgtcaaccaa agagtgccct cctcccctct cagcctcctc 60
   tttagctagc ctccccatct catcacaacg catgtctgtg acctttggta atcatttaca 120
   gtgccacacg gaaccctgta ttttgcacac agcaaaacaa acaatgttta gctttattta 180
   tggtatttga tgctgtaaat gga 203
<210> 294
   <211> 487
   <212> DNA
   <213> Homo sapiens
   <400> 294
   aagaaccagt gtcaatccgc agaccctctg tgaagccagg ccggccgggc cgagccagca 60
   gcccctctcc ctagactcag aggcgccgcg gggaggggtg gccccgccga ggcttcaggg 120
   gccccctccc caccaaaggg ttcacctcac acttgaatgt acaacccacc ccactgtcgg 180
   gaaggcctcc gtcctcggcc cctgcctctt gctgctgtcc tgtccccgag cccctgcagg 240
   tccccccccg cccccccact caagagttag agcaggtggc tgcaggcctt gggcccggag 300
   ggaaggccac tgccggccac ttggggcaga cacagacacc tcaaggatct gtcacggaag 360
   gcgtcctttt tccttgtagc taacgttagg cctgagtagc tcccctccat ccttgtagac 420
   gctccagtcc ctactactgt gacggcattt ccatccctcc cctgcccggg aagggacctt 480
   gcaggga 487
<210> 295
   <211> 528
   <212> DNA
   <213> Homo sapiens
<220>
   <221> misc_feature
   <222> (153)..(153)
   <223> n is a, c, g, or t
   <220>
   <221> misc_feature
   <222> (351)..(351)
   <223> n is a, c, g, or t
   <400> 295
   ctggccgggg atttgcgaac caaagcgacc attgagctca aggccctcag gctgctgaac 60
   ttccagaggc agctgcgcca ggaggtggtg gtgtgcatgc ggagggacac agcgctggag 120
   acagccctca atgctaaggc ctacaagcgc agnaagcgcc agtccctgcg cgaggcccgc 180
   atcactgaga agctggagaa gcagcagaag atcgagcagg agcgcaagcg ccggcagaag 240
   caccaggaat acctcaatag cattctccag catgccaagg atttcaagga atatcacaga 300
   tccgtcacag gcaaaatcca gaagctgacc aaggcagtgg ccacgtacca ngccaacacg 360
   gagcgggagc agaagaaaga gaacgagcgg atcgagaagg agcgcatgcg gaggctcatg 420
   gctgaagatg aggaggggta ccgcaagctc atcgaccaga agaaggacaa gcgcctggcc 480
   tacctcttgc agcagacaga cgagtacgtg gctaacctca cggagctg 528
<210> 296
   <211> 438
   <212> DNA
   <213> Homo sapiens
<220>
   <221> misc_feature
   <222> (121)..(121)
   <223> n is a, c, g, or t
   <400> 296
   cagggcaact cccagggatg tggtgacatg cagggttcaa gtgttcttgg ttccaggcac 60
   ctcccggctc acggggagct cagaggtcca tgccgaggag accaggcagg acctcccgag 120
   nctgcgcccc ggccggccca tgcgttttgt gatcccaagt gactctgtgg gaagggtggg 180
   gacgaggcgt cgggagggta tacagggagc ccctcccgtg catggctgcc cccccgttca 240
   ttttctccac cacagccgct tgcacgtata gatactgtgg tcccctttct tttaatatat 300
   aaattatgta tggtgaagtg gagtgtattg tgtaggtccc gtatttaatg cctctgactg 360
   cctttgaagc gcagccctct gtggcccgca gccccctgag cctggctgtt gtgtggtatt 420
   tatgctctct ttgtctgc 438

<210> 297
   <211> 497
   <212> DNA
   <213> Homo sapiens
   <400> 297
   aagctcccat tttgtaacca ctagtttgcg gttgacttga gtactctggt gacttcctgc 60
   gtcaagcgtt ctcaagctgt gagaatgtgc gcagctccag gcaggttttc tctcggagag 120
   ttaagtcttc ccttgaaggc agggaagcag gatggataca catatatcac acgcataaaa 180
   caccaggtgc gggagcagcc cagactcaag gctgactaaa ctggaggctg aataccgtgg 240
   aggtccacat gcagcttccc tggagggcag gccggaggcg ctcccgcccc tgggcttgag 300
   gatgctgcac cccgtgggct tccaggcctg cccagatgat gccttcaggc ctctgtccct 360
   ggcggccatc ctcaggccga ttttgaccag caatgataga ctcttcttaa ccctttcaaa 420
   ataaattttt cagtgggaca gaaaggagag ttaaaaaaca tttttttaaa ggtggtaaca 480
   tctgacccac aaaggga 497
<210> 298
   <211> 557
   <212> DNA
   <213> Homo sapiens
<220>
   <221> misc_feature
   <222> (73)..(73)
   <223> n is a, c, g, or t
   <220>
   <221> misc_feature
   <222> (244)..(244)
   <223> n is a, c, g, or t
   <400> 298
   cctcatccgc tacatgagct ctgggcctgt ggtggccatg gtctgggaag ggtacaatgt 60
   cgtccgcgcc tcnagggcca tgattggaca caccgactcg gctgaggctg ccccaggaac 120
   cataaggggt gacttcagcg tccacatcag caggaatgtc atccacgcca gcgactccgt 180
   ggagggggcc cagcgggaga tccagctgtg gttccagagc agtgagctgg tgagctgggc 240
   agangggggc cagcacagca gcatccaccc agcctgaggc tcaagctgcc cttaccaccc 300
   catcccccac gcaggaccaa ctacctccgt cagcaagaac ccaagcccac atccaaacct 360
   gcctgtccca aaccacttac ttccctgttc acctctgccc caccccagcc cagaggagtt 420
   tgagccacca acttcagtgc ctttctgtac cccaagccag cacaagattg gaccaatcct 480
   ttttgcacca aagtgccgga caacctttgt ggtggggggg ggtcttcaca ttatcataac 540
   ctctcctcta aagggga 557
<210> 299
   <211> 449
   <212> DNA
   <213> Homo sapiens
<220>
   <221> misc_feature
   <222> (60)..(61)
   <223> n is a, c, g, or t
   <400> 299
   atagggtttt cttgggcgag gatgtgctgg attaggaaag gtgacatgac acaggcagan 60
   nagagtggca cccaccacag aatacagtgt gtgttattac gaggagccag cagttgagcc 120
   taaggtcctt ctacctacct ggtattggca tttgaggtcg gaaaccctct actgccccat 180
   aagccaggaa aagtgaaaag agaacacagt tcctttaaga actggcagca aggcttgagg 240
   ccttatgtat gtagctgagt cagcaaggta catgatgctg tctgctttca aaaggacttt 300
   tctctcctag ctgactgact ccttccttag ttcaaggaac agctgagaca gacctctgct 360
   gagtagctct gtgatgacaa agccttggtt taactgaggt gatcctcagg ttgtgaggtt 420
   tattagtccc caaggcaaac acaaatatt 449
<210> 300
   <211> 311
   <212> DNA
   <213> Homo sapiens
<220>
   <221> misc_feature
   <222> (125)..(126)
   <223> n is a, c, g, or t
   <400> 300
   atcaagtcca actgaaacat cagaacaaat aagagagaaa taagaataga atgaatgacc 60
   ccaaaatagg gttttcttgg gcgaggatgt gctggattag gaaaggtgac atgacacagg 120
   cagannagag tggcacccac cacagaatac agtgtgtgtt attacgagga gccagcagtt 180
   gagcctaagg tccttctacc tacctggtat tggcatttga ggtcggaaac cctctactgc 240
   cccataagcc aggaaaagtg aaaagagaac acagttcctt taagaactgg cagcaaggct 300
   tgaggcctta t 311
<210> 301
   <211> 395
   <212> DNA
   <213> Homo sapiens
   <400> 301
   gctctggtgc tagatgccac tgtagccaga tctccaacag tgccttggac catggactca 60
   tactcaactg agtaagaagg ggctggtgcc cagtcggggt ggctgagctg gtccttaata 120
   ggttgtttct tggtcttgct ttcttcatgc cctccccact gctcctgcca cctttagata 180
   agtttctcta gctaattttg tggccaatgt aaaattcgtc atcaacctaa caaacacaac 240
   cttctcagca gcatttctcc cctgtgatgg aaataaagtg tttagggcag tgggaggaga 300
   aaattctcca ggtgaatggg gaagggtctg ttccagcctc tccctactcc catcccattt 360
   ccaccaactg gggaactgtg actatctatc tcccc 395
<210> 302
   <211> 517
   <212> DNA
   <213> Homo sapiens
   <400> 302
   tatgttatgt gtgtgactcc cttgtgtgta tctgtgccag cctcagcctc cgagttgctt 60
   ttccctctgg ccctgactct cactgactca ccgatgtgat gtgcaggccc acttcttacc 120
   ccagatagcc tcgggcgctg cctgtagtca tgctgacagc tgtacagtag ccgccaagac 180
   tgctgacagc tggagacggt tctggtttca actacggtat attgatatcg gaagtattct 240
   agacagatcc tcggttgggt tttctagcta catgtttgta ttgcacagat ccccacctgc 300
   catcctatag tgttgtcttc ctgtgtgttc cggggcttct gggcagctgg gcctgcccgg 360
   ggaagtcctt gcaggtggga ggccatacag agacccactg tgtgccactg agcgtcccac 420
   tgctgctggg caactggagg actgcagggg gcgccaggtg actctctcct tttatatcac 480
   agcagctcct gtgctgacct tcaagttacg ttttgga 517
<210> 303
   <211> 520
   <212> DNA
   <213> Homo sapiens
<220>
   <221> misc_feature
   <222> (51)..(51)
   <223> n is a, c, g, or t
   <220>
   <221> misc_feature
   <222> (392)..(392)
   <223> n is a, c, g, or t
   <400> 303
   tgtagtgttg taaacctgcc tcacaaaata catggtaata acttttcttt naaaaaaaaa 60
   aaaaaagaca gcctttacac catttctagt ggcacactat tttggcaatg ttatgcacca 120
   cttcaatttc cccattgtga cccctatcac ttcatttgat atcccttttt gacccaccca 180
   tctccttcat atatgggcat gtccatagat tgacaaagaa agtttacact tttgaataaa 240
   gatgcaaagt atgcaaaaac attaatactg atgcgaaaaa ataaaaaata aaagagaaac 300
   aaggcagagg aagaaggtgt ttaagctctc ctcgacctgt tggaatggtg gttaacagaa 360
   tgatttgaga tgggatctgt ggggagggga gnaaaaaaaa aaacaacaaa atttggtgct 420
   taaaaaaaag taaaataaaa aaagacatct ttaaaatcaa tccctggttg tagacaagtt 480
   ctccaaaacc agtacctggc accactccaa caaacaaacg 520
<210> 304
   <211> 329
   <212> DNA
   <213> Homo sapiens
   <400> 304
   gctggcttcg tttttccaag gagcctttgg tgagttcaat tatctggtaa atatccagcg 60
   cttcacctga aagatagtgc aaattggtta ggatgccacc tcaagaactg taactgagag 120
   ctcagaagtg agcaaaggag cttaatgcta aggtcaaaag gagagtgaaa ggttgagaac 180
   aattgccacg aacggtaatg ttacatgtta ggagggtctg ttttcttttt atataagtgt 240
   gtcttagata tattttaaat agaaaataag ctttctgatt tacttgtttg gtatttaaag 300
   cacagtttgt ttttctgtca cctatagag 329
<210> 305
   <211> 521
   <212> DNA
   <213> Homo sapiens
<220>
   <221> misc_feature
   <222> (481)..(481)
   <223> n is a, c, g, or t
   <400> 305
   tacatttttc cacgagctgg tgcagacagc tctgccatca ggcagctgtg tggacacctt 60
   gttaaaggac ttgtgcaaaa tgtacaccac acttacagcc cttgtcagat attatctcca 120
   ggtgtgtcag agctccggag gaattccaaa aaatatggaa aagctggtga agctgtctgg 180
   ttctcatctg acccccctgt gttattcttt catttcttac gtacagaata agagtaagag 240
   cctgaactat acgggagaga aaaaggagaa acctgctgcc gttgccacag ccatggccag 300
   agttcttcgg gaaaccaagc caatccctaa cctcatcttt gccatagaac agtatgaaaa 360
   atttctcatc cacctttcta agaagtccaa ggtgaacctg atgcagcaca tgaagctcag 420
   cacctcacga gacttcaaga tcaaaggaaa catcctagac atggttcttc gagaggatgg 480
   ngaagatgaa aatgaagagg gcactgcatc agagcatggg g 521
<210> 306
   <211> 496
   <212> DNA
   <213> Homo sapiens
   <400> 306
   ctttctgcct gtactggatc tgttattttc agggaaacag gccccagggc ccccctgagc 60
   ctcaccctaa gcccttaggc ctctgagagt gctgttgggt tctatttatt tatttatttg 120
   ttcctttgtt ccctacccgt gcccccagtg tcttccctgc tgagtaccag gagaggtcct 180
   gccccatcct ctctctgaag ccagggccct tccattccat ttagcctttg gatcatcctg 240
   gctgggagaa gtgggaccga gccacccagc cccactatcc ccaagcagcc ctacagccgg 300
   gatgggaggc acgtggcctc tcttttatcc gtctatttat tttgtaagtg tattcgtgtg 360
   gaggaggttg ttgctttatt tttttaaggc tctggagtgt tgtgtatggt ttcttttcac 420
   atcccagcct cccatgggca cttctaagaa gagaggggat ttcttggaaa aggagagagg 480
   aatcccctag agcagg 496
<210> 307
   <211> 503
   <212> DNA
   <213> Homo sapiens
<220>
   <221> misc_feature
   <222> (158)..(158)
   <223> n is a, c, g, or t
   <220>
   <221> misc_feature
   <222> (216)..(217)
   <223> n is a, c, g, or t
   <220>
   <221> misc_feature
   <222> (231)..(231)
   <223> n is a, c, g, or t
   <220>
   <221> misc_feature
   <222> (250)..(250)
   <223> n is a, c, g, or t
   <220>
   <221> misc_feature
   <222> (261)..(261)
   <223> n is a, c, g, or t
   <220>
   <221> misc_feature
   <222> (291)..(291)
   <223> n is a, c, g, or t
   <220>
   <221> misc_feature
   <222> (297)..(297)
   <223> n is a, c, g, or t
   <220>
   <221> misc_feature
   <222> (341)..(341)
   <223> n is a, c, g, or t
<220>
   <221> misc_feature
   <222> (352)..(352)
   <223> n is a, c, g, or t
   <220>
   <221> misc_feaures
   <222> (365)..(365)
   <223> n is a, c, g, or t
   <400> 307
   gcgggccaca gacgtcggaa gaaactcccg tatttgcagc tggaactgca gcccacggcg 60
   ccccggtttt cctccccgcc ctgtccctct ctggtcaaac aacatactaa agaggcgagg 120
   caatgactgt tggccagttc tcaccgggga aaaacccnac tgttaggatg gcatgaacat 180
   ttccttagat cgtggtcagc tccgaggaat gtggcnncca ggctctttga ngagccatgg 240
   gctgcacccn ggccgtaggc ntagtgtaac tcgcatccca ttgcagtgcc ngtttcnttg 300
   actgtgttgc tgtctcttag attaaccgtg ctgaggctcc nacatagctc cntggacctg 360
   tgtcntagta catactgaag cgatggtcag agtgtgtaga gtgaagttgc tgtgcccaca 420
   ttgtttgaac tcgcgtaccc cgtagataca ttgtgcaacg ttcttctgtt attcccttga 480
   ggtggtaact tcgtatgttc agt 503
<210> 308
   <211> 434
   <212> DNA
   <213> Homo sapiens
   <400> 308
   tgagagctgt ctaggtctgt atcccagatt gttgcttaat gacatctgac agatgcattg 60
   ttttctgaaa tcagcttaag acaccaattg tggcaactgg aaactcatta cctgctgcat 120
   tggatcaact atggaagttg gagcaggggt gggcggaggt cacctaacca atcaatggaa 180
   ggcaactcac acctgctcca agcctcagct ttgagaaaca aacacgttta taagaaaaaa 240
   tatatagcta ttattacaga agtgaatatg ttgtgctctc ttactgctct tggtgcattg 300
   acagtttctg tatctcaacc ctattcatct ttatgaaaaa gcattctgaa gatctatcct 360
   cagcactgct gagtgtgcag tcacactttc ctaccaaccc ccttcttacc atctctagct 420
   gccatttgtg gggg 434
<210> 309
   <211> 572
   <212> DNA
   <213> Homo sapiens
<220>
   <221> misc_feature
   <222> (163)..(163)
   <223> n is a, c, g, or t
   <220>
   <221> misc_feature
   <222> (486)..(486)
   <223> n is a, c, g, or t
   <220>
   <221> misc_feature
   <222> (547)..(547)
   <223> n is a, c, g, or t
   <400> 309
   aacaggcccc tatagggaag cagttccatg aaaatgatta attctttcca aaagacttaa 60
   aattttttcc tatttcaatt ttcctttcaa aaaaggaaat acattcatgt agttcaaaac 120
   ttaagaaaac aaaagtctgt tcagcaaaag actcccactc cgnttcccca aacgctgagc 180
   cccacccccc atccctggta gcaagaagtg tttccaattt taaggttaag aaacaaagtc 240
   cctggatttg tgttagggat gtcttcctga gagtgggttg tgttccgttt gaccctggcg 300
   gttgacctcg gcccactagg atcatgccgc cctctccagg gaggagggcc tccccatcac 360
   cctgtacagt ggcaccccag ccctggcact gcccgccctt gctcagcgta cctttcccat 420
   ggcactctga cgtactggat gtttggtttc tgaagttact ggctgtttac cctgccggga 480
   tgtaancccc tccagggcag gggcttgtct cgtgttcagt gctgcatccc agctgctggg 540
   cacggtncct ggtccatggc gttcaataaa ta 572
<210> 310
   <211> 549
   <212> DNA
   <213> Homo sapiens
<220>
   <221> misc_feature
   <222> (73)..(73)
   <223> n is a, c, g, or t
   <400> 310
   tttttgatgt gcgtgctgtc tgtcctatgg agcctctgca gactcgttct cgtgacccag 60
   tggcataccg ttnggtgtct gatgtgtgcc cagatcgttc tgccacttgc actgtgcttg 120
   ctcctaagca aaagggaaaa ggagcgcgcg tgatagaaga aaagcactgg gagaactaac 180
   agaggagaaa ggtgaaacac acacacattc ttaaggcaat aaaactaggg ggtgtatatt 240
   atcttctggt gcatgttctt ttctggaaaa tatggtagct cgccaaccgc atctgctcat 300
   ctgatattca aacacacagt attcgtgaat aagttgattc tgtcccccac gtggactctg 360
   tgctcaccca ttgtctcatt gccagtggtg tccaagggcc cccgttggga cccacggctc 420
   tcgtccctct gctccgtgtg tctcatgcca gcagcacgtc gccatccgtc accagaatta 480
   gtcctcacag cctaggacca gttttgtatc aaactcgtct gatgttttga tgccatttgt 540
   cttttgtaa 549
<210> 311
   <211> 463
   <212> DNA
   <213> Homo sapiens
<220>
   <221> misc_feature
   <222> (37)..(37)
   <223> n is a, c, g, or t
   <400> 311
   gctacatgag ggtgtccctg tccagctttc tggcacntga gtcctgtgtg gagagttacc 60
   tcctcttcca gggactgtgc tgttgggaac tttgggcaag tcacttacct ctttgtgcct 120
   caatttctgt ataatatttc taagctacct cactgaggtg gtatgaagat tcactaatgt 180
   atgtagcgtg tttgtcaatc ctccagtgaa aagcactatc tagatcacat tttggatcac 240
   attagccaaa tgcagtaaat ggccaaatta gatgtgtgct gaagacaatc agtcactggg 300
   tctatattaa acagcaacca gagcaacaaa tggcaaacaa tttctatttt caagtttctt 360
   tgcatatttt tttggtgcaa aaccatttat aaactttttt ttctaacact agtgtctaca 420
   gcagcattca aaaaaattct gttacctttt ctgtattagg att 463
<210> 312
   <211> 238
   <212> DNA
   <213> Homo sapiens
   <400> 312
   tgggatctca gatcctttgt cactgcctat agacttgtag ctgctgtctc tctttgtccc 60
   tgcagagaat cacgtcctgg aactgcatgt tcttgcgact cttgggactt catcttaact 120
   tctcgctgcc ccagccatgt tttcaaccat ggcatccctc ccccaattag ttccctgtca 180
   tcctcgtcaa ccttctctgt aagtgcctgg taagcttgcc cttgcttaag aactcaaa 238
<210> 313
   <211> 497
   <212> DNA
   <213> Homo sapiens
<220>
   <221> misc_feature
   <222> (26)..(26)
   <223> n is a, c, g, or t
   <220>
   <221> misc_feature
   <222> (61)..(61)
   <223> n is a, c, g, or t
   <220>
   <221> misc_feature
   <222> (64)..(64)
   <223> n is a, c, g, or t
   <220>
   <221> misc_feature
   <222> (68)..(68)
   <223> n is a, c, g, or t
   <220>
   <221> misc_feature
   <222> (117)..(117)
   <223> n is a, c, g, or t
   <220>
   <221> misc_feature
   <222> (148)..(148)
   <223> n is a, c, g, or t
   <220>
   <221> misc_feature
   <222> (173)..(173)
   <223> n is a, c, g, or t
   <220>
   <221> misc_feature
   <222> (214)..(214)
   <223> n is a, c, g, or t
   <220>
   <221> misc_feature
   <222> (218)..(218)
   <223> n is a, c, g, or t
   <220>
   <221> misc_feature
   <222> (231)..(231)
   <223> n is a, c, g, or t
   <220>
   <221> misc_feature
   <222> (275)..(275)
   <223> n is a, c, g, or t
   <220>
   <221> misc_feature
   <222> (293)..(293)
   <223> n is a, c, g, or t
   <220>
   <221> misc_feature
   <222> (305)..(305)
   <223> n is a, c, g, or t
   <220>
   <221> misc_feature
   <222> (318)..(318)
   <223> n is a, c, g, or t
   <220>
   <221> misc_feature
   <222> (323)..(323)
   <223> n is a, c, g, or t
   <220>
   <221> misc_feature
   <222> (435)..(435)
   <223> n is a, c, g, or t
   <400> 313
   gcagtgagcc aagacagtgc cagtgnactc cagcctcggt gacagcgcaa ggctccgtct 60
   naanaatnaa aaaaaaaaaa aaaaaaaaaa ggccgggcgc agtggctcaa gcctgtngtc 120
   ccagcacttt gggaggctga ggcgggcnga tcacctgagg tcaggagttt tgngatcagc 180
   cttggcaaca cggtgaaacc ccatctctac taanaatnca aaattagcca ngcatgctgg 240
   cacatgcctg taatcccagc tactcgggag gctgnggtac gagaatcgct tgnacctggg 300
   aggcngagga tgcagtgngc cgngatcacg ccattgcact ccagcctggg ggacaagagt 360
   gaatctgtgt ctcaccaaaa aaaaaaagaa aaagaaagat gcttaacaaa ggttaccata 420
   agccacaaat tcatnaccac ttatccttcc agtttcaagt agaatatatt cataacctca 480
   ataaagttct ccctgct 497
<210> 314
   <211> 563
   <212> DNA
   <213> Homo sapiens
<220>
   <221> misc_feature
   <222> (431)..(431)
   <223> n is a, c, g, or t
   <400> 314
   gcagcagatc atgagtgacc cagccatgcg ccttatcctg gaacagatgc agaaggaccc 60
   ccaggcactc agcgaacact taaagaatcc tgtaatagca cagaagatcc agaagctgat 120
   ggatgtgggt ctgattgcaa ttcggtgatg acttgttcat ccccccttcc cttcgccctc 180
   atgtggaaag aggagctggg accgcggcga gcagcacgga gcggaaggga gagcagggga 240
   gagaaggcct catctctcta tatttataca taaccccggg gaagacacag agactcgtac 300
   ctgcgctgtt tgtgccgccg ctgcctctgg gccctcccag cacacgcatg gtctcttcac 360
   cgctgccctc gagttccatg tctctttccc ctgcccctag ttgctgtctc ggctgctctc 420
   ccatagttgg nttttttttt tatttggggc agtgggcatg ttatggggag gggagggggt 480
   tcttccagcc tcaggtccca gctgtctcac gttgtttatt ctgcgtcccc ttctccaata 540
   aaacaagcca gttgggcgtg gtt 563
<210> 315
   <211> 524
   <212> DNA
   <213> Homo sapiens
<220>
   <221> misc_feature
   <222> (29)..(29)
   <223> n is a, c, g, or t
   <220>
   <221> misc_feature
   <222> (39)..(39)
   <223> n is a, c, g, or t
   <220>
   <221> misc_feature
   <222> (41)..(41)
   <223> n is a, c, g, or t
   <220>
   <221> misc_feature
   <222> (45)..(45)
   <223> n is a, c, g, or t
   <220>
   <221> misc_feature
   <222> (47)..(47)
   <223> n is a, c, g, or t
   <220>
   <221> misc_feature
   <222> (55)..(55)
   <223> n is a, c, g, or t
   <220>
   <221> misc_feature
   <222> (187)..(187)
   <223> n is a, c, g, or t
   <220>
   <221> misc_feature
   <222> (373)..(373)
   <223> n is a, c, g, or t
   <400> 315
   aacagcacct ttctcattga gcttcctcna ctgacctcng ncccncnttg ggatntcatc 60
   ttctgaccga accctgatgt tcagtggcag agacagccca tagccagaac tgtgggtaga 120
   ccagggttgg ggtgtgcggt ttgggacagc ccaaacccca gccgctgtgt caaggcctag 180
   gacgccntgc tgccatcaaa agggggttcc aggtttccat cagtggccta aagaagggac 240
   ttcttgttgt actgaggagt gcggaattaa agagatttga ctccctttag tattgggggc 300
   agtccgttcc ccagacactg tggcctctga agtggaaact gaaagctgca tacctgggaa 360
   agaactttct agnaataggc aatggccttc agtggaagag ggagggctgg aggtgtgccc 420
   agtacttgga tgttcatctg tccacaacag ctttttgttt ttttaaaaaa gctaaaatgg 480
   aaatggattt tatcataaag gatgacatcg ttttcttcta caat 524

<210> 316
   <211> 559
   <212> DNA
   <213> Homo sapiens
   <400> 316
   ggtgtgcttc gtgctgtagt tatcgttagt tcctcttccc gagatggggc cgccgagaga 60
   ccccagcgcc tttgaaaagc aaggtttgtg ctgcgcttcc agttccgaaa agcagatgtt 120
   taagcccttg gactgagggt gggatcgcag ctccgaagac ggagaggagg gaaatggggc 180
   cctttcccct ctattgcatc cccctgcccg actccttccc cgcacccacg tgccctagat 240
   tcatggcaga aaatgaccaa atcctgtgta tttgttttat atatttaata actgttttaa 300
   atgaaagttt tagtaaaaaa aatacaaaac aaaaagatta aattgctatt gctgtagtaa 360
   gagaagctct ttgtatctga acatagttgt atttgaaatt tgtggttttt taatttattt 420
   aaaattgggg ggagggcatg ggaaggattt aacaccgata tattgttacc gctgaaaatg 480
   aactttatga accttttcca agttgatcta tccagtgacg tggcctggtg ggcgtttctt 540
   cttgtactta tgtggtttt 559
<210> 317
   <211> 504
   <212> DNA
   <213> Homo sapiens
<220>
   <221> misc_feature
   <222> (44)..(44)
   <223> n is a, c, g, or t
   <220>
   <221> misc_feature
   <222> (63)..(63)
   <223> n is a, c, g, or t
   <220>
   <221> misc_feature
   <222> (94)..(94)
   <223> n is a, c, g, or t
   <400> 317
   gtgcctcagg agtattcttc tacaccagct gctgttaaaa tgtncaatga actctagtcc 60
   canggaatac agaagtgctc ttattaccag tttncccact tgtggccgcc tttgcaaaga 120
   tccatattct aatttaagtc cccaacctct gaatttggtt ttaagtttac ctagtgactg 180
   actactctct ttataaaaaa gaccttatac ttaatgatca tttccaaagg agaccactcc 240
   ttaactttta ctgcaaaccc aacaagatga gacacttaaa cccagacaga tgtaacaaag 300
   gatttttgtt gtctaagtcc caaagtatta tatagaaagt tcctgctttt atgggtaaac 360
   ttattacctt aatatgttct gtggtttgct gttaaccaag attctcccat ttaaaatgcc 420
   acagaccgac cctcaaggca gatccgaaag cctagtagtt agttgcactg ggttgttttg 480
   acaagctacc acacgtctta agta 504
<210> 318
   <211> 568
   <212> DNA
   <213> Homo sapiens
   <400> 318
   acaggcggtg tgagcatcca tgtgtggtct tggtctaaac cagctcttga acaggttaaa 60
   gcaaacagca ataacaaaac aaaaactact gatgctgagc gttttgatcc tagtaatatt 120
   tcaaatattg tccttctgca tatgttctat ccatatttga ttccaatata cattattaag 180
   ctttcttggg tactattttg ctggggctct tgcgtgaagg tggtacctgt ctcatgatcc 240
   ttaaaagaga gaggcttttt tcatccaaag ctgtagtgtt gggaactggg gtgggagagg 300
   cactttttgg aattctgaaa gaatcatatc tgtgtatata catactgagt ggggaaggat 360
   gggggttggc aggggttgag ggaggtggga acaaacagtg agtatgggaa caggcagtca 420
   cctcgagtgt gggaggtcac ctgggtccgt cgtcttcctt ctgtatggtg ttgggtttat 480
   gtacacacta taacacttcc tgtgtgagtt catgtacctg tctgtgagtg ctttggtgta 540
   ttgagcctca gtacactcca agggcatt 568
<210> 319
   <211> 543
   <212> DNA
   <213> Homo sapiens
<220>
   <221> misc_feature
   <222> (36)..(36)
   <223> n is a, c, g, or t
   <220>
   <221> misc_feature
   <222> (62)..(66)
   <223> n is a, c, g, or t
   <220>
   <221> misc_feature
   <222> (402)..(402)
   <223> n is a, c, g, or t
   <400> 319
   ttaaagtact tctctagtca ttgaagtttt tttttncttt acataaatat tgatatattc 60
   tnnnnnctac tcaaagtgcc aaaggctaca gtttttaatg acttaacaaa ttgtaccaca 120
   ttgttaagga catataatga tagacactag aactcagacc tctgcatgta tatttgataa 180
   catgtctttt gtaaaacaaa aattacaaaa aaatttgttt acattccact ggtaccttaa 240
   tttaaaataa atcagactaa aaggtggtat ctcttcttag tgttctattt atcttatttg 300
   ctaatgggag cacttcttcc tttgttaggc tgtgctttac tgataaaacc aagtattgaa 360
   taaagagagt taattatctt tttaaagtaa ataaaattat gnaaatatat atagtatata 420
   taaagtactg tgtttaaaaa aatgttatgc aatgttttcc aaactgataa agtttgtaaa 480
   gtgctataaa tgtattttgt taagtacaga taaaagctat tgtgtgagta tattgtgcta 540
   aaa 543
<210> 320
   <211> 258
   <212> DNA
   <213> Homo sapiens
   <400> 320
   gagagacgct ccattgtgaa taaagagctc ataccagctc ctaagcccta ttaagaagag 60
   gcctggtcct ctaatgcctt gtttccattt cagttgttct ttgagagaca gaatgatgta 120
   ctaaccattc gtgattatta agatagggtt gggtcagggc ttagggaggg ggcagaaata 180
   ttggggatag aaaaaaaatc tgatcattcc tcagtgctac ccatttctgt cctgtgtggg 240
   ctgcttagct agacagca 258
<210> 321
   <211> 263
   <212> DNA
   <213> Homo sapiens
<220>
   <221> misc_feature
   <222> (92)..(92)
   <223> n is a, c, g, or t
   <220>
   <221> misc_feature
   <222> (190)..(190)
   <223> n is a, c, g, or t
   <400> 321
   aggggaagaa acgacagcct cacttctgta tggactgctg atgtggcctg ccatcctgtt 60
   cagcgggcat tgtctttgga gcagcaggag antaggatgc ctctcactca catgccagtt 120
   cctggctggc cagctgctca gggctcaggc tggggcctcc cattgacatc ctccccctac 180
   actccctctn tgagcctccg tcgcccctcc tgttgggtaa gggtgttgag tgtgacttgt 240
   gctgaaaacc tggttcatat ata 263
<210> 322
   <211> 529
   <212> DNA
   <213> Homo sapiens
   <400> 322
   gactgtctca tgtatctgca agggccgagg aaattaatga cccaaggagg ctatgatatg 60
   gtccaaaaac ttttcctgga ttttttccgt aggcggctga gccagaggcc aactgcagag 120
   gaactggaac agaggaacat tttgaaacct cggaatgaac aagaggaaca ggaggagaag 180
   agagagatca agaggaggct aacccgaaag ctcagtcaaa ggcccacggt ggaagagctt 240
   cgggaaagaa agatcctcat ccgcttcagt gactacgtgg aggtggctga cgctcaggac 300
   tatgaccgca gggcagataa gccgtggacc cgcctcaccg ctgcagacaa agctgccatc 360
   cgaaaggagc tcaatgaatt caaaagcact gagatggaag ttcatgaatt gagtagacac 420
   ttaacaaggt ttcaccgacc ttaacagtcg aattcctctt gagtgctatg ctgtcttcaa 480
   aacataaatt tataagaacc ataagtgctg gtatttattc acttcccca 529
<210> 323
   <211> 467
   <212> DNA
   <213> Homo sapiens
   <400> 323
   gacatggtac cagatgcgct gcagcagaac ccgggcgcct tcaggctagc tcccgccctg 60
   cctgcccggc cccaccgagg cctgagcacg ttcccgggtg ccgagcactg cctccgggct 120
   tcccccaaga ccacgcttag cggtggcttc ttcgttgctg taattgaacg ggtcgagatg 180
   ccgacgtgag tgagtggggg catgcttggg aggcgcagga tggtactggc acatctaaca 240
   tctacacttc tctagctcag cctcacaggc caaagcatca gcaccagaac gcacacccag 300
   cccagcccca aagagaaaga agagacagca aagagccgca gccggtgctt gcacaccgcc 360
   ttgcacatag cagaggctcc aggctgactc cttcctggtg ggaaaggaag atgcctgtcc 420
   tctccgtgga ggaccctggg ccctcaccgc aggcagcagt ttgcatt 467
<210> 324
   <211> 145
   <212> DNA
   <213> Homo sapiens
   <400> 324
   gagaattccg aattggggaa cacacgatac ctgtttttct tttccgttgc tggcagtact 60
   gttgcgccgc agtttggagt cactgtagtt aagtgtggat gcatgtgcgt caccgtccac 120
   tcctcctact gtattttatt ggaca 145
<210> 325
   <211> 208
   <212> DNA
   <213> Homo sapiens
<220>
   <221> misc_feature
   <222> (85)..(85)
   <223> n is a, c, g, or t
   <220>
   <221> misc_feature
   <222> (100)..(100)
   <223> n is a, c, g, or t
   <220>
   <221> misc_feature
   <222> (102)..(102)
   <223> n is a, c, g, or t
   <220>
   <221> misc_feature
   <222> (119)..(123)
   <223> n is a, c, g, or t
   <220>
   <221> misc_feature
   <222> (125)..(126)
   <223> n is a, c, g, or t
   <220>
   <221> misc_feature
   <222> (128)..(128)
   <223> n is a, c, g, or t
   <220>
   <221> misc_feature
   <222> (148)..(148)
   <223> n is a, c, g, or t
   <400> 325
   cctggggctg agcaaggcct acgtaggcca gaagagcagc ttcacagtag actgcagcaa 60
   agcaggcaac aacatgctgc tggtnggggt tcatggcccn angaccccct gcgaggagnn 120
   nnngnngnag cacgtgggca gccggctnta cagcgtgtcc tacctgctca aggacaaggg 180
   ggagtacaca ctggtggtca aatggggg 208
<210> 326
   <211> 354
   <212> DNA
   <213> Homo sapiens
   <400> 326
   gctccactgc ttaaaccaca ggacctggtt aactcctcac caagcttccc acgaccctgg 60
   ttgccaatgg gcgcgggaga cattgtatac acatcatgct atttaaaata cgttcaaact 120
   atagtgtaaa tgctaattaa ccatattggt atataaccgg aattttatat taaaaggggc 180
   ctccttttta aatatatgcc gtgtaaaaaa tgtacttata ggaacatctc tttgaattgt 240
   atttcttgta tattacatac ttagagagag actcttttag ccaggcaaag tcttttttgg 300
   ctgtggctgg aataaatcat ttattacttg ggagtcccat tttggacact aata 354
<210> 327
   <211> 518
   <212> DNA
   <213> Homo sapiens
<220>
   <221> misc_feature
   <222> (61)..(65)
   <223> n is a, c, g, or t
   <220>
   <221> misc_feature
   <222> (71)..(71)
   <223> n is a, c, g, or t
   <220>
   <221> misc_feature
   <222> (73)..(73)
   <223> n is a, c, g, or t
   <220>
   <221> misc_feature
   <222> (112)..(112)
   <223> n is a, c, g, or t
   <220>
   <221> misc_feature
   <222> (163)..(163)
   <223> n is a, c, g, or t
   <220>
   <221> misc_feature
   <222> (295)..(295)
   <223> n is a, c, g, or t
   <400> 327
   aaggactggt atctttctgt gagcaataag gactggataa agactgcata tccttgtgtc 60
   nnnnncagca ncnatacaat aaggagggtt ttaatgtgaa gcaggcaatc tnccagcccc 120
   ttctggtctt ggatgaaata gttgcacaga gtattgcacc aanaatacac aatggaggct 180
   gaaaagttca acatatttta agtcaattaa tcaaattgca ttgattcttg atgctttctt 240
   agaggcctac atgatttctt agattgctct gataaactat cataaggggt ccacntcccc 300
   tcatttagct cccccaggga tttcttttcc cccatgtcat acacccagtc ctaaatcaac 360
   ccccaaggct atccttccat cccttctgca gagggaactt ttgtcagact ctgcaacaaa 420
   ctcctagctc tatccagagt gtcctctgct gctaagattg gtatctttct cctcaaaagc 480
   ctggatggtg aatgggggtg cattagtcag aattctcc 518
<210> 328
   <211> 509
   <212> DNA
   <213> Homo sapiens
   <400> 328
   ccaaaggttg tttctcccat tgtgcatgtc cttcagtctc ggccccatac ccatcacccc 60
   attcttcacc ctcatgcttc catcccaagg caaacatgtg tcttcatcgg aatctatggg 120
   tgttgaagtt aaatgtgggg gcagagattt aacaccatga cactaataca aatcaaccat 180
   tcttcacttt caaatggtta atcactacag gaaggcgaac tcttttcttg gtttttgttt 240
   aaaaacattt tatacatata tatgtatata tgtgtgtgta tgtatggaca taggtatgta 300
   tatgcacatg tacatgtata tatgtatata tccatcttca atataaatat atcataagtg 360
   agagttgtaa atactccttg gtcatatgtc tgtctttctc atagtatcat atcttcaatg 420
   ttatgttaac aactccattt attgattgat gaaatcgtgt gtagacctgt atcctcctga 480
   catagtttat gtagggtctc ttctcaaat 509
<210> 329
   <211> 539
   <212> DNA
   <213> Homo sapiens
<220>
   <221> misc_feature
   <222> (27)..(27)
   <223> n is a, c, g, or t
   <220>
   <221> misc_feature
   <222> (40)..(40)
   <223> n is a, c, g, or t
   <220>
   <221> misc_feature
   <222> (49)..(49)
   <223> n is a, c, g, or t
   <220>
   <221> misc_feature
   <222> (64)..(64)
   <223> n is a, c, g, or t
   <220>
   <221> misc_feature
   <222> (84)..(84)
   <223> n is a, c, g, or t
<220>
   <221> misc_feature
   <222> (90)..(91)
   <223> n is a, c, g, or t
   <400> 329
   atgaacgacc tggtgtccga gtaccancag taccaggacn ccacggccna cgaacaaggg 60
   gagntcgagg aggaggaggg cgangacgan ncgtagatgc ccccgcgaga cgggttaggg 120
   aaagcggagg aggaaagcga gggggtgggg ggcttcccgg gacgataacc tggcagtgga 180
   aggaaagaag catggtctac tttaggtgtg cgctgggtct ctggtgctct tcactgttgc 240
   ctgtcacttt tttttccttt tttgtaatat tgatgacatc aatgtaacat ttgagatatt 300
   tctgaattac tgttgtaatg gctaaaatca cataaacgtt tgtgtcggaa tggtgtcctc 360
   tctttctctt cctttttctc tttattaacg atttaaatgt aactttctga acacattgca 420
   ttgaattctt cctttaacaa aaagcaaagg cgtaggtaaa agctcaaatg aatttattct 480
   ttcggtatgg taaaattgaa ccaatcacag ttaagatgag agatcaacct gagttttaa 539
<210> 330
   <211> 471
   <212> DNA
   <213> Homo sapiens
   <400> 330
   taaaaaacag caccctatcc tgcttcccca catttctgtt cctccaatga agggctaaga 60
   ctatttagta atctctttct taagcagagg agtggcaagg atggcaatct tgaattttat 120
   tttctgtaga gatagcattt cttctggtgc ggagctgaaa ggaatccacc cagaagttct 180
   gtagcatcct gcgtgcagcc tcctggagcc ccagactcca tctgggggag ggacttgttt 240
   acaagcagtt ctgaccacct tagtggtgta ctgttttcta ggcaaaaaat atctgtctgt 300
   tgtactgtat agcctttaaa atgcagtcca ggaatgagac tcttttaaga aacacatcct 360
   gcttctgcaa ttccagagag tgctggggga aaaaaaggga taaaaattcc tacctactca 420
   tcagtgtttg aaagatggag ctgaatagct tttcttgttc ctggactagg c 471
<210> 331
   <211> 559
   <212> DNA
   <213> Homo sapiens
<220>
   <221> misc_feature
   <222> (56)..(59)
   <223> n is a, c, g, or t
   <220>
   <221> misc_feature
   <222> (61)..(66)
   <223> n is a, c, g, or t
   <220>
   <221> misc_feature
   <222> (68)..(69)
   <223> n is a, c, g, or t
   <220>
   <221> misc_feature
   <222> (88)..(88)
   <223> n is a, c, g, or t
<220>
   <221> misc_feature
   <222> (127)..(127)
   <223> n is a, c, g, or t
   <400> 331
   tgcacttgcc cacccaagag aaggagctcg gtgacatttg aggatgaagt ggaacnnnnc 60
   nnnnnngnng ccaagaactc gattcttnat gtgaaagctg aagtacacaa gtccttggac 120
   agttacncag caagcttggc caaagccatt gaggccgaag ccaaaatcaa cttatttggg 180
   gaggaggctt tgccaggggt cttggttaca gcacggactg tcccgggggg cggcttcggg 240
   ggccgccgag gcagcagaac tcttgtgagc cagaggctgc agttgcagag catcgaagaa 300
   ggagatgttt tagctgccga gcagagatga gggcctcagg gtgccgtggg gctgcagcct 360
   gagaggctgg cccggggagg agttcccatc accgcctgtg ccgcggcctt gggagcatgt 420
   cactgtgtac agctggccac acacagggaa ggagcagcat ctggtatgca gccaccagga 480
   caaggactga aaataatgtc tacagtccac agcttcagca tttccagaga ccacatgtga 540
   gcttctttta ggtcccagt 559
<210> 332
   <211> 115
   <212> DNA
   <213> Homo sapiens
   <400> 332
   tcccgacggg cagaggagcc tgggtcccga ggggacaagg agcctgggtt gcccccaccc 60
   cgctgaggga gttcctcttg ccccctaccc ccggggcttg tatatagatt ataaa 115
<210> 333
   <211> 486
   <212> DNA
   <213> Homo sapiens
<220>
   <221> misc_feature
   <222> (96)..(96)
   <223> n is a, c, g, or t
   <220>
   <221> misc_feature
   <222> (99)..(100)
   <223> n is a, c, g, or t
   <220>
   <221> misc_feature
   <222> (106)..(108)
   <223> n is a, c, g, or t
   <220>
   <221> misc_feature
   <222> (119)..(119)
   <223> n is a, c, g, or t
   <220>
   <221> misc_feature
   <222> (152)..(152)
   <223> n is a, c, g, or t
   <220>
   <221> misc_feature
   <222> (175)..(175)
   <223> n is a, c, g, or t
   <220>
   <221> misc_feature
   <222> (212)..(213)
   <223> n is a, c, g, or t
   <220>
   <221> misc_feature
   <222> (226)..(226)
   <223> n is a, c, g, or t
   <220>
   <221> misc_feature
   <222> (233)..(233)
   <223> n is a, c, g, or t
   <220>
   <221> misc_feature
   <222> (237)..(237)
   <223> n is a, c, g, or t
   <220>
   <221> misc_feature
   <222> (248)..(248)
   <223> n is a, c, g, or t
   <220>
   <221> misc_feature
   <222> (250)..(250)
   <223> n is a, c, g, or t
   <220>
   <221> misc_feature
   <222> (252)..(252)
   <223> n is a, c, g, or t
   <220>
   <221> misc_feature
   <222> (263)..(263)
   <223> n is a, c, g, or t
   <220>
   <221> misc_feature
   <222> (266)..(266)
   <223> n is a, c, g, or t
   <220>
   <221> misc_feature
   <222> (296)..(297)
   <223> n is a, c, g, or t
   <220>
   <221> misc_feature
   <222> (302)..(303)
   <223> n is a, c, g, or t
   <220>
   <221> misc_feature
   <222> (321)..(321) <223> n is a, c, g, or t
   <220>
   <221> misc_feature
   <222> (337)..(338)
   <223> n is a, c, g, or t
   <220>
   <221> misc_feature
   <222> (341)..(341)
   <223> n is a, c, g, or t
   <220>
   <221> misc_feature
   <222> (351)..(351)
   <223> n is a, c, g, or t
   <220>
   <221> misc_feature
   <222> (409)..(409)
   <223> n is a, c, g, or t
   <220>
   <221> misc_feature
   <222> (446)..(446)
   <223> n is a, c, g, or t
   <220>
   <221> misc_feature
   <222> (450)..(450)
   <223> n is a, c, g, or t
   <220>
   <221> misc_feature
   <222> (455)..(455)
   <223> n is a, c, g, or t
   <220>
   <221> misc_feature
   <222> (461)..(461)
   <223> n is a, c, g, or t
   <400> 333
   tgacctgctg tagaacatag ggatactgca ttctggaaat tactcaattt agtggcaggg     60
   tggtttttta attttcttct gtttctgatt tttgtngtnn ggggtnnntg tgtgtgttng    120
   tgtgtgtgtg tgtgtgtgtg tgtgtgtgtg tntaacagag aatatggcca gtgcnttgag    180
   ttctttctcc ttctctctct ctcttttttt tnnaaataac tcttcnggga agntggnttt    240
   ataagccntn tngccaggtg tanacntgtt gtgaaatacc caccactaaa gttttnnaag    300
   tnnccatatt ttctccattg ngccttctta tgtattnnca nagattattc ntgtgcactt    360
   taaatttact taacttacca taaatgcagt gtgacttttc ccacacagnt ggattgtgag    420
   gctcttaact tcttaaaagt ataggnggcn tcgtngtgaa ntcctataag cagtctttat    480
   gtctct                                                               486

<210> 334
   <211> 473
   <212> DNA
   <213> Homo sapiens
<220>
   <221> misc_feature
   <222> (191)..(192)
   <223> n is a, c, g, or t
   <400> 334
   ccaggccggg gctggaggga ttcggccgcg gcctccggtc ctgggcgctt cccttttaag 60
   caagggcgcc tcacctgctc ttcaagaaac agcgagaggg agacccaggg ggctgaaact 120
   tgaactctgg ttcttttaaa attaattttg gttggtgttg ggggaggcgc gagtgcgtgt 180
   gagaagaacc nncccacccc gcgcaagggg aagcctcctg tctccccttt ccccgcgtcc 240
   gagaaggcgg aaacccacag tgttacctga cttatgaaac ttgaaaccgc ctctggagcc 300
   gccattctgc agagtatttg gaaaaagaaa aaagggttta tgcttacgtc tctggggtcg 360
   gggggattat gtcacgagcg ttcaaactgc tggaaatctc aaaactgtac tgtctttatt 420
   tttgtatatt gtatttatat ataaaaagaa acgtctacgt atgcatgcta aat 473
<210> 335
   <211> 562
   <212> DNA
   <213> Homo sapiens
<220>
   <221> misc_feature
   <222> (241)..(243)
   <223> n is a, c, g, or t
   <220>
   <221> misc_feature
   <222> (247)..(247)
   <223> n is a, c, g, or t
   <220>
   <221> misc_feature
   <222> (251)..(253)
   <223> n is a, c, g, or t
   <220>
   <221> misc_feature
   <222> (256)..(256)
   <223> n is a, c, g, or t
   <220>
   <221> misc_feature
   <222> (259)..(259)
   <223> n is a, c, g, or t
   <220>
   <221> misc_feature
   <222> (261)..(264)
   <223> n is a, c, g, or t
   <220>
   <221> misc_feature
   <222> (339)..(348)
   <223> n is a, c, g, or t
   <220>
   <221> misc_feature
   <222> (352)..(353)
   <223> n is a, c, g, or t
<220>
   <221> misc_feature
   <222> (355)..(355)
   <223> n is a,c,g,or t
   <220>
   <221> misc_feature
   <222> (357)..(357)
   <223> n is a, c, g, or t
   <220>
   <221> misc_feature
   <222> (359)..(360)
   <223> n is a, c, g, or t
   <220>
   <221> misc_feature
   <222> (362)..(366)
   <223> n is a, c, g, or t
   <220>
   <221> misc_feature
   <222> (404)..(404)
   <223> n is a, c, g, or t
   <220>
   <221> misc_feature
   <222> (537)..(537)
   <223> n is a, c, g, or t
   <400> 335
   gaggcatgac ggattgcacc tgaatcctat ctgacgtttc attccagcaa gaggggctgg 60
   ggaagattac attttttttc ctttggaaac tgaatgccat aatctcgatc aaaccgatcc 120
   agaataccga agatcggcac aggacagaaa agcgagtcgc aggaggaagg gagatgcagc 180
   cgcacagggg atgattaccc tcctaggacc gcggtggcta agtcattgca ggaacggggc 240
   nnngttntct nnnggnacna nnnnggagct catctctttg gggtcacagt tctattttgt 300
   ttgtgagttt gtattattat tattattatt attattatnn nnnnnnnntt tnntntntnn 360
   gnnnnntgag caactcaaag aggcagaaga ggagaatgac tttnccagaa tagaagtgga 420
   gcagtgatca ttattctccg ctttctcttt ctaatcaaca cttgaaaagc aaagtgtctt 480
   ttcagccttt ccatctttac aaataaaact caaaaaagcc gtccagctta tcccatnctc 540
   tgattgtctt ctgacttaag gg 562
<210> 336
   <211> 189
   <212> DNA
   <213> Homo sapiens
<220>
   <221> misc_feature
   <222> (85)..(85)
   <223> n is a, c, g, or t
   <400> 336
   tctgacttcc atctgggggc tgagaccacc cttgcctgcc cccttctttc tgccttaaga 60
   atgtcctttt aggctgggca tggtnggctc acgcctgtaa ccccagcact ttgggaggcg 120
   gagacgggca gataacctga ggtcaggatt tcgagaccaa cctgacctac atggagaaac 180
   tccgcctct 189
<210> 337
   <211> 523
   <212> DNA
   <213> Homo sapiens
<220>
   <221> misc_feature
   <222> (38)..(38)
   <223> n is a, c, g, or t
   <220>
   <221> misc_feature
   <222> (47)..(47)
   <223> n is a, c, g, or t
   <220>
   <221> misc_feature
   <222> (74)..(74)
   <223> n is a, c, g, or t
   <220>
   <221> misc_feature
   <222> (86)..(86)
   <223> n is a, c, g, or t
   <220>
   <221> misc_feature
   <222> (109)..(109)
   <223> n is a, c, g, or t
   <220>
   <221> misc_feature
   <222> (434)..(434)
   <223> n is a, c, g, or t
   <220>
   <221> misc_feature
   <222> (456)..(456)
   <223> n is a, c, g, or t
   <400> 337
   tgaggagatt gccatggcga ccgtcacagc gctgcgcngc acagtgnccc ccgctgtcac 60
   tgggatcacc ttcntgtctg gaggcnagag tgaggaggag gcgtccatna acctcaatgc 120
   cattaacaag tgccccctgc tgaagccctg ggccctgacc ttctcctacg gccgagccct 180
   gcaggcctct gccctgaagg cctggggcgg gaagaaggag aacctgaagg ctgcgcagga 240
   ggagtatgtc aagcgagccc tggccaacag ccttgcctgt caaggaaagt acactccgag 300
   cggtcaggct ggggctgctg ccagcgagtc cctcttcgtc tctaaccacg cctattaagc 360
   ggaggtgttc ccaggctgcc cccaacactc caggccctgc cccctcccac tcttgaagag 420
   gaggccgcct cctnggggct ccaggctggc ttgccngcgc tctttcttcc ctcgtgacag 480
   tggtgtgtgg tgtcgtctgt gaatgctaag tccatcaccc ttt 523
<210> 338
   <211> 493
   <212> DNA
   <213> Homo sapiens
<220>
   <221> misc_feature
   <222> (161)..(161)
   <223> n is a, c, g, or t
   <220>
   <221> misc_feature
   <222> (163)..(163)
   <223> n is a, c, g, or t
   <220>
   <221> misc_feature
   <222> (165)..(176)
   <223> n is a, c, g, or t
   <400> 338
   tattgtcatc tgatatacac ataaaacaac tcacattgtt ggagttaact aattatcccc 60
   atttcatggt tttcagtggc aacttactga cccttgtttt tgcctgtgct tgtatgcatg 120
   cattttcaag caagtaataa agcagcctca tttaattctg nanannnnnn nnnnnnacat 180
   atagactgaa tgctataatc aaatctattg acagtatctg cagttctttc agaattccag 240
   ggcaaataat ataacgacct gatatctttc tacaggaata ttttcagaca ttatatagca 300
   cattactgat ttaatgcttt tacttttatc tttcaaaaca aattcactaa aaattaacag 360
   ctatgattct gaagtcacct ttctcaaacc ttgaaaatga gctctaggat ctctataaac 420
   atttctaaca cttttcctgt agttaccata gacagacatc tgtcgttaga cctgtgtggt 480
   atttcaaaga act 493
<210> 339
   <211> 463
   <212> DNA
   <213> Homo sapiens
<220>
   <221> misc_feature
   <222> (61)..(61)
   <223> n is a, c, g, or t
   <400> 339
   tttgcacttc cttcggagag catctaagat tggagaggtt gatgtcgagc aacatacttt 60
   ngccaaatac ctgatggaac taactatgtt ggactatgac atggtgcact ttcctccttc 120
   tcaaattgca gcaggagctt tttgcttagc actgaaaatt ctggataatg gtgaatggac 180
   accaactcta caacattacc tgtcatatac tgaagaatct cttcttccag ttatgcagca 240
   cctggctaag aatgtagtca tggtaaatca aggacttaca aagcacatga ctgtcaagaa 300
   caagtatgcc acatcgaagc atgctaagat cagcactcta ccacagctga attctgcact 360
   agttcaagat ttagccaagg ctgtggcaaa ggtgtaactt gtaaacttga gttggagtac 420
   tatatttaca aataaaattg gcaccatgtg ccatctgtac ata 463
<210> 340
   <211> 262
   <212> DNA
   <213> Homo sapiens
   <400> 340
   taagtgtgaa gaatgcgaga agagcttcaa acagcgctct gacctcttta aacaccacag 60
   aatccacact ggggagaagc cctatggatg ttccgtctgt gggaaacgct tcaatcagag 120
   tgcaaccctc attaaacacc agagaattca cactggggaa aagccttaca aatgtcttga 180
   atgtggggaa agatttagac aaagtacaca ccttatccga caccaaagaa ttcatcaaaa 240
   taaagtgctg tcggctgggc gt 262
<210> 341
   <211> 459
   <212> DNA
   <213> Homo sapiens
<220>
   <221> misc_feature
   <222> (181)..(181)
   <223> n is a, c, g, or t
   <220>
   <221> misc_feature
   <222> (287)..(287)
   <223> n is a, c, g, or t
   <220>
   <221> misc_feature
   <222> (316)..(319)
   <223> n is a, c, g, or t
   <220>
   <221> misc_feature
   <222> (324)..(325)
   <223> n is a, c, g, or t
   <220>
   <221> misc_feature
   <222> (328)..(330)
   <223> n is a, c, g, or t
   <220>
   <221> misc_feature
   <222> (362)..(362)
   <223> n is a, c, g, or t
   <220>
   <221> misc_feature
   <222> (375)..(375)
   <223> n is a, c, g, or t
   <220>
   <221> misc_feature
   <222> (381)..(381)
   <223> n is a, c, g, or t
   <220>
   <221> misc_feature
   <222> (386)..(386)
   <223> n is a, c, g, or t
   <220>
   <221> misc_feature
   <222> (397)..(397)
   <223> n is a, c, g, or t
   <220>
   <221> misc_feature
   <222> (403)..(403)
   <223> n is a, c, g, or t
   <220>
   <221> misc_feature
   <222> (408)..(408)
   <223> n is a, c, g, or t
   <220>
   <221> misc_feature
   <222> (411)..(411)
   <223> n is a, c, g, or t
   <220>
   <221> misc_feature
   <222> (418)..(418)
   <223> n is a, c, g, or t
   <220>
   <221> misc_feature
   <222> (420)..(420)
   <223> n is a, c, g, or t
   <220>
   <221> misc_feature
   <222> (430)..(430)
   <223> n is a, c, g, or t
   <400> 341
   tattcatgaa ttcttgcaca ttatgaagaa agagtccatg tggtcagtgt cttacccggt 60
   gtagggtaaa tgcacctgat agcaataact taagcacacc tttataatga ccctatatgg 120
   cagatgctcc tgaatgtgtg tttcgagcta gaaaatccgg gagtggccaa tcggagattc 180
   ngtttcttat ctataataga catctgagcc cctggcccat cccatgaaac ccaggctgta 240
   gagaggattg aggccttaag ttttgggtta aatgacagtt gccaggngtc gctcattagg 300
   gaaaggggtt aagtgnnnnt gctnnatnnn ctgcatgatg tttgcaggca gttgtggttt 360
   tnctgcccag cctgncacca ncgggnccat gcggatntgt tgntccancc naacaccncn 420
   ggaccatttn tgtatgtaag acaattctat ccagcccgc 459
<210> 342
   <211> 492
   <212> DNA
   <213> Homo sapiens
<220>
   <221> misc_feature
   <222> (254)..(254)
   <223> n is a, c, g, or t
   <220>
   <221> misc_feature
   <222> (315)..(315)
   <223> n is a, c, g, or t
   <220>
   <221> misc_feature
   <222> (388)..(388)
   <223> n is a, c, g, or t
   <400> 342
   tggggctgag caaggcctac gtaggccaga agagcagctt cacagtagac tgcagcaaag 60
   caggcaacaa catgctgctg gtgggggttc atggcccaag gaccccctgc gaggagatcc 120
   tggtgaagca cgtgggcagc cggctctaca gcgtgtccta cctgctcaag gacaaggggg 180
   agtacacact ggtggtcaaa tggggggacg agcacatccc aggcagcccc taccgcgttg 240
   tggtgccctg agtntggggc ccgtgccagc cggcagcccc caagcctgcc ccgctaccca 300
   agcagccccg ccctnttccc ctcaaccccg gcccaggccg ccctggccgc ccgcctgtca 360
   ctgcagccgc ccctgccctg tgccgtgntg cgctcacctg cctccccagc cagccgctga 420
   cctctcggct ttcacttggg cagagggagc catttggtgg cgctgcttgt cttctttggt 480
   tctgggaggg gt 492
<210> 343
   <211> 333
   <212> DNA
   <213> Homo sapiens
<220>
   <221> misc_feature
   <222> (274)..(274)
   <223> n is a, c, g, or t
   <220>
   <221> misc_feature
   <222> (299)..(299)
   <223> n is a, c, g, or t
   <400> 343
   gaagtcagct gggcattcaa agaagctaga ctgagaacgc ctgagaagaa ccagctacgg 60
   gaagagcttt gggaagcaaa ggcagaggcc ctggggtggg agcaggcttg ttttattgga 120
   aggaccagaa aactggtaag tgtgacccag atcaagtgtg aggagatgag gctggggata 180
   gtcaggggct ggatcaccca gggccttgtg ggccccacat agggttttgg gttttattct 240
   cagggcaatg ggaagctgtt ggatggtttg atgnaagggg agtgacagga tccgatgtnc 300
   ctatttaaga atttaagagg gtcgggtgcg gtg 333
<210> 344
   <211> 514
   <212> DNA
   <213> Homo sapiens
<220>
   <221> misc_feature
   <222> (41)..(41)
   <223> n is a, c, g, or t
   <220>
   <221> misc_feature
   <222> (43)..(43)
   <223> n is a, c, g, or t
   <220>
   <221> misc_feature
   <222> (68)..(68)
   <223> n is a, c, g, or t
   <220>
   <221> misc_feature
   <222> (91)..(91)
   <223> n is a, c, g, or t
   <220>
   <221> misc_feature
   <222> (97)..(97)
   <223> n is a, c, g, or t
   <220>
   <221> misc_feature
   <222> (103)..(103)
   <223> n is a, c, g, or t
   <220>
   <221> misc_feature
   <222> (109)..(109)
   <223> n is a, c, g, or t
   <220>
   <221> misc_feature
   <222> (133)..(133)
   <223> n is a, c, g, or t
   <220>
   <221> misc_feature
   <222> (138)..(138)
   <223> n is a, c, g, or t
   <220>
   <221> misc_feature
   <222> (150)..(150)
   <223> n is a, c, g, or t
   <220>
   <221> misc_feature
   <222> (158)..(158)
   <223> n is a, c, g, or t
   <220>
   <221> misc_feature
   <222> (170)..(170)
   <223> n is a, c, g, or t
   <220>
   <221> misc_feature
   <222> (316)..(316)
   <223> n is a, c, g, or t
   <220>
   <221> misc_feature
   <222> (411)..(411)
   <223> n is a, c, g, or t
   <220>
   <221> misc_feature
   <222> (481)..(481)
   <223> n is a, c, g, or t
   <400> 344
   gaaacgtttg caacatgatc aaggtgttag ttctccacca nanaagttgt attcttcttt 60
   tgccaccnca aaccatcaca gagtctttaa ntgcaantca atnggtcant gctagtcaaa 120
   gctatgttct tanaaaancc ccagacagcn tcagagcntc agaaaatccn tgtggagtgg 180
   ctgctctgta ccgtgggcat ccggcagcca ggaagtgaga caacataatt ataactttgt 240
   tttatgatgc tgcatcattt gtactgttta ggtcgacgtg aggacatcat cttatttaga 300
   attttccgtt tggcantctc ttttgggtgg gagttatgct gggggttgta aataatgaca 360
   aggctgagat ttttatgatg tttaaattgg gcacaatgat tttgacctta ntccccaaac 420
   ttcttttctt ttctactgtt taacatacac aggctattta tacacgtccc cagctcccat 480
   ntgaaacctg tgactcaggt ttatgaatgg tgtt 514
<210> 345
   <211> 387
   <212> DNA
   <213> Homo sapiens
<220>
   <221> misc_feature
   <222> (289)..(289)
   <223> n is a, c, g, or t
   <220>
   <221> misc_feature
   <222> (302)..(302)
   <223> n is a, c, g, or t
   <220>
   <221> misc_feature
   <222> (309)..(309)
   <223> n is a, c, g, or t
   <220>
   <221> misc_feature
   <222> (318)..(318)
   <223> n is a, c, g, or t
   <220>
   <221> misc_feature
   <222> (324)..(324)
   <223> n is a, c, g, or t
   <220>
   <221> misc_feature
   <222> (357)..(357)
   <223> n is a, c, g, or t
   <220>
   <221> misc_feature
   <222> (359)..(359)
   <223> n is a, c, g, or t
   <400> 345
   gagacgtagg tagccgtagt tggacggacg ggcagggccg gcggggcagc cccctccgcg 60
   cccccggccg tcccccctca tcgccccgcg cccaccccca tcgcccctgc ccccggcggc 120
   ggcctcgcgt gcgagggggc tcccttcacc tcggtgcctc agttccccca gctgtaagac 180
   agggacgggg cggcccagtg gctgagagga gccggctgtg gagccccgcc cgccccccac 240
   cctctaggtg gcccccgtcc gaggaggatc gttttctaag tgcaatacnt tggcccgccg 300
   gnttcccgnt gcccccantc gcgntcacgc aataaccggc ccggcccccg tccgcgngng 360
   tcccccggtg acctcgggga gcagcac 387
<210> 346
   <211> 550
   <212> DNA
   <213> Homo sapiens
   <400> 346
   ctccttgccc ctattgtgta gcagaaaccc cactttccct tggatattgg ggttaaccat 60
   cctgacagtg cagtgatctc tttctctgcc aatatttcaa cataaggagc cccagatggc 120
   acaagatcat cttccaattt aacagaccca taactatatt ccctggtgga agcagttcct 180
   cttggtcact agagatttcc aaacccacaa aacctaaggt ttcttggtta aaggccatgt 240
   ttgtgggata tgctgagatg aatatgctgt ggtttgaatg tgtcccccaa agttcatatg 300
   ttggaaactt gattcccatt gcaacagtgt tgagatctgg ggcccaatga gaggtgatta 360
   ggccatgagg gcggagtgaa tggattaatg cagttatctc aagagtgggt ttgttatgaa 420
   gggggtgttt ggtcctcttt tctctcttgc ccatgtgatt ccttccacca tgtttatgat 480
   gcaacaagaa ggtcctcacc agatgctggt tccttgatct tgtattttgc agcctccaaa 540
   atcgtgagcc 550
<210> 347
   <211> 535
   <212> DNA
   <213> Homo sapiens
<220>
   <221> misc_feature
   <222> (256)..(256)
   <223> n is a, c, g, or t
   <220>
   <221> misc_feature
   <222> (502)..(502)
   <223> n is a, c, g, or t
   <400> 347
   tagagatcat ctagtcccat caactcacta tatatatgag gaacctgagg tccagagtgg 60
   ggaagtgtct tacccaaggt cacatggttt cagagaaatt atgttgaatc caataagcct 120
   tcccggacat tccaagcctc ttaaccatgg catctatgtt gaggatgtca atgtttattt 180
   cagcaaagga cgtcatggct tttaaaaact ccttttaagc ctccttgttt tgatgtcacc 240
   ttggtaggct gggccntctg agaggttgga agctctaggc attgttctct ttggatccag 300
   ggatgctaag tagaaactgc atgagccacc agtgccccgg caccctttaa caccaccaga 360
   tgggtgtttt cccccatcca ccactggcag ggttgcccct tccctccaat catcactgtg 420
   ctcctttttt cccggcctac gaggcagctc ctgccactat ctttagagcc aataaagaga 480
   attaaaaacc tgtgcaccag gnagcatctt ttaaatacac tagccattct cttgc 535

<210> 348
   <211> 517
   <212> DNA
   <213> Homo sapiens
<220>
   <221> misc_feature
   <222> (210)..(210)
   <223> n is a, c, g, or t
   <220>
   <221> misc_feature
   <222> (481)..(481)
   <223> n is a, c, g, or t
   <400> 348
   ttcgctggat gcctcctgaa agcatcatgt accggaagtt cactacagag agtgatgtat 60
   ggagcttcgg ggtgatcctc tgggagatct tcacctatgg aaagcagcca tggttccaac 120
   tctcaaacac ggaggtcatt gagtgcatta cccaaggtcg tgttttggag cggccccgag 180
   tctgccccaa agaggtgtac gatgtcatgn tggggtgctg gcagagggaa ccacagcagc 240
   ggttgaacat caaggagatc tacaaaatcc tccatgcttt ggggaaggcc accccaatct 300
   acctggacat tcttggctag tggtggctgg tggtcatgaa ttcatactct gttgcctcct 360
   ctctccctgc ctcacatctc ccttccacct cacaactcct tccatccttg actgaagcga 420
   acatcttcat ataaactcaa gtgcctgcta cacatacaac actgaaaaaa ggaaaaaaaa 480
   naaagaaaaa aaaaccctgt aaggcagttt ggcaaat 517
<210> 349
   <211> 459
   <212> DNA
   <213> Homo sapiens
<220>
   <221> misc_feature
   <222> (83)..(83)
   <223> n is a, c, g, or t
   <220>
   <221> misc_feature
   <222> (298)..(298)
   <223> n is a, c, g, or t
   <400> 349
   ggacaaacag cctctgcaat acttgaggag cttgttagaa acccaaactc acagccccct 60
   ccagacctac gtacagaatg tanattagca agattcgcta ggtggtttat gtgcacgtta 120
   aagtttaaga agcactgcct gagaatccct tggtcctaat taattctttt ccacactcag 180
   atttgctaat gggtttcacc ttatctcttg actcttgttt gatggcaaca ggaaatagta 240
   gcatttcagg aagggtggaa aatataaaaa gcactcccaa cccaagcctc caaaaaanca 300
   gcaattttca ttttgtgtcc atatattccc ttctaatcat tgtcctcatg caagattttt 360
   tttcataaag atgatctgct acataatttt atatcatact ctttctccta acattacatc 420
   acaagtatac tttcatgttg ctgctacatt cttcacact 459
<210> 350
   <211> 485
   <212> DNA
   <213> Homo sapiens
<220>
   <221> misc_feature
   <222> (33)..(34)
   <223> n is a, c, g, or t
   <220>
   <221> misc_feature
   <222> (88)..(88)
   <223> n is a, c, g, or t
   <220>
   <221> misc_feature
   <222> (288)..(288)
   <223> n is a, c, g, or t
   <220>
   <221> misc_feature
   <222> (349)..(349)
   <223> n is a, c, g, or t
   <220>
   <221> misc_feature
   <222> (380)..(380)
   <223> n is a, c, g, or t
   <400> 350
   tttattctta ttcccgtatc tttgagagag gannagagtg ggattgctac ccacatttta 60
   atgaaggtgg agctgagccg tagaactntc tgggagccat ccaacctggc tgtggctcat 120
   aacaaggtat tgatcacttc ctttggcctg agtgagtcca gggtgcctag acaagaggta 180
   gcagcctgtg gatgtccagc acctttgcag ggaatacagg gcccaatctg gcacatgccc 240
   cttttcctcc aggcccagag caggggctgt tgccgaaagg ctgtgganca acaagttgac 300
   atctgacctg acatttgcct atgaacgttt gtcacacttc cgctgtgant tgctgaggta 360
   agcaagctgt ggggccttcn caaggcggag caggccagat ccagggctgg ggaacccctt 420
   agagagagga agacaataat taacaatagc taacacttac agaggcttat agtcagccct 480
   catcc 485
<210> 351
   <211> 553
   <212> DNA
   <213> Homo sapiens
   <400> 351
   agtgttcttc tctctggcaa agatttgtgt actgttgggg gaagattcat gttatttctc 60
   aggtagactt tactttttga gattctgtgt gtgtgtgtgt gtgtgtgtgt gtgtgtatgt 120
   gtgtgtgtgt gtgtattttg cctggtgggg ggttaaaggc agatagaatg tagttgttta 180
   tgagtttata ctttctcttt agcataatag atgccctgtt tattttctca gaatgtgaca 240
   ataaaattag gaaaggagag gaattcagag gcccatgttg cagttcatgg caaagtttta 300
   cccaaatatt tccttcagaa acatttagtc atagcaagcc atataaatta ttgtctgcaa 360
   ctggtatcag aaaaagaaat cagtaggtgg ggactgtaga ccccaatggt gcatctgttt 420
   acaatctttc ttttccaagg ttttaaggtt catgaataac atgagggaat tttgggagag 480
   ctaccacatc agtactttgg cacgcattaa ctgttccaca ggaaaactag ggttgcttca 540
   gggctatttt tgt 553
<210> 352
   <211> 447
   <212> DNA
   <213> Homo sapiens
<220>
   <221> misc_feature
   <222> (186)..(186)
   <223> n is a, c, g, or t
   <220>
   <221> misc_feature
   <222> (193)..(193)
   <223> n is a, c, g, or t
   <220>
   <221> misc_feature
   <222> (297)..(297)
   <223> n is a, c, g, or t
   <220>
   <221> misc_feature
   <222> (319)..(319)
   <223> n is a, c, g, or t
   <400> 352
   gccttttggg agtgcgtggt ggtggtctgg gtgtatggag ctgaccgctt cacggacgac 60
   attgcctgta tgatcgggta ccgaccttgc ccctggatga aatggtgctg gtccttcttc 120
   accccgctgg tttgcatggg catcttcatc ttcaacgttg tgtactacaa gccgctggtc 180
   tacaanaaca ccnacgtgta cccgtggtgg ggtgaggcca tgggctgggc cttcgtgctg 240
   tcctccatgc tgtgcatgcc actgcacctc ctgggctgcc tcctcagggc caagggnacc 300
   atggctgagt gctggaagna cctgacccag cccatctggg gcctccacca cttggagtac 360
   cgagctcagg atgcagatgt caggggcctg accaccctga ccccagtgtc cgagagcagc 420
   aaggtcgtcg tggtggagag tgtcatg 447
<210> 353
   <211> 538
   <212> DNA
   <213> Homo sapiens
   <400> 353
   gccagctttg ggctgagcta acaggaccaa tggattaaac tggcatttca gtccaaggaa 60
   gctcgaagca ggtttaggac caggtcccct tgagaggtca gaggggcctc tgtgggtgct 120
   gggtactcca gaggtgccac tggtggaagg gtcagcggag ccccagtgcc tccttgtgca 180
   tagaccttct tctcccaccc ccttctgccc ctgggtcccc ggccatccag cggggctgcc 240
   agagaacccc agacctgccc ttacagtagt gtagcgcccc ctccctcttt cggctggtgt 300
   agaatagcca gtagtgtagt gcggtgtgct tttacgtgat ggcgggtggg cagcgggcgg 360
   cgggctccgc gcagccgtct gtccttgatc tgcccgcggc ggcccgtgtt gtgttttgtg 420
   ctgtgtccac gcgctaaggc gaccccctcc cccgtactga cttctcctat aagcgcttct 480
   cttcgcatag tcacgtagct cccaccccac cctcttcctg tgtctcacgc aagtttta 538
<210> 354
   <211> 556
   <212> DNA
   <213> Homo sapiens
<220>
   <221> misc_feature
   <222> (27)..(27)
   <223> n is a, c, g, or t
   <220>
   <221> misc_feature
   <222> (74)..(74)
   <223> n is a, c, g, or t
   <220>
   <221> misc_feature
   <222> (91)..(91)
   <223> n is a, c, g, or t
   <220>
   <221> misc_feature
   <222> (100)..(100)
   <223> n is a, c, g, or t
   <220>
   <221> misc_feature
   <222> (109)..(109)
   <223> n is a, c, g, or t
   <220>
   <221> misc_feature
   <222> (112)..(112)
   <223> n is a, c, g, or t
   <220>
   <221> misc_feature
   <222> (121)..(121)
   <223> n is a, c, g, or t
   <220>
   <221> misc_feature
   <222> (138)..(138)
   <223> n is a, c, g, or t
   <220>
   <221> misc_feature
   <222> (155)..(155)
   <223> n is a, c, g, or t
   <220>
   <221> misc_feature
   <222> (162)..(162)
   <223> n is a, c, g, or t
   <220>
   <221> misc_feature
   <222> (169)..(169)
   <223> n is a, c, g, or t
   <220>
   <221> misc_feature
   <222> (181)..(182)
   <223> n is a, c, g, or t
   <220>
   <221> misc_feature
   <222> (184)..(184)
   <223> n is a, c, g, or t
   <220>
   <221> misc_feature
   <222> (186)..(186)
   <223> n is a, c, g, or t
   <400> 354
   gttgacgaca agttctacag caagctngat caagaggatg cgctcctggg ctcctaccct 60
   gtagatgacg gctnccgcat ccacgtcatt naccacagtn gcgcccgcnt tngtgagtat 120
   naggacgtgt cccgggtnga gaagtacacg atctncacaa gnaagcctna cgaccagagg 180
   nnangnacgg gcaggtgggc gtcgagggac acggtccgct ctttcctgaa gcgcagcaag 240
   ctcggccggt acaacgagga ggagcgggct cagcaggagg ccgaggccgc ccagcgcctg 300
   gccgaggaga aggcccaggc cagctccatc cccgtgggca gccgctgtga ggtgcgggcg 360
   gcgggacaat cccctcgccg gggcaccgtc atgtatgtag gtctcacaga tttcaagcct 420
   ggctactgga ttggtgtccg ctatgatgag ccactgggga aaaatgatgg cagtgtgaat 480
   gggaaacgct acttcgaatg ccaggccaag tatggcgcct ttgtcaagcc agcagtcgtg 540
   acggtggggg acttcc 556
<210> 355
   <211> 497
   <212> DNA
   <213> Homo sapiens
   <400> 355
   cgctctgcct cacggaaaga cagatcaaga tttggtttca gaaccggcgc atgaagtgga 60
   aaaacgagaa caagaccgcg gccccggcca ccaccggcca agacagggct gaagcagagg 120
   aggaagagga agagtgaccg atggagaaag ggcacaggaa gagacatgag aagggagacg 180
   aagacaagca gctctgggaa ctgaatcagg aaactcaaat cgaataggga actaaaaaac 240
   aaaacaaaaa acaaaaaaaa acaaaaaaaa accctattta aatgaaacga gtttaaaaac 300
   attttttaag gagggaggtt tggttttttt gtacaatatg aaaaggacat tatctacctg 360
   ttctgtagct ttctggaatt tacctcccct tttctatgtt gctattgtaa ggtctttgta 420
   aaatcttgca gttttgtaag ccctctttaa tgctgtcttt gtggactgtg ggtctggact 480
   aaccctgtgg ttgcctg 497
<210> 356
   <211> 533
   <212> DNA
   <213> Homo sapiens
   <400> 356
   attacaggct ctttaatcca tctggaaatg atttttgtat atggtgtgag gtgggaggac 60
   acaccatgct ccccattcgc tggatgcctc ctgaaagcat catgtaccgg aagttcacta 120
   cagagagtga tgtatggagc ttcggggtga tcctctggga gatcttcacc tatggaaagc 180
   agccatggtt ccaactctca aacacggagg tcattgagtg cattacccaa ggtcgtgttt 240
   tggagcggcc ccgagtctgc cccaaagagg tgtacgatgt catgctgggg tgctggcaga 300
   gggaaccaca gcagcggttg aacatcaagg agatctacaa aatcctccat gctttgggga 360
   aggccacccc aatctacctg gacattcttg gctagtggtg gctggtggtc atgaattcat 420
   actctgttgc ctcctctctc cctgcctcac atctcccttc cacctcacaa ctccttccat 480
   ccttgactga agcgaacatc ttcatataaa ctcaagtgcc tgctacacat aca 533
<210> 357
   <211> 534
   <212> DNA
   <213> Homo sapiens
   <400> 357
   gtatcatttt ctaggtaagg atgctaatct gtctccaagc caaataacac acagtaaatc 60
   atggcaccag gatttgaatc tgggtcttta tacatcatag cccatgctgt tctcactgta 120
   ttttgctttt tccaagtata accccgtttt cacacgaatg gccccttcac atatttgaag 180
   actaccgtcg tgtccgtgct gaccctttct ccctgccaca catggctgga gtgcaatggc 240
   gcgatctcgg ctcactgcaa cctctgtctc ccaggttcag gaaaatggct ttgtaaagaa 300
   gcttgagcct aaatctggct ggatgacttt tctagaagtt acaggaaaga tctgtgaaat 360
   gctcttctgt cctgaagcaa tactgttgac cagaaaggac actccatatt gtgaaaccgg 420
   cctaattttt ctgactctta cgaaaacgat tgccaacaca tacttctact tttaaataaa 480
   caactttgat gatgtaactt gaccttccag agttacagaa attttgtccc tatt 534
<210> 358
   <211> 260
   <212> DNA
   <213> Homo sapiens
   <400> 358
   cctgttccac tgacatttct tagacattca gcaaaacccc caccttaacc tcttttcttt 60
   cttgagggtt ggtcctgtcc ccacctccac cctcccaccc cctggaagag gaagggcccg 120
   ggcatcagtg gctagtccaa ataaaatatg ggcttgggga tggaatgggt ggtggtaagt 180
   tcacagagtg tagttagatc ccaactccca tgacctctgg cttcagtggt gggtggggca 240
   gggcagatga aagggcttca 260
<210> 359
   <211> 399
   <212> DNA
   <213> Homo sapiens
   <400> 359
   cgcccggacc agatacattc cgtgtacatc acgcccgggg cagacctgcc agtgcagggc 60
   gccctggagc ccctagaaga ggatggccag ccacctgggg ccaagcggag gtactcggat 120
   cccccaacgt actgcctgcc ccccgcctcg ggccagacca atggctgaga gccacagctg 180
   acaaagtctg catgtccgag gacggcccct gcactggagc tgggcgccag agctgcagag 240
   ctagtgttcg gccctcagag aaggatccag aatcaaaagc tcaagagtga cgtgaggtgg 300
   gcaccggccc caagtgcaga gtcaaggcag ggagaggccg gctggagcca ggccccctcg 360
   cacgcagccc ccaaatcatg gacgcacctg tggggagca 399
<210> 360
   <211> 458
   <212> DNA
   <213> Homo sapiens
   <400> 360
   ttcgctggat gcctcctgaa agcatcatgt accggaagtt cactacagag agtgatgtat 60
   ggagcttcgg ggtgatcctc tgggagatct tcacctatgg aaagcagcca tggttccaac 120
   tctcaaacac ggaggtcatt gagtgcatta cccaaggtcg tgttttggag cggccccgag 180
   tctgccccaa agaggtgtac gatgtcatgc tggggtgctg gcagagggaa ccacagcagc 240
   ggttgaacat caaggagatc tacaaaatcc tccatgcttt ggggaaggcc accccaatct 300
   acctggacat tcttggctag tggtggctgg tggtcatgaa ttcatactct gttgcctcct 360
   ctctccctgc ctcacatctc ccttccacct cacaactcct tccatccttg actgaagcga 420
   acatcttcat ataaactcaa gtgcctgcta cacataca 458
<210> 361
   <211> 518
   <212> DNA
   <213> Homo sapiens
   <400> 361
   gccaacgcta ccaaggtctg tgggtcagat ggagtcacat acggcaacga gtgtcagctg 60
   aagaccatcg cctgccgcca gggcctgcga ggggctatcg agaggagctc actgtgggat 120
   ggggttgacc tctgccgcct gcctgggtat ctgggcctgg ccatggctgt gttcttcatg 180
   tgttgatttt atttgacccc tggagtggtg ggtctcatct ttcccatctc gcctgagagc 240
   ggctgagggc tgcctcactg caaatcctcc ccacggcgtc agtgaaagtc gtccttgtct 300
   caggatgacc aggggccagc cagtgtctga ccaaggtcaa ggggcaggtg cagaggtggc 360
   agggatggct ccgaagccag aaatgcctta aactgcaacg tcccgtccct tccccacccc 420
   catcccatcc ccacccccag ccccagccca gtcctcctag gagcaggacc cgatgaagcg 480
   ggcggcggtg gggctgggtg ccgtgttact aactctag 518
<210> 362
   <211> 560
   <212> DNA
   <213> Homo sapiens
<220>
   <221> misc_feature
   <222> (76)..(76)
   <223> n is a, c, g, or t
   <220>
   <221> misc_feature
   <222> (153)..(153)
   <223> n is a, c, g, or t
   <220>
   <221> misc_feature
   <222> (236)..(236)
   <223> n is a, c, g, or t
   <220>
   <221> misc_feature
   <222> (238)..(238)
   <223> n is a, c, g, or t
   <220>
   <221> misc_feature
   <222> (245)..(245)
   <223> n is a, c, g, or t
   <220>
   <221> misc_feature
   <222> (249)..(249)
   <223> n is a, c, g, or t
   <220>
   <221> misc_feature
   <222> (426)..(426)
   <223> n is a, c, g, or t
   <220>
   <221> misc_feature
   <222> (446)..(446)
   <223> n is a, c, g, or t
   <220>
   <221> misc_feature
   <222> (451)..(451)
   <223> n is a, c, g, or t
   <220>
   <221> misc_feature
   <222> (487)..(487)
   <223> n is a, c, g, or t
<220>
   <221> misc_feature
   <222> (490)..(490)
   <223> n is a, c, g, or t
   <220>
   <221> misc_feature
   <222> (502)..(502)
   <223> n is a, c, g, or t
   <220>
   <221> misc_feature
   <222> (525)..(525)
   <223> n is a, c, g, or t
   <220>
   <221> misc_feature
   <222> (528)..(528)
   <223> n is a, c, g, or t
   <400> 362
   aggacctggt gacatgacat aaactccaag acagaacctc agtttacagc acacgaaaaa 60
   aatatcttgc caacantgta atgacaaaat aaattcccgt gaagttccac aaccaggccg 120
   ggcatggtgg ctcatgcctg taatcccagc acnttgggag gtgaggtggg tggatcatct 180
   gaggtcagga gttcaagacc agcctggcca acatggagaa actccgtctc tactananat 240
   acaanaatna accaggcttg gtggtgtatg cctgtaatcc cagctacttg ggaggctgag 300
   gcaggagaat cgcttgatcc caggaaggca gaggtggcag tgagctgaga acgcacaact 360
   gcactccagc ctgggtgacg agcaaaactc catctcaaaa caaaagttcc acaaccagcc 420
   tggagntgtg tagccccttt gtccanggaa nttgactagt caatcagtga cacctggtac 480
   tggcagnttn gggagtggca gnccaggatg gacagcagtg ggganggnac catttggcat 540
   aaggccgttg ggcttcagga 560
<210> 363
   <211> 390
   <212> DNA
   <213> Homo sapiens
   <400> 363
   aagaatcaga gctgctcctt cctgtgaatc ctaggtggcc ctatgtcttc tgtggagtta 60
   cagtataaag cagggagcta attaagagta ttaaaactta aaaccatttt ttgactctga 120
   ttttaagtac atttttatat gtcagttgct gcccttcaca ctaccaggcc ctgcagccac 180
   agtgttctgt tggagaaact tggggaagtg ttttctgaac cagttctttt tcttggggta 240
   gagcgtgaaa tccagacctg tttttgaaag gacagcacag gaggagaaaa gtgactggga 300
   cgatgcttcc tctcatccaa aacacatgca gagtcacatc ctcatcctag tgtttggcag 360
   tttgagaccg ctaccctgaa cttaagagct 390
<210> 364
   <211> 532
   <212> DNA
   <213> Homo sapiens
   <400> 364
   acccggtgtg ttcttgtata gtcagtggca tcagcacccg tcagccggcc ttttccttca 60
   ggttcgtcag gctcaccggt tctcactgtg tctgggaagt aggactgatg gtcatcttca 120
   tgacaggcgg catctccact aagcctgtgt aactgttccc tctttggttt tcttagcttt 180
   tgaatttgaa gaagtacttt tgaagactcc cattttaaga accgtgcaga ttttgctacc 240
   aaaagtcttc accactgtgt tcttaagtga atgttaattt ctgaggtttg ggactttgtg 300
   gtggtttttt tcttcttttc ttttccattc ttctttcttt ctttttatgt tgtttgctgt 360
   aaatgctgca catccagatt gcatatcagg acattggtta ttttatgctt tcttggatat 420
   aaccatgatc agagtgccat ggccactacc ccactgtttg ctctcctgca aatcaactgc 480
   ttttaattta cacttaaaca aattgttttg agtgttagct actgcctttc ta 532
<210> 365
   <211> 471
   <212> DNA
   <213> Homo sapiens
   <400> 365
   gcttctacgt catcttcgac agagcccaga agagggtggg cttcgcagcg agcccctgtg 60
   cagaaattgc aggtgctgca gtgtctgaaa tttccgggcc tttctcaaca gaggatgtag 120
   ccagcaactg tgtccccgct cagtctttga gcgagcccat tttgtggatt gtgtcctatg 180
   cgctcatgag cgtctgtgga gccatcctcc ttgtcttaat cgtcctgctg ctgctgccgt 240
   tccggtgtca gcgtcgcccc cgtgaccctg aggtcgtcaa tgatgagtcc tctctggtca 300
   gacatcgctg gaaatgaata gccaggcctg acctcaagca accatgaact cagctattaa 360
   gaaaatcaca tttccagggc agcagccggg atcgatggtg gcgctttctc ctgtgcccac 420
   ccgtcttcaa tctctgttct gctcccagat gccttctaga ttcactgtct t 471
<210> 366
   <211> 505
   <212> DNA
   <213> Homo sapiens
   <400> 366
   tggattgggg cagtctttgt gcgttggcat tggaggtgat ccttttaatg gaacagattt 60
   tattgactgc ctcgaaatct ttttgaacga ttctgccaca gaaggcatca tattgattgg 120
   tgaaattggt ggtaatgcag aagagaatgc tgcagaattt ttgaagcaac ataattcagg 180
   tccaaattcc aagcctgtag tgtccttcat tgctggttta actgctcctc ctgggagaag 240
   aatgggtcat gccggggcaa ttattgctgg aggaaaaggt ggagctaaag agaagatctc 300
   tgcccttcag agtgcaggag ttgtggtcag tatgtctcct gcacagctgg gaaccacgat 360
   ctacaaggaa tttgaaaaga ggaagatgct atgaaagaaa aaaaaaattc ctaaaactgt 420
   ggaatggatc acgtagacat gtaacccagc agcagtttgc ttctgttgtc cactgattaa 480
   tcagcctatg tgcctgacac tggtc 505
<210> 367
   <211> 312
   <212> DNA
   <213> Homo sapiens
   <400> 367
   gtgggagcac gaacgaggtg ggagttctgt ccccccatgc ctggccctaa agtctcttgc 60
   acaccagctc gtcactgcct gccctaccca cctctgtcca gtctacacac ccagcccagg 120
   cttaactcat gccaactcca ccctacatgg ctgccctgtg ccctcgggat aaaccccaag 180
   cccctgagct tgtgtttaaa gccgttggcc ttgctccccc agctttgtca gctcaggtct 240
   gtctacaccc agatggtagc gcttgtgaca ctggcctggc agtcctgctc acagtgttct 300
   gtgcctgtgt gc 312
<210> 368
   <211> 501
   <212> DNA
   <213> Homo sapiens
   <400> 368
   gtgtccgaag ttgagatggc ctgccctact ggcaaagagg tgacaggaag gctgggagca 60
   gctttgttaa attgtgttca gttctgttac acagtgcatt gccctttgtt gggggtatgc 120
   atgtatgaac acacatgctt gtcggaacgc tttctcggcg tttgtccctt ggctctcatc 180
   tcccccattc ctgtgcctac tttgcctgag ttcttctacc cccgcagttg ccagccagat 240
   tgggagtctg tttgttccaa tgggttgagc tgtctttgtc gtggagatct ggaactttgc 300
   acatgtcact actggggagg tgttcctgct ctagcttcca cgatgaggcg ccctctttac 360
   ctatcctctc aatcactact cttcttgaag cactattatt tattcttccg ctgtctgcct 420
   gcagcagtac tactgtcaac atagtgtaaa tggttctcaa aagcttacca gtgtggactt 480
   ggtgttagcc acgctgttta c 501

<210> 369
   <211> 569
   <212> DNA
   <213> Homo sapiens
   <400> 369
   cctgcgtgtt gagtgtgtgg gcggcagtgc tttccggagg cctggtccat ctggagtttt 60
   gaggggtgag gggaccagag cagtgggacc agcatgggga tcagcttccc ttccccacct 120
   gggagccagg gactgtccgg gtagccagtt ttggtcctgc cagctgcctc cctgatccct 180
   ccccactctc gccccttctc tatgaactta aaatcaaaaa accacttccc tcccatctcc 240
   tccctgctcc tgcgtggagg gggaatgtgt gctggccagg gtggaggact gagcacctga 300
   gcctggggct ggctccccgg ggtccccgac tcagctggtg gctgtggagc tgagtcccct 360
   ccccgtaacc tctgcaaggc cagcacccac catcactacc tgcacctgct gtggtcccac 420
   cctctggagg cctgggaacc tggctgcagc ctgggaaggc tggagaggca gacggtggga 480
   cccaccagct ctctccccat cccgcttctt ccctggggcc aggccctacc tgtgtggtgg 540
   tgggtgggct gtcaagacgt gtcatgtac 569
<210> 370
   <211> 459
   <212> DNA
   <213> Homo sapiens
   <400> 370
   cagcatcgac gcacgcgaga tcttcgatct gattcgctcc atcaatgacc cggagcatcc 60
   actgacgcta gaggagttga acgtagtaga gcaggtgcgg gttcaggtta gcgaccccga 120
   gagtacagtg gctgtggctt tcacaccaac cattccgcac tgcagcatgg ccacccttat 180
   tggtctgtcc atcaaggtca agcttctgcg ctcccttcct cagcgtttca agatggacgt 240
   gcacattact ccggggaccc atgcctcaga gcatgcagtg aacaagcaac ttgcagataa 300
   ggagcgggtg gcagctgccc tggagaacac ccacctcttg gaggttgtga atcagtgcct 360
   gtcagcccgc tcctgagcct ggcctttgac ccctcagcct gcatactggt atcctggtcc 420
   cagctcctgc cagggctgtt accgttgttt tcttgaatc 459
<210> 371
   <211> 333
   <212> DNA
   <213> Homo sapiens
   <400> 371
   tgcagccctg tctacactgg ataatcattc cctggccata tcagtcatca tgatggttgt 60
   ggctggcttc ttcaccctct gtgccgtgct ctcagtcttc ctcctgcagc gggtgcactc 120
   cctctaccga cggacagggg ccagcttcca gcaggcccag gaggagtttt cccagggcat 180
   cttcagcagc agaaccttcc acagagctgc ttcatctgct gcccaaggag ccttccaggg 240
   gaattagtcc tcctctcttc tctccccctc agcctttctc tcgcctgcct tctgagctgc 300
   actttccgtg ggtgccttat gtggtggtgg ttg 333
<210> 372
   <211> 422
   <212> DNA
   <213> Homo sapiens
   <400> 372
   gcgtgttctc ctacgtgaag gtggcagcca gctccctgct gcatggcggg ggccggccgg 60
   cattgctggc agccggcgtg gccatccagg tgggctctct gctcggcgct gttgctatgt 120
   tccccccgac cagcatctat cacgtgttcc acagcagaaa ggactgtgca gacccctgtg 180
   actcctgagc ctgggcaggt ggggaccccg ctccccaaca cctgtctttc cctcaatgct 240
   gccaccatgc ctgagtgcct gcagcccagg aggcccgcac accggtacac tcgtggacac 300
   ctacacactc cataggagat cctggctttc cagggtgggc aagggcaagg agcaggcttg 360
   gagccaggga ccagtggggg ctgtagggta agcccctgag cctgggacct acatgtggtt 420
   tg 422
<210> 373
   <211> 439
   <212> DNA
   <213> Homo sapiens
   <400> 373
   tctgactcta gatgggacac ttgacagtga cttgaaacat ttgcatattc aggaatgcat 60
   gagatttcaa gagagcctac agtatgaaat cattttcaca aaataagcag cttgcttctg 120
   aaatgctgtc tttcccagta gctactcacc tgcctctggt ggctgggatt cagatgccac 180
   aaaactgtca gtatctatag accaggtctg tgccacctcc tctctcctct gtgctcagtg 240
   aggaggcagt aaatgaagtt acaggctagc acaataccta actcatgttt cccagtacac 300
   ctgtagatat tactgtactt ttatgttctc aagaaataag ttgttgccta ttcagtgtta 360
   cagatttctt tgtttctttt taattaaaat acaagaagca gctgaggaaa gggagacaag 420
   gtattttatt tctgactga 439
<210> 374
   <211> 453
   <212> DNA
   <213> Homo sapiens
   <400> 374
   aggctcaggc ccatgaggta tggagacacc ctggccccca ggagctggag gcaccgccca 60
   ctcccctggc attccagctt tgcaggtgac cctcctctac ccaaagctct gtccccctgc 120
   tcccactcca gaagaactgc ggcacgtgct tcgggcagcc tagccacagg ctttgagcgc 180
   ctgcattcct gggggctgga gggtggggtg ccaaaggccc tgagcaaaag ccagagctcc 240
   tctcatcaaa gcctttacaa ggtactgggc ccagaggctt tgccttgaca gagtggccca 300
   gggtttcaag ggaggaggaa cctcccccta cctaggaccc ttcctgtggg gggtctacag 360
   agtcagggac agaagggaag ggacccacag gaagtcacag tggtgcccag ggatgtgtca 420
   gcccccagcc acggggacgc gggattcaag aat 453
<210> 375
   <211> 488
   <212> DNA
   <213> Homo sapiens
   <400> 375
   ttaatcccat gcatgccaaa cacttttcac acctaccgac ccattctcct tctgcttctc 60
   ttgccctctt cttcacacca aaatatgatc gtgtccctgc cgcagaatat gtatttccta 120
   attgctgtgg ccaagcgcct gtgtgccgaa tcgcttgctt ctgatcccgc tccgtgtaac 180
   ctaagtgcgc tgcaggcaaa gcccaggcca cggctgcgtc actactgatg ttcacgatgc 240
   cacacagtca cacacctaat tcattctcaa gtcggagcaa cacataccaa ccttgacctt 300
   atcctcaagc tccagggcag cctggccgag cagcccctgc tccctcctgg agacccttgt 360
   cacctcccga gctcctcctg gagacccctg tcacctcctg accaaccttt cccagggcgg 420
   caccgatcac cgagcagccg tgcgtgtatc tcaaggaact aaataagatg acgctactcc 480
   tcatagca 488
<210> 376
   <211> 485
   <212> DNA
   <213> Homo sapiens
   <400> 376
   gacttgccta gatctttgtt gtatcttggg gacttttact ttgttgtttg atgcttaaac 60
   ttcaaaattc tctgtattca aatttgattg tggcgaatct acttcaaaaa ggaaaaataa 120
   tccaactttg tggatattaa atggaaggtt tgctgttttg atctagttgt ttccagtgga 180
   gcagttttat gaaatatgtt ctataagatg tacatttttt cattgtaaca tagaaattgt 240
   aaataattga ttaaagtgct gcattttgat gaattttttc tagccatttt taaagagaaa 300
   actaggaatt gagtattttg tgtacggtat gtttccatcc tccctcccct tcctcctccc 360
   ctcctctctc tctcttccta cctatttaat tttcatttgt catgaggttt ttggatttgc 420
   caatgatctg ctggacatca tgccccatgt catagagaat aaagctgatg attgtaccag 480
   tctta 485
<210> 377
   <211> 569
   <212> DNA
   <213> Homo sapiens
   <400> 377
   ggaaccatgg acacagtttc tctcagtggg actattccag ttcaaatgct tttggaaatt 60
   ggtttggaca aactaaagaa agattatatc agttttttca taggtcagga acttgcatct 120
   ttgaatcatt tggaatactt cattgctcca tcagtagata tacaagaaca ggtttatcgt 180
   gtccaaaaac tccaccatat tctagaaata ttagtcagtt gcatgccttt cattaaatct 240
   caacatgaac tcctcttttc tttaacacag atctgcataa agtattacaa acaaaatcct 300
   cttgatgagc aacacatttt tcagctgcca gtcagaccaa ctgctgtaaa gaacttatat 360
   caaagtgaga agccacagaa atggagagtg gaaatatata gtggtcaaaa gaagattaag 420
   acagtttggc aactgagtga cagctcaccc ataggccatc tgaattttca caaacctgat 480
   ttttcggaat taacactaag cggtagcctg gaagaaagga tattctttac taacatggtt 540
   acctgcagcc aggtgcattt caagtgaag 569
<210> 378
   <211> 336
   <212> DNA
   <213> Homo sapiens
   <400> 378
   tcctggttcc ctgagggtcc tcagggtgga ggacaggttt ggcccagaaa gactagccag 60
   aggcctgatg gtcccaggtg gctctggata tactttggat atggatttaa atggtctcta 120
   agagccgggg gtagggggca ggaaaagtgg gttgtctttg cccctcaaag tccacctacc 180
   tagaaaccaa gcccacggtc ttggccgtga ccctgataat aaatgggctc tctcagaggc 240
   gccagcccct ccctccccag ccggaggcgt catctctctt ctgtaccact agagggagct 300
   ctgatgcagc tggagagcag cgctcaaggc tctcgc 336
<210> 379
   <211> 525
   <212> DNA
   <213> Homo sapiens
   <400> 379
   agaccatcca acggcgacta aatgagattg aggctgcctt gagggagcta gaggccgagg 60
   gcgtgaagct ggagctggcc ttgaggcgcc agagcagttc cccagaacag caaaagaaac 120
   tatgggtagg acagctgcta cagctcgttg acaagaaaaa cagcctggtg gctgaggagg 180
   ccgagctcat gatcacggtg caggaattga atctggagga gaaacagtgg cagctggacc 240
   aggagctacg aggctacatg aaccgggaag aaaacctaaa gacagctgct gatcggcagg 300
   ctgaggacca ggtcctgagg aagctggtgg atttggtcaa ccagagagat gccctcatcc 360
   gcttccagga ggagcgcagg ctcagcgagc tggccttggg gacaggggcc cagggctaga 420
   cgagggtggg ccgtctgctt tcgttcccac aaagaaagca cctcacccca gcacagtgcc 480
   acccctgttc atctgggctg cctggcagag agccttgctg tttac 525
<210> 380
   <211> 525
   <212> DNA
   <213> Homo sapiens
   <400> 380
   cccggtgtgg ccacgagtcg ggttgcactg ctgtgatcca tcctcatctc ctaaagatgc 60
   atcctgactt atctccacac ttgcacactg aagaatgcaa cgtcttgatt aacttgctta 120
   aggaatgtca caaaaatcac aacattctga aattttttgg ttattgtaat gatgttgatc 180
   gggagttgag aaaatgcctg aagaatgagt acgtagaaaa caggaccaag agcagggagc 240
   atggcattgc aatgcgaaag aaacttttta atcctccaga ggaatccgaa aaataaattg 300
   tattttcact cgatgccttg gctgagagaa gacctaaaga ctctgggttg atacctgaaa 360
   gaatcctgtc ttatttggtc tccataatcc tttgaatgga aagtgacctg tgagagattg 420
   aaccatggag aaatatgaaa accctggatt ctgagtattt gttgggcagg gcgtttagta 480
   ctgtctcccc tttaccagca aacctgactt caccatgttt attcc 525
<210> 381
   <211> 520
   <212> DNA
   <213> Homo sapiens
   <400> 381
   aaggatctta actgtgttcg cattttttat ccaagcactt agaaaaccta caatcctaat 60
   tttgatgtcc attgttaaga ggtggtgata gatactattt ttttttcata ttgtatagcg 120
   gttattagaa aagttgggga ttttcttgat ctttattgct gcttaccatt gaaacttaac 180
   ccagctgtgt tccccaactc tgttctgcgc acgaaacagt atctgtttga ggcataatct 240
   taagtggcca cacacaatgt tttctcttat gttatctggc agtaactgta acttgaatta 300
   cattagcaca ttctgcttag ctaaaattgt taaaataaac tttaataaac ccatgtagcc 360
   ctctcatttg attgacagta ttttagttat ttttggcatt cttaaagctg ggcaatgtaa 420
   tgatcagatc tttgtttgtc tgaacaggta tttttataca tgctttttgt aaaccaaaaa 480
   cttttaaatt tcttcaggtt ttctaacatg cttaccactg 520
<210> 382
   <211> 261
   <212> DNA
   <213> Homo sapiens
   <400> 382
   actcatctgg cttcagcaga ttgccaccaa gaggatacag gtggtcaggt cctggctggc 60
   tttgtctttg ggcctgggca ggcttaggat ttgactttct ttgaagtacc tgatgctgat 120
   tgattccact aatagtagga agcaagagac ttaactatga gggacgttat gtgaatctta 180
   agtcttacca gtccttgcat tagtacatta aatttggatg ttttggaagc aaattcatac 240
   gatcgtgagt gatttctcca a 261
<210> 383
   <211> 424
   <212> DNA
   <213> Homo sapiens
   <400> 383
   caacacagac tacaggttcc gcgtatgtgc gtgtcgtcgc tgtttagaca cctctcagga 60
   gctaagcgga gccttcagcc cctctgcggc ttttgtatta caacgaagtg aggtcatgct 120
   tacaggggac atggggagct tagatgatcc caaaatgaag agcatgatgc ctactgatga 180
   acagtttgca gccatcattg tgcttggctt tgcaactttg tccattttat ttgcctttat 240
   attacagtac ttcttaatga agtaaaccca acaaaactag aggtatgaat taatgctaca 300
   cattttaata cacacattta ttcagatact ccccttttta aagccctttt gttttttgat 360
   ttatatactc tgttttacag atttagctag aaaaaaaatg tcagtgtttt ggtgcacctt 420
   tttg 424
<210> 384
   <211> 460
   <212> DNA
   <213> Homo sapiens
   <400> 384
   gcagcactct taacttacga tctcttgaca tacggtttct ggctgagagg cctggcccgc 60
   taaggtgaaa aggggtgtgg caaaggagcc tactccaaga atggaggctg taggaatata 120
   acctcccacc ctgcaaaggg aatctcttgc ctgctccatc tcataggcta agtcagctga 180
   atcccgatag tactaggtcc ccttccctcc gcatcccgtc agctggaaaa ggcctgtggc 240
   ccagaggctt ctccaaaggg agggtgacat gctggctttt gtgcccaagc tcaccagccc 300
   tgcgccacct cactgcagta gtgcaccatc tcactgcagt agcacgccct cctgggccgt 360
   ctggcctgtg gctaatggag gtgacggcac tcccatgtgc tgactccccc catccctgcc 420
   acgctgtggc cctgcctggc tagtccctgc ctgaataaag 460
<210> 385
   <211> 434
   <212> DNA
   <213> Homo sapiens
   <400> 385
   ttgttttcga gaacccagat ccctctgatg gttttgtcct catccctgac ctcaagtgga 60
   accaacagca gctcgatgac ttgtacttga tcgccatctg ccatcgccgg ggcatcagat 120
   ccctacgcga ccttactccg gagcacttgc cgctgctcag gaacatcctc caccaggggc 180
   aggaggccat cctgcagcgc taccggatga agggagacca tctgcgagta tacctgcact 240
   acctgccctc ctactaccac ctgcatgtgc acttcaccgc cctgggcttc gaggcccccg 300
   gctcaggcgt ggagcgggcc cacctgctgg ctgaggtgat cgagaacttg gagtgtgacc 360
   ctaggcacta ccagcagcgc acgctcacct tcgccctcag ggctgacgac cccctgctca 420
   agctcttgca ggag 434
<210> 386
   <211> 416
   <212> DNA
   <213> Homo sapiens
   <400> 386
   tgctggctgg ccatttactt ccagccctta tgaggagttt cccctgctga agagccctgc 60
   ctgccccaga tcataccccc ttcctgcctg taacccttac cggctccata tggggtacaa 120
   aggtctggcc tcctcacccc aacttgggaa accctctggg gccatcccag ctccagagcc 180
   ccttgtgggg tcagtgagac ctcattgtgg ccacattaca gcccagtgcc tctccctgac 240
   aagcctgtac ccagccggct cagcccacag cactgtccta tgaaccttcc tgcacgccat 300
   tctccacctc agtatctgct ttcggggaac ccaacctgcg acagtgcttc tgtgtgtttt 360
   cagtcctgca ggtttgaact ctgactttgg agacttttcc agttatctcg tggaat 416
<210> 387
   <211> 477
   <212> DNA
   <213> Homo sapiens
   <400> 387
   aattcctgtg catgttctat aatctgacac cctgaaagca agtttccttt cgtcattcac 60
   atgctcttgt tctgccgtga ctgttcaggt gtatggtagt aagtaaatgt attaacatgg 120
   tgaacagtag taatattcta tcatagagta ttagcccttg caagttttca gggcgtcttt 180
   tccgacttca gtttttgtga taaagaatgt gaacagttgt tagatgttct cagtgattca 240
   actttaaaac aaatttctcg tgatgattca tttcaaaatc ctgagtgagt ctgactgaaa 300
   aatacgagag aaaagagagt ggtttccgtt tgcagctaca cagctgtgtg catcgacgtt 360
   ctcctggggt gtgtgccaag cgaaacccag gggtgaattg gattcttgaa gagaccaaag 420
   cctgtaactg tccagcttct aatttcaaaa cgggtccatt agggcttcgt tgtgtta 477
<210> 388
   <211> 548
   <212> DNA
   <213> Homo sapiens
   <400> 388
   gactagtaaa ttgtctgcct cctatagcag aaaggtgaat gtacaaactg ttggtggccc 60
   tgaatccatc tgaccagctg ctggtatctg ccaggactgg cagttctgat ttagttagga 120
   gagagccgct gataggttag gtctcatttg gagtgttggt ggaaaggaaa ctgaaggtaa 180
   ttgaatagaa tacgcctgca tttaccagcc ccagcaacac aaagaatttt taatcacacg 240
   gatctcaaat tcacaaatgt taacatggat aagtgatcat ggtgtgcgag tggtcaattg 300
   agtagtacag tggaaactgt taaatgcata acctaatttt cctgggactg ccatattttc 360
   ttttaactgg aaatttttat gtgagttttc cttttggtgc atggaactgt ggttgccaag 420
   gtatttaaaa gggctttcct gcctccttct ctttgattta tttaatttga tttgggctat 480
   aaaatatcat ttttcaggtt tattctttta gcaggtgtag ttaaacgacc tccactgaac 540
   tgggtttg 548
<210> 389
   <211> 492
   <212> DNA
   <213> Homo sapiens
   <400> 389
   tgtatggttt tcacctggac accgtgtaga atgcttgatt acttgtactc ttcttatgct 60
   aatatgctct gggctggaga aatgaaatcc tcaagccatc aggatttgct atttaagtgg 120
   cttgacaact gggccaccaa agaacttgaa cttcaccttt taggatttga gctgttctgg 180
   aacacattgc tgcactttgg aaagtcaaaa tcaagtgcca gtggcgccct ttccatagag 240
   aatttgccca gctttgcttt aaaagatgtc ttgtttttta tatacacata atcaataggt 300
   ccaatctgct ctcaaggcct tggtcctggt gggattcctt caccaattac tttaattaaa 360
   aatggctgca actgtaagaa cccttgtctg atatatttgc aactatgctc ccatttacaa 420
   atgtaccttc taatgctcag ttgccaggtt ccaatgcaaa ggtggcgtgg actccctttg 480
   tgtgggtggg gt 492
<210> 390
   <211> 354
   <212> DNA
   <213> Homo sapiens
   <400> 390
   gaatcatttc attcactttg ggagaggcct ataattacat ttatttgcaa tgtttctctt 60
   cgctagattg ttacatagct cccattctgt tggttttgct tacagcatat ggtaaccaag 120
   gttagatgcc agttaaaatt ccttagaaat tggatgagcc ttgagattgc ttcttaactg 180
   ggacatgaca tttttctagc tcttatcaag aataacaact tccacttttt tttaaactgc 240
   acttttgact ttttttatgg tataaaaaca ataatttata aacataaaag ctcattgtgt 300
   tttttagact tttgatatta tttgatactg tacaaacttt attaaatcaa gatg 354
<210> 391
   <211> 537
   <212> DNA
   <213> Homo sapiens
   <400> 391
   gagccctaga tgttcctgga agttggcccc ctttatgaaa accacttccc acagccagtg 60
   ggaactgcca gaggaagatc tggcgtcaca tggctcccag gaaagtgctg tgccctatcc 120
   ccactgatac catctgattc cccgatgcct gtgcctgttc cacctggacg gtggccccct 180
   cagcctggca gcctctggac agagaggaag gaaggattgg aaaagtcccc gcagcacagc 240
   gacggtggga agatgcctta cgtctgatct tgatgggggc actggcctgg agcctgggcc 300
   cacctgcttc tggggggttg gggagcaggc cagatggagg tggtggtgcc aggaagaaat 360
   ggagcgatga ctgactgtgg ggtgggccca ggatttccac atcttggtga agttgcccct 420
   gggaagggca gctgggggca gtggcgccag ttcccttcca tggtctcccg gctggcaatg 480
   tggtgaagct gagtttctgt ccaatgagca ggaagattct gagacatttc gcctgag 537
<210> 392
   <211> 258
   <212> DNA
   <213> Homo sapiens
   <400> 392
   tggaccccga gctgttgagg tacttgctgg gacggattct tgcgggaagc gcggactccg 60
   agggggtggc agccccgcgc cgcctccgcc gtgccgccga ccacgatgtg ggctctgagc 120
   tgccccctga gggcgtgctg ggggcgctgc tgcgtgtgaa acgcctagag accccggcgc 180
   cccaggtgcc tgcacgccgc ctcttgccac cctgagcact gcccggatcc cgtgcaccct 240
   gggacccaga agtgcccc 258
<210> 393
   <211> 513
   <212> DNA
   <213> Homo sapiens
   <400> 393
   ttccataggc cgatgctctg aaagaagaga cgtggggctc gagagattta aagattttat 60
   ttttacaaat cacagctgat agacagcgaa gccttcccca tagagaccgt gctccaactc 120
   gggcctgggg cactgctcgc tgctcccagg aagggggtgg cgtgacaggc aggaacctgc 180
   gaagtccaga gtccagggtg gagcgcacca gcctcagcca gagcagccac gacagccaca 240
   gtgtgtgcac tcgatgatgc ggccctgcaa cggaggagga cagtgagacg atgccactgc 300
   gccacgctcg cccctgcaca ctcacatatg tggcaaccct cccacgaagg acctgccacc 360
   atgccatata gggacacacc tcagaaaccc ttccttgaca gctctggaca gggaaaattt 420
   ggctccctca tgaaggtaga accagctgct gttgacaccg aggttacatc tgtatgtcta 480
   tttataatat gttctgcaaa tccaacacac gtt 513
<210> 394
   <211> 402
   <212> DNA
   <213> Homo sapiens
   <400> 394
   gcacctcgga gttgcagctg tgacactcat aggttactcc caggagtgtg ctgagcagaa 60
   ggcaagctct tgctggatga aacccctcca ggtggggttg gggagacttg atattcacat 120
   ccaacagttt gaaaagggag agctcaattc ccagcgtcac cccatggctt gtgttgcctg 180
   ctacgcattg acttggatct ccaggagtcc cctgcacata ccttctccat cgtgtcagct 240
   gtgtttctct tgattccgtg acacccggtt tattagttca aaagtgtgac accttttctg 300
   ggcaaggaac agccccttta aggagcaaat cacttctgtc acagttatta tggtaatatg 360
   aggcaatctg attagcttca cagactgagt ctccacaaca cc 402
<210> 395
   <211> 518
   <212> DNA
   <213> Homo sapiens
   <400> 395
   ggcggcgcca gcgggaatta aatcgcagaa agtaccaggc actaggtcgg cgctgccggg 60
   agatcgagca ggtgaacgag cgggtcctga acaggctcca tcaggtgcag aggataactc 120
   ggaggctgca gcaggaacgg aggttcctca tgagagtgct ggactcctac ggggatgact 180
   accgggccag ccagttcacc attgtgctgg aggatgaggg cagccagggc acggatgccc 240
   ccaccccagg caatgcggag aatgagcctc cagagaaaga gacactgtcc ccgcccagaa 300
   ggactcctgc acccccagaa cccggcagcc cagcccccgg tgaggggccc agtgggcgga 360
   agaggcggcg agtgccacgg gatggacgcc gagcaggaaa tgcgctgact ccagagctgg 420
   ccccggtgca gattaaggtt gaggaagact ttggctttga agcagatgag gccctggatt 480
   ccagttgggt ttctcggggt ccagacaaac tgctgccc 518
<210> 396
   <211> 444
   <212> DNA
   <213> Homo sapiens
   <400> 396
   cgacttccga aggtcaccgg gagcgggttc tcagcctctc ccaagccctg gctactgagg 60
   cgtcgcagtg gcacagaatg atgacaggtg gaaatttgga ctcccaggga gaccctcttc 120
   ccggtgtgcc gctgcctcct tcggacccca cgcgccagga gacccctccc cccagatctc 180
   ccccggtggc taattcgggt tccacggggt tctctcgccg agggagtggg cgtggaggag 240
   gtcccacccc ctgggggccc gcgtgggatg ccgggatcgc ccctccggtc ctgccacaag 300
   acgagggggc atggcctctg cgagtcactc tgctacaatc cagcttgtaa tccgcccaaa 360
   agcggcagcc aatcggagcg cgaggacgtg gtctggaggt accgccgaag atctgggacc 420
   actcagggca tcagggggcg tggt 444
<210> 397
   <211> 414
   <212> DNA
   <213> Homo sapiens
   <400> 397
   ggtcctcctg gtgagtcatt ggagctatga aggggaaggg gtcgtatcac tttgtctctc 60
   ctacccccac tgccccgagt gtcgggcagc gatgtacata tggaggtggg gtggacaggg 120
   tgctgtgccc cttcagaggg agtgcagggc ttggggtggg cctagtcctg ctcctagggc 180
   tgtgaatgtt ttcagggtgg ggggagggag atggagcctc ctgtgtgttt ggggggaagg 240
   gtgggtgggg cctcccactt ggccccgggg ttcagtggta ttttatactt gccttcttcc 300
   tgtacagggc tgggaaaggc tgtgtgaggg gagagaaggg agagggtggg cctgctgtgg 360
   acaatggcat actctcttcc agccctagga ggagggctcc taacagtgta actt 414
<210> 398
   <211> 480
   <212> DNA
   <213> Homo sapiens
   <400> 398
   tcaagctgga agatacctct ctggccccgg cacatgtcac ccctgcactc ctgccttccc 60
   gtgggcactt ccacatcctc tgggcctctg gcagttccca gggactgttt tcacctctgc 120
   tgtctctggg gtcagctgct gctcatcagc tgcccgctag catgtggcca ggggtgcagg 180
   gtggcggggg gtcagcagca tgtccctggg caggccctgg gcaccctgtc tcccctggtc 240
   tcactgctga cctgggctgg tcccagcctg gattggcctc atccaggatc tttggtcacc 300
   ccacgctgcc ccatcttgcc tgctgttcca gttctggtca agggccttgg gggctggccc 360
   cccaccaggc cttctagagc agcaccagtc tcagggccct gggaccagct gccctacttc 420
   ccaggtttgt agccaggaga agggggcatc acagagctga tggtccaata aggggggtgt 480
<210> 399
   <211> 533
   <212> DNA
   <213> Homo sapiens
   <400> 399
   aggtgaagcg aagccactct tacctctccc ttcccctccc acctgccccc tgcgtaggca 60
   cccagacttg gagagacccg tctgctgtta atacttccat cctcttcctt cccaaagagc 120
   agatcccaag gcatttactc cttgggtctg tctcgcttta tctgtcgccc ctcccagcgc 180
   tgagagcctc ccctggctgt cagcagcact gtgttccagg ctcttgtctg aacaccgcag 240
   cccctccttc gctccttcca gagctcagca tgtcacagca aggactgccg cattggtgat 300
   ggagggccag ctgaggggaa gttgctggtg agtttccttt tctccatttc tagcatatgg 360
   acacctggcc tctgcttgag cacttaggtg acaggaactt ccgcacctcc tgaggccctg 420
   gatgattcta attgttagaa attctaattg ttagaaatcc ttccttataa tgaatgaatt 480
   ctgctttcct ataatttcta cctattgggc cttgttctgt tctctggaac taa 533
<210> 400
   <211> 509
   <212> DNA
   <213> Homo sapiens
   <400> 400
   cgctttgagc tgcgcgagga cgggcgcccc gagctgcccc cgcaggccca cggtctcggc 60
   gtagacggtg cctgcaggcc ctgcagcgac gctgagctgc tcctggccgc atgcaccagc 120
   gacttcgtaa ttcacgggat catccatggg gtcacccatg acgtggagct gcaggagtct 180
   gtcatcactg tggtggccgc ccgtgtcctc cgccagacac cgccgctgtt ccaggcgggg 240
   cgatccgggg accaggggct gacctccatt cgtaccccac tgcgctgtgg cgtccacccg 300
   ggcccaggca ccttcctctt catgggctgg agccgctttg ggaggccccg gctgggctgt 360
   gccccacgat tccaggagtt ccgccgtgcc tacgaggctg cccgtgctgc ccacctccac 420
   ccctgcgagg tggcgctgca ctgaggggct gggtgctggg gaggggctgg taggagggag 480
   ggtgggccca ctgctttgga ggtgatggg 509
<210> 401
   <211> 481
   <212> DNA
   <213> Homo sapiens
   <400> 401
   cagtggcttc cagcagagtt gacctgggat ttctgtcatg ggtgtccctc tgtactcaga 60
   atgggttcag gccaagtcgg tgaagatgga tgttggcaaa ataggaggat accctcattt 120
   gctgaatggg ggacctgctc tgagcctgcc caggggccag gcctgctcca ggttaaactg 180
   gacggaaggc ccaggtctca gtttctttca accaggagag gccgctgcct agagcccctc 240
   cccacctttt cctggatggg tgaggcaagc caggagagca agcagtgttg tcctcacggg 300
   aggaggactg agcgactggg aaaactcggc tctacatctc acccagaacg gcttttagaa 360
   acaccacagc tggagagtcc tggctgagcc ttgggagttt cagctctttg gcggggtgcc 420
   caggtgccat gcgatcagcg aagcctgcga gttggcagga ctctgaggtt tcctgcagac 480
   c 481
<210> 402
   <211> 481
   <212> DNA
   <213> Homo sapiens
   <400> 402
   cagtggcttc cagcagagtt gacctgggat ttctgtcatg ggtgtccctc tgtactcaga 60
   atgggttcag gccaagtcgg tgaagatgga tgttggcaaa ataggaggat accctcattt 120
   gctgaatggg ggacctgctc tgagcctgcc caggggccag gcctgctcca ggttaaactg 180
   gacggaaggc ccaggtctca gtttctttca accaggagag gccgctgcct agagcccctc 240
   cccacctttt cctggatggg tgaggcaagc caggagagca agcagtgttg tcctcacggg 300
   aggaggactg agcgactggg aaaactcggc tctacatctc acccagaacg gcttttagaa 360
   acaccacagc tggagagtcc tggctgagcc ttgggagttt cagctctttg gcggggtgcc 420
   caggtgccat gcgatcagcg aagcctgcga gttggcagga ctctgaggtt tcctgcagac 480
   c 481
<210> 403
   <211> 534
   <212> DNA
   <213> Homo sapiens
   <400> 403
   agcatactat gcagcgttgg gaactaggcc acctattaat atggaagaac tggatgaatc 60
   ataccagaaa gtaattgaac tcttctctgt atgcactaat gaagacccta aagatcgtcc 120
   ttctgctgca cacattgttg aagctctgga aacagatgtc tagtgatcat ctcagctgaa 180
   gtgtggcttg cgtaaataac tgtttattcc aaaatattta catagttact atcagtagtt 240
   attagactct aaaattggca tatttgagga ccatagtttc ttgttaacat atggataact 300
   atttctaata tgaaatatgc ttatattggc tataagcact tggaattgta ctgggttttc 360
   tgtaaagttt tagaaactag ctacataagt actttgatac tgctcatgct gacttaaaac 420
   actagcagta aaacgctgta aactgtaaca ttaaattgaa tgaccattac ttttattaat 480
   gatctttctt aaatattcta tattttaatg gatctactga cattagcact ttgt 534

<210> 404
   <211> 213
   <212> DNA
   <213> Homo sapiens
   <400> 404
   cgctggacgt ggccagcgac agccagtcgg agatgcagga gaagcacccc agcctgaacg 60
   gcggcggggc cctcaacggc ccggggagct ggggggcgct catggggggc aagcgggacc 120
   ccgaggactc ggacgtgttc gaggaggaca cgcacctgtg agcgcagcga ggcgcaggcc 180
   gagtgggccg ccaggaccaa gcgaggtgga ccc 213
<210> 405
   <211> 406
   <212> DNA
   <213> Homo sapiens
   <400> 405
   ccccagtgtc cgagctggat cgtgcggacg cctggctcct ccgaaaagcg cacgagacag 60
   ccttcctctc ctggttccgc aatggcctcc tggcatcggg catcggggtc atctccttca 120
   tgcagagtga catgggtcgg gaagcagcat atggcttctt cctgctgggc ggcctgtgcg 180
   tggtgtgggg cagcgcctcg tacgccgtgg gcctggcggc gctgcgagga cccatgcagc 240
   tgacgctggg gggcgcggcc gtgggcgcgg gcgccgtgct ggccgccagc ctgctctggg 300
   cgtgcgccgt gggcctctac atggggcagc tggagctgga cgtggagctg gtgcccgagg 360
   acgacgggac ggcctccgcg gaaggccctg atgaggcggg tcggcc 406
<210> 406
   <211> 432
   <212> DNA
   <213> Homo sapiens
   <400> 406
   ttggctgttc cagcaggtgg ggcgctggcc tcggtgaggg cacagcagca aggttcacgg 60
   atatccgtgt gtcttgtctg tggccaccag gcacaggttt ggcttccggt cagtgtcccg 120
   acactgtgcg ggaggtgaca acagagcaaa gcagcgcagg ggtcagggag gtggagacac 180
   tgctgaaatc acactacccc accctcagct gaagccccac gttccacaaa cttggggtca 240
   tagattgtcc agtcactggc tccctccctg tcagcacagc acagaggaag gggctaactg 300
   aatcttttac cacttctggc ctggctccag aactttgttc tagattcctt aaaagtcggt 360
   agctgatgtc aaactcaatt gagcagtagc tttgatccct tggtctgggg gtcgaaggaa 420
   gatggcgctg tt 432
<210> 407
   <211> 472
   <212> DNA
   <213> Homo sapiens
   <400> 407
   gggaggaccg gctaatactg tgaattcttg tgtcatcgtt tggggtttta cttgatacca 60
   ctagctataa gcctaatctc ataatgtatt tcttttttga aactgatttg tttagcattt 120
   tgttttcaga agagccattc tttattaagt tttcatagaa aataatgtta aggtagattt 180
   agtttgaatg ttttttcata tgaaaaagag gcttttattc ttttccatag tttagacatc 240
   actggcgtct tctgagtttt atgagacagg aaactaagtt tactatctgt aaatgtaaac 300
   atatgtccat taagaaacat gtagtttttt tttagaatgt aataacccag tggcttactg 360
   tttttcttaa tctcttttaa aaaaacttta gaagaatctt ttaggaacta atatctcttg 420
   ttctgaagaa acatttatct gacgttcagc agttcctaca gttttacttc ag 472
<210> 408
   <211> 519
   <212> DNA
   <213> Homo sapiens
   <400> 408
   gctgtggttg tggagttcag ggacctgtgg cggatccgga gcccctgtgg tgactgcgaa 60
   ggcttcgacg tgcacatcat ggacgacatg attaagcgtg ccctggactt cagggagagc 120
   agggaagctg agccccaccc gctgtgggag tacccatgcc gcagcctctc cgagccctgg 180
   cagatcctga cctttgactt ccagcagccg gtgcccctgc agcccctgtg tgccgagggc 240
   accgtggagc tcagaaggcc cgggcagagc cacgcagcgg tgctatggat ggagtaccac 300
   ctgaccccgg agtgcacgct cagcactggc ctcctggagc ctgcagaccc cgaggggggc 360
   tgctgctgga acccccactg caagcaggcc gtctacttct tcagccctgc cccagatccc 420
   agagcactgc tgggtggccc acggactgtc agctatgcag tggagtttca ccccgacaca 480
   ggcgacatca tcatggagtt caggcatgca gatacccca 519
<210> 409
   <211> 469
   <212> DNA
   <213> Homo sapiens
   <400> 409
   aggttgcaag aacattcctc tactttctgc taagccttgg aaacagttgg gaaaagtagt 60
   ttgaccctca cagttcacat tcagctcagc agagcaagac cccagagatg cttagagaca 120
   ggacacctgg ccatcaaacc cagtttggcc cagcctggtt gggtgacttt gtgggagcca 180
   cttaacagct ctgggtccct gttttaccat cctgggagca aggccctgca gctccacgag 240
   acctttaccc cgggaagaag ccgccaccca tgaaagcatt tctgaagccc ctttctaaga 300
   caaggctcag catcttgata tttttgacag attcctccca agtctggctc tgggaggtat 360
   gtacccatct caaatgttcc caagataaat tcatccttca ggaaatggaa atgaacttgc 420
   ttactaatgt gtgattccta gttgtagcca ccggatgtgc tgaggccta 469
<210> 410
   <211> 495
   <212> DNA
   <213> Homo sapiens
   <400> 410
   gtccagtccc agaccaatgg aggccccagc cccacaccca aggcccaccc gccgcggagc 60
   cccccgcccc ggccgcagcg cagctgctct ctggacctgg gagatgccgg gtgctacggt 120
   tatgccaggc gcctgggagg agcttgggcc cgacggagcc actctgtgca tggggggctg 180
   ctcggggcag ggtgccgggg ggtaggaggc agcgccgagc ggctggaaga gagtgtggtg 240
   tgatggacgg gcagcttcct gtgtgctcca agggatgagc ctcgtggggc agagggcccg 300
   gggccgccgc ctggcctggg agtccctccc tggtttttat tctcagtacc tcaggctccc 360
   ctgtgtactt ggaggggcag ggagcccttt cctcggttct ggcctccaga ccagggtaag 420
   ggcaggcccc tccaacaggt gctcacagcc accgaggcag gggctgcagc cacccactgg 480
   gagtcttgtt tttat 495
<210> 411
   <211> 349
   <212> DNA
   <213> Homo sapiens
   <400> 411
   aaacttgcgt ttgagccgtt gagctaattc tgcaattttc taccaaacag agcgctggtg 60
   gccccggagc agggctgtga cattggctgg tggagcaccc ttcctgtgtt ctccctttgt 120
   tccagcgccg cgatggtgag atcactgttc caagcagggg gacggctcgc gataggacaa 180
   agagagcagg acctccagac tctggggacc ctgcagacct tgacaatttg cctgactcat 240
   tcctgacctc ttgtcatttt ggcctgaagg ctacaaattc agggtcagct gtatgcacta 300
   agtcaaataa tgaatttctt cctccctctc gcaaccgacc aaaattttg 349
<210> 412
   <211> 562
   <212> DNA
   <213> Homo sapiens
   <400> 412
   tcccggctac atgggagcgc ggtgtgagtt cccagtgcac cccgacggcg caagcgcctt 60
   gcccgcggcc ccgccgggcc tcaggcccgg ggaccctcag cgctaccttt tgcctccggc 120
   tctgggactg ctcgtggccg cgggcgtggc cggcgctgcg ctcttgctgg tccacgtgcg 180
   ccgccgtggc cactcccagg atgctgggtc tcgcttgctg gctgggaccc cggagccgtc 240
   agtccacgca ctcccggatg cactcaacaa cctaaggacg caggagggtt ccggggatgg 300
   tccgagctcg tccgtagatt ggaatcgccc tgaagatgta gaccctcaag ggatttatgt 360
   catatctgct ccttccatct acgctcggga ggtagcgacg ccccttttcc ccccgctaca    420
   cactgggcgc gctgggcaga ggcagcacct gctttttccc tacccttcct cgattctgtc    480
   cgtgaaatga attgggtaga gtctctggaa ggttttaagc ccattttcag ttctaactta 540
   ctttcatcct attttgcatc cc 562
<210> 413
   <211> 458
   <212> DNA
   <213> Homo sapiens
   <400> 413
   aacaatcctg aaggcctggg attttttgtc tgaaaatcaa ctgcagactg taaatttccg 60
   acagagaaag gaatctgtag ttcagcactt gatccatctg tgtgaggaaa agcgtgcaag 120
   tatcagtgat gctgccctgt tagacatcat ttatatgcaa tttcatcagc accagaaagt 180
   ttgggatgtt tttcagatga gtaaaggacc aggtgaagat gttgaccttt ttgatatgaa 240
   acaatttaaa aattcgttca agaaaattct tcagagagca ttaaaaaatg tgacagtcag 300
   cttcagagaa actgaggaga atgcagtctg gattcgaatt gcctggggaa cacagtacac 360
   aaagccaaac cagtacaaac ctacctacgt ggtgtactac tcccagactc cgtacgcctt 420
   cacgtcctcc tccatgctga ggcgcaatac accgcttc 458
<210> 414
   <211> 560
   <212> DNA
   <213> Homo sapiens
   <400> 414
   agtatcccat tggttctggt cgtgtgactt tcaataacca acggagttac ctgaaagcag 60
   tcagcgctgc ttttgtggag atcaaaacca ccaagttcac aaagaaggtt cagattgacc 120
   cctacctgga agattctctg tgtcatatct gcagttctca gcctggtcct ttcttctgtc 180
   gagatcaggt ctgcttcaaa tacttctgcc ggagctgctg gcactggcgg cacagcatgg 240
   agggcctgcg ccaccacagc cccctgatgc ggaaccagaa gaaccgagat tccagctaga 300
   ggagctggcc ttgcccagtg gcctgtggcg cccaaagctg gcaggtcagg caagcagcct 360
   gcaccaccct gccactggcg accagggagc tggcttccca aggacaaggg aaaattgtag 420
   tcacctttgc acttgctgaa tctgtctttg tttctgcact aattaatgca cattgagttt 480
   tgtcaggttt tgttttcagg gggtgtacca agggcaagga ccctctggct taccctccaa 540
   gcgactctgt agttttccca 560
<210> 415
   <211> 443
   <212> DNA
   <213> Homo sapiens
   <400> 415
   agaagtacaa catctccttc cacaagcggt acggcaccaa gatcatcaaa cgccagcgga 60
   agaacgccac ccaggaggcc ctgcgcaaag gggacgatgt caaattcgag gagtttgtgg 120
   cctatctcat cgacccacac acccagcggg aggagccttt caacgaacac tggcaaaccg 180
   tctactcact ctgccatccc tgccacatcc actatgacct cgtgggcaag tacgagacac 240
   tggaagagga ttctaattac gtcctgcagc tggcaggagt gggcagctac ctgaagttcc 300
   ccacctatgc aaagtctacg agaactactg atgaaatgac cacagaattc ttccagaaca 360
   tcagctcaga gcaccaaacg cagctgtacg aagtctacaa actcgatttt ttaatgttca 420
   attactcagt gccaagctac ctg 443
<210> 416
   <211> 357
   <212> DNA
   <213> Homo sapiens
   <400> 416
   gatcttcttg gccatgaaaa ccatgagata cagccgtatg tgaatggagc tctgtacagc 60
   atcctttctg ttccatccat tcgtgaggaa gcaagagcaa tgggaatgga agacatccta 120
   cgctgcttca tcaaagaagg caatgctgaa atgatccgcc agatagaatt catcatcaag 180
   cagctaaatt ccgaagagct accagatggt gttcttgaat ctgatgatga tgaagatgaa 240
   gatgatgaag aggaccatga catcatggaa gccgatctgg acaaagacga actgatccag 300
   ccccagcttg gagaactctc aggagagaag cttctgacca cagagtacct ggggatc 357
<210> 417
   <211> 487
   <212> DNA
   <213> Homo sapiens
   <400> 417
   aacttattga agagcgtcgc caacaattcc ttgcagacaa acaacgtgaa ctagaagagt 60
   ggcagttgca gcaaaggcgg caaggattta ttaatgcaat tattgaagaa gaaaggctaa 120
   aacttcttaa agagcatgct acaaacttac taggctatct ccctaaagga gtatttaaaa 180
   aagaggatga tattgatctg cttggtgaag agttcaggaa agtatatcaa caaaggagtg 240
   aaatttgtga agagaaatga tatcatcaaa attgggtaaa gcatagattt tttgtatgtt 300
   accactagat gtcagcataa cttttgtttt acagctcagt ggcattaggt atccattgtc 360
   tgtttggatt ttgtaaatca tcactgaatt tcataacttg taaacaatta tcagatacaa 420
   attaatttta atcaagctgt tatttttgta ctgataattt caaaatccga tttctacaac 480
   actacag 487
<210> 418
   <211> 523
   <212> DNA
   <213> Homo sapiens
   <400> 418
   gaatcggaca tgtccaaacc accgtgttac gaagaggcgg tgctgatggc agagccgccg 60
   ccgccctata gcgaggtgct cacggacacg cgcggcctct accgcaagat cgtcacgccc 120
   ttcctgagtc gccgcgacag cgcggagaag caggagcagc cgcctcccag ctacaagccg 180
   ctcttcctgg accggggcta cacctcggcg ctgcacctgc ccagcgcccc tcggcccgcg 240
   ccgccctgcc cagccctctg cctgcaggcc gaccgtggcc gccgggtctt ccccagctgg 300
   accgactcag agctcagcag ccgcgagccc ctggagcacg gagcttggcg tctgccggtc 360
   tccatcccct tgttcgggag gactacagcc gtatagaggg gcgcccggcg ccccgggccc 420
   caccggcgga ctcctggcct gactgcgggg ctttttaaat gcttccctgg actgcgggga 480
   ggggcggggg gagggaggga tttcttatcc cgtttgttac att 523
<210> 419
   <211> 506
   <212> DNA
   <213> Homo sapiens
   <400> 419
   taatacccaa ctgactaact aaacaaatat caacttgtaa tactcaatga atttttttgc 60
   catttacatt tgaccgttgg ctttagtgaa tgtccatatt taatttttta aggcaccatt 120
   acacagttta tcctacattt atcacatttc ttaaagtgtt aagattctat ggctcatttc 180
   tatgtatttt tcttacttta caaaataacc tgaaacagta tagattttgt aacacttaat 240
   ttgagcagct tttttattac attgaattat ataaagtgca tgttacctta gaaaaattag 300
   tatttgctgc tttactcttt tgcaaaacat ttgctgtaat gaatggattt gtatttccaa 360
   tatgtatctt gactgcattt tgtaatattt actgctttat tcctaattct gctttaaagt 420
   actgaactgg gcatgaaaca ttaaaatatt aatccagaaa ctgtataaac tggatgttgc 480
   ttaaaatctg tatcactgcc atgttg 506
<210> 420
   <211> 504
   <212> DNA
   <213> Homo sapiens
   <400> 420
   actgcggcct ctgggatgga gagcaataca tcatcatctt tggagaattt agcgacggcg 60
   cctgtgaacc agatccaaga aacaatttct gataattgtg tggtgatttt ctcaaaaaca 120
   tcctgttctt actgtacaat ggcaaaaaag cttttccatg acatgaatgt taactataaa 180
   gtggtggaac tggacctgct tgaatatgga aaccagttcc aagatgctct ttacaaaatg 240
   actggtgaaa gaactgttcc aagaatattt gtcaatggta cttttattgg aggtgcaact 300
   gacactcata ggcttcacaa agaaggaaaa ttgctcccac tagttcatca gtgttattta 360
   aaaaaaagta agaggaaaga atttcagtga tgtttatact aataagtttg ctagtacagt 420
   gtcagttatt taaagtggta atgcccgata atgtctttta aatgtttgag gatgttttaa 480
   atacatgcat tgtcttcacg aaga 504
<210> 421
   <211> 472
   <212> DNA
   <213> Homo sapiens
   <400> 421
   gactttgatt ggtagcatcc acgccctccc tgggctcata agccagacca tcaggcagca 60
   gcagagagat ttcattgagg ctcagatgga gagctacgac aagcacgtca cttacaatgc 120
   tgagcggtcc cggtcctcgt ccaggaggcg gcggtcctct tccacagcac caccaacttc 180
   atcagagagt agctagaaga gaataagtta accacaaaat aagacttttt gccatcatat 240
   ggtcaatatt ttagctttta ttgtaaagcc cctatggttc taatcagcgt tatccgggtt 300
   ctgatgtcag aatcctggga acctgaacac taagttttag gccaaaatga gtgaaaactc 360
   tttttttttc tttcagatgc acagggaatg cacctattat tgctatatag attgttcctc 420
   ctgtaatttc actaactttt tattcatgca cttcaaacaa actttactac ta 472
<210> 422
   <211> 475
   <212> DNA
   <213> Homo sapiens
   <400> 422
   atatggccat cgtgtcagca gagagagtct ctgtacacag ccccgtgaac cctgaggagt 60
   ggagtcatac acgaagggcg tgtggccatc gtgtcagcag agagagtctc tgtacacagc 120
   cccgtgaacc ctgaggagtg gagtcatacg cgaagggtgt gtggccaggc tgcagagctg 180
   cgtgccgttt gtgtccgagc atcacgtgtg gctccagccc ttatttctgc cagtgtagac 240
   acctctgtct gccccactgt cctggggtcg ctcttgggag gcacaggcat gggtgtgtct 300
   ggcctcattc tgtatcagtc cagtgtgttc ctgtcatagt ttgtgtctcc caggcaggcc 360
   atggtagggg cctcgcaggg gccattgggg agcacagggc caggctgggg tgaggagagc 420
   tcccctgttt tctgtttaat tgatgagcct gggaaaggag tgtgttctgc ctgcc 475
<210> 423
   <211> 485
   <212> DNA
   <213> Homo sapiens
   <400> 423
   actcacatcc agtccgtttg taaaatacac ccaggatgag acctgcacgc aagtggctca 60
   cagcagcacg atttgtgaca gcccgaggcg gagaacaccg aacacccagt gaaggtgagg 120
   ggatcagcac ggcgcggcca cccacgcacc cacgcgctgg aatgagactc agccacaagg 180
   aggtgcgaag ctctgaccca ggccacagtg cggatgcacc ttgaggatgt cacgctcagt 240
   gagagacacc agacacagaa gggtacgctg tgatcccact tctatgaaat gtccaggaca 300
   gaccaatcca cagaatcagg gagaggattc gtgggtgccg ggactgggga gggggacctg 360
   ggggtgacta ggtgacataa tggggacagg gctgccttct gggtgatgag aatgttctgg 420
   aatcagatgg gatggctgca cggcgtggtg aaggtactga acgccacctc actgtaagac 480
   ggtag 485
<210> 424
   <211> 538
   <212> DNA
   <213> Homo sapiens
   <400> 424
   ttgtggagaa cctggacagc ctgcccccca aagttccaca gcgggaggcc tccctgggtc 60
   ccccgggagc ctccctgtct cagaccggtc taagcaagcg gctggaaatg caccactcct 120
   cttcctacgg ggttgactat aagaggagct accccacgaa ctcgctcacg agaagccacc 180
   aggccaccac tctcaaaaga aacaacacta actcctccaa ttcctctcac ctctccagaa 240
   accagagctt tggcagggga gacaacccgc cgcccgcccc gcagagggtg gactccatcc 300
   aggtgcacag ctcccagcca tctggccagg ccgtgactgt ctcgaggcag cccagcctca 360
   acgcctacaa ctcactgaca aggtcggggc tgaagcgtac gccctcgcta aagccggacg 420
   taccccccaa accatccttt gctccccttt ccacatccat gaagcccaat gatgcgtgta 480
   cataatccca gggggagggg gtcaggtgtc gaaccagcag gcaaggcgag gtgcccgc 538
<210> 425
   <211> 381
   <212> DNA
   <213> Homo sapiens
   <400> 425
   caaacggaac ttgccgcgtc gaggactgtc gggctacagc atgctggcca tagggattgg 60
   aaccctgatc tacgggcact ggagcataat gaagtggaac cgtgagcgca ggcgcctaca 120
   aatcgaggac ttcgaggctc gcatcgcgct gttgccactg ttacaggcag aaaccgaccg 180
   gaggaccttg cagatgcttc gggagaacct ggaggaggag gccatcatca tgaaggacgt 240
   gcccgactgg aaggtggggg agtctgtgtt ccacacaacc cgctgggtgc cccccttgat 300
   cggggagctg tacgggctgc gcaccacaga ggaggctctc catgccagcc acggcttcat 360
   gtggtacacg taggccctgt g 381
<210> 426
   <211> 457
   <212> DNA
   <213> Homo sapiens
   <400> 426
   gaccaggagg aattcggtct tccagcaggg gatgaagaac aagatcttga tatttggcct 60
   ctttgaagag acagccctgg ctgctttcct ttcctactgc cctggaatgg gtgttgctct 120
   taggatgtat cccctcaaac ctacctggtg gttctgtgcc ttcccctact ctcttctcat 180
   cttcgtatat gacgaagtca gaaaactcat catcaggcga cgccctggcg gctgggtgga 240
   gaaggaaacc tactattagc cccccgtcct gcacgccgtg gagcatcagg ccacacactc 300
   tgcatccgac acccaccccc tctttgtgta cttcagtctt ggagtttgga actctaccct 360
   ggtaggaaag caccgcagca tgtggggaag caagacgtcc tggaatgaag catgtagctc 420
   tatgggggga ggggggaggg ctgcctgaaa accatcc 457
<210> 427
   <211> 478
   <212> DNA
   <213> Homo sapiens
   <400> 427
   ttgcctctta cggggttcgg caggatgggg accctgcttt cctctacttg ctgtcagctc 60
   ctcgagaagc cccagccaca ggacctagcc ctcagcaccc ccagaagatg gacggggaac 120
   ttggacgctt gtttccccca tcattggggc tacccccagg cccccagcca gctgcctcca 180
   gcctgcccag tccactccag cccagctggt cctgtccttc ctgcaccttc atcaatgccc 240
   cagaccgccc tggctgtgag atgtgtagca cccagaggcc ctgcacttgg gacccccttg 300
   ctgcagcttc cacctagcag ccaccagagg ttacaagggg agagtggccc ttccctcaca 360
   agtccgacat ctccaggccc ccactgaact ccggggacct ctactgactg cttgctggga 420
   cagtcaccag ggttgggggg aagggccaca aaatgaaacc attaaagacc cttaagag 478
<210> 428
   <211> 501
   <212> DNA
   <213> Homo sapiens
   <400> 428
   acaggtgtgt gctaccacat cttgctagtt ttgtattttt agcagagatg ggggtttcac 60
   catgttggcc aggctagtct cgaactcctg acctcaggtg atccacctgc cttggcctcc 120
   caaagcactg ggattacaag catgagccac tgtgcccagc ctgttccact gacatttctt 180
   agacattcag caaaaccccc accttaacct cttttctttc ttgagggttg gtcctgtccc 240
   cacctccacc ctcccacccc ctggaagagg aagggcccgg gcatcagtgg ctagtccaaa 300
   taaaatatgg gcttggggat ggaatgggtg gtggtaagtt cacagagtgt agttagatcc 360
   caactcccat gacctctggc ttcagtggtg ggtggggcag ggcagatgaa agggcttcag 420
   tgggaacctc tgagagcatt ttcctgttcc ccctatcaac cgcccccagt gataacatct 480
   gtgaagccag ccattactca a 501
<210> 429
   <211> 474
   <212> DNA
   <213> Homo sapiens
   <400> 429
   tttcagctca gtgcccatgg gcaaggatca tgatttccat tccgtgttac aatgacaata 60
   tttaatgagc ataaccttct cagtctcctg ctctcaaatt taggacagag ccgctaagga 120
   caaaacaatc cctcccgtgc tttatgatgg cagcaggggc tggggagcct ctgagggact 180
   ctttcattct gcagttgtct ggaagcctgg gtggcgtcat gagctgaagg atcatgcttt 240
   cctgtcctgg ctccataggt tataggctgg ctggtgaaag gttcacgtgg cccaggctga 300
   acttcattgc ctagctttgg atgtgctttc tgccataaag actgattttt gttcgttctg 360
   agccttcaag gaatttgttt tttacaactg gaatatgctc ctgtgtgtgt taacagatca 420
   tggatgtttt atgttttcac tgatcattta aagagtttga cctcagagct ccag 474
<210> 430
   <211> 316
   <212> DNA
   <213> Homo sapiens
   <400> 430
   gggctcccaa agcgacaaga tcgttaggga gagaggccca gggtggggac tgggaattta 60
   aggagagctg ggaacggatc ccttaggttc aggaagcttc tgtgcaagct gcgaggatgg 120
   cttgggccga agggttgctc tgcccgccgc gctagctgtg agctgagcaa agccctgggc 180
   tcacagcacc ccaaaagcct gtggcttcag tcctgcgtct gcaccaccca atcaaaagga 240
   tcgttttgtt ttgtttttaa agaaaggtga gattggcttg gttcttcatg agcacatttg 300
   atatagctct ttttct 316
<210> 431
   <211> 482
   <212> DNA
   <213> Homo sapiens
   <400> 431
   taatttgagc cacattccca actctaactc agcacacact gccagtcttc cccaatatct 60
   gtctcctctc aattccccac cacaccttat aaaattgtaa tcaaagatat ctcactctgt 120
   cattgttaat ctaagaataa aaacactgac tttaatacgg ttttactaag tttcaacctt 180
   ctaattaggt aggcctctag gtattctgca gatcactgct ggtcttgata gccattaata 240
   tatgtttgta ttatgttatt tttcaactaa atcgcagttg gaaaaaaaac atatttaata 300
   ttatgccctt ggatctgtta ctgcatcact agcacttgtg atgcaataga acacttcgcc 360
   tgtactgaaa gggccaagag taaatgcctt gttttgtttt tttgttttgt tttgttttgc 420
   tttttgttaa aacatgtcta tagagttggc agttaatgct gaatttgtca aatacccctt 480
   cc 482
<210> 432
   <211> 511
   <212> DNA
   <213> Homo sapiens
<220>
   <221> misc_feature
   <222> (32)..(32)
   <223> n is a, c, g, or t
   <220>
   <221> misc_feature
   <222> (34)..(34)
   <223> n is a, c, g, or t
   <220>
   <221> misc_feature
   <222> (37)..(37)
   <223> n is a, c, g, or t
   <220>
   <221> misc_feature
   <222> (73)..(73)
   <223> n is a, c, g, or t
   <220>
   <221> misc_feature
   <222> (284)..(285)
   <223> n is a, c, g, or t
   <400> 432
   gcatatagca ataaagaccc ccctccaccc tngnaanccc catcccccac cgggcctttg 60
   tccctgcctt ggnttttctc cccttctcat tctcctctcc cctttcctca ctgaaggctg 120
   tgagttgctt tcaatgtgac aacactatga tgtcatttgg aaggatttgc caggacagac 180
   tgattctgag tcctgggtgc cgtatgtgta tgcggcagtg ttgtcaggcg atcttgtttg 240
   aagctctatg ttgccataat taccatcaag tacacactgt tggnncaaaa ggctaacacc 300
   tgactttaga aaatgctgat ttgagaacaa aaggaaaggt cttttttcac tgcttaaagt 360
   ggggtcactt tgataccttt gcggtcatgt ctgtgtctga tgagtgtaga atctctggat 420
   gtgcactgtc agtcatgtgt ccaccaggcc tcgaatatca tatgggaaat gtcatagtta 480
   aaaacgtaca gccaggcccg tgtgctgtta a 511
<210> 433
   <211> 445
   <212> DNA
   <213> Homo sapiens
   <400> 433
   tggcctcttg atatacctcg agcttcccct gtgctcccca gccccaggac cactggcccc 60
   ttggcctgag gggctggggg ccccacgacc tgcagcgtcg agtccgggag agagcccgga 120
   gcggcgtgcc atctcggctc ggccttgctg agagcctccg ccctggcttt ctccctgtct 180
   ggtttcagtg gctcacgttg gtgctacaca gctagaatag atatatttag agagagagat 240
   atttttaaga caaagcccac aattagctgt cctttaacac cgcagaaccc cctcccagaa 300
   gaagagcgat ccctcggacg gtccgggcgg gcaccctcag ccgggctctt tgcagaagca 360
   gcaccgctga ctgtgggccc ggccctcaga tgtgtacata tacggctatt tcctatttta 420
   ctgttcttca gatttagtac ttgta 445
<210> 434
   <211> 443
   <212> DNA
   <213> Homo sapiens
   <400> 434
   agcttgtctg gtaagtggct tctctgtgaa ttgcctgtaa cacatagtgg cttctccgcc 60
   cttgtaaggt gttcagtaga gctaaataaa tgtaatagcc aaaccccact ctgttggtag 120
   caattggcag ccctatttca gtttattttt tcttctgttt tcttcttttc tttttttaaa 180
   cagtaaacct taacagatgc gttcagcaga ctggtttgca gtgaattttc atttctttcc 240
   ttatcacccc cttgttgtaa aaagcccagc acttgaattg ttattacttt aaatgttctg 300
   tatttgtatc tgtttttatt agccaattag tgggatttta tgccagttgt taaaatgagc 360
   attgatgtac ccatttttta aaaaagcaag cacagccttt gcccaaaact gtcatcctaa 420
   cgtttgtcat tccagtttga gtt 443

<210> 435
   <211> 536
   <212> DNA
   <213> Homo sapiens
   <400> 435
   gacggcgtca aggtcgtggg acgtgacacg accgctgcgg cgtcagctca gccttgcaag 60
   accccaggcg cccgcgctgc acctgcgact gtcgccgccg ccgtcgcagt cggaccaact 120
   gctggcagaa tcttcgtccg cacggcccca gctggagttg cacttgcggc cgcaagccgc 180
   cagggggcgc cgcagagcgc gtgcgcgcaa cggggaccac tgtccgctcg ggcccgggcg 240
   ttgctgccgt ctgcacacgg tccgcgcgtc gctggaagac ctgggctggg ccgattgggt 300
   gctgtcgcca cgggaggtgc aagtgaccat gtgcatcggc gcgtgcccga gccagttccg 360
   ggcggcaaac atgcacgcgc agatcaagac gagcctgcac cgcctgaagc ccgacacggt 420
   gccagcgccc tgctgcgtgc ccgccagcta caatcccatg gtgctcattc aaaagaccga 480
   caccggggtg tcgctccaga cctatgatga cttgttagcc aaagactgcc actgca 536
<210> 436
   <211> 464
   <212> DNA
   <213> Homo sapiens
   <400> 436
   tatgaacttg cgtgggctac tgcttgtagc tttggtggtt ctcgaccgtt tgtggtagca 60
   gtagatgaca tcatgtttca gaaacctgtt gaggttggct cattgctctt tctttcttca 120
   caggtatgct ttactcagaa taattatatt caagtcagag tacacagtga agtggcctcc 180
   ctgcaggaga agcagcatac aaccaccaat gtctttcatt tcacgttcat gtcggaaaaa 240
   gaagtgccat tggttttccc aaaaacatat ggagagtcca tgttgtactt agatgggcag 300
   cggcatttca actccatgag tggcccagcg accttgagaa aggactacct tgtggagccc 360
   taagaacacc acatttgttg aaaactagca ctctacccac agtgacgtgg tatctgatga 420
   agacctgatc gagtgtattg attttagtat tgcttcgtgt cctc 464
<210> 437
   <211> 533
   <212> DNA
   <213> Homo sapiens
   <400> 437
   gcgcagcatg gaggactttg tcacttgggt ggactcgtcc aagatcaagc ggcacgtgct 60
   agagtacaat gaggagcgcg atgacttcga tctggaagcc tagcggatct cccactttgc 120
   atggctgtct tttacagatg ggaaaactga ggcctgatgc tggagattct atgagggtgc 180
   tctcctcaag ggtatcagac ggtcgtaggt tcttaagaat ttgattcatc agtggcaggc 240
   catgcataga gccacgggag gtgcgtcctt gttttccagg aaatgttctt agaacttgga 300
   ctactgatta ttaattgact gtgccttggg aaacagtggg aagtaacttg gtgcagcact 360
   ggggtattgt tggactggtt caattcgttt aactcgaatt cttgctcctg gccgtggtta 420
   agctgtgtac agatgatgga gagtttggcc tcaagttttt ataaactgag cgagactagt 480
   gttcaggatc tcctcccttg tttaaatgtc aataaatgcc ccaactgctt tgt 533
<210> 438
   <211> 502
   <212> DNA
   <213> Homo sapiens
   <400> 438
   cccgaggacg acgacgagga cgaggaggac acggtgactc ggctgggccc cgacgacacg 60
   ctgccgggcc ccgagctgtc cgcagagccg gacgggcccc tcaacgtcaa cgtcttcacg 120
   tcggcggagg agctggagcg ggcgcagcgg ctggaggagc gcgaacggat cctgcgggag 180
   atctggcgca ccgggcagcc ggacctgctg ggcacaggca cgctggggcc cagccccacg 240
   gccacgggca ccctgggccg catgcactat tactgatggg ccccggctcc cgctgcaagg 300
   cgctcggggt accggacctg cacatgagct cagagctacc ccacaccttc ggactgcctc 360
   ggcccccaca gctcccaggt gctactgggc gtggaccgcc accccctgag aggctccctt 420
   ccccagtcct gccagaagac cccgggggcg gggagggggc agcatgcagg gtccccactc 480
   cctctctggg gtcgatgaag ag 502
<210> 439
   <211> 485
   <212> DNA
   <213> Homo sapiens
   <400> 439
   ctcccccttt gaaactcaag cacagctgcg aggagggcag cgaggaggga cccctctctc     60
   atggttgtct ctttcccccg ctatgtcata ggtagtggag gaagcgaagg aagtgaacgc 120
   tgaatgtgac gcatttctga agagctcagc tgtcaccggg catagcctgg aagccccaag 180
   tctgttctga ctttgcctgg ctgtctcctt gacccgcctc ctagatcatt gtccttgatg 240
   tccaggctgg gtcatttaaa atagagatgc aatcaggaag gttgggggac ttgggactgt 300
   ggctgaattg agaccttgct gatgtattca tgtcagcacc tgagtcacag cccaggtgcc 360
   cggaagcagc ctcttcgcat aggcagtgat ttgcgattac tttaaagctc accttttttc 420
   ttcccctctc tgttcgctgc tgtcagcata atgattgtgt tccttcccta tgggatccat 480
   ctgtt 485
<210> 440
   <211> 525
   <212> DNA
   <213> Homo sapiens
   <400> 440
   cagcctagcc ttcaagtggt gtgagcggcc tgagtggata cacgtggata gccggccctt 60
   tgcctccctg agccgtgact caggggctgc cctgggcctg ggcattgcct tgcactctcc 120
   ctgctatgcc caggtgcgtc gggcacagct gggaaatggc cagaagatag cctgccttgt 180
   gctggccatg gggctgctgg gccccctgga ctggctgggc cacccccctc agatcagcct 240
   cttctacatt ttcaatttcc tcaagtacac cctctggcca tgcctagtcc tggccctcgt 300
   gccctgggca gtgcacatgt tcagtgccca ggaagcaccg cccatccact cttcctgact 360
   tcttgtgtgc ctccctttcc tttccctccc acaaagccaa cactctgtga ccaccacact 420
   ccaggaggca gccccatccc cttccagccc ctaagtaggc cctcccctcc ctaaatctgc 480
   ttccgcacca cctggtctta gccccaaaga tgggccttct ctctc 525
<210> 441
   <211> 403
   <212> DNA
   <213> Homo sapiens
   <400> 441
   cgcaagcccc tgatgggcgc agaaaattcg ggacagacca cgtagaggtg ggctcccaag 60
   caggtgcgga cggcaccagg ccgcccaagg catcgctgcc acctgagctc cagccgccca 120
   caaactgctg catgagtggc tgccccaact gcgtgtgggt ggagtacgcg gacaggctgc 180
   tgcagcactt ccaggacggt ggggagcggg ccctggctgc cctggaggag cacgtggctg 240
   atgagaacct caaggccttc ctcaggatgg agatccggct gcacaccagg tgcggaggct 300
   gagccatccc tgctggactc cctaccgcag gacggagtcc aggacgcagc cgcagcctcc 360
   ttccttcaca ccccctcaca gactccttgt gtccaacggg aat 403
<210> 442
   <211> 346
   <212> DNA
   <213> Homo sapiens
   <400> 442
   taggggggag atttgaccgg caggcttctg cggagggctg cttctacaac gctgactacc 60
   tggcggcccg agcccggctg gcaggtgaac tggcaggcca ggaagaggag gaagccctgg 120
   aggggctgga ggtgatggat gttttcctcc ggttctcagg gctccacctc tttcgggccg 180
   tagagccagg gctggtgcag aagttctccc tgcgagactg cagcccacgg ctcagtgaag 240
   aactctacca ccgctgccgc ctcagcaacc tggaggggct agggggccgt gcccagctgg 300
   ctatggctct ctttgagcag gagcaggcca atagcactta gcccgc 346
<210> 443
   <211> 378
   <212> DNA
   <213> Homo sapiens
<220>
   <221> misc_feature
   <222> (146)..(146)
   <223> n is a, c, g, or t
   <220>
   <221> misc_feature
   <222> (220)..(220)
   <223> n is a, c, g, or t
   <400> 443
   ggggagggca gaaagatcac acacaaggct gtcacttcat acttgcagga ttgcacagca 60
   gccgggcaga ggcgctcctc acttcccaga tggggcggcg ggcagcagag acgcacctca 120
   cttcctagac agtgcggcag ccaggncaca ggcacacctc acttcccaga cagttgggcg 180
   gccaggcaag cgctcctcac ttcccagatg gggcggctcn cgggaagcgg ggctcctcac 240
   ttcccagaca gggtggccag gcagaggtgc tcctcacttc ccagaacaat tctttatgaa 300
   tttgataaag gactgaagtg caactgaaag ctgctagtga tgatctggta atatacaatt 360
   tgtccagtag ccagtttg 378
<210> 444
   <211> 556
   <212> DNA
   <213> Homo sapiens
   <400> 444
   ctgtgcatgg cacggctcaa gacagtcctg aaatacgtgc tgtttcttct gggtacactg 60
   gtcatcgcca tgtccttgca gctggaccgc aggggcatgt ggaacatgct ggggccctgc 120
   ctctttgcct tcgtgatcat ggcctccatg tgggcttacc gctgcgggca ccggcgccag 180
   tgctacccca cctcgtggca gcgctgggcc ttctacctcc tgcccggcgt ctctatggcc 240
   tctgtgggca tcgccatcta cacctccatg atgactagcg acaactacta ctacacccac 300
   agcatctggc acatcctgct ggccgggagc gcagccttgc tgctgccgcc acctgaccag 360
   cccgccgagc cctgggcctg ctcgcagaaa ttcccctgcc actatcagat ctgcaagaac 420
   gatcgggagg aactgtacgc agtgacgtga cactggcctg gggacagctg ctgctctgat 480
   gacctcttca gccaggagct gtatcgaggg ggaggcgcct ggtccagccc tggacagatt 540
   gatttccagc tgaata 556
<210> 445
   <211> 499
   <212> DNA
   <213> Homo sapiens
<220>
   <221> misc_feature
   <222> (338)..(338)
   <223> n is a, c, g, or t
   <400> 445
   tgcctaagcc tgtctgtgct tcagaggccc ctccagtccc tggctgtggg gtaactgggg 60
   gtatgagctg tggccacagg tgagcaaggc agggaactgc aatccagccc tggccgcggg 120
   aggggccatc tctggccaat gctgctgtgc cttcaaggac tgacaagtta cgtaggggca 180
   gaggtcgcca gctagccagt gtctcctcca tctggggggc gtctgtccac ttgtcacctt 240
   aggttttcac tcatttgtca ccttggggtt ttgctctgtg tgtttcatat ccaacggcaa 300
   tacttgcagg gggacagagt cctctaaata ctccaatnct gcggttttta caaacataaa 360
   gggggagacc ccaagtggag gaccctgggc ctggagctcc ctcccaaact ttgtccagca 420
   tccagcctgt tccctgggct cactggggag ggagttgtct tcatagcaca cccagagcca 480
   gggatccctt tgtagtttt 499
<210> 446
   <211> 462
   <212> DNA
   <213> Homo sapiens
<220>
   <221> misc_feature
   <222> (352)..(352)
   <223> n is a, c, g, or t
   <400> 446
   agcatctttc aagctccgtt actatggcga tggccatgat gttacaatcc cacttgcctg 60
   aataatcaag tgggaagggg aagcagaggg aaatggggcc atgtgaatgc agctgctctg 120
   ttctccctac cctgaggaaa aaccaaaggg aagcaacagg aacttctgca actggttttt 180
   atcggaaaga tcatcctgcc tgcagatgct gttgaagggg cacaagaaat tggagctgga 240
   gaagattgat gaaagtgcag gtgtgtaagg aaatagaaca gtctgctggg agtcagacct 300
   ggaattctga ttccaaactc tttattactt tgggaagtca ctcagcctcc cngtagccat 360
   ctccagggtg acggaaccca gtgtattacc tgctggaacc aaggaaacta acaatgtagg 420
   ttactagtga ataccccaat ggtttctcca attatgccca tg 462
<210> 447
   <211> 361
   <212> DNA
   <213> Homo sapiens
   <400> 447
   gtggacctac ctgataaata ccctttcaaa tctccatcta taggattcat gaataaaatt 60
   ttccatccca acattgatga agcgtcagga actgtgtgtc tagatgtaat taatcaaact 120
   tggacagctc tctatgatct taccaatata tttgagtcct tcctgcctca gttattggcc 180
   tatcctaacc ccatagatcc tctcaatggt gacgctgcag ccatgtacct ccaccgacca 240
   gaagaataca agcagaaaat taaagagtac atccagaaat acgccacgga ggaggcgctg 300
   aaagaacagg aagagggtac cggggacagc tcatcggaga gctctatgtc tgacttttcc 360
   g 361
<210> 448
   <211> 527
   <212> DNA
   <213> Homo sapiens
   <400> 448
   gatcccgcca ggcatgtgtg tgtgaatgca tgtgcaaagc tctccatcag aagggtggtg 60
   tgggccctgc aggtcctacc cctcggcctt gaagctccct cgggctgcgg actctgcctc 120
   ctgggtctga gcattagaac caggagaggg gtgtccctgg gcagagccag gggtgcaaac 180
   agcctgcagc catctggcct tttaagtata gtgtgtcgca tttccgggta ggaaggtagc 240
   atttcaagtt caaagagagg tcaagtcatg caaccatctt tcctccagca cttttggggt 300
   aaggaggaca gtttttgtta tggtttaggg gaaattttca tgaaattttc accattacca 360
   atagattact gatgtccatg gcaagtgatc tgttcttggt attttgtttt gttttgtttt 420
   ggtttttaaa tgtaatcacc cattggtcag gcccaggact ggtcaccatg agctctgcta 480
   gccacggccc caacgatgct tccggctctc atggattcca cagcaaa 527
<210> 449
   <211> 390
   <212> DNA
   <213> Homo sapiens
<220>
   <221> misc_feature
   <222> (35)..(35)
   <223> n is a, c, g, or t
   <220>
   <221> misc_feature
   <222> (93)..(93)
   <223> n is a, c, g, or t
   <220>
   <221> misc_feature
   <222> (108)..(108)
   <223> n is a, c, g, or t
   <220>
   <221> misc_feature
   <222> (187)..(189)
   <223> n is a, c, g, or t
   <400> 449
   ttctagtgtt tccccagtta ttgtgaccat ccaanccagg atatatgtaa atgcggatat 60
   ccatattgca gacatgaaaa aggttatcac aangtagttt ttccaaanct tttttctaca 120
   atctggtgtg gttagaaaaa gtaatgtaat aataggaagg gataataccc aaaaaattct 180
   ttttaannnt gcttcaggca tgttgaaaac acttggtgga tcttcagaaa cctgactaag 240
   gccatgtaaa cttatagaga gctgagagta gccagaatct tcataaaata ttccactatc 300
   agttcttgat tgccgacgaa tgaatggttg accttcatct tcccagccca tcagtggctg 360
   ttgttcactt ctctccatag ctttggcaag 390
<210> 450
   <211> 515
   <212> DNA
   <213> Homo sapiens
<220>
   <221> misc_feature
   <222> (214)..(214)
   <223> n is a, c, g, or t
   <220>
   <221> misc_feature
   <222> (224)..(224)
   <223> n is a, c, g, or t
   <400> 450
   cctaaggcct atcagcttct atcagcccgc agtgcctgcc tgctgggcct gttggccgcc 60
   accaacgcgc tgaccaatgg cgtgctgcct gccgtgcaga gcttttcctg cttaccctac 120
   gggcgtctgg cctaccacct ggctgtggtg ctgggcagtg ctgccaatcc cctggcctgc 180
   ttcctggcca tgggtgtgct gtgcaggtcc ttgncagggc tggncggcct ctctctgctg 240
   ggcgtgttct gtgggggcta cctgatggcg ctggcagtcc tgagcccctg cccgcccctg 300
   gtgggcacct cggcgggggt cgtcctcgtg gtgctgtcgt gggtgctgtg tcttggcgtg 360
   ttctcctacg tgaaggtggc agccagctcc ctgctgcatg gcgggggccg gccggcattg 420
   ctggcagccg gcgtggccat ccaggtgggc tctctgctcg gcgctgttgc tatgttcccc 480
   ccgaccagca tctatcacgt gttccacagc agaaa 515
<210> 451
   <211> 387
   <212> DNA
   <213> Homo sapiens
   <400> 451
   gcagcgtgag ggtgcactca gggtgttgtt agagcgtctc gtgtgtgcta gacgcacccc 60
   tactcgttcc tatagaacac agaggacata ggaaaccctt aaaacacaca tgggattctc 120
   tggtcacagt tttgggttca ggctatgctg ctttgggcag gtggagcacc ccccgaggaa 180
   gcctgcaagt ccagggcaca ggctgccttt tggagggagg gctggcccat aggtgctgct 240
   ggctccccgc caccagctgg gcctcagccc tcacggcatt cctgctgagc accgtggggc 300
   acccagggag caggggcgtc agggatcctg ctgccggcac ccctgtgccg ctggcatgag 360
   ggccgtgtcc ccactgtgaa ggatgaa 387
<210> 452
   <211> 449
   <212> DNA
   <213> Homo sapiens
   <400> 452
   gtctcttaga aggacactgg tcattggatt taaaggccac ctgggtaatt tatagtgatc 60
   taatctcaag aatctttcct taattacatg caaatactct ttatccaaat tagtttgcat 120
   tcacaaattc tggagcttag tacttggaca tatattttgg ggggttgatg gttggagggg 180
   cttttattca actcagtaca tcttaataag gaattaatgc cccccaactt gccttacaag 240
   tcatatatta aaaacaatgt tggcctggca cagtggctca tgcctgtaat ctcaacactt 300
   tgggaagcca agggaggagg atcacttgag cccaggagtt ggagaccagc ctggataaca 360
   aagggagacc cagtttctac aaaatattta aaaattagcc aggcatgatg gtgcatgcct 420
   gtggtcctag ctattcaggg aactgaggt 449
<210> 453
   <211> 548
   <212> DNA
   <213> Homo sapiens
   <400> 453
   gccggccctt tgcaatgaat gactcttcct gagcctggca ccaggagccc taggcaggcc 60
   gccgtctccc cactcacagc cccagcaggt aagcagtgta gacaaaccct tggggctttt 120
   ttatttggag aaccgtccag catgcatcct ggcccacggc ctgagcaagc tgcagccctt 180
   ctgaggccat gggcttcgtt ggctaagttg ggggtcttag ccttgcatgc gttgtgggca 240
   tcaaatctac ctccaaaaga cccatcctgg ggagccctct ggcccctcgt tgccttttca 300
   cttcaaaacc tcttttttct gggagaggcc ctgaaccctg tgcgggagag ctggtcctcc 360
   agccctggca ggccctcagc cagcttccca gcaagacaaa gggcaccctt gtggctttgg 420
   gacctaagtg gttggggttc ccgaggtcac tgaggactgg tacctcggga acgcaagctg 480
   tcagtggaac tgtcccacaa gaattcacag gtctcaaagc aggaacagtg ggtttgtgtc 540
   tcacctga 548
<210> 454
   <211> 569
   <212> DNA
   <213> Homo sapiens
<220>
   <221> misc_feature
   <222> (268)..(268)
   <223> n is a, c, g, or t
   <220>
   <221> misc_feature
   <222> (290)..(290)
   <223> n is a, c, g, or t
   <220>
   <221> misc_feature
   <222> (295)..(295)
   <223> n is a, c, g, or t
   <220>
   <221> misc_feature
   <222> (324)..(325)
   <223> n is a, c, g, or t
   <400> 454
   ttgtcttcta cgaccagctg aagcaagtga tgaatgcgta cagagtcaag ccggccgtct 60
   ttgacctgct cctggctgtt ggcattgctg cctacctcgg catggcctac gtggctgtcc 120
   aggtgagcag tgcccaggct cagcacttca gcctcctcta caagaccgtc cagaggctgc 180
   tcgtgaaggc caagacacag tgacacagcc acccccacag ccggagcccc cgccgctcca 240
   cagtccctgg ggccgagcac gagttggnag gggaccctct tctcccgtcn tgccntcggg 300
   ttgcccgcct cctccagaga cttnncaagg gcccatcacc actggcctct gggcacttgt 360
   gctgagactc tgggacccag gcagctgcca ccttgtcacc atgagagaat ttggggagtg 420
   cttgcatgct agccagcagg ctcctgtctg ggtgccacgg ggccagcatt ttggagggag 480
   cttccttcct tccttcctgg acaggtcgtc atgatggatg cactgactga ccgtctgggg 540
   ctcaggctgg tgtgggatgc agccggccg 569
<210> 455
   <211> 516
   <212> DNA
   <213> Homo sapiens
   <400> 455
   gtaggggtta caattcacat tccttattct gagaatttgg ccccagctgt ttgcctttga 60
   ctccctgacc tccagagcca gggttgtgcc ttattgtccc atctgtgggc ctcattctgc 120
   caaagctgga ccaaggctaa cctttctaag ctccctaact tgggccagaa accaaagctg 180
   agcttttaac tttctccctc tatgacacaa atgaattgag ggtaggagga gggtgcacat 240
   aacccttacc ctacctctgc caaaaagtgg gggctgtact ggggactgct cggatgatct 300
   ttcttagtgc tacttctttc agctgtccct gtagcgacag gtctaagatc tgactgcctc 360
   ctcctttctc tggcctcttc ccccttccct cttctcttca gctaggctag ctggtttgga 420
   gtagaatggc aactaattct aatttttatt tattaaatat ttggggtttt ggttttaaag 480
   ccagaattac ggctagcacc tagcatttca gcagag 516
<210> 456
   <211> 334
   <212> DNA
   <213> Homo sapiens
   <400> 456
   aattaagcat tttcttgcct cctttgcttc atcttttcac aacagctgga tagagggatc 60
   agaaatgact gtgtcatggt gctcattcac tgcaaactcc cagttgcaag ctccttggct 120
   cccccggagg gagcaagaat ctcatagttc agagacacag agggcctttt agccctaatg 180
   accttttgga tgggactgca actcatgact atcctgatat tggaagaaag gactttgtta 240
   atcttctccc ccatagctct gctgcgtagg tctacatctt actcagaatc actacacatt 300
   cctttagtct tcctccaagc tccagagcca ttgg 334
<210> 457
   <211> 569
   <212> DNA
   <213> Homo sapiens
   <400> 457
   gggcaggttt ggagcccatg ggaccccgtg ggtctctgtc caggagcagc agaggaggct 60
   gacaggccct gctccctctg ctctgggggt gtctgggagc cccagctcac accctcccaa 120
   tgcttatatg ctgaagctca cagaatgggc ttcttgcctg acagcaagtc aaagaatgag 180
   tttaatatca aagtgtaagc ttactttcca tccccaagcc agcctgcccc ctgccccatt 240
   tcccatgagc acacttctgg ggaaggaaaa caggctcctg gccttcactc tcagcagagc 300
   tttggagatg ccccaggcat gccctgagct ccttctgtgt acctgctccc acttctgagc 360
   cacccgctgc ccctccgcac tgctggcaaa cccagttcct gcctcagcca ggtctccttc 420
   cctggtttcc agtcacacag agcccagcag ctttctcttt cagtcccata agggcagcct 480
   tgtgtccctg gccacacttc cacccgccag ggtcttcctc cccatctttc catccttcct 540
   gctgagcttc cacagagctc gtttgcaaa 569
<210> 458
   <211> 467
   <212> DNA
   <213> Homo sapiens
   <400> 458
   tacctcggag ctgatgctgg gcggaaccaa cacactggtg ctgcacaaca cgtgtgagga 60
   ctcgctgctg gccgcaccca tcatgctgga cctagcgctg ctgaccgagc tgtgccagcg 120
   cgtgagcttc tgcactgaca tggaccccga gccgcagacc ttccaccccg tgctgtccct 180
   gctcagcttc ctcttcaagg cgccactagt gccgcccggc agcccggtgg tcaatgcgct 240
   tttccgccag cgcagctgca tcgagaacat cctcagggcc tgcgtggggc tcccgccaca 300
   gaaccacatg ctcctggaac acaaaatgga gcgcccaggg cccagcctca agcgagttgg 360
   acccgtggct gccacctacc ctatgttgaa caagaaagga ccggtacccg ctgccaccaa 420
   tggctgcacc ggtgatgcca atgggcatct gcaagaggag cccccaa 467
<210> 459
   <211> 254
   <212> DNA
   <213> Homo sapiens
   <400> 459
   attagctata gattccactg gccttaaaca tacaattaag tgtatacatg atatagtgca 60
   cacacaaaag ccacctttaa ttattgaaat aacctgtatt ctttttggaa atcatttaag 120
   tttggtattg aagtactata ttttttgtgc atcaatgtat ttttctattt acaagcctat 180
   gtaaaagtga agtgtatctt cagtgaacca tgtgccaatt aagctgtaat aaaaaagtgg 240
   tctagtctgt caaa 254
<210> 460
   <211> 338
   <212> DNA
   <213> Homo sapiens
<220>
   <221> misc_feature
   <222> (95)..(95)
   <223> n is a, c, g, or t
   <220>
   <221> misc_feature
   <222> (99)..(99)
   <223> n is a, c, g, or t
   <400> 460
   cttttgctga ggttttctct gaggtttttt tgatgcttta taggaaacta ttttttaaaa 60
   aaagccattt cccacccaag gacacagtgg atgtntttnc cctgactcca gcagggcaag 120
   gaatgtagcc gagaggttgt gtgggctggg ctctggtgcc ctcttccctg gccaggacac 180
   ctctcctcct gattcccttg gcaccttgtc tttctgtctg tttacctgtc tccctgcctg 240
   cccatctgca tcttttgcag cccactctga cttccatctg ggggctgaga ccacccttgc 300
   ctgccccctt ctttctgcct taagaatgtc cttttagg 338

<210> 461
   <211> 544
   <212> DNA
   <213> Homo sapiens
<220>
   <221> misc_feature
   <222> (158)..(172)
   <223> n is a, c, g, or t
   <220>
   <221> misc_feature
   <222> (182)..(185)
   <223> n is a, c, g, or t
   <220>
   <221> misc_feature
   <222> (220)..(220)
   <223> n is a, c, g, or t
   <220>
   <221> misc_feature
   <222> (257)..(257)
   <223> n is a, c, g, or t
   <220>
   <221> misc_feature
   <222> (305)..(320)
   <223> n is a, c, g, or t
   <220>
   <221> misc_feature
   <222> (401)..(401)
   <223> n is a, c, g, or t
   <220>
   <221> misc_feature
   <222> (504)..(504)
   <223> n is a, c, g, or t
   <400> 461
   agggagtccc agagccctgg accttgggcc tagaccgcgt gataaaactg ggttgaggga 60
   tgctggaacc agttacgact gaagtcagtg tagacctgag ctgggaggga acctgttagt 120
   ctccccacct cttccctgaa gagacaggca cccctccnnn nnnnnnnnnn nngagggagt 180
   gnnnnttctg ccttgagtcc ccaggggaaa aaaaaaaaan gatatttatg aaataaatgg 240
   taatttgtgt aaataangct ttaaggttcc cagaatatgc aaattggtat taatttattc 300
   aaagnnnnnn nnnnnnnnnn acatatattt agagattaac tcatacattt aaagtttttt 360
   tcattttacg tgagcatcta tattgtacag ggctgggggg ncccttggct gcgggagaag 420
   gcccagagcc ctggaggagc caccaccccg ccggcccctc gacccctcgg cccctcggcc 480
   cctccgcccg ggtttggctc gccnggcccg cgggctccac ctcaggtttt cacttttcgc 540
   tccg 544
<210> 462
   <211> 238
   <212> DNA
   <213> Homo sapiens
   <400> 462
   ttttcctggg actgccatat tttcttttaa ctggaaattt ttatgtgagt tttccttttg 60
   gtgcatggaa ctgtggttgc caaggtattt aaaagggctt tcctgcctcc ttctctttga 120
   tttatttaat ttgatttggg ctataaaata tcatttttca ggtttattct tttagcaggt 180
   gtagttaaac gacctccact gaactgggtt tgacctctgt tgtactgatg tgttgtga 238
<210> 463
   <211> 388
   <212> DNA
   <213> Homo sapiens
<220>
   <221> misc_feature
   <222> (26)..(26)
   <223> n is a, c, g, or t
   <220>
   <221> misc_feature
   <222> (36)..(36)
   <223> n is a, c, g, or t
   <220>
   <221> misc_feature
   <222> (53)..(53)
   <223> n is a, c, g, or t
   <220>
   <221> misc_feature
   <222> (215)..(215)
   <223> n is a, c, g, or t
   <220>
   <221> misc_feature
   <222> (254)..(275)
   <223> n is a, c, g, or t
   <220>
   <221> misc_feature
   <222> (298)..(298)
   <223> n is a, c, g, or t
   <400> 463
   gggtcgtatc actttgtctc tcctancccc cactgncccc gagtgtcggg cancgatgta 60
   catatggagg tggggtggac agggtgctgt gccccttcag agggagtgca gggcttgggg 120
   tgggcctagt cctgctccta gggctgtgaa tgttttcagg gtggggggag ggagatggag 180
   cctcctgtgt gtttgggggg aagggtgggt ggggncctcc cacttggccc cggggttcag 240
   tggtatttta tacnnnnnnn nnnnnnnnnn nnnnntggga aaggctgtgt gagggganag 300
   aagggagagg gtgggcctgc tgtggacaat ggcatactct cttccagccc taggaggagg 360
   gctcctaaca gtgtaactta ttgtgtcc 388
<210> 464
   <211> 345
   <212> DNA
   <213> Homo sapiens
<220>
   <221> misc_feature
   <222> (67)..(83)
   <223> n is a, c, g, or t
   <220>
   <221> misc_feature
   <222> (137)..(137)
   <223> n is a, c, g, or t
   <220>
   <221> misc_feature
   <222> (143)..(146)
   <223> n is a, c, g, or t
   <220>
   <221> misc_feature
   <222> (148)..(155)
   <223> n is a, c, g, or t
   <220>
   <221> misc_feature
   <222> (157)..(157)
   <223> n is a, c, g, or t
   <220>
   <221> misc_feature
   <222> (160)..(160)
   <223> n is a, c, g, or t
   <220>
   <221> misc_feature
   <222> (162)..(162)
   <223> n is a, c, g, or t
   <220>
   <221> misc_feature
   <222> (164)..(164)
   <223> n is a, c, g, or t
   <220>
   <221> misc_feature
   <222> (166)..(168)
   <223> n is a, c, g, or t
   <220>
   <221> misc_feature
   <222> (170)..(188)
   <223> n is a, c, g, or t
   <220>
   <221> misc_feature
   <222> (247)..(248)
   <223> n is a, c, g, or t
   <400> 464
   gatttgaccg gcaggcttct gcggagggct gcttctacaa cgctgactac ctggcggccc 60
   gagcccnnnn nnnnnnnnnn nnngcaggcc aggaagagga ggaagccctg gaggggctgg 120
   aggtgatgga tgttttnctc cgnnnntnnn nnnnncnccn cntntnnngn nnnnnnnnnn 180
   nnnnnnnngt gcagaagttc tccctgcgag actgcagccc acggctcagt gaagaactct 240
   accaccnntg ccgcctcagc aacctggagg ggctaggggg ccgtgcccag ctggctatgg 300
   ctctctttga gcaggagcag gccaatagca cttagcccgc ctggg 345
<210> 465
   <211> 244
   <212> DNA
   <213> Homo sapiens
<220>
   <221> misc_feature
   <222> (92)..(128)
   <223> n is a, c, g, or t
   <400> 465
   tgaagtgcaa ctgaaagctg ctagtgatga tctggtaata tacaatttgt ccagtagcca 60
   gtttgttttt attgtgtttt ctaaccataa gnnnnnnnnn nnnnnnnnnn nnnnnnnnnn 120
   nnnnnnnnac acaaaaaaat ggtcaccgca ggccatacta ccaatgaaat ggtaggtaaa 180
   caaatcttct ggtcaagaga aaaaaaaaag aaatagcact ctgcatgctt tgctctacaa 240
   gatg 244
<210> 466
   <211> 578
   <212> DNA
   <213> Homo sapiens
<220>
   <221> misc_feature
   <222> (138)..(138)
   <223> n is a, c, g, or t
   <220>
   <221> misc_feature
   <222> (141)..(141)
   <223> n is a, c, g, or t
   <220>
   <221> misc_feature
   <222> (145)..(145)
   <223> n is a, c, g, or t
   <220>
   <221> misc_feature
   <222> (148)..(148)
   <223> n is a, c, g, or t
   <220>
   <221> misc_feature
   <222> (165)..(165)
   <223> n is a, c, g, or t
   <220>
   <221> misc_feature
   <222> (168)..(170)
   <223> n is a, c, g, or t
   <220>
   <221> misc_feature
   <222> (377)..(377)
   <223> n is a, c, g, or t
   <220>
   <221> misc_feature
   <222> (424)..(451)
   <223> n is a, c, g, or t
   <220>
   <221> misc_feature
   <222> (453)..(453)
   <223> n is a, c, g, or t
   <220>
   <221> misc_feature
   <222> (485)..(485)
   <223> n is a, c, g, or t
   <220>
   <221> misc_feature
   <222> (487)..(487)
   <223> n is a, c, g, or t
   <220>
   <221> misc_feature
   <222> (489)..(489)
   <223> n is a, c, g, or t
   <220>
   <221> misc_feature
   <222> (495)..(495)
   <223> n is a, c, g, or t
   <220>
   <221> misc_feature
   <222> (497)..(497)
   <223> n is a, c, g, or t
   <220>
   <221> misc_feature
   <222> (517)..(517)
   <223> n is a, c, g, or t
   <220>
   <221> misc_feature
   <222> (522)..(522)
   <223> n is a, c, g, or t
   <220>
   <221> misc_feature
   <222> (528)..(528)
   <223> n is a, c, g, or t
   <220>
   <221> misc_feature
   <222> (531)..(531)
   <223> n is a, c, g, or t
   <400> 466
   gaaatccttc ctgctcaggc tttcattcta aaactacagt cttcattaaa gctgaacttt 60
   ctgggtagct gagcttatat gcccggcatc tgaatgagag ctctctttgt aactgtgtga 120
   cttgagatct agtttgcnag ntccnggnaa acaatacatg tgttnttnnn tttgtgtttg 180
   ctcagcaagc agatgtctga gatgtaagaa gcttttcttt tcctgtggca ttgattctga 240
   cttagagctg aagtaaagat cactgaaaca tcacgtcaag ttgaagtcac tcataggtct 300
   ttgtccttta ggcaggacag gagagtcatt aagaagcatt tcactgtagc attctatcac 360
   aatatcatct ggaattnttt tctttgccca gaaagcctta acttgcctct agagaatccc 420
   tggnnnnnnn nnnnnnnnnn nnnnnnnnnn ntncaactct tctgctgtgg aagtttgaag 480
   cgacngncna ggcanancca gagaatttcc tcaagtngcc tntaggtncc ntgttatctt 540
   atgcccccac ccctccctca acaatatgag tgatccag 578
<210> 467
   <211> 481
   <212> DNA
   <213> Homo sapiens
   <400> 467
   gcggtggagc cgcaaccaaa atgcagattt tcgtgaaaac ccttacgggg aagaccatca 60
   ccctcgaggt tgaaccctcg gatacgatag aaaatgtaaa ggccaagatc caggataagg 120
   aaggaattcc tcctgatcag cagagactga tctttgctgg caagcagcta gaagatggac 180
   gtactttgtc tgactacaat attcaaaagg agtctactct tcatcttgtg ttgagacttc 240
   gtggtggtgc taagaaaagg aagaagaagt cttacaccac tcccaagaag aataagcaca 300
   agagaaagaa ggttaagctg gctgtcctga aatattataa ggtggatgag aatggcaaaa 360
   ttagtcgcct tcgtcgagag tgcccttctg atgaatgtgg tgctggggtg tttatggcaa 420
   gtcactttga cagacattat tgtggcaaat gttgtctgac ttactgtttc aacaaaccag 480
   a 481
<210> 468
   <211> 452
   <212> DNA
   <213> Homo sapiens
   <400> 468
   gtaaaggctg ttctggcttt ttatcttctt agctcatctt aaataagcag tacacttgga 60
   tgcagtgcgt ctgaagtgct aatcagttgt aacaatagca caaatcgaac ttaggatttg 120
   tttcttctct tctgtgtttc gatttttgat caattcttta attttggaag cctataatac 180
   agttttctat tcttggagat aaaaattaaa tggatcactg atattttagt cattctgctt 240
   ctcatctaaa tatttccata ttctgtatta ggagaaaatt accctcccag caccagcccc 300
   cctctcaaac ccccaaccca aaaccaagca ttttggaatg agtctccttt agtttcagag 360
   tgtggattgt ataacccata tactcttcga tgtacttgtt tggtttggta ttaatttgac 420
   tgtgcatgac agcggcaatc ttttctttgg tc 452
<210> 469
   <211> 515
   <212> DNA
   <213> Homo sapiens
   <400> 469
   ggtcacgttc ttggatcctc agaactcttt gctcttgtcg gggtgggggt gggaactcac 60
   gtggggagcg gtggctgaga aaatgtaagg attctggaat acatattcca tggactttcc 120
   ttccctctcc tgcttcctct tttcctgctc cctaaccttt cgccgaatgg ggcagacaaa 180
   cactgacgtt tctgggtggc cagtgcggct gccaggttcc tgtactactg ccttgtactt 240
   ttcattttgg ctcaccgtgg attttctcat aggaagtttg gtcagagtga attgaatatt 300
   gtaagtcagc cactgggacc cgaggatttc tgggaccccg cagttgggag gaggaagtag 360
   tccagccttc caggtgggcg tgagaggcaa tgactcgtta cctgccgccc atcaccttgg 420
   aggccttccc tggccttgag tagaaaagtc ggggatcggg gcaagagagg ctgagtacgg 480
   atgggaaact attgtgcaca agtctttcca gagga 515
<210> 470
   <211> 378
   <212> DNA
   <213> Homo sapiens
   <400> 470
   ccctggtttg cagctgtttt caaagccccc gataatcgct cttttccact ccaagatgcc 60
   ctcataaacc aatgtggcaa gactactgga cttctatcaa tggtactcta atcagtcctt 120
   attatcccag cttgctgagg ggcagggaga gcgcctcttc ctctgggcag cgctatctag 180
   ataggtaagt gggggcgggg aagggtgcat agctgtttta gctgagggac gtggtgccga 240
   cgtccccaaa cctagctagg ctaagtcaag atcaacattc cagggttggt aatgttggat 300
   gatgaaacat tcatttttac cttgtggatg ctagtgctgt agagttcact gttgtacaca 360
   gtctgttttc tatttgtt 378
<210> 471
   <211> 511
   <212> DNA
   <213> Homo sapiens
   <400> 471
   aacactgcat aacccgtttc tttgaggagt gtgaccccaa caaggataag cacatcaccc 60
   tgaaggagtg gggccactgc tttggaatta aagaagagga catagatgaa aatctcttgt 120
   tttgaacgaa gattttaaag aactcaactt tccagcatcc tcctctgttc taaccacttc 180
   agaaatatat gcagctgtga tacttgtaga tttatattta gcaaaatgtt agcatgtatg 240
   acaagacaat gagagtaatt gcttgacaac aacctatgca ccaggtattt aacattaact 300
   ttggaaacaa aaatgtacaa ttaagtaaag tcaacatatg caaaatactg tacattgtga 360
   acagaagttt aattcatagt aatttcactc tctgcattga cttatgagat aattaatgat 420
   taaactatta atgataaaaa taatgcattt gtattgttca taatatcatg tgcacttcaa 480
   gaaaatggaa tgctactctt ttgtggttta c 511
<210> 472
   <211> 215
   <212> DNA
   <213> Homo sapiens
   <400> 472
   ttctgagtgt agtgtggtag gacccggcgg gtgtgcagca actgccctgg agccccagcc 60
   cctgcgtcca tctgtgctgt gcgccccaca gtagacgtgc agacgtccct gagaggttct 120
   tgaagatgtt tatttatatt gtcctttttt actggaagac gtacgcatac tccatcgatg 180
   ttgtatttgc agtggctgag gaattcttgt acgca 215
<210> 473
   <211> 381
   <212> DNA
   <213> Homo sapiens
   <400> 473
   ctctcttagc tcagttactc aattcatacg tagtattttt taaaataatt ttatatctgt 60
   gtaccacccc atatatttca tattactgtt tcacatgtac agctttctac ttctttgtaa 120
   gaacaccaac caaccaaggt ttaagtgatt aataggcttg agcaccgggt ggcagatgtt 180
   ctatgcagtg tggttcaagt ttctttgacc gcacttatat gcattgctaa tatggaattt 240
   aagataccat acacagtctc tcatggacct atctctattg tagaattatg acttatgtct 300
   tacttggcaa atttttctga atgtgacctt tttttgctga tttgctgggt ttgggattaa 360
   ctagcattat tttgccacct t 381
<210> 474
   <211> 484
   <212> DNA
   <213> Homo sapiens
   <400> 474
   gccattacag tatccaatgt cttttgacag gtgcctgtcc ttgaaaaaca aagtttctat 60
   ttttattttt aattggttta gttcttaact gctggccaac tcttacatcc ccagcaaatc 120
   atcgggccat tggatttttt ccattatgtt catcaccctt atatcatgta cctcagatct 180
   ctctctctct cctctctctc agttatatag tttcttgtct tggacttttt ttttcttttc 240
   tttttctttt tttttttgct ttaaaacaag tgtgatgcca tatcaagtcc atgttattct 300
   ctcacagtgt actctataag aggtgtgggt gtctgtttgg tcaggatgtt agaaagtgct 360
   gataagtagc atgatcagtg tatgcgaaaa ggtttttagg aagtatggca aaaatgttgt 420
   attggctatg atggtgacat gatatagtca gctgcctttt aagaggtctt atctgttcag 480
   tgtt 484
<210> 475
   <211> 563
   <212> DNA
   <213> Homo sapiens
   <400> 475
   agagtgcagt tcccatgagt cacttcctga acccattgac caaaggtgga cagagacaat 60
   cctgtagacc ttgacattca gaaagatgtg agctgcttac tgatcatata tgcatacgtt 120
   tctttacagc agaggaaacc attgtccaca aaactgatgt tcttttgggg ttttatgtac 180
   agacttgtcc aatcatgtgt gtggttcctg cgagttgctg atgactccgc attgaagctc 240
   tctgagttct ttgattttaa gttgggttta tggaattttt tcaaatgttg gaaggcgtgt 300
   ggttcttcct gccctccctc ccctttggga aatatgaaag caaatgttta gaagaattcc 360
   ttttgaaaag ctgtgtcgtg ttccctgtga aactgagcag gtgtgtgttg gcgcgctaag 420
   tgccacatgc ttgtgtgtag aggaggaggt ggccctgccg gctccgcgct gctgtgcctg 480
   tgatccctac ctgctccccg ctcctgttgc cagcagcact cactgcactc ctttgtcata 540
   tactctgcat cactgtcata ctc 563
<210> 476
   <211> 295
   <212> DNA
   <213> Homo sapiens
   <400> 476
   agaaatgcct cacagctatc gtgaagtgcg ccacaagcaa accagctttc tttgcagaga 60
   agcttcatca agccatgaaa ggtgttggaa ctcgccataa ggcattgatc aggattatgg 120
   tttcccgttc tgaaattgac atgaatgata tcaaagcatt ctatcagaag atgtatggta 180
   tctccctttg ccaagccatc ctggatgaaa ccaaaggaga ttatgagaaa atcctggtgg 240
   ctctttgtgg aggaaactaa acattccctt gatggtctca agctatgatc agaag 295
<210> 477
   <211> 360
   <212> DNA
   <213> Homo sapiens
   <400> 477
   gcaataactc tgggaggggc tcgagagggc tggtccttat ttatttaact tcacccgagt 60
   tcctctgggt ttctaagcag ttatggtgat gacttagcgt caagacattt gctgaactca 120
   gcacattcgg gaccaatata tagtgggtac atcaagtcca tctgacaaaa tggggcagaa 180
   gagaaaggac tcagtgtgtg atccggtttc tttttgctcg cccctgtttt ttgtagaatc 240
   tcttcatgct tgacatacct accagtatta ttcccgacga cacatataca tatgagaata 300
   taccttattt atttttgtgt aggtgtctgc cttcacaaat gtcattgtct actcctagaa 360

<210> 478
   <211> 461
   <212> DNA
   <213> Homo sapiens
   <400> 478
   agcccacagt gcctgtacag gaaggtgcct ggccatgtca cctggctgct aggccagagc 60
   catgccaggc tgcgtccctc cgagcttggg ataaagcaag gggaccttgg cgctctcagc 120
   tttccctgcc acatccagct tgttgtccca atgaaatact gagatgctgg gctgtctctc 180
   ccttccagga atgctgggcc cccagcctgg ccagacaaga agactgtcag gaagggtcgg 240
   agtctgtaaa accagcatac agtttggctt ttttcacatt gatcattttt atatgaaata 300
   aaaagatcct gcatttatgg tgtagttctg agtcctgaga cttttctgcg tgatggctat 360
   gccttgcaca caggtgttgg tgatggggct gttgagatgc ctgttgaagg tacatcgttt 420
   gcaaatgtga gtttcctctc ctgtccgtgt ttgtttagta c 461
<210> 479
   <211> 541
   <212> DNA
   <213> Homo sapiens
   <400> 479
   catgtgcaca cagattattt tttggctcca aaactggatt gcaaaagaaa gaggagaaga 60
   atattttgtg tgttcctggt attcttttat aagtaaagtt tacccaggca tggaccagct 120
   tcagccaggg acaaaatccc ctcccaaacc actctccaca gctttttaaa aatacttcta 180
   ctcttaacaa ttacctaagg cttcctcaac tgccccaaat ctcttaatag cttctagtgc 240
   tgctacaatc taagtcaggt caccagaggg aagagaacat ggcattaaaa gaatcacatc 300
   ttcagaagag aagacactaa tattattacc catatacatg atttcagaag atgacataag 360
   attcctctta aagaggaaat gtcaggaatc aagccactga atccttaaag agaaaagttg 420
   aatatgagtc attgtgtctg aaaactgcaa agtgaactta actgagatcc agcaaacagg 480
   ttctgtttaa gaaaaataat ttatactaaa tttagtaaaa tggacttctt attcaaagca 540
   t 541
<210> 480
   <211> 488
   <212> DNA
   <213> Homo sapiens
   <400> 480
   gttttggctg aaattctcct ggaggtcggt aggttcagcc aaggttttat aaggctgatg 60
   tcaatttctg tgttgccaag ctccaagccc catcttctaa atggcaaagg aaggtggatg 120
   gccccagcac agcttgacct gaggctgtgg tcacagcgga ggtgtggagc cgaggcctac 180
   cccgcagaca ccttggacat cctcctccca cccggctgca gaggccagag gcccccagcc 240
   cagggctcct gcacttactt gcttatttga caacgtttca gcgactccgt tggccactcc 300
   gagaggtggg ccagtctgtg gatcagagat gcaccaccaa gccaagggaa cctgtgtccg 360
   gtattcgata ctgcgacttt ctgcctggag tgtatgactg cacatgactc gggggtgggg 420
   aaaggggtcg gctgaccatg ctcatctgct ggtccgtggg acggtgccca agccagaggc 480
   tgggttca 488
<210> 481
   <211> 547
   <212> DNA
   <213> Homo sapiens
<220>
   <221> misc_feature
   <222> (97)..(99)
   <223> n is a, c, g, or t
   <220>
   <221> misc_feature
   <222> (135)..(135)
   <223> n is a, c, g, or t
   <220>
   <221> misc_feature
   <222> (258)..(258)
   <223> n is a, c, g, or t
   <400> 481
   agcatcggag ccattcattc ggagaaaacg ttttgatcaa aatggagact tttgtagtcg 60
   tttcaaaaga gcacctgagt catgtgtatt cccggcnnnc tttataaatg acccggtcaa 120
   gttggtttca aagtncgaca ggcttgtctg tttactagct gcgtggcctt ggacgggtgg 180
   ctgacatctg taaagaatcc tcctgtgatg aaactgagga atcgggtggc cgggcaagct 240
   gggaagagca aagccagnag ctgcgctgcc tcaataccca caaaagacca ttcccagtat 300
   acataagcac aggatgtttt tctcaagagg gatgtattta tcacttggac atctgtttat 360
   aatataaaca gacatgtgac tgggaacatc ttgctgccaa aagaatccta ggcagtggct 420
   cattgtatgt gaggttgaac cacgtgaaat tgccaatatt aggctggctt ttatctacaa 480
   agaaggagtt tcatggggtt cagcctaaca gttatggaaa ctacagtcct tataaaccat 540
   tggcatg 547
<210> 482
   <211> 451
   <212> DNA
   <213> Homo sapiens
   <400> 482
   ggcactgtgt gggtcaactg ctatgatgtg tttggagccc agtcaccctt tggtggctac 60
   aagatgtcgg ggagtggccg ggagttgggc gagtacgggc tgcaggcata cactgaagtg 120
   aaaactgtca cagtcaaagt gcctcagaag aactcataag aatcatgcaa gcttcctccc 180
   tcagccattg atggaaagtt cagcaagatc agcaacaaaa ccaagaaaaa tgatccttgc 240
   gtgctgaata tctgaaaaga gaaatttttc ctacaaaatc tcttgggtca agaaagttct 300
   agaatttgaa ttgataaaca tggtgggttg gctgagggta agagtatatg aggaaccttt 360
   taaacgacaa caatactgct agctttcagg atgattttta aaaaatagat tcaaatgtgt 420
   tatcctctct ctgaaacgct tcctataact c 451
<210> 483
   <211> 364
   <212> DNA
   <213> Homo sapiens
   <400> 483
   atgatccaga aatacttaac acgtgaatat tttgctaaaa aagcatatat aactatttta 60
   aatatccatt tatcttttgt atatctaaga ctcatcctga tttttactat cacacatgaa 120
   taaaggcctt tgtatctttc tttctctaat gttgtatcat actcttctaa aacttgagtg 180
   gctgtcttaa aagatataag gggaaagata atattgtctg tctctatatt gcttagtaag 240
   tatttccata gtcaatgatg gtttaatagg taaaccaaac cctataaacc tgacctcctt 300
   tatggttaat actattaagc aagaatgcag tacagaattg gatacagtac ggatttgtcc 360
   aaat 364
<210> 484
   <211> 468
   <212> DNA
   <213> Homo sapiens
   <400> 484
   ttagcgttca tccgtgtaac ccgctcatca ctggatgaag attctcctgt gctagatgtg 60
   caaatgcaag ctagtggctt caaaatagag aatcccactt tctatagcag attgtgtaac 120
   aattttaatg ctatttcccc aggggaaaat gaaggttagg atttaacagt catttaaaaa 180
   aaaaatttgt tttgacggat gattggatta ttcatttaaa atgattagaa ggcaagtttc 240
   tagctagaaa tatgatttta tttgacaaaa tttgttgaaa ttatgtatgt ttacatatca 300
   cctcatggcc tattatatta aaatatggct ataaatatat aaaaagaaaa gataaagatg 360
   atctactcag aaatttttat ttttctaagg ttctcatagg aaaagtacat ttaatacagc 420
   agtgtcatca gaagataact tgagcaccgt catggcttaa tgtttatt 468
<210> 485
   <211> 357
   <212> DNA
   <213> Homo sapiens
   <400> 485
   cagggctgtc atcaacatgg atatgacatt tcacaacagt gactagttga atcccttgta 60
   acgtagtagt tgtctgctct ttgtccatgt gttaatgagg actgcaaagt cccttctgtt 120
   gtgattccca ggacttttcc tcaagaggaa atctggattt ccacctaccg cttacctgaa 180
   atgcaggatc acctacttac tgtattctac attattatat gacatagtat aatgagacaa 240
   tatcaaaagt aaacatgtaa tgacaataca tactaacatt cttgtaggag tggttagaga 300
   agctgatgcc tcatttctac attctgtcat tagctattat catctaacgt ttcagtg 357
<210> 486
   <211> 436
   <212> DNA
   <213> Homo sapiens
   <400> 486
   gagtggacta ttaaatgtgc ctaaatgaat tttgcagtaa ctggtattct tgggttttcc 60
   tacttaatac acagtaattc agaacttgta ttctattatg agtttagcag tcttttggag 120
   tgaccagcaa ctttgatgtt tgcactaaga ttttatttgg aatgcaagag aggttgaaag 180
   aggattcagt agtacacata caactaattt atttgaacta tatgttgaag acatctacca 240
   gtttctccaa atgccttttt taaaactcat cacagaagat tggtgaaaat gctgagtatg 300
   acacttttct tcttgcatgc atgtcagcta cataaacagt tttgtacaat gaaaattact 360
   aatttgtttg acattccatg ttaaactacg gtcatgttca gcttcattgc atgtaatgta 420
   gacctagtcc atcaga 436
<210> 487
   <211> 470
   <212> DNA
   <213> Homo sapiens
<220>
   <221> misc_feature
   <222> (63)..(63)
   <223> n is a, c, g, or t
   <220>
   <221> misc_feature
   <222> (83)..(83)
   <223> n is a, c, g, or t
   <400> 487
   tctgaggcta gatatgtctg gctgaagatt tgatgtggtt cctccttaag ctatgcgtcc 60
   tgnttaataa taggtactgt acngggctct gtgtaagtgt cgttggggta ggacctatat 120
   tttaatactg ttcctaacat ttcattttac tagcgagaaa tctttgattt cattttattc 180
   tttgtaattc tagacactag attgtagttt agccataact gatgtttttt aaaaagggat 240
   atattttctt gcacagttgt tcaaaaaaga gacaagtttc agtcctcaat gctgtccttt 300
   gttttacagg tacaagtttt ctagctcaga caaactatga aaaactgtag actattctca 360
   aggtattaac tcgcagaccc tctgggggta ggggctgttt tctaagttac aggcagagtg 420
   ggactgagat ggtacagtgt gcacagacag gtactgagct gacagactgg 470
<210> 488
   <211> 446
   <212> DNA
   <213> Homo sapiens
   <400> 488
   ggcttcattt caagagtcat ccagcaatga gagaatcctg cctctgtaga ccaacatcca 60
   gtgtgatttt gtgtctgaga ccacacccca gtagcaggtt acgccatgtc accgagcccc 120
   attgattccc agagggtctt agtcctggaa agtcaggcca acaagcaacg tttgcatcat 180
   gttatctctt aagtattaaa agttttattt tctaaagttt aaatcatgtt tttcaaaata 240
   tttttcaagg tggctggttc catttaaaaa tcatcttttt atatgtgtct tcggttctag 300
   acttcagctt ttggaaattg ctaaatagaa ttcaaaaatc tctgcatcct gaggtgatat 360
   acttcatatt tgtaatcaac tgaaagagct gtgcattata aaatcagtta gaatagttag 420
   aacaattctt atttatgccc acaacc 446
<210> 489
   <211> 549
   <212> DNA
   <213> Homo sapiens
   <400> 489
   cggtggaggt cttgccggag gtagcagtgg aagctactac tccagcagca gtgggggtgt 60
   cggcctaggt ggtgggctca gtgtgggggg ctctggcttc agtgcaagca gtggccgagg 120
   gctgggggtg ggctttggca gtggcggggg tagcagctcc agcgtcaaat ttgtctccac 180
   cacctcctcc tcccggaaga gcttcaagag ctaagaacct gctgcaagtc actgccttcc 240
   aagtgcagca acccagccca tggagattgc ctcttctagg cagttgctca agccatgttt 300
   tatccttttc tggagagtag tctagaccaa gccaattgca gaaccacatt ctttggttcc 360
   caggagagcc ccattcccag cccctggtct cccgtgccgc agttctatat tctgcttcaa 420
   atcagccttc aggtttccca cagcatggcc cctgctgaca cgagaaccca aagttttccc 480
   aaatctaaat catcaaaaca gaatccccac cccaatccca aattttgttt tggttctaac 540
   tacctccag 549
<210> 490
   <211> 474
   <212> DNA
   <213> Homo sapiens
   <400> 490
   gagggaaggt gattggtagt gagttaaaag aaaaagagag gaaaagagag tagttttgtc 60
   ttcaagtaaa atgtctggtt gtgccagaca tttcacaagt gtgaaaggag ataggagaag 120
   ctcaacttga gggcgtgtag taagttgtag aaggctcgag gggacgtgga cttatttgcc 180
   ttggtttgca atacctgcaa ataatgagtt tgaaaagaaa caatgaaatg tgttaaaaat 240
   ttgaccatat tagataaatt ttggtggatt tagtcataag atggaaaaag actggtgaat 300
   cttttattac aaaatgtttc tgttaaaatg ggatcatcat ctttgaaagg ggggaggagg 360
   agtaaaagcc cgattataat ggtgatcaat tcaagtcagt gttgactatt ctgtgaaata 420
   tatttggcca gtggaaatga taatcagaaa agactgtaaa tagatccatc caaa 474
<210> 491
   <211> 378
   <212> DNA
   <213> Homo sapiens
   <400> 491
   agaacatggt aagcctggta ttttttaatc aaacaaaata tttatgaaat gggttttctc 60
   ttaattctgg attcatcatg gctttctaat accaattgta atatttacaa tattcaccaa 120
   aacttagaat tttgcaaatg caggaattct gccagtgttt ctttgctaag ccttgcatgc 180
   aaaatttgaa attttaacat tggcacccaa aacctacatg gaatgtatgt ctggagtatt 240
   tcaaacttta cattgaaaca taatttcctt ggaaaacaaa ccataagcct gaggaggttt 300
   ttatcaactg gaatgcttta tattagtttg tttttcactg tacattcctc attttacatt 360
   catttaacct gccgatta 378
<210> 492
   <211> 542
   <212> DNA
   <213> Homo sapiens
   <400> 492
   gaaaaagcac ctgaattctc aatgcagggt ctaaaagctg gtgttattgc tgttattgtg 60
   gttgtggtga tagcagttgt tgctggaatt gttgtgctgg ttatttccag aaagaagaga 120
   atggcaaagt atgagaaggc tgagataaag gagatgggtg agatgcatag ggaactcaat 180
   gcataactat ataatttgaa gattatagaa gaagggaaat agcaaatgga cacaaattac 240
   aaatgtgtgt gcgtgggacg aagacatctt tgaaggtcat gagtttgtta gtttaacatc 300
   atatatttgt aatagtgaaa cctgtactca aaatataagc agcttgaaac tggctttacc 360
   aatcttgaaa tttgaccaca agtgtcttat atatgcagat ctaatgtaaa atccagaact 420
   tggactccat cgttaaaatt atttatgtgt aacattcaaa tgtgtgcatt aaatatgctt 480
   ccacagtaaa atctgaaaaa ctgatttgtg attgaaagct gcctttctat ttacttgagt 540
   ct 542
<210> 493
   <211> 456
   <212> DNA
   <213> Homo sapiens
   <400> 493
   tcagcagtat agggaccttc cgcacaagct ctgtgttaag attgacaata atagtggggc 60
   cattttcatt ttagtctttt ctaagagtca accacaggca tttaagtcag ccaaagaata 120
   ttgttacctt aaagcactat tttatttata gatatatcta gtgcatctac atctctatac 180
   tgtacactca cccataattc aaacaattac accatggtat aaagtgggca tttaatatgt 240
   aaagattcaa agtttgtctt tattactata tgtaaattag acattaatcc actaaactgg 300
   tcttcttcaa gagagctaag tatacactat ctggtgaaac ttggattctt tcctataaaa 360
   gtgggaccaa gcaatgatga tcttctgtgg tgcttaagga aacttactag agctccacta 420
   acagtctcat aaggaggcag ccatcataac cattga 456
<210> 494
   <211> 513
   <212> DNA
   <213> Homo sapiens
   <400> 494
   atgctggttt ctgtagggta tttttaattt tgtcagaaat tttagattgt gaatattttg 60
   taaaaaacag taagcaaaat tttccagaat tcccaaaatg aaccagatac cccctagaaa 120
   attatactat tgagaaatct atggggagga tatgagaaaa taaattcctt ctaaaccaca 180
   ttggaactga cctgaagaag caaactcgga aaatataata acatccctga attcaggcat 240
   tcacaagatg cagaacaaaa tggataaaag gtatttcact ggagaagttt taatttctaa 300
   gtaaaattta aatcctaaca cttcactaat ttataactaa aatttctcat cttcgtactt 360
   gatgctcaca gaggaagaaa atgatgatgg tttttattcc tggcatccag agtgacagtg 420
   aacttaagca aattaccctc ctacccaatt ctatggaata ttttatacgt ctccttgttt 480
   aaaatctgac tgctttactt tgatgtatca tat 513
<210> 495
   <211> 492
   <212> DNA
   <213> Homo sapiens
   <400> 495
   tcctgtctat cacaatcagc ctctgaaccc cgcgcccagc agagacccac actaccagga 60
   cccccacagc actgcagtgg gcaaccccga gtatctcaac actgtccagc ccacctgtgt 120
   caacagcaca ttcgacagcc ctgcccactg ggcccagaaa ggcagccacc aaattagcct 180
   ggacaaccct gactaccagc aggacttctt tcccaaggaa gccaagccaa atggcatctt 240
   taagggctcc acagctgaaa atgcagaata cctaagggtc gcgccacaaa gcagtgaatt 300
   tattggagca tgaccacgga ggatagtatg agccctaaaa atccagactc tttcgatacc 360
   caggaccaag ccacagcagg tcctccatcc caacagccat gcccgcatta gctcttagac 420
   ccacagactg gttttgcaac gtttacaccg actagccagg aagtacttcc acctcgggca 480
   cattttggga ag 492
<210> 496
   <211> 536
   <212> DNA
   <213> Homo sapiens
   <400> 496
   ctcaaagagt atatgttccc tccaggtcag ctgcccccaa accccctcct tacgctttgt 60
   cacacaaaaa gtgtctctgc cttgagtcat ctattcaagc acttacagct ctggccacaa 120
   cagggcattt tacaggtgcg aatgacagta gcattatgag tagtgtgaat tcaggtagta 180
   aatatgaaac tagggtttga aattgataat gctttcacaa catttgcaga tgttttagaa 240
   ggaaaaaagt tccttcctaa aataatttct ctacaattgg aagattggaa gattcagcta 300
   gttaggagcc cattttttcc taatctgtgt gtgccctgta acctgactgg ttaacagcag 360
   tcctttgtaa acagtgtttt aaactctcct agtcaatatc caccccatcc aatttatcaa 420
   ggaagaaatg gttcagaaaa tattttcagc ctacagttat gttcagtcac acacacatac 480
   aaaatgttcc ttttgctttt aaagtaattt ttgactccca gatcagtcag agcccc 536
<210> 497
   <211> 555
   <212> DNA
   <213> Homo sapiens
   <400> 497
   aagttactct catcagtcgt tcatggtcac aacctgaggt actctgctga gtgggcaagg 60
   ctgaagtaag aggcctgtgg aatgcagcat tacctgctgg acagagcagg gcaggcagtt 120
   ctatgccttg gagctcctga ctgcagggac tctgtcccca cactcagaaa gactcagctc 180
   actcaatgag agaatgtgat ttactttata gaacgtataa tcaactttgt tgaataattt 240
   gttctattaa ggctgtctaa aatgtgatgt cttcatcata gtatgaagtg ttgaaaatta 300
   ataacgagcc tagtttagga aaaagctgct taaaactgtg gctctaagag agtaatcata 360
   aaatacctta gataaaattg cactatggaa ttttcattga gtatgtttaa attattggct 420
   tgtctactaa tacatctgct tcaaaatgaa catatttcat aaaattggca tcaattttaa 480
   tgacgctcct ggtatggaac ctcagatata ccctattgga gacaatcctt tgatcataaa 540
   ttctccccaa ctata 555
<210> 498
   <211> 507
   <212> DNA
   <213> Homo sapiens
   <400> 498
   gcagaacact gcagtcagat cctgttactt gcttcagtgg accgaaatct gtattctgtt 60
   tgcgtacttg taatatgtat attaagaagc aataactatt tttcctcatt aatagctgcc 120
   ttcaaggact gtttcagtgt gagtcagaat gtgaaaaagg aataaaaaat actgttgggc 180
   tcaaactaaa ttcaaagaag tactttattg caactctttt aagtgccttg gatgagaagt 240
   gtcttaaatt ttcttccttt gaagctttag gcagagccat aatggactaa aacattttga 300
   ctaagttttt ataccagctt aatagctgta gttttccctg cactgtgtca tcttttcaag 360
   gcatttgtct ttgtaatatt ttccataaat ttggactgtc tatatcataa ctatacttga 420
   tagtttggct ataagtgctc aatagcttga agcccaagaa gttggtatcg aaatttgttg 480
   tttgtttaaa cccaagtgct gcacaaa 507
<210> 499
   <211> 213
   <212> DNA
   <213> Homo sapiens
   <400> 499
   acttttgtat cttttatcct gggagcactg cgttttccta gctgtgttat tcctggttta 60
   attcagcaga gaaggtaagg tgtgaaccta cctgccttgg agaggcccag gtcccaaatc 120
   tcttcaaatt cttcacatgt ttaactttaa ggatttgaac catgaagtca taggttacag 180
   acctcagttt tatgccccat tggattactt ttt 213
<210> 500
   <211> 173
   <212> DNA
   <213> Homo sapiens
   <400> 500
   tttcttttga ggcatgcaca tctggaatta aggtcaaact aattctcaca tccctctaaa 60
   agtaaactac tgttaggaac agcagtgttc tcacagtgtg gggcagccgt ccttctaatg 120
   aagacaatga tattgacact gtccctcttt ggcagttgca ttagtaactt tga 173
<210> 501
   <211> 531
   <212> DNA
   <213> Homo sapiens
   <400> 501
   ctgttagctc ctcactgtgg taaatgccac acacctttaa gtagataagc agacgatagt 60
   tatctgttct tttgacttaa tctcatttgg tttgattttc cctctactaa ggctttccta 120
   ccttcttcag gctgcctaag acatgtaagc gaaacacttc aataattgtc catgaggaga 180
   aaaaaagcat tgtcatgcat gaaggaaact gaacttgagg tggcctcctt gcttgttaca 240
   tacctgggta tgtgtaggca gtttagtgca tctttgcctc tcagttgaaa cctgtataac 300
   cctgttacaa agctgtgttg ttgcttcttg tgaaggccat gatattttgt tttttcccca 360
   attaattgct attgtgttat tttactaact tctctctgta ttttttcttg cattgacatt 420
   atagacattg aggacctcat ccaaacaatt taaaaatgag tgtgaagggg gaacaagtca 480
   aaatattttt aaaagatctt caaaaataat gcctctgtct agcatgccaa c 531
<210> 502
   <211> 511
   <212> DNA
   <213> Homo sapiens
   <400> 502
   aagagaatgt tcctactcac acttcagctg ggtcacatcc atccctccat tcatccttcc 60
   atccatcttt ccatccatta cctccatcca tccttccaac atatatttat tgagtaccta 120
   ctgtgtgcca ggggctggtg ggacagtggt gacatagtct ctgccctcat agagttgatt 180
   gtctagtgag gaagacaagc atttttaaaa aataaattta aacttacaaa ctttgtttgt 240
   cacaagtggt gtttattgca ataaccgctt ggtttgcaac ctctttgctc aacagaacat 300
   atgttgcaag accctcccat gggggcactt gagttttggc aaggctgaca gagctctggg 360
   ttgtgcacat ttctttgcat tccagctgtc actctgtgcc tttctacaac tgattgcaac 420
   agactgttga gttatgataa caccagtggg aattgctgga ggaaccagag gcacttccac 480
   cttggctggg aagactatgg tgctgccttg c 511
<210> 503
   <211> 324
   <212> DNA
   <213> Homo sapiens
   <400> 503
   gtatgacaac ccgggatcgt ttgcaagtaa ctgaatccat tgcgacattg tgaaggctta 60
   aatgagttta gatgggaaat agcgttgtta tcgccttggg tttaaattat ttgatgagtt 120
   ccacttgtat catggcctac ccgaggagaa gaggagtttg ttaactgggc ctatgtagta 180
   gcctcattta ccatcgtttg tattactgac cacatatgct tgtcactggg aaagaagcct 240
   gtttcagctg cctgaacgca gtttggatgt ctttgaggac agacattgcc cggaaactca 300
   gtctatttat tcttcagctt gccc 324
<210> 504
   <211> 122
   <212> DNA
   <213> Homo sapiens
   <400> 504
   cttgcccttt gtacacaagt tcccagggtg agcagctttt ggatttaata tgaacatgta 60
   cagcgtgcat agggactctt gccttaagga gtgtaaactt gatctgcatt tgctgatttg 120
   tt 122
<210> 505
   <211> 444
   <212> DNA
   <213> Homo sapiens
   <400> 505
   gaagccctgg aaaatcgcct gagatacaga tgaagattag aaatcgcgac acatttgtag 60
   tcattgtatc acggattaca atgaacgcag tgcagagccc caaagctcag gctattgtta 120
   aatcaataat gttgtgaagt aaaacaatca gtactgagaa acctggtttg ccacagaaca 180
   aagacaagaa gtatacacta acttgtataa atttatctag gaaaaaaatc cttcagaatt 240
   ctaagatgaa tttaccaggt gagaatgaat aagctatgca aggtattttg taatatactg 300
   tggacacaac ttgcttctgc ctcatcctgc cttagtgtgc aatctcattt gactatacga 360
   taaagtttgc acagtcttac ttctgtagaa cactggccat aggaaatgct gtttttttgt 420
   actggacttt accttgatat atgt 444
<210> 506
   <211> 212
   <212> DNA
   <213> Homo sapiens
   <400> 506
   cattcctagc cgagtgtgac acagtggagc agaacatctg ccaggagact gagcggctgc 60
   agtctacaaa ctttgccctg gccgagtgag gtgtagcaga aaaaggctgt gctgccctga 120
   agaatggcgc caccagctct gccgtctctg gatcggaatt tacctgattt cttcagggct 180
   gctgggggca actggccatt tgccaatttt cc 212
<210> 507
   <211> 433
   <212> DNA
   <213> Homo sapiens
   <400> 507
   gccagcgctc tgacatgcag aaggtgaccc tgggcctgct tgtgttcctg gcaggctttc 60
   ctgtcctgga cgccaatgac ctagaagata aaaacagtcc tttctactat gactggcaca 120
   gcctccaggt tggcgggctc atctgcgctg gggttctgtg cgccatgggc atcatcatcg 180
   tcatgagtgc aaaatgcaaa tgcaagtttg gccagaagtc cggtcaccat ccaggggaga 240
   ctccacctct catcacccca ggctcagccc aaagctgatg aggacagacc agctgaaatt 300
   gggtggagga ccgttctctg tccccaggtc ctgtctctgc acagaaactt gaactccagg 360
   atggaattct tcctcctctg ctgggactcc tttgcatggc agggcctcat ctcacctctc 420
   gcaagagggt ctc 433
<210> 508
   <211> 442
   <212> DNA
   <213> Homo sapiens
   <400> 508
   ctcagcgagc actgagctgg ccctacttcc aggatggatg catcacactc aaggacagga 60
   gcctggtcct tccctgatgg cctttggacc cagggcctga cttgagccac tccttccttc 120
   aggactctgc gggaggctgg ggccccatct tgatctttga gcccattctt ctgggtgtgc 180
   tttttgggac catcactgag agtcaggagt tttactgcct gtagcaatgg ccagagcctc 240
   tggcccctca cccaccatgg accagcccat tggccgagct cctggggagc tcctgggacc 300
   cttggctatg aaaatgagcc ctggctccca cctgtttctg gaagactgct cccggcccgc 360
   ctgcccagac tgatgagcac atctctctgc cctctccctg tgttctgggc tggggccacc 420
   tttgtgcagc ttcgaggaca gg 442
<210> 509
   <211> 536
   <212> DNA
   <213> Homo sapiens
   <400> 509
   aatctgaaga ttaaccattt ttttgtctta gaatatcaaa aagaaaaaga aaaaggtgtt 60
   ctagctgttt gcatcaaagg aaaaaaagat ttattatcaa ggggcaatat ttttatcttt 120
   tccaaaataa atttgttaat gatacattac aaaaatagat tgacatcagc ctgattagta 180
   taaattttgt tggtaattaa tccattcctg gcataaaaag tctttatcaa aaaaaattgt 240
   agatgcttgc tttttgtttt ttcaatcatg gccatattat gaaaatacta acaggatata 300
   ggacaaggtg taaatttttt tattattatt ttaaagatat gatttatcct gagtgctgta 360
   tctattactc ttttactttg gttcctgttg tgctcttgta aaagaaaaat ataatttcct 420
   gaagaataaa atagatatat ggcacttgga gtgcatcata gttctacagt ttgtttttgt 480
   tttcttcaaa aaagctgtaa gagaattatc tgcaacttga ttcttggcag gaaata 536
<210> 510
   <211> 325
   <212> DNA
   <213> Homo sapiens
   <400> 510
   atatgtattc attcactttc aagatttgtt ttggtgtcaa aataacatga aaaggtagat 60
   ggagttgctt ctgttgaatt agctctgcca ccaatatgta tcttcataca cgtttggaaa 120
   tgtttcctgc agcattaggt atgacttgtt ctgagtactg cttccggtgc taaaatgaac 180
   aaagaatttg tacttaatgg catggactct ggagaatcta tgcgaatcaa cctttctacc 240
   ttaatatctc cccaaaaatg tatagtgcct tgtttttatg tacagtttat atacagaaaa 300
   gtttgctctg catttttgat gatgg 325
<210> 511
   <211> 555
   <212> DNA
   <213> Homo sapiens
   <400> 511
   tgggaggccc tgtaagagcc tggtgaaatg ggagagtgag aataaaatgg tctgtgagca 60
   gaagctcctg aagggagagg gccccaagac ctcgtggacc agagaactga ccaacgatgg 120
   ggaactgatc ctgaccatga cggcggatga cgttgtgtgc accagggtct acgtccgaga 180
   gtgagtggcc acaggtagaa ccgcggccga agcccaccac tggccatgct caccgccctg 240
   cttcactgcc ccctccgtcc caccccctcc ttctaggata gcgctcccct taccccagtc 300
   acttctgggg gtcactggga tgcctcttgc agggtcttgc tttctttgac ctcttctctc 360
   ctcccctaca ccaacaaaga ggaatggctg caagagccca gatcacccat tccgggttca 420
   ctccccgcct ccccaagtca gcagtcctag ccccaaacca gcccagagca gggtctctct 480
   aaaggggact tgagggcctg agcaggaaag actggccctc tagcttctac cctttgtccc 540
   tgtagcctat acagt 555
<210> 512
   <211> 513
   <212> DNA
   <213> Homo sapiens
   <400> 512
   ttccttgttt tggcttcttt tcagaatgcc gggagagtac atgcagggat tccatctaat 60
   caccctcagc actctttctc tggtcctgct ggatagattt agatttcctt tcttttttta 120
   gggcctcagt ctgctatctc ctttggtggc taccaccact cactcccttg atatcttcta 180
   ctcccttgcc ttcaccttgc ttaagactga gaagggagtt agattttgtc actagctctt 240
   ctttttcctc actgtgtacc ccaccaaaca agattagttc aagttaaaaa gaacctactg 300
   gaggtaaact gggagagcaa gtgttggatc tgggctggtc cctttcccat aaaattaggt 360
   ccctggttgt atgttcccat agcaccccat acttcctctc tcagaataat catttccctt 420
   gtaatgctca gcatccgcat cctgcttgac tgcaaacttg ctgaaggtag ggactgtttg 480
   tcttggactt cgctgccagt ccttagaaca gtg 513
<210> 513
   <211> 519
   <212> DNA
   <213> Homo sapiens
<220>
   <221> misc_feature
   <222> (46)..(46)
   <223> n is a, c, g, or t
   <220>
   <221> misc_feature
   <222> (117)..(118)
   <223> n is a, c, g, or t
   <400> 513
   ggaatttgca ccatcatgtt tcagtgaaga tgctgtaaat aggttnagat tttactgtct 60
   atggatttgg ggtgttacag tagccttatt caccttttta ataaaaatac acatgannac 120
   aagaaagaaa tggcttttct tacccagatt gtgtacatag agcaatgttg gttttttata 180
   aagtctaagc aagatgtttt gtataaaatc tgaattttgc aatgtattta gctacagctt 240
   gtttaacggc agtgtcattc ccctttgcac tgtaatgagg aaaaaatggt ataaaaggtt 300
   gccaaattgc tgcatatttg tgccgtaatt atgtaccatg aatatttatt taaaatttcg 360
   ttgtccaatt tgtaagtaac acagtattat gcctgagtta taaatatttt tttctttctt 420
   tgttttattt taatagcctg tcataggttt taaatctgct ttagtttcac attgcagtta 480
   gccccagaaa atgaaatccg tgaagtcaca ttccacatc 519
<210> 514
   <211> 563
   <212> DNA
   <213> Homo sapiens
   <400> 514
   agagcttcct gatctgggtg aatgaggagg atcatacacg ggtgatctcc atggagaagg 60
   gtggtaacat gaagagagtg tttgaaagat tctgccgagg cctcaaagag gtggagagac 120
   ttatccaaga acgtggctgg gagttcatgt ggaatgagcg tttgggatac atcttgacct 180
   gtccatctaa cctgggcact ggacttcggg caggagtgca catcaaactg cccctgctaa 240
   gcaaagatag ccgcttccca aagatcctgg agaacctaag actccaaaaa cgtggtactg 300
   gaggagtgga cactgctgct acaggcggtg tctttgatat ttctaatttg gaccgactag 360
   gcaaatcaga ggtggagctg gtgcaactgg tcatcgatgg agtaaactat ttgattgatt 420
   gtgaacggcg tctggagaga ggccaggata tccgcatccc cacacctgtc atccacacca 480
   agcattaact ccccatcgcc agctgatgac tcaagattcc caggagtttt gctcattcta 540
   atgatggccc attctacttg ctc 563
<210> 515
   <211> 549
   <212> DNA
   <213> Homo sapiens
<220>
   <221> misc_feature
   <222> (76)..(76)
   <223> n is a, c, g, or t
   <400> 515
   aaactactaa ccactgcaag ctcttgtcaa attttagttt aattggcatt gcttgttttt 60
   tgaaactgaa attacntgag tttcattttt tctttgaatt tatagggttt agatttctga 120
   aagcagcatg aatatatcac ctaacatcct gacaataaat tccatccgtt gttttttttg 180
   tttgtttgtt ttttcttttc ctttaagtaa gctctttatt catcttatgg tgcagcaatt 240
   ttaaaatttg aaatatttta aattgttttt gaactttttg tgtaaaatat atcagatctc 300
   aacattgttg gtttcttttg tttttcattt tgtacaactt tcttgaattt agaaattaca 360
   tctttgcagt tctgttaggt gctctgtaat taacctgact tatatgtgaa caattttcat 420
   gagacagtca tttttaacta atgcagtgat tctttctcac tactatctgt attgtggaat 480
   gcacaaaatt gtgtaggtgc tgaatgctgt aaggagttta ggttgtatga attctacaac 540
   cctataata 549
<210> 516
   <211> 443
   <212> DNA
   <213> Homo sapiens
   <400> 516
   agaagtctca gctaagctca cgtcctgaga aagctcaaag gtttggaagg agcagaaaac 60
   ccttgggcca gaagtaccag actagatgga cctgcctgca taggagtttg gaggaagttg 120
   gagttttgtt tcctctgttc aaagctgcct gtccctaccc catggtgcta ggaagaggag 180
   tggggtggtg tcagaccctg gaggccccaa ccctgtcctc ccgagctcct cttccatgct 240
   gtgcgcccag ggctgggagg aaggacttcc ctgtgtagtt tgtgctgtaa agagttgctt 300
   tttgtttatt taatgctgtg gcatgggtga agaggagggg aagaggcctg tttggcctct 360
   ctgtcctctc ttcctcttcc cccaagattg agctctctgc ccttgatcag ccccaccctg 420
   gcctagacca gcagacagag cca 443
<210> 517
   <211> 516
   <212> DNA
   <213> Homo sapiens
   <400> 517
   aatgatggaa tgttgactgt gtttggcaca caggacacgg accttcatgg aagtccttgc 60
   tctgcgtggc atctgtcagc ttttcacctt tcattcttat tcttcacttt tgctgctgag 120
   cctagctgta caaacttgca ctttcatttg ctaatataaa ttcagtttta ttttaccatt 180
   ttagagacta ctaatgatta aatgtagaag gagagggtgc acatgttttt atgtggagtg 240
   tttaaaagat aaatttatac cactgtaatg tgcagctttt attaaaagag aaattggttg 300
   aactgctagg ttgaatgaga gacttcatct attggactat tttttttaat ccaggcatat 360
   ggtctttagt aatggcttgt aatttgtgaa aacattaatt tgggggtttt ccctgttttc 420
   agttgtccat gtacacatag tcattatatt agaaaagaaa gctgttcaac aaacttgttt 480
   aatttgttta aatcaacata gcatgaaaca ccaaat 516
<210> 518
   <211> 516
   <212> DNA
   <213> Homo sapiens
   <400> 518
   gtagtgtatc actgagtcat ttgcagtgtt ttctgccaca gacctttggg ctgccttata 60
   ttgtgtgtgt gtgtgggtgt gtgtgtgttt tgacacaaaa acaatgcaag catgtgtcat 120
   ccatatttct ctacatcttc tcttggagtg agggaggcta cctggagggg atcagcccac 180
   tgacagacct taatcttaat tactgctgtg gctagagagt ttgaggattg ctttttaaaa 240
   aagacagcaa actttttttt ttatttaaaa aaagatatat taacagtttt agaagtcagt 300
   agaataaaat cttaaagcac tcataatatg gcatccttca atttctgtat aaaagcagat 360
   ctttttaaaa aagatacttc tgtaacttaa gaaacctggc atttaaatca tattttgtct 420
   ttaggtaaaa gctttggttt gtgttcgtgt tttgtttgtt tcacttgttt ccctcccagc 480
   cccaaacctt ttgttctctc cgtgaaactt accttt 516
<210> 519
   <211> 379
   <212> DNA
   <213> Homo sapiens
<220>
   <221> misc_feature
   <222> (170)..(170)
   <223> n is a, c, g, or t
   <400> 519
   aatgcgaagg ctaagtgtca ccccctttct ctgcctctgg ctgggccttg ctaagggcca 60
   aggaaagaaa gacatttttt agggggcagc cagtccaaat gccaaaagaa gaccagttct 120
   tgccctgatt gtatgaaatt tgacattttg gcactttttt tttttttttn ggccaatcag 180
   attttctatg ttctaaggac atggctgctg tagaatagca cagacgtgga tgataaatta 240
   tccccagaag cagcatgaca gaatgcctcg gggagcactt ggaagggaaa ttgcagttct 300
   gttgaaatag aggaaaatcc cttggtaaag acacagcctg ttaggctcgt gtgggcctcc 360
   agtatgttca ccaggggaa 379
<210> 520
   <211> 466
   <212> DNA
   <213> Homo sapiens
   <400> 520
   agtagtgcct gtggtttagc ccaccaatct tgatgactaa aagtagctga tgcattgtgc 60
   atatgatgct tgagatggtt tttgcaaaag cagaaatcgc tgcaaggtaa tcacaataga 120
   taaaagtggt attttaaacc tttgaaataa atggatgtaa ctgtaccttg gtacagcttt 180
   tcacttgttt agtttttaaa cgttagtata atctgaataa ataaaatgtt gccaaattca 240
   atgtagaaag aatgtgacaa cacaccttgg gtagttctgc ttgtgttttt gcatattgta 300
   aaagcagtgt cacagctaaa aagaaagaaa tcgtttctaa cagtaaatta ttgtgcttta 360
   gttgctagtt tgtactgaga gttgacctct ccctgtgcag ttttttgttc taaacttgta 420
   taaataacaa ttgtgtaatg tgtctccctc ctacattgta acaatt 466
<210> 521
   <211> 547
   <212> DNA
   <213> Homo sapiens
   <400> 521
   tggggacttg tggatcattc cttcctccct gcaggagctt cccaagctgg tcacagagtc 60
   tcctgggcac aggttataca gaccccagcc ccattcccat ctactgaaac agggtctcca 120
   caagaggggc cagggaatat gggtttttaa caagcgtctt acaaaacact tctctatcat 180
   gcagccggag agctggctgg gagccctttt gttttagaac acacatcctt cagcagctga 240
   gaaatgaaca cgaatccatc ccaaccgaga tgccattaac attcatctaa aaatgttagg 300
   ctctaaatgg acgaaaaatt ctctcgccat cttaataaca aaataaacta caaattcctg 360
   acccaaggac actgtgttat aagaggcgtg ggctcccctg gtggctgacc aggtcagctg 420
   ccctggcctt gcacccctct gcatgcagca cagaagggtg tgaccatgcc ctcagcacca 480
   ctcttgtccc cactgaacgg caactgagac tgggtacctg gagattctga agtgcctttg 540
   ctgtggt 547
<210> 522
   <211> 502
   <212> DNA
   <213> Homo sapiens
   <400> 522
   gcatcaggct aagaccctgt gtcctccacc atgcactcac ccctagccct ggttagctga 60
   cagtcagctg tggggaacac agctacaacc ctaccctggc agggacctga gagcatctca 120
   ggaggggcag cgcatgtgtg catgtgctgt gtgagtgagc acacccgtgt gcacactcat 180
   acacatgtgc acacacacgc actctccccg ctcaggggcc tggaggtctg gctgagcccc 240
   tggggaaagg tgagttcttt catctccctc ctccaggtcg gagtgcctgg agtcaggtgt 300
   cgaggccaca ttgctggctg ccccctcttt gtagctccta taaagggccc acacctggtg 360
   gatacctggt tgagcgtgtg gtctctgccc cagcctgtcc ttgtcacgat cacaggcctt 420
   gcttttgtaa caatgatgac cccggcctgt ctcatcttct gaagaggaaa agtcaaagtg 480
   ttgctgtggc tccatatttc aa 502
<210> 523
   <211> 387
   <212> DNA
   <213> Homo sapiens
   <400> 523
   gtgatagaca cttcgggtgg acccctcgac ctcatggctt gcagagtggt tgcggccagc 60
   acccgggaga tggcgatgct catagctcag gccttacaga cgattaacta tgggcgggat 120
   gatgagaagt gactgcggct gaggcaaagc tgctcccaag gcctccctgg gctgctgtgg 180
   gctcctgggg aggtggccct cgtggcccac gctccatgcc agtggctcac gctctgctcc 240
   tggctacccc agagggagtt gtcacgctac agtgagtggc tggcctttta aatcgacgtc 300
   tctctcacca ggatttggtg tttagctgtt tctctcttta atctcacgta gcctttttca 360
   ggttagtacg tgttcttctg tcagggc 387
<210> 524
   <211> 320
   <212> DNA
   <213> Homo sapiens
   <400> 524
   gtgaattttc catgaatgtt tttaatattc tcatctcaac attgtgatat atgctactaa 60
   aaaccttttc atatacatct tacctcattt caagtgaatt attttaatct ttttctctct 120
   ttccaaaaat ttacaggaat gtttagtgta attggatttc gctatcagtt cccatcctta 180
   agttttgata ttcaatatct gatagataca ctgcatcttt ggtcatctaa gatttgttta 240
   caaatgtgca aattatttag agcatagact ttataagcat taaaaaaaac taatggaggt 300
   aaaacctaaa tgcgatgtga 320
<210> 525
   <211> 543
   <212> DNA
   <213> Homo sapiens
   <400> 525
   ccaggactac agaataccat cccctggtac cgtgtagttg ccgaagtcca gatctgccat 60
   ggcaaaacgg aggctgtggg ccaggtccac atcttcttcc aggatgggat ggtgacgttg 120
   actccaaaca agggtgtgtg ggtgaatggt ctccgagtgg atctcccagc tgagaagtta 180
   gcatctgtgt ccgtgagtcg tacacctgat ggctccctgc tagtccgcca gaaggcaggg 240
   gtccaggtgt ggcttggagc caatgggaag gtggctgtga ttgtcagcaa tgaccatgct 300
   gggaaactgt gtggggcctg tggaaacttt gacggggacc agaccaatga ttggcatgac 360
   tcccaggaga agccagcgat ggagaaatgg agagcgcagg acttctcccc atgttatggc 420
   tgatcagtca tccaccagga acgaagattt cctgaagaag acctggtccc tctggaggtt 480
   gcggtggctg aaggatgcat catgtgctcc taccctgctc taccgctttt ctgggtcaca 540
   gag 543
<210> 526
   <211> 541
   <212> DNA
   <213> Homo sapiens
   <400> 526
   tcatcacttc cttctggttt tatgtatttg tagactatgc agcttttcat taaactgcaa 60
   gtatatacaa gacagatctg aaattaggcc tgagtgttcc gatccaccac tgtactagta 120
   aataaaaatc cacctacctt ttatgtggaa aattatgtgc tattgagtaa cttttagctc 180
   ttttttaaaa aatgggtgaa atttaagtgt cttttttatg agaatgacac atgaagagat 240
   ctgagagcaa tctcatgtag tcttccatga acctgcaatt gtttggtatg cgtcagcatt 300
   ttccaatttc caggttggat ctagagctgc tgttgatcac tcaggcatac taatggattc 360
   atttagatgg gtccaagctg cagtccatga gcaataacag actaccccag atactgcagt 420
   ttacgcagtg cttagtaaat gagatttgtg gaactaagtt attagttacc tgaggcttct 480
   taagaaagtc ttcttttttg accagttgat gtgaaagagg gagcatgtga cacagccagt 540
   a 541
<210> 527
   <211> 543
   <212> DNA
   <213> Homo sapiens
   <400> 527
   gacagtttga cttgaatgca acagcaggaa aattttgcaa gttacataat tgtatataca 60
   gtaggttttc ttaagtctct tcggttcatc ctttgtaatt tgtgtgtgta tctgtagtat 120
   tgcaggcttt tggagactat tcttacaggc agtatgtcag tcatcaaaga aaatgctgtc 180
   acctgccatt gttgtatttg tgggtattta tagttgtatg tatgtaaatg catcagtgtg 240
   tagattgcat atcagtgtat ggtacatgta catcaaaatt atttttgtcc ttaatcagtg 300
   tgatatgaaa agcaagtaca acctcatagg actgattata taatgaagtt gttgagagta 360
   tatatagtgg tattgtttta ttaaacttaa actcaaataa tattttgatt aaaattttta 420
   ataagacttt atgctagaaa attctttgag ctttgaatca ccagggcaaa aatgactttc 480
   aactaacctt gtgaatcttt tgcagtgtac tgtgtgcaat accaagggca tagctccctg 540
   taa 543

<210> 528
   <211> 520
   <212> DNA
   <213> Homo sapiens
   <400> 528
   tcccagcaac aaactcctca tgataactgc acacaatctg aaaaccactg aaggacaagc 60
   caaccacagc agccaagccc actccttgca gcatgggtac tggtggcaca ccagacagtg 120
   acactgcccc acaaaggcct gggcccgtgg gggctgctgc ctggcatgac atctctccag 180
   atttctggct taaaaccaac tttccatccg agaagcctcc tcagtagtta ctctgctcat 240
   gagacagatc tgggctccaa gccaggaaag gtgaacagaa accacaagtg tccagccctc 300
   ggtgctggag tggacgttaa ttgtcagcca ccagactgtc ccggcaccta cagagaatgt 360
   ttcacagttc tggcatttaa atcctttgat agtggattgt gctgctgtta gccttagttt 420
   cagtgcttta caagtctcgc ttattatctc attggtattt aggtatacaa aacagttgat 480
   tattcaccac gccaatatct gggtctctgt atctcatgta 520
<210> 529
   <211> 358
   <212> DNA
   <213> Homo sapiens
   <400> 529
   aaatgaaaag tccaccttgt cttctctcag aaaacctttg ttgttcattg tttggccaat 60
   gaatcttcaa aaacttgcac aaacagaaaa gttggaaaag gataatacag actgcactaa 120
   atgttttcct ctgttttaca aactgcttgg cagccccagg tgaagcatca aggattgttt 180
   ggtattaaaa tttgtgttca cgggatgcac caaagtgtgt accccgtaag catgaaacca 240
   gtgttttttg tttttttttt agttcttatt ccggagcctc aaacaagcat tataccttct 300
   gtgattatga tttcctctcc tataattatt tctgtagcac tccacactga tctttgga 358
<210> 530
   <211> 451
   <212> DNA
   <213> Homo sapiens
   <400> 530
   gacaagctac gtggagcctg gttcaggtcc ttttagtgag tctaccatta ccatttccct 60
   gtatattccc tctgaacagc aatttgatcc acccaggcct ttagagtcag atgtcttcat 120
   tgaagataga gccgaaatga ctgtgtttgt acggtctttc gatggatttt ctagtgccca 180
   aaagaatcaa gaacaacttt tgacattagc aagcatttta agggaagatg gaaaagtttt 240
   cgatgagaag gtttactaca ctgcaggcta caacagtcct gtcaaattgc ttaatagaaa 300
   taatgaagtg tggttgattc aaaaaaatga acccaccaaa gaaaacgaat gagaaaaatg 360
   aaaggaagtt ctgctgtcag aggcaaaaca tctgtttatc atagacatca acatgaccta 420
   taagtaaagt gcgtgtctag tgtcttctat t 451
<210> 531
   <211> 440
   <212> DNA
   <213> Homo sapiens
   <400> 531
   gactcccgag ggctagggct agagcagacc cgggtaagta aaggcagacc cagggctcct 60
   ctagcctcat acccgtgccc tcacagagcc atgccccggc acctctgccc tgtgtctttc 120
   atacctctac atgtctgctt gagatatttc ctcagcctga aagtttcccc aaccatctgc 180
   cagagaactc ctatgcatcc cttagaaccc tgctcagaca ccattacttt tgtgaacgct 240
   tctgccacat cttgtcttcc ccaaaattga tcactccgcc ttctcctggg ctcccgtagc 300
   acactataac atctgctgga gtgttgctgt tgcaccatac tttcttgtac atttgtgtct 360
   cccttcccaa ctagactgta agtgccttgc ggtcagggac tgaatcttgc ccgtttatgt 420
   atgctccatg tctagcccat 440
<210> 532
   <211> 225
   <212> DNA
   <213> Homo sapiens
   <400> 532
   aagcagtcga ccgcacttat ggtaatcagt tttgtataac ttaaaataat taaataaatg 60
   aataaatcca aaacaaacat gcagtacttt tgttgtatgg gattggtggg ctgatttaca 120
   tgtatggtta ctaaaaagta ccagcatgtt aactttatta caatttgtat tactttctct 180
   gtagttccta atggattcaa ttacggactc tggatatttg cactt 225
<210> 533
   <211> 436
   <212> DNA
   <213> Homo sapiens
   <400> 533
   tcctgatgtg ccagaacttc gaccctttct ctgagagaga tgatcgtgcc tataaatagt 60
   aggaccaatg ttgtgattaa catcatcagg cttggaatga attctctcta aaaataaaat 120
   gatgtatgat ttgttgttgg catccccttt attaattcat taaatttctg gatttgggtt 180
   gtgacccagg gtgcattaac ttaaaagatt cactaaagca gcacatagca ctgggaactc 240
   tggctccgaa aaactttgtt atatatatca aggatgttct ggctttacat tttatttatt 300
   agctgtaaat acatgtgtgg atgtgtaaat ggagcttgta catattggaa aggtcattgt 360
   ggctatctgc atttataaat gtgtggtgct aactgtatgt gtctttatca gtgatggtct 420
   cacagagcca actcac 436
<210> 534
   <211> 127
   <212> DNA
   <213> Homo sapiens
   <400> 534
   agataccccg aagccatggc aagcaagggc ttgcaggacc tgaagcaaca ggtggagggg 60
   accgcccagg aagccgtgtc agcggccgga gcggcagctc agcaagtggt ggaccaggcc 120
   acagagg 127
<210> 535
   <211> 517
   <212> DNA
   <213> Homo sapiens
   <400> 535
   ataaaatgtc tacgtctttc tccagtttct gagccctatg cacattggct tgtgggcttg 60
   ttcttcctgc caaatgatca gagagggaac attccattta tttgtagtgg atttcctctg 120
   gagggcatgt acccacacta aataccaact gctcttcctc agctgtagtc cccaacatca 180
   gacttggcac gtggtggaca ctaacacaca ggcactcaat gaatgagtga aggaaataaa 240
   agtcaccccc cgttggtgag aaggtgccta tccccctgag tcctcagtgc aggaccagtg 300
   gatgaaaggc aaggtaaaga ggcccaagat aggctggctt cccccgttca aggtatagtc 360
   tgcctttaag ggagttttag aaccaacatg caagacattg aaagaaatct tgcaagagcc 420
   attattgact tagatccaaa acagcctctc tcatgtctaa aaaggcacag aattttgcag 480
   atctgaggaa gagggatgca ttaccttttt gcttctt 517
<210> 536
   <211> 512
   <212> DNA
   <213> Homo sapiens
<220>
   <221> misc_feature
   <222> (30)..(30)
   <223> n is a, c, g, or t
   <220>
   <221> misc_feature
   <222> (34)..(34)
   <223> n is a, c, g, or t
   <220>
   <221> misc_feature
   <222> (473)..(473)
   <223> n is a, c, g, or t
   <400> 536
   gttgtcgcag ccggggaagg gaagctaccn agcnatctag tgcgtagagg tcatggacgc 60
   cgttaaacat cctacagtgc aagcgcagcc cccgaccacg aagagttgtc ttgctcaaat 120
   atcaacagtg ctgcagtgta gaaacttgat cgttggtttt cttttaatgc aaaactctca 180
   taaaaacctt tcacttttcc tgtcattgat tatatgcttg atacacccaa aaagaaaagg 240
   ggaggggcac caattcacct acactccagt ggctccatca cctttaaaaa tatttataaa 300
   atagttccaa aaatctgata tctgaaaagc aatccaagcc tgtgtaaatg ggaatcactg 360
   ataagtatca tcatctgtat cagcttggct tggacatgaa aaattgattc tctttatgtc 420
   actccttgca cctggacaaa ttcaatcccc ggtacttaag tcacactgcc aanccctcgg 480
   ccctgactat tgtcttgatt gctgttcctt tc 512
<210> 537
   <211> 245
   <212> DNA
   <213> Homo sapiens
   <400> 537
   ctgtcacaaa tagcagcacc actttggatt gattttgctc tccaggacat cagcacatgg 60
   ccctgatcag cactaccaca tccaaacata agtcactgaa aaacacttaa tatttatgag 120
   ttggtaatga caagggacat tgtataaagt actatttgct agattcatgc ctcaaaagtt 180
   attataaaca gacctttatt aaacacatct tgaaagatgt agaagtccct ctatagtcta 240
   gtata 245
<210> 538
   <211> 435
   <212> DNA
   <213> Homo sapiens
   <400> 538
   caacgtctaa ctggacttcc caagataaat ggtaccagcg tcctcttaaa agatgcctta 60
   atccattcct tgaggacaga ccttagttga aatgatagca gaatgtgctt ctctctggca 120
   gctggccttc tgcttctgag ttgcacatta atcagattag cctgattctc ttcagtgaat 180
   tttgataatg gcttccagac tctttgcgtt ggagacgcct gttaggatct tcaagtccca 240
   tcatagaaaa ttgaaacaca gagttgttct gctgatagtt ttggggatac gtccatcttt 300
   ttaagggatt gctttcatct aattctggca ggacctcacc aaaagatcca gcctcatacc 360
   tacatcagac aaaatatcgc cgttgttcct tctgtactaa agtattgtgt tttgctttgg 420
   aaacacccac tcact 435
<210> 539
   <211> 498
   <212> DNA
   <213> Homo sapiens
   <400> 539
   caggaggcca tgactacatc acagccaggc ggcattccct gccacagtgg cggcttgaat 60
   catcaagaaa tggataaatg gggctttagt aaatcaggct tgcaggctca aagctgcaat 120
   ctgcccactc tcaggtactg agactttgtg ggcctcagac accaggaaga aagttgggat 180
   acagtcattt gagttaaaaa gggaatgacc cctcagaaac ccacattagc agtgttactc 240
   ttggaactgc ctttactttt aacgctctct gttctgaaaa agaggtgttt ggttacgtgt 300
   gagccaacat cacgttttgt tagctgtgat ttacctttgt ccgtttaaaa gacttcacgg 360
   agccattctg tatacaaggt gtgctctttc caatgtagaa ggggttatgg aaaagggtgc 420
   gatcctttgc tgtaaactgg agagaccagt cccaaacaga ggggaatttt aagcccttct 480
   catcacccaa ttggatgt 498
<210> 540
   <211> 474
   <212> DNA
   <213> Homo sapiens
   <400> 540
   cctgaggggc ctcttatggg ctgggttcta cccaggtgct aggaacactc cttcacagat 60
   gggtgcttgg aggaaggaaa cccagctctg gtccatagag agcaaaacgc tgtgctgccc 120
   tgcccaccct ggcctctgca ctcccctgct gggtgtggcg cagcatattc aggaagctca 180
   gggccctggc tcaggtgggg tcactctggc agctcagaga gggtgggagt gggtccaatg 240
   cactttgttc tggctcttcc aggctgggag agcctttcag gggtgggaca ccctgtgatg 300
   gggccctgcc tcctttgtga ggaagccgct ggggccagtt ggtccccctt ccatggactt 360
   tgttagtttc tccaagcagg acatggacaa ggatgatcta ggaagacttt ggaaagagta 420
   ggaagacttt ggaaagactt ttccaaccct catcaccaac gtctgtgcca tttt 474
<210> 541
   <211> 437
   <212> DNA
   <213> Homo sapiens
   <400> 541
   tggcactcgg tggcagtcac cataaaacaa cacatcctgc acctggaact ggacacagac 60
   agtagctaca cagctggaca gatccccttc ccacctgcca gcactcaaga gccactacac 120
   cttggaggtg ctccagccaa tttgacgaca ctgaggatcc ctgtgtggaa atcattcttt 180
   ggctgtctga ggaatattca tgtcaatcac atccctgtcc ctgtcactga agccttggaa 240
   gtccaggggc ctgtcagtct gaatggttgt cctgaccagt aacccaagcc tatttcacag 300
   caaggaaatt caccttcaaa agcactgatt acccaatgca cctccctccc cagctcgaga 360
   tcattcttca attaggacac aaaccagaca ggtttaatag cgaatctaat tttgaattct 420
   gaccatggat acccatc 437
<210> 542
   <211> 428
   <212> DNA
   <213> Homo sapiens
   <400> 542
   atctctgcct gtgcttatcc agataagaag accaaaatcc cgctgggaaa aacccaggcc 60
   ttgacattgt tattcaaatg gcccctccag aaagtttaat gatttccatt tgtatttgtg 120
   ttgatgatgg accacttgac catcacattt cagtattcat agatgactgt cacattttaa 180
   aatgttccca cttgagcagg tacacaactg gtcataattc ctgtctgtgt aattcgatgt 240
   atatttttcc aaacatgtag ctattgtttg ctttgatttt tgcttggcct cctttatgat 300
   gtgcatgtcc ttgaaggctg aatgaacagt ccctttcagt tcagcagatc aacaggatgg 360
   agctcttcat gactgtctcc agcaatagga tgatttacta taaatttcat ccaactactt 420
   gtgatctc 428
<210> 543
   <211> 259
   <212> DNA
   <213> Homo sapiens
   <400> 543
   atgttttgct aatgctcgta tctccttgat tacataatgt tagtagcact gagaccccca 60
   tggtaatgta acttaattat aagctatgtc actaccctcc tgtaaaatac tattggacag 120
   acacagaggg acccttggct cctgtgtctg gtccacacac cacagaagct tgtattatca 180
   gtgaatataa atgtactaca tttgcatgcc ttttgggttt gccttaattc ttacctcatt 240
   tgcatcctat cgatctgga 259
<210> 544
   <211> 446
   <212> DNA
   <213> Homo sapiens
   <400> 544
   taacaggcac cttatctact cattagtgaa gagataattg gattacacag gcaggcttgt 60
   ttactacatc cagaatgtag aaactgcttt cttcaacatc ttggttctag ctagtaataa 120
   caatataatt ctttggcaga tattcagaat aacattttaa actacatttt cttagaaaat 180
   tgcattcttg tagtgagcag tgtatggtct cttttgttca gaatttaaaa ctgataacca 240
   atgaaagcct tttctcttat tcctctaccg tcatttacat gataatctga agctaatatg 300
   acaatattta aatactaagt ggtactaggg aactacaaga atactgtaaa gcttaagcca 360
   ttgttatcac tgtcatttag catttaataa caaaactata cagaattatg tgcataccaa 420
   tgaatgtttt gtaccatcta gttaaa 446
<210> 545
   <211> 563
   <212> DNA
   <213> Homo sapiens
   <400> 545
   ccatagcaac aagtgacctg cccctcagac tcaagatccc agataccaga gctggaggag 60
   tcatagggca ttactggtag gcaggaaaac tgagggtcga acaaatggaa gaatgcggtg 120
   atcatagacc aaagacacac agataattaa ccccatgtgt ccacccaggc caaagttctt 180
   cctgctaccc cacagtggat gtccaggcag atggtcccca catgatgggg aagcagaggg 240
   catagtgtgg ttttgtggga cttgttcatg ttttgtagtg tgggctcaac agtgccaaag 300
   gaaacactag ggaaaagttg gtgaaacatg ccagctagca ggaccagtaa aggcataatc 360
   aggcatttgg caaagcttgc ttttctaatt caatgatagg ttctaatagg aaatttttga 420
   agatttttta aaacaatgtt atagtggcac ttccccagta tggaataaat aacatgcatt 480
   cttttttcaa tatactgtca tattcagatg tcattaaaat aaatggatga gtcacagagg 540
   agctatcaga tgctctcatg act 563
<210> 546
   <211> 484
   <212> DNA
   <213> Homo sapiens
   <400> 546
   tatgtgacgc tggacctttt ctttacccaa ggatttttaa aactcagatt taaaacaagg 60
   ggttacttta catcctacta agaagtttaa gtaagtaagt ttcattctaa aatcagaggt 120
   aaatagagtg cataaataat tttgttttaa tctttttgtt tttcttttag acacattagc 180
   tctggagtga gtctgtcata atatttgaac aaaaattgag agctttattg ctgcatttta 240
   agcataatta atttggacat tatttcgtgt tgtgttcttt ataaccaccg agtattaaac 300
   tgtaaatcat aatgtaactg aagcataaac atcacatggc atgttttgtc attgttttca 360
   ggtactgagt tcttacttga gtatcataat atattgtgtt ttaacaccaa cactgtaaca 420
   tttacgaatt atttttttaa acttcagttt tactgcattt tcacaacata tcagacttca 480
   ccaa 484
<210> 547
   <211> 402
   <212> DNA
   <213> Homo sapiens
   <400> 547
   acatttgata gtttttcacc ccttggcttt attttatata aacttttgtt tttcagcagt 60
   tctgaacttt ttagtatttt ataaatggtc caaaaaatgc ctgtttcaga agtttttgaa 120
   ttcagtgcat ttcctcttga tttgtctggg ttaaaaccat tccttttgta tgaaatgttt 180
   tgacttagga atcattttat gtacttgttc tacctggatt gtcaacaact gaaagtacat 240
   atttcatcca aatcaagcta aaatttattt aagttgattc tgagagtaca ggtcagtaag 300
   cctcattatt tggaatttga gagaagtata ggtgatcgga tctgtttcat ttataaaagg 360
   tccagttttt aggactagta cattcctgtt attttctggg tt 402
<210> 548
   <211> 503
   <212> DNA
   <213> Homo sapiens
   <400> 548
   agttagaaca tttgctgtca gccacatatt gagatgacac taggtgcaat agcagggata 60
   gattttgttg gtgagtagtc tcatgccttg agatctgtgg tggtcttcaa aatggtggcc 120
   agccagatca aggatgtagt atctcatagt tcccaggtga tatttttctt attagaaaaa 180
   tattataact catttgttgt ttgacactta tagattgaaa tttcctaatt tattctaaat 240
   tttaagtggt tctttggttc cagtgcttta tgttgttgtt gtttttggat ggtgttacat 300
   attatatgtt ctagaaacat gtaatcctaa atttaccctc ttgaatataa tccctggatg 360
   atatttttta tcataaatgc agaataatca aatacatttt aagcaagtta agtgtcctcc 420
   atcaattctg tattccagac ttgggaggat gtacagttgc tgttgtgtga tcaaacatgt 480
   ctctgtgtag ttccagcaaa tca 503
<210> 549
   <211> 440
   <212> DNA
   <213> Homo sapiens
<220>
   <221> misc_feature
   <222> (331)..(331)
   <223> n is a, c, g, or t
   <400> 549
   ggactagagc aacatcgtgc tgcccaaagg actaacctat gcaaactagt tcacatttta 60
   gtggatgtcg cagttaatgt gtaataagac attatttccc ctgcataatg tacaacagca 120
   ttgaaatgac acattaagcc tagcatcaca ttgtatagta cagtcactca caaacccttc 180
   aaggctaccc taatcattaa cattaatatt tgtttaaaag caaatcaccg atttatctat 240
   tgaaactact taaatgacgg caaaccagga atgacagatg gctgtgtcag caatggcttt 300
   aatgtgttcc ctgcaagtgg tctcctatga ntagaactgc gttctcaaat gcactctctt 360
   cagggtctta atattctgtg ttttctctct gtatttgtaa aacattataa cacattaatt 420
   tcctatctct acacatttgg 440
<210> 550
   <211> 505
   <212> DNA
   <213> Homo sapiens
   <400> 550
   gtcaaggcat tgtatgttgc ttctgtggtt attattctgt gatgcttaga ctacttgaac 60
   ccataaactt ggaagaatct ttgagcaaat tttctcagtt gtctgtatga cttcagtata 120
   ttcctgggaa tgccatagga ttttttgtgc ttgatacatg gtatccagtt tgcatagtat 180
   cacttctttg taatccagtt gctgttaaga atgatgtact ttaaaggaaa agagaaaact 240
   gcatcacagt cccattctcc agtgtccatg caatgaattg ctgagcattt aggaagcagc 300
   accaagtcta ttacaggcat ggtgtgaaac ttgatgtttg acctgtgatc aaaattgaac 360
   cattgtacag tttggcttct gtttgcttca aaatatgtag aattgtggtt gatgattaat 420
   ttgcgagact aactttgaga gtgtaacagt tttgaagaaa acattgaatg ttttacaaat 480
   gaaggggctt cacggaatgt tacaa 505
<210> 551
   <211> 476
   <212> DNA
   <213> Homo sapiens
   <400> 551
   ccaaatttca tttcagccac ttctgcagga tccctactgc caacctggaa tggagacttt 60
   tatctacttc tctctctctg aagatgtcaa atcgtggttt agatcaaata tatttcaagc 120
   tataaaagca ggaggttatc tgtgcagggg gctggcatca tgtatttagg ggcaagtaat 180
   aatggaatgc tactaagata ctccatattc ttccccgaat cacacagaca gtttctgaca 240
   ggcgcaactc ctccattttc ctcccgcagg tgagaaccct gtggagatga gtcagtgcca 300
   tgactgagaa ggaaccgacc cctagttgag agcaccttgc agttccccga gaactttctg 360
   attcacagtc tcattttgac agcatgaaat gtcctcttga agcatagctt tttaaatatc 420
   tttttccttc tactcctccc tctgactcta agaattctct cttctggaat cgcttg 476
<210> 552
   <211> 493
   <212> DNA
   <213> Homo sapiens
   <400> 552
   aggaaataac ccagttctgc accactggtt tttgtagcta tctcgtaagg ctgctggctg 60
   aaaactgtgt ctatgcaacc ttccaagtgc ggagtgtcaa ccaactggac gggagagagt 120
   actgctccta ctccaggact ctcacaaagc tgatgagctg tacttcagaa aaaaataata 180
   atttccatgt tttgtatata tctgacaaaa ctggcaacat cttacagact actgacttga 240
   agacaacctc ttttatattt ctctatttct gggctgatga atttgttttc atctgtcttt 300
   tcccccttca gaattttcct tggaaaaaaa atactagcct agctggtcat ttctttgtaa 360
   ggtagttagc aattttaagt ctttctttgg tcaacttttt tttaatgtga aaagttaggt 420
   aagacacttt tttactgctt ttatgttttt ctgtcttgtt ttgagaccat gatggttaca 480
   cttttggttc cta 493
<210> 553
   <211> 481
   <212> DNA
   <213> Homo sapiens
   <400> 553
   cctcttggtg cctaacctgg attagtaatg tgcattcagg tgaattttca gctgaggctc 60
   tgagaactgg tactctcagt gtgttctggt catcttgtgg cttagttgta gaagcaggtg 120
   tgtctcttgc ctctgcttgc ctcctactgc acactcagca cccaggactg gaatcaccga 180
   ctactgaatc tcctacatgt attgctgcta cttcaagctc ctccacttga aaccttatga 240
   ttttccaagg ggagatggga cagtgtcatc taaatattcc gaatgtttgg ccttctgaga 300
   aaagagcttc tagtaattga accatgggtt tcccagcttc tggagggttg gccgtgggct 360
   gtgtacatgt gtgtgcccag gggtgagtgt ttctcaggat tcctaacgat tcaaattacc 420
   gttgagtata tataaagaat cgagtctctg tatggaagaa caaatgtgtg cattcacccc 480
   c 481
<210> 554
   <211> 377
   <212> DNA
   <213> Homo sapiens
   <400> 554
   ttgaaagttg tgggtcagct gaccaggtag aggattcaag actcaatgtg gaaaaaatat 60
   tttaaactac tgattgaatg ttaatggtca atgctagcac aatattccta tgctgcaata 120
   cattaaaata actaagcaag tatatttatt tctagcaaac agatgtttgt tttcaaaata 180
   cttctttttc attattggtt ttaaaaaagc attatccttt tatctcacaa ataagtaata 240
   tctttcagtt attaaatgat agataatgcc tttttggttt tgtgtggtat tcaactaata 300
   catggtttaa agtcacagcc gtttgaatat attttatctt ggtagtacat tttctccctt 360
   aggaatatac atagtct 377
<210> 555
   <211> 482
   <212> DNA
   <213> Homo sapiens
   <400> 555
   gagctgactg acatatcttt aaatactttg tactaacttt atcacactta ctgtgtcata 60
   gaatatcata cagtttatac gctcatagtt ctcttgtgaa cacttcaaac atcgctaagc 120
   atttgatctg gccatgtata tggtagctgt gttttaattt gagaatcttg agggtagagc 180
   cacaaatttc aattcttaca tttccatttg caaagtgact agagaaaaag aaatcagctt 240
   aaatgaggta ttaagtaatg tttagagttg taggtattaa ctagaatata aatccttaga 300
   aattgtcttt ataccttcaa aaattatact atgcatttat catagaaatg tgattacaaa 360
   gaagtctgac taccatgtct ttaaacatat ggcatctctc aacttttctt ccttatgggg 420
   ctacatttgt tcatttccag cagtagcata aacttacggt gacatggtag acttgtctct 480
   aa 482
<210> 556
   <211> 515
   <212> DNA
   <213> Homo sapiens
<220>
   <221> misc_feature
   <222> (89)..(89)
   <223> n is a, c, g, or t
   <220>
   <221> misc_feature
   <222> (110)..(110)
   <223> n is a, c, g, or t
   <220>
   <221> misc_feature
   <222> (227)..(227)
   <223> n is a, c, g, or t
   <220>
   <221> misc_feature
   <222> (250)..(250)
   <223> n is a, c, g, or t
   <220>
   <221> misc_feature
   <222> (276)..(276)
   <223> n is a, c, g, or t
   <400> 556
   aattgccaca ttttcttatg gcattaaaaa ttttacaaaa acataatttt aatggctata 60
   ttatattcca tttaatggat gcaactcang tttatttaac cattcccatn gttgttaact 120
   atttaggttg tttctaattt tcattattat aaagttgcag aaatttggtg tacataaaac 180
   tgtctccata taattgatta ttaggatata ttcccatgaa ggattcnttt ttttaaaaaa 240
   atgtgaaatn tcatcttgta cttacacctt tcatgnaaag ggatttcctg cttttgtact 300
   gcatgggtgg cagttgtgag gaaaagccag tcaaatgacc tttttacaaa agaaatgcag 360
   tggtcacttc agttgagagt gactttttaa tacaacaaga tcaactagaa gaattcaact 420
   gtctcaagaa tcaaggtacc ccaatatatc tcgcaattcc aaactttgtt tgagggactc 480
   gttatccagc tcttggtagc cacacctgca atgta 515
<210> 557
   <211> 430
   <212> DNA
   <213> Homo sapiens
<220>
   <221> misc_feature
   <222> (43)..(44)
   <223> n is a, c, g, or t
   <220>
   <221> misc_feature
   <222> (46)..(46)
   <223> n is a, c, g, or t
   <220>
   <221> misc_feature
   <222> (120)..(120)
   <223> n is a, c, g, or t
   <220>
   <221> misc_feature
   <222> (358)..(358)
   <223> n is a, c, g, or t
   <400> 557
   gggtccatct gtagcaaatg ggttgagtgt gtcagtatgt ggnntnggtt actgtgtatt 60
   cgccaggaat caccccgata ggctgccacc ctattaggtg atacctgttt aatatgttgn 120
   ccaggtagac tagtagttgc atcagtttgc tgtaacaagt aaccagtgag gtaacacagt 180
   ggtgaagcag gtcaggggag gtcaggagga tgtctgagag aaagaagtcc gggagatgaa 240
   tggctgtcta ggaaggagga tgtcagtgca cggttagtgt ttgagcagag ggcagacttg 300
   taaagtacct gtagtgaaaa gaatgtgggg acccgattag cagaaaggtg tttgcacnta 360
   ctttatacaa aatacagaat actttatatt ggaagtgaaa gaaatgaacg tggactttta 420
   cacatgtgca 430
<210> 558
   <211> 437
   <212> DNA
   <213> Homo sapiens
<220>
   <221> misc_feature
   <222> (137)..(137)
   <223> n is a, c, g, or t
   <400> 558
   taaattgtcc ctcgtattat ttctccacgt ctgttttagt ttaatgtctc ctaagctttt 60
   ctctcatagc gtagacctag ggaagggatg ggaagattgc ccagtccccg atggctgcgc 120
   acacaggagg cggcggngca caaggcaagt gagtttgcac tgtcagcccc agaccgtaag 180
   cttggctaca ctgatgtttt tctttactaa ggatactatt caaaaattaa cattttcatc 240
   tcagtaagtt tttagaacat caaaatgttt tctgagctcc aagtggctag gttgtaaaag 300
   ttttataata atttgcaatt aaaatacatg atacatatta atccattaaa gactagtggg 360
   aatgtatcag ccagagtagc aagtaatttt tgttttataa atcatagtat ctgtcatctt 420
   gcagtattac caatgct 437
<210> 559
   <211> 519
   <212> DNA
   <213> Homo sapiens
<220>
   <221> misc_feature
   <222> (49)..(49)
   <223> n is a, c, g, or t
   <220>
   <221> misc_feature
   <222> (61)..(61)
   <223> n is a, c, g, or t
   <400> 559
   gtaatgaaga gctaactgtg ttataatcat cttgcttttg cctgaattng gagaaagtat 60
   nataattaag ttcccagtat cagaaatgtc cttacataag attaaaatat cttgatgact 120
   aataccattc tatgagaaag agtagttata tgcccagact gtattaattt actttagaaa 180
   ctaatgtttg aagtaatgga aaaaatttta aattataaag ctaaggtgca ataacatttg 240
   ctacttattt atagaattat ttgaagaatt ttgtttttga agtaatgctt taaggagtat 300
   aagatattca agataaatta tactataaaa tgattttatt gaaagttgaa ggttacacaa 360
   attgttttag gtatgagcag aagaggttaa ggtatttcta aaggtaacat atagtcaaga 420
   gtttcctcaa aatagttatt tggagaagaa tcagaatgtc tgtgtatttc ttgtctgttt 480
   ctatgttgtc ttatagctct gactaaatgt gtttaccta 519

<210> 560
   <211> 412
   <212> DNA
   <213> Homo sapiens
   <400> 560
   acagccacag tttatcctga accgcaaaac aaagaagcat ttgtccgctc ccagatgtat 60
   agtactgatt atgaccagat tctacctgat tgttattctt ggcctgaaga ggtgcagaaa 120
   atacagacca aagttgacca gtaggataat agcaaacatt tctaactcta ttaatgaggt 180
   ctttaaacct ttcataattt ttaaaggttg gaatctttta taatgattca taagacactt 240
   agattaagat tttactttaa cagtctaaaa attgatagaa gaatatcgat ataaattggg 300
   ataaacatca catgagacaa ttttgcttca ctttgccttc tggttattta tggtttctgt 360
   ctgaattatt ctgcctacgt tctctttaaa agctgttgta cgtactacgg ag 412
<210> 561
   <211> 433
   <212> DNA
   <213> Homo sapiens
   <400> 561
   ggagctgcta tgaagtacct ttcttatgtt gctaggctac tgtttctgaa agccctggat 60
   ctctttgcac caaaaatggt ccagatagac tctttttaag gatcttggct gctttttact 120
   agaaggttgc ttttatgagc atatttatac tgctgaagga tgagtgttaa ttttaattaa 180
   ctttgccgtt ttgtagagaa aactattcac aagataaatt ccaagtcttt tcacctgtca 240
   ggcatgcata ttttaatatc tgtttggata gtcagaagta gaatcataaa ggtaaaatat 300
   gagttgttac tttgtttctt cgatgtcata ttttatgtgt aatatatatg taaagggcca 360
   ttcttaagtt ctctccttaa acttaatgct gtcaagtgtt agatgtgtgc atgtgaactt 420
   gttgcactgc aga 433
<210> 562
   <211> 490
   <212> DNA
   <213> Homo sapiens
   <400> 562
   aatactctga gtttcatagt gattgaggca taactatcaa tcacaaaagt atattcaaaa 60
   attatatttt gaacaactcg aatcactcat ttgtttccat attaaaatca caaactcatc 120
   cattaatgta gataaagcac tgtttggata tgagatgtag caaattccaa tacattattg 180
   gacttccatt tggaatcata tgggatactg ctggtcttat cctgtccctc ctccaggtag 240
   agagaccaca tgcaggctca acataacata agctagaaaa attagatgac tgaatttcta 300
   tggcatattg ataataaaat tcattccatt tgctgattgt ctgaaatttt ctagaatact 360
   aataaaatac atactataga ttctttatta gtgaagtatg cactaatcaa tactttgaac 420
   acaaagcctg tgttactgat ttggccgttt tgtgaagaaa catttatctt tgtacgttct 480
   tctattgtgc 490
<210> 563
   <211> 475
   <212> DNA
   <213> Homo sapiens
   <400> 563
   cagaccggca gtcatcatgg cagtttcagc gttcaaacag caatagctca agtgtgataa 60
   ctactgagga taataaaatc cacattcact taggaagtcc ttacatgcaa gctgtagcca 120
   gcccttcagc accactgcag gataaccgaa ctcaaggctt aattaacggg gcactaaaca 180
   aaacaaccaa taaagtcacc agcagtatta ctatcacacc aacagccaca cctcttcctc 240
   gacaatcaca aattacagta agtaatatat ataactgacc acgctcaccc tcatccagtc 300
   catactgata tttttgcaag gaactcaatc cttttttaat catccctcca tatcccccaa 360
   gactgactga actcgtactt tgggaaggtt tgtgcatgaa ctatacaaga gtatctgaaa 420
   ctaactgttg cctgcatagt catatcgagt gtgcacttac tgtatatctt ttcat 475
<210> 564
   <211> 306
   <212> DNA
   <213> Homo sapiens
   <400> 564
   gaggcccaga taatgagctg agattcagca tcccctggag gagtcggggt ctcagcagaa 60
   ccccactgtc cctccccttg gtgctagagg cttgtgtgca cgtgagcgtg cgagtgcacg 120
   tccgttattt cagtgacttg gtcccgtggg tctagccttc ccccctgtgg acaaaccccc 180
   attgtggctc ctgccaccct ggcagatgac tcactgtggg ggggtggctg tgggcagtga 240
   gcggatgtga ctggcgtctg acccgcccct tgacccaagc ctgtgatgac atggtgctga 300
   ttctgg 306
<210> 565
   <211> 490
   <212> DNA
   <213> Homo sapiens
   <400> 565
   tctggttgcc tatagtgctc tgggatccca ccgagaagaa ccatgggtgg acccgaactc 60
   cccggtgctc ttggaggacc cagtcctttg tgccttggca aaaaagcaca agcgaacccc 120
   agccctgatt gccctgcgct accagctaca gcgtggggtt gtggtcctgg ccaagagcta 180
   caatgagcag cgcatcagac agaacgtgca ggtgtttgaa ttccagttga cttcagagga 240
   gatgaaagcc atagatggcc taaacagaaa tgtgcgatat ttgacccttg atatttttgc 300
   tggcccccct aattatccat tttctgatga atattaacat ggagggcatt gcatgaggtc 360
   tgccagaagg ccctgcgtgt ggatggtgac acagaggatg gctctatgct ggtgactgga 420
   cacatcgcct ctggttaaat ctctcctgct tggtgatttc agcaagctac agcaaagccc 480
   attggccaga 490
<210> 566
   <211> 491
   <212> DNA
   <213> Homo sapiens
   <400> 566
   aagcaaatag tgccctcagc tactgcagaa gaaaagtccc actgaggaaa agaaagtctt 60
   gtgattttta aaggcaagtt ttcaagtgct ctcatagttc tatcctctaa ttccattaaa 120
   tccatactag gagcgtcagt gagggttttc atagcttttg gaaatacttt ggtctctgaa 180
   ctgtaattag caagaagtaa aaacagaaac gtcaaacgtc aaatgtttgc tttgttacct 240
   ggaggactaa atgtagatgt ctttagtata ctttgtatgt tcttaaatat tggaagataa 300
   ttttgtgaat ctgtagattt tattttttca gtcttacctt acaaatttct tttctatgaa 360
   taatagagga actcacggca ctctgccact tgttaatgaa aggaagtgca gaggatttag 420
   aaaagtacat gatccccaga ccacaacaaa ccaaaacata aactcatgtc tgtgtcccat 480
   ggtcatagtc a 491
<210> 567
   <211> 501
   <212> DNA
   <213> Homo sapiens
   <400> 567
   agaagatggc cgggaactcg atcctgctgg ctgctgtctc tattctctcg gcctgtcagc 60
   aaagttattt tgctttgcaa gttggaaagg caagattaaa atacaaagtt acgcccccag 120
   cagtcactgg gtcaccagag tttgagagag tatttcgggc acaacaaaac tgtgtggagt 180
   tttatcctat attcataatt acattgtgga tggctgggtg gtatttcaac caagtttttg 240
   ctacttgtct gggtctggtg tacatatatg gccgtcacct atacttctgg ggatattcag 300
   aagctgctaa aaaacggatc accggtttcc gactgagtct ggggattttg gccttgttga 360
   ccctcctagg tgccctggga attgcaaaca gctttctgga tgaatatctg gacctcaata 420
   ttgccaagaa actgaggcgg caattctaac tttttctctt ccctttaatg cttgcagaag 480
   ctgttcccac catgaaggta a 501
<210> 568
   <211> 474
   <212> DNA
   <213> Homo sapiens
   <400> 568
   agatcacaga gcagcaagtt catacaacat gcatgttctc ctctatctta gaggggtatt 60
   cttcttgaaa ataaaaaata ttgaaatgct gtatttttac agctacttta acctatgata 120
   attatttaca aaattttaac actaaccaaa caatgcagat cttagggatg attaaaggca 180
   gcatttgatg atagcagaca ttgttacaag gacatggtga gtctattttt aatgcaccaa 240
   tcttgtttat agcaaaaatg ttttccaata ttttaataaa gtagttattt tataggggat 300
   acttgaaacc agtatttaag ctttaaatga cagtaatatt ggcatagaaa aaagtagcaa 360
   atgtttactg tatcaatttc taatgtttac tatatagaat ttcctgtaat atatttatat 420
   actttttcat gaaaatggag ttatcagtta tctgtttgtt actgcatcat ctgt 474
<210> 569
   <211> 444
   <212> DNA
   <213> Homo sapiens
   <400> 569
   gaaactgctg agacctattt ccctttcttg gggagagaat aagtgacagc tgattaaagg 60
   cagagacaca ggactgcttt caggctcctg gtttattctc tgattgactg agctccttcc 120
   accagaaggc actgcctgca ggaagaagat gatctgatgg ccgtgggtgt ctgggaagct 180
   cttcgtggcc tcaatgccct cctttatcct catctttctt ctatgcagaa caaaaagctg 240
   catctaataa tgttcaatac ttaatattct ctatttatta cttactgctt actcgtaatg 300
   atctagtggg gaaacatgat tcattcactt aaaatactga ttaagccatg ggcaggtact 360
   gactgaagat gcaatccaac caaagccatt acattttttg agttagatgg gactctctgg 420
   atagttgaac ctcttcactt tata 444
<210> 570
   <211> 464
   <212> DNA
   <213> Homo sapiens
   <400> 570
   gtgatggttg gcttgagtac ctttttaaat ctagcccagt ataaacatta gcctgcttaa 60
   tatttagaca tttataggta gaattctgag cactcaactc atgtttggca ttttaaagta 120
   aaaacaagtg tgacttcgag gaccaaagaa attgtcagct atacatttat ctttatgaac 180
   tcatttatat tcctttttaa tgactcgttg ttctaacatt tcctagaagt gttcttataa 240
   aggtctaatg tatccacagg ctgttgtctt attagtaaat gcaaagtaat gactttgtct 300
   gttttactct agtctttagt acttcaaaat taccttttca tatccatgat cttgagtcca 360
   tttgggggat ttttaagaat ttgatgtatt tcaatacact gttcaaaatt aaattgttta 420
   attttatgta tgagtatgta tgttcctgaa gttggtccta ttta 464
<210> 571
   <211> 499
   <212> DNA
   <213> Homo sapiens
   <400> 571
   aaatatcagt tactcagccc tgggccccac cacctaggcc actcctccaa aggaagtcta 60
   ggagctggga ggaaaagaaa agaggggaaa atgagttttt atggggctga acggggagaa 120
   aaggtcatca tcgattctac tttagaatga gagtgtgaaa tagacatttg taaatgtaaa 180
   acttttaagg tatatcatta taactgaagg agaaggtgcc ccaaaatgca agattttcca 240
   caagattccc agagacagga aaatcctctg gctggctaac tggaagcatg taggagaatc 300
   caagcgaggt caacagagaa ggcaggaatg tgtggcagat ttagtgaaag ctagagatat 360
   ggcagcgaaa ggatgtaaac agtgcctgct gaatgatttc caaagagaaa aaaagtttgc 420
   cagaagtttg tcaagtcaac caatgtagaa agctttgctt atggtaataa aaatggctca 480
   tacttatata gcacttact 499
<210> 572
   <211> 468
   <212> DNA
   <213> Homo sapiens
   <400> 572
   ggtgcaacag gaccaatggg ccagcaaggc atccctggca tccctgggcc cccgggtccc 60
   atgggccagc caggcaaggc tggccactgt aatccctctg actgctttgg ggccatgccg 120
   atggagcagc agtacccacc catgaaaacc atgaaggggc cttttggctg aaattcccca 180
   cctgcctttg gatgaaagac tccgttggga ataaatggcc aaagcttata ggactctgtg 240
   acaggttgtg aatgtttttt ttttttgttg ttgttgtttt taattgctgt taatattttt 300
   taaataataa agaaacaaaa ctatctgccc tttcccttcc agtgggttcc tctggtgctg 360
   cagccagagc tccctgttgc cctccttttc ccgtttagtc ccaggaacaa aaagggcatt 420
   tgggtacagg ggcatatacc tgtaatccta gctattcaag gggctgag 468
<210> 573
   <211> 406
   <212> DNA
   <213> Homo sapiens
   <400> 573
   gggtctgaat ctagcaccat gacggaacta gagacagcca tgggcatgat catagacgtc 60
   ttttcccgat attcgggcag cgagggcagc acgcagaccc tgaccaaggg ggagctcaag 120
   gtgctgatgg agaaggagct accaggcttc ctgcagagtg gaaaagacaa ggatgccgtg 180
   gataaattgc tcaaggacct ggacgccaat ggagatgccc aggtggactt cagtgagttc 240
   atcgtgttcg tggctgcaat cacgtctgcc tgtcacaagt actttgagaa ggcaggactc 300
   aaatgatgcc ctggagatgt cacagattcc tgcagagcca tggtcccagg cttcccaaaa 360
   gtgtttgttg gcaattattc ccctaggctg agcctgctca tgtacc 406
<210> 574
   <211> 535
   <212> DNA
   <213> Homo sapiens
   <400> 574
   ccttctctga tttcttcagc agggtcaaaa gacagttact agcaatgggg aatgcttgtc 60
   actgtggaga aagagttttg tatatgtctg ataccgttgt tataacaaaa caaatttttt 120
   tactatagtt ttttgttttc tacctgcaca cccaccagaa gagcacaaag caaggccatt 180
   gcaacaggca tttaaaaatt attatcaaac atgcacatgc ttgtacacac acacacacac 240
   acacacaaac aggggcattt gtaaaggtgt ccctggaatg taagatttat aatgtttaag 300
   gcaaggtgaa ggcattgcca agtgtgtgtc gctcatagga ctagtgtata ttcactgaaa 360
   gttaacctga tgatttgtta ttgtttgaac catatgctga tttgcttctg gtttctgttt 420
   agtgtgttct ctctgataag gggctgaaag attctgcatc acacatcctc tgagacctac 480
   catgtcgcac actttgttaa tgacaaactt cactctacac tatacagtac cttgt 535
<210> 575
   <211> 401
   <212> DNA
   <213> Homo sapiens
   <400> 575
   ggcctcccaa agatgctagt attatgggcg tgaaccacca tgcccagccg aaaagctttt 60
   gaggggctga cttcaatcca tgtaggaaag taaaatggaa ggaaattggg tgcatttcta 120
   ggacttttct aacatatgtc tataatatag tgtttaggtt cttttttttt tcaggaatac 180
   atttggaaat tcaaaacaat tgggcaaact ttgtattaat gtgttaagtg caggagacat 240
   tggtattctg ggcagcttcc taatatgctt tacaatctgc actttaactg acttaagtgg 300
   cattaaacat ttgagagcta actatatttt tataagacta ctatacaaac tacagagttt 360
   atgatttaag gtacttaaag cttctatggt tgacattgta t 401
<210> 576
   <211> 396
   <212> DNA
   <213> Homo sapiens
   <400> 576
   attcttctaa ttgctgtgtg tcccaggcag ggagacggtt tccagggagg ggccggccct 60
   gtgtgcaggt tccgatgtta ttagatgtta caagtttata tatatctata tatataattt 120
   attgagtttt tacaagatgt atttgttgta gacttaacac ttcttacgca atgcttctag 180
   agttttatag cctggactgc tacctttcaa agcttggagg gaagccgtga attcagttgg 240
   ttcgttctgt actgttactg ggccctgagt ctgggcagct gtcccttgct tgcctgcagg 300
   gccatggctc agggtggtct cttcttgggg cccagtgcat ggtggccaga ggtgtcaccc 360
   aaaccggcag gtgcgatttt gttaacccag cgacga 396
<210> 577
   <211> 318
   <212> DNA
   <213> Homo sapiens
   <400> 577
   ttccacatca gtaactgccc tggggtttgt gctgtacaaa tacaagctcc tgccacggtc 60
   ttgaagttct gttcttatgc tctctgctca ctggttttca ataccaccaa gaggaaaata 120
   ttgacaagtt taaaggctgt gtcattgggc catgtttaag tgtactggat ttaactacct 180
   ttggcttaat tccaatcatt gttaaagtaa aaacaattca aagaatcacc taattaattt 240
   cagtaagatc aagctccatc ttatttgtca gtgtagatca actcatgtta attgatagaa 300
   taaagccttg tgatcact 318
<210> 578
   <211> 411
   <212> DNA
   <213> Homo sapiens
   <400> 578
   ctttgcgggc acagagactg ccacaaagtg gagcggctac atggaagggg cagttgaggc 60
   tggagaacga gcagctaggg aggtcttaaa tggtctcggg aaggtgaccg agaaagacat 120
   ctgggtacaa gaacctgaat caaaggacgt tccagcggta gaaatcaccc acaccttctg 180
   ggaaaggaac ctgccctctg tttctggcct gctgaagatc attggatttt ccacatcagt 240
   aactgccctg gggtttgtgc tgtacaaata caagctcctg ccacggtctt gaagttctgt 300
   tcttatgctc tctgctcact ggttttcaat accaccaaga ggaaaatatt gacaagttta 360
   aaggctgtgt cattgggcca tgtttaagtg tactggattt aactaccttt g 411
<210> 579
   <211> 201
   <212> DNA
   <213> Homo sapiens
   <400> 579
   tgggagcatg gtgagcagcc ctggtgctca gcagccatac ctatgggaca cacactacga 60
   aaaggatgcc tttagggttt gggggagatt ttactccttt cttcaacaac tattcactgg 120
   acaagttctc tgctcccatg acgcgccagg cacagttctg caagtatatt gtgaatgtat 180
   tgttctagtg ggatacacaa a 201
<210> 580
   <211> 336
   <212> DNA
   <213> Homo sapiens
   <400> 580
   gggatcctat ttagctctta gtaccactaa tcaaaagttc ggcatgtagc tcatgatcta 60
   tgctgtttct atgtcgtgga agcaccggat gggggtagtg agcaaatctg ccctgctcag 120
   cagtcaccat agcagctgac tgaaaatcag cactgcctga gtagttttga tcagtttaac 180
   ttgaatcact aactgactga aaattgaatg ggcaaataag tgcttttgtc tccagagtat 240
   gcgggagacc cttccacctc aagatggata tttcttcccc aaggatttca agatgaattg 300
   aaatttttaa tcaagatagt gtgctttatt ctgttg 336
<210> 581
   <211> 521
   <212> DNA
   <213> Homo sapiens
   <400> 581
   atatcttctt caggctctga caggcctcct ggaaacttcc acatattttt caactgcagt 60
   ataaagtcag aaaataaagt taacataact ttcactaaca cacacatatg tagatttcac 120
   aaaatccacc tataattggt caaagtggtt gagaatatat tttttagtaa ttgcatgcaa 180
   aatttttcta gcttccatcc tttctccctc gtttcttctt tttttggggg agctggtaac 240
   tgatgaaatc ttttcccacc ttttctcttc aggaaatata agtggttttg tttggttaac 300
   gtgatacatt ctgtatgaat gaaacattgg agggaaacat ctactgaatt tctgtaattt 360
   aaaatatttt gctgctagtt aactatgaac agatagaaga atcttacaga tgctgctata 420
   aataagtaga aaatataaat ttcatcacta aaatatgcta ttttaaaatc tatttcctat 480
   attgtatttc taatcagatg tattactctt attatttcta t 521
<210> 582
   <211> 484
   <212> DNA
   <213> Homo sapiens
   <400> 582
   gaagttgttc aactatcctt gccactggaa gaccaaacaa ggttttcact gctttttctt 60
   ttacataata tgctgagaat tatttcttat gctttttact acaaacaaaa ttactcacct 120
   ggattaaaga ttaaggcctt aatctgttta gattatcttt aatctccatg aaatcgtgaa 180
   ataagacaag aatagtgttt cagctgtagg ccattttaca gctaattgcc cataaattgt 240
   agcatttatt gacctgaagt actaagctaa ttgtcttgac tactcaaagc ccctgaattg 300
   ttgtcaactt tcccctttgt gttgtgtagc cctaacgtca tttagcttgt tgtctgatgc 360
   ctccagtagg acacctccga tggagctttg atttctgagc agcgaaagct cccttcctaa 420
   gatgcatctc gcataggctg cctatgatga aggaccgtgc acctccactc caacagagtg 480
   ctga 484
<210> 583
   <211> 503
   <212> DNA
   <213> Homo sapiens
   <400> 583
   tatcggctac atatgcagtc tgtgaattat gtaacatact ctatttcttg agggctgcaa 60
   attgctaagt gctcaaaata gagtaagttt taaattgaaa attacataag atttaatgcc 120
   cttcaaatgg tttcatttag ccttgagaat ggttttttga aacttggcca cactaaaatg 180
   tttttttttt tttacgtaga atgtgggata aacttgatga actccaagtt cacagtgtca 240
   tttcttcaga actccccttc attgaatagt gatcatttat taaatgataa attgcactcg 300
   ctgaaagagc acgtcatgaa gcaccatgga atcaaagaga aagatataaa ttcgttccca 360
   cagccttcaa gctgcagtgt tttagattgc ttcaaaaaat gaaaaagttt tgcctttttc 420
   gatatagtga ccttctttgc atattaaaat gtttaccaca atgtcccatt tctagttaag 480
   tcttcgcact tgaaagctaa cat 503
<210> 584
   <211> 465
   <212> DNA
   <213> Homo sapiens
   <400> 584
   cagaagggct ggatgccccg ggagagcgtg ctcccacacc tgcaggtgca gcacctgacc 60
   ggggggctca tcgaccccaa gaggacaggc cgcatcccca tccagcaggc cctcctctcc 120
   gggatgatca gtgaagagct ggcccagctc ctgcaggacg agtccagcta cgagaaggat 180
   ttgacagacc ccatctccaa ggaacggctg agctacaagg aggccatggg ccgctgccgc 240
   aaagaccccc tgagcggcct gctgctcctg ccagcggcac tggaggggta ccgctgctac 300
   cgctccgcct cccccaccgt cccgcgctcc cttcgctgac acgggccaag gagccagtgg 360
   ggaagtgcgt gtgttgggcc aggtaggata cgtacacctc ttgcctcaga gcagcctcat 420
   cccaggcagt gggtcttccc tctgtccaac cactgtttta ttatt 465
<210> 585
   <211> 360
   <212> DNA
   <213> Homo sapiens
<220>
   <221> misc_feature
   <222> (271)..(271)
   <223> n is a, c, g, or t
   <400> 585
   ttttgtattc tatcccagat cacaggaaag ttataaaaat caaaccgtca ccctttagtt 60
   tgcttgaact ttagtaaacc acctgcttag ggactttgaa cttaaatata tccccttcct 120
   caagtggtgc tattttaaaa ctaaaaaaaa ctttgaattg gctatttttt taatgcaata 180
   ttttttttct gaattcatta tgatccccat attgggtaat gctgaacatt tatctgaaac 240
   agatgaggat attattattt tgtatccaaa nagaaattca gataaaggga aatttgacta 300
   gtgtaatctg agatatgtca tagggatttc tttctgacaa aagggtgctt tgctgttctt 360
<210> 586
   <211> 520
   <212> DNA
   <213> Homo sapiens
   <400> 586
   gatgacgggg gatctgaggc tgtgtctctg ccttgtcttt agaggacttc agcgtccaag 60
   actggggccc acccttctca ccagcactaa atgcactaac aaggactcca gacctgcagc 120
   cccagacccg ccgtagtata agcctaacaa gcaacacgta gcaccttagt ctttgttcca 180
   ggagagctga gcaagctggt gaaaccactc tccttccttt aaacaccgtt tcaaccaacc 240
   tctccctgga gccaacctgt aaaaagtggg ttgattgctg acagcatggt cttccctccc 300
   tgcatttcag acataccagt tactgaaagc aaatcagttt taagtgattt ctcagtgctg 360
   aaaagcctgt ccaggtttcc ttccctttcc caagcctctc tctgtaatac tccctttggg 420
   cgaagctaac atcggtgcct ccccgacctt gctgactagg cacatgggac gcaaaggagg 480
   gagggaagca aggccttgcc tggcgagttg tcatgtggtt 520
<210> 587
   <211> 468
   <212> DNA
   <213> Homo sapiens
   <400> 587
   taaaccagtc cactgttata cccggggcac tctaaccatc acaatcaatc aatcaaattc 60
   ccttaaattt gtatggcact ggaactttgg caaagcactt ttgacaagtt gtgtctgatt 120
   ggagcttcat gatagccttg tgacatcttt agggcaggat tcttatcccc attttgcaga 180
   tgaaaaccct gagtcacaga tttctgtggg actgtggatc tcactggaag ctatccaaga 240
   gcccactgtc accttctaga ccacatgata gggctagaca gctcagttca ccatgattct 300
   cttctgtcac ctctgctggc acaccagtgg caaggcccag aatggcgacc tctctttagc 360
   tcaatttctg ggcctgaggt gctcagactg cccccaagat caaatctctc ctggctgtag 420
   taacccagtg gaatgaattt ggacatgccc caatgcttct atatgcta 468
<210> 588
   <211> 523
   <212> DNA
   <213> Homo sapiens
   <400> 588
   tttggtggtt ttattctatc ggtataaagg catcgatatt ttagatgcac ccgtgtttgt 60
   aaaaatgtag agcacaatgg aattatgctg gaagtctcaa ataatatttt tttcctattt 120
   tatactcatg gaagagataa gctaaagagg ggacaataat gagaaatgtt ggtgtgcttt 180
   tctaagcatt taaaacataa ttgccaattg aaaccctaaa tatgtttaca taccattaag 240
   atatgattca tgtaacaatg ttaaattaat tataatggga ttgggtttgt tatctgtggt 300
   agtatatatc ctagtgttcc tatagtgaaa taagtagggt tcagccaaag ctttctttgt 360
   tttgtacctt aaattgttcg attacgtcat caaaagagat gaaaggtatg tagaacaggt 420
   tcacgtgatt acctttttct tttggcttgg attaatattc atagtagaac tttataaaac 480
   gtgtttgtat tgtaggtggt gtttgtatta tgcttatgac tat 523
<210> 589
   <211> 465
   <212> DNA
   <213> Homo sapiens
   <400> 589
   ctcacacttg tctgttcttc agtgctggag gtcctggcag ggtcaggctg gggtaagccg 60
   gggttccaca gggcccagcc ctggcagggg tctggccccc caggtaggcg gagagcagtc 120
   cctccctcag gaactggagg aggggactcc aggaatgggg aaatgtgaca ccaccatcct 180
   gaagccagct tgcacctcca gtttgcacag ggatttgtcc tgggggctga gggccctgtc 240
   cccacccccg cccttggtgc tgtcataaaa gggcaggcag gggcaggctg aggagttgcc 300
   cgttgccccc cagagactga ctctcagagc cagagatggg atgtgtgagt gtgtgtgtgt 360
   gtgtgtgcgc gcgcgcgcgc gtgtgtgtgt gcacgcactg gcctgcacag agagcatggg 420
   tgagcgtgta aaagcttggc cctgtgccct acagtgggga cagct 465
<210> 590
   <211> 532
   <212> DNA
   <213> Homo sapiens
   <400> 590
   gaggaacttg ccaaactaag gactagggtg cagaaggaaa attagcacca ataaagagga 60
   aatatgaaag gattcttgaa gatttccagt tttgcaactg cataatagct atgcccaagg 120
   agtcaactat tgtatatatt gcagatttgc ctttttaaaa aaatcactaa ttctacaatg 180
   tgccagatac atgtttccta tgcccaggaa gttatgaaga cttcaacaat taaactgaaa 240
   ccaggggaag cttgcttagt tttgggtttc attataaact cttagcctca gtccaggtta 300
   atctgaagtt tgaaagctca gattaagcaa gccatgccaa gaaactggac gatgtgtaag 360
   cctagactct aaaattcaag atgtgtgaaa taatataagt caaaagcaag aaaaacgtaa 420
   tcccgtctga actcaagtag tcattcatat aaatttgaac acacctgctg tgcctagaca 480
   agtgtctttc tgtaagagct gtaactctga gatgtgctaa ataaaccctc tt 532
<210> 591
   <211> 129
   <212> DNA
   <213> Homo sapiens
   <400> 591
   aatcttcctg ttgaatgctt catgacttga attctacttt gataaaaaca ttgccatact 60
   gctttttatc ttgatgaatt catctggcat tgctttgcct tatcatctca tctggagttt 120
   ttaaatgcc 129
<210> 592
   <211> 476
   <212> DNA
   <213> Homo sapiens
   <400> 592
   cacttggcag aaggaccgtg cccggcggcc tcattttgac cagctggtgg ctgcatttga 60
   caagatgatc cgcaagccag ataccctgca ggctggcggg gacccagggg aaaggccttc 120
   ccaggcccttctgacccctg tggccctgga ctttccttgt ctggactcac cccaggcctg 180
   gctttcagcc attggactgg agtgctacca ggacaacttc tccaagtttg gcctctgtac 240
   cttcagtgat gtggctcagc tcagcctaga agacctgcct gccctgggca tcaccctggc 300
   tggccaccag aagaagctgc tgcaccacat ccagctcctt cagcaacacc tgaggcagca 360
   gggctcagtg gaggtctgag aatgacgata cccgtgactc agccctggac actggtccga 420
   gaagggacat gtgggacgtg agccgggctc caacagcctc tgtgagagat gcccca 476
<210> 593
   <211> 537
   <212> DNA
   <213> Homo sapiens
   <400> 593
   gcaggccata ctggttccat tgttctgtat aatactgaat aaataaattt acttttacat 60
   gatcgtataa gtttctagat aagataaaca aattctgttt aaattttttt aataaaaatc 120
   ttaaaacact ttttttctaa cctagactga gaaattcatg tttacttttc taggtgtatg 180
   atactttgta aagttgatac tttcctaaga atttaacatg tcatattttt gaaatagatt 240
   taagtgtgct tcttattgct aaaaatacta aatgtcatgg gtcatagtat ctgatatcaa 300
   tatcgttgat aacatatcca caggtaacac catgatgtag gcataaatgg aaaacaaaaa 360
   ccctactatt tcaaatatat tgtacttttt tatttctgta agccaactgt gtgccatttt 420
   cactggactt ttaaatctag actttagtga tgtctacatt gtaaatgatc ttttgtggat 480
   atttgtcact tggtttcaga aagttcacaa atgtagcaac agctcacatg actgagt 537
<210> 594
   <211> 543
   <212> DNA
   <213> Homo sapiens
   <400> 594
   tggccgagac agagtgccgc tatgccacgc agctgcagca gatccagggg ctcattggtg 60
   gcctggaggc ccagctgagt gagctccgat gcgagatgga ggctcagaac caggagtaca 120
   agatgctgct tgacataaag acacggctgg agcaggagat cgctacttac cgcagcctgc 180
   tcgagggcca ggatgccaag atggctggca ttggcatcag ggaagcctct tcaggaggtg 240
   gtggtagcag cagcaatttc cacatcaatg tagaagagtc agtggatgga caggtggttt 300
   cttcccacaa gagagaaatc taagtgtcta ttgcaggaga aacgtccctt gccactcccc 360
   actctcatca ggccaagtgg aggactggcc agagggcctg cacatgcaaa ctccagtccc 420
   tgccttcaga gagctgaaaa gggtccctcg gtcttttatt tcagggcttt gcatgcgctc 480
   tattccccct ctgcctctcc ccaccttctt tggagcaagg agatgcagct gtattgtgta 540
   aca 543

<210> 595
   <211> 568
   <212> DNA
   <213> Homo sapiens
   <400> 595
   gcatgttagt ttggtgctac acagtgttga tttttgtgat gtcctttggt catgtttctg 60
   ttagactgta gctgtgaaac tgtcagaatt gttaactgaa acaaatattt gcttgaaaaa 120
   aaaagttcat gaagtaccaa tgcaagtgtt ttattttttt tcttttttcc agcccataag 180
   actaagggtt taaatctgct tgcactagct gtgccttcat tagtttgcta tagaaatcca 240
   gtacttatag taaataaaac agtgtatttt gaagtttgac tgcttgaaaa agattagcat 300
   acatctaatg tgaaaagacc acatttgatt caactgagac cttgtgtatg tgacatatag 360
   tggcctataa atttaatcat aatgatgtta ttgtttacca ctgaggtgtt aatataacat 420
   agtatttttg aaaaagtttc ttcatcttat attgtgtaat tgtaaactaa agataccgtg 480
   ttttctttgt attgtgttct accttccctt tcactgaaaa tgatcacttc atttgatact 540
   gtttttcatg ttcttgtatt gcaaccta 568
<210> 596
   <211> 360
   <212> DNA
   <213> Homo sapiens
<220>
   <221> misc_feature
   <222> (250)..(250)
   <223> n is a, c, g, or t
   <400> 596
   attttaagcc ctatcactga cacatcagca tgttttctgc tttaaattaa aattttatga 60
   cagtatcgag gcttgtgatg acgaatcctg ctctaaaata cacaaggagc tttcttgttt 120
   cttattaggc ctcagaaaga agtcagttaa cgtcacccaa aagcacaaaa tggattttag 180
   tcaaatattt attggatgat acagtgtttt ttaggaaaag catctgccac aaaaatgttc 240
   acttcgaaan tctgagttcc tggaatggca cgttgctgcc agtgccccag acagttcttt 300
   tctaccctgc gggcccgcac gttttatgag gttgatatcg gtgctatgtg tttggtttat 360
<210> 597
   <211> 538
   <212> DNA
   <213> Homo sapiens
<220>
   <221> misc_feature
   <222> (314)..(319)
   <223> n is a, c, g, or t
   <400> 597
   gtcaattaga gcgatcccaa ggcatgggac caggcctgct tgcctatgtg tgatggcaat 60
   tggagatctg gatttagcac tggggtctca gcaccctgca ggtgtctgag actaagtgat 120
   ctgccctcca ggtggcgatc accttctgct cctaggtacc cccactggca aggccaaggt 180
   ctcctccacg ttttttctgc aattaataat gtcatttaaa aaatgagcaa agccttatcc 240
   gaatcggata tagcaactaa agtcaataca ttttgcagga ggctaagtgt aagagtgtgt 300
   gtgtgtgtgt gtgnnnnnnc gtgcatgtgt gtgtgtgtgt atgtgtgtga ataagtcgac 360
   ataaagtctt taattttgag caccttacca aacataacaa taatccatta tccttttggc 420
   aacaccacaa agatcgcatc tgttaaacag gtacaagttg acatgaggtt agtttaattg 480
   tacaccatga tattggtggt atttatgctg ttaagtccaa acctttatct gtctgtta 538
<210> 598
   <211> 521
   <212> DNA
   <213> Homo sapiens
   <400> 598
   atgggatttt ctagtttcct gccttcagag tatctaatcc tttaatgatc tggtggtctc 60
   ctcgtcaatc catcagcaat gcttctctca tagtgtcata gacttgggaa acccaaccag 120
   taggatattt ctacaaggtg ttcattttgt cacaagctgt agataacagc aagagatggg 180
   ggtgtattgg aattgcaata cattgttcag gtgaataata aaatcaaaaa cttttgcaat 240
   cttaagcaga gataaataaa agatagcaat atgagacaca ggtggacgta gagttggcct 300
   ttttacaggc aaagaggcga attgtagaat tgttagatgg caatagtcat taaaaacata 360
   gaaaaatgat gtctttaagt ggagaattgt ggaaggattg taacatggac catccaaatt 420
   tatggccgta tcaaatggta gctgaaaaaa ctatatttga gcactggtct ctcttggaat 480
   tagatgttta tatcaaatga gcatctcaaa tgttttctgc a 521
<210> 599
   <211> 532
   <212> DNA
   <213> Homo sapiens
   <400> 599
   aacagcaagc ctaagtcttc tctgagagga gtttcgtgag ctgaagaaca agctgctcat 60
   ggcaagggct ggccccagaa ccctgcaaga gaggccttct gtggatggag aactaggcct 120
   tctcaaagct aaggacaaaa tccagctaac ccagtccctc ggcccaggcc tcctttcgtg 180
   ctttgtgctt ggtggggggg atttcgaggg actttgcact ggactctggg aacctttcat 240
   cattaaaaaa agggggacca ttggggcctg agccaaggaa ctttccttct actgccttat 300
   agtgcttaaa cattctccgc ctccagggtg cagattcaga gctggccaga gtttcagtga 360
   tagccgtatg ttaaacagaa tctcacctca gtctcctgga gggagatgtt taagaggggt 420
   taacacatca gatgggaggg tcagcccggt gacctctaag gtatcttcta acctagaaac 480
   tcaccataat tatggtgcaa ggtcagtgtg tctctgagat ctatgtctgt tg 532
<210> 600
   <211> 447
   <212> DNA
   <213> Homo sapiens
   <400> 600
   tggagcaggt agctgtgctg gcgtctttgg gaatcctttc tttcctggga ctggtggctg 60
   gggccctggc actggggctc tggctgaggc tgagacgggg tgggaaggat ggatccccaa 120
   agcctgggtt cttggcctca gtgattccag tggacaggcg tccaggagct ccaaacctgt 180
   agaggaccca ggagggcttc ggcagattcc acctataatt ctgtcttgct ggtgtggata 240
   gaaaccaggc aggacagtag atccctatgg ttggatctca gctggaagtt ctgtttggag 300
   cccatttctg tgagaccctg tatttcaaat ttgcagctga aaggtgcttc tacctctgat 360
   ttcaccccag agttggagtt ctgctcaagg aacgtgtgta atgtgtacat ctgtgtccat 420
   gtgtgaccat gtgtctgtga ggcaggg 447
<210> 601
   <211> 447
   <212> DNA
   <213> Homo sapiens
   <400> 601
   tggagcaggt agctgtgctg gcgtctttgg gaatcctttc tttcctggga ctggtggctg 60
   gggccctggc actggggctc tggctgaggc tgagacgggg tgggaaggat ggatccccaa 120
   agcctgggtt cttggcctca gtgattccag tggacaggcg tccaggagct ccaaacctgt 180
   agaggaccca ggagggcttc ggcagattcc acctataatt ctgtcttgct ggtgtggata 240
   gaaaccaggc aggacagtag atccctatgg ttggatctca gctggaagtt ctgtttggag 300
   cccatttctg tgagaccctg tatttcaaat ttgcagctga aaggtgcttc tacctctgat 360
   ttcaccccag agttggagtt ctgctcaagg aacgtgtgta atgtgtacat ctgtgtccat 420
   gtgtgaccat gtgtctgtga ggcaggg 447
<210> 602
   <211> 547
   <212> DNA
   <213> Homo sapiens
   <400> 602
   cttcgttcgc agagcttttc agattgtgga atgttggata aggaattata gacctctagt 60
   agctgaaatg caagacccca agaggaagtt cagatcttaa tataaattca ctttcatttt 120
   tgatagctgt cccatctggt catgtggttg gcactagact ggtggcaggg gcttctagct 180
   gactcgcaca gggattctca caatagccga tatcagaatt tgtgttgaag gaacttgtct 240
   cttcatctaa tatgatagcg ggaaaaggag aggaaactac tgcctttaga aaatataagt 300
   aaagtgatta aagtgctcac gttaccttga cacatagttt ttcagtctat gggtttagtt 360
   actttagatg gcaagcatgt aacttatatt aatagtaatt tgtaaagttg ggtggataag 420
   ctatccctgt tgccggttca tggattactt ctctataaaa aatatatatt taccaaaaaa 480
   ttttgtgaca ttccttctcc catctcttcc ttgacatgca ttgtaaatag gttcttcttg 540
   ttctgag 547
<210> 603
   <211> 543
   <212> DNA
   <213> Homo sapiens
   <400> 603
   gcagagacct ccctctgaaa aacacaaaga atggactctc tcctgggatg aggacttgct 60
   ttctttacct ccggttcttt ccatgtctta gttggatgtc cctgaaatgg acacaggctg 120
   tgcattgtgc cagaaacatt gtgttatctt ttatgttgtt gttgttgctg ttaaactata 180
   atatgtgact tcttttttta ttattttttg tttgaatgct ttaaaaatct ttaagtctgt 240
   ggactgctga tgtacagtgc ctttgctgct atggatcaaa tcaaagaccg tgtagatata 300
   ctttattgta taagtagaaa attacttaat ttcatactag aaatggatgg atgctgcaag 360
   ttgaaatgga ctgtccattg acgttcctaa tgtggtagca gaaaaaaatg gtgtcttaag 420
   tgcttagtgt ttgatgtcat taacagtttc gtaaaactct acagtgtaga aagattttga 480
   tactaaactg tgcgttgtac atagttctaa tgcattgtat tgaccaccag tacttctata 540
   atg 543
<210> 604
   <211> 473
   <212> DNA
   <213> Homo sapiens
   <400> 604
   gagcgcccat atgcatgcaa caaatgtgga aaggccttca cccagagctc acaccttatt 60
   gggcaccaga gaacccacaa taggacaaag cgaaagaaga aacagcctac ctcatagctc 120
   tcaagccagt tgaagaaacc ttgccttttc agcttgaccc tgcaatataa catgcacagg 180
   cctgcttgtg aatcaggact gaatgtgaaa gggaagtatt gagtgaggac attcccaaaa 240
   ccaaaggaca actgaggaga ctgcccagca cataatgaat aaataagaaa atgagtgagg 300
   agttattaac atcatttgga aaaaagattt cccattcact tgatattgtt tgttcactca 360
   tttagtcatt aaaagtgaga ttaataaaat ctgaaaatgt tatataataa ctttaaaaag 420
   ccaggtaatt aataatctgc actgatatta catccacagt accacagtat tta 473
<210> 605
   <211> 465
   <212> DNA
   <213> Homo sapiens
   <400> 605
   gaaaactggg gtttgcatca ctccactgca cagtgttagt gggacctggg ggcaagtccc 60
   ttgacttctc tgagcctcag tttccttatg tgaaagttgc tggaaccaaa atggagtcac 120
   ttatgccaaa ctctaataaa atggagtcgg gggggcacat agaagccctc acacacacat 180
   gcccgtaaca ggatttatca ccaagacacg cctgcatgta agaccagaca cagggcgtat 240
   ggaaaagcac gtcctcaaag actgtagtat tccagatgag ctgcagatgc ttacctacca 300
   cggccgtctc caccagaaaa ccatcgccaa ctcctgcgat cagcttgtga cttacaaacc 360
   ttgtttaaaa gctgcttaca tggacttctg tcctttaaaa cgttcccctt ggctgtggcc 420
   ctctgtgtat gcctgggatc cttccaagca ctcatagccc agata 465
<210> 606
   <211> 373
   <212> DNA
   <213> Homo sapiens
   <400> 606
   tgcgctggtt tgcggctttg ggaaataaaa taccgttgta tatattctgg caggggtgtt 60
   ctagcttttt gaggacagct cctgtatcct tctcatcctt gtctctccgc ttgtcctctt 120
   gtgatgttag gacagagtga gagaagtcag ctgtcacggg gaaggtgaga gagaggatgc 180
   taagcttcct actcactttc tcctagccag cctggacttt ggagcgtggg gtgggtggga 240
   caatggctcc ccactctaag cactgcctcc cctactcccc gcatctttgg ggaatcggtt 300
   ccccatatgt cttccttact agactgtgag ctcctcgagg gcagggaccg tgccttatgt 360
   ctgtgtgtga tca 373
<210> 607
   <211> 364
   <212> DNA
   <213> Homo sapiens
   <400> 607
   gccaaaatga tacctggagg cttatctgag gccaaacccg ccactccaga aatccaggag 60
   attgttgata aggttaaacc acagcttgaa gaaaaaacaa atgagactta tggaaaattg 120
   gaagctgtgc agtataaaac tcaagttgtt gctggaacaa attactacat taaggtacga 180
   gcaggtgata ataaatatat gcacttgaaa gtattcaaaa gtcttcccgg acaaaatgag 240
   gacttggtac ttactggata ccaggttgac aaaaacaagg atgacgagct gacgggcttt 300
   tagcagcatg tacccaaagt gttctgattc cttcaactgg ctactgagtc atgatccttg 360
   ctga 364
<210> 608
   <211> 477
   <212> DNA
   <213> Homo sapiens
   <400> 608
   tctgcagcct tgctgttcat tgccaccgtc gacaatgcct ggtgggtagg agatgagttt 60
   tttgcagatg tctggagaat atgtaccaac aacacgaatt gcacagtcat caatgacagc 120
   tttcaagagt actccacgct gcaggcggtc caggccacca tgatcctctc caccattctc 180
   tgctgcatcg ccttcttcat cttcgtgctc cagctcttcc gcctgaagca gggagagagg 240
   tttgtcctaa cctccatcat ccagctaatg tcatgtctgt gtgtcatgat tgcggcctcc 300
   atttatacag acaggcgtga agacattcac gacaaaaacg cgaaattcta tcccgtgacc 360
   agagaaggca gctacggcta ctcctacatc ctggcgtggg tggccttcgc ctgcaccttc 420
   atcagcggca tgatgtacct gatactgagg aagcgcaaat agagttccgg agctggg 477
<210> 609
   <211> 480
   <212> DNA
   <213> Homo sapiens
   <400> 609
   cgcgagggca tcatcaccat agagtcccag gatggaggac ccttcccgca gctgggcagc 60
   cgtgccgggc tcttccagca cccgctgcaa agcgagtaca gcagcatcac caccacccac 120
   accagcgcca ccgagccctt cctagtggat gggccgaccc tgggggccca gcacctggag 180
   gcaggcggct ccctcacccg gcatgtgacc caggagtttg tgagccggac actgaccacc 240
   agcggaaccc ttagcaccca catggaccaa cagttcttcc aaacttgacc gcaccctgcc 300
   ccacccccgc catgtcccac taggcgtcct cccgactcct ctcccggagc ctcctcagct 360
   actccatcct tgcacccctg ggggcccagc ccacccgcat gcacagagca ggggctaggt 420
   gtctcctggg aggcatgaag ggggcaaggt ccgtcctctg tgggcccaaa cctatttgta 480
<210> 610
   <211> 523
   <212> DNA
   <213> Homo sapiens
   <400> 610
   aacagagatg tcccccaggg agcacatcaa gggcaaagag accaccccct ctagcctagc 60
   agtgacccag accatggcca ccaaagctcc cgagtgtgtg gaggacccag atatggcaaa 120
   ccagaggaag actgccctgg agttctgtgg agagacttgg agctctctct gcacattctt 180
   cctcagcata gtgcaggaca cgtcatgcta atgaggtcaa aagagaacgg gttcctttaa 240
   gagatgtcat gtcgtaagtc cctctgtata ctttaaagct ctctacagtc cccccaaaat 300
   atgaactttt gtgcttagtg agtgcaacga aatatttaaa caagttttgt attttttgct 360
   tttgtgtttt ggaatttgcc ttatttttct tggatgcgat gttcagaggc tgtttcctgc 420
   agcatgtatt tccatggccc acacagctat gtgtttgagc agcgaagagt ctttgagctg 480
   aatgagccag agtgataatt tcagtgcaac gaactttctg ctg 523
<210> 611
   <211> 556
   <212> DNA
   <213> Homo sapiens
   <400> 611
   gcagccacca gcgaatgcta ggtctcggac taagcctacc tgctctccaa gtctcagtgg 60
   cttcatctgt caagtgggac tctgtcacac cagccattct tatctctctg tgctgtggaa 120
   gcaacaggaa tcaagagact gccctccttg tccacccacc tatgtgccaa ctgttgtaac 180
   taggctcaga gatgtgcacc catgggctct gacagaaagc agatcctcac cctgctacac 240
   atacaggatt tgaactcaga tctgtctgat aggaatgtga aagcacggac tcttactgct 300
   aacttttgtg tatcgtaacc agccagatcc tcttggttat ttgtttacca cttgtattat 360
   taatgccatt atccctgaat tccccttgcc accccaccct ccctggagtg tggctgagga 420
   ggcctccatc tcatgtatca tctggatagg agcctgctgg tcacagcctc ctctgtctgc 480
   ccttcacccc agtggccact cagcttccta cccacacctc tgccagaaga tcccctcagg 540
   actgcaacag gcttgt 556
<210> 612
   <211> 193
   <212> DNA
   <213> Homo sapiens
   <400> 612
   gtcccaagtg caacaaggag gtgtacttcg ccgagagggt gacctctctg ggcaaggact 60
   ggcatcggcc ctgcctgaag tgcgagaaat gtgggaagac gctgacctct gggggccacg 120
   ctgagcacga aggcaaaccc tactgcaacc acccctgcta cgcagccatg tttgggccta 180
   aaggctttgg gcg 193
<210> 613
   <211> 402
   <212> DNA
   <213> Homo sapiens
   <400> 613
   agacggtgca gtcggctgca tactcccagt cgggagtgtg gtcagtctgc ctgctgctgt 60
   gcggtagctc cagaaccacc tcgttcctgg ttttgtttgg attttggcat cttgtttttc 120
   taacaacaaa caatggagaa aaagaattga ttcttagtga cacagaagat tgccttacgc 180
   tcgtgagcgt gagaagccat aagagagaga ccgaattctg tggctcagca cacaggactg 240
   acccacagcc caggcagcgg gtgtgtggag atggcgccct gtcctgccaa ggggcgccag 300
   gagcagagcc agggcctggc gagctggcgt ggagcccaca ggattcagca gcatggacag 360
   tcactcttgc actattcctt ctccaagcca gaaaccacat tt 402
<210> 614
   <211> 536
   <212> DNA
   <213> Homo sapiens
   <400> 614
   aatgctgaac tccttgttag cccttcagat tgttaggagt ggttctcatt tggtctgcca 60
   gaatactggg ttcttagttg acaacctaga atgtcagatt tctggttgat ttgtaacaca 120
   gtcattctag gatgtggagc tactgatgaa atctgctaga aagttagggg gttcttattt 180
   tgcattccag aatcttgact ttctgattgg tgattcaaag tgttgtgttc cctggctgat 240
   gatccagaac agtggctcgt atcccaaatc tgtcagcatc tggctgtcta gaatgtggat 300
   ttgattcatt ttcctgttca gtgagatatc atagagacgg agatcctaag gtccaacaag 360
   aatgcattcc ctgaatctgt gcctgcactg agagggcaag gaagtggggt gttcttcttg 420
   ggacccccac taagaccctg gtctgaggat gtagagagaa caggtgggct gtattcacgc 480
   cattggttgg aagctaccag agctctatcc ccatccaggt cttgactcat ggcagc 536
<210> 615
   <211> 548
   <212> DNA
   <213> Homo sapiens
   <400> 615
   agccatccca tgttagagct tctcaagagg aagacagccc agactctttc agttctctgg 60
   attctgagat gtgcaaagac taccgagtat tgcccaggat aggctatctt tgtccaaagg 120
   atttaaagcc tgtctgtggt gacgatggcc aaacctacaa caatccttgc atgctctgtc 180
   atgaaaacct gatacgccaa acaaatacac acatccgcag tacagggaag tgtgaggaga 240
   gcagcacccc aggaaccacc gcagccagca tgcccccgtc tgacgaatga caggaagatt 300
   gttgaaagcc atgagggaaa aaataaaccc cagttctgaa tcacctacct tcaccatctg 360
   tatatacaaa gaattcttcg gagcttgtct tatttgctat agaaaacaat acagagcttt 420
   tgggaatgga atcactgatt ttcagtcttt tccatttctt tcctcctaga atctgtgatc 480
   tgagggtata aagacatttc caccaagttt gagccctcaa aatgtcctga ttacaatgct 540
   gtctgtcc 548
<210> 616
   <211> 371
   <212> DNA
   <213> Homo sapiens
   <400> 616
   tttctggcct tcacccagac gaagaccttc cacgaggcca gcgaggactg catctcgcgc 60
   gggggcaccc tgagcacccc tcagactggc tcggagaacg acgccctgta tgagtacctg 120
   cgccagagcg tgggcaacga ggccgagatc tggctgggcc tcaacgacat ggcggccgag 180
   ggcacctggg tggacatgac cggcgcccgc atcgcctaca agaactggga gactgagatc 240
   accgcgcaac ccgatggcgg caagaccgag aactgcgcgg tcctgtcagg cgcggccaac 300
   ggcaagtggt tcgacaagcg ctgccgcgat cagctgccct acatctgcca gttcgggatc 360
   gtgtagccgg c 371
<210> 617
   <211> 545
   <212> DNA
   <213> Homo sapiens
   <400> 617
   tgccgtgggt tttcaagttt actcatttct atggttgcaa ataactctaa aacttattat 60
   ataaactttc atattatagg cagaacacaa tggctaaata tctgttgcat gtactttaaa 120
   gtttattata aaatataaac agatatataa agatgttgac tcttacctgt gattttgcat 180
   ggtcagactc ggtgtcaggt acggagagga ttctcatgac tgtcttacct ctactgaata 240
   ttctagtgag ttatatgatt tacggagtga ttaacagagg tctatataaa gttacttttc 300
   ccctttactt aattatattg tagtgtgcag ataacaaaac tgctaccttc tcatccaagt 360
   ggtctgtaga attcatgtcc cttacagtgg tcatttaaag tcaatattta tttatgtatg 420
   taataaaaaa agttggattt ttgtgtatgt ctgtcacatt atttagagag aagtaatctt 480
   gtaaaaatgt tttgtaaaaa acaaaaaagt attgtaaata gtcttgatat tctgtgactc 540
   attat 545
<210> 618
   <211> 423
   <212> DNA
   <213> Homo sapiens
   <400> 618
   agaggtctcc ctataccgag acccaccatc cttccatcct gaggaccgcc ccaaccctcg 60
   gagcccccca ctcagtaggt ctgaaggcct ccatttgtac cgaaacaccc cgctcacgct 120
   gacagcctcc taggctccct gaggtacctt tccacccaga ccctccttcc ccaccccata 180
   agccctgaga ctcccgcctt tgacctgacg atcttccccc ttcccgcctt caggttcctc 240
   ctaggcgctc agaggccgct ctggggggtt gcctcgagtc cccccacccc tccccaccca 300
   ccaccgctcc cgcggcaagc cagcccgtgc aacggaagcc aggccaactg ccccgcgtct 360
   tcagctgttt cgcatccacc gccaccccac tgagagctgc tcctttgggg gaatgtttgg 420
   caa 423
<210> 619
   <211> 543
   <212> DNA
   <213> Homo sapiens
   <400> 619
   taacatcagc tgcctatgcc tatgataagg tagcagtctg cattcttatg gccattagat 60
   gttacaaact ccttgcctct aaagtcagat catgaaggga taggtgttca tctaaggtta 120
   cagttatgtt accgaaacac aaaactgcca aaatcttact ctgctgttat gaatgtttac 180
   catcagcatt attttatcat ttaatatgtg ctcactgatt gttaactgta gcttcagcgc 240
   gtgccaagca gttgacttaa taggatcatc ttgtgaattt gtttacgtga tgccaagcat 300
   caagtcatgt tttctttagt gtgtgtgctt acacaggtgt taaacagttt ttctctattt 360
   taaactgagc cttcttttta atatattccc gaagagatat gtaaataagc tctcagagtt 420
   tctgtgatga tttgttgagc cttgctggac aagtggtttg tttgtgtgca aaccaaactt 480
   tctttaccca gtgcaataga tttgtttgac tgcttgtgtc tttttatgac ctgtttgcct 540
   ttt 543
<210> 620
   <211> 406
   <212> DNA
   <213> Homo sapiens
   <400> 620
   gcagactggg agttgctagc aaacaaatgg cttacttaca aaagcagctt ttagttcaga 60
   cttagttttt ataaaatgag aattctgact tacttaacca ggtttgggat ggagatggtc 120
   tgcatcagct ttttgtatta acaaagttac tggctctttg tgtgtctcca ggtaactttg 180
   cttgattaaa cagcaaagcc atattctaaa ttcactgttg aatgcctgtc ccagtccaaa 240
   ttgtctgtct gctcttattt ttgtaccata ttgctcttaa aaatcttggt ttggtacagt 300
   tcataattca ccaaaaagtt catataattt aaagaaacac taaattagtt taaaatgaag 360
   caatttatat ctttatgcaa aaacatatgt ctgtctttgc aaagga 406
<210> 621
   <211> 530
   <212> DNA
   <213> Homo sapiens
   <400> 621
   gactctttga aatgacatgt tcccttaagg tactgaagct ttatttgcat atttatttca 60
   gatgtttcga gtaaacttga aaagggtagg cacgaagcaa tttgttgctg cttgtcaccc 120
   ccaagtcccc gtggaggttc tgtattttaa gaaacagtgc gttgagtgta cagattttat 180
   ttatgcgtaa tttaatgggg tctgtaaata ctggtgcact tcttacgact tttttgagac 240
   atgggatcca attttaatat taacttttaa tggtgatggg gtaatctata acacatcata 300
   aggttttatt catatatata cagggtatta agaattaaga ggatgctggg ctctgttctt 360
   ggcttggaag attctattta attgaaactc tctgttcaga aagcaataac tttgtctcgt 420
   tcctgttggg ctgaacccta aggtgagtgt gcagtacagt gtgtgtgggt gaaatggaga 480
   tttggaattg aactctctgc ctgtaaatgt tccccaaata attgttgtgt 530
<210> 622
   <211> 434
   <212> DNA
   <213> Homo sapiens
   <400> 622
   aacggccatt tgggatgcca gggtggatga aaaggtgaag aaatcagggg attgagactt 60
   gggtgggtgg gcatctctca ggagccccat ctccgggcgt gtcacctcct gggcagggtt 120
   ctgggaccct ctgtgggtga cgcacaccct gggatggggc tagtagagcc ttcaggcgcc 180
   ttcgggcgtg gactctggcg cactctagtg gacaggagaa ggaacgcctt ccaggaacct 240
   gtggactagg ggtgcaggga cttccctttg caaggggtaa cagaccgctg gaaaacactg 300
   tcactttcag agctcggtgg ctcacagcgt gtcctgcccc ggtttgcgga cgagagaaat 360
   cgcggcccac aagcatcccc catcccttgc aggctggggg ctgggcatgc tgcatcttaa 420
   ccttttgtat ttat 434
<210> 623
   <211> 417
   <212> DNA
   <213> Homo sapiens
   <400> 623
   ggagtttgtt gacctcatga acagcaaaga atccaagttt accttcaaga tgaatccagg 60
   tgatgtgatt acttttgata actggcgctt acttcatggc cgacgtagct atgaagcagg 120
   aactgagata tcccgccatc tagaaggagc ttatgctgac tgggatgtgg tcatgtcaag 180
   gcttcgtatc ttaaggcaga gggtggagaa tggaaactga agtcacctgt agataatttt 240
   aataagattc caatgaccat attttgtgag atatggcaca ttattcacag accatgatct 300
   ttgtgattta catataattt ccttaacaat gaacatgtaa cttctctcac aagagtactc 360
   tttactttgt aatcatatac aatgtcaact ttttagatgt ttcaccactc ttttgca 417
<210> 624
   <211> 317
   <212> DNA
   <213> Homo sapiens
   <400> 624
   cgccatcacc gagcgcttga tgtgcgcgga gagcaatcgc cgggacagct gcaagggtga 60
   ctccgggggc ccgctggtgt gcgggggcgt gctcgagggc gtggtcacct cgggctcgcg 120
   cgtttgcggc aaccgcaaga agcccgggat ctacacccgc gtggcgagct atgcggcctg 180
   gatcgacagc gtcctggcct agggtgccgg ggcctgaagg tcagggtcac ccaagcaaca 240
   aagtcccgag caatgaagtc atccactcct gcatctggtt ggtctttatt gagcacctac 300
   tatatgcaga aggggag 317
<210> 625
   <211> 383
   <212> DNA
   <213> Homo sapiens
   <400> 625
   ttttgcgtga ccccctgagt ggggaaaggc aggctgttgc atggtggcct gagcgagcag 60
   aattcctcca gggacaatgg cgtctcttgg ccacatcttg gttttctgtg tgggtctcct 120
   caccatggcc aaggcagaaa gtccaaagga acacgacccg ttcacttacg actaccagtc 180
   cctgcagatc ggaggcctcg tcatcgccgg gatcctcttc atcctgggca tcctcatcgt 240
   gctgagcaga agatgccggt gcaagttcaa ccagcagcag aggactgggg aacccgatga 300
   agaggaggga actttccgca gctccatccg ccgtctgtcc acccgcaggc ggtagaaaca 360
   cctggagcga tggaatccgg cca 383
<210> 626
   <211> 317
   <212> DNA
   <213> Homo sapiens
   <400> 626
   gggccacgcc aggaatattc agaaaataat gagaactaca ttgaagtgcc attgattttt 60
   gatcctgtca caagagagga tttgcacatg gattttaaat gtgttgtcca taataccctg 120
   agttttcaga cactacgcac cacagtcaag gaagcctcct ccacgttctc ctggggcatt 180
   gtgctggccc cactttcact ggccttcttg gttttggggg gaatatggat gcacagacgg 240
   tgcaaacaca gaactggaaa agcagatggt ctgactgtgc tatggcctca tcatcaagac 300
   tttcaatcct atcccaa 317
<210> 627
   <211> 397
   <212> DNA
   <213> Homo sapiens
   <400> 627
   gggatagtcc atatgcaagc agctccaaag gaggaatgtg ccctggagat catcaaaggg 60
   ggagctctgc gccaagaaga agtgtattat gacagctcac tctggaccac tcttctgatc 120
   agaaatccat gcaggaagat cctggaattt ctctactcaa cgagctataa tatggacaga 180
   ttcataaaca agtaggaact ccctgagggc tgggcatgct gagggatttt gggactgttc 240
   tgtctcatgt ttatctgagc tcttatctat gaagacatct tcccagagtg tccccagaga 300
   catgcaagtc atgggtcaca cctgacaaat ggaaggagtt cctctaacat ttgcaaaatg 360
   gaaatgtaat aataatgaat gtcatgcacc gctgcag 397
<210> 628
   <211> 561
   <212> DNA
   <213> Homo sapiens
   <400> 628
   attgctgcta cttatataat tgccaaaaag tgaaataatg tgtagttcat gtaaataata 60
   cattatattt ctattttatt atgaagaagg tgaatagcca tatttgtaaa atgacaatca 120
   tgtgtgttaa cccagtgctt tccattcgtg aaaacacatt tgctttttgt gatatgcaca 180
   atgtagataa gtgttctgtc tgactttctt ttttgatata gaagtataaa gaattgtggt 240
   ttatatattt aaaagtgtca agctgagtat taaaatgtat gcatgttgtc taagaaattg 300
   aatacttgaa tgtgtctcac agtttgaaat aagctatttg atgtaatact tcttgtgtgt 360
   atgcacatga aacttagatt ttacatgaag tattttttca gtattatatg taccctctga 420
   aatacatagg gatatgcgta ttataccaaa atgttgctga aaaatgggca cttaaagctt 480
   tcagaatatg tcagtgctga tgtagcatgc ttgttgcaat tgcctttttt ctgtataaat 540
   gtctttaatg caatatactg g 561
<210> 629
   <211> 514
   <212> DNA
   <213> Homo sapiens
   <400> 629
   cagactgttc agtgtttgtc aagcttctgg tctaatatgt actcgaaaga ctttccgctt 60
   acaatttgta gaaacacaaa tatcgttttc catacagcag tgcctatata gtgactgatt 120
   ttaactttca atgtccatct ttcaaaggaa gtaacaccaa ggtacaatgt taaaggaata 180
   ttcactttac ctagcaggga aaaatacaca aaaactgcag atacttcata tagcccattt 240
   taacttgtat aaactgtgtg acttgtggcg tcttataaat aatgcactgt aaagattact 300
   gaatagttgt gtcatgttaa tgtgcctaat ttcatgtatc ttgtaatcat gattgagcct 360
   cagaatcatt tggagaaact atattttaaa gaacaagaca tacttcaatg tattatacag 420
   ataaagtatt acatgtgttt gattttaaaa gggcggacat tttattaaaa tcaatattgt 480
   ttttgctttt tctgaggagt ctctttcagt ttca 514

<210> 630
   <211> 527
   <212> DNA
   <213> Homo sapiens
   <400> 630
   gattctctgt accaagtgat gcagcaatgc tgggaggcag acccagcagt gcgacccacc 60
   ttcagagtac tagtggggga ggtggagcag atagtgtctg cactgcttgg ggaccattat 120
   gtgcagctgc cagcaaccta catgaacttg ggccccagca cctcgcatga gatgaatgtg 180
   cgtccagaac agccgcagtt ctcacccatg ccagggaatg tacgccggcc ccggccactc 240
   tcagagcctc ctcggcccac ttgacttagt tcttgggctg gacctgctta gctgccttga 300
   gctaacccca aggctgcctc tgggccatgc caggccagag cagtggccct ccaccttgtt 360
   cctgcccttt aactttcaga ggcaataggt aaatgggccc attaggtccc tcactccaca 420
   gagtgagcca gtgagggcag tcctgcaaca tgtatttatg gagtgcctgc tgtggaccct 480
   gtcttctggg cacagtggac tcagcagtga ccacaccaac actgacc 527
<210> 631
   <211> 489
   <212> DNA
   <213> Homo sapiens
   <400> 631
   gagggtgatg ccatctaacc ctgcccctgt ccaccccggg tgggtgaaac tcactgagca 60
   gccaagactg ttgcccgagg actcactgta tggtgccctc tccaaagggt cgggagggta 120
   gctctccagg ccagagcttg tgtccttcaa cagagaggcc agcggcaact ggtccgttac 180
   tggccaaggg ctctgaagaa tcaacggtgc tggtacagga tacaggaata aattgtatct 240
   tcacctggtt cctaccctcg tccctacctg tcctgatcct ggtcctgaag acccctcgga 300
   acaccctctc ctggtggcag gccacttccc tcccagtgcc agtctccatc caccccagag 360
   aggaacaggc gggtgggcca tgtggttttc tccttcctgg ccttggctgg cctctggggc 420
   aggggtggtg gagagatgga agggcatcag gtgtagggac cctgccaagt ggcacctgat 480
   ttactctag 489
<210> 632
   <211> 546
   <212> DNA
   <213> Homo sapiens
   <400> 632
   gccaacatca ccatcattga gcaccagaag tgtgagaacg cctaccccgg caacatcaca 60
   gacaccatgg tgtgtgccag cgtgcaggaa gggggcaagg actcctgcca gggtgactcc 120
   gggggccctc tggtctgtaa ccagtctctt caaggcatta tctcctgggg ccaggatccg 180
   tgtgcgatca cccgaaagcc tggtgtctac acgaaagtct gcaaatatgt ggactggatc 240
   caggagacga tgaagaacaa ttagactgga cccacccacc acagcccatc accctccatt 300
   tccacttggt gtttggttcc tgttcactct gttaataaga aaccctaagc caagaccctc 360
   tacgaacatt ctttgggcct cctggactac aggagatgct gtcacttaat aatcaacctg 420
   gggttcgaaa tcagtgagac ctggattcaa attctgcctt gaaatattgt gactctggga 480
   atgacaacac ctggtttgtt ctctgttgta tccccagccc caaagacagc tcctggccat 540
   atatca 546
<210> 633
   <211> 493
   <212> DNA
   <213> Homo sapiens
<220>
   <221> misc_feature
   <222> (87)..(87)
   <223> n is a, c, g, or t
   <400> 633
   cactgctagc agggcttcaa ccaggaaggg atcaacccag gaagggatga tcaggagagg 60
   cttccctgag gacataatgt gtaaganagg tgagaagtgc tcccaagcag acacaacagc 120
   agcacagagg tctggaggcc acacaaaaag tgatgctcgc cctgggctag cctcagcaga 180
   cctaaggcat ctctactccc tccagaggag ccgcccagat tcctgcagtg gagaggaggt 240
   cttccagcag cagcaggtct ggagggctga gaatgaacct gactagaggt tctggagata 300
   cccagaggtc ccccaggtca tcacttggct cagtggaagc cctctttccc caaatcctac 360
   tccctcagcc tcaggcagtg gtgctcccat cttcctcccc acaactgtgc tcaggctggt 420
   gccagccttt cagaccctgc tcccagggac ttgggtggat gcgctgatag aacatcctca 480
   agacagtttc ctt 493
<210> 634
   <211> 489
   <212> DNA
   <213> Homo sapiens
   <400> 634
   agtatttccc atttatcgca gacctttttt aggaagcaag cttaatggct gataatttta 60
   aattctctct cttgcaggaa ggactatgaa aagctagaat tgagtgttta aagttcaaca 120
   tgttatttgt aatagatgtt tgatagattt tctgctactt tgctgctatg gttttctcca 180
   agagctacat aatttagttt catataaagt atcatcagtg tagaacctaa ttcaattcaa 240
   agctgtgtgt ttggaagact atcttactat ttcacaacag cctgacaaca tttctatagc 300
   caaaaatagc taaatacctc aatcagtctc agaatgtcat tttggtactt tggtggccac 360
   ataagccatt attcactagt atgactagtt gtgtctggca gtttatattt aactctcttt 420
   atgtctgtgg attttttcct tcaaagttta ataaatttat tttcttggat tcctgataat 480
   gtgcttctg 489
<210> 635
   <211> 155
   <212> DNA
   <213> Homo sapiens
   <400> 635
   gcaacggaag agtcctgggc ggaaggaggc ttctgtatgc ttgtgaaaaa gaacaatctg 60
   tgccaacgga aggttcttca acaactttgc tgcaaaacat gtacatttca aggctgagca 120
   gccatcttag atttctttgt tcctgtagac ttata 155
<210> 636
   <211> 355
   <212> DNA
   <213> Homo sapiens
   <400> 636
   tgggttaagc ctgcagggat cccggtgctc tgtctcctgt gaagatggac ggtatttcaa 60
   cggccaggac tgccagccct gccaccgctt ctgcgccact tgtgctgggg caggagctga 120
   tgggtgcatt aactgcacag agggctactt catggaggat gggagatgcg tgcagagctg 180
   tagtatcagc tattactttg accactcttc agagaatgga tacaaatcct gcaaaaaatg 240
   tgatatcagt tgtttgacgt gcaatggccc aggattcaag aactgtacaa gctgccctag 300
   tgggtatctc ttagacttag gaatgtgtca aatgggagcc atttgcaagg atgca 355
<210> 637
   <211> 469
   <212> DNA
   <213> Homo sapiens
   <400> 637
   agcctatcct taataaatcc tccactctct ggaaggagac tgaggggctt tgtaaaacat 60
   tagtcagttg ctcattttta tgggattgct tagctgggct gtaaagatga aggcatcaaa 120
   taaactcaaa gtatttttaa atttttttga taatagagaa acttcgctaa ccaactgttc 180
   tttcttgagt gtatagcccc atcttgtggt aacttgctgc ttctgcactt catatccata 240
   tttcctattg ttcactttat tctgtagagc agcctgccaa gaattttatt tctgctgttt 300
   tttttgctgc taaagaaagg aactaagtca ggatgttaac agaaaagtcc acataaccct 360
   agaattctta gtcaaggaat aattcaagtc agcctagaga ccatgttgac tttcctcatg 420
   tgtttcctta tgactcagta agttggcaag gtcctgactt tagtcttaa 469
<210> 638
   <211> 455
   <212> DNA
   <213> Homo sapiens
   <400> 638
   gcttctgtca ctgaattatc tcccaagtgc tggcagactg aatgttgatg tcattcgagc 60
   caagcaactt cttcagacag atgtgagcca aggttcagac ccctttgtga aaatccagct 120
   ggtgcatgga ctcaaacttg tgaaaaccaa gaagacgtcc ttcttaaggg gcacaattga 180
   tcctttctac aatgaatcct tcagcttcaa agttccccaa gaagaactgg aaaatgccag 240
   cctagtgttt acagttttcg gccacaacat gaagagcagc aatgacttca tcgggaggat 300
   cgtcattggc cagtactctt caggcccctc tgagaccaac cactggaggc gcatgctcaa 360
   cacgcaccgc acagccgtgg agcagtggca tagcctgagg tcccgagctg agtgtgaccg 420
   cgtgtctcct gcctccctgg aggtgacctg agggc 455
<210> 639
   <211> 418
   <212> DNA
   <213> Homo sapiens
   <400> 639
   ggaactctaa accttgtgat gactactaac aaatgtaaaa ttatgagtga ttaagaaaac 60
   attgctttgt ggttatcact ttaagttttg acacctagat tatagtctta gtaatagcat 120
   ccactggaaa aggtgaaaat gttttattca gcatttaact tacatttgta ctttagagta 180
   tttttgtata aaatccatag atttatttta catttagagt atttacacta tgataaagtt 240
   gtaaataatt ttctaagaca gtttttatat agtctacagt tgtcctgatt tcttattgaa 300
   tttgttagac tagttctctt gtcttgtgat ctgtgtacaa ttttagtcac taagactttc 360
   ctccaagaac taagccaact tgatgtgaaa agcacggctg tatataatgg tgatgtca 418
<210> 640
   <211> 505
   <212> DNA
   <213> Homo sapiens
   <400> 640
   taagacttgt actatgtgtg gccatgaact gacatatgaa aaaatgtgat tttttagttc 60
   agtgacctgt tttatagaat tttatattta aataaaggaa atttagattg gtccttttca 120
   aaattcaaaa aaaaaagcaa catcttcata gatgaatgaa acccttgtat aagtaatact 180
   tcagtaataa ttatgtatgt tatggcttaa aagcaagttt cagtgaaggt cacctggcct 240
   ggttgtgtgc acaatgtcat gtctgtgatt gccttcttac aacagagatg ggagctgagt 300
   gctagagtag gtgcagaagt ggtaggtcag ctacaaattt gaggacaaga taccaaggca 360
   aaccctagat tggggtagag ggaaaagggt tcaacaaagg ctgaactgga ttcttaacca 420
   agaaacaaat aatagcaatg gtggtgcacc actgtacccc aggttctagt catgtgtttt 480
   ttaggacgat ttctgtctcc acgat 505
<210> 641
   <211> 533
   <212> DNA
   <213> Homo sapiens
   <400> 641
   atcctacaac ccaccttgaa ggtataactg gatccagaga gggaaggact gacaagaagg 60
   aattattcag aaaaacactg acagatgttt tataaattgt acagaaaaat agttaaaaat 120
   gcaataggtt gaagttttcc agatatgttt ctctctgaaa ttactgtgaa tatttaacaa 180
   acacttactt gatctatgtt atgaaataag tagcaaattg ccagcaaaat gtcttgtacc 240
   ttttctaaag tgtattttct gatgtgaact tccttcccct tacttgctag gtttcaataa 300
   tttaaaagag tcaaacacta taaatgagta agttgacgat gttttaagat tgcacctggc 360
   agtgtgcctt tttgcaacaa atatttacct ggcagtgtgc ctttttgcaa caaatattta 420
   ctttgcactt ggagctgctt ttaattttag caaaatgttt tatgcaaggc acaataggaa 480
   gtcagttctc ctgcacttcc tcctcatgta gtctggagta ctttctaaag ggc 533
<210> 642
   <211> 493
   <212> DNA
   <213> Homo sapiens
   <400> 642
   ttgaacaaac cctcactgag cacctctgat gttgagcacc tgctgaatac tgagcactga 60
   atgggggagg gggaggggag cacggggtga gtcaacctgg gactcggtct cagggatatg 120
   cctaccaata gcgggtatcg taaggcatgt acccaaacat aacggatgta aggcagaaag 180
   tgatcggaga aggaatgaga aagtgtgcgt gatgttaatg aaaagtcata tgcagctaga 240
   gcagacccag gaaagctttc tggaagagat tgcatctgag gaaattcagg aaggatcttt 300
   gtagattggg gggagattct aaattgaagg ggtgataggg tgaggggcca gagggaagtc 360
   tgctgtgttc tcatgtagga tgtcagccct ccctgcaact tctctttttg gccaatgtct 420
   tttcactttc ctgacccttt agaatcatcc ccagccagac gcaatcatgg aagttgcctt 480
   attgtcactg gtt 493
<210> 643
   <211> 555
   <212> DNA
   <213> Homo sapiens
   <400> 643
   caccacctac ctatgatgcc gtggtacaga tggagtacct tgacatggtg gtgaatgaaa 60
   cactcagatt attcccagtt gctattagac ttgagaggac ttgcaagaaa gatgttgaaa 120
   tcaatggggt attcattccc aaagggtcaa tggtggtgat tccaacttat gctcttcacc 180
   atgacccaaa gtactggaca gagcctgagg agttccgccc tgaaaggttc agtaagaaga 240
   aggacagcat agatccttac atatacacac cctttggaac tggacccaga aactgcattg 300
   gcatgaggtt tgctctcatg aacatgaaac ttgctctaat cagagtcctt cagaacttct 360
   ccttcaaacc ttgtaaagaa acacagatcc ccttgaaatt agacacgcaa ggacttcttc 420
   aaccagaaaa acccattgtt ctaaaggtgg attcaagaga tggaacccta agtggagaat 480
   gagttattct aaggacttct actttggtct tcaagaaagc tgtgccccag aacaccagag 540
   atttcaactt agtca 555
<210> 644
   <211> 300
   <212> DNA
   <213> Homo sapiens
   <400> 644
   ttctttaggg ctcttcctac agccttgaga agtagatagg catcagagta tggtactata 60
   ggaatcagaa aaattcaaaa caaatgtgga ttaagtgttt aggctctatg tggctcacgc 120
   agccagaatc cttaagtctg tgtgtttctg tgtctcaaga ctgggctcac attctggctt 180
   tgtccataac aatgctctgg gatttcaggg agttccctca tttgtaaaat gagggggtca 240
   gagcaggtga tatccatgtt tcttcccttt ctgatattgt tgtctgtggc atattctttg 300
<210> 645
   <211> 551
   <212> DNA
   <213> Homo sapiens
<220>
   <221> misc_feature
   <222> (114)..(114)
   <223> n is a, c, g, or t
   <220>
   <221> misc_feature
   <222> (119)..(120)
   <223> n is a, c, g, or t
   <220>
   <221> misc_feature
   <222> (127)..(127)
   <223> n is a, c, g, or t
   <220>
   <221> misc_feature
   <222> (129)..(129)
   <223> n is a, c, g, or t
   <220>
   <221> misc_feature
   <222> (149)..(149)
   <223> n is a, c, g, or t
   <220>
   <221> misc_feature
   <222> (152)..(152)
   <223> n is a, c, g, or t
   <220>
   <221> misc_feature
   <222> (163)..(163)
   <223> n is a, c, g, or t
   <220>
   <221> misc_feature
   <222> (167)..(168)
   <223> n is a, c, g, or t
   <220>
   <221> misc_feature
   <222> (189)..(189)
   <223> n is a, c, g, or t
   <220>
   <221> misc_feature
   <222> (243)..(243)
   <223> n is a, c, g, or t
   <400> 645
   ctgctacttt ggaagatggc tctggaggaa actctcatat ggctaaaaag gcaggctagt 60
   ttcttacttc tacaggggta gagccttaaa aaagaacgtg ctacaaattg gttntcttnn 120
   agggttncng gttctccctg cccccaatnc cnatatactt tantgcnntt ttatttttgc 180
   ctttacggnc tctgtgtctt tctgcaagaa ggcctggcaa aggtatgcct gctgttggtc 240
   ccntcgggat aagataaaat ataaataaaa ccttcagaac tgttttggag caaaagatag 300
   cttgtacttg gggaaaaaaa ttctaagttc ttttatatga ctaatattct tggttagcaa 360
   gactggaaag aggtgttttt ttaaaatgta cataccagaa caaagaacat acagctctct 420
   gaacatttat tttttgaaca gaggtggttt ttatgtttgg acctggtaat acagatacaa 480
   aaactttaat gaggtagcaa tgaatattca actgtttgac tgctaagtgt atctgtccat 540
   attttagcaa g 551
<210> 646
   <211> 468
   <212> DNA
   <213> Homo sapiens
   <400> 646
   tctgcagtga gtgcaaccgc accttcccca gccacacggc tctcaaacgc cacctgcgct 60
   cacatacagg cgaccacccc tacgagtgtg agttctgtgg cagctgcttc cgggatgaga 120
   gcacactcaa gagccacaaa cgcatccaca cgggtgagaa accctacgag tgcaatggct 180
   gtgacaagaa gttcagcctc aagcatcagc tggagacgca ctatagggtg cacacaggtg 240
   agaagccctt tgagtgtaag ctctgccacc agcgctcccg ggactactcg gccatgatca 300
   agcacctgag aacgcacaac ggcgcctcgc cctaccagtg caccatctgc acagagtact 360
   gccccagcct ctcctccatg cagaagcaca tgaagggcca caagcccgag gagatcccgc 420
   ccgactggag gatagagaag acgtacctct acctgtgcta tgtgtgaa 468
<210> 647
   <211> 416
   <212> DNA
   <213> Homo sapiens
   <400> 647
   tcaagtcctc tggtggcagt tccagcgtga ggtttgtttc taccacttat tccggagtaa 60
   ccagataaag agatgccctc tgtttcatta gctctagttc tcccccagca tcactaacaa 120
   atatgcttgg caagaccgag gtcgatttgt cccagcctta ccggagaaaa gagctatggt 180
   tagttacact agctcatcct attcccccag ctctttcttt tctgctgttt cccaatgaag 240
   ttttcagatc agtggcaatc tcagttccct tgctatgacc ctgctttgtt ctttcccgag 300
   aaacagttca gcagtgacca ccacccacat gacatttcaa gcaccacctt aagccagcca 360
   gagtaggacc agttagacct agggtgtgga cagctccttg catcttaaca ctgtgc 416
<210> 648
   <211> 555
   <212> DNA
   <213> Homo sapiens
   <400> 648
   tcaggtgacc tgaatctttc ccttaaccgt acagtttctc gatggaattg tgtgatcaga 60
   aggtggaatt ctagtgatag gcgacctcag acccgcattc atgttctgtg tgcctcttct 120
   attgcacata cactgatttt tagcattgtc tattcctatt tttcctttgc ccattgtact 180
   tccatatatc ttttcattaa cttacttgct gccttttttt tttcttggta cacatttaaa 240
   taaagtaatc cttaacctgt gctgtaaagt tcacccttgg catgctgttc caagaacctg 300
   ggtttgaatc ccaatcgttg tgaaacatac tcagtattga taaaaccttt ttaataagtg 360
   atgcagagca gccaaggata tgttgaccca gatgtcaacc aggctatttt tatacttaaa 420
   acatgtcagc agagcatagg cagaataaaa tggtttaaat accccacagc aaatagagta 480
   actgacaaac caccaaaaac tgaaacccca gacccaccag aaagacaagt gtctagcaat 540
   gccttggtac ctgat 555
<210> 649
   <211> 343
   <212> DNA
   <213> Homo sapiens
   <400> 649
   ctgcccagcc tgagtggctc agatgggatc ccgtatcgaa ccgtctctga gtggctcgag 60
   tccatacgca tgaaacgcta catcctgcac ttccactcgg ctgggctgga caccatggag 120
   tgtgtgctgg agctgaccgc tgaggacctg acgcagatgg gaatcacact gcccgggcac 180
   cagaagcgca ttctttgcag tattcaggga ttcaaggact gatccctcct ctcaccccat 240
   gcccaatcag ggtgcaagga gcaaggacgg ggccaaggtc gctcatggtc actccctgcg 300
   ccccttccca caacctgcca gactaggcta tcggtgctgc ttc 343
<210> 650
   <211> 438
   <212> DNA
   <213> Homo sapiens
<220>
   <221> misc_feature
   <222> (195)..(195)
   <223> n is a, c, g, or t
   <400> 650
   atcactattt tgaagcacag ctttacagat gagtatctat gatacatatg tataataaat 60
   tttgattggg tattaaaagt attagaaggt ggttataatt gcagagtatt ccatgaatag 120
   tacactgaca caggggtttt actttgagga ccagtgtagt caagggaaaa catgagttaa 180
   aaagaaaagc aggcnatatt gcagtcttga ttctgccact tacaggatag ataacgcctg 240
   aactttaatg acaagatgat ccaaccataa aggtgctctg tgcttcacag tgaatctttt 300
   ccccatgcag gagtgtgctc ccctacaaac gttaagactg atcatttcaa aaatctatta 360
   gctatatcaa aagccttaca ttttaatata ggttgaacca aaatttcaat tccagtaact 420
   tctattgtaa ccattatt 438
<210> 651
   <211> 389
   <212> DNA
   <213> Homo sapiens
   <400> 651
   tccccaagac tttactagtg ccgataaact ttctcaaaga gcaaccagta tcacttccct 60
   gtttataaaa cctctaacca tctctttgtt ctttgaacat gctgaaaacc acctggtctg 120
   catgtatgcc cgaatttgta attcttttct ctcaaatgaa aatttaattt tagggattca 180
   tttctatatt ttcacatatg tagtattatt atttccttat atgtgtaagg tgaaatttat 240
   ggtatttgag tgtgcaagaa aatatatttt taaagctttc atttttcccc cagtgaatga 300
   tttagaattt tttatgtaaa tatacagaat gttttttctt acttttataa ggaagcagct 360
   gtctaaaatg cagtggggtt tgttttgca 389
<210> 652
   <211> 385
   <212> DNA
   <213> Homo sapiens
   <400> 652
   aaacagttgc tcacctacag acagtgcaac ataaattagc agaattaaaa acacatatat 60
   gtgtaacccg agcatttgtg gacaactgtc tccagctgca tgaagcgaaa cgtttggact 120
   ccgccactgc ttgcatggcg aaatattggg catctgagtt acaaaatagt gtagcttacg 180
   actgtgtaca gctccatgga ggttggggat acatgtggga gtacccaatt gcaaaagctt 240
   atgtggatgc cagagttcag ccaatctatg gtggtacaaa tgaaataatg aaggagctga 300
   ttgcaagaga gattgtcttt gacaagtaga catctgccca catcctggag tcctattaca 360
   gctaatctcg ttttaaatct gctca 385
<210> 653
   <211> 464
   <212> DNA
   <213> Homo sapiens
   <400> 653
   gtagactcgg ctgcggagta ctaccgcctc cacttggagg gctaccacgg caccgcaggg 60
   gactccatga gctaccacag cggcagtgtc ttctctgccc gtgatcggga ccccaacagc 120
   ttgctcatct cctgcgctgt ctcctaccga ggggcctggt ggtacaggaa ctgccactac 180
   gccaacctca acgggctcta cgggagcaca gtggaccatc agggagtgag ctggtaccac 240
   tggaagggct tcgagttctc ggtgcccttc acggaaatga agctgagacc aagaaacttt 300
   cgctccccag cggggggagg ctgagctgct gcccacctct ctcgcacccc agtatgactg 360
   ccgagcactg aggggtcgcc ccgagagaag agccagggtc cttcaccacc cagccgctgg 420
   aggaagcctt ctctgccagc gatctcgcag cactgtgttt acag 464
<210> 654
   <211> 479
   <212> DNA
   <213> Homo sapiens
   <400> 654
   gaccttcccg ctgcggacag ggaagaggca acctggccag cggcggcccg ctctgggggc 60
   cggggtacgc gaccacccaa ccgagcagag gctttgggta cagacccccc agctactcga 120
   cagcctacct gcctggcagc tatggctctt cccactgcaa actggaagcc ccctcaccgt 180
   gctccctccc tcagagtgac cctaggctcc agggggaact gctgcccacc tatacccact 240
   acctgccccc tggctctccc actccataca accctcccct tgctggtgcc cccatgcccc 300
   taacccacct ctaaccctca tggacgcaga cctcacggga cgggcctcat cctccttttt 360
   taatccagca gcatccccta ccccaggctg tcaacccttt ctcctgttgg actacagttc 420
   agaggcagcc tgcagtcctc ccatgatagc cagggagagc cgcacaacat acaattata 479
<210> 655
   <211> 469
   <212> DNA
   <213> Homo sapiens
   <400> 655
   tcatcaggct ccagttattc tccatctccc agctcagctt tttctgtctg taagcctgat 60
   tttcaggaag gctctttcct agtgatggag atgaccacca tcagctccag gcttctatcc 120
   tgctaaccca gtaacccagt gggaagagat ttacttattc caataattcc aagtggagag 180
   tgtcattgac ccgtttgggg tctcatctct acttctaggg gaatgaaaca ctttgagtgg 240
   ccaggcctgt gtcatgtgct aattcctaga gccagggaaa taaggtctga ggattcagga 300
   tggggtgaaa ggtggttgct taaaggaaaa tgaaatacaa ttagcagaat aaggggaaac 360
   gagtggtctg ctctgctcgg gcaaaacaag agatgcccat tactgtgagg gacccttgaa 420
   gtctggactc ttaaatgggt ttttgctgat ttcctgggtg catgctagg 469
<210> 656
   <211> 445
   <212> DNA
   <213> Homo sapiens
   <400> 656
   aaggaagggc atccttctgc cttttttatt tttttaagct gtaaaaagag agaaaactta 60
   tttgagtgat tatttgttat ttgtacagtt cagttcctct ttgcatggaa tttgtaagtt 120
   tatgtctaaa gagctttagt cctagaggac ctgagtctgc tatattttca tgacttttcc 180
   atgtatctac ctcactattc aagtattagg ggtaatatat tgctgctggt aatttgtatc 240
   tgaaggagat tttccttcct acacccttgg acttgaggat tttgagtatc tcggaccttt 300
   cagctgtgaa catggactct tcccccactc ctcttatttg ctcacacggg gtattttagg 360
   cagggatttg aggagcagct tcagttgttt tcccgagcaa aggtctaaag tttacagtaa 420
   ataaaatgtt tgaccatgcc ttcat 445
<210> 657
   <211> 535
   <212> DNA
   <213> Homo sapiens
   <400> 657
   ccatcacctt ctcaactggg aaacccctga aatgctctca gagcacctct gacgcctgaa 60
   gaagttatac cttcctcttc ccctttacca aataaagcaa agtcaaacca tcatctggaa 120
   acagtggcca cttttcactg acctctcttc gacatctagt caacccaccc aatatgccac 180
   tgggtttcgc tcccaattcc accccaccct ccattacaga gctcaccacg ccctcctaga 240
   tcaccgtccc caacacaccc attgcctctc aaggccctta tctcagcccc ttcctgtggc 300
   catttccctc agtgcccaga tgattccctg ggtgagggag acactggggc accctcagag 360
   gttggagcag gctccctgct gtccctggat cctggacaga tggctcagta aactgtggga 420
   ctaggtgcag acttttgcct tcttggagtc ctgggtctcc tctgagaggt ctgggtggtg 480
   ctcctcctac gcctctagag gtctctgtgt tcctcatttt ccttcaaaag cgggc 535
<210> 658
   <211> 522
   <212> DNA
   <213> Homo sapiens
   <400> 658
   aaataggcac tcacaatgac aaccagagcc agtttcttgt ctttttatac attttgtcat 60
   cccagagact cggtatttgc ttactgtgtt tcaagtagag gaaatcgtgg tcttgaacta 120
   ttctgtacca cagcaaacaa tctatgttgc tttactatca actgctgtaa tcgtttataa 180
   aacttaccta gctccttccc ttcttctatc atagctttaa acattagaat tcataggcaa 240
   atcagttaaa acattaggat cataggcaaa tcagttacct tgcagaaaga gctttgtatg 300
   acagacattg tcttatttta tttctgtaaa atattagctg tatgaatatg atttaattaa 360
   caagaaaaca tttcttcctg attgacaaca gtgttagcaa ggtgcaaagc gaaactggtt 420
   gctcaagttg atagaaaaca aaattctgaa tatcttcaaa ttaattcggt aaaaacacat 480
   tattttttca tatgtgatgt attcatgcag aacaactatc tt 522
<210> 659
   <211> 567
   <212> DNA
   <213> Homo sapiens
   <400> 659
   cgcttctgca agaccacgaa cacagtggag cctctgaggg ggaatctggt gaagaaggac 60
   tgtgcggagt cgtgcacacc cagctacacc ctgcaaggcc aggtcagcag cggcaccagc 120
   tccacccagt gctgccagga ggacctgtgc aatgagaagc tgcacaacgc tgcacccacc 180
   cgcaccgccc tcgcccacag tgccctcagc ctggggctgg ccctgagcct cctggccgtc 240
   atcttagccc ccagcctgtg accttccccc cagggaaggc ccctcatgcc tttccttccc 300
   tttctctggg gattccacac ctctcttccc cagccggcaa cgggggtgcc aggagcccca 360
   ggctgagggc ttccccgaaa gtctgggacc aggtccaggt gggcatggaa tgctgatgac 420
   ttggagcagg ccccacagac cccacagagg atgaagccac cccacagagg atgcagcccc 480
   cagctgcatg gaaggtggag gacagaagcc ctgtggatcc ccggatttca cactccttct 540
   gttttgttgc cgtttatttt gtactca 567
<210> 660
   <211> 392
   <212> DNA
   <213> Homo sapiens
   <400> 660
   ggctggctca agaagcacgc gtactgctcc aacctcagct tccgcctcta cgaccagtgg 60
   cgagcctgga tgcagaagtc gcacaagacc cgcaaccagg acgaggggat cctgccctcg 120
   ggcagacggg gcacggcgag aggtcctgcc agataagctg taggggctca ggccaccctc 180
   cctgccacgt ggagacgcag aggccgaacc caaactgggg ccacctctgt accctcactt 240
   cagggcacct gagccaccct cagcaggagc tggggtggcc cctgagctcc aacggccata 300
   acagctctga ctcccacgtg aggccacctt tgggtgcacc ccagtgggtg tgtgtgtgtg 360
   tgtgagggtt ggttgagttg cctagaaccc ct 392
<210> 661
   <211> 196
   <212> DNA
   <213> Homo sapiens
   <400> 661
   ttttcataac tgagcccact cgcaagttgg agccatcagt gggatacgcc acattttgga 60
   agccccagca tcgtgtactt accagtgtgt tcacaaaatg aaatttgtgt gagagctgta 120
   cattaaaaaa aacatcatta ttattattat ttgcagtcat ggagaaccac ctacccctga 180
   cttctgttta gtctcc 196

<210> 662
   <211> 489
   <212> DNA
   <213> Homo sapiens
   <400> 662
   aaagcccttc atctaatatt tgttgctatt gccaattttt caatgaaatg acctaaaaac 60
   aacaaaaaaa aataacctat acggtagttg ctttaggggg tggggggatg ctatctgtta 120
   gtgcttaaaa gggggtaaat gcttgccgct ttagaggtgg atggtgctca taaaaggccc 180
   cagtcggggg tatttaaaaa ggactgaaca gaaatcctta gctagtagaa tggcagcacg 240
   ctgtaaaatt attactgtat tgtgtactgg ctataagatg tagacacctt tcagtaagcc 300
   aatcatttgt aaccattcta gcagtgtcat attaggttaa taaggctgct gtgttttaaa 360
   gggcattttt atttgggttt tggtgaaatt ctttaatttg ttgattatat tcacataaaa 420
   tcagcattca ttgacacata gctctaatga catatgtatg aaaaaccata cactggatga 480
   cctagtcga 489
<210> 663
   <211> 386
   <212> DNA
   <213> Homo sapiens
   <400> 663
   cgccctggca cggtgctgag aattcgcggc ttggttcctc ccaatgccag caggttccat 60
   gtaaacctgc tgtgcgggga ggagcagggc tccgatgccg ccctgcattt caacccccgg 120
   ctggacacgt cggaggtggt cttcaacagc aaggagcaag gctcctgggg ccgcgaggag 180
   cgcgggccgg gcgttccttt ccagcgcggg cagcccttcg aggtgctcat catcgcgtca 240
   gacgacggct tcaaggccgt ggttggggac gcccagtacc accacttccg ccaccgcctg 300
   ccgctggcgc gcgtgcgcct ggtggaggtg ggcggggacg tgcagctgga ctccgtgagg 360
   atcttctgag cagaagccca ggcggc 386
<210> 664
   <211> 523
   <212> DNA
   <213> Homo sapiens
   <400> 664
   gagagggcat atgcatcctc tgtcctgatc taggtgtcta tagctgaggg gtaagaggtt 60
   gttgtagttg tcctggtgcc tccatcagac tctccctact tgtcccatat ttgcaagggg 120
   aggggatttg gggctggggc tccattcacc aaagctgagg tggcttctca ttaacccctt 180
   aggactctga agggtatgga cctacgtgaa tgtgtgtcag ggggagactt gctggtgggt 240
   tagtggtcct caggatgtga taaaaacatc cagtgtaaaa aggaagttgg aatgggagtt 300
   ggcgggcagt gaacgagtgt ggggaaggat tggtgctggg gcaacaggaa ggggcctggg 360
   gccgtttggc tgcactaact ttggtagctc agtgtgcatc taaagtggga ctggggaggg 420
   agctaagctt gggctgggct gcttggggct tggcataggg tggaaagggc taccctgggg 480
   cttctgaccc ccctgtagta tgtgtggagg gtgccctccc gtc 523
<210> 665
   <211> 446
   <212> DNA
   <213> Homo sapiens
   <400> 665
   aagagggccc agcaaggtaa tttatggttg agctgatgtc aattggttct tgtcttgagt 60
   cgactcaatt tagcccaagt gctgaaacaa gaaatgtcat ttttttcatc aaagacacca 120
   gggcagattt ttaagtaaag aaagacaatt ggacccttaa gaatttatgc atttgtaaag 180
   ttgctgttga tccaaatatt ttcaagccat gtaatccatt ggttttgtgg gcagtttaat 240
   aaacctgaac ctttgtgtgt tttctaattg tacctgagtt gaccatcctt tctttttata 300
   gtatatttct tgtatgatat tttgtaaagc tctcacctgg ttcttttatg gggacttttc 360
   gtttttgggc aactccagtg tatttatgtg aaactttata agagaattaa tttttccatt 420
   tgcatattaa tatgttcctc cacaca 446
<210> 666
   <211> 554
   <212> DNA
   <213> Homo sapiens
   <400> 666
   gttttggttt tgactcacct gaaagttttt ttggtttaaa agaagaatag gcggggcacg 60
   gtggctcatg cctgtaatcc cagcactttg ggaggctgag gcaggtggat cacgaggtca 120
   ggagatcgac accatcctgg ctaacacggt gaaaccccgt ctctactaaa aaatacaaaa 180
   aattagctgg gtgtggtggt gggggtgggc gcctgtgatc ccagctacgt gggaggctga 240
   ggcagcagac tggtgtgaac ccgggaggtg gagcttgcag tgagccgaga tcgcgccact 300
   gcactccagc ctgggcgaca gagcgagact ccatctcaaa aaaaaaaaaa aaaaaaagaa 360
   agaaagaaaa ataattttgg gagtttctgg aaaggtacta ggatttctca aaaggatttg 420
   tcttctccct tgtgaaagac agatgtcaga ctaatcaggc ttatccgatg tgctacatga 480
   gatggaaatg cgtgtgaaat agtaagtcac actaagtctt ctggaggttc tatttacggg 540
   tttggtttga tatg 554
<210> 667
   <211> 504
   <212> DNA
   <213> Homo sapiens
   <400> 667
   gaaagagcta gattctctct ctcaaaaaaa aaaaaaaaaa ggaaagaaag aaagaaaaga 60
   aaagaaaatc cctttttgct ttaacttgcc cttgcaggtt tgtagaaact caattgttga 120
   aatttgggtg gataaatttc tggattttct atctattcca tgttggacca ataccacact 180
   gccctagtca ctgttgcatt atagtatatc tttaaaggag taatgggaat ccttcaacta 240
   catttttttc cccaataatt tttggctatt ctgcttcttt tgtgtttcta tgtaaatttt 300
   atcatcagtg tgtctatttc tacaaatagt cctgataggg tttgaattgg gatttctgtg 360
   aatctataga tcaatctgag gagacttaat aatgatattg attctcccaa ttcatgaata 420
   tagtataccc ctgtatttat ttgttttctt gaatttcttt tatcattgtt ttgtagtttt 480
   caccatgaca gtcttgcaca tatt 504
<210> 668
   <211> 342
   <212> DNA
   <213> Homo sapiens
   <400> 668
   caaaggcatt acctgcctca tcgatattat aggggtccat cacaacccaa ctgtgtggcc 60
   ggatcctgag gtctacgacc ccttccgctt tgacccagag aacagcaagg ggaggtcacc 120
   tctggctttt attcctttct ccgcagggcc caggaactgc atcgggcagg cgttcgccat 180
   ggcggagatg aaagtggtcc tggcgttgat gctgctgcac ttccggttcc tgccagacca 240
   cactgagccc cgcaggaagc tggaattgat catgcgcgcc gagggcgggc tttggctgcg 300
   ggtggagccc ctgaatgtag gcttgcagtg actttctgac cc 342
<210> 669
   <211> 463
   <212> DNA
   <213> Homo sapiens
   <400> 669
   gagagattat ttctgtggtc taaaggttaa aaagccaaca acctgttacc aattatttca 60
   gctttttttg ttttaataag tgtgacaact taaaacttgt ttctatttaa agtgaaatgt 120
   atctttcaac tgtttagtta cccagctgtt taatattcca gtcttcccaa agtgaaaaga 180
   tttgtataca aatgttttct atgatttaat aaaaatatat ggcacaaaaa accacttcgc 240
   cgggtcgcgc cccgacggcc gggcccggga gacgcgccgg cagccccggc accttgccaa 300
   agtttcaaac ccgggaaaat aaacgtaagc taaggatccc ccccatgtat ccaacctcat 360
   gctctatggg acccaggcca tccccgtgag gttctccaga tcctccatgc cttggacgaa 420
   aggtgttgga tcactggtgc atcatgacac caaatctata gtt 463
<210> 670
   <211> 459
   <212> DNA
   <213> Homo sapiens
   <400> 670
   tgagcctggg gttctggtgt tagaatattt ttaagtaggc tttactgaga gaaactaaat 60
   attggcatac gttatcagca acttcccctg ttcaatagta tgggaaaaat aagatgactg 120
   ggaaaaagac acacccacac cgtagaacat atattaatct actggcgaat gggaaaggag 180
   accattttct tagaaagcaa ataaacttga tttttttaaa tctaaaattt acattaatga 240
   gtgcaaaata acacataaaa tgaaaattca cacatcacat ttttctggaa aacagacgga 300
   ttttacttct ggagacatgg catacggtta ctgacttatg agctaccaaa actaaattct 360
   ttctctgcta ttaactggct agaagacatt catctatttt tcaaatgttc tttcaaaaca 420
   tttttataag taatgtttgt atctatttca tgctttact 459
<210> 671
   <211> 265
   <212> DNA
   <213> Homo sapiens
   <400> 671
   ccggaaccga cgagtcctga ggagagaacc ggtgcgtcct gaggagagaa ccggcgctgg 60
   gcaacacggg cctgcaaact cgacaggacc ctgcccgagg ggccctcgcg ccaacctgga 120
   ccggtccccg cctcctccgc tgcccaatct ctcagaccca ccccacctgc aggcccagac 180
   cacgtgggac agaactcctg cccaccctac cccgagggag gcgaacccgc acttccaggc 240
   ttgggaggac catggggcac aatgc 265
<210> 672
   <211> 478
   <212> DNA
   <213> Homo sapiens
   <400> 672
   gagtggaatg cttcctagaa gttactgaat gcaccatggt caaaacggat tagggcattt 60
   gagaaatgca tattgtatta ctagaagatg aatacaaaca atggaaactg aatgctccag 120
   tcaacaaact atttcttata tatgtgaaca tttatcaatc agtataattc tgtactgatt 180
   tttgtaagac aatccatgta aggtatcagt tgcaataata cttctcaaac ctgtttaaat 240
   atttcaagac attaaatcta tgaagtatat aatggtttca aagattcaaa attgacattg 300
   ctttactgtc aaaataattt tatggctcac tatgaatcta ttatactgta ttaagagtga 360
   aaattgtctt cttctgtgct ggagatgttt tagagttaac aatgatatat ggataatgcc 420
   ggtgagaata agagagtcat aaaccttaag taagcaacag cataacaagg tccaagat 478
<210> 673
   <211> 513
   <212> DNA
   <213> Homo sapiens
<220>
   <221> misc_feature
   <222> (215)..(215)
   <223> n is a, c, g, or t
   <400> 673
   aatcacccaa ggatggatat caggagaata tctctggaaa atacatacaa actgtttatt 60
   caacttctga taggtctgtc attgaaagag atatgtgcac ttactgccga aaacccttgg 120
   gtgtagaaac taaaatgatt ttagatgaat tacaaatttg ctgccattct acttgcttta 180
   agtgtgaaat atgcaagcag cctttggaaa atctncaagc gggtgatagt atttggattt 240
   atagacagac aatacactgt gaaccttgct actctaaaat tatggcaaag tggattccat 300
   aactctggca caaggaaatc aagatgaaaa gcactcatta aggaattaaa gttacaagtt 360
   ttatcttaat aatatgtaat ctagaaaagc tttcacattg aagatcaact cttgtacaaa 420
   attaacaatt ctgttattgc ataagtaatc taattgtctt caataaggtc acacacataa 480
   aaagagccat ctggtctctg gctagagtta gca 513
<210> 674
   <211> 514
   <212> DNA
   <213> Homo sapiens
   <400> 674
   gaatattttc cacaagatgc tgcaatgtga gttatcactt catttatctt aaagaaagac 60
   taaactggtt gtcagttaca tctgacagaa aaaaaaaaaa aatcactgtg taaccaggtt 120
   aagtggtaaa ataatccagg gcgtcagtca aaggcatttt gctgacttta atattgatta 180
   tatttttaac agggaattta aggaaaatat tacctgggaa ttaaaaaata tatatatatt 240
   aaaacaagaa ttttcctttg cctctgtcta gcttaaacct actacctcaa gctgcttaag 300
   ttccttaagt attgtttgta atcaccaata aataagtgca tttgtaattc atcagtcatt 360
   attagctttt attaaaagaa gattacgttt tacaatgtaa ctataatctc ttgaatttgg 420
   tatcttatta atgagtttta aagatgtaaa acctaacctt ttttaaagct ccattgtctt 480
   atgtttttag aggcttttcc gtaaacatat atct 514
<210> 675
   <211> 387
   <212> DNA
   <213> Homo sapiens
   <400> 675
   tccagcggag gccacaagtc ctcctcttcc gggtccgtgg gcgagtcttc atctaaggga 60
   ccaagatact aacaaaacca gagtaatcaa gacaattatt gaagaggtgg cgcccgacgg 120
   tagagttctt tcatctacgg ttgaatcaga aaccaagaaa cactactatt aaactgcatg 180
   aatctccctt cacacagacc attatttaca gatgcatgga aaacaaagtc tccaagaaaa 240
   cacttctgtc ttgatggtct atggaaatag accttgaaaa taaggtgtct acaaggtgtt 300
   ttgtggtttc cgtatttctt cttttcactt taccagaaag tgttctttaa tggaaagaaa 360
   aacaactttc tgttctcatt tactaat 387
<210> 676
   <211> 520
   <212> DNA
   <213> Homo sapiens
   <400> 676
   ttcaccatgg accgggaagt gcgcaaaatc aaacaaggcc tgggcttgaa atttgctgag 60
   ctggtgtata ccggtttacg gcctagccct gagtgtgaat ttgtccgcca ctgcatcgcc 120
   aagtcccagg agcgagtgga agggaaagtg caggtgtccg tcctcaaggg ccaggtgtac 180
   atcctcggcc gggagtcccc actgtctctc tacaatgagg agctggtgag catgaacgtg 240
   cagggtgatt atgagccaac tgatgccacc gggttcatca acatcaattc cctcaggctg 300
   aaggaatatc atcgtctcca gagcaaggtc actgccaaat agacccgtgt acaatgagga 360
   gctggggcct cctcaatttg cagatccccc aagtacaggc gctaattgtt gtgataattt 420
   gtaattgtga cttgttctcc ccggctggca gcgtagtggg gctgccaggc cccagctttg 480
   ttccctggtc cccctgaagc ctgcaaacgt tgtcatcgaa 520
<210> 677
   <211> 465
   <212> DNA
   <213> Homo sapiens
   <400> 677
   gcactatggt ttgttgccta cctagctgca tctataatgt cagcttatcc taaggctgtc 60
   cacgtactta atttacttaa gtgttcattt taagtaacgt gctcactgtg tataggaatt 120
   tgtattttgg aggtgcttga tctatctaca aaagaaaaaa ttaattagga attactttat 180
   tataaaatgc tcctagaagt cttaattgtg tttatttttt aaaaaaacaa atgttagact 240
   tgtgtgcatg gaagtaatta aggtacatca ttattgtagt ttgaaagttg tacatgataa 300
   gacattttgt ttttactgta tgtttttact gaatgatcta ttccccatcc caaggcaagc 360
   atgaataaaa ttaggttaaa cgtagcatgt ggcatcgcag tctcttagaa tttgtttcat 420
   ctattttatt ttattgaata ctgtctgtat ctttggttat cctgt 465
<210> 678
   <211> 548
   <212> DNA
   <213> Homo sapiens
   <400> 678
   agtctgctga agaggcattg cacagatcaa acaaggatgg atcatttctt attcggaaaa 60
   gctctggcca tgattccaaa caaccatata cactagttgt attctttaat aagcgagtat 120
   ataatattcc tgtgcgattt attgaagcaa caaaacaata tgccttgggc agaaagaaaa 180
   atggtgaaga gtactttgga agtgttgctg aaatcatcag gaatcatcaa catagtcctt 240
   tggttcttat tgacagtcag aataacacaa aagattccac cagactgaag tatgcagtta 300
   aagtttcata aagggggaaa aaaaagatca ataccattgc ttcagacact ttcccaaagt 360
   ttctcctttt gagaaaaagt cccaaaactt catattttgg attatgaatc atccagtaat 420
   aaaatggaag atggagtcag ctattgaagt ggtcatccat ttctttttaa gaagctcatg 480
   tggacttgtt ctattgcctg acctgatgaa ctgttaatat ctggtgaggt tgagttatca 540
   tgctacta 548
<210> 679
   <211> 345
   <212> DNA
   <213> Homo sapiens
   <400> 679
   gggattggca gcggctgcat catcagtggc gggggctccg tctgcggagg tggttcctct 60
   ggaggcggcg gcggcggctc ctccgtgggt ggctccggga gtggcaaggg cgtcccgatc 120
   tgccaccaga cccagcagaa gcaggcgcct acctggccgt ccaaatagat cccccagggt 180
   accacggagg cgaaggagtt ggaggtgttt tccaggggca ccgatgggct tagagctctc 240
   atgatgctac ccgaggtttg caaatccttc atgtcttaac ctacctggaa gaagccattg 300
   agctctccgg ctgcatctag ttctgctgtt tagcctcttt ggttt 345
<210> 680
   <211> 474
   <212> DNA
   <213> Homo sapiens
   <400> 680
   ttattcttag cgtcactggt ctggctttca gaattaacat acaaggttgc cacacctagt 60
   tctgcccagc tttatgtctt ttattccagt attccaccaa agtttgtttt cctgcattcc 120
   agttctcaag tcttaagata aagattgtac ttgacagttt agtatatcca taaaactatt 180
   tgaggtggtt aaggttcttg ggttcatttt ccttaatact ttgctgaata ttgtagattg 240
   taggcaatga aaaagtctac taaattagga aaaccttgaa taattaggta tcctaggtaa 300
   gagcccctaa acatcaagca atctgtgagt ctgtaaagaa ataaatattt tttggattat 360
   tcttatctaa ttccacccct gttggaagat gatttctttg ttctttgcaa ctatggaagc 420
   tgtgaaaatc atcacaagtg cctctgaaag cgagtgttag gttggttaga gggt 474
<210> 681
   <211> 479
   <212> DNA
   <213> Homo sapiens
   <400> 681
   gctggaggtg acgctactga gaactttgag gatgtcgggc actctacaga tgccagggaa 60
   atgtccaaaa cattcatcat tggggagctc catccagatg acagaccaaa gttaaacaag 120
   cctccagaac cttaaaggcg gtgtttcaag gaaactctta tcactactat tgattctagt 180
   tccagttggt ggaccaactg ggtgatccct gccatctctg cagtggccgt cgccttgatg 240
   tatcgcctat acatggcaga ggactgaaca cctcctcaga agtcagcgca ggaagagcct 300
   gctttggaca cgggagaaaa gaagccattg ctaactactt caactgacag aaaccttcac 360
   ttgaaaacaa tgattttaat atatctcttt ctttttcttc cgacattaga aacaaaacaa 420
   aaagaactgt cctttctgcg ctcaaatttt tcgagtgtgc ctttttattc atctacttt 479
<210> 682
   <211> 460
   <212> DNA
   <213> Homo sapiens
   <400> 682
   tgaagctttt ggttccagcg tgaccttctc ttttagataa agatgagccc ccaccaccac 60
   cgactctccc aacccagact ctcccactcc agaatgtaga agcctgtctc tgtacctcta 120
   actggcagca agttaaattt ttgtcattta tctctgatgg cactttgagg gaaaagaatg 180
   tccacataca gtttttgaaa gatcttctct ccaaaccagt tagttagagc cagtgacgcc 240
   tctgtgttct ggggcggaat ctgtgctgtc taggtttgtg cttctagcca tgcccattcc 300
   cgcccccacc atgcctcttt gcattgccca ttttccagat gtgtattctg ttgaggaccc 360
   aggcccatcc agggatttca tctctaagcc tggcagtgct ggggggaaat gtgtttctgt 420
   gtatatagct cctcttgtcc actctgcttt cggaagtgct 460
<210> 683
   <211> 493
   <212> DNA
   <213> Homo sapiens
   <400> 683
   gtgagttatc acttcattta tcttaaagaa agactaaact ggttgtcagt tacatctgac 60
   agaaaaaaaa aaaaaaatca ctgtgtaacc agggttaagt ggttaaaata atccagggcg 120
   tcagtcaaag gcattttgct gactttaata ttgattatat ttttaacagg gaatttaagg 180
   aaaatattac cggggaatta aaaaatatat atatattaaa acaagaattt tcctttgccc 240
   ctgtccagcc taaacctacc tacctcaagg ctgcctaagt tcctaagtat tgtttgtaat 300
   cacccaataa ataagtgcat ttgtaattca tcagtcatta ttagctttta ttaaaagaag 360
   attacgtttt acaatgtaac tataatctct tgaatttggt atcttattaa tgagttttaa 420
   agatgtaaaa cctaaccttt tttaaagctc cattgtctta tgtttttaga ggcttttccg 480
   taaacatata tct 493
<210> 684
   <211> 343
   <212> DNA
   <213> Homo sapiens
   <400> 684
   aaggaagagt ctaggctgag caacatgaag gggcccccaa ccttctgcag cctcctgctg 60
   ctgtcattgc tcctgagccc agaccctaca gcagcattcc tactgccacc cagcactgcc 120
   tgctgtactc agctctaccg aaagccactc tcagacaagc tactgaggaa ggtcatccag 180
   gtggaactgc aggaggctga cggggactgt cacctccagg ctttcgtgct tcacctggct 240
   caacgcagca tctgcatcca cccccagaac cccagcctgt cacagtggtt tgagcaccaa 300
   gagagaaagc tccatgggac tctgcccaag ctgaattttg gga 343
<210> 685
   <211> 522
   <212> DNA
   <213> Homo sapiens
   <400> 685
   ctaaaatttg ttaccacatc attgcttcct ttctacagga cgaattgagg cttaaacttt 60
   actgttaatg atactggttc attttaatgt gcttgttggt atgttgctat ttttcatttc 120
   atagctttca aaaatcatgc taattgtata cttgtctagt ttaaggctat tttaaaatat 180
   gtacaatact attcacagca tttagttcgt ttaattttta ttataaagca atctactaaa 240
   aaagtacaac tgtatttgaa cttttcaata gttgtttgtg agctatgata atcaaaagtc 300
   attaaagtct tttttaacaa acattcgtgc ttacttttca acataattcc cagttatata 360
   cagaaaaaga tttccacctg tcacgtatct gcctctttta cctgagcaat ggtgtagttc 420
   ttagacctaa ggtctgtaat tgcaatactt ttaaagaaag atgttgctct aagtgctgtt 480
   tgttagttat gaaatcagat ttttctgctt gttcttaatg ct 522
<210> 686
   <211> 555
   <212> DNA
   <213> Homo sapiens
   <400> 686
   catttactac agtgtctcag ccttgataaa gggcagtgga ttgcctgttg ttcggtgttg 60
   tgaatagcac ctctgaataa gattagagtg tttcttaatt catttcaaac tctaaaatta 120
   gattaatggt ggtgctaaga aagagtatta attactttgg gaatggtcaa aattaacatt 180
   aaaaacattt tagacaaaaa gtttcattgt acattcaaag aaaatgtaag tttggaagta 240
   ctaaaagact attttatact tgttgattaa tcggaatgtt tgttgtatgc cttcattttc 300
   catttcactt atatgtgcat gtccatatat gttaattttc attgtagcaa agctaatgga 360
   aataaagcta atgctctagt tgaaagaaaa ggaaaactcc tgaaatccta gaatgtcttg 420
   ttatttttag ctgactgtaa aatattatga acagtctttg tgtattgtgc ttaatgcttt 480
   tgtaagaaac agaatttgaa atatttcatc cttgtcatgc tcaaaatttt gttacatgct 540
   tgttattcag agtat 555
<210> 687
   <211> 455
   <212> DNA
   <213> Homo sapiens
   <400> 687
   gaaatttttg tcactcccag aggtgagaca agccatccac gtggggaatc agacttttaa 60
   tgatggaact atagttgaaa agtacttgcg agaagataca gtacagtcag ttaagccatg 120
   gttaactgaa atcatgaata attataaggt tctgatctac aatggccaac tggacatcat 180
   cgtggcagct gccctgacag agcactcctt gatgggcatg gactggaaag gatcccagga 240
   atacaagaag gcagaaaaaa aagtttggaa gatccttaaa tctgacagtg aagtggctgg 300
   ttacatccgg caagcgggtg actcccatca ggtaattatt cgaggtggag gacatatttt 360
   accctatgac cagcctctga gagcttttga catgattaat cgattcattt atggaaaagg 420
   atgggatcct tatgttggat aaactacctt cccga 455
<210> 688
   <211> 382
   <212> DNA
   <213> Homo sapiens
   <400> 688
   gatagcaaac actgggggca ccttaagatt ttgcacctgt aaagtgcctt acagggtaac 60
   tgtgctgaat gctttagatg aggaaatgat ccccaagtgg tgaatgacac gcctaaggtc 120
   acagctagtt tgagccagtt agactagtcc cccggtctcc cgattcccaa ctgagtgtta 180
   tttgcacact gcactgtttt caaataacga ttttatgaaa tgacctctgt cctccctctg 240
   atttttcata ttttcctaaa gtttcgtttc tgttttttaa taaaaagctt tttcctcctg 300
   gaacagaaga cagctgctgg gtcaggccac ccctaggaac tcagtcctgt actctggggt 360
   gctgcctgaa tccattaaaa at 382
<210> 689
   <211> 451
   <212> DNA
   <213> Homo sapiens
   <400> 689
   agcaggtctc ccacgagtaa tggtgggaga agccgggcct acatgccccc gcggagccgc 60
   agccgggacg acctctatga ccaagacgac tcgagggact tcccacgctc ccgggacccc 120
   cactacgacg acttcaggtc tcgggagcgc cctcctgccg accccaggtc ccaccaccac 180
   cgtacccggg accctcggga caacggctcc aggtccgggg acctccccta tgatgggcgg 240
   ctactggagg aggctgtgag gaagaagggg tcggaggaga ggaggagacc ccacaaggag 300
   gaggaggaag aggcctacta cccgcccgcg ccgcccccgt actcggagac cgactcgcag 360
   gcgtcccgag agcgcaggct caagaagaac ttggccctga gtcgggaaag tttagtcgtc 420
   tgatctgacg ttttctacgt agcttttgta t 451
<210> 690
   <211> 358
   <212> DNA
   <213> Homo sapiens
   <400> 690
   ggagcagtgg actgccacaa gccaccatgt aacccctctc acctgccgtg cgttctggct 60
   gtggaccagt aggactcaag gtggacgtgc gttctgcctt ccttgttaat tttgtaataa 120
   ttggagaaga tttatgtcag cacacactta cagagcacaa atgcagtata taggtgctgg 180
   atgtatgtaa atatattcaa attatgtata aatatatatt atatatttac aaggagttat 240
   tttttgtatt gattttaaat ggatgtccca atgcacctag aaaattggtc tctctttttt 300
   taatagctat ttgctaaatg ctgttcttac acataatttc ttaattttca ccgagcag 358
<210> 691
   <211> 473
   <212> DNA
   <213> Homo sapiens
   <400> 691
   cccctgaacg tgttttgcga catggagact gatgggggcg gctggctggt gttccagcgc 60
   cgcatggatg gacagacaga cttctggagg gactgggagg actatgccca tggttttggg 120
   aacatctctg gagagttctg gctgggcaat gaggccctgc acagcctgac acaggcaggt 180
   gactactcca tgcgcgtgga cctgcgggct ggggacgagg ctgtgttcgc ccagtacgac 240
   tccttccacg tagactcggc tgcggagtac taccgcctcc acttggaggg ctaccacggc 300
   accgcagggg actccatgag ctaccacagc ggcagtgtct tctctgcccg tgatcgggac 360
   cccaacagct tgctcatctc ctgcgctgtc tcctaccgag gggcctggtg gtacaggaac 420
   tgccactacg ccaacctcaa cgggctctac gggagcacag tggaccatca ggg 473
<210> 692
   <211> 521
   <212> DNA
   <213> Homo sapiens
   <400> 692
   tagcccttgt ttttaacaca cgctccagcc cttcatcagc ctgggcagtc ttaccaaaat 60
   gtttaaagtg atctcagagg ggcccatgga ttaacgccct catcccaagg tccgtcccat 120
   gacataacac tccacacccg ccccagccaa cttcatgggt cactttttct ggaaaataat 180
   gatctgtaca gacaggacag aatgaaactc tgcgggtctt tggcctgaaa gttgggaatg 240
   gttgggggag agaagggcag cagcttattg gtggtctttt caccattggc agaaacagtg 300
   agagctgtgt ggtgcagaaa tccagaaatg aggtgtaggg aattttgcct gccttcctgc 360
   agacctgagc tggctttgga atgaggttaa agtgtcaggg acgttgcctg agcccaaatg 420
   tgtagtgtgg tctgggcagg cagaccttta ggttttgctg cttagtcctg aggaagtggc 480
   cactcttgtg gcaggtgtag tatctggggc gagtgttggg g 521
<210> 693
   <211> 388
   <212> DNA
   <213> Homo sapiens
   <400> 693
   ctgggattac aggcttgagc ccccgcgccc agccatcaaa atgcttttta tttctgcata 60
   tgtttgaata ctttttacaa tttaaaaaaa tgatctgttt tgaaggcaaa attgcaaatc 120
   ttgaaattaa gaaggcaaaa tgtaaaggag tcaaactata aatcaagtat ttgggaagtg 180
   aagactggaa gctaatttgc ataaattcac aaacttttat actctttctg tatatacatt 240
   ttttttcttt aaaaaacaac tatggatcag aatagccaca tttagaacac tttttgttat 300
   cagtcaatat ttttagatag ttagaacctg gtcctaagcc taaaagtggg cttgattctg 360
   cagtaaatct tttacaactg cctcgaca 388
<210> 694
   <211> 565
   <212> DNA
   <213> Homo sapiens
   <400> 694
   aatgctcaga agttgcctat gtgtgacaaa tgtggcactg ggattgttgg tgtgtttgtg 60
   aagctgcggg accgtcaccg ccaccctgag tgttatgtgt gcactgactg tggcaccaac 120
   ctgaaacaga agggccattt ctttgtggag gatcaaatct actgtgagaa gcatgcccgg 180
   gagcgagtca caccacctga gggttatgaa gtggtcactg tgttccccaa gtgagccagc 240
   agatctgacc actgttctcc agcaggcctc tgctgcagct ttttctctca gtgttctggc 300
   cctctcctct cttgaaagtt ctctgcttac tttggttttc cctctgcttg taaaacattg 360
   agtcccctcc ctgccttggt taattgactc acaccagctg tgcgatgccc gcttttacaa 420
   ttaaaggaaa actgttttgt tcagtgtcac cttgtcagca acactgtgtc ccttcgcccc 480
   accgttcttc tctgctgcat ttggacatca gccaaatttg aacccaatca aatataacgt 540
   gtctgacact gattttgttt ttact 565
<210> 695
   <211> 564
   <212> DNA
   <213> Homo sapiens
   <400> 695
   tagaccatct ccatttttag cacttggcag cctcatgatc cttttataaa tgtgagatta 60
   acaggagagc agcaatacga ttttgccaat ggaataacag atttgccggc attcactgaa 120
   agagggcaga tattgggtcc ttgtaacttc aactgactct tccaaattgt atgaatttat 180
   caatgtatta cacaaatcca gtttcagaat gataaaaaat gttagaccaa ataatgcggc 240
   taattaacag tcgtatgatt tctagcccat gggtttaaaa ctgtatctta aagagtcatt 300
   ttaaaataat ataaatatta aaaaatgtaa ctgctatctt aatgttctga aataaaacat 360
   tttaaaatat aaatcctgta gtttaaaagg aagaaatggt gggaaggaaa agtagagaaa 420
   gaaatgccaa ttacaggcca aagcgttatt tgccaagttt tcttagaatg aattttacca 480
   atgtatgagt tcttgttaac agaatgtgta acggaaatac tgaaagactt ttgcttaaag 540
   tggcattatt gactgctgat gtga 564
<210> 696
   <211> 480
   <212> DNA
   <213> Homo sapiens
<220>
   <221> misc_feature
   <222> (431)..(431)
   <223> n is a, c, g, or t
   <400> 696
   gaaggctgga ttctatctac ataagtcctt tcaattccac cagggccaga gcagctccac 60
   cactgtgcac ttagccatga tggcaacaga aaccaagaga cacaattacg caggtattta 120
   gaagcagagg gacaaccaga aggcccttaa ctatcaccag tgcatcacat ctgcacactc 180
   tcttctccat tccctagcag gaacttctag ctcatttaac agataaagaa actgaggccc 240
   acggtttcag ctagacaatg atttggccag gcctagtaac caaggccctg tctctggcta 300
   ctccctggac cacgaggctg attcctctca tttccagctt ctcagtttct gcctgggcaa 360
   tggccagggg ccaggagtgg ggagagttgt gatggagggg agaggggtca cacccacccc 420
   ctgcctggtt ntaggctgct gcacaccaag gccctgcatc tgtctgctct gcatatatgt 480

<210> 697
   <211> 525
   <212> DNA
   <213> Homo sapiens
   <400> 697
   atttagtcaa ctggcccaag gcagcgaggc ttctacagtc ccacacccca tagccgcctg 60
   ggctggggct tactgggggc tgaaggttct ggacatgaac aagggtcagg tagaagagaa 120
   aggcttcccc tacaccccag cctcctgctg tcccctgaag cccaggactg cgttgtatgc 180
   tttccatcca ctcaccttac cccatagcat cttgcggccc agaaaccaga gccatttgtc 240
   tcagacccta aatcaataat cacaaacccc aaaacgggag agagcagtga aaacatgcag 300
   ggctgtggac gggggaaggg ttgtggcggg tgttctgagg ctgagaggac acctatatgc 360
   gtatttcctc tacacacatc accccccttc tataatctta agccatgact agcctggtgg 420
   cgtgttagtt tctgcccagt tctaccccct catgtgcttc ttctgaatac tgaatgtgac 480
   tgtttgaaag ctggtagaat tcatccctct tactgtagat aacac 525
<210> 698
   <211> 552
   <212> DNA
   <213> Homo sapiens
   <400> 698
   atgtcatccg tatttcaacc ggagtaaatt gctagatttc tgcaagtcga aagatattgt 60
   tctggttgcc tatagtgctc tgggatctca acgagacaaa cgatgggtgg acccgaactc 120
   cccggtgctc ttggaggacc cagtcctttg tgccttggca aaaaagcaca agcgaacccc 180
   agccctgatt gccctgcgct accagctgca gcgtggggtt gtggtcctgg ccaagagcta 240
   caatgagcag cgcatcagac agaacgtgca ggtttttgag ttccagttga ctgcagagga 300
   catgaaagcc atagatggcc tagacagaaa tctccactat tttaacagtg atagttttgc 360
   tagccaccct aattatccat attcagatga atattaacat ggagggcttt gcctgatgtc 420
   taccagaagc cctgtgtgtg gatggtgacg cagaggacgt ctctatgccg gtgactggac 480
   atatcacctc tacttaaatc cgtcctgttt agcgacttca gtcaactaca gctgagtcca 540
   taggccagaa ag 552
<210> 699
   <211> 503
   <212> DNA
   <213> Homo sapiens
   <400> 699
   ttacagtgca gtttagttaa tctattaata ctgactcagt gtctgccttt aaatataaat 60
   gatatgttga aaacttaagg aagcaaatgc tacatatatg caatataaaa tagtaatgtg 120
   atgctgatgc tgttaaccaa agggcagaat aaataagcaa aatgccaaaa ggggtcttaa 180
   ttgaaatgaa aatttaattt tgtttttaaa atattgttta tctttattta tttgggggta 240
   atattgtaag ttttttagaa gacaattttc ataacttgat aaattatagt tttgtttgtt 300
   agaaaagtag ctcttaaaag atgtaaatag atgacaaacg atgtaaataa ttttgtaaga 360
   ggcttcaaaa tgtttatacg tggaaacaca cctacatgaa aagcagaaat cggttgctgt 420
   tttgcttctt tttccctctt atttttgtat tgtggtcatt tcctatgcaa ataatggagc 480
   aaacagctgt atagttgtag aat 503
<210> 700
   <211> 497
   <212> DNA
   <213> Homo sapiens
   <400> 700
   gtgaaacaat tccagggcat gcccccttgc acatacacaa tgccaagtca gtttcttcca 60
   caacaggcca cttactttcc cccgtcacca ccaagctcag agcctggaag tccagataga 120
   caagcagaga tgctccagaa tttaacccca cctccatcct atgctgctac aattgcttct 180
   aaactggcaa ttcacaatcc aaatttaccc accaccctgc cagttaactc acaaaacatc 240
   caacctgtca gatacaatag aaggagtaac cccgatttgg agaaacgacg catccactac 300
   tgcgattacc ctggttgcac aaaagtttat accaagtctt ctcatttaaa agctcacctg 360
   aggactcaca ctggtgaaaa gccatacaag tgtacctggg aaggctgcga ctggaggttc 420
   gcgcgatcgg atgagctgac ccgccactac cggaagcaca caggcgccaa gcccttccag 480
   tgcggggtgt gcaaccg 497
<210> 701
   <211> 505
   <212> DNA
   <213> Homo sapiens
   <400> 701
   tgaacgaatt tattttcccc tcagtttttg agggcattaa aaaggcatta aatcaagaca 60
   aatcatgtgc ttgagaaaaa taaaattaat gaaaacacag cacttatgtt ggtttagctg 120
   cagcctcctt ggaggtagaa tttatttatt taaaattact ggttgcatca agaacccata 180
   gggtgtacaa aaggttctat aaaatctgca ttatagagac aaagaggcag gcaaatccat 240
   gtcacaaggg taaagcttac agtttacaaa ctgggaacgc cagggtgtag gatataaaaa 300
   cgcactcttg agaaaacaaa tgtaatcagg gtgctgaaaa cttgcatggt gctttcagac 360
   attagccttg ttcaacaaat ttcttgtatt gacagatcca tagtgtgcat gggcagacac 420
   attttgcctc tatgtctctt aaaattttaa ttaaaaatac tctttccagt aatcctaatt 480
   tgcacgaaga tataatgtcc acatt 505
<210> 702
   <211> 450
   <212> DNA
   <213> Homo sapiens
   <400> 702
   gcagcactta caatcactaa ttcccttaag gttgaaactg taatgacata aaaagggtcg 60
   atgatatttc actgatggta gatcgcagcc cctgcaacgt agcctttgtt acatgaagtc 120
   cgctgggaaa tagatgttct gtctctatga caatatattt taactgactt tctagatgcc 180
   ttaatatttg catgataagc tagttttatt ggtttagtat tcttgttgtt tacgcatgga 240
   atcactattc ctggttatct caccaacgaa ggctaggagg cggcgtcaga ggtgctgggt 300
   gacagagcca tgagccagcc attttataag cactctgatt tctaaaagtt aaaaaaaata 360
   tatgaaatct ctgtagcctt tagttatcag tacagattta ttaaatttcg gcccttaacc 420
   cagccttttc cagtgtgtaa cccagtttga 450
<210> 703
   <211> 542
   <212> DNA
   <213> Homo sapiens
   <400> 703
   tgcggaaata cctgaaatac agcaaaaata tcctggaccg gcaagatcct ccctctgtgg 60
   tggtcaccag ccaccaggcc ccaggagaaa agaagaaact gaagtgcctg gcctacgact 120
   tctacccagg gaaaattgat gtgcactgga ctcgggccgg cgaggtgcag gagcctgagt 180
   tacggggaga tgttcttcac aatggaaatg gcacttacca gtcctgggtg gtggtggcag 240
   tgcccccgca ggacacagcc ccctactcct gccacgtgca gcacagcagc ctggcccagc 300
   ccctcgtggt gccctgggag gccagctagg aagcaagggt tggaggcaat gtgggatctc 360
   agacccagta gctgcccttc ctgcctgatg tgggagctga accacagaaa tcacagtcaa 420
   tggatccaca aggcctgagg agcagtgtgg ggggacagac aggaggtgga tttggagacc 480
   gaagactggg atgcctgtct tgagtagact tggacccaaa aaatcatctc accttgagcc 540
   ca 542
<210> 704
   <211> 503
   <212> DNA
   <213> Homo sapiens
   <400> 704
   gaattctcga actgcatgta ttgtgccaat ctgtcctgag tgttcatgct ttgtaccaaa 60
   tttaatgaac gcgtgttctg taatcaaact gcaaatattg tcataaccaa catccaaaat 120
   gacggctgct atatataagt gtttgtcata tggaatttaa tcgtaagcca tgatcataat 180
   gttaactaaa taactttatg tggcactgcc tagtaaggga actatggaaa ggtttggatt 240
   tctccaaatc tgggagaatt ttcaaaataa gaaaataacc tttatatgat atactatgac 300
   taggctgtgt atttcttttc agggattttt ctaccttcag ggttggatgt agtttagtta 360
   ctattaccat agccaacctg tagttttaca tatacatttt cttgtggagc aatagagttc 420
   tccattttac agaagcattt taaatgtagt ttgaatattt tccacaagat gctgcaatgt 480
   gagttatcac ttcatttatc tta 503
<210> 705
   <211> 396
   <212> DNA
   <213> Homo sapiens
<220>
   <221> misc_feature
   <222> (148)..(149)
   <223> n is a, c, g, or t
   <220>
   <221> misc_feature
   <222> (151)..(154)
   <223> n is a, c, g, or t
   <220>
   <221> misc_feature
   <222> (156)..(156)
   <223> n is a, c, g, or t
   <400> 705
   agtcaaatgc caaacactag ctctgtatta atccccatca ttactggtaa agcctcattt 60
   gaatgtgtga attcaataca ggctatgtaa aatttttact aatgtcatta ttttgaaaaa 120
   ataaatttaa aaatacattc aaaattanna nnnnanacaa gcttaattgt taatattccc 180
   taaacacaat tttatgaagg gagaagacat tggtttgttg acaataacag tacatctttt 240
   caagttctca gctatttctt ctacctctcc ctatcttaca tttgagtatg gtaacttatg 300
   tcatctatgt tgaatgtaag cttataaagc acaaagcata catttcctga ctggtctaga 360
   gaactgatgt ttcaatttac ccctctgcta aataaa 396
<210> 706
   <211> 49
   <212> DNA
   <213> Homo sapiens
   <400> 706
   gtctttgcta taccactgac tgtattgaaa accaaagtat taagagggg 49
   <210> 707
   <211> 262
   <212> DNA
   <213> Homo sapiens
   <400> 707
   ggatcgcagt catccagaga tgtgacctcc tccagccgcc aaatccgcac caaggtcatg 60
   gatgtgcacg atggcaaggt ggtgtccacc cacgagcagg tccttcgcac caagaactga 120
   ggctgcccag ccccgctcag gcctaggagg ccccccgtgt ggacacagat cccactggaa 180
   gatcccctct cctgcccaag cacttcacag ctggaccctg cttcaccctc accccctcct 240
   ggcaatcaat acagcttcat ta 262
<210> 708
   <211> 396
   <212> DNA
   <213> Homo sapiens
   <400> 708
   ggcaaactgc ttaatcttgt ggattttgta gatggtttca aatgactgaa ctgcattcag 60
   atttacgagt gaaaggaaaa attgcattag ttggttgcat gaactttgaa gggcagatat 120
   tactgcacaa actgccatct cgcttcattt ttttaactat gcatttgagt acagactaat 180
   ttttaaaata tgctaaactg gaagattaaa cagatgtggc ccaaactgtt ctggatcagg 240
   aaagtcatac tgttcacttt caagttggct gtcccccccg ccgccccccc ccacccccat 300
   atgtacagat gataataggg tgtggaatgt cgtcagtggc aaacatttca cagattattt 360
   tgtttctgtc ttcaacattt ttgacactgt gctaat 396
<210> 709
   <211> 455
   <212> DNA
   <213> Homo sapiens
   <400> 709
   gctggaggtg acgctactga gaactttgag gatgtcgggc actctacaga tgccagggaa 60
   atgtccaaaa cattcatcat tggggagctc catccagatg acagaccaaa gttaaacaag 120
   cctccggaaa ctcttatcac tactattgat tctagttcca gttggtggac caactgggtg 180
   atccctgcca tctctgcagt ggccgtcgcc ttgatgtatc gcctatacat ggcagaggac 240
   tgaacacctc ctcagaagtc agcgcaggcc gagcctgctt tggacacggg agaaaagaag 300
   ccattgctaa ctacttcaac tgacagaaac cttcacttga aaacaatgat tttaatatat 360
   ctctttcttt ttcttccgac attagaaaca aaacaaaaag aactgtcctt tctgcgctca 420
   aatttttcga gtgtgccttt ttattcatct acttt 455
<210> 710
   <211> 501
   <212> DNA
   <213> Homo sapiens
   <400> 710
   gaacagaacc tgagtcgtcg gactttcaaa agcctcttca gagcaagcga tgagagtgtt 60
   ttatccatgc ataaagtctg tgaagcggga ggactttttg taaatagccc agaagagccc 120
   agcctcagca ggatggtcac tgaggaggaa atccagttct atgtgcagca gttcaagaag 180
   tctggtttca gaggtcctct aaactggtac cgaaacatgg aaaggaactg gaagtgggct 240
   tgcaaaagct tgggacggaa gatcctgatt ccggccctga tggtcacggc ggagaaggac 300
   ttcgtgctcg ttcctcagat gtcccagcac atggaggact ggattcccca cctgaaaagg 360
   ggacacattg aggactgtgg gcactggaca cagatggaca agccaaccga ggtgaatcag 420
   atcctcatta agtggctgga ttctgatgcc cggaacccac cggtggtctc aaagatgtag 480
   aacgcagcgt gtgcccacgc t 501
<210> 711
   <211> 379
   <212> DNA
   <213> Homo sapiens
   <400> 711
   gttttcactg cttgtatgat gtttcccatt catacaccta taaatctcta acaagaggcc 60
   ctttgaactg ccttgtgttc tgtgagaaac aaatatttac ttagagtgga aggactgatt 120
   gagaatgttc caatccaaat gaatgcatca caacttacaa tgctgctcat tgttgtgagt 180
   actatgagat tcaaattttt ctaacatatg gaaagccttt tgtcctccaa agatgagtac 240
   tagggatcat gtgtttaaaa aaagaaaggc tacgatgact gggcaagaag aaagatggga 300
   aactgaataa agcagttgat cagcatcatt ggaacatggg gacgagtgac ggcaggagga 360
   ccacgaggaa ataccctca 379
<210> 712
   <211> 256
   <212> DNA
   <213> Homo sapiens
   <400> 712
   aatcctgtac caaatctgac atattatgcc tgaatgactc cactgttttt ctctaatgct 60
   tgatttaggt agccttgtgt tctgagtaga gcttgtaata aatactgcag cttgagaaaa 120
   agtggaagct tctaaatggt gctgcagatt tgatatttgc attgaggaaa tattaatttt 180
   ccaatgcaca gttgccacat ttagtcctgt actgtatgga aacactgatt ttgtaaagtt 240
   gcctttattt gctgtt 256
<210> 713
   <211> 423
   <212> DNA
   <213> Homo sapiens
<220>
   <221> misc_feature
   <222> (369)..(370)
   <223> n is a, c, g, or t
   <400> 713
   atagtaccag taggggctta taataaagga ctgtaatctt atttaggaag ttgacttata 60
   gtacatgata aatgatagac aattgaggta agttttttga aattatgtga cattttacat 120
   taaatttttt ttacattttt tgggcagcaa tttaaatgtt atgactatgt aaactacttc 180
   tcttgttagg taattttttt cacctagatt tttttcccaa ttgagaaaaa tatatactaa 240
   acaaaatagc aataaaacat aatcactcta tttgaagaaa atatcttgtt ttctgccaat 300
   agatttttta aaatgtagtc agcaaaatgg gggtggggaa gcagagcatg tcctagttca 360
   atgttgacnn tttttttttt tttaaagaaa agcattaaga cataaaattc tttcactttg 420
   gca 423
<210> 714
   <211> 398
   <212> DNA
   <213> Homo sapiens
<220>
   <221> misc_feature
   <222> (42)..(42)
   <223> n is a, c, g, or t
   <220>
   <221> misc_feature
   <222> (103)..(103)
   <223> n is a, c, g, or t
   <220>
   <221> misc_feature
   <222> (164)..(164)
   <223> n is a, c, g, or t
   <220>
   <221> misc_feature
   <222> (225)..(225)
   <223> n is a, c, g, or t
   <220>
   <221> misc_feature
   <222> (286)..(286)
   <223> n is a, c, g, or t
   <220>
   <221> misc_feature
   <222> (347)..(347)
   <223> n is a, c, g, or t
   <400> 714
   tacatcttgc cagaaggttt ccctcgccaa caaacagttg anaatttaag ggaagaagca 60
   aaagctaaac tgtctttgac cctaagatag atagaaagct atnttatttg tcttcagtgt 120
   tcaaggcatg actagtattt ctaattagcc taataaattc ccancacttt ctgaagtgaa 180
   cactaatggt attgtcctac taaaactgtc attgtttctt ttttntttaa ctggtcagtc 240
   attcacaata agctatgagg gtaaataaat atgtgttata acaagntaaa ccgtagttgc 300
   aagaatatac catgaagatt aaagtaggct gggtttcatt tccatcnttc ccacacatct 360
   cattgaattt gatggttgac ttaattggca ccataact 398
<210> 715
   <211> 480
   <212> DNA
   <213> Homo sapiens
<220>
   <221> misc_feature
   <222> (207)..(208)
   <223> n is a, c, g, or t
   <400> 715
   tacttaggtc aaatttctgt tctctcttcc ccaaataata ttaaagtatt atttgaactt 60
   tttaagatga ggcagttccc ctgaaaaagt taatgcagct ctccatcaga atccactctt 120
   ctagggatat gaaaatctct taacacccac cctacataca cagacacaca cacacacaca 180
   cacacacaca cacacacaca cacacanntt caccctaagg atccaatgga atactgaaaa 240
   gaaatcactt ccttgaaaat tttattaaaa aacaaacaaa caaacaaaaa gcctgtccac 300
   ccttgagaat ccttcctctc cttggaacgt caatgtttgt gtagatgaaa ccatctcatg 360
   ctctgtggct ccagggtttc tgttactatt ttatgcactt gggagaaggc ttagaataaa 420
   agatgtagca cattttgctt tcccatttat tgtttggcca gctatgccaa tgtggtgcta 480
<210> 716
   <211> 559
   <212> DNA
   <213> Homo sapiens
   <400> 716
   taccctcgca gcagtgtctc tgaggactag caaagtctgg aggcagatga atggtttctg 60
   accctcacca gggctgtgga agggtggggg tgggtcatta tagtattcag gatttacagt 120
   gcagtattca cgtgtaactt ttaagttttc agtacagtgc ttttatacct ttaatgcaat 180
   gttgtattca tttgggtact attgtgtagt atttaggatg tatgcatgtt tgtttatatg 240
   taagcttggt tggtgctttc gcttttgtgc tacctttctt ggatttttgt accagagatg 300
   tgctaaactg atgaaataca ttgagaaagt ttccatctta ttcttttata tgggactgat 360
   gatgtgtgtt ggggtagact gctcctgcag agtttggaag aagtcaccag caaagccggc 420
   ctaaccaaga aaagtcaagg cccttcatga ccttgctggg cacagaaaac accctcgtgg 480
   agtacactaa tttgaactgg actggtctca gtgtgagcac ttggcacact ttactaaaca 540
   catatacaac cccaccgtg 559
<210> 717
   <211> 382
   <212> DNA
   <213> Homo sapiens
   <400> 717
   tccagccctc cggagagtgg gcttggccct aggccctcca gctcagccag aaaaagccca 60
   gaaacccagg tgctggacca gggccctcag ggaggggacc ctgcggctag agtgggctag 120
   gccctggctt tgcccgtcag atttgaacga atgtgtgtcc cttgagccca aggagagcgg 180
   caggaggggt gggaccaggc tgggaggaca gagccagcag ctgccatgcc ctcctgctcc 240
   ccccacccca gccctagccc tttagccttt caccctgtgc tctggaaagg ctaccaaata 300
   ctggccaagg tcaggaggag caaaaatgag ccagcaccag cgccttggct ttgtgttagc 360
   atttcctcct gaagtgttct gt 382
<210> 718
   <211> 486
   <212> DNA
   <213> Homo sapiens
<220>
   <221> misc_feature
   <222> (77)..(77)
   <223> n is a, c, g, or t
   <220>
   <221> misc_feature
   <222> (351)..(351)
   <223> n is a, c, g, or t
   <220>
   <221> misc_feature
   <222> (457)..(457)
   <223> n is a, c, g, or t
   <400> 718
   ggtgatcgaa aactgtggcc atgtggaacc cggtcttgtg ggggactgtt tctccatctt 60
   gactcagaca gttcctngga aacaccgggg ctctgttttt attttctttg atgtttttct 120
   tctttagtag cttgggctgc agcctccact ctctagtcac tggggaggag tattttttgt 180
   tatgtttggt ttcatttgct ggcagagctg gggctttttg tgtgatccct cttggtgtga 240
   gttttctgac ccaaccagcc tctggttagc atcatttgta catttaaacc tgtaaatagt 300
   tgttacaaag caaagagatt atttatttcc atccaaagct cttttgaaca nccccccccc 360
   tttaatccct cgttcaggac gatgagcttg ctttccttca acctgtttgt tttcttattt 420
   aagactattt attaatggtt ggaccaatgt actcacngct gttgcgtcga gcagtcctta 480
   gtgaaa 486
<210> 719
   <211> 181
   <212> DNA
   <213> Homo sapiens
   <400> 719
   tgagggtttc agagagcctt tttctaggcc tacatgcttt gtgaacaagt ccctgtaatt 60
   gttgtttgta tgtataattc aaagcaccaa aataagaaaa gatgtagatt tatttcatca 120
   tattatacag accgaactgt tgtataaatt tatttactgc tagtcttaag aactgctttc 180
   t 181
<210> 720
   <211> 464
   <212> DNA
   <213> Homo sapiens
   <400> 720
   tccctgtaat tgttgtttgt atgtataatt caaagcacca aaataagaaa agatgtagat 60
   ttatttcatc atattataca gaccgaactg ttgtataaat ttatttactg ctagtcttaa 120
   gaactgcttt ctttcgtttg tttgtttcaa tattttcctt ctctctcaat ttttggttga 180
   ataaactaga ttacattcag ttggcctaag gtggttgtgc tcggagggtt tcttgtttct 240
   tttccatttt gtttttggat gatatttatt aaatagcttc taagagtccg gcggcatctg 300
   tcttgtccct attcctgcag cctgtgctga gggtagcagt gtatgagcta ccagcgtgca 360
   tgtcagcgac cctggcccga caggccacgt cctgcaatcg gcccggctgc ctcttcgccc 420
   tgtcgtgttc tgtgttagtg atcactgcct ttaatacagt ctgt 464
<210> 721
   <211> 426
   <212> DNA
   <213> Homo sapiens
   <400> 721
   ttcgacttgc atttttgcag gagcagtatc atgaagccta aacgcgatgg atatatgttt 60
   ttgaaggcag aaagcaaaat tatgtttgcc actttgcaaa ggagctcact gtggtgtctg 120
   tgttccaacc actgaatctg gaccccatct gtgaataagc cattctgact catatcccct 180
   atttaacagg gtctctagtg ctgtgaaaaa aaaaaaatgc tgaacattgc atataactta 240
   tattgtaaga aatactgtac aatgacttta ttgcatctgg gtagctgtaa ggcatgaagg 300
   atgccaagaa gtttaaggaa tatgggagaa atagtgtgga aattaagaag aaactaggtc 360
   tgatattcaa atggacaaac tgccagtttt gtttcctttc actggccaca gttgtttgat 420
   gcatta 426
<210> 722
   <211> 445
   <212> DNA
   <213> Homo sapiens
   <400> 722
   agccggagcc ggatgcagta ggactggact cgggccatat ccgtggtgcc gtcaacatgc 60
   ctttcatgga cttcctgact gaggatggct tcgagaaggg cccagaagag ctccgtgctc 120
   tgttccagac caagaaggtg gatctctcgc agcctctcat tgccacgtgc cgcaagggag 180
   tcaccgcctg ccacgtggcc ttggctgcct acctctgcgg caagcctgat gtggccgtgt 240
   acgatggctc ctggtccgag tggtttcgcc gggccccccc agagagccgt gtgtcccagg 300
   gaaagtctga gaaggcctga gccgtgacct cttctgctta ctgtaactgc ggccggttta 360
   gtgaccccat gacttacagc cggttcttac ctcttaggtg aaggagatga catgtttttt 420
   agaattgctg tgcaaggctc accct 445
<210> 723
   <211> 501
   <212> DNA
   <213> Homo sapiens
   <400> 723
   gcagggctag ttattccgat ttcttgcaca attatttagc tttttgtaag ttcaacatgt 60
   aaattttaaa gacataaata tagagagact tatgtgtttg aatataaatg atatatatgg 120
   attagcatgt acctgtatat tattaaacat gcaatgaact gactggtaag tgacgtctaa 180
   ttgtatggct agcaatgtaa tttattcaga ctgtattttt gtacagagca gtgcactcta 240
   acctatgcct ctgtgtcctc tttaatgcct aaagctgtgc ctagaaattt catctgtctt 300
   aaaagtaaaa tatacttcat gctgtttatg ctattagttt ctgtactgct attctatatt 360
   tattattttt aaatatatga catgtttact acttaaacat gaattcatgg tatcctggtt 420
   atttttttta agtcatctgg gggaaaacct gtttatcact ccagtgattt tgagtttgca 480
   gtttcacaat cagttcttca t 501
<210> 724
   <211> 477
   <212> DNA
   <213> Homo sapiens
   <400> 724
   aaggagctta ttcctggctc catcgctaac acgttgactg cttattatgg gaaagttttc 60
   tctgaagcca gggagaagca ttgattgatg tgggcaaatc caagctccag ccaggtcgca 120
   gtcccaaatg ccgacatcac tgactccagg gaccagggac atggagaaag ctgtttatga 180
   tatctttaac caggccctct tactagagct ggtgtttgtg actggccaac aagatgtggc 240
   tatgccaggg gacatctgag tatgtgccca gtcatctttt ttcacaggtt gaagggagag 300
   aaaagatttt gagttaaggt cattggctgc tctactctgt cccctacctg gtcacctagt 360
   gatagcccca gtggagatac tgtccataca aggtcttccc agaggctgga taccacagta 420
   aaaggccagg ccaggagggg taggagacta tggagatctt acctcctgat aaatgtg 477
<210> 725
   <211> 444
   <212> DNA
   <213> Homo sapiens
   <400> 725
   atctattcca tgtgtgattt gcttgtagaa acaattttga aagccccttg aggaaaataa 60
   aaatcaagaa gaacactttt ctcccttttc catacaaatt aaaacttaac agcatcaaat 120
   tattgggacc agaaaccaag taatgtataa tgtggctttt gttgagttaa ataagatgct 180
   atataatgga gaagaatttg aaaatgcaca aaaaaatcaa tctacattat cagaacctgc 240
   agtgaaatta aacttatgtt aaataaaacc agtttgcagg tgcacaaact atgagggtct 300
   tgtatccacg taacacaggt agttacaaaa acatgttatt gtactgtgta aagatgcata 360
   gtcatctcat ttggttggct ttgtaccttg tacctttttt agccttggct tttgttgaac 420
   tagaaccctc agcacatact gtgt 444
<210> 726
   <211> 475
   <212> DNA
   <213> Homo sapiens
   <400> 726
   gagagctcgc tttgagtgac tgggttttgt gattgcctct gaagcctatg tatgccatgg 60
   aggcactaac aaactctgag gtttccgaaa tcagaagcga aaaaatcagt gaataaacca 120
   tcatcttgcc actaccccct cctgaagcca cagcagggtt tcaggttcca atcagaactg 180
   ttggcaaggt gacatttcca tgcataaatg cgatccacag aaggtcctgg tggtatttgt 240
   aactttttgc aaggcatttt tttatatata tttttgtgca catttttttt tacgtttctt 300
   tagaaaacaa atgtatttca aaatatattt atagtcgaac aattcatata tttgaagtgg 360
   agccatatga atgtcagtag tttatacttc tctattatct caaactactg gcaatttgta 420
   aagaaatata tatgatatat aaatgtgatt gcagcttttc aatgttagcc acagt 475
<210> 727
   <211> 317
   <212> DNA
   <213> Homo sapiens
   <400> 727
   gattttctag tgctggtatt tgttgactac catgcagaag ggctatcttt ctattcacgt 60
   caaacttttg gttgtgtggg gttttttgtt gttttttggg ttttgttttt taatacttta 120
   gggtcctgat ttgtgggaac agaccttctt gtaaataacc actatttgag ttgtggcagg 180
   aggatgataa agcacgcggc ccctcccaaa ggagcccttg agctagggag gtggtgcagt 240
   cagcctcgct ctcaacgtga cccggggaat gaccacccag agggatgagc tagcctgtag 300
   aggggaactg gggtcca 317
<210> 728
   <211> 496
   <212> DNA
   <213> Homo sapiens
   <400> 728
   tctggttgcc tatagtgctc tgggatccca tcgagaagaa ccatgggtgg acccgaactc 60
   cccggtgctc ttggaggacc cagtcctttg tgccttggca aaaaagcaca agcgaacccc 120
   agccctgatt gccctgcgct accagctgca gcgtggggtt gtggtcctgg ccaagagcta 180
   caatgagcag cgcatcagac agaacgtgca ggtgtttgaa ttccagttga cttcagagga 240
   gatgaaagcc atagatggcc taaacagaaa tgtgcgatat ttgacccttg atatttttgc 300
   tggcccccct aattatccat tttctgatga atattaacat ggagggcatt gcatgaggtc 360
   tgccagaagg ccctgcgtgt ggatggtgac acagaggatg gctctatgct ggtgactgga 420
   cacatcgcct ctggttaaat ctctcctgct tggcgacttc agtaagctac agctaagccc 480
   atcggccgga aaagaa 496

<210> 729
   <211> 425
   <212> DNA
   <213> Homo sapiens
   <400> 729
   gaagcacggt atgatgacca aacataaaaa gtgttttata attgttggtg ttttaataac 60
   aactaatatt attactctga tagttaaact aactcgagat tctcagagtt tatgccccta 120
   tgattggatt ggtttccaaa acaaatgcta ttatttctct aaagaagaag gagattggaa 180
   ttcaagtaaa tacaactgtt ccactcaaca tgccgaccta actataattg acaacataga 240
   agaaacgaat tttcttaggc ggtataaatg cagttctgat cactggattg gactgaagat 300
   ggcaaaaaat cgaacaggac aatgggtaga tggagctaca tttaccaaat cgtttggcat 360
   gagagggagt gaaggatgtg cctacctcag cgatgatggt gcagcaacag ctagatgtta 420
   caccg 425
<210> 730
   <211> 400
   <212> DNA
   <213> Homo sapiens
   <400> 730
   gaacacgcag agagtttccc tagatatact cctgcctcca ggtgctggga cacacctttg 60
   caaaatgctg tgggaagcag gagctgggga gctgtgttaa gtcaaagtag aaaccctcca 120
   gtgtttggtg ttgtgtagag aataggacat agggtaaaga ggccaagctg cctgtagtta 180
   gtagagaaga atggatgtgg ttcttcttgt gtatttattt gtatcataaa cacttggaac 240
   aacaaagacc ataagcatca tttagcagtt gtagccattt tctagttaac tcatgtaaac 300
   aagtaagagt aacataacag tattaccctt tcactgttct cacaggacat gtacctaatt 360
   atggtactta tttatgtagt cactgtattt ctggattttt 400
<210> 731
   <211> 459
   <212> DNA
   <213> Homo sapiens
<220>
   <221> misc_feature
   <222> (32)..(32)
   <223> n is a, c, g, or t
   <220>
   <221> misc_feature
   <222> (78)..(78)
   <223> n is a, c, g, or t
   <220>
   <221> misc_feature
   <222> (82)..(82)
   <223> n is a, c, g, or t
   <220>
   <221> misc_feature
   <222> (242)..(242)
   <223> n is a, c, g, or t
   <400> 731
   tcacaaactt ttatactctt tctgtatata cntttttttt ctttaaaaaa caactatgga 60
   tcagaatagc cacatttnga anactttttg ttatcagtca atatttttag atagttagaa 120
   cctggtccta agcctaaaag tgggcttgat tctgcagtaa atcttttaca actgcctcga 180
   cacacataaa cctttttaaa aatagacact ccccgaagtc ttttgttcgc atggtcacac 240
   anctgatgct tagatgttcc agtaatctaa tatggccaca gtagtcttga tgaccaaagt 300
   cctttttttc catctttaga aaactacatg ggaacaaaca gatcgaacag ttttgaagct 360
   actgtgtgtg tgaatgaaca ctcttgcttt attccagaat gctgtacatc tattttggat 420
   tgtatattgt gtttgtgtat ttacgctttg attcatagt 459
<210> 732
   <211> 528
   <212> DNA
   <213> Homo sapiens
   <400> 732
   aacactaggg ccttggaaat tcctgtactg tgtctcatgg atttggcact agccaaagcg 60
   aggcaccctt actggcttac ctcctcatgg cagcctactc tccttgagga tgagtagcca 120
   gggtaagggg taaaggatag taagcataga aaccattaga aagtgggctt aatggagttc 180
   ttgtggcctc agctcaatgc agttagctga agaattgaaa gtttttgttt ggagacgttt 240
   ataacagaaa tggaagcaga gttttcatta atccttttac cttttttttt ttcttggtaa 300
   tcccctaaaa taacagtatg tgggatattg aatgttaaag ggatattttt tctattattt 360
   ttataattgt acaaaattaa gcaaatgtta aaagttttat atgctttatt aatgttttca 420
   aaaggtatta tacatgtgat acatttttta agcttcagtt gcttgtcttc tggtactttc 480
   tgttatgggc ttttggggag ccagaagcca atctacaatc tctttttg 528
<210> 733
   <211> 570
   <212> DNA
   <213> Homo sapiens
<220>
   <221> misc_feature
   <222> (233)..(233)
   <223> n is a, c, g, or t
   <220>
   <221> misc_feature
   <222> (252)..(252)
   <223> n is a, c, g, or t
   <220>
   <221> misc_feature
   <222> (259)..(259)
   <223> n is a, c, g, or t
   <220>
   <221> misc_feature
   <222> (347)..(347)
   <223> n is a, c, g, or t
   <400> 733
   ggatttttag gtcagcccag gggagaaaga taactgctaa aattcccctg taccccatcc 60
   tttcttgtcc tttccccttc agatggagac ttcattatgt taatgaacaa gatatgaaga 120
   aaatggcact cattgtggcc ttgttgaatt atgttgtgta tgttttaaca tctctgatgc 180
   tgtgttacta aaattacaag gacctgcttt ttaaaaggcc agaacaattg tcntgaaatt 240
   agtaacaatg cntgcatcnt agattggagt gctgcacaaa caaacataag agcaaagcaa 300
   aactgtatca catagggttt ttggtcactc acaacctgaa ttcaccnaca gctggaatag 360
   ctgtggaaaa caaaataaaa caacaaaatt aataatgaaa tggaggggaa ttctagaatt 420
   atatgctaaa tgcatatttt atgatttgct gtattaactg atgataaaac taatggcaga 480
   aaaagaagtt gagcaatttc tatgtaatgt acagatacta gcattgcaca tatagtctgc 540
   tttctgttcc tccagaattt gagtcctgtt 570
<210> 734
   <211> 246
   <212> DNA
   <213> Homo sapiens
   <400> 734
   agttcaagta cagtgactat ttcaagccat tttccacagg aaaacgagtg tgtgctggag 60
   aaggcctggc tcgcatggag ttgtttcttt tgttgtgtgc cattttgcag cattttaatt 120
   tgaagcctct cgttgaccca aaggatatcg acctcagccc tatacatatt gggtttggct 180
   gtatcccacc acgttacaaa ctctgtgtca ttccccgctc atgagtgtgt ggaggacacc 240
   ctgaac 246
<210> 735
   <211> 358
   <212> DNA
   <213> Homo sapiens
   <400> 735
   ccgggggcct atggcagtga tgctgtgttg gtttcctagg gatgctctaa cgaattacca 60
   caaacctggt ggattgaaac agcagaactt gattccctta cagttctgga ggctggaaat 120
   ctgggatgga ggtgttggca gggctgtggt ccctttgaag gctctgggga agaatccttc 180
   cttggctctt tttagcttgt ggcggcagtg ggcagtccgt ggcattcccc agcttattgc 240
   tgcatcactc cagtctctgt ctcttctgtt ctctcctctt ttaacaacag tcattggatt 300
   tagggcccac cctaatcctg tgtgatctta tcttgatcct tattaattaa acctgcaa 358
<210> 736
   <211> 454
   <212> DNA
   <213> Homo sapiens
   <400> 736
   gtagctctga tgagaatggg gtcccagatg gctcaggctg tgacctccct gggcaccacc 60
   ctccccaggc tgggtgtgga ggagttgggg ccccctgcct tcaggaggct tgtagtttag 120
   aagggaagta ggcattacca tagacgactc ctagaggaca gtgctatgta aaaatgtgtg 180
   tctataaatg tttatcatgc atgtattcta gagctcattc atttattcaa caaacatttg 240
   gtgagcacct atttcggttc gagaaacttc atttatctcc tataattggc aaacttaaaa 300
   atgcagcaga aacttacatt ccaaccttag agactcatag tgagcacaag gaaagttttg 360
   ccctgagatt catggttatg gctgggtacc accaaataga agaatggctt aggggagtgc 420
   cccttcactg agatgtgttt ctttgttgaa cttt 454
<210> 737
   <211> 226
   <212> DNA
   <213> Homo sapiens
   <400> 737
   aacgaactga actaggcctg gtggaaggag gcgcactttc ctcctggcag aatgctagct 60
   ctgagccagt tcagtacctg gaggaggagc aggggcgtgg agggcgtgga gggcgtggga 120
   gcgtgggagg cgggagtgga gtggaagaag agggagagat ggagcaaagt gagggccgag 180
   tgagagcgtg ctccagcctg gctcccacag gcagctttaa ccatta 226
<210> 738
   <211> 560
   <212> DNA
   <213> Homo sapiens
   <400> 738
   tctactgcgt gacttgccat gagaccaagt ttgccaagca ttgcgtgaag tgcaacaagg 60
   ccatcacatc tggaggaatc acttaccagg atcagccctg gcatgccgat tgctttgtgt 120
   gtgttacctg ctctaagaag ctggctgggc agcgtttcac cgctgtggag gaccagtatt 180
   actgcgtgga ttgctacaag aactttgtgg ccaagaagtg tgctggatgc aagaacccca 240
   tcactgggaa aaggactgtg tcaagagtga gccgcccagt ctctaaagct aggaagcccc 300
   cagtgtgcca cgggaaacgc ttgcctctca ccctgtttcc cagcgccaac ctccggggca 360
   ggcatccggg tggagagagg acttgtccct cgtgggtggt ggttctttat agaaaaaatc 420
   gaagcttagc agctcctcgt ggcccgggtt tggtaaaggc tccagtgtgg tggcctatga 480
   aggacaatcc tggcacgact actgcttcca ctgcaaaaaa tgctccgtga atctggccaa 540
   caagcgcttt gttttccacc 560
<210> 739
   <211> 440
   <212> DNA
   <213> Homo sapiens
   <400> 739
   cccattcggc gtagtaccca gagagctcaa gatgtgtggc agttttcgga tggaagctcg 60
   agagccctta agttctgaga aaatttgaag cccccagggg tggggtggac gcgtgccgcc 120
   cagtcgacgt cagcgtggtc tgtcatcctg ctagtttgtg atgttttctg acagtagcct 180
   ccaagaagcc gttgtgcgaa gacagagtcc tgcagagtcc ttccagccta ggcctgcagc 240
   gccattttat ttatattttt taataaaaag taaaaacaaa aaaacagacc cacattggaa 300
   cagtgaatca gtcccataga gagggcccgt ggaccatcgc tgtcatgagt gatgccctgg 360
   cccttctgaa accagccaac ctaattacct gtattgtgga aatgcgcatg agtccccaac 420
   cccttgtttc tatacattct 440
<210> 740
   <211> 473
   <212> DNA
   <213> Homo sapiens
   <400> 740
   tggaggcgca ggcacaaggt ttgttggaga ctgaaccgtt gcaaggaaca gacgaagatg 60
   cagtagccag tgctgacttc tctagcatgc tctctgagga ggaaaaggaa gagttaaaag 120
   cagagttagt tcagctagaa gacgaaatta caacactacg acaagttttg tcagcgaaag 180
   aaaggcatct agttgagata aaacaaaaac tcggcatgaa cctgatgaat gaattaaaac 240
   agaacttcag caaaagctgg catgacatgc agactaccac tgcctacaag aaaacacatg 300
   aaaccctgag tcacgcaggg caaaaggcaa ctgcagcttt cagcaacgtt ggaacggcca 360
   tcagcaagaa gttcggagac atgagacgaa agtaggcggt acgaacccta atggaggcag 420
   ttttgaggag gtcctcagct ccacggccca tgccagtgcc cagagcttgg cag 473
<210> 741
   <211> 255
   <212> DNA
   <213> Homo sapiens
   <400> 741
   gttcctgaaa tcctgagtgt tgcctgccag tcgccatgag aacttcctac cttctgctgt 60
   ttactctctg cttacttttg tctgagatgg cctcaggtgg taactttctc acaggccttg 120
   gccacagatc tgatcattac aattgcgtca gcagtggagg gcaatgtctc tattctgcct 180
   gcccgatctt taccaaaatt caaggcacct gttacagagg gaaggccaag tgctgcaagt 240
   gagctgggag tgacc 255
<210> 742
   <211> 566
   <212> DNA
   <213> Homo sapiens
   <400> 742
<210> 743
   <211> 555
   <212> DNA
   <213> Homo sapiens
   <400> 743
   gcattccacc ggcggctacg gtggtggcaa ttccggcggc ggcggcggcg gcctacgggg 60
   gcggcactcc ggcggcggca gcagctccgg cggcggatac ggcggcggca gctccagcgg 120
   aggccacaag tcctcctctt ccgggtccgt gggcgagtct tcatctaagg gaccaagata 180
   ctaacaaaac cagagtaatc aagacaatta ttgaagaggt ggcgcccgac ggtagagttc 240
   tttcatctat ggttgaatca gaaaccaaga aacactacta ttaaactgca tcaagaggag 300
   agagtctccc ttcacacaga ccattaattt acagatgcat ggaaaacaaa gtctccaaga 360
   aaacacttct gtcttgatgg tctatggaaa tagaccttga aaataaggtg tctacaaggt 420
   gttttgtggt ttctgtattt cttcttttca ctttaccaga aagtgttctt taatggaaag 480
   aaaaacaact ttctgttctc atttactaat gaatttcaat aaactttctt actgatgcaa 540
   acgtctgaga ttact 555
<210> 744
   <211> 436
   <212> DNA
   <213> Homo sapiens
   <400> 744
   ttcgtgatgg tgttgatcct cttcctggga gcctccatgg tctacctgat ccgggtggca 60
   cggaggaacc aggagcgtgc cctgcgcacc gtctggagct ccggacatga caaggagcag 120
   ctggtgaaga acacatatgt cctgtgaccg ccctgtcgcc aagaggactg gggaagggag 180
   gggagactat gtgtgagctt tttttaaata gcgggattga ctcggatttg agtgatcatt 240
   agggctgagg tgtgtttctc tgggaggtag gacggctgct tcctggtctg gcagggatgg 300
   gtttgctttg gaaatcctct aggaggctcc tcctcgcatg gcctgcagtc tggcagcagc 360
   cccgagttgt ttcctcgctg atcgatttct ttcctccagg tagagttttc tttgcttatg 420
   ttgaattcca ttgcct 436
<210> 745
   <211> 505
   <212> DNA
   <213> Homo sapiens
   <400> 745
   ggctccatga aggtcctttg gcacagctct gctcctcccc tgcctgccaa agcccccctt 60
   taggccttgg gtggctggaa ggctttgtta agggactagg agaaatgggg gtatctttcc 120
   cctttcctgc cctttctgct catctcaacc tctcacagag gtgtcttctc cccctaacct 180
   acagcttttt gtacaagcca ttttgtgtaa attatttata tttaatatta ttccctgctt 240
   tgtcaggagc aggtactagg ctctggggca gtgaggaact agatccttct ctcctcagcc 300
   tagggtggag gtcactgcac taccacccac ctctggaaga ctggctgtga aaagtcaggt 360
   ggcagaaacc tggggccaca tagagcctct ctcttttcct gtttcttggc tctagaagat 420
   cagcactgca ctgttagctg agagtgcggg caagacataa actgtccaga gtttgaaggt 480
   tctcggaaag accggagggc ttctc 505
<210> 746
   <211> 471
   <212> DNA
   <213> Homo sapiens
   <400> 746
   gagggccgaa cccacatgac aaagagtgac tccctgccct ccttccgggt ctccaccctg 60
   cctctggagt cacaccaccc cgacccaaac accatgggcg gggccagcca ccgggacagg 120
   gctctctcgg tgactgccac cgtaggggaa accaaaggga aggaccctgc cccagcccag 180
   cctcccccag ctaggaaaca gaacgtgggc agagacgtga ccaagccatc cccagcccca 240
   aacactgacc gccccatctc tctttctaat gagaaggact ttgtggtacg gcggaggcgg 300
   gggaaagaga gtttgcgtag cagccctcac aaaaaggcct tgtaacgggg agggcccagg 360
   ggcaggactg tggagacccg tcctgaacgg gcgactgtgt cttgactacc tttcaaaacc 420
   agcactgtgt gggaatgtcc gccaggcaga gctcggagcc tcattgagac a 471
<210> 747
   <211> 256
   <212> DNA
   <213> Homo sapiens
   <400> 747
   cgctaggtgc ctgctaggtg catggccaca gagcatgggc tgggcctggg cacaggagga 60
   gcagctgctt tggtcggggt ggagactcgc agcagctgct acccacagcc tattccactc 120
   ctccccatct ccaggcgctg ggaggggggc cctcaccccg tcacgcctcg ctccctcctg 180
   gccctctggt ccagcccctc acgcctcctc tcagtctact caattgtgac tgtccctcct 240
   gatgtatttt ttttct 256
<210> 748
   <211> 528
   <212> DNA
   <213> Homo sapiens
   <400> 748
   agccctgcgt tgtgtgtttt cagatgagtt actgttaaca ggtaggttcg tgtaggcctt 60
   gctgggcact ctgtacaatt agttgcttat tacgtatgat tactcgcagc gatctattgt 120
   tccatataac caaaaagcat ggtttattca ttgaaacacg gttgacctga actcgtgcct 180
   taggaattaa tgccccctta tggaacctgc ctgaattgca cctgcgggtg gaggctccgg 240
   ctgtgaagtc actgaacaga acgtcgctga tggagaaagg gctcccgcag aaggaacggc 300
   ctgtaccgtg cgctccggca caatcgcgtc tcttgtgtct cactcacgga aagaaacaac 360
   ctgaaggcca tcccgtcggt ctgcacgtaa ccgtgaagac gtgtggccgc gtcccacctg 420
   cggctgggta ccctgcaccc ggcactgtag gagtcacgtg cagcctttct caggggactg 480
   tcattgaaaa ggaaacgttt gatgtctgtg tcagctgtct ttgtagtt 528
<210> 749
   <211> 518
   <212> DNA
   <213> Homo sapiens
   <400> 749
   agatgtgcgc aggagtacct gtcccgggtg aagaaggagg agcagaggta ccaggccctg 60
   aaggtgcacg cggaggagaa actggacagg gccaatgctg agattgctca ggttcgaggc 120
   aaggcccagc aggagcaagc cgcccaccag gccagcctgc ggaaggagca gctgcgagtg 180
   gacgccctgg aaaggacgct ggagcagaag aataaagaaa tagaagaact caccaagatt 240
   tgtgacgaac tgattgccaa aatggggaaa agctaactct gaaccgaatg ttttggactt 300
   aactgttgcg tgcaatatga ccgtcggcac actgctgttc ctccagttcc atggacaggt 360
   tctgttttca ctttttcgta tgcactactg tatttccttt ctaaataaaa ttgatttgat 420
   tgtatgcagt actaaggaga ctatcagaat ttcttgctat tggtttgcat tttcctagta 480
   taattcatag caagttgacc tcagagttcc tgtatcag 518
<210> 750
   <211> 545
   <212> DNA
   <213> Homo sapiens
   <400> 750
   aaatagcatt aaactggaat tgacagagtg agttgagcat ctctgtctaa cctgctcttt 60
   ctctctggtg ctcctcatct cacccctacc ttggaattta ataagcttca ggcatttcca 120
   attgcagact aaaaccactt ctaccatctc ctctagtatt ttccatgtat caggacagag 180
   atgtcttatg tagggaaggg gcaggtatga agtgaggtag attatctata cctctcactc 240
   attcaggatt ctcgctccca tgctgctgtc ccttcattct cacactcaca ggaatgctat 300
   gtgatggcca gctgcttccc ttcttggtta tccactgcag ctgctagtta gaaaggtttg 360
   cagggatgac ttttagtaaa tcatggggat tttattgatt tattatcact tataggattt 420
   tgtggggtgg gagtggggag caggaattgc actcagacat gacatttcaa ttcatctctg 480
   caaatgaaaa gggttcttcc tcttggggga aatctgtgtg tcagttctgt cagctgcaag 540
   ttctt 545
<210> 751
   <211> 421
   <212> DNA
   <213> Homo sapiens
   <400> 751
   gagtattaca ttggccttgg gggacagaaa ggaggaagtt ctgacttttc agggctacct 60
   tatttctact aaggacccag agcaggcctg tccatgccat tccttcgcac agatgaaact 120
   gagctgggac tggaaaggac agcccttgac ctgggttctg ggtataattt gcacttttga 180
   gactggtagc taaccatctt atgagtgcca atgtgtcatt tagtaaaact taaatagaaa 240
   caaggtcctt caaatgttcc tttggccaaa agctgaaggg agttactgag aaaatagtta 300
   acaattactg tcaggtgtca tcactgttca aaaggtaagc acatttagaa ttttgttctt 360
   gacagttaac tgactaatct tacttccaca aaatatgtga atttgctgct tctgagaggc 420
   a 421
<210> 752
   <211> 375
   <212> DNA
   <213> Homo sapiens
   <400> 752
   aagctatgtg tatcttctgt gtaaagcagt ggcttcactg gaaaaatggt gtggctagca 60
   tttccctttg agtcatgatg acagatggtg tgaaaaccat ctaagtttgc ttttgaccat 120
   cacctcccag tagcaatttg ctttcataat ccatttagca atccaggcct ctgttgaaaa 180
   gataatatga gggagaaggg aacacatttc cttctgaact tacttcccta agtcactttc 240
   cttatgtatc atctaataca atgatggttg agtgaaaata cagaaggggt gtttgagtat 300
   tcagatttca taaaacactt ccttggaata tagctgcatt aacttggaaa gaagcctgtt 360
   gggccagaag acaga 375
<210> 753
   <211> 532
   <212> DNA
   <213> Homo sapiens
   <400> 753
   caggattggc caagtccatc ggggtgtcca acttcaacca caggctgctg gagatgatcc 60
   tcaacaagcc agggctcaag tacaagcctg tctgcaacca ggtggaatgt catccttact 120
   tcaaccagag aaaactgctg gatttctgca agtcaaaaga cattgttctg gttgcctata 180
   gtgctctggg atcccatcga gaagaaccat gggtggaccc gaactccccg gtgctcttgg 240
   aggacccagt cctttgtgcc ttggcaaaaa agcacaagcg aaccccagcc ctgattgccc 300
   tgcgctacca gctgcagcgt ggggttgtgg tcctggccaa gagctacaat gagcagcgca 360
   tcagacagaa cgtgcaggtg tttgaattcc agttgacttc agaggagatg aaagccatag 420
   atggcctaaa cagaaatgtg cgatatttga cccttgatat ttttgctggc ccccctaatt 480
   atccattttc tgatgaatat taacatagag ggtgttgcac gacatctagc ag 532
<210> 754
   <211> 159
   <212> DNA
   <213> Homo sapiens
   <400> 754
   tcactgagca ccacattctc tagcttcttg ttgaggctgg aactgtttct ttaaaatccc 60
   ttaattttcc catctcaaaa ttatatctgt acctgggtca tccagctcct tcttgggtgt 120
   ggggaaatga gttttctttg atagtttctg cctcactca 159
<210> 755
   <211> 378
   <212> DNA
   <213> Homo sapiens
   <400> 755
   acatctccat tacaaatgcc acagttgaag acagtggaac ctactactgt acgggcaaag 60
   tgtggcagct ggactatgag tctgagcccc tcaacattac tgtaataaaa gctccgcgtg 120
   agaagtactg gctacaattt tttatcccat tgttggtggt gattctgttt gctgtggaca 180
   caggattatt tatctcaact cagcagcagg tcacatttct cttgaagatt aagagaacca 240
   ggaaaggctt cagacttctg aacccacatc ctaagccaaa ccccaaaaac aactgatata 300
   attactcaag aaatatttgc aacattagtt tttttccagc atcagcaatt gctactcaat 360
   tgtcaaacac agcttgca 378
<210> 756
   <211> 436
   <212> DNA
   <213> Homo sapiens
   <400> 756
   agtgagaaga tctgcaccgt ccagttggtg ggtaacagct ggacccctgg ctaccccgag 60
   acccaggagg cgctctgccc gcaggtgaca tggtcctggg accagttgcc cagcagagct 120
   cttggccccg ctgctgcgcc cacactctcg ccagagtccc cagccggctc gccagccatg 180
   atgctgcagc cgggcccgca gctctacgac gtgatggacg cggtcccagc gcggcgctgg 240
   aaggagttcg tgcgcacgct ggggctgcgc gaggcagaga tcgaagccgt ggaggtggag 300
   atcggccgct tccgagacca gcagtacgag atgctcaagc gctggcgcca gcagcagccc 360
   gcgggcctcg gagccgttta cgcggccctg gagcgcatgg ggctggacgg ctgcgtggaa 420
   gacttgcgca gccgcc 436
<210> 757
   <211> 441
   <212> DNA
   <213> Homo sapiens
   <400> 757
   gagagctcct gtttactaag caagcttttg tgtttattat cctcattttt actgaacatt 60
   gttagttttg gggtaatgga aacccacttt ttcattgtaa tgactttggg ggcttttgtt 120
   agtaagggtg ggtggggtga tgggttgcag acggaggtca ggtcttcctc tttcctgaga 180
   ctggatctgt tcaaacagca aacgcccaca gatggcccag aggtggtggt agtcagggtg 240
   tgtgggtgtt tttagggttc tttagtgttg tttctttcac ccaggggtgg tggtcccagc 300
   cagtttggtg ctgacggtga gaggaaatta gaatctgttt gcaaattgtc caacccaccc 360
   cctcaacatg aggggcttcc attttctgtg ttttgtaagg gaactgtttc cttcatgccg 420
   ccatgttcct gatattagtt c 441
<210> 758
   <211> 437
   <212> DNA
   <213> Homo sapiens
   <400> 758
   ttctacctga acacttgtac tcttgaagtc acaacaaaat aatgatgagc ttttcacatc 60
   acctttatgg tttcaatccc tagctcaaag cttcctggaa tcttttattt tttgtaaact 120
   tttttttctt ttgttaaaat aaataaaaca ttcaatgttt ttctcctttt ctctcttatt 180
   acttctttcc tttggcattt tcaatttgaa atgctttcct ttggttgttg gttttattct 240
   ccccctaccc ctcccctttt cttattattc agaatataaa cctgcaaagc tctgctctgt 300
   tttggttttg aaagtttaag cttttctgct tctgtgagag cacaggcttc tgtccctttt 360
   gattccaact gaacttttgt gttctctaat gatactaaca cggtgtaggt tttacagtct 420
   cctaatttgt actggta 437
<210> 759
   <211> 402
   <212> DNA
   <213> Homo sapiens
   <400> 759
   cttaactctt ttgacatctg ctattgtgac acatcccatt gctggcaatg tggtgcacac 60
   tccgaaactt ttaactactg ttttgtaagc ctccaagggt ggcattgcag ggtccttagg 120
   caatgttttg tttgccttta tgcagagagg tgctccaagt gctgtgattg agcaccgtgc 180
   tagaggaact gtaatgcttc agaagttgta gcttatacaa aggaaacagg tcctgctggc 240
   ttaatttaaa cagttattgc atgaagtagc gtggaggccc tggactgctg ctcgttcttt 300
   aggatggact gttctggtat ctggtattgg tttagagact gttaataagg gacatcacaa 360
   ggtgatggga ttcatttgaa gcactctatt tctgttttaa tg 402
<210> 760
   <211> 501
   <212> DNA
   <213> Homo sapiens
   <400> 760
   cagaaaaggc ataccacgag cagctgtcgg tggcagagat caccaatgcc tgctttgagc 60
   ctgccaacca gatggtaaag tgtgatcccc ggcacggcaa gtacatggcc tgctgcctgc 120
   tgtaccgtgg agatgtggtg cccaaggatg tcaacgctgc cattgccgcc atcaagacca 180
   agcgcagcat tcagtttgtg gactggtgcc ccacaggctt caaggttggt atcaactacc 240
   agcctcccac tgtggtgcct gggggtgacc tggccaaggt gcagcgtgcc gtgtgcatgc 300
   tgagcaacac gaccgccatc gccgaggcct gggcccgcct ggaccacaag ttcgacctga 360
   tgtatgccaa gagggcgttt gtgcactggt atgtgggtga gggcatggag gagggtgagt 420
   tctccgaggc ccgtgaggat atggctgccc tggagaagga ttatgaggag gtgggcatcg 480
   actcctatga ggacgaggat g 501
<210> 761
   <211> 441
   <212> DNA
   <213> Homo sapiens
<220>
   <221> misc_feature
   <222> (35)..(35)
   <223> n is a, c, g, or t
   <400> 761
   tgttacatgg cagcttaggc agactagatc ttgtnttttc caatgcagca taatgagtat 60
   gatctatttc ttttcaaata atctttgaga tcccaggaaa aaaaaaatgc tctgctccat 120
   tgagctataa tgtaaatgtg tttgtttaaa aaacaggtga ggcaagtgag tgatttattg 180
   ttcctgagga agtatatctg attttttttc tcatactcca aaagctagtc cctactcttt 240
   aataaaaata atgggtaact ttttgttttt cactagcgaa cttccatgac atttcctttc 300
   tatgtagtgt gattaatgca atacatatta tagttatcta tacacagtgt aagatttaac 360
   aaactgaaat gatccacctc atatgtgagt ccgtccaaaa gatgttactg ctctgggtgg 420
   gccagtgttc tatatcggtt a 441

<210> 762
   <211> 521
   <212> DNA
   <213> Homo sapiens
<220>
   <221> misc_feature
   <222> (82)..(82)
   <223> n is a, c, g, or t
   <220>
   <221> misc_feature
   <222> (89)..(89)
   <223> n is a, c, g, or t
   <400> 762
   ctgtgcgacg agtttcagct ggccaagaaa ggagtcaagt tattaaaaag catcacaatg 60
   tagatctcca ggctggtttt tngtttttng ttgttaagac tggggaaagg gggactattt 120
   attctgcctt aaatcaatgg caaataagtc aagatgacat tttgtgaatg tagactatgg 180
   atacactcct aatagattga tgtagtcata aaagggggtc aagtagatgt ttttctgtta 240
   tgtaagcaat aatttttccg tgtcttattg agtatggcta gcgattattt attacatgct 300
   agatgggttc tttgcatgtg ggttccatat aggtgcagaa atttcctcag ccactggagg 360
   gatttcgacc atatttgtca tttggatgag ctgttattag attgaaatct acacatcatt 420
   tcattaaaaa ttgtgcctta gaaaacgcaa agctgttgca catggcgata aattatggat 480
   gcagtacatt gaagagagat gaagtcactt ccaagtttcc a 521
<210> 763
   <211> 462
   <212> DNA
   <213> Homo sapiens
<220>
   <221> misc_feature
   <222> (64)..(65)
   <223> n is a, c, g, or t
   <220>
   <221> misc_feature
   <222> (115)..(115)
   <223> n is a, c, g, or t
   <220>
   <221> misc_feature
   <222> (121)..(121)
   <223> n is a, c, g, or t
   <220>
   <221> misc_feature
   <222> (422)..(422)
   <223> n is a, c, g, or t
<220>
   <221> misc_feature
   <222> (432)..(432)
   <223> n is a, c, g, or t
   <400> 763
   gggggctcag tgagcactac tcacagatcc acacctgacc ctgttgggtc gagtcaggct 60
   gggnnttggt ctgcactgta gcacctgtgt tctttgagtt cacatcatga atgtnggtga 120
   nttcccagat accatctcag gcttaaccta gcacatccta tttcttttct tctatgatat 180
   ccaaattgga ctgacctcac ttcaaagttg ctgtcccatt ttgtcaccct atcttatctc 240
   ggggaaattg cagactgatg gccagaccaa ctctgttgaa attcttgcat agagcaaacc 300
   tgtgctcatt tttaagtggc atgggagagg ccccaagcct agtaaagcct agtctgtgtc 360
   ttcacagtgc tggtagaatg tgtttgtgtg tataaatata tgatatagat ttatatatgt 420
   tnctaacgcc anatattgaa ggccaacata actggtggac ag 462
<210> 764
   <211> 495
   <212> DNA
   <213> Homo sapiens
   <400> 764
   gtgaaccagg agatttagtg cttttatatt catttccttg catttaagaa aatatgaaag 60
   cttaaggaat tatgtgagct taaaactagt caagcagttt agaaccaaag gcctatatta 120
   ataaccgcaa ctatgctgaa aagtacaaag tagtacagta tattgttatg tacatatcat 180
   tgttaataca gtcctggcat tctgtacata tatgtattac atttctacat ttttaatact 240
   cacatgggct tatgcattaa gtttaattgt gataaatttg tgctgttcca gtatatgcaa 300
   tacactttaa tgttttattc ttgtacataa aaatgtgcaa tatggagatg tatacagtct 360
   ttactatatt aggtttataa acagttttaa gaatttcatc cttttgccaa aatggtggag 420
   tatgtaattg gtaaatcata aatcctgtgg tgaatggtgg tgtactttaa agctgtcacc 480
   atgttatatt ttctt 495
<210> 765
   <211> 458
   <212> DNA
   <213> Homo sapiens
<220>
   <221> misc_feature
   <222> (82)..(82)
   <223> n is a, c, g, or t
   <400> 765
   gcaatcttgg aatcctcaac tgcagtaagc atttcaaaat gcaaacaaac tgcttaacaa 60
   ctgacaagac accagcccat angctgctct tccaacagtg ggttctagct ttgaacaaaa 120
   gtgctaaaca tttccttgaa tatattcttc ctctttttgt cctcatcact caatactggt 180
   gctcttgtca caggtagaac agcttgtttc ttttccatct attcaagtgt gtttctaatt 240
   ctaaaatgct gatcttctct ggagtctatg gtaggcaatt atggtcactg gaatagtttg 300
   tcttgtttta aaatattatt ggtgcatgta caacagcatc caacatatct gtcttgttcc 360
   tagatatata gctctgattt taggcctttt gtgcatacca ttacaatatg gtggggtaag 420
   acattctaca gtagcctgtg ctgaactgat ctcttaaa 458
<210> 766
   <211> 414
   <212> DNA
   <213> Homo sapiens
   <400> 766
   aattctcact gttcactttt aactgacaaa gaaaaacaag tggaaactac agaaactgtg 60
   gtagaacttt tacttgctgg tctggtcttg gttgtaccca tctttggcca gtcacataac 120
   tactcaagaa accttcccaa tagagtacaa caggatgaga ctctgaaatc actttcagta 180
   ttccctgcta gatattgatt gttatttcaa gtattaagtg taagctttta atggataatt 240
   agtataactg tggatggcat ctgattttgt ttttaattct gtggattgtg tttaagcaat 300
   tcaatagtat gttcctgatt ttgagatgct aagtggtatt gcacagttgt cactttatca 360
   agtgtgtaca acagtcccat gaagtttata gagcataccc ttgtatagct tcag 414
<210> 767
   <211> 441
   <212> DNA
   <213> Homo sapiens
   <400> 767
   tttcgagggg gcaaggaggg acagaaaagt aacctcttct taagtggaat attctaataa 60
   gctacctttt gtaagtgcca tgtttattat ctaatcattc caagttttgc attgatgtct 120
   gactgccact cctttctttc aaggacagtg ttttttgtag taaaatcact ggtttataca 180
   aagctttatt tagggggtaa agttaagctg ctaaaacccc atgttggctg ctgctgttga 240
   gatactgtgc tttgggagta aaaaaagaaa gttatttctt tgtcttaaag aatttttaaa 300
   aaattagtca tgagacttat tcatctttcc agggaacata ctgattggtc ttaaaagact 360
   agacagttaa gtaaaaggtg gctggaacat ctatttttct acaaaactgg aaaaatgaac 420
   ctggttctag aagaatgtac a 441
<210> 768
   <211> 529
   <212> DNA
   <213> Homo sapiens
   <400> 768
   gcagccaagg tctgtgttca gcacttggtc tctgttgtta cgtaaaataa taagcattta 60
   aaatagttta cagatatttt tgaccagttc cttttagaga ttctttcaga gaagaaacca 120
   gatctgacct gtttattgtt ggcgcttgtt gaaaacgagc tttctttccc atgatagtgc 180
   ttcgtttttg aagtgttgaa gctgtgctcc ccttaaatcg tggcaggaga gattaaggta 240
   attacaacac tcagttctat gtcttacaag cactttgtct tgtctctgca agaaaattcg 300
   attccagtca tttcccataa aatacagaca ttttaccaac ataatatgct ttgattgatg 360
   cagcattatg ctttgggcag tattacaaaa tagctggcga gtgctttctg tatttaaata 420
   ttgtaaaaag aaaataagtt ataactgtta taaagcagaa cttttgttgc attttttaaa 480
   ctgttgaagt cactgtgtat gtttgtttgg tcaatgtttc cgcagtatt 529
<210> 769
   <211> 474
   <212> DNA
   <213> Homo sapiens
   <400> 769
   gaactcatgt gatttaccct tttcaacttt ttggaaaacg atttaattta ttctaattag 60
   attaacccta ttaatctatg gattgggtat caaaatgaat gccagtccag atgtgcctag 120
   acacgaaatt ggagctgagg actctcacga tatgcaagtt catccaacgt gaagatacca 180
   taagcttttt ctctgaacca gagaaatgaa agtcagttta agaggctgat agatcttggc 240
   cctgttaagg catccacttc acagttctga aggctgagtc agccccactc cacagttagg 300
   ccaagaatta gattttaaaa cttcatctgt ctgtcccagt taactgttaa ataaggcctc 360
   atcctccact gaagagtatg gattgaagga ttgtgaacta tgtttagtgt gattgtgaac 420
   ttggtgccta atgttccatg tctgaagttt gccccagtgc tacacgttgg agta 474
<210> 770
   <211> 536
   <212> DNA
   <213> Homo sapiens
   <400> 770
   ccctcaagcc tgggctcatg gagcccctgc ccagggccct caggtgggcg gaaagtccat 60
   cccctccgcc cttcaggaag gatgctcccg tgtgcagggg tctcctgcct gtgccatcca 120
   ctggggctcg agacaatttc ccactcacct gtgaggccgg tgtggctgct tcccttgtaa 180
   atagttgttc tctggtaaga agccaaatat ttaagctcac ttcttcccag agagaggaag 240
   ctctgctcag gcctccagcg ttggctggcc atggccacag ccagatggag gagcccatcc 300
   ccaggagact caggcagtgg cctggagagg ctttgttctg taacggtgcc ttttcttagg 360
   gtccaggcag gaatgaagcc aataatttat tgctttccat tctgtggtat gatgtgcgtg 420
   tgcgtgagtg tgtggcccct gtttattccc ctcctgtcaa gaatgaagtg gattcagttc 480
   aggtactttt gagggttgtt gtgctgaccc tgtggttgtc gctgatgtac acacat 536
<210> 771
   <211> 549
   <212> DNA
   <213> Homo sapiens
<220>
   <221> misc_feature
   <222> (225)..(225)
   <223> n is a, c, g, or t
   <400> 771
   ggatgggctg gaccaggtgg gacagattag ctgatgccct tgtcacctgc cctctgtgca 60
   ccctgagagc tcacagtaac actgtgtgtg tcaccatata actgcacctc acccccgcac 120
   gtgtgcatga ctcgcagaga atattccagc aattgtgtac ccctgggcca gtctctttga 180
   accctgaggg tggccaggat ctggagctgc atctctaagg ggccnaggct ttggggacca 240
   ttgccaaagg tggactcagg aggaaagaca cttaaagaca cttttacatg tctagtaatt 300
   cttgatgttc atcttcagca ccagtggaaa cacatgaact tcgatgcagg tccagagacc 360
   atggacactc ccacgaggct cagctctcag gcacccccta cacttcagtt gagggaaaag 420
   ctcaagtgcc ttaggcccgt ggaccacagt cttggctgag atcaaaggga tgagcaacag 480
   ggacttctgc cacagtgaca atggaattgt gttgtgcctt acttcagagg tggtctcttc 540
   tttcttgta 549
<210> 772
   <211> 443
   <212> DNA
   <213> Homo sapiens
   <400> 772
   ttcctgagtt gaaacttctc ctgtggttac tggtattgag aaatcagcta ccaaagtgaa 60
   aaaggacaag atcaattctt ttctagtcag ttctaagact gctagagaga gataccaggc 120
   ccttagcctt gctctcagta gcgtcagccc cagttctgag cctccccaca ttacacttaa 180
   caagcagtaa aggagtgagc actttgggtc cttagactca tgtctgggga ggaagagcaa 240
   gtagaaaagt ggcattttct tgattggaaa gggggaagga tcttattgca cttgggctgt 300
   tcagaatgta gaaaggacat atttgaggaa gtatctattt gagcactgat ttactctgta 360
   aaaagcaaaa tctctctgtc ctaaactaat ggaagcgatt ctcccatgct catgtgtaat 420
   ggttttaacg ttactcactg gag 443
<210> 773
   <211> 475
   <212> DNA
   <213> Homo sapiens
<220>
   <221> misc_feature
   <222> (192)..(192)
   <223> n is a, c, g, or t
   <220>
   <221> misc_feature
   <222> (195)..(195)
   <223> n is a, c, g, or t
   <220>
   <221> misc_feature
   <222> (222)..(222)
   <223> n is a, c, g, or t
   <220>
   <221> misc_feature
   <222> (351)..(351)
   <223> n is a, c, g, or t
   <400> 773
   taatctcacg gctcttgatc tggaaacttc agagtacaaa ttggtggatg gtggaaggca 60
   ggacacgtat ctctgtctga cggaaaacag acctcggggc tggcgtaaac cctgctgcca 120
   ggccctctcc ccactgcccc aaaccggcct agacacgaag accaaagcag cctgcacagg 180
   gcaaggcccc cngcngaatc ctgcagagca aactcaggtt ancttgggtc catgaccgtt 240
   tgcattcgaa acacaataca ctgcctcgtt ctctcagtta gcagctgggc agcagcgcac 300
   cattcatcat ttaggcttgt ggtttgttgt ttactctacc aatgttatgt ngaaactgca 360
   ttgtaaaaag agaagaaaat ggcaggtttt ccaggtccac ggaaaggttt ggcctgacgc 420
   tggagtgcgg tgatgaactt acgtgacaat gattgtattc ctcagtagca cttta 475
<210> 774
   <211> 504
   <212> DNA
   <213> Homo sapiens
   <400> 774
   gaattcacac ggtactcaga ggcactgctg gggaagtttg ttggtcttta ttagataaat 60
   ttccagagac ctgtccataa tacccaacag aacatgactg tttctttgag gaaagggtta 120
   taatgtctgt ggtgtacaag tcgtttttgg tataacttct ttcctgctgc tgctgcttcc 180
   cggcaaacat agttttccta tttcaggcag agtgcggtat attccaggaa acactgtttc 240
   ctactcactt agcttacttc tttgttgaat gcctcactaa tggcaagttt caagatgttt 300
   tgggtgacaa tgcacacatg ctgggcaaaa gggtgatggc cagtggctgg cagctgggcc 360
   agcagaagct aggacatctg tgagttgtca ttctcatcta tccatgtcca ctggcctgcc 420
   agcatccgcc agtgccttgc cagtgtgcac ggtcccacac tgtggcccct gagtccccta 480
   atgtacacgc tgcagccaga atgc 504
<210> 775
   <211> 417
   <212> DNA
   <213> Homo sapiens
   <400> 775
   gacgagtagt cagttattgc ttgctagcta cacaccaggg ttgatccatt ttaaaacttt 60
   tggcattttg tcctcatggg ccataaatac agaaccttgt attttaatta aattttttta 120
   caaaaggagg cacatgcaca atctccatgt aacaaacctt tagcagtagg atgtattata 180
   cgacagttac ttaatttcta gagttcaggc ctctgggatc aaccccagac tgggccagaa 240
   tgttagtgaa ggttttattg tgcccggttg gaggataacg ttctttgggt actttttgtg 300
   ggttgcaaat gaactcaatt gccacaagtt ttaaactggt gtaaatcaag cttgacttaa 360
   tgtgattgtt actgttatat ccagcctata ctgctagcag ctgctcatac tgcagtc 417
<210> 776
   <211> 304
   <212> DNA
   <213> Homo sapiens
<220>
   <221> misc_feature
   <222> (238)..(238)
   <223> n is a, c, g, or t
   <400> 776
   aaaagcgctt cagtgccact agcttaccgg tacactagac taagcccttg atgacttatt 60
   gcatgataca gtaccaggaa caacaggtgg cctaaataca tgaaaagcag tgtaagctag 120
   tgacactaaa gccagtcttg tattactgta tttttgacag aatggttttg aaaactgtgc 180
   tacagggact gatgtggcaa atatatctct ttatgcagaa ggaagtcttt ttttttcntt 240
   tttttttttt taagaagtat ggctttttat gcatccttca tcgagggcat tgaagttgca 300
   tgga 304
<210> 777
   <211> 554
   <212> DNA
   <213> Homo sapiens
<220>
   <221> misc_feature
   <222> (163)..(163)
   <223> n is a, c, g, or t
   <400> 777
   gccattcccc aggctatgaa gtgacatagg cctcccccac ggtgcctgtg tacggagcct 60
   gatgacttca ctgggagcct tctggaatcc tgcagagggt caaggagcag ggatgttgga 120
   tgcccacctg tcaagagttc agatcaaagt tgcgctgaga gcntcacaat tttggttcag 180
   ccttgacgcg tttgtccaac agctcattgg ctccctttgt atgatatcgt ggtcttctca 240
   catggtgccc agtcaccaat atttataatg aggtctaact acagcagtag tttttcatat 300
   atatctctaa aacattttgt tatattgaaa aaagtaatag aaatcaagat gtgttgatga 360
   aataaaatgt gtatctgagt gagaaaacaa gtatggtgag gtcactttaa tgtttcacag 420
   cgatctcaga tctaggcctc aggtagaatg gaagctgttc tgcattcact gattaacgtt 480
   gctaaactct tggtgaggca cgagctacca gccaattgct cttcatcaca gctatctgtc 540
   ttttagtgcc acaa 554
<210> 778
   <211> 147
   <212> DNA
   <213> Homo sapiens
   <400> 778
   gacaggaggg tgtccacata tgttaacatc agttggatct cctatagaag tttctgctgc 60
   tctctttcct tctccctgag ctggtaactg caatgccaac ttcctgggcc tttctgacta 120
   gtatcacact tctaataaaa tccacaa 147
<210> 779
   <211> 560
   <212> DNA
   <213> Homo sapiens
<220>
   <221> misc_feature
   <222> (175)..(177)
   <223> n is a, c, g, or t
   <220>
   <221> misc_feature
   <222> (179)..(181)
   <223> n is a, c, g, or t
   <220>
   <221> misc_feature
   <222> (190)..(190)
   <223> n is a, c, g, or t
   <220>
   <221> misc_feature
   <222> (422)..(422)
   <223> n is a, c, g, or t
   <220>
   <221> misc_feature
   <222> (426)..(426)
   <223> n is a, c, g, or t
   <220>
   <221> misc_feature
   <222> (429)..(430)
   <223> n is a, c, g, or t
   <400> 779
   gctccacatg agccatgcat gcttagcaat ccaagtgcag agctctttgc tccaggagtg 60
   aggagactgg gaggtgaaat ggggaaatgg aagggtttgg aggcagagct gaaaacaggg 120
   ttggaaggat ttcctgaatt agaagacaaa cgttagcata cccagtaagg aaaannngnn 180
   naggggccan ggggaacccg tgaggatcac tctcaaatga gattaaaaac aaggaagcag 240
   agaatggtca gagaatggga ttcagattgg gaacttgtgg ggatgagagt gaccaggttg 300
   aactgggaag tggaaaaagg agtttgagtc actggcacct agaagcctgc ccacgattcc 360
   taggaaggct ggcagacacc ctggaaccct ggggagctac tggcaaactc tcctggattg 420
   gncctnatnn ttttggtggg aaaggctgcc ctggggatca actttccttc tgtgtgtggc 480
   tcaggagttc ttctgcagag atggcgctat ctttcctcct cctgtgatgt cctgctccca 540
   accatttgta ctcttcatta 560
<210> 780
   <211> 559
   <212> DNA
   <213> Homo sapiens
<220>
   <221> misc_feature
   <222> (36)..(36)
   <223> n is a, c, g, or t
   <220>
   <221> misc_feature
   <222> (51)..(51)
   <223> n is a, c, g, or t
   <220>
   <221> misc_feature
   <222> (56)..(56)
   <223> n is a, c, g, or t
   <400> 780
   acttctcagc aaataaatct cccttaagta ggaaanctag atttcatatt ngcttncttt 60
   gaattaacag caactttcca caggtaaatc tgttcttgca aagatgtgag cagaatagtt 120
   aaaaataata tttttatgtt tcatggttct aaatggaagc cataaatgca gtaaatacta 180
   tctgttgttt aactacttta atcgtcattt tttacatttt caagtttatt aggttaagaa 240
   aaacagggca gccttggaag gcagctacta cagaaaactg cagttttgcg ttaaagataa 300
   agtagtattt tcagctccct gaaaaaccat tcctgctgaa actgctgtag aaattgtgaa 360
   gctgcatgag tggagagtat tgaatctgtg gttatagtag ttttctcagg tttgtttatc 420
   ttgatgtttg atgcactgtg ttttatagtt attaaaattg agtaatatta tttctatgca 480
   gtgttatgtg tcattggcct tttgtgaatg tgcatgtttt aaactgcaaa ttttaaacat 540
   tttgtcctct aattgttat 559
<210> 781
   <211> 507
   <212> DNA
   <213> Homo sapiens
   <400> 781
   atattcctac atcaagttac tactgagagt aaatttattt tgagttttat cccgtaagtt 60
   ctgttttgat tttttttaaa aaacaaaccc ttttagtcac tttaatcaga attttaaatg 120
   ttcatgttac ataccaaatt ataatatcta atggagcaat ttgtcttttg ctatattctc 180
   caagattatc tcttaagacc atatgccccc tgttttaatg tttcttacat cttgttttta 240
   ctcatttctg actggacaaa gttcttccaa acaattctga gaaacaaaaa cacacacgca 300
   gaattaacaa ttcttttccc tgtgcttctt atgtaagaat cctcctgtgg cctctgcttg 360
   tacagaactg ggaaacaaca cttggttagt ctcttttaag ttacaaaaag ccaattgatg 420
   tttcttattc tttttaaatt ttaaatattt tgttataaat actcacagga taccttattt 480
   ccctagctat catctcctga cttaatg 507
<210> 782
   <211> 480
   <212> DNA
   <213> Homo sapiens
   <400> 782
   aaaatccaag acactatgcc aatgcaaccg tgactacttt gggagattgg tagtctcttt 60
   tgatggtgat agtgatgggg tgcactatca taatcacatc aggtctgctt tttgctttta 120
   atgttaacta atgaagttcc agagatgggc cttagaaatg tgttttaaga attaacaagg 180
   agtctcaaaa agaaatgaga gggatgcttc ctttcccttg catctacaaa acaagagaga 240
   gactgttctg ttgtaaaact ctttcaaaaa ttctgatatg gtaaggtact tgagaccctt 300
   caccagaatg tcaatctttt tttctgtgta acatggaaac ttgtgtgacc attagcattg 360
   ttatcagctt gtactggtct cataactctg gttttggaag aataatttgg aaattgttgc 420
   tgtgttctgt gaaaataacc tccccaaaat aattagtaac tggttgttct acttggtaat 480
<210> 783
   <211> 341
   <212> DNA
   <213> Homo sapiens
   <400> 783
   gttcagtaca tcatgctctt gtgcctctgc ctgcttttcc tgcgttccca ccctgtattc 60
   cccccgcctt tcgggtttcc agggcttcga gcttgatctt ttgaaagttt tattctatta 120
   aatttttgct atatcttctg gttttctgaa aaagctttag aatggtttct ataccctttg 180
   tatcactgca tttttccata tcatctccgg ttcgatcgcg tccagatgga aaacggaagc 240
   agaggcttct aatcgtcgca tttactggct ccagtgcaac acatccatct gaaaacactc 300
   ggaagtctgg tgcttggaga gggtgccatt gtctcttgta c 341
<210> 784
   <211> 490
   <212> DNA
   <213> Homo sapiens
   <400> 784
   acatgcatac tttattgtgg gccatgaacc aaatggttct tacttttcct ggacttaaag 60
   aaaaaaagag gtttaagttt gttgtggcca atgtcgaaac ctacaagatt tccttaaaat 120
   ctctaataga ggcattactt gctttcaatt gacaaatgat gccctctgac tagtagattt 180
   ctatgatcct tttttgtcat tttatgaata tcattgattt tataattggt gctatttgaa 240
   gaaaaaaatg tacatttatt catagataga taagtatcag gtctgacccc agtggaaaac 300
   aaagccaaac aaaactgaac cacaaaaaaa aaggctggtg ttcaccaaaa ccaaacttgt 360
   tcatttagat aatttgaaaa agttccatag aaaaggcgtg cagtactaag ggaacaatcc 420
   atgtgattaa tgttttcatt atgttcatgt aagaagcccc ttatttttag ccataatttt 480
   gcatactgaa 490
<210> 785
   <211> 398
   <212> DNA
   <213> Homo sapiens
   <400> 785
   ccttactaaa agcccctcat atatcaatta ctttatttca ttatgactac ttaggttccg 60
   ggctggggac aagttcactt aaaaaggcaa tgttatttaa caggtcacca gttaagactt 120
   ctgctttgta gatacatgca gaagccatca aacaaggggg agcttttaac tgcaacaata 180
   agctaaagta tgtaaaatac tacattctat tcagtcttgg agtgttttgt agaaagttat 240
   cttcagccaa atctttgctg aagactggtt gtggagtgtt ggtaaatgct ttgtgttttt 300
   atgtaaaata ttttctaaac aaaaaatgtt aaaagtacat gtcctctgta gtaaactgat 360
   atctatatat atgaatcatt caagcctaaa gtctagta 398
<210> 786
   <211> 528
   <212> DNA
   <213> Homo sapiens
<220>
   <221> misc_feature
   <222> (106)..(106)
   <223> n is a, c, g, or t
   <220>
   <221> misc_feature
   <222> (185)..(185)
   <223> n is a, c, g, or t
   <220>
   <221> misc_feature
   <222> (189)..(190)
   <223> n is a, c, g, or t
   <220>
   <221> misc_feature
   <222> (196)..(196)
   <223> n is a, c, g, or t
   <220>
   <221> misc_feature
   <222> (245)..(245)
   <223> n is a, c, g, or t
   <220>
   <221> misc_feature
   <222> (284)..(284)
   <223> n is a, c, g, or t
   <400> 786
   ggaagaagac aagccccact agggccaagg gcagcagagc cctgccgagt gagaggctgt 60
   ggggcagcgg ctctgtcctg tgccttacca gccctgggga gggggncatt tggctggaag 120
   actggaattt aattgccatc gtctttgatt ttgtgacatt tctgcttgga agtgtgaact 180
   acccncccnn cccccngctt cctgctcctt agcatgcgtg cagctctctc ctgttttggg 240
   tgttnccctt ggacactcca gctcggggac tgctggcgtg tgantgtgca gattcccctg 300
   tgtggtcgaa cctaagaact gtggcttgga agtgatgctc catgtgacga cgactttgct 360
   ttctttcctc ttagtgagga ggtgattcgt agatcccaac tgcctatgta atgtaaataa 420
   tgtacattta atttattgct atggtagcac attgtatttg ttaatgtaca aaacaaattc 480
   taaaaggttg acaaatgtat attttgttgc ttaaatgtgt ctttgcag 528
<210> 787
   <211> 543
   <212> DNA
   <213> Homo sapiens
   <400> 787
   tatactcact caaggcagtg caagatcttg aagtactttt tagcagttaa gtaatattga 60
   attgtattga atagtttaca tagtttattc tagtctttga aaattactga acatggacaa 120
   tgtgcatgtc attgacatct gccttagaac ttctgggaca atcctgattc gagagattct 180
   atcccattat ttacatatac caaaaatact ttgttaattt aatgtgttgg cttcccaact 240
   cctgaacacg acacaatttt attattagat tttgtatggt gattttaggc tatgaaaaca 300
   tgatcattat atgtatatag atacattttt atttgttaca aatgtttgag cagctcacta 360
   gcccacccct cctctatttt gggtaagaga atttactacc ttttttaact atgtagttga 420
   gagcaacatg tattttgtta tttttagaat ggtcagtata ttgctataaa attttaaatg 480
   agactatgaa agttaaagta ttctgattct ggttaaatta acgaatatgg ttccaggccc 540
   tgt 543

<210> 788
   <211> 444
   <212> DNA
   <213> Homo sapiens
<220>
   <221> misc_feature
   <222> (33)..(34)
   <223> n is a, c, g, or t
   <220>
   <221> misc_feature
   <222> (36)..(47)
   <223> n is a, c, g, or t
   <220>
   <221> misc_feature
   <222> (49)..(49)
   <223> n is a, c, g, or t
   <220>
   <221> misc_feature
   <222> (51)..(53)
   <223> n is a, c, g, or t
   <220>
   <221> misc_feature
   <222> (55)..(56)
   <223> n is a, c, g, or t
   <220>
   <221> misc_feature
   <222> (58)..(58)
   <223> n is a, c, g, or t
   <220>
   <221> misc_feature
   <222> (60)..(61)
   <223> n is a, c, g, or t
   <220>
   <221> misc_feature
   <222> (63)..(63)
   <223> n is a, c, g, or t
   <220>
   <221> misc_feature
   <222> (66)..(74)
   <223> n is a, c, g, or t
   <220>
   <221> misc_feature
   <222> (76)..(80)
   <223> n is a, c, g, or t
<220>
   <221> misc_feature
   <222> (85)..(85)
   <223> n is a, c, g, or t
   <220>
   <221> misc_feature
   <222> (208)..(208)
   <223> n is a, c, g, or t
   <400> 788
   tccagcggag gccacaagtc ctcctcttcc ggnncnnnnn nnnnnnntnc nnntnngngn 60
   ncnagnnnnn nnnnannnnn ccaanatact aacaaaacca gagtaatcaa gacaattatt 120
   gaagaggtgg cgcccgacgg tagagttctt tcatctatgg ttgaatcaga aaccaagaaa 180
   cactactatt aaactgcatc aagagganag agtctccctt cacacagacc attatttaca 240
   gatgcatgga aaacaaagtc tccaagaaaa cacttctgtc ttgatggtct atggaaatag 300
   accttgaaaa taaggtgtct acaaggtgtt ttgtggtttc tgtatttctt cttttcactt 360
   taccagaaag tgttctttaa tggaaagaaa aacaactttc tgttctcatt tactaatgaa 420
   tttcaataaa ctttcttact gatg 444
<210> 789
   <211> 548
   <212> DNA
   <213> Homo sapiens
   <400> 789
   gtatcggaac agtacaacat ctaaagagta aatttggaaa aggctacttt ttggaaatta 60
   aattgaagga ctggatagaa aacctagaag tagaccgcct tcaaagagaa attcagtata 120
   ttttcccaaa tgcaagccgt caggaaagtt tttcttctat tttggcttat aaaattccta 180
   aggaagatgt tcagtccctt tcacaatctt tttttaagct ggaagaagct aaacatgctt 240
   ttgccattga agaatatagc ttttctcaag caacattgga acaggttttt gtagaactca 300
   ctaaagaaca agaggaggaa gataatagtt gtggaacttt aaacagcaca ctttggtggg 360
   aacgaacaca agaagataga gtagtatttt gaatttgtat tgttcggtct gcttactggg 420
   acttctttct ttttcactta attttaactt tggtttaaaa agttttttat tggaatggta 480
   actggagaac caagaacgca cttgaaattt ttctaagctc cttaattgaa atgctgtggt 540
   tgtgtgtt 548
<210> 790
   <211> 196
   <212> DNA
   <213> Homo sapiens
   <400> 790
   agaatacttg taaaagcata tcacatctta aaccagtggt gcacatgtgg atttacagct 60
   catggactct actgttcagc tttaatttat aaaacatatc acacatttaa tgttatacag 120
   tatttacata tagtggaaca tagggataac tcagttttat gtaaattttt gttaagtgtt 180
   gtagcctgcc cagagt 196
<210> 791
   <211> 542
   <212> DNA
   <213> Homo sapiens
<220>
   <221> misc_feature
   <222> (208)..(208)
   <223> n is a, c, g, or t
   <220>
   <221> misc_feature
   <222> (461)..(461)
   <223> n is a, c, g, or t
   <400> 791
   agctagaatt aattgcccac tctcccaccc taccagtgca gcccggcaag ggcaggaatt 60
   gggaggccta gggtgggcat gaaagcttgg gaagcactgt cgtctctcag acaggcgtcc 120
   taaagacctc taggctggaa gcttgggctt gcaagtggat ccgggaccga gggtggtctc 180
   ttggacaacc ccaggaactt ggaccaangc agagccaatc ttgcaaactg gccatggatg 240
   gggaagtgcc cggtagccag catgagccac actaggaaag aggaggaggg tgcagccaaa 300
   cttaaggcac cggcaagtgt tgtcagcact ggaggagacc ccgccagtgg ggtgaggcca 360
   gccaagtccc tgtgttacga atggtgggcc aaggggctgt ctgctcggtc ccagtaggac 420
   aggcagagct ccaggctggc accatggtag gcctccaggg naagagctgg gaggcaggaa 480
   tggcacactg ggcaggcttg cccattcctg gccctgagaa tggagctgta gcctcatgga 540
   ca 542
<210> 792
   <211> 522
   <212> DNA
   <213> Homo sapiens
   <400> 792
   tgctgtcaaa tccttaatag ctacaggagc tactgaggga aatcagtgtc attatttaaa 60
   gtcacgcctt gtgtttttac tactttattc agcaggatta aacctgaata acttttggct 120
   gttgtgctaa tagtgtaaat aaaataagcc tgccttcata aaacactaac ttttaaaagg 180
   aataaacgac ttctaaaatt atgcctatta acatgtgtaa ttagtcggca gctcaaatgt 240
   ttgggagtgc aagaaattag gcaccccagg atataggtca tacagggata tataaaagcc 300
   atgctcatta caaaatgagc agttgatgtt ttatgtggca ttaagacaat caagtcctca 360
   caactctgga atgtcttctt atactgatgc tgaatttatg aatccaaatt aatttccaac 420
   aggttggaat cagatttaat gtgagatcat gatagacaag accacagagg acgtatgctc 480
   tatttcttgt tggccaacag cttctttcta atgttctgtg aa 522
<210> 793
   <211> 450
   <212> DNA
   <213> Homo sapiens
<220>
   <221> misc_feature
   <222> (34)..(34)
   <223> n is a, c, g, or t
   <400> 793
   gctcgacgta tttcaaacat ttcaaaatgc tttnatctat gtttatcaca ttttaatacc 60
   acagcactta taatgatgtc actacatata gaagctcaaa gttaagggat ttgctgaaga 120
   ctgtaaagtt aatggaagaa ttgagacaaa aatccagtgt agctggccac ttatccaggg 180
   ctttttctac ttcatcacaa ggaatgtttt gaaagtgtct gcttttttta tccttaaaat 240
   tcacctgtca gggaggcatt aaaaatttgg aaatgtatgc cagcaaaatg tgagctctgt 300
   attttttggc attcttatgt ttgggtttaa taagattaag aaaatgatac tgggaatttt 360
   ctttttcctg aaactttgaa tcaccctagt aagtcaaagt actaaaaaat gtactagatc 420
   attaagactt atgtgctctt actgattgaa 450
<210> 794
   <211> 544
   <212> DNA
   <213> Homo sapiens
   <400> 794
   cacaggcagg tgactactcc atgcgcgtgg acctgcgggc tggggacgag gctgtgttcg 60
   cccagtacga ctccttccac gtagactcgg ctgcggagta ctaccgcctc cacttggagg 120
   gctaccacgg caccgcaggg gactccatga gctaccacag cggcagtgtc ttctctgccc 180
   gtgatcggga ccccaacagc ttgctcatct cctgcgctgt ctcctaccga ggggcctggt 240
   ggtacaggaa ctgccactac gccaacctca acgggctcta cgggagcaca gtggaccatc 300
   agggagtgag ctggtaccac tggaagggct tcgagttctc ggtgcccttc acggaaatga 360
   agctgagacc aagaaacttt cgctccccag cggggggagg ctgagctgct gcccacctct 420
   ctcgcacccc agtatgactg ccgagcactg aggggtcgcc ccgagagaag agccagggtc 480
   cttcaccacc cagccgctgg aggaagcctt ctctgccagc gatctcgcag cactgtgttt 540
   acag 544
<210> 795
   <211> 558
   <212> DNA
   <213> Homo sapiens
<220>
   <221> misc_feature
   <222> (158)..(158)
   <223> n is a, c, g, or t
   <400> 795
   gaatcttcac agtaacattt cagaaaggtg cttttttggt actcttcatg ggaacagttt 60
   agcagccatg agtgatcttc ctttgaaaga gaatgaaaga ccctgtgaca tttcacttca 120
   aaaataagcc ctgtagctct ttacggtcgc atagtatnaa attataccct gcatgctgac 180
   cctcgcttgg aatggaatgc cagaaatgca tggcagcagc taataagtaa agctgattaa 240
   ctatttattt gtcaatgtta ttatttaatg agctttcaca tgtgatttgt ttcaaaactt 300
   taatttttta atgttttgaa actttttcat ggacctaaat attttcctat atgatttgtg 360
   gttgattaga aatatgaaat acatgttgta gatatgtaaa atgaatattt tagtctcctt 420
   attacatata tgttcatggt gaactttatc aatagtatgg atctttttaa atcaataaga 480
   tgctttgtaa agttgaaata agtaatactt tcttgtttaa tctgtgcaat cagaaggtgt 540
   cttgaccttc aattcaat 558
<210> 796
   <211> 431
   <212> DNA
   <213> Homo sapiens
<220>
   <221> misc_feature
   <222> (178)..(178)
   <223> n is a, c, g, or t
   <400> 796
   gcacacagag atttgagaac cattgttctg aatgctgctt ccatttgaca aagtgccgtg 60
   ataatttttg aaaagagaag caaacaatgg tgtctctttt atgttcagct tataatgaaa 120
   tctgtttgtt gacttattag gactttgaat tatttcttta ttaaccctct gagttttngt 180
   atgtattatt attaaagaaa aatgcaatca ggattttaaa catgtaaata caaattttgt 240
   ataacttttg atgacttcag tgaaattttc aggtagtctg agtaatagat tgttttgcca 300
   cttagaatag catttgccac ttagtatttt aaaaaataat tgttggagta tttattgtca 360
   gttttgttca cttgttatct aatacaaaat tataaagcct tcagagggtt tggaccacat 420
   ctctttggaa a 431
<210> 797
   <211> 358
   <212> DNA
   <213> Homo sapiens
   <400> 797
   agagcgacgg ctgcaacagt gcctttttgt ctgttccctt gaccaatctt actgagaatg 60
   gcctgatgtg ccccgcctgc actgcgagct tcagggacaa atgcatgggg cccatgaccc 120
   actgtactgg aaaggaaaac cactgcgtct ccttatctgg acacgtgcag gctggtattt 180
   tcaaacccag atttgctatg cggggctgtg ctacagagag tatgtgcttt accaagcctg 240
   gtgctgaagt acccacaggc accaatgtcc tcttcctcca tcatatagag tgcactcact 300
   ccccctgaaa agctatctga acagaggaag ataatgtagt gtgaagtccc catttgtc 358
<210> 798
   <211> 475
   <212> DNA
   <213> Homo sapiens
<220>
   <221> misc_feature
   <222> (61)..(62)
   <223> n is a, c, g, or t
   <220>
   <221> misc_feature
   <222> (64)..(76)
   <223> n is a, c, g, or t
   <220>
   <221> misc_feature
   <222> (78)..(81)
   <223> n is a, c, g, or t
   <400> 798
   caatctatat tcacaggccc atacttcagt cagtccaatc atagtacagt gatcgaccaa 60
   nngnnnnnnn nnnnnncnnn nttgtaaaat acggatcatt tgtattttgg ggtgataaaa 120
   tagttcacca tgggtatgag atatttattc tttaaatcaa agtaaattag aatttttaaa 180
   aagcacaaaa ctgcaggaca gtttatgaaa taggtggcac tattagggaa tcttccttta 240
   aagcaagaaa tcatgttatt tagaaagaaa aactaatctt aaacatacta tttctaataa 300
   atatttatat ttttatgaaa taaagaggta tgtggaaatt aatatttggt gatgttggac 360
   agtggaaaag tatctagagt ttttacctgc cttatctgaa ttcttcttga aacttgagct 420
   taaactctaa tagctgtttc cctttctatt ctgaacaact gtctccattt ttcaa 475
<210> 799
   <211> 519
   <212> DNA
   <213> Homo sapiens
   <400> 799
   gaacagttct atgccaccag agaccactat tttaccaact ccctcctgtc attttttgag 60
   atgatcttgg atcttcgctg gacttatgtt cttttctaca gcccaaggga ggttaaagtg 120
   gtggccaaag gattttgtag tgccaatggg atcacagtct cagcagacca gaagtatgtc 180
   tatgtagctg atgtagcagc taagaacatt cacataatgg aaaaacatga taactgggat 240
   ttaactcaac tgaaggtgat acagttgggc accttagtgg ataacctgac tgtcgatcct 300
   gccacaggag acattttggc aggatgccat cctaatccta tgaagctact gaactataac 360
   cctgaggacc ctccaggatc agaagtactt cgcatccaga atgttttgtc tgagaagccc 420
   agggtgagca ccgtgtatgc caacaatggc tctgtgcttc agggcacctc tgtggcttct 480
   gtgtaccatg ggaaaattct cataggcacc gtatttcac 519
<210> 800
   <211> 466
   <212> DNA
   <213> Homo sapiens
<220>
   <221> misc_feature
   <222> (411)..(411)
   <223> n is a, c, g, or t
   <400> 800
   ctccagcgac ccaatggcgt gtaactcgcc gcagtctcca gcggtgtggg agccccaggg 60
   ctcgtcccgc tcgctcagcc accaccctca tgcccaccct ccgacctcca accagtcccc 120
   agcgtccagc tacctggaga actctgcatc ctggtacaca agtgcagcca gctcaatcaa 180
   ttcccacctg ccgccgccgg gctccttaca gcacccgctg gcgctggcct ccgggacact 240
   ctattagatg ggctgctctc tcttactctc ttttttggga ctactgtgtt ttgctgttct 300
   agaaaatcat aaagaaagga attcatatgg ggaagttcgg aaaactgaaa aagattcatg 360
   tgtaaagctt ttttttgcat gtaagttatt gcatttcaaa agaccccccc ntttttttac 420
   agaggacttt ttttgcgcaa ctgtggacac tttcaatggt gccttg 466
<210> 801
   <211> 549
   <212> DNA
   <213> Homo sapiens
<220>
   <221> misc_feature
   <222> (148)..(149)
   <223> n is a, c, g, or t
   <220>
   <221> misc_feature
   <222> (189)..(189)
   <223> n is a, c, g, or t
   <220>
   <221> misc_feature
   <222> (191)..(194)
   <223> n is a, c, g, or t
   <220>
   <221> misc_feature
   <222> (339)..(339)
   <223> n is a, c, g, or t
   <220>
   <221> misc_feature
   <222> (399)..(399)
   <223> n is a, c, g, or t
   <400> 801
   gaggcctcac tctaagttat taccgtcccc ttcattgttt tcaaagacat gtggtgatat 60
   agtttttaaa aataactatt ttgttataga tcataatatg cataaaactg tacagaaata 120
   ttttgtaatg tgttgatttt aaaaaaanna tctgtaaata aagttttaaa aaaagaattc 180
   aaatggcana nnnngaaata tgtagatatt ttgctattta tttaaaggag tattttaaga 240
   gatattgaac tatctgaaat tgaccagtaa tcaaagttcc aatcatctga atgcttttcc 300
   ttgaggtaga atgtgagtct cagaaatgac tgcattacnt gccctttttt gcaccttttc 360
   tgtcttttta ttttgcagaa caacaacaac aacaaaatng tgccttagct gtattttttt 420
   gtctagggga gtttgtttct gtctgacaaa gcaacatttt ttgcagaaaa cagtggatgt 480
   attaaatact gtatcatacc aaaaacactg caggtgtata tagatgcttt ctgtcatact 540
   gtgttttca 549
<210> 802
   <211> 515
   <212> DNA
   <213> Homo sapiens
<220>
   <221> misc_feature
   <222> (101)..(101)
   <223> n is a, c, g, or t
   <220>
   <221> misc_feature
   <222> (106)..(108)
   <223> n is a, c, g, or t
   <220>
   <221> misc_feature
   <222> (125)..(126)
   <223> n is a, c, g, or t
   <220>
   <221> misc_feature
   <222> (222)..(222)
   <223> n is a, c, g, or t
   <220>
   <221> misc_feature
   <222> (225)..(228)
   <223> n is a, c, g, or t
   <400> 802
   actgtgagtt ccactgaata cattttaatg tctgtaggaa gaatcaaaac acctatttaa 60
   agatggcaat atataataat cattttaaaa gtatttgatt naaccnnnta attttccaga 120
   aatgnnaaaa aaaaaaatca gctctaaaac caaagctgat ttcagaaaat ttgaaaatgt 180
   aaatcagccc tatccataat atagtttctc taaaacttta tnttnnnnag tcattttaaa 240
   ataatataac tattaaaaaa tgtaactgct atcttaatgt tctgaaataa tttaaaacat 300
   tttaaaatat gaatactgta gtataaaaga aagaaatggt gggaacgaaa agcagagaaa 360
   gaaatgccaa ttccagtcca aagttttatt tgccaagttt tcttagaatg aattttacca 420
   gtttatgaat tattgtaaac agaatgtgtc atggaaatac tgaaagattt ttccctagag 480
   tggccttatt gactgctggt gtgatgccac tgtaa 515
<210> 803
   <211> 197
   <212> DNA
   <213> Homo sapiens
   <400> 803
   tcagctttac cctctgaact tctgatcgaa ggtcatccct ctccagcttg agtggatcaa 60
   agatgacaag ggccaatgga accaagtttg agtcttgcca ggtcaatact tgggtcctga 120
   gtatggtgac tagtatctgt tttgttatgt gtgtattatt ccagccagaa tgggaaatgc 180
   taattcagct cctccag 197
<210> 804
   <211> 483
   <212> DNA
   <213> Homo sapiens
<220>
   <221> misc_feature
   <222> (48)..(48)
   <223> n is a, c, g, or t
   <220>
   <221> misc_feature
   <222> (212)..(212)
   <223> n is a, c, g, or t
   <400> 804
   ggaattcttg ttcaatactg gcaggagtga aaattggtag aacctttnta gaaggcaatt 60
   tggcaacatg tatgaaaacc taaatgttga tacaccttta cccagcagtt tgtttaggaa 120
   tttatcctaa tgaataaaag ttgtccaagt cttcaaacat gagcccaaag gtatatttca 180
   tgatgtttat gatattaaaa cattggaaac anctgaaaca tccttcagta aaagatggat 240
   taaataaatt ccatgcagtt gtcatttaaa aatatttaga tatatgttta ttgctatgga 300
   tatatgttcc caaaatatta ttgaatcaaa aagtagacta caggatatat gttgaatatg 360
   agctcattta taacattgaa tattttaaga taatgtatgt ttcatagaga gatcttcacc 420
   aaatgttaag gatttttttt tctgggctgt ggtatttggg tgatctttac attcttcaga 480
   ctc 483
<210> 805
   <211> 508
   <212> DNA
   <213> Homo sapiens
<220>
   <221> misc_feature
   <222> (224)..(224)
   <223> n is a, c, g, or t
   <220>
   <221> misc_feature
   <222> (260)..(261)
   <223> n is a, c, g, or t
   <400> 805
   ggttacctcc cacagaacgt ggtggactcc ttcttccccc gcagcatgac ccggttttat 60
   gccaaccttc agaaagcagt gaagcaattc catgagtaat gctatcgtta cttcttggca 120
   aagaactccc gtgactcatc gaggagctcc agctgttggg acaccaagga gcctgggagc 180
   acgcagaggc ctgtgttcac tctttggaac aagctgatgg actncgcatc tctgagaatg 240
   ccaaccagag gcggcagccn ncccttcctg cctcctgccc cactcagggt tggcgtgtga 300
   tgagccattc atgtgttcca aactccatct gcctgttacc caaacacgcc tctcctggca 360
   gggtagaccc aggcctctaa ccatctgaca gagactcggc ctggacacca tgcgatgcac 420
   tctggcacca aggctttatg tgcccatcac tctcagagac cacgtttccc tgactgtcat 480
   agagaatcat catcgccact gaaaacca 508
<210> 806
   <211> 494
   <212> DNA
   <213> Homo sapiens
   <400> 806
   ccctggatgc gcaagctgca cataagtcat gacaacatag gcggcccgga aggcaaaagg 60
   gcccggacgg cctacacgcg ctaccagacc ctggagctgg agaaggagtt ccacttcaac 120
   cgttacctga cccgcagaag gaggattgaa atagcacatg ctctttgcct ctccgagaga 180
   caaattaaaa tctggttcca aaaccggaga atgaagtgga aaaaagataa taagctgaaa 240
   agcatgagca tggccgcggc aggaggggcc ttccgtccct gagtatctga gcgtttaaag 300
   tactgagcag tattagcgga tcccgcgtag tgtcagtact aaggtgactt tctgaaactc 360
   ccttgtgttc cttctgtgaa gaagccctgt tctcgttgcc ctaattcatc ttttaatcat 420
   gagcctgttt attgccatta tagcgcctgt ataagtagat ctgctttctg ttcatctctt 480
   tgtcctgaat ggct 494
<210> 807
   <211> 533
   <212> DNA
   <213> Homo sapiens
<220>
   <221> misc_feature
   <222> (26)..(26)
   <223> n is a, c, g, or t
   <220>
   <221> misc_feature
   <222> (42)..(42)
   <223> n is a, c, g, or t
   <220>
   <221> misc_feature
   <222> (48)..(48)
   <223> n is a, c, g, or t
   <220>
   <221> misc_feature
   <222> (75)..(75)
   <223> n is a, c, g, or t
   <220>
   <221> misc_feature
   <222> (83)..(83)
   <223> n is a, c, g, or t
   <220>
   <221> misc_feature
   <222> (121)..(121)
   <223> n is a, c, g, or t
   <400> 807
   aagtggggca aggatggacc agcagnaagg ggggtaaggc tnctgttnca cttccccctg 60
   cctccacaga acgangccac ggnattccgt tatcttcctc cagttttgtt ccttctccag 120
   ncctcagttc caccaggtgt caggactgca tgggggcctg gggcaggcag aggagtcagg 180
   ccagggtccc tgacggagca gcactcagca tgtgagtgag gccacagaaa aactctgccc 240
   cactgcttct tacctcacgg gggtggcttt cagggattct ttagcgcagc agattaaaat 300
   cttgccacag tcgagaaatt gacaacaagc ttccatgctg tacatggttc tctttttctc 360
   tcttttattt ttaaaaagaa aacccagaaa gatgtaccag atttgtgtaa atgagggtat 420
   gccagaaggt ggccagtttt gctttatgat cttatgaagg aagatttgtg accctacgta 480
   tatatataca cacacataca tatatatata tatcccgaac caacaacggg act 533
<210> 808
   <211> 358
   <212> DNA
   <213> Homo sapiens
<220>
   <221> misc_feature
   <222> (146)..(146)
   <223> n is a, c, g, or t
   <220>
   <221> misc_feature
   <222> (180)..(181)
   <223> n is a, c, g, or t
   <400> 808
   gaaactgtat gggtagcttt tttgtttgtt ttttgttttg tttttgtttt tgtttttgtt 60
   tttagttgta ggtcgcagcg gggaaatttt ttgcgactgt acacatagct gcagcattaa 120
   aaacttaaaa aaattgttaa aaaaanaaaa aaagggaaaa catttcaaaa aaaaaaaaan 180
   ngataaacag ttacaccttg ttttcaatgt gtggctgagt gcctcgattt tttcatgttt 240
   ttggtgtatt tctgatttgt agaagtgtcc aaacaggttg tgtgctggag ttccttcaag 300
   acaaaaacaa acccagcttg gtcaaggcca ttacctgttt cccatctgta gttattcg 358
<210> 809
   <211> 424
   <212> DNA
   <213> Homo sapiens
<220>
   <221> misc_feature
   <222> (263)..(263)
   <223> n is a, c, g, or t
   <400> 809
   agaacctgtc gtaccagcat catgagctgg atgcaggagc ccatggctga aaggagttaa 60
   aacgcccagt ggtcattaag tgaaacatct tttatcaacc tgcaaaagct gcagcgttct 120
   ctgccaggtc aaatgggcat gtttagaaaa taagagaaga tggctgagta tagctaatga 180
   ataaatggtt gtttctttag aaaattaaac acacacagag tgtaagagga gaggatacgg 240
   ccctccctga aggataaagt ccncctggac ggtgccctgc cctcgcttct cacattaact 300
   gcccaggaat gtcatgctga ttggttcccg gaagggtgtt tggcaagggg cagtgtatgg 360
   agctacgtgt agaaggagag aaatttgtgt gtggcttttg taaattttga ccgattgcag 420
   caat 424

<210> 810
   <211> 478
   <212> DNA
   <213> Homo sapiens
<220>
   <221> misc_feature
   <222> (333)..(333)
   <223> n is a, c, g, or t
   <220>
   <221> misc_feature
   <222> (360)..(360)
   <223> n is a, c, g, or t
   <400> 810
   tagagactcc cctctaaata atttactcct acattgtaaa tacattgatg ccaacaaaat 60
   tccaactgct actaacaaag gtttggttgg tgataagcta ataacagcta ctttgtgtag 120
   gaggtaaata tgtgtactgg agggggtaaa aatccattta ggttatggca aagatgggaa 180
   tcaaactgta aaactcatag ccccataaaa ttaatattct ttgttaagtg ccagagggtt 240
   taagagaact tcttgcttag agtttattga taataataat gcttcagaat atcccattta 300
   aatgtacagt gtaaatatgt aaaatatttt acnttcccag gcaagtttgt ggctgtattn 360
   ccacttagtg gctctttgtg actggcagtt ctgtatatct gaaacaaata agctgtaagc 420
   acttttgtta aaactttgtc aaataatcct tttatgtact tgttctcaga cctgttct 478
<210> 811
   <211> 529
   <212> DNA
   <213> Homo sapiens
   <400> 811
   ggggtcttgt ctgtcaaagc aaatgataag ttcactcagg ccattattga ctgctgaact 60
   ctcttccttc ccaactcttc cttgaaagag aaaaaaatac tttgccttct tgctctcctt 120
   atcaaatgtt tttgtacaaa tagtgtaagc ctgtttaagc aaaccaatta aaataggcac 180
   tgattatttt gatctgtttg taacaaatga atgtaagtac tatttacatg gtgtgcctag 240
   gaggagctga aatcattggc actttaatcc atattgtaaa gatcagtatc aaaagcatag 300
   tgttcttcac ctctcctcct cagcatccat ctctatatac ttgattaaat ggaaaagtct 360
   cttttatcac ctctatgtaa agttttatgg gtagttatcg tcagtgtatt taaatatatc 420
   ttctagtatg ttttaaaggc tggtcttcaa tactgtggag acaaaaaata aaagagcgta 480
   tgaaaagtac gttagacttt tgctggcatt caagtcatgg ctagtctgt 529
<210> 812
   <211> 554
   <212> DNA
   <213> Homo sapiens
   <400> 812
   aatagctaca gactggaagc cagccaaatc tccattgata gggaattgat ggaaggaact 60
   agggtatatc tatacaatgg gatactacac agctgtagaa aggactgcga actatttttg 120
   tagttctggt ctggagaaat ctccagaata taggaaatga aaaatgtaaa gcacagaaga 180
   gaatgtatgg tgtgctgtct gttgtataac gaagagacaa atggaaaaaa tatgtatttg 240
   ctttttttgt aaagcaatag aagaattagt tataccaata actaataaaa tgatctcctt 300
   gttagtggtg gtagggagct agacaaggat ggcaactatt tctgtatctt acataccttt 360
   tattttgagg ccctgtcaat gttttatata ataaacattt tttgaaaagg caactcttaa 420
   aactaaaaca aacttaacag tctgtcaagt tggtgatata accccacaga agacttactt 480
   caagtgactt gaaaacttag tattttgtct gtactttgct aatggaatat atcctacaga 540
   ccaaacaacc acaa 554
<210> 813
   <211> 533
   <212> DNA
   <213> Homo sapiens
   <400> 813
   ctggcctttg gtgaccactg agaaggacac ttcacgggcc cagagctcct ggtactgccc 60
   ttcctttgag ggccgtggag ggctgtggac agcccagcaa cctgtcgctc ttggaggctg 120
   gtgtggcctt gaggagggaa gcctcgcatg gccgctggaa gagaggcgcc tcctggcctg 180
   gctctgcaga acccaggggc acgctctggg cctgggctga ggaagtcccg ctctccccgc 240
   ggctctgagt tggactgagg acaggtgtgg gcgccagtgt gggtgcaggc gcaggtgcag 300
   gcacagggcc actgtcctcc aggcaggctt tttggtgcta ggccctggga ctggaagtcg 360
   cccagcccgt atttatgtaa aggtatttat gggccactgc acatgcccgc tgcagccctg 420
   ggatcagctg gaagctgcct gtcatctcct gcccaatccc cagaaaccct gattcaggtc 480
   tgcaggctcc tgcgggctca ccaggctgct ggctccggta ccatgtaaac cta 533
<210> 814
   <211> 493
   <212> DNA
   <213> Homo sapiens
<220>
   <221> misc_feature
   <222> (76)..(76)
   <223> n is a, c, g, or t
   <400> 814
   agttttgctt ttgactccag gaacaaaaag gtaaatccca catcccagtt tctcagaagt 60
   ccctgtttat tccaantgcc atcagatgtg tgcaatgtgg caaactgaag ctgcacagtg 120
   ttggtttcct tgtattctga ggatgttaaa gactttgtta aatggttatc caattgctct 180
   ttcacaggta gcctattaaa ctattttaat atgttttttt aaacctcata aaaatctagc 240
   acactcttct cttgagcagt tagcagacct aaagcaagcc tgaattggct atgcagtaca 300
   ttgtattctg tttgggggaa tttgttttag ccattttctt taattaccag ttttccagaa 360
   cactcttagc tatgttgaca tgaggcagtt ccttccaggt gattctgttt ccttaagtat 420
   tatataaact gtgccaatac agacaaagca taatcaatat aatctgaatt attgttatct 480
   ttacctcctg agt 493
<210> 815
   <211> 295
   <212> DNA
   <213> Homo sapiens
   <400> 815
   gtatttggtc ccagttgggt acattttaaa atcctgattt tggagactta aaaccaggtt 60
   aatggctaag aatgggtaac atgactcttg ttggattgtt attttttgtt tgcaatgggg 120
   aatttataag aagcatcaag tctctttctt accaaagtct tgttaggtgg tttatagttc 180
   ttttggctaa caaatcattt tggaaataaa gattttttac tacaaaaatg aaatttgttt 240
   ggacttccac ttgagacagt aaagagagta ttagacaccc agtaaaaact gccat 295
<210> 816
   <211> 422
   <212> DNA
   <213> Homo sapiens
   <400> 816
   atggctctgg aaaaccagct gctacttcca aatctattgt ccataatggt ttctttctga 60
   ggttgcttct tggcctcaga ggaccccagg ggatgtttgg aaatagcctc tctacccttc 120
   tggagcatgg tttacaaaag ccagctgact tctggaattg tctatggagg acagtttggg 180
   tgtaggttac tgatgtctca actgaatagc ttgtgtttta taagctgctg ttggctatta 240
   tgctggggga gtcttttttt tttatattgt atttttgtat gccttttgca aagtggtgtt 300
   aactgttttt gtacaaggaa aaaaactctt ggggcaattt cctgttgcaa gggtctgatt 360
   tattttgaaa ggcaagttca cctgaaattt tgtatttagt tgtgattact gattgcctga 420
   tt 422
<210> 817
   <211> 352
   <212> DNA
   <213> Homo sapiens
   <400> 817
   gtcacacttt atggtctctg gaccccttaa tgtctgattc atgtagcaga agccagctag 60
   attttcatct gtctctattc attttgttgt gatgtcatgg atcatgtggc ctctggaaaa 120
   ctctactgta tactcgagaa tgagaatata acaggcaaaa taacattatc atgaaaatag 180
   ttttgacctc atgaacccca tgaaaggttc cccagaccaa aattttagaa tcactggtat 240
   agggtaacac tttattgtgt aaattcagtt ctctgtaccc cacttaaata tgtattatta 300
   tctcttgaca ttattttccc aaaaaatgct gtttgatttc ttacttgttc tg 352
<210> 818
   <211> 335
   <212> DNA
   <213> Homo sapiens
   <400> 818
   acaaggccca ggctggggcc agggccagag gggaaggccc tggattctca ctcatgtgag 60
   atcttgaatc tctttctttg ttctgtttgt ttagttagta tcatctggta aaatagttaa 120
   aaaacaacaa aaaactctgt atctgtttct agcatgtgct gcattgactc tattaatcac 180
   atttcaaatt caccctacat tcctctcctc ttcactagcc tctctgaagg tgtcctggcc 240
   agccctggag aagcactggt gtctgcagca cccctcagtt cctgtgcctc agcccacagg 300
   ccactgtgat aatggtctgt ttagcacttc tgtat 335
<210> 819
   <211> 261
   <212> DNA
   <213> Homo sapiens
   <400> 819
   gaatgaagaa aagtcgcctc aacgacaaac aaaagcaccg actagatttc cttcagctga 60
   tgattgactc ccagaattcg aaagaaactg agtcccacaa agctctgtct gatctggagc 120
   tcgcagccca gtcaataatc ttcatttttg ctggctatga aaccaccagc agtgttcttt 180
   ccttcacttt atatgaactg gccactcacc ctgatgtcca gcagaaactg caaaaggaga 240
   ttgatgcagt tttgcccaat a 261
<210> 820
   <211> 245
   <212> DNA
   <213> Homo sapiens
   <400> 820
   ggtgagggga tgacccctgg agatgaaggg aagaggtgaa gccttagcaa aaatgcctcc 60
   tcaccactcc ccaggagaat ttttataaaa agcataatca ctgattcctt cactgacata 120
   atgtaggaag cctctgagga gaaaaacaaa gggagaaaca tagagaacgg ttgctactgg 180
   cagaagcata agatctttgt acaatattgc tggccctggt tcacctgttt actgttatca 240
   caata 245
<210> 821
   <211> 273
   <212> DNA
   <213> Homo sapiens
   <400> 821
   acttaggtaa ttgtagggcg aggattataa atgaaatttg caaaatcact tagcagcaac 60
   tgaagacaat tatcaaccac gtggagaaaa tcaaaccgag cagggctgtg tgaaacatgg 120
   ttgtaatatg cgactgcgaa cactgaactc tacgccactc cacaaatgat gttttcaggt 180
   gtcatggact gttgccacca tgtattcatc cagagttctt aaagtttaaa gttgcacatg 240
   attgtataag catgctttct ttgagtttta aat 273
<210> 822
   <211> 492
   <212> DNA
   <213> Homo sapiens
   <400> 822
   ttgtcaaggg gctttgcatt caaactgctt ttccagggct atactcagaa gaaagataaa 60
   agtgtgatct aagaaaaagt gatggtttta ggaaagtgaa aatatttttg tttttgtatt 120
   tgaagaagaa tgatgcattt tgacaagaaa tcatatatgt atggatatat tttaataagt 180
   atttgagtac agactttgag gtttcatcaa tataaataaa agagcagaaa aatatgtctt 240
   ggttttcatt tgcttaccaa aaaaacaaca acaaaaaaag ttgtcctttg agaacttcac 300
   ctgctcctat gtgggtacct gagtcaaaat tgtcattttt gttctgtgaa aaataaattt 360
   ccttcttgta ccatttctgt ttagttttac taaaatctgt aaatactgta tttttctgtt 420
   tattccaaat ttgatgaaac tgacaatcca atttgaaagt ttgtgtcgac gtctgtctag 480
   cttaaatgaa tg 492
<210> 823
   <211> 519
   <212> DNA
   <213> Homo sapiens
<220>
   <221> misc_feature
   <222> (118)..(118)
   <223> n is a, c, g, or t
   <220>
   <221> misc_feature
   <222> (125)..(125)
   <223> n is a, c, g, or t
   <220>
   <221> misc_feature
   <222> (133)..(133)
   <223> n is a, c, g, or t
   <220>
   <221> misc_feature
   <222> (136)..(136)
   <223> n is a, c, g, or t
   <400> 823
   gagtatacat cggtgcaggc ttcctggatg acagttgggt gatatgtgtc atgtggccta 60
   aaagcctcca tgtcatttga cctacgaatt ctatctttgg gaatttatcc taagaaanta 120
   cttanggatt tanttngtga taagatgttc atcccagcat tgcaatggag aaaaatggga 180
   agcaatggtt tggttgggaa tttattcctt ttctgctgta acgaaagttt gcaatagggg 240
   attgcttaag taaattattg tatctccatc cagatggtgg agtaccgcgc agacattaaa 300
   agtcatgtaa aagaacatct gactgaaaga aaaatgctcc ttgaatatta aaaggttgta 360
   aaaatagtgc atgttatgtg atttcaattt tgttttttaa aatatgggtg tatgcttgta 420
   tacgtagagc agataaaaaa gacggaaggc atactaaaaa atgttgagtg gttatctttg 480
   tatggtggaa caaagtcact gtaattttca tctttggtt 519
<210> 824
   <211> 375
   <212> DNA
   <213> Homo sapiens
<220>
   <221> misc_feature
   <222> (310)..(310)
   <223> n is a, c, g, or t
   <220>
   <221> misc_fcature
   <222> (312)..(312)
   <223> n is a, c, g, or t
   <400> 824
   tcccttgcct tcactgtaat gcttaatggt tgtgtagtct tatacgtgac tcctgacttc 60
   aaggatcctg gtctgtacct ctttaggtca acacgttttg agtgaactgg tgttggttat 120
   ttggaattag atataaagtc atatattctt tggtgaggaa tggcttcata taggagttca 180
   cattcaaaac aagctttgac aaaataatag agtgaaaatt ggtagatcag agttgagctg 240
   attggaggac caaattaaaa gactggctgg gcatgatggc tcacacctga aaacccagca 300
   ctttgggagn cnaaggcagg cagattgttt gagcccagga attcaagacc agcctagata 360
   acctgggtat cccag 375
<210> 825
   <211> 387
   <212> DNA
   <213> Homo sapiens
<220>
   <221> misc_feature
   <222> (74)..(74)
   <223> n is a, c, g, or t
   <220>
   <221> misc_feature
   <222> (99)..(99)
   <223> n is a, c, g, or t
   <220>
   <221> misc_feature
   <222> (112)..(112)
   <223> n is a, c, g, or t
   <220>
   <221> misc_feature
   <222> (128)..(128)
   <223> n is a, c, g, or t
   <400> 825
   gagcacatat cttacaaaac accaaaaaat tcatagtgaa gagaaatcaa atatacatac 60
   tgagtgtggg gaanccatta gacaaaactc ttctttttna caacaataaa ancctcacac 120
   tggagagntt ctctgaatgc cttaagaatt tggttaatat ggagaccctt cccagggaaa 180
   cagaaggagg atcgtgaaaa ctgttgacta cttagaatga tcacatggtt tagtggagag 240
   agcatgattc tgggttttaa aagtcatgga tctcaatctc agctcctatt actaactaga 300
   tcttttactt tggggtaagt cacttcatat ctttaggcct taatttcctc atctgaaaaa 360
   ctggaaggcc tgacttgttg agcttta 387
<210> 826
   <211> 178
   <212> DNA
   <213> Homo sapiens
<220>
   <221> misc_feature
   <222> (119)..(119)
   <223> n is a, c, g, or t
   <220>
   <221> misc_feature
   <222> (124)..(124)
   <223> n is a, c, g, or t
   <400> 826
   tatgactgct aaaagaacca acccaggaca gagccacaat cttcctctat ttcattgtaa 60
   tttatatatt tcacttgtat tcatttgtaa aactttgtat tagtgtaaca tactccccnc 120
   agtntacttt tacaaacgcc tgtaaagact ggcatcttca caggatgtca gtgtttaa 178
<210> 827
   <211> 426
   <212> DNA
   <213> Homo sapiens
   <400> 827
   gagagtggtt ggggggagtg ggagaggttt gggggctggg aagacaaggg aaaagaaaat 60
   gcaggtatat gctatttgtg ttcattttgt ctttgaaaat cgtaagtgta cagcatcatt 120
   ctcgggcaga gtctgggagg acttgagttg actgctacag tttatgatct tccctaaaca 180
   tcgacgttcc tggaaatctt tggcctctga gctgacttct tctctgttgc ttgtgagcca 240
   ggaatttaac agctctgttg tatgtgcagg ctgcagatgc ttctcttcag cttttgctat 300
   ccaatgtgtg tgtgtgtgtg tgtgtgtgtg tgtgtgtgtg tgtgtgtgtg tgtgtgttgg 360
   ataaacttaa aaaacctgtt gcttccatgc aacggcccac acaacttggg actcatggtc 420
   agcctc 426
<210> 828
   <211> 400
   <212> DNA
   <213> Homo sapiens
   <400> 828
   tctgttccaa aatgtacgga ccccacttac aatgaaattg tagtatatga tgaagtcaca 60
   gagctccaag gacatgtctt aatgcttatt gtgaagagta aaactgtatt tgtgggagca 120
   attaacatcc gactctgtag tgtcccactc gataaagaaa aatggtatcc attaggaaac 180
   agtataattt gaccattgct atgaacatat gcattattca ttaactactt gtattttttt 240
   cacttccggg cctctgaatc acataagtaa ggcatctttg ttgtcaaaga cagcacaggg 300
   tattaaggac acagaaaaaa aatcagaatt agtcttttgt gttgtttatt ttctacctgt 360
   gctttcattg ttttttcata atcttttctc cttcagtgga 400
<210> 829
   <211> 520
   <212> DNA
   <213> Homo sapiens
   <400> 829
   taaagccttt aactggtcct caactcttac taaacataag agaattcata ctggagagaa 60
   gccctacaaa tgtgaagaat gtggcaaagc ttttaaccgg tcctcaaacc ttactcgaca 120
   taagaaaatt catactggag agaaaccata caaacctaaa agatgtgaca gtgcttttga 180
   caacacccca aacttttcta gacataaaag aaatcatatg ggtgagaaat cctagaaatg 240
   tgaagaatgt gacaaagcct ttaagcggtt gtcacacttg attgtatata agataattca 300
   tactggagaa aactcccaga agtgtgacaa atgtgacaaa acatttaatt aattctcata 360
   ccttattgca caggaaagca tttatacttg agaaaaattg tataaagaat ggaaaagtca 420
   ttaatatctg ctcatatctt aacatcagcg agttggtatt taataaaagc attatcaatg 480
   aaattactgg caaaagatct ttcagaccat ataagcctgc 520
<210> 830
   <211> 347
   <212> DNA
   <213> Homo sapiens
<220>
   <221> misc_feature
   <222> (223)..(223)
   <223> n is a, c, g, or t
   <400> 830
   cactgctagc agggcttcaa ccaggaaggg atcaacccag gaagggatga tcaggagagg 60
   cttccctgag gacataatgt gtaagagagg tgagaagtgc tcccaagcag acacaacagc 120
   agcacagagg tctggaggcc acacaaaaag tgatgctcgc cctgggctag cctcagcaga 180
   cctaaggcat ctctactccc tccagaggag ccgcccagat tcntgcagtg gagaggaggt 240
   cttccagcag cagcaggtct ggagggctga gaatgaacct gactagaggt tctggagata 300
   cccagaggtc ccccaggtca tcacttggct cagtggaagc cctcttt 347
<210> 831
   <211> 519
   <212> DNA
   <213> Homo sapiens
<220>
   <221> misc_feature
   <222> (326)..(326)
   <223> n is a, c, g, or t
   <220>
   <221> misc_feature
   <222> (374)..(376)
   <223> n is a, c, g, or t
   <220>
   <221> misc_feature
   <222> (398)..(401)
   <223> n is a, c, g, or t
   <400> 831
   gaaccacctc aatgcaaaga ttctacggga aaatgtgggc cccctccacc tattgacaat 60
   ggggacatta cttcattccc gttgtcagta tatgctccag cttcatcagt tgagtaccaa 120
   tgccagaact tgtatcaact tgagggtaac aagcgaataa catgtagaaa tggacaatgg 180
   tcagaaccac caaaatgctt acatccgtgt gtaatatccc gagaaattat ggaaaattat 240
   aacatagcat taaggtggac agccaaacag aagctttatt tgagaacagg tgaatcagct 300
   gaatttgtgt gtaaacgggg atatcngtct ttcatcacgt tctcacacat tgcgaacaac 360
   atgttgggat gggnnnctgg agtatccaac ttgtgcannn ngatagaatc aatcataaaa 420
   tgcacacctt tattcagaac tttagtatta aatcagttct taatttcatt tttaagtatt 480
   gttttactcc tttttattca tacgtaaaat tttggatta 519
<210> 832
   <211> 416
   <212> DNA
   <213> Homo sapiens
   <400> 832
   cagcccactc tcaagatttt gaagacattt gcctttgttt tcctccagaa actttatagt 60
   tttagctgtt ggatctgtga ttatcaccag ttgatttttg tgtatggtgt gaggggggga 120
   tcaagattta ttttgtatat ggacatccat ctactctaca catttattga aaaaaacaac 180
   acctttcttt tcccattgaa ttgcgtgggg actttgttaa taaatgaatg gtcatatatt 240
   tgggtctgtt tctggactct gttctttcca cttggactaa ttatccattc ttgcatcagt 300
   accatacttt tttaattact gtagtttatg gtaagtcttg acatggtatt gtaaaccctc 360
   cagttttgtt cttttaaaca aatgttttga ctatttaagt gctttacatt tccata 416
<210> 833
   <211> 482
   <212> DNA
   <213> Homo sapiens
   <400> 833
   agcagatgga gcccaaaagc ttttggtgaa ggccaaagca gctgagaaag cagcaaatat 60
   tctattaaat cttgacaaaa cattgaacca gttacaacaa gctcaaatca ctcaaggacg 120
   ggcaaactct accattacac agctgactgc caatataaca aaaataaaaa agaatgtgct 180
   gcaggaattt gttgagctga aaaaacaata tgctattctc caacgtaaga caagcactac 240
   aggactaaca aaggagacat taggaaaagt taaacagcta aaagatgcgg cagaaaaatt 300
   ggctggagat acagaggcca agataagaag aataacagat ttagaaagga aaatccaaga 360
   tttgaatcta agtagacaag caaaagctga tcaactgaga atattggaag atcaagttgt 420
   tgccattaaa aatgaaattg ttgaacaaga aaaaaaatat gctaggtgct atagctaggc 480
   ag 482
<210> 834
   <211> 212
   <212> DNA
   <213> Homo sapiens
   <400> 834
   ccttatcatc cgtcacaggg gtcagaaagg acctcgaggg cctccaccag caggtcacct 60
   tctgtgatcc ccatcccaag gcactggtgg tgactctgct tcctgcactg acccagagcc 120
   tctgcctgtg cactgcaagc tgtgtctact caggccccaa ggggactctc tgtttccatt 180
   ctccccccac agacctgtca agagaagcat ga 212
<210> 835
   <211> 264
   <212> DNA
   <213> Homo sapiens
   <400> 835
   ttcctaaatg gtcttccttt tccatttttt cccttgtaaa ataatctgct tttaatttag 60
   cgagctcttc tcatgtgttt atcatttaaa tgaataagta aatgagggca gtttgcttac 120
   tggttaagaa aggatgcagg ctttagggct ggaagcacct ggtttcaaag cctggctctg 180
   cctcttatca gctgcgtaac ctttggacaa gttgctttat tgctctaagt ttcagtttcc 240
   tcctgtgtca actctagagg actg 264
<210> 836
   <211> 484
   <212> DNA
   <213> Homo sapiens
<220>
   <221> misc_feature
   <222> (190)..(190)
   <223> n is a, c, g, or t
   <220>
   <221> misc_feature
   <222> (420)..(420)
   <223> n is a, c, g, or t
   <220>
   <221> misc_feature
   <222> (424)..(424)
   <223> n is a, c, g, or t
   <400> 836
   tgggatttag tcagtcacag agatactatt actatgagta agaaattaat ggcaaaggaa 60
   ttaatccaag aatagaagaa tgaagcaagt tcactttcaa tcaagaaact tcataatact 120
   ttcagggaag ttatcttttc ctgtcaatct gtttaaaata tgctatagta tttcattagt 180
   ttggtggtan cttattttta ttgtgtaatg atctttaaac gctatatttc agaaatatta 240
   aatggaagaa atcaatatca tggagagcta actttagaaa actagctgga gtattttagg 300
   agattctggg tcaagtaatg ttttatgttt ttgaaagttt aagttttaga cactccccaa 360
   atttctaaat taatcttttt cagaaatatc gaaggagcca aaaatataaa acagttctgn 420
   atanccaaag tggctatatc aacatcaggg ctagcacatc tttctctatt atccttctat 480
   tgga 484
<210> 837
   <211> 383
   <212> DNA
   <213> Homo sapiens
<220>
   <221> misc_feature
   <222> (319)..(319)
   <223> n is a, c, g, or t
   <400> 837
   gacagaccaa agttaaacaa gcctccggaa actcttatca ctactattga ttctagttcc 60
   agttggtgga ccaactgggt gatccctgcc atctctgcag tggccgtcgc cttgatgtat 120
   cgcctataca tggcagagga ctgaacacct cctcagaagt cagcgcagga agagcctgct 180
   ttggacacgg gagaaaagaa gccattgcta actacttcaa ctgacagaaa ccttcacttg 240
   aaaacaatga ttttaatata tctctttctt tttcttccga cattagaaac aaaacaaaaa 300
   gaactgtcct ttctgcgcnc aaatttttcg agtgtgcctt tttattcatc tactttattt 360
   tgatgtttcc ttaatgtgta att 383
<210> 838
   <211> 507
   <212> DNA
   <213> Homo sapiens
   <400> 838
   gattcctgtg ggtccagctt tggaactggg aaacctttct tcggatccgc actcattcca 60
   ctgatgccag ctgcccctga aggatgccag tactgtggtg tgtgagtctc agcagccgcc 120
   cacacgctcc taactctgct gcatggcaga tgcctaggtg gaaatagcaa aaacaaggcc 180
   cgggctgggg ccagggccag aggggaaggc cctggattct cactcatgtg agatcttgaa 240
   tctctttctt tgttctgttt gtttagttag tatcatctgg taaaatagtt aaaaaacaac 300
   aaaaaactct gtatctgttt ctagcatgtg ctgcattgac tctattaatc acatttcaaa 360
   ttcaccctac attcctctcc tcttcactag cctctctgaa ggtgtcctgg ccagccctgg 420
   agaagcactg gtgtctgcag cacccctcag ttcctgtgcc tcagcccaca ggccactgtg 480
   ataatggtct gtttagcact tctgtat 507
<210> 839
   <211> 502
   <212> DNA
   <213> Homo sapiens
   <400> 839
   ctggagtctg gggtgtgttg tcatagagat ggtgactggc aaggtttgca cagatgaaga 60
   atgaagccta gtagaatatg gacttggaaa attctcttaa tcactactgt atgtaatatt 120
   tacataaaga ctgtgctgag aagcagtata agccttttta accttccaag actgaagact 180
   gcacaggtga caagcgtcac ttctcctgct gctcctgttt gtctgatgtg gcaaaaggcc 240
   ctctggaggg ctggtggcca cgaggttaaa gaagctgcat gttaagtgcc attactactg 300
   tacacggacc atcgcctctg tctcctccgt gtctcgcgcg actgagaacc gtgacatcag 360
   cgtagtgttt tgacctttct aggttcaaaa gaagttgtag tgttatcagg cgtcccatac 420
   cttgttttta atctcctgtt tgttgagtgc actgactgtg aaacctttac cttttttgtt 480
   gttgttggca agctgcaggt tt 502
<210> 840
   <211> 328
   <212> DNA
   <213> Homo sapiens
   <400> 840
   gatttctttt caccattcgt acataatact gaaccacttg tagatttgat tttttttttt 60
   aatctactgc atttagggag tattctaata agctagttga atacttgaac cataaaatgt 120
   ccagtaagat cactgtttag atttgccata gagtacactg cctgccttaa gtgaggaaat 180
   caaagtgcta ttacgaagtt caagatccaa aaggcttata aaacagagta atcttgttgg 240
   ttcaccattg agaccgtgaa gatactttgt attgtcctat tagtgttata tgaacataca 300
   aatgcatctt tgatgtgttg ttcttggc 328
<210> 841
   <211> 546
   <212> DNA
   <213> Homo sapiens
   <400> 841
   gacacaggca ggtgactact ccatccgcgt ggacctgcgg gctggggacg aggctgtgtt 60
   cgcccagtac gactccttcc acgtagactc ggctgcggag tactaccgcc tccacttgga 120
   gggctaccac ggcaccgcag gggactccat gagctaccac agcggcagtg tcttctctgc 180
   ccgtgatcgg gaccccaaca gcttgctcat ctcctgcgct gtctcctacc gaggggcctg 240
   gtggtacagg aactgccact acgccaacct caacgggctc tacgggagca cagtggacca 300
   tcagggagtg agctggtacc actggaaggg cttcgagttc tcggtgccct tcacggaaat 360
   gaagctgaga ccaagaaact ttcgctcccc agcgggggga ggctgagctg ctgcccacct 420
   ctctcgcacc ccagtatgac tgccgagcac tgaggggtcg ccccgagaga agagccaggg 480
   tccttcacca cccagccgct ggaggaagcc ttctctgcca gcgatctcgc agcactgtgt 540
   ttacag 546
<210> 842
   <211> 399
   <212> DNA
   <213> Homo sapiens
   <400> 842
   tcacaaactt ttatactctt tctgtatata catttttttt ctttaaaaaa caactatgga 60
   tcagaatagc cacatttaga acactttttg ttatcagtca atatttttag atagttagaa 120
   cctggtccta agcctaaaag tgggcttgat tctgcagtaa atcttttaca actgcctcga 180
   cacacataaa cctttttaaa aatagacact ccccgaagtc ttttgttcgc atggtcacac 240
   actgatgctt agatgttcca gtaatctaat atggccacag tagtcttgat gaccaaagtc 300
   ctttttttcc atctttagaa aactacatgg gaacaaacag atcgaacagt tttgaagcta 360
   ctgtgtgtgt gaatgaacac tcttgcttta ttccagaat 399
<210> 843
   <211> 543
   <212> DNA
   <213> Homo sapiens
   <400> 843
   gtggaatgtc atccttactt caaccagaga aaactgctgg atttctgcaa gtcaaaagac 60
   attgttctgg ttgcctatag tgctctggga tccctccgag aagaaccatg ggtggacccg 120
   aactccccgg tgctcttgga ggacccagtc ctttgtgcct tggcaaaaaa gcacaagcga 180
   accccagccc tgattgccct gcgctaccag ctacagcgtg gggttgtggt cctggccaag 240
   agctacaatg agcagcgcat cagacagaac gtgcaggtgt ttgaattcca gttgacttca 300
   gaggagatga aagccataga tggcctaaac agaaatgtgc gatatttgac ccttgatatt 360
   tttgctggcc cccctaatta tccgatctct gatgaatatt aacatggagg gcattgcatg 420
   aggtctgcca gaaggccctg cgtgtggatg gtgacacaga ggatggctct atgctggtga 480
   atattaacat ggagggcatt gcatgaggtc tgccagaagg ccctgcgttg tggatggtga 540
   cac 543

<210> 844
   <211> 496
   <212> DNA
   <213> Homo sapiens
   <400> 844
   ccccgattca gtcccggatt gtgggaggct gggagtgtga gcagcattcc cagccctggc 60
   aggcggctct gtaccatttc agcactttcc agtgtggggg catcctggtg caccgccagt 120
   gggtgctcac agctgctcat tgcatcagcg atgtgaaggt cgtggagttg cccacccagg 180
   aacccgaagt ggggagcacc tgtttggctt ccggctgggg cagcatcgaa ccagagaatt 240
   tctcatttcc agatgatctc cagtgtgtgg acctcaaaat cctgcctaat gatgagtgca 300
   aaaaagccca cgtccagaag gtgacagact tcatgctgtg tgtcggacac ctggaaggtg 360
   gcaaagacac ctgtgtgggt gattcagggg gcccgctgat gtgtgatggt gtgctccaag 420
   gtgtcacatc atggggctac gtcccttgtg gcacccccaa taagccttct gtcgccgtca 480
   gagtgctgtc ttatgt 496
<210> 845
   <211> 330
   <212> DNA
   <213> Homo sapiens
   <400> 845
   gcttctcctt gccagagcta ttatgttcaa gctcctgcaa gtggctcaac ctcccagtac 60
   tgtgtcactg acccatgctc tgctccctgt tccaccagct actgctgtct ggctccccgg 120
   accttcgggg tgagtcccct gagacgctgg attcagcggc cccagaactg caacacagga 180
   tcatctggct gctgtgagaa ttcgggaagc tctgggtgct gtggttctgg gggctgtggc 240
   tgcagctgtg gatgtggcag ctctgggtgc tgctgtttgg gaattatccc catgaagtcc 300
   cgaagtcctg cgttgctgtg accatgaaga 330
<210> 846
   <211> 453
   <212> DNA
   <213> Homo sapiens
   <400> 846
   sgatgaaatc tcactgctaa tgctcagaga tcttttttca ctgtaagagg taacctttaa 60
   caatatgggt attacctttg tctcttcata ccggttttat gacaaaggtc tattgaattt 120
   atttgtttgt aagtttctac tcccatcaaa gcagctttct aagttattgc cttggttatt 180
   atggatgata gttatagccc ttataatgcc ttaactaagg aagaaaagat gttattctga 240
   gtttgtttta atacatatat gaacatatag ttttattcaa ttaaaccaaa gaagaggtca 300
   gcagggagat actaaccttt ggaaatgatt agctggctct gttttttggt taaataagag 360
   tctttaatcc tttctccatc aagagttact taccaagggc aggggaaggg ggatatagag 420
   gtcacaagga aataaaaatc atctttcatc ttt 453
<210> 847
   <211> 152
   <212> DNA
   <213> Homo sapiens
<220>
   <221> misc_feature
   <222> (53)..(53)
   <223> n is a, c, g, or t
   <220>
   <221> misc_feature
   <222> (87)..(87)
   <223> n is a, c, g, or t
   <220>
   <221> misc_feature
   <222> (100)..(100)
   <223> n is a, c, g, or t
   <220>
   <221> misc_feature
   <222> (110)..(110)
   <223> n is a, c, g, or t
   <400> 847
   caccctgaac tcctatgtta ccaatgtgta tcgtctccct ctccctaaag tgnacttaat 60
   ctttgctttc ttttgcacaa tgtcttnggt tgcaagtcan aagcctgagn caaataaaat 120
   tccagtaatt tcgaagaatg tggtgttggt gc 152
<210> 848
   <211> 383
   <212> DNA
   <213> Homo sapiens
<220>
   <221> misc_feature
   <222> (112)..(113)
   <223> n is a, c, g, or t
   <220>
   <221> misc_feature
   <222> (267)..(267)
   <223> n is a, c, g, or t
   <400> 848
   cttgactgaa gatattttgc tagggaagtg aaactttaaa attttgtaga ttttaaaaaa 60
   tattgttgaa tggtgtcatg caaaggattt atatagtgtg ctcccactaa cnntgtacag 120
   atcaggacac atatttttag acatctaagt ctgtagctta aatggaggtt actcttccat 180
   catctagaat tgtttactta gtaattgttg tttcttttat tattatagac ttactatcag 240
   ttttattttg ccaagtatgc aacaggnata tcactagtat atgaaaatgt aaatatcact 300
   tgtgtactca aacaaaagtt ggtcttaagc ttccaccttg agcagccttg gaaacctaac 360
   ctgcctcttt tagcataatc aca 383
<210> 849
   <211> 506
   <212> DNA
   <213> Homo sapiens
   <400> 849
   tttgccttca gtaatcccct taaggagaaa atatatggac ctgatttcag ccttcagaat 60
   ctccaaaaga ggagtcatca attcatagag cacactaggg tgttaggaga gagctttgca 120
   tactctgaga ggctacttgg aaaggcattt tcccaggaga gctctgtcag gtggctgcgc 180
   ttcagcccca cccctacacc acagggtctc cttgggtatg ttcttgggca agcaatcaca 240
   aagccagaga agctgtaagc tgcctgccgg gcctgaggag ctccaaccag ggaagactgg 300
   atgtgaggag aggagtcact gtcaccaggt cacagactga ctgaggtgat ggtaggatga 360
   ggaggaacag atgcccttct ttaattggtt ctcagttaac ttctcagagg ctctggagaa 420
   cgggacagtg gctttctagc ctctgaatgt tccaaataaa attttttggt cttggcccct 480
   gtactgtttt acctctaaat tctggc 506
<210> 850
   <211> 244
   <212> DNA
   <213> Homo sapiens
   <400> 850
   ccgcgcgtgt ggacgggtcc aaatgcaagt gctcccggaa gggacccaag atccgctaca 60
   gcgacgtgaa gaagctggaa atgaagccaa agtacccgca ctgcgaggag aagatggtta 120
   tcatcaccac caagagcgtg tccaggtacc gaggtcagga gcactgcctg caccccaagc 180
   tgcagagcac caagcgcttc atcaagtggt acaacgcctg gaacgagaag cgcagggtct 240
   acga 244
<210> 851
   <211> 538
   <212> DNA
   <213> Homo sapiens
   <400> 851
   atctatccgt accaaatgat gttgaataat tacatatctt tcttgactat actgatttct 60
   tattttggtc actattacta aatctctgtt aatattctct cttttaactg aaaagggatg 120
   ggatagaagg gtttgcaatg ccatattatt ggtggagggc tgttttaaca tctttgaagt 180
   atggcttgct gaatatcttt accaacatct tgaatatata ttctagtgtc cacaagattt 240
   agcaaaaaga taaagcttgg gtggaatatc attttaaaat gttcatgttc tgttctatat 300
   tttcttcacc tactctccaa atattgtaat gcaaaaagtc tcagtaatga tttggtagta 360
   ttaattttgt ggtcattgtt tctcttcgat aaatttattt tcattaaata cttgttagag 420
   ggttttgaaa tgtttttcaa atatgtgaaa tgtgaaactg ctgtctttta tattaaagta 480
   attaaagaaa atgtattgtg attgaaatta ttttggcctc cacaagatgg ctctatga 538
<210> 852
   <211> 554
   <212> DNA
   <213> Homo sapiens
   <400> 852
   caccaagact aatctcagcc aaacctgctg cttggtggtg ccagcccctt gtccaccttc 60
   tcttgaggcc acagaactcc ctggggctgg ggcctctttc tctggcctcc cctgtgcacc 120
   tggggggtcc tggcccctgt gatgctcccc catccccacc cacttctaca tccatccaca 180
   ccccagggtg agctggagct ccaggctggc caggctgaac ctcgcacaca cgcagagttc 240
   tgctccctga ggggggcccg ggaggggctc cagcaggagg ccgtgggtgc cattcggggg 300
   aaagtggggg aacgacacac acttcacctg caagggccga caacgcaggg gacaccgtgc 360
   cggcttcaga cactcccagc gcccactctt acaggcccag gactggagct ttctctggcc 420
   aagtttcagg ccaatgatcc ccgcatggtg ttgggggtgc tggtgtgtct tggtgcctgg 480
   acttgagtct caccctacag atgagaggtg gctgaggcac cagggctaag caattaaacc 540
   agttaagtct ccca 554
<210> 853
   <211> 549
   <212> DNA
   <213> Homo sapiens
   <400> 853
   tcacctcggc gtactatcgt ggtgcagtgg gggccctcct ggtgtttgac ctaaccaagc 60
   accagaccta tgctgtggtg gagcgatggc tgaaggagct ctatgaccat gctgaagcca 120
   cgatcgtcgt catgctcgtg ggtaacaaaa gtgacctcag ccaggcccgg gaagtgccca 180
   ctgaggaggc ccgaatgttc gctgaaaaca atggactgct cttcctggag acctcagccc 240
   tggactctac caatgttgag ctagcctttg agactgtcct gaaagaaatc tttgcgaagg 300
   tgtccaagca gagacagaac agcatccgga ccaatgccat cactctgggc agtgccaggc 360
   tggacaggag cctggcctgg ggagaagagg gcctgttgca tcagcctctg accttggcca 420
   gcaccacctg cccccactgg ctttttgtgc cccttgtccc cacttcagcc ccaggacctt 480
   tccttgccct ttggttccag atatcagact gttccctgtt cacagcaccc tcagggtctt 540
   aaggtcttc 549
<210> 854
   <211> 554
   <212> DNA
   <213> Homo sapiens
   <400> 854
   ggcagctgaa ctcgggtagt ccagtggcct agctggtacc acatctattc ccatccagag 60
   acattctctg gcaagtgttc tcagctgaaa agtggttggg gatgattctt accttggtaa 120
   ttaaatgaag ctacacattt gggtaatcta gcaaatgaag tattttttcc ctcttggcaa 180
   cttgtgtcag agttactctg gtctgagtca actttcgctg gggaaaacct atggaaccta 240
   ctgcaaaaag attgtccaaa atgcctaaga aaatactcct ctgatgcatt tagccttcaa 300
   ccctacctgt cttgctgaag ggagaaaaat gttttagtac attataggcc cagcagcttt 360
   tattcatgtc caccagctag ttgcacagag aatcatgtgt acctaactaa ggatgatcta 420
   ggataagtaa ctcctgtttt atattgagta ttttagggaa gtctttaaaa gacttgtttt 480
   atatctataa atctaggtta ttacaaatac aagaattttg taccttaaat aagcctcatt 540
   tctatttctt cttc 554
<210> 855
   <211> 542
   <212> DNA
   <213> Homo sapiens
   <400> 855
   atccagctag attgcagttt aataattaaa ctgtacatac tgtgcatata atgaattttt 60
   atcttatgta aattattttt agaacacaag ttgggaaatg tggcttctgt tcatttcgtt 120
   taattaaagc tacctcctaa actatagtgg ctgccagtag cagactgtta aattgtggtt 180
   tatatacttt ttgcattgta aatagtcttt gttgtacatt gtcagtgtaa taaaaacaga 240
   atctttgtat atcaaaatca tgtagtttgt ataaaatgtg ggaaggattt atttacagtg 300
   tgttgtaatt ttgtaaggcc aactatttac aagttttaaa aattgctatc atgtatattt 360
   acacatctga taaatattaa atcataactt ggtaagaaac tcctaattaa aaggtttttt 420
   ccaaaattca ggttattgaa aatttttcat tttattcatt taaaaactag aataacagat 480
   atataaaagt gttaatcttt gtgctatatg gtatgaaata caatattgta ctcagtgttt 540
   tg 542
<210> 856
   <211> 320
   <212> DNA
   <213> Homo sapiens
   <400> 856
   ggatctcttt attgcacaga ctgaatggct ttacatgttt ctaatgtgaa ttaggcatgt 60
   gaagcagtgg gtgtccaccc gtgtccctca tgggtgagcc ctccagctgt gagcccaggc 120
   agtgtggtca ccgagtgagg accctcctca ccaggaaccg catccctgtg ctgcctccac 180
   ctgagagttg ctagggggtt cttgtcgaga tcatgtcatc agcaccccta agtcaagtca 240
   cgggtttcca tagccaggca gttggtatgt acaattcagt tcagcgtatg aacttgtatc 300
   tctaatctga tgtccatttt 320
<210> 857
   <211> 501
   <212> DNA
   <213> Homo sapiens
   <400> 857
   atttgttgaa gcctactgca tgccagccca ctgctcatcc acgtggtctg ccatgcctac 60
   gaggaaggcc agcgcatgca ggactggtct ctaatgctgt ggtcattgca cagaagggaa 120
   aggtctcaag gaagagtcaa ctgggacaag cacaagccca ccggacatgg ccttggtaaa 180
   ggttagcaga ctggtgtgtg tggatctgca gtgcttcact ggaaataatt tattcattgc 240
   agatactttt taggtggcat tttattcatt tcctgtgctt taaataaaca aatgtaccaa 300
   aaaacaagta tcaagctgtt taagtgcttc ggctacttgt cccctggttc agtagaggcc 360
   ccggtttccc agttgttgac tgtgacaggc tcagcatggg ctcagcagat gctgtcttaa 420
   tttgtggatg atacagaaag ccaggctttg ggatacaagt tctttcctct tcatttgatg 480
   ccgtgcactg tgtgaagcag a 501
<210> 858
   <211> 531
   <212> DNA
   <213> Homo sapiens
   <400> 858
   aatgtttaat tgtttggatc tgcacagttt ggtttttgca caaaagtcat ttaaaaaaat 60
   ctgagtaatt gtcaaatatt aaaagaaaga tattcttcct gtaaggaata cagtttttag 120
   tcaaagtggc cattacatcc tctttttaat ttacataata cagatacttg agaaagttgt 180
   tgtggtgttg tatgccaaga aaattctttt tattggtgcc tatattgtaa caattatttt 240
   taatgcattg tattttgaag taacggttca gttaaatttt tcacctgctg tgtaactgaa 300
   gcacaattac agtttataat catctgtaga agtctggaga taattttgca actcatgtta 360
   tgggttaaat gaatattttt gtaaaagtaa aagcaacaaa tttataaatt gattatttga 420
   aactttacaa cacaattgca tcccaaatac aaattgtatt gcttattcat tatagctatt 480
   cgtcctgtaa tctgtttcta ggtgaagcat actccagtgt tttaggggtt t 531
<210> 859
   <211> 493
   <212> DNA
   <213> Homo sapiens
   <400> 859
   ggcagcccac aagtttctcg tggggagatg gaggcagagc ccagggtagg ggacagagct 60
   gctggggcct ttccttgcct gggaatctgt cccaggaaga gcttccccac tcccatcccc 120
   caaattggaa aaaccgtaca ttcaagcctg tttggccctg aaattcttaa gaatctggtt 180
   aagaattaac tcactaatgt caaaagtcaa aacctcctag gggttgtcct gggagtcagg 240
   ttcacgggta cagaagatga atctcagatg tcactcaacc tgagccgtca ttctctgtgg 300
   cagggctgcc ctgggtttct cttactcaat ccctggagtg taagcatttg gattgtgtca 360
   cagattacct ttttaccttt tctttctttt tttttctttt tttcaatatc agtgcccaca 420
   ccttactgag tattgagttt tagagctttc gcttgatgtg cttgaccaag agacttcttt 480
   tgtatccttt tct 493
<210> 860
   <211> 527
   <212> DNA
   <213> Homo sapiens
   <400> 860
   ttcacgggcc gacgactgag tggaactgag gcccacgtac tggggctggt gaatcacgct 60
   gtggcccaga acgaggaggg ggacgccgcc taccagcggg cacgagcact ggcccaggag 120
   atcctgcccc aggcccccat tgccgtgcgg ctgggcaaag tagccattaa ccgaggaacg 180
   gaggtggaca ttgcatctgg gatggccatt gaagggatgt gctatgccca gaatattcca 240
   acccgggacc ggctagaggg catggcagcc ttcagggaga agcggactcc caaatttgtt 300
   ggcaaatgac ccccatttta accttcagca tgggagatgc atgccctgaa gagcaggatc 360
   cagaaggaag atttgtggcc agattgcctt catcatttca cctctccaga cttccatttc 420
   ttcacaagga tgatgatgga aataaaatga ctggcgtgat gcctggaacc aaggtgctga 480
   tcctaccacc tactgctacc ttccttagct tcaccctggc tagaaat 527
<210> 861
   <211> 464
   <212> DNA
   <213> Homo sapiens
   <400> 861
   atgtacctta ttagagcacc agaactaatt tgctaagtct tttgtttagt cctgcaagac 60
   tgatgcttaa tacacagtct gttctcctgt gtctaggtca ggaactccag tttgcttttc 120
   tgttttgtgt cctggtagca gctgttgagt aactttcatt ggaggttggg aaggaagtga 180
   ggagaaagtg ttcttgttta gtgttttatt tcctataata ggatgctgcc taacccagtt 240
   catctctatg tcctgttcac tgaatattcc gggtaattga aagaaaatat aatggatggg 300
   ctccattaaa accagctcaa aaataaattc ttgtcagtaa agatttcttg tcaagatgtc 360
   ttggattgca cttttgttga ggaaagacag tgtaaatagt taaagaatgt tgataaaatt 420
   gaaacatttg gttgtggaat tgtgtgtggt tttagagggt ttct 464
<210> 862
   <211> 548
   <212> DNA
   <213> Homo sapiens
   <400> 862
   tgcattacta tgacccttcc aaagaagaga acaggccagt gggtgggttt tctcttcgtg 60
   gttcactcgt gtctgctctg gaagataatg gcgttcccac tggggttaaa gggaatgtcc 120
   agggaaacct cttcaaagtg attactaagg atgacacaca ctattacatt caggccagca 180
   gcaaggctga gcgagccgag tggattgaag ctatcaaaaa gctaacatga caaggacctg 240
   agggaaccag gattcctccc tcctaccaga tgacacagac aagagttcct ggagaatggg 300
   agtgttaaga cttttgactt ctttgtaagt tttgtactgc tttggagagt gaatgctgcc 360
   aagagttcct cagattacaa acagcagtgg tgccatttcc ttccccatct tcatgttaca 420
   aacctggaaa ggctagaaca gccattaggc gtcagcatct tgacttttcc ccagcatcac 480
   aaacagccat ttcctcgggc accaaagtag gttccctttg ttggaacaat tacactggcc 540
   atgccata 548
<210> 863
   <211> 505
   <212> DNA
   <213> Homo sapiens
   <400> 863
   cgtaggggtg ctgaggttgc ccaggggtcc tgacaacacc agaggatttc atggccatga 60
   gaggagcagg gcctgtgtat aaataccttc tatttttaat acaagctcca ctgaaaacca 120
   ccttcgtttt caaggttctg acaaacacct ggcatgacag aatggaattc gttccccttt 180
   gagagatttt ttattcatgt agacctctta atttatctat ctgtaatata cataaatcgg 240
   tacgccatgg tttgaagacc accttctagt tcaggactcc tgttcttccc agcatggcca 300
   ctattttgat gatggctgat gtgtgtgagt gtgatggccc tgaagggctg taggacggag 360
   gttccctggg ggaagtctgt tctttggtat ggaatttttc tctcttcttt ggtatggaat 420
   ttttcccttc agtgactgag ctgtcctcga taggccatgc aagggcttcc tgagagttca 480
   ggaaagttct cttgtgcaac agcaa 505
<210> 864
   <211> 554
   <212> DNA
   <213> Homo sapiens
   <400> 864
   gagacagcaa cagccgtagc aaaagcagct gctgctcctg ctatgagggt gtatatattt 60
   tttacccaaa gctctggaat tgtacattta ttttttaaaa ctcaaagagg gaaagagcct 120
   tgtatcatat gtgaacattg tatcataggt aatgttgtac agaccctttt atacagtgat 180
   ctgtcttgtt cctgcagcaa aaatcctcta tggacatagg aggtgctgtg tcccatgcct 240
   tcttgccctg acagtgtccc atgggccccc ttctgctccc tgccccctcc ctgctactgc 300
   tgatgcactg tcctctccct gcagcccctg gcttcccagc cttcctcctg accccttcca 360
   acagccttgg aactccagct gccaccaccc tctgggtcgg acactgggac ccactggccc 420
   agtcttggct gctgcttacc cctagccttg atgcctgccc agggaccccc agccccctcc 480
   cgttgccctg cagctttaac agagtgaacc atgtgtattg tacaggcgcg gttgtcattg 540
   cagaaaccgc tggg 554
<210> 865
   <211> 498
   <212> DNA
   <213> Homo sapiens
   <400> 865
   cttcctgcag cacgtggtgc tggcggcctg cgccctcctc tgcattctca gcattatgct 60
   gctgccggag accaagcgca agctcctgcc cgaggtgctc cgggacgggg agctgtgtcg 120
   ccggccttcc ctgctgcggc agccaccccc tacccgctgt gaccacgtcc cgctgcttgc 180
   cacccccaac cctgccctct gagcggcctc tgagtaccct ggcgggaggc tggcccacac 240
   agaaaggtgg caagaagatc gggaagactg agtagggaag gcagggctgc ccagaagtct 300
   cagaggcacc tcacgccagc catcgcggag agctcagagg gccgtcccca ccctgcctcc 360
   tccctgctgc tttgcattca cttccttggc cagagtcagg ggacagggag ggagctccac 420
   actgtaacca ctgggtctgg gctccatcct gcgcccaaag acatccaccc agacctcatt 480
   atttcttgct ctatcatt 498
<210> 866
   <211> 461
   <212> DNA
   <213> Homo sapiens
   <400> 866
   tgtcctcatc tctgcaaagt tcagcttcct tccccaggtc tctgtgcact ctgtcttgga 60
   tgctctgggg agctcatggg tggaggagtc tccaccagag ggaggctcag gggactggtt 120
   gggccaggga tgaatatttg agggataaaa attgtgtaag agccaaagaa ttggtagtag 180
   ggggagaaca gagaggagct gggctatggg aaatgatttg aataatggag ctgggaatat 240
   ggctggatat ctggtactaa aaaagggtct ttaagaacct acttcctaat ctcttcccca 300
   atccaaacca tagctgtctg tccagtgctc tcttcctgcc tccagctctg ccccaggctc 360
   ctcctagact ctgtccctgg gctagggcag gggaggaggg agagcagggt tgggggagag 420
   gctgaggaga gtgtgacatg tggggagagg accagctggg t 461
<210> 867
   <211> 398
   <212> DNA
   <213> Homo sapiens
   <400> 867
   aaaccggagg tatcttcaaa ggcatggaga cctggttcca gtaaatgtcc caccagtggg 60
   gtatagaaag catgctcatg accctgccgt gtcgtctgag gtacccgttc ttatcctagt 120
   ggttcaggaa gagaaaacgc agtttgcact ttcaagacag cttctctaag gctggcatgt 180
   tatctccttg ctttgctttt tgccgtttta aaatgtgtaa ttgttccagc attccaatgg 240
   tcttgtgcat agcaggggac tgtaaccaaa aataaacatg tatttgtgta attggtttga 300
   agaagtcttg aatagctctt tactgtctta cttggggttg ataagatttg agtgtttgca 360
   attttttact aaatgtagct ccaagtctta aatggctt 398
<210> 868
   <211> 489
   <212> DNA
   <213> Homo sapiens
   <400> 868
   gaatttctgc tggactttat ctgggcagag gaaggatgga atgaaggtag aaaaggcaga 60
   attacagctg agcggggaca acaaagagtt cttctctggg aaaagttttg tcttagagca 120
   aggatggaaa atggggacaa caaaggaaaa gcaaagtgtg acccttgggt ttggacagcc 180
   cagaggccca gctccccagt ataagccata caggccaggg acccacagga gagtggatta 240
   gagcacaagt ctggcctcac tgagtggaca agagctgatg ggcctcatca gggtgacatt 300
   caccccaggg cagcctgacc actcttggcc cctcaggcat tatcccattt ggaatgtgaa 360
   tgtggtggca aagtgggcag aggaccccac ctgggaacct ttttccctca gttagtgggg 420
   agactagcac ctaggtaccc acatgggtat ttatatctga accagacaga cgcttgaatc 480
   aggcactat 489
<210> 869
   <211> 495
   <212> DNA
   <213> Homo sapiens
   <400> 869
   gtatttcatt ctcgtatggt gctagagtta gattaatctg cattttaaaa aactgaattg 60
   gaatagaatt ggtaagttgc aaagactttt tgaaaataat taaattatca tatcttccat 120
   tcctgttatt ggagatgaaa ataaaaagca acttatgaaa gtagacattc agatccagcc 180
   attactaacc tattcctttt ttggggaaat ctgagcctag ctcagaaaaa cataaagcac 240
   cttgaaaaag acttggcagc ttcctgataa agcgtgctgt gctgtgcagt aggaacacat 300
   cctatttatt gtgatgttgt ggttttatta tcttaaactc tgttccatac acttgtataa 360
   atacatggat atttttatgt acagaagtat gtctcttaac cagttcactt attgtactct 420
   ggcaatttaa aagaaaatca gtaaaatatt ttgcttgtaa aatgcttaat atcgtgccta 480
   ggttatgtgg tgact 495
<210> 870
   <211> 517
   <212> DNA
   <213> Homo sapiens
   <400> 870
   catagctccc catagtcagg tgtaccagcc agccaaacca acaccacttc ctagaaaaag 60
   atcagaagct agtcctcatg aaaacacaaa tcataaatcc ccccacaaaa attccatatc 120
   tctgaaagag caagaagaaa gcttaggcag ccctgtccac cattccccat ttgatgctca 180
   gacaactgga gatgggactg aggatccatc cttaacagct ttaaggatga gaatggcaaa 240
   gctgggaaaa aaggtgatct aagagttgta ccacctatat aaacatcctt tgaagaagaa 300
   actaagaagc atttgcaaat ttctcttctg gatattttgt ttattttttt cttaagtcca 360
   aaaattatca ttacagtgta ccatattaag ccatgtgaat aagtagtagt cattatttgt 420
   gaaaaattcc caaaagctgg ggaaaacaat gtgtaacttt tccagttact tgacacgatt 480
   cagtggggga aaaccagcat tttttattct attgata 517
<210> 871
   <211> 519
   <212> DNA
   <213> Homo sapiens
   <400> 871
   tgtctacaca cgttgcaggg gcatactaat agagtctatt cattacagtt tgatggtatc 60
   catgtggtga gtggatctct tgatacatca atccgtgttt gggatgtgga gacagggaat 120
   tgcattcaca cgttaacagg gcaccagtcg ttaacaagtg gaatggaact caaagacaat 180
   attcttgtct ctgggaatgc agattctaca gttaaaatct gggatatcaa aacaggacag 240
   tgtttacaaa cattgcaagg tcccaacaag catcagagtg ctgtgacctg tttacagttc 300
   aacaagaact ttgtaattac cagctcagat gatggaactg taaaactatg ggacttgaaa 360
   acgggtgaat ttattcgaaa cctagtcaca ttggagagtg gggggagtgg gggagttgtg 420
   tggcggatca gagcctcaaa cacaaagctg gtgtgtgcag ttgggagtcg gaatgggact 480
   gaagaaacca agctgctggt gctggacttt gatgtggac 519
<210> 872
   <211> 372
   <212> DNA
   <213> Homo sapiens
   <400> 872
   caccaagacg actgcttcag cttcttctct tatccttact ttctttaata gatatttatt 60
   aaactgtcca gtgaaaaggt gccacaatgc ccagtattgt aaacaacagg tttgcattca 120
   tgaagctttc attcattctg gagtctacta atttacctga atggtgtttg cattctgtga 180
   aatgcctctc cacgttgcat atgtcacact tttgtctgca cataactctt ttttcacaag 240
   aagggtcact gccacaacag cacagtcagc gggtgaatta caggtgcctg ctgcctgcct 300
   acctgggtaa tctgatcttg tctgtatcgc cgtgtgctca tcactgaaga attgcaggcc 360
   actcatgtca gt 372
<210> 873
   <211> 486
   <212> DNA
   <213> Homo sapiens
   <400> 873
   ctggagaagc actgccattc agcctcctgc tccagctgtt cacatgcaga aatgctctct 60
   tcacaggcag agaagcctgt ggctaaagtt tccacatccc attaactcag tgcttttgtc 120
   tttttcatga catggcacat agagaaaata tttttttcta gcacacaaga gcaacctgaa 180
   aggctgctcc tggctagggg actctgtccc gggggaccgt gtcctccccc atgtcctgcc 240
   taggccctca gaggaccagg ggatcatgtc tccaggtaac ccgactgtag cccctgctgg 300
   ctgagctcca gcctgtgccc actgataata gcagggacgg cctttctctt agagcagctg 360
   ataagtttcc ctacctgatg gccccctctg acataaactg cacacctggg gtgatggctt 420
   aaagccagaa agagctgagg gagttaagag ggccaacctt agggcacgtg ggcattatta 480
   aaggtc 486
<210> 874
   <211> 532
   <212> DNA
   <213> Homo sapiens
   <400> 874
   gagacagact tggcaaggga ccccctggtt ctgagccagt agctgccatc tggaaattcc 60
   tcttttagcc tctccttaga ggtgaatgtg aatgaagcct cccaggcacc cgctgaattt 120
   ctgaggcctt gcttaaagct cagaagtggt ttaggcattt ggaaaatctg gttcacatca 180
   taaagaactt gatttgaaat gttttctata gaaacaagtg ctaagtgtac cgtattatac 240
   ttgatgttgg tcatttctca gtcctatttc tcagttctat tattttagaa cctagtcagt 300
   tctttaagat tataactggt cctacattaa aataatgctt ctcgatgtca gattttacct 360
   gtttgctgct gagaacatct ctgcctaatt taccaaagcc agaccttcag ttcaacatgc 420
   ttccttagct tttcatagtt gtctgacatt tccatgaaaa caaaggaacc aactttgttt 480
   taaccaaact ttgtttggtt acagttttca ggggagcgtt tcttccatga ca 532
<210> 875
   <211> 498
   <212> DNA
   <213> Homo sapiens
   <400> 875
   caccaagccg acctcagagt tgttcatctt ccttatggga caaaaccggt tgaccagaaa 60
   atgggcagag agagatgacc tcggaagcat ttccacagat ggtgtcaggg tttcaagaag 120
   tcttagggct tccaggggtc ccctggaagc tttagaatat ttatgggttt ttttttcaaa 180
   tatcaattat atggtagatt gaggattttt tttctgtagc tcaaaggtgg agggagttta 240
   ttagttaacc aaatatcgtt gagaggaatt taaaatactg ttactaccaa agatttttat 300
   taataaaggc ttatattttg gtaacacttc tctatatttt tactcacagg aatgtcactg 360
   ttggacaatt attttaaaag tgtataaaac caagtctcat aaatgatatg agtgatctaa 420
   atttgcagca atgatactaa acaactctct gaaatttctc aagcaccaag agaaacatca 480
   ttttagcaaa ggccagga 498

<210> 876
   <211> 547
   <212> DNA
   <213> Homo sapiens
   <400> 876
   gccatcactc ttttttgtga ggagcctaaa tacattcttc ctggggtcca gagtccccat 60
   tcaaggcagt caagttaaga cactaacttg gccctttcct gatggaaata tttcctccat 120
   agcagaagtt gtgttctgac aagactgaga gagttacatg ttgggaaaaa aaagaacgca 180
   ttaacttagt agaactgaac caggagcatt aagttctgaa attttgaatc atctctgaaa 240
   tgaagcaggt gtctcctgcc ctctcatcaa tccgtctggg tgccagaact caaggttcag 300
   tggacacatc cccctgttag agaccctcat gggctaggac ttttcatcta ggatagattc 360
   aagaccttta cctcagaatt atgtaaactg tgattgtgtt ttagaaaaat tattatttgc 420
   taaaaccatt taagtctttg tatatgtgta aatgatcaca aaaatgtatt ttataaaatg 480
   ttctgtacaa taaagttaca cctcaaagtg tactcttgga atggattctt tcctgtaaag 540
   tcttatc 547
<210> 877
   <211> 342
   <212> DNA
   <213> Homo sapiens
<220>
   <221> misc_feature
   <222> (163)..(163)
   <223> n is a, c, g, or t
   <400> 877
   tgccgtcagc cgaactttgt tatggaggga gcagcctcac acaagcagaa acactcctgt 60
   ggatggtatt gtagcatgta ttgtttattt tagtcaatag accctctcct tataaatggt 120
   gtttagtctt cctgttgcat ttcatgggcc tgggggtttc ctngcagagg atattggagc 180
   ccctttttgt gacattacca attacatctt tgtccacgtt taatactttg ttttggaaaa 240
   tttaaatgct gcagatttgt gtagagttct aataccaaag acagaagtaa atgttttcca 300
   tatactttgt cttgcctgta tgcagccctt gtgtaatatg gt 342
<210> 878
   <211> 400
   <212> DNA
   <213> Homo sapiens
   <400> 878
   tgtttttatt tgtaccgtcc acttgtgcct tactgtatcc tgtgtcatgt ccaatcagtt 60
   gtaaacaatg gcatctttga acagtgtgat gagaatagga atgtggtgtt ttaaagcagt 120
   gttgcatttt aatcagtaat ctacctggtg gatttgtttt taaccaaaaa gatgaattat 180
   caatgatttg taattatatc ggttgatctt ttttgaaaag atgaaccaaa ggatttgact 240
   gctaatattt tattccttac actttttttc tgaataagtc tctcataatg agtgcagtgt 300
   cagactgtgc ctactctgat ggtatgtgcc atttgtaaaa taaaatagag cagaaaaaca 360
   caaaaagaga acactggttc agacattcag tgggcaagta 400
<210> 879
   <211> 509
   <212> DNA
   <213> Homo sapiens
   <400> 879
   gcccctcacc aatgcatatg aagagtatgc ttggggaaga gcttaggaat ggggtgggca 60
   tgggactgct gggtagcagc ctttgagcaa atctgcatct tctcttattt ctgacctttt 120
   tccacgtgcc cagtcctatt tctgccagtt gaaggcatac taatattctt tatactattt 180
   aatcttttgc agaaacctta ctattataac ttgctactct ccagatacca attcttcatg 240
   ccgagagcat cggaaatgtt tttgtgtctt actgatgttt tcatgatcaa cttgtaaatg 300
   taagcagttg acttcataaa aggtatttta actattcttg gagtcctttg ctacccaagc 360
   acctggtttc accatgcgat cactgacttc tctacagtga agactctttc ttaatatagg 420
   atttcgctgt gctcttttga ttaaaaatat ctaaccttaa aagacgtaaa aatgtatctg 480
   tgaaatctca ctttgttagc gttgctgct 509
<210> 880
   <211> 371
   <212> DNA
   <213> Homo sapiens
   <400> 880
   ttctctgttc tcctggaagt ccagggaaga aggagggccc cagccttaaa tttagtaatc 60
   tgccttagcc ttgggaggtc tgggaagggc tggaaatcac tggggacagg aaaccacttc 120
   cttttgccaa atcagatccc gtccaaagtg cctcccatgc ctaccaccat catcacatcc 180
   cccagcaagc cagccacctg cccagccggg cctgggatgg gccaccacac cactggatat 240
   tcctgggagt cactgctgac accatctctc ccagcagtct tggggtctgg gtgggaaaca 300
   ttggtctcta ccaggatccc tgccccacct ctccccaatt aagtgccttc acacagcact 360
   ggtttaatgt t 371
<210> 881
   <211> 317
   <212> DNA
   <213> Homo sapiens
   <400> 881
   aaatgttgct aagtcctggt atgatggtgt gagcttcctt ggggaagtac ttcttgagtt 60
   atgtaactaa caggatgttt tactacagat ctggatggct attcagataa catggcaaaa 120
   aatgatagca gaagatcatt aaaaacttaa aatatatttt attagaaaac atttatctat 180
   gaatgaatat ttccttgatg ctggtctctg cacacatatg cttggttact tgcatgcatt 240
   cattggttgt tcaataagtg agatgattac agataatact gtattttcct tatatggaaa 300
   accgttatag acccaat 317
<210> 882
   <211> 534
   <212> DNA
   <213> Homo sapiens
   <400> 882
   tatattcatc ttttcagggt aaatttgttt ttctgagttt ctcgtaatgc tcatttttac 60
   atgctgctac tagctttttt ttttaaaaaa agtaaaagtt gctgctttct aaaatattaa 120
   ttgccttata tttgaaagtg ccattgcaat cgtaagtaga ctatgtattt cctataatga 180
   tgtctgatat ttaaatagga aatcagacaa acaatattca gaaagtttaa gcatataaac 240
   tttttatttt taacttgcct agatccctgt attccaaaac ctgctgcatc ataataaata 300
   tatctatata tatttagcat aagacgtgat atttttaatt tcttttttaa aaaattatat 360
   ttgtctctta gagttaaaat tttctttata taatattgtc atatgtcata gttttaatac 420
   aattcacatg atttctatgt ttcttaatga tattttgttg tgtaaaattg atcggattga 480
   ttaaaaaaca aattctctgg aatttgtgcg ttcatgcttt ttcgtattct ttat 534
<210> 883
   <211> 500
   <212> DNA
   <213> Homo sapiens
   <400> 883
   gatgcatgta tcatacgtgc tttaagcaag tcatgtggcc aagcctagca tcatggagcc 60
   agaaagtata gccttgctgt ctgtctacat catgatgtat aaattgatat atctacatga 120
   attatagaaa cttagaagtg atctttattc agtcttataa tttttacatg aagaatctta 180
   ggcctaggag gagaaaatga ttttctttct attacctaac tagattgggg catatttctg 240
   ataaagaccc acctctagtg agattcatct tttttgtttg tgtgactata ttccatagag 300
   aagaaagatg ggatagctca acttcattat ataccaaagc aaaacacatg ccaaatgatg 360
   actacatttt accaacatat ttagacgagt attcttgact agtgtttact atctataccc 420
   ccaaaactac tactatatag acagaatgga aagtatttct atttgtcctt tttttgtttt 480
   ctgttctaat tgtcagggac 500
<210> 884
   <211> 491
   <212> DNA
   <213> Homo sapiens
   <400> 884
   gaggaggaac tgacgcagct acgccacgaa ctggagcggc agaacaatga ataccaagtg 60
   ctgctgggca tcaaaaccca cctggagaag gaaatcacca cgtaccgacg gctcctggag 120
   ggagagagtg aagggacacg ggaagaatca aagtcgagca tgaaagtgtc tgcaactcca 180
   aagatcaagg ccataaccca ggagaccatc aacggaagat tagttctttg tcaagtgaat 240
   gaaatccaaa agcacgcatg agaccaatga aagtttccgc ctgttgtaaa gtctattttc 300
   ccccaaggaa agtccttgca cagacaccag tgagtgagtt ctaaaagata cccttggaat 360
   tatcagactc agaaactttt attttttttt ttctgtaaca gtctcaccag acttctcata 420
   atgctcttaa tatattgcac ttttctaatc aaagtgcgag tttatgaggg taaagctcta 480
   ctttcctact g 491
<210> 885
   <211> 493
   <212> DNA
   <213> Homo sapiens
   <400> 885
   cccccatgtt acctggactg gaacagactg tgaatatagc agaaggttcc aagaactctg 60
   gtgtctgacc tagaagaggc acagttctct ctactggaaa gaaaacgatg tagccgattg 120
   cacaagggtg ccaagggaag acccaggatg gcccatcaaa ggaacctggg ggaggatgca 180
   ggaggctgaa gggatgcacc tggcatttct ctcactgtgc tcttaccgca tcagcaaccc 240
   ccaacttttg ggcctactct gccccccatg cgtgaatacc ctgcttggat gctgtgcttt 300
   tccggtttgt ctctaagccc ctttctccag ggcatgttgg tttccctggc ctctcagtgt 360
   cctaactgga gcccagagtg ccttgttctg agccaggaga cggctgagca ctggccctcc 420
   acacctaagc gtcctttaca ttaacttatt ggtcttgtat aacacctggt gccattgcca 480
   agtggctgtg tcc 493
<210> 886
   <211> 518
   <212> DNA
   <213> Homo sapiens
   <400> 886
   gacaacaatg aagtagcccc tgaacagcat ggagttgctg tgagtttgtt cgttgcagac 60
   ctttgtgttg ggtcctggga atctgagctt tgttccctgt gcatggtgga taattgaaac 120
   caagaggaca tgggatagac cttgtgacag accaattctg tgacccctgt cttctgggtc 180
   acattattca ttgttgattt aaatacagga ctaccaaaca gtacaaatct atcatgagtc 240
   tggtagaaaa gtaaaagtaa aagctgcaca cgttacatac tgtttattgt tctaatgtac 300
   aactaactat ttgcatataa tgtgatttaa tttattgctg ttttgtgtag aaaaggagaa 360
   ctaatgactg tggatataac ccatgttttg tataatatat tttatttctt gtgcgaactg 420
   gtcatttaaa atatctactt catttgatgt ttggatataa atgtgtatgt gtccttgtaa 480
   atgtttctat caagcaagaa tgccacgtac tcagagta 518
<210> 887
   <211> 533
   <212> DNA
   <213> Homo sapiens
   <400> 887
   gctcctggca attagctgga ctccatgacc cacccctggt gcagcataga tccgacgtct 60
   gtctgggcga agggtagggg tgggtagggg cgggaagcct gagtgcaaat gtcatttccc 120
   tctactgcct cttcctgcct ctccccaccc tgcccacatc cacagagggg agagaagggt 180
   catagctaaa tgcaacaaag tctgtatctt gtcccaacct gcttttctgt tctgttagca 240
   tatcataaag taagcctttc tggtgaagga aggttgctat gaaacttttt ttcttggtgg 300
   aaatggccaa gtttaggcac tctgcttttt gccttacact aatgcttaga aagctgtctt 360
   ttcagtggtg ttgcagcccc cagatgtgtg gccaacctct gctgcaaagg aatctcttgc 420
   tgagtccagg ccaccaatca ggcaaatagc ccatacattt gatcgttgta aaccatgaag 480
   tcttttcttg caagacgttt ttcttctgct gtggtatctt gcccttaaaa att 533
<210> 888
   <211> 516
   <212> DNA
   <213> Homo sapiens
   <400> 888
   tggtcacagc gctagtcatt catttttgag aagttgcttc ttttacatca gaaaaccagt 60
   caatcatatg gagacttctt ttgtgatgaa aaagggcttt agaagttaaa tacatgcatg 120
   cacatgaaaa catgcacaac cacagcctca atcttgtatt tagtttgggg aaagagaaga 180
   gaatttcctg tggattattt tttcctcaag tgcacctctc tggctaaccc aactctgcaa 240
   gaaagcactg tgactaaaac atacataacg cctgcataaa tattccatgg tttcagttaa 300
   atttcagttt ttagccttta cacatgaggt caaggagtga cgaaaataca agcaggaaaa 360
   aatgaaatat ctggtttttg ctgaatgctt aatttatttt ttactgtgcc actccaatat 420
   ttatcaaatc caatagcatg aatgcttctc tgtagtaata ctaattttgt gccttttgtc 480
   tgctttctta agaccagttg ttcacacttt gtagat 516
<210> 889
   <211> 529
   <212> DNA
   <213> Homo sapiens
   <400> 889
   cctcccttcc tggagggatg gccagggaag gagaaaacag agaactgaca cctttgaaac 60
   cacagaatgt gttacatgca gactcgctca agggcataag ttattgtgaa cgtttttgcc 120
   aatcactgct caacagccct gctagatttt gtatgatgct gaattattat gcagactaat 180
   tccacccagt tgagacacac catgcttgtt cacttgtatt tattgaaact gtggattctt 240
   gcccgtgctg tcccttgtat ttactttaag cactgatcac ttatcattca ttcggtatgg 300
   ttttccctgt cccttgtaca cattctggta tgaatttgta aaaataacct gctacaaatt 360
   ggttgaatgt ttctgtctgt ggtgcgaacc agcattaacg gatggggcac gtgcccaact 420
   gaggaacagg agaagaaatc accaatttgg gctctcagag ctaagacaca cttattgatt 480
   ctgttgcaca ttttgcactg gtttatggcg attgttttct tggacggat 529
<210> 890
   <211> 490
   <212> DNA
   <213> Homo sapiens
   <400> 890
   tagagaccca tgtcatctta acctaaaggg aaatcttatt gcgttatcat aaaattgatg 60
   atatcttagg gtcagaattg cccttttttt tattttgaat gggaagctct cactaaaaca 120
   atcctgagat ttcttaattt catggttctt taaatattat aaacacagag tcaacataga 180
   atgaaattgt atttgttaaa atacacacat tggaggacaa gagcagatga ctacttttcg 240
   aagtaatgct gctccttcct aaaagtctgt tttcaatcct ggtaatatta ggggcactgc 300
   ggcacctaag aagccttaaa tgagagctaa tccaatttag agagcgatgg tgtcagcatt 360
   tcggtctgca tatctgtgtg tccgtatctg cgtttgtgtg cgtgtacgtg tgcccctgtg 420
   tgtgggccca gttttcaggc atgtagaata agcatggagt catattgagg aggactcact 480
   tcttgaagat 490
<210> 891
   <211> 433
   <212> DNA
   <213> Homo sapiens
<220>
   <221> misc_feature
   <222> (289)..(289)
   <223> n is a, c, g, or t
   <400> 891
   tggggaggtg aacctgtctt catcggactc tccctaccac tacacgaagg tgacctacag 60
   ccaggaggac gtggacaagc tgctgcacct gacacattac aatgtctgca acaaccagga 120
   gcagctgctg gaggctctgc gccaggcagt gcagcggagg cggcagcgca ggccccactg 180
   atggccgggg cccctgccac ccctaactct cattcattcc ctggctgctg agttgcaggt 240
   gggaactgtc atcacgcagt gcttcagagc ctcgggctca ggtggcacng tcccagggtc 300
   caggctgagg gctgggagct cccttgcgcc tcagcagttt gcagtggggt aaggaggcca 360
   agcccatttg tgtaatcacc caaaaccccc cggcctgtgc ctgttttccc ttctgcgcta 420
   ccttgagtag ttg 433
<210> 892
   <211> 399
   <212> DNA
   <213> Homo sapiens
   <400> 892
   gaactatcac aattataact taccaacaag aagggaatgc aggtagttgt ttaggagatg 60
   gtacattttt tatataacat tcacttcctt gtgtatttga tagtcttttc atggtttata 120
   acattttctc ctgtaaagat aggctaattt ctgaaataat aattaaattt atagaaagcc 180
   gagaggaaat tgctagttta ttcctggtag aggaatttct gtatttgaaa attctccaga 240
   aggaataata taaactgtgg actttgggtg ataatgatat gtaggttcgt cagttgttaa 300
   caaatgtatc cctctgttgg gggctattga taatggggaa ggctgtgcat gtgtgggagt 360
   aggaggtgta tgggacatct ctgtaccttc taatcaatt 399
<210> 893
   <211> 356
   <212> DNA
   <213> Homo sapiens
   <400> 893
   aattcttcag tcacgctgct ttaaaatggg acaaaatcta ttaagttgaa ccatatataa 60
   ttgtggatat ttggctgttt ttaatctgac aagcagtaac ttcatatggt ttgccttaat 120
   atatatttgt tttagtcatg aactcataat ccattgatgc tctttcatga gaagagatat 180
   gacccatatt tccttattga tattattggt acaggcagac aaccctggta ggagagatgg 240
   attctggggt catgaccttt cgtgattatc cgcaaatgca aacagtttca gatctaatgg 300
   tttaatttag ggagtaatta tattaatcag agtgttctgt tattctcaat ctttat 356
<210> 894
   <211> 498
   <212> DNA
   <213> Homo sapiens
   <400> 894
   ggctgagcac cagtgagttc tttgcctcta ctctgaccct agacaacctg gggagggacc 60
   ctgtgcccgc aaaccagaca cataggacaa agtttatcta taacctggaa gaccatgagt 120
   ggtgtgaaaa catggagtcc gttttatagt gactaaagga gggctgaact ctgtattagt 180
   aatccaaggg tcattttttt cttaaaaaaa gaaaaaaagg ttccaaaaaa aaccaaaact 240
   cagtacacac acacaggcac agatgcacac acacgcagac agacacaccg actttgtcct 300
   ttttctcagc atcagagcca gacaggattc agaataagga gagaatgaca tcgtgcggca 360
   gggtcctgga ggccactcgc gcggctgggc cacagagtct actttgaagg cacctcatgg 420
   ttttcaggat gctgacagct gcaagcaaca ggcactgcca aattcaggga acagtggtgg 480
   ccagcttgga ggatggac 498
<210> 895
   <211> 453
   <212> DNA
   <213> Homo sapiens
   <400> 895
   aagcttctac tcctgcagta agcacagatc gcactgcctc aataacttgg tattgagcac 60
   gtattttgca aaagctactt ttcctagttt tcagtattac tttcatgttt taaaaatccc 120
   tttaatttct tgcttgaaaa tcccatgaac attaaagagc cagaaatatt ttcctttgtt 180
   atgtacggat atatatatat atatagtctt ccaagataga agtttacttt ttcctcttct 240
   ggttttggaa aatttccaga taagacatgt caccattaat tctcaacgac tgctctattt 300
   tgttgtacgg taatagttat caccttctaa attactatgt aatttactca cttattatgt 360
   ttattgtctt gtatcctttc tctggagtgt aagcacaatg aagacaggaa ttttgtatat 420
   ttttaaccaa tgcaacatac tctcagcacc taa 453
<210> 896
   <211> 465
   <212> DNA
   <213> Homo sapiens
   <400> 896
   atattggtca ttgatcttcg ttcatgaatt agtctacaga aaaaaaatgt tctgtaaaat 60
   tagtctgttg aaaatgtttt ccaaacaatg ttactttgaa aattgagttt atgtttgacc 120
   taaatgggct aaaattacat tagataaact aaaattctgt ccgtgtaact ataaattttg 180
   tgaatgcatt ttcctggtgt ttgaaaaaga agggggggag aattccaggt gccttaatat 240
   aaagtttgaa gcttcatcca ccaaagttaa atagagctat ttaaaaatgc actttatttg 300
   tactctgtgt ggcttttgtt ttagaatttt gttcaaatta tagcagaatt taggcaaaaa 360
   taaaacagac atgtattttt gtttgctgaa tggatgaaac cattgcattc ttgtacactg 420
   atttgaaatg ctgtaaatat gtcccaattt gtattgattc tcttt 465
<210> 897
   <211> 447
   <212> DNA
   <213> Homo sapiens
   <400> 897
   cctgtctggt cacacgagcc agtgtgagtg gaggcagagg agtgaggccc acgggcagcg 60
   cccaggagcc caccttcccc tctggcccag ccaccactgc ctctcagctt caacaggtga 120
   caggctgctt tcgtgacttg atattggtgt catagcattt ggcctacatt aaaagcccca 180
   atttcagggg aaaggacaaa atggagagtg actgaggtgc tgacctcagg gcaaggctgg 240
   tgaaccctgc agcgggccag ctatggtggg aagcctggca tttggggtgc tccttgcaac 300
   gtcttaagca agcgaccccc ctgacatagc aaaaggtggc aacccatgga ggcagaaaga 360
   aggacgccag cctgaccctt atctgaaacg tcctaagcag agttaatcct ggctgctcag 420
   gagaggcgac acatttcaaa tctccac 447
<210> 898
   <211> 468
   <212> DNA
   <213> Homo sapiens
   <400> 898
   aactgtgtat acattcttac tgtttgaaca actattgcct ttaattaaat gtttcatttt 60
   tctccagagt ccccaaagcc acatggcatt attatagtca tttttgagat gcctgtagag 120
   aatgaaagta ttgactccgt tagagggaaa atgggtttct ctgggtgaat tccaacgaag 180
   catacctagg ggtaacagtg aacctacctg ggtttgtttt gttttggtaa ggatttatgt 240
   agtgtctggc tgtaagcaag aatgagtgga ttataaactt gaagatttct ctgttaaagt 300
   cacaaaaatg atcgacaaac aatatttttg tgatgtttat ttaaacgttg tattttataa 360
   catacttcaa ggaagagtat cgaagtaagt tgctttataa attaagacta aattcgtatg 420
   gatgcagaat tcaattaata aaatttgagc ctgttacgta aattgaat 468
<210> 899
   <211> 528
   <212> DNA
   <213> Homo sapiens
   <400> 899
   agtgttgtgt agcttaatcc ttctgaagtc tttttgtcat gtagctatta atctgtggct 60
   atgaaatgat cagaaatgct aagtgagatc aatatttgtt tggaaaaaaa atcttgggaa 120
   acaacccaag ggttttcgct gttgttgttt ttctttttct atttttgttt acttagtcct 180
   ttagctagtg gatttaattt tgttgtgcct gcttcatttt gcaataacaa tgcagtagaa 240
   tttaaaactt ggatgcttaa gaggcctgca tatagataag aatttcaggc aaaactacat 300
   ttattgttaa taacagcttg ttcataggct cttgtatttt atgtaactgt gataaataat 360
   gaaacttagt tatattgagg ttattgtttg tcggtgaagt gttagtcaca gtattttcaa 420
   aagtttgcac atattgttct gtgtaattgt gtaagccata attacagtgt ttaattctct 480
   tttcctatta catcattcat tgaaagtgat cactttacca ttttgaaa 528
<210> 900
   <211> 483
   <212> DNA
   <213> Homo sapiens
   <400> 900
   ttgatgtgtc cgctgtgtat gttagctgaa ctttgatgag caaaatttcc tgagcgaaac 60
   actccaaaga gataggaaaa cttgccgcct cttctttttt gtcccttaat caaactcaaa 120
   taagcttaaa aaaaatccat ggaagatcat ggacatgtga aatgagcatt tttttctttt 180
   cttttttttt tttttttttt aacaaagtct gaactgaaca gaacaagact ttttcctcat 240
   acatctccaa attgtttaaa cttactttat gagtgtttgt ttagaagttc ggaccaacag 300
   aaaaatgcag tcagatgtca tcttggaatt ggtttctaaa agagtaaggc atgtccctgc 360
   ccagaaactt aggaagcatg aaataaatca aatgtttatt ttccttctta tttaaaatca 420
   tgctaatgca acagaaatag agggtttgtg ccaaatgcta tgaacggccc tttcttaaag 480
   aca 483
<210> 901
   <211> 393
   <212> DNA
   <213> Homo sapiens
   <400> 901
   tgccaggggt ggtcccacct aaagatgcta gcccctctcc aggtgggcat aaggagtaac 60
   agatggcaaa accacaaact attttgatgg actgtgctgc agtatcacca gaagacatta 120
   gggggcagta ggcccccaca caaaaccttc aggcttgaat tttaaagggg aggactttct 180
   gccaactttt cttgtatgcc ttgggaaagc cagttgccct gaacccagca gacaccatgg 240
   aatgtccttt gcacgcatta aatggtacag aactgaagcc tcggaagcaa tttggaactc 300
   gatcttctct tccttaaatg aaaagttatt gaccaaatgg actttttaaa agacacagga 360
   cccttaactt tgccccaaag tgaggggctc cac 393
<210> 902
   <211> 563
   <212> DNA
   <213> Homo sapiens
   <400> 902
   tgtttctcac catatgcttt tgttggcatt atgcagtaac cattgtcatc gttggaatga 60
   attatgcttt cattacctgg ttggttaaat ctagacttaa gaggctctgc tcctcagaag 120
   ttggacttct gaaaaatgct gaacgagaac aagaatcaga agaagaaatg tgactttgat 180
   gagcttccag tttttctaga taaacctttt cttttttaca ttgttcttgg ttttgtttct 240
   cgatcttttg tttggagaac agctggctaa ggatgactct aagtgtactg tttgcatttc 300
   caatttggtt aaagtatttg aatttaaata ttttcttttt agctttgaaa atattttggg 360
   tgatactttc attttgcaca tcatgcacat catggtattc aggggctaga gtgatttttt 420
   tccagattat ctaaagttgg atgcccacac tatgaaagaa atatttgttt tatttgcctt 480
   atagatatgc tcaaggttac tgggcttgct actatttgta actccttgac catggaatta 540
   tacttgttta tcttgttgct gca 563
<210> 903
   <211> 471
   <212> DNA
   <213> Homo sapiens
   <400> 903
   aactccctgt ggccgacatg agggcactcc tgacaggcaa ggactgcccc catgtccggg 60
   agaagggctc cgggaagcag aacaaggacc tctatgagtt ggccttctca atcagctatg 120
   accgtgggga ggaggaagcg tacctcaact tcattgcccc ctccaagcgg gagttctacc 180
   tgtggacaga tgggctcagt gccttgctgg gcagtcccat gggcagcgag cagacacggc 240
   tggacctgga gcagctgctg accatggaga ccaagctgcg tctgctggag ctggagaacg 300
   tgcccatccc cgagcggcca ccccctgtgc ccccaccccc caccaacttc aacttctgct 360
   atgactgcag catcgctgaa ccttgacagt gtggctggcc atgggccaca gctgcggcca 420
   ctgcagcagc catgaagggc agtgggtaga ggagtgcagg caccctgacc a 471
<210> 904
   <211> 495
   <212> DNA
   <213> Homo sapiens
   <400> 904
   gcagctctac gacgtgatgg acgcggtccc agcgcggcgc tggaaggagt tcgtgcgcac 60
   gctggggctg cgcgaggcag agatcgaagc cgtggaggtg gagatcggcc gcttccgaga 120
   ccagcagtac gagatgctca agcgctggcg ccagcagcag cccgcgggcc tcggagccgt 180
   ttacgcggcc ctggagcgca tggggctgga cggctgcgtg gaagacttgc gcagccgcct 240
   gcagcgcggc ccgtgacacg gcgcccactt gccacctagg cgctctggtg gcccttgcag 300
   aagccctaag tacggttact tatgcgtgta gacattttat gtcacttatt aagccgctgg 360
   cacggccctg cgtagcagca ccagccggcc ccacccctgc tcgcccctat cgctccagcc 420
   aaggcgaaga agcacgaacg aatgtcgaga gggggtgaag acatttctca acttctcggc 480
   cggagtttgg ctgag 495
<210> 905
   <211> 437
   <212> DNA
   <213> Homo sapiens
   <400> 905
   ctacaaccag atgcatcacc ttctaaaact ggtacattaa cctcaatacc agttacaatt 60
   ccagaaaaca cctcacagtc tcaagtaata gacactgagg gtggaaaaaa tgcaagcact 120
   tcagcaacca gccggtctta ttccagtatt attttgccgg tggttattgc tttgattgta 180
   ataacacttt cagtatttgt tctggtgggt ttgtaccgaa tgtgctggaa ggcagatccg 240
   ggcacaccag aaaatggaaa tgatcaacct cagtctgata aagagagcgt gaagcttctt 300
   accgttaaga caatttctca tgagtctggt gagcactctg cacaaggaaa aaccaagaac 360
   tgacagcttg aggaattctc tccacaccta ggcaataatt acgcttaatc ttcagcttct 420
   atgcaccaag cgtggaa 437
<210> 906
   <211> 434
   <212> DNA
   <213> Homo sapiens
   <400> 906
   gtctacctgg ccagtggagt ggtccatgct aagtctaaca ctcctgggag ctcaggaggc 60
   ttctgagctt ctcctgtact gtgcatcgtg agggccagag acaggaatgt aaggattggc 120
   aactgtgtta cctttcaagt ttatctcaat aaccaggtca tcagggaccc attgttctct 180
   tcagaaccct atctgggaga gaaggcgaac cacctccggg tttccatcat gtcaaggtca 240
   caggcatcca tgtgtgcaaa ccatctgccc cagctgcctc cacagactgc tgtctccttg 300
   tcctcctcgg ccctgcccca cttcagggct gctgtgagat ggaattccag gaaagaactt 360
   caggtgtctg gaccctttct atctagataa tatttttaga ttcttctgct ccctagtgac 420
   ctacctgggg gcaa 434
<210> 907
   <211> 551
   <212> DNA
   <213> Homo sapiens
   <400> 907
   gccgccctgt aggctgggga tgggctgctg tgtgaatgtt gacgttcgtt tcatggagaa 60
   aggggaggtg aaagattgaa gagcaggttc ctgtcaatgt tctgagttcg agctggaggt 120
   gtagattgaa tagtctacat ggtctgtgag tgtgtgagat gaacccttcc atcctttgac 180
   acctggttgt atgtgtaggc taagaaggaa ggaccctcct gtcagtgtgc aaagctgtaa 240
   tctcatggac tagaagagag ggggccaagg ggatggacag gagaagtcat gcagaatcta 300
   agcaggaatg cagatagaac acatctaggc tcttttcccc aggagagtga tgatggagca 360
   tatagatctg gctcaaattc agcctccatc acttaccagt caggaaccct ggcgatatca 420
   ctttaacttt ctgaacctca gagtcttcac ctataagacg gggaaaataa taccaccctt 480
   tcaagattgt tgagataaat aagtgatata aaacatgtaa agcttagttc tggccacagt 540
   gtagctactc a 551
<210> 908
   <211> 413
   <212> DNA
   <213> Homo sapiens
   <400> 908
   ccttttccta agcaccagcg gaaggagctg tgccccggga tggagtgagg gtggagggcg 60
   cgtcagccac gggtgggcct tgtgtcgcct cgtatcggcc caggtaggtt gttggcctct 120
   tacttgggct gacctgaccc ccgaaagaga aacagacaac tctgttctca ggattgggga 180
   tggacggctt cggccaagcg ttttagcctc attcactcag gccccactca gcactctgcc 240
   agccaagacc attgatttgg aaaatccggt ccccacccgc taatgagctg ttgacactgt 300
   tgttccttgc tgaattggat tgttgacttg tagttcagag gcgtacaact agttggcgat 360
   tagacttgtt atgtgatgtt accagcctga aatgcgatca ccccgtagga aat 413
<210> 909
   <211> 535
   <212> DNA
   <213> Homo sapiens
   <400> 909
   tatgtagtgt gctttttgtc cctttccttc tatcacccta cattccagca tcttaccttc 60
   atatgcagta aaagaaagaa agaaaaaaaa aggaaaaaaa aaaaaaaacc aatgttttgc 120
   agtttttttc attgccaaaa actaaatggt gctttatatt tagattggaa agaatttcat 180
   atgcaaagca tattaaagag aaagcccgct ttagtcaata cttttttgta aatggcaatg 240
   cagaatattt tgttattggc cttttctatt cctgtaatga aagctgtttg tcgtaacttg 300
   aaattttatc ttttactatg ggagtcacta tttattattg cttatgtgcc ctgttcaaaa 360
   cagaggcact taatttgatc ttttattttt ctttgttttt attttttttt ttatttagat 420
   gaccaaaggt cattacaacc tggcttttta ttgtatttgt ttctggtctt tgttaagttc 480
   tattggaaaa accactgtct gtgttttttt ggcagttgtc tgcattaacc tgttc 535
<210> 910
   <211> 366
   <212> DNA
   <213> Homo sapiens
   <400> 910
   tcgctgtgag taccttcacc agaaattgtc ccacattaaa ggtctcatcc tggagtttga 60
   ggaaaagaac aggggcagct gaagttatca agggaatttt tgagcctctg cttagtgaaa 120
   cacaaaggaa caaagcagct ataaactaaa tagaatgcaa ctatctgctt ttcttatgct 180
   gaccactgga gtccatggtg gcaagtagag agctgctcta ggttcttgag gtttggtttt 240
   cattattaat ttttagggta tgggcactgt gcaaagactc catagctgtg cctaggagtc 300
   taggaaaagt gacagaggct tggctttttt acctttagtt cagccaagtc attttcaagt 360
   cctgag 366
<210> 911
   <211> 532
   <212> DNA
   <213> Homo sapiens
   <400> 911
   gccacttggc attagagggt ctttcatggg gagagaagga gactgaatta ctctaagcaa 60
   aatgtgaaaa gtaaggaaat cagcctttca tcccggtcct aagtaaccgt cagccgaagg 120
   tctcgtggaa cacaggcaaa cccgtgattt tggtgctcct tgtaactcag ccctgcaaag 180
   caaagtccca ttgatttaag ttgtttgcat ttgtactggc aaggcaaaat atttttatta 240
   ccttttctat tacttattgt atgagctttt gttgtttact tggaggtttt gtcttttact 300
   acaagtttgg aactatttat tattgcttgg tatttgtgct ctgtttaaga aacaggcact 360
   tttttttatt atggataaaa tgttgagatg acaggaggtc atttcaatat ggcttagtaa 420
   aatatttatt gttcctttat tctctgtaca agattttggg cctctttttt tccttaatgt 480
   cacaatgttg agttcagcat gtgtctgcca tttcatttgt acgcttgttc aa 532
<210> 912
   <211> 404
   <212> DNA
   <213> Homo sapiens
   <400> 912
   gtatcatgtt ttactacata ggttaatttt ttaagggatg ttgcaaaggg attactagag 60
   aaagacaaaa tgtgaccaaa aaaaagcatg aatatttctt aagtatctca acaacatgtc 120
   aaagctgcat gtgtaggatg tatgctgttt gtacaaacta tttcagaata ttttgtaagc 180
   tataacatat ttattgtgca ttaaaattaa atactttttc cccaaaggca tgcagtcatg 240
   agaattacag aaaatttgca acatataaag tagtttgatc taagaggatt caacaccttt 300
   gttttgttgc tcagtgtgta atgactgaga tttgtaaatc tttgtgaaca ttctgtactg 360
   gttcccaaga gctattcatt ccctgctacc tgatttcagc acaa 404
<210> 913
   <211> 503
   <212> DNA
   <213> Homo sapiens
   <400> 913
   tgttccaggt ggccatagtc agtcaccatg tgtgggctca gggaccccca ggaccaggat 60
   gtgtctcagc ctggagaaat ggtggggggg cagtgtctag ggactagagt gagaagtagg 120
   ggagctactg atttggggca aagtgaaacc tctgcttcag acttcagaaa caaatctcag 180
   aagacaagct gacctgacaa gtactatgtg tgtgcatgtc tgtatgtgtg ttggggcggt 240
   gagtgtaagg atgcagtggg agcatggatg ctggcatctt agaaccctcc ctactcccat 300
   acctcctcct cttctgggct ccccactgtc agacgggctg gcaaatgcct tgcaggaggt 360
   agaggctgga cccatggcaa gccatttaca gaaacccact cggcacccca gtctaacacc 420
   acaactaatt tcacccaagg ttttaagcac gttctttcat cagaccctgg cccaatacct 480
   atgtatgcaa tgctcctcag ccc 503
<210> 914
   <211> 331
   <212> DNA
   <213> Homo sapiens
   <400> 914
   gccagaaaga cacaaacagc cctccgggcc tttacgctgg actctggctt ggcaggctcc 60
   aggcagggtc ctctgggaag ttactctaga aaacgaaggg aggaggagca caagatcctc 120
   agcaacgaac acctgcactt agaaaaagtg gacagcttct gccaaccaca ccctacccat 180
   ggtactgtat gctattaact cctggaaacg ccccgtaaat gcgagttgtt tttgtatttg 240
   tgtgttgaga tgggccttgt ggtttctctg tactcagagc acatttcttg taattactat 300
   tgttattttt attgtcatga ctgcccctga g 331
<210> 915
   <211> 434
   <212> DNA
   <213> Homo sapiens
   <400> 915
   tccagattat ctctcctgga cagcctcgtc cccctacagc acagtgccac cctacagccc 60
   tgggagctca ggccccgcaa ccccaggggt caacatggcc aacagcatcg ccagcctccg 120
   tctcaaggcc aaggagttca gcctgcacca cagccaggtg cctacggtga actgaagtcc 180
   agtcccacca ggacccagac gcctccctgg gtggacagca atagaaaagg gggcagacgc 240
   ccaggaagtg accttctcct ggatgagctc tcctggcccg tctgtccagc ctggactccc 300
   gagcccacga ggctgttgag gcccctgcag ccgggcccag ctcttctgtc cttggccacc 360
   agagactgca gcccacaacc cttggagggg ttgggccgga aggtggaaga gcctgccaag 420
   gacctcattt agtt 434
<210> 916
   <211> 488
   <212> DNA
   <213> Homo sapiens
   <400> 916
   tagactctgg ccttcaccaa tagtctctct gcaagacaga aacctccatc aaacctcaca 60
   tttgtgaact caaacgatgt gcaatacatt tttttctctt tccttgaaaa taaaaagaga 120
   aacaagtatt ttgctatata taaagacaac aaaagaaatc tcctaacaaa agaactaaga 180
   ggcccagccc tcagaaaccc ttcagtgcta cattttgtgg ctttttaatg gaaaccaagc 240
   caatgttata gacgtttgga ctgatttgtg gaaaggaggg gggaagaggg agaaggatca 300
   ttcaaaagtt acccaaaggg cttattgact ctttctattg ttaaacaaat gatttccaca 360
   aacagatcag gaagcactag gttggcagag acactttgtc tagtgtattc tcttcacagt 420
   gccaggaaag agtggtttct gcgtgtgtat atttgtaata tatgatattt ttcatgctcc 480
   actatttt 488
<210> 917
   <211> 381
   <212> DNA
   <213> Homo sapiens
   <400> 917
   gagatgttca tgttgctgag ctgtaagcag gagcaccctg tcttctctgg tctttgactt 60
   gattaaagta tctccgcttt cttgggaggg aataggggat gttttatcag tgaatgtgcc 120
   atacacctta tggtccactt catgtgcctt tcagacttca aagcgcgcgc gcatgtgtgt 180
   gtgtgtgtgt gtgtgtgtgt gtgtgtgtgt gtgcttcttt ttctctccta aaaatcgata 240
   agtagctcca cctgaagagg gatggaacct ctgggtcagg aaacagctgg aatccacact 300
   cacctcattc ccattgtttg gatcatgctt ctttccaaca cgtgttcaca atctccaaag 360
   ggactgtatt tcttctctgt g 381
<210> 918
   <211> 569
   <212> DNA
   <213> Homo sapiens
   <400> 918
   gctggctgac aggatccctg tgttgtaatt ggtccctcct ttcagctctc tagtgagatg 60
   cccgtgtctg tgcgtgtgcg tgtgtgtttc atacagctag cattagatgg gtgatgtttc 120
   ttacttatca tccctaacta ttgcaacttg accttaaaaa gacaaaaccc cacaaaactc 180
   ttcctgccac gggcttgcag attgaagcac tttcgatgtt gggcgctggc gtttgtgttc 240
   tgggcaccac cgtgaccctg cccagatggc tataatatta ttttatacac aaaccttttt 300
   tttcataaat gttataattt tgtgtctgtc tttataaact attataagta ctatttttgt 360
   tataattcaa aatagatatt tagtataaag tttttgctgt taaatatttg ttatttagta 420
   aaatatgaat tttgctctat tgtaaacatg gttcaaaata ttaatatgtt tttatcacag 480
   tcgttttaat attgaaaaag cacttgtgtg ttttgttttg atatgaaact ggtaccgtgt 540
   gagtgttttt gctgtcgtgg ttttaatct 569
<210> 919
   <211> 460
   <212> DNA
   <213> Homo sapiens
   <400> 919
   gtagaccaca attcactttt tagttttctt ttacttaaat cccatctgca gtctcaaatt 60
   taagttctcc cagtagagat tgagtttgag cctgtatatc tattaaaaat ttcaacttcc 120
   cacatatatt tactaagatg attaagactt acattttctg cacaggtctg caaaaacaaa 180
   aattataaac tagtccatcc aagaaccaaa gtttgtataa acaggttgct ataagcttgg 240
   tgaaatgaaa atggaacatt tcaatcaaac atttcctata taacaattat tatatttaca 300
   atttggtttc tgcaatattt ttcttatgtc caccctttta aaaattatta tttgaagtaa 360
   tttatttaca ggaaatgtta atgagatgta ttttcttata gagatatttc ttacagaaag 420
   ctttgtagca gaatatattt gcagctattg actttgtaat 460
<210> 920
   <211> 540
   <212> DNA
   <213> Homo sapiens
   <400> 920
   gaggacaata tccatgactg ctcaaaactt aaaagtcctt tgggggtcaa atggcatacg 60
   gcagtcacct atgtgaacag ctgcttgttt gtggccgtgc tggtgattct gatcggatgt 120
   tacatagcca tatccaggta catccacaaa tccagcaggc aattcataag tcagtcaagc 180
   cgaaagcgaa aacataacca gagcatcagg gttgttgtgg ctgtgttttt tacctgcttt 240
   ctaccatatc acttgtgcag aattcctttt acttttagtc acttagacag gcttttagat 300
   gaatctgcac aaaaaatcct atattactgc aaagaaatta cacttttctt gtctgcgtgt 360
   aatgtttgcc tggatccaat aatttacttt ttcatgtgta ggtcattttc aagaaggctg 420
   ttcaaaaaat caaatatcag aaccaggagt gaaagcatca gatcactgca aagtgtgaga 480
   agatcggaag ttcgcatata ttatgattac actgatgtgt aggcctttta ttgtttgttg 540
<210> 921
   <211> 232
   <212> DNA
   <213> Homo sapiens
   <400> 921
   ttccccacct ttcgtgtaag gtgctactga acatgacagc ttcttgtcat gacaggaaac 60
   ttgcatcagt tggatatcct tttgagaaac tgaattttgc aaagggccaa atttccccaa 120
   actgaacggg ctcaggaaat gttcctttac actcagaaca ttctatttta agtatattat 180
   ttattgttgg cagttcctca gggatttccc ttttctctgt attggtcagt gt 232
<210> 922
   <211> 424
   <212> DNA
   <213> Homo sapiens
   <400> 922
   aaatgactgt cttatcactc ttatttgaca tttcgtaggt gtaagagaaa tggaaatgaa 60
   tggtttcaac aaagatcatt taatacagca gagcatggca tgaccaagca tctttgtaaa 120
   gtgttagatg gaaaatgctg tgtgctgcca tggtaatcag aaataataac ctgttaggga 180
   tgtattctag gaaatcagaa gtagttctct tttcttgctg gattattgct tagataactc 240
   ttgttttctg gtaaaacttt agttgtattg ccatccactc ctttttcaaa tgagtttaat 300
   gccataaagc tgatattctt tgtccgatta atttgaaatc tgcacagaag ctgttttagt 360
   cattaatgtg taacaaaagt agcttataga atatggactg ccttattgct gttgcttatc 420
   attt 424
<210> 923
   <211> 571
   <212> DNA
   <213> Homo sapiens
   <400> 923
   agtctgaagg cgaaagttcc agcaaattaa agcagaagtt ggaagctcat atggaaaaac 60
   tcacagaggt ccatgaagaa ttacagaaga aacaagaact cattgaagat cttcagccag 120
   atataaatca aaatgtacaa aagatcaatg aacttgaagc tgctcttcag aagaaagatg 180
   aagatatgaa agcaatggag gaaagatata aaatgtactt ggagaaagcc agaaatgtaa 240
   taaaaacttt ggatcccaag ttaaatccag catcagctga aataatgcta ctaagaaagc 300
   agttggcaga gaaagagaga agaattgaga ttctggagag tgaatgcaaa gtagcaaaat 360
   tccgtgatta tgaagaaaaa ctcattgttt ctgcgtggta taataagagt ctagcattcc 420
   agaaactggg gatggaatct agacttgtga gcggcggtgg tgcctgcagt gacactggtg 480
   cgtgcactcc tgcgcggtct ttcttagcgc agcaacggca catcaccaac accagaagaa 540
   atctctctgt taaagtccct gctacaacat c 571
<210> 924
   <211> 385
   <212> DNA
   <213> Homo sapiens
   <400> 924
   aaaacacctg aatgactcta agactgatat gtattttcaa gtctaagctg tcttacagaa 60
   gatcttttat aaatgtttcc ttataaatat ctcaccatta caacaaattg ttttaactgt 120
   ttttctatta gctctagctg catatttgat gtaaatgaca attactgaaa aaatgtcaga 180
   aaaaacattt tcagtactaa cattaaagtg ccatatgtaa aaaagaaaaa tgtgatttgt 240
   ataactaaat aacacacaaa catcaagagg ctatttatac aaataattta tttccactag 300
   ggaaagtgca ttactggtga aggtattatc aatttattct acttgcttat aatgttacag 360
   tgaatgttct ggcttactct gcctc 385
<210> 925
   <211> 386
   <212> DNA
   <213> Homo sapiens
   <400> 925
   cctcaaccca gagagctttg caatcagctt gacctgtggg gactcagaag accctcctgc 60
   cgatgtggca atcgaactca aagctgtgtt cacagatcgg cagctactca gaaattcttg 120
   tatatctggg gagaggggtg aagaacagtc agcaatccct tactttccat tcattccaga 180
   ccagccattc agggtggaaa ttctttgtga gtacccacgt ttccgagtgt ttgtggatgg 240
   acaccaactt tttgattttt accatcgcat tcaaacgtta tctgcaattg acaccataaa 300
   gataaatgga gacctccaga tcaccaagct tggctgattt aaaccacctc tatttcaaat 360
   aggatcacgt gccacaacta tctgac 386
<210> 926
   <211> 480
   <212> DNA
   <213> Homo sapiens
   <400> 926
   ctggacccgt gaagtcttca gctcctgcag ctctgaagtg gttctgaaca caccacagcc 60
   atcagcactg gaatgcaaag accagaacaa acagaaggaa gccagcagcc aagccggggc 120
   agtttcagtc tccaccccaa atgcaggact gtagaagcgg ccaggaagaa aaccaccccc 180
   tcttaaggtt gtttttgtga ccgttctttg gagcattgtt ctaaaaatgg gaaattacat 240
   attgctgtgc caagggcaac aaacacctgc agttaaagga ataccttccg cgaggcggct 300
   tttcggagca tgcatgttta tagctccagc caggccagac cgagggctgc tgcataagcc 360
   ctgcttggtg catttcttca cttgcaaggg gacagagtgt gggcttaggt ttgggactag 420
   agggggcttt ggcaactatg gtgctcaggt gattatcctt cgctcgttta tccaataaac 480
<210> 927
   <211> 514
   <212> DNA
   <213> Homo sapiens
   <400> 927
   aaccagaaca acctgcactt ctgccaaggc cagggccagc aggacggcag gactctaggg 60
   aggggtgtgg cctgcagctc attcccagcc agggcaactg cctgacgttg cacgatttca 120
   gcttcattcc tctgatagaa caaagcgaaa tgcaggtcca ccagggaggg agacacacaa 180
   gccttttctg caggcaggag tttcagaccc tatcctgaga atggggtttg aaaggaaggt 240
   gagggctgtg gcccctggac gggtacaata acacactgta ctgatgtcac aactttgcaa 300
   gctctgcctt gggttcagcc catctgggct caaattccag cctcaccact cacaagctgt 360
   gtgacttcaa acaaatgaaa tcagtgccca gaacctcggt ttcctcatct gtaatgtggg 420
   gatcataaca cctacctcat ggagttgtgg tgaagatgaa atgaagtcat gtctttaaag 480
   tgcttaatag tgcctggtac atgggcagtg ccca 514
<210> 928
   <211> 554
   <212> DNA
   <213> Homo sapiens
   <400> 928
   aaggggacac gtgacagccg tttgttcccc aagacattct aggtttgcaa gaaaaatatg 60
   accacactcc agctgggatc acatgtggac ttttatttcc agtgaaatca gttactcttc 120
   agttaagcct ttggaaacag ctcgacttta aaaagctcca aatgcagctt taaaaaatta 180
   atctgggcca gaatttcaaa cggcctcact aggcttctgg ttgatgcctg tgaactgaac 240
   tctgacaaca gacttctgaa atagacccac aagaggcagt tccatttcat ttgtgccaga 300
   atgctttagg atgtacagtt atggattgaa agtttacagg aaaaaaaatt aggccgttcc 360
   ttcaaagcaa atgtcttcct ggattattca aaatgatgta tgttgaagcc tttgtaaatt 420
   gtcagatgct gtgcaaatgt tattatttta aacattatga tgtgtgaaaa ctggttaata 480
   tttataggtc actttgtttt actgtcttaa gtttatactc ttatagacaa catggccgtg 540
   aactttatgc tgta 554
<210> 929
   <211> 547
   <212> DNA
   <213> Homo sapiens
   <400> 929
   gaacgtcgta tgagatccta caatggaaga ataaaatcac ctcattcttc atttcagatc 60
   tgaacattag cagtgatcta gatttttttt tttttaaaca aaattaagtg tgcttagagt 120
   catccctcta catgggctgt ggctgtcagc ccataggttt gtcagtttca catcaaaact 180
   gtgggtataa actgttgaaa ccaatcacat taaaatattt agctgggcac agtggtgtgc 240
   atctgtagtc ccagctactt gggaggctga ggcaggagga tcgcttaagc acaggagttg 300
   gaatccagcc tgagcaacag agcaaaaccc cgtctctaaa atacaaataa aatatttgtg 360
   tagtttttga ttaaaattga ctacagcggt cagtataaaa tacatgtcgc ttttaaggaa 420
   gtgctcttta tgtatctaac agatggaagt ttttgcattg gtaagagcat ttatatatgc 480
   tttgtttcag ggtttatgga tttgtattca tatattgtca aataggtttc atactctaat 540
   tttactt 547
<210> 930
   <211> 402
   <212> DNA
   <213> Homo sapiens
   <400> 930
   gatgagatgg ttgttgccct agtctgttgg tagaaccaga aatcaatatg ttgtctttta 60
   ggttaaagct tgtaccaaaa tatttatttc ccccatttca agccctgagt caaacatttt 120
   tttctcttaa taatagacct gaaatgtttt attagtattt ctgtgaaatc agttgattct 180
   tgtgccattt ttgtatatgt aattgtaatt ttgcccatgt taggccctct aaaaaatgtt 240
   tgacatcctt tgagatattt tattactaaa atctgatctt ttttggctac tgcaaaaatc 300
   tattcagcaa gaaggtatca gctgcatacc ttgcacagtg gagctgacta cctataaact 360
   ctccctaagg catttgttta caggtgtatt ccattttagc ag 402
<210> 931
   <211> 452
   <212> DNA
   <213> Homo sapiens
   <400> 931
   cgccgactct tttcactgag tttccagagg aagactagcg cggccaccgc gaagccgcca 60
   acccaccgga gagggggctt ctgaacttgg actcctggga acatggacaa gcccggcgct 120
   gccacgccgg ggcctccacc gcctgggcct gagcctgacc gggccattcc caaatttggg 180
   acgcggaagg agaggctctc ggagcagaag aggccagata ccctgaagca taaagtttaa 240
   cgtcaaaagt ttaacatgga gaaggcggtt ccgttctgaa gcgtggtctg ctgtcccctg 300
   ggcgtgaggc ctcctgggcc tgtcgggcct ccgatttcat cctcagacgt aatgctcacc 360
   aacagcactt gcactgagtt gactcttgca cactcgactc cataatatga tgctttttaa 420
   gatgtatgtt cacaccaata attgcctgct tc 452
<210> 932
   <211> 496
   <212> DNA
   <213> Homo sapiens
   <400> 932
   tgacaggacc aggatgtccc tcatctttgc caaccagaca gaggaggata tcttggtcag 60
   aaaagagctt gaagaaattg ccaggactca cccagaccag ttcgacctgt ggtacaccct 120
   ggacaggcct cccattggct ggaagtacag ctcaggcttc gttactgccg acatgatcaa 180
   ggagcacctt cctcctccag cgaagtccac gctcatcctg gtgtgtggcc cgccaacact 240
   gatccagacg gcggctcacc ctaacctgga gaagctgggt tatacccagg acatgatttt 300
   cacctactaa caaacacctc catgtgctca gcaaatttgc atgtcccttt tcatctgttt 360
   cagagtaagt tcaatttcac cacggtaaac tgggatgttt tcaaaagtgc cttgccatgt 420
   accttcgcgc acacactggt tctcctcttt tgggtgtggg cctaacaaaa agggctcaag 480
   gggctggaga ctggct 496
<210> 933
   <211> 487
   <212> DNA
   <213> Homo sapiens
   <400> 933
   ggcccacctc agctgtagtg gtacctgcca cggggccagc cccccacagc gcaggggctg 60
   gtctgctgcg gatctcagtg aaggaggtgg tgcggaggca agaggctggg ctaggtgagc 120
   ctagcttggt ggccctggtg gtgtttgggg ccctcactgc tgccctggtt ctggctactg 180
   tgttgctgac cctgagggcc tggcgccggg gtgtctgccc ccctggaccc tgttgctacc 240
   ctgccccaca ctatgctcca gcgtgccagg accaggagtg tcaggttagc atgctgccag 300
   cagggctccc cctgccacgt gacttgcccc ctgagcctgg aaagaccaca gcactgtgat 360
   ggaggtgggg gctttctggc ccccttcctc acctcttcca cccctcagac tggagtggtc 420
   cgttctcacc acccttcagc ttgggtacac acacagagga gacctcagcc tcacaccaga 480
   aatatta 487
<210> 934
   <211> 321
   <212> DNA
   <213> Homo sapiens
   <400> 934
   tccattacca agagctcatg ccacccggtt cctgcatgcc agaggagccc aagccaaaga 60
   ggggaagacg atcgtggccc cggaaaagga ccgccaccca cacttgtgat tacgcgggct 120
   gcggcaaaac ctacacaaag agttcccatc tcaaggcaca cctgcgaacc cacacaggtg 180
   agaaacctta ccactgtgac tgggacggct gtggatggaa attcgcccgc tcagatgaac 240
   tgaccaggca ctaccgtaaa cacacggggc accgcccgtt ccagtgccaa aaatgcgacc 300
   gagcattttc caggtcggac c 321
<210> 935
   <211> 194
   <212> DNA
   <213> Homo sapiens
   <400> 935
   gcatcagtga atcgggccac atctgcagcc agatgttcga aggccagatc ctggacgtga 60
   agggaggccg gggctacgac cgggaccacg tggtgctatg ggagccggat gaggacaggg 120
   catcccagat ctggactatc cacgtgcttt gaaacttttc ccctcaccct ccagccctgg 180
   aggcttttgc tggg 194
<210> 936
   <211> 415
   <212> DNA
   <213> Homo sapiens
   <400> 936
   aaagactgga acccacgttc tcagctctca ccaagtggac tttttgcggg gtgtggcggc 60
   cgggtctcga ccacagcgtg gatcaccggc tgtttaggaa actgcagctg cacaacgtgg 120
   ggtgcaaaac tgccccgctt cctttacagc tcttctcaac cctcacctcc atcccccgtc 180
   acccaggcac cttcgcttcc agatgctgcc aggctgtcac tcaattcggt catttcattc 240
   atttatcaca catgggcact ggggttgggc taacagcaag agacaatagg cctttgttcc 300
   tatttattgg gtactgctta cgtgctaagc agatcagttt atttaatgct tgcaacgact 360
   ctctgaggta gaaaatattg ttaattccgt tcaggatccc ggctacataa tctgt 415
<210> 937
   <211> 523
   <212> DNA
   <213> Homo sapiens
   <400> 937
   agctcacgat gggcagtgtt ctccatacta ttattagctc tcatgtcccc cctgggccta 60
   gccgtagggc tggctgtgac tggaggggac tctgaaggag ggcggggctt agcccaggct 120
   gtgttagagg gtgtggcagc tggtaccttc ctgtatgtca ccttcctaga aattcttcca 180
   cgggagctag ctagtcctga ggcccctcta gctaagtgga gctgtgtagc cgctggtttt 240
   gccttcatgg cctttattgc cttgtgggcc tgagagattc ctggcttttc tgatggacct 300
   atttaggaca acctctctat ccccagggag acctcccaaa tggctttgac cctcagacat 360
   ttctttactc agactaaata gcattcagta ggactggact ggaccccagg tttcctttac 420
   atgagatccc atttctcacc ctggactaag acaaagatat ttaggttgag cagctattaa 480
   ttggagaatt ggtacagaga cgctccagat tttattctta tcc 523
<210> 938
   <211> 511
   <212> DNA
   <213> Homo sapiens
   <400> 938
   aaggaaactc atctccgagg ttgacagcga cggcgacggc gaaatcagct tccaggagtt 60
   cctgacggcg gcaaggaagg ccagggccgg cctggaggac ctgcaggtcg ccttccgcgc 120
   cttcgaccag gatggcgacg gccacatcac cgtggacgag ctcaggcggg ccatggcggg 180
   gctggggcag ccgctgccgc aggaggagct ggacgccatg atccgcgagg ccgacgtgga 240
   ccaggacggg cgggtgaact acgaggagtt cgcgaggatg ctcgcccagg agtgaggctc 300
   cccgcctgtg tccccctggc tgcgctctga gccttcaggg ccaccgcccg ctgctgcttt 360
   tgtgctggga ctctccgggg aaacctggtc ggtggatggg aaactgcctc cccctgggag 420
   gaaggctttg cgctccgggg cctggatgcg gcgccctcgg gccgcctgcg agcccctctc 480
   tgccttcaga ccttgggcag aaggaggcct c 511
<210> 939
   <211> 389
   <212> DNA
   <213> Homo sapiens
   <400> 939
   ctagaatttc catgtctctg cttagctgtg ctggcagcta gcagctggct gtgtttgcag 60
   tgcaaatagc tctgttcttg gaaatcctgc tcatggtatg tccccagtgg tttcttcatc 120
   cacatcatct aaagcctgaa cccgttcttc tctggttcaa gtcagtggct gacacggact 180
   tgtatctcct tcagagctcg gctggcaccc agcctccctt ctccttccac tcccttagta 240
   cactggagtg ccgagccctg ccttccaccc agcgtccatc cagcccctgt cctcacctct 300
   ccggcacctc ctcctccttc tgcatttcct atcttcctgt gtcttgtgca tgggaagcag 360
   ccttcagtgc cttcatgaat tcaccttcc 389
<210> 940
   <211> 466
   <212> DNA
   <213> Homo sapiens
   <400> 940
   gcatgtgttt ggtatcttca acagtagacc aagaatctaa catcactctc agtaatatag 60
   agaccggaat acatggttta taggaaatga tcaaatgatc caaaaaaact ccacattttt 120
   taagaagttg gaatttgatt tcatgcataa ctgtattaaa acattaaata gaaataatgt 180
   catttgaatg aaaatcttat cacattaaat tcactgtgaa ggcagcatac ttaaaggaat 240
   ttgatttcat gcataactgt attaaaacat taaatagaaa taatgtcatt tgaatgaaaa 300
   tcttatcaca ttaaattcac tgtgaaggca gcatacttaa atttttattt tgaaaagtct 360
   aaaaggctta gatttttaaa atttaataat tatttctaca aattttctat ttttcttgag 420
   gtgattctca actagcaatt ggaactccta ggctctatta acataa 466
<210> 941
   <211> 505
   <212> DNA
   <213> Homo sapiens
   <400> 941
   ttcctgttac tttcacctca ggtcgtaact ttctttatgt gtttcattac agctccaaaa 60
   agccttccag aatttcctga ggcaaaaaca cccttccctt ttgagaaacc taggggcaca 120
   ttgggtaata agagtacctt aaatttaata ttaaggctgt gggtggtgat tgcttaattc 180
   tgcaggacac atttactgca tcttatttct ggaaacctca tgaactgata gttaggcaaa 240
   caaatggttg atttgatttt tttttaataa atctatttgg attttctgca aattcggtaa 300
   aacccatcag tcttaattcc acataatcca cttagctttt tgtccttaaa aatgctgaca 360
   gtctgacacc aaactctggt ctctctctga ccactaatca aatgttctct ggatggatac 420
   atactgattt cttactgata tataatgact ttttattgta ttggtatact gcaggcttct 480
   ggtagccact taaccatacc agcaa 505
<210> 942
   <211> 545
   <212> DNA
   <213> Homo sapiens
   <400> 942
   aactgatggc tggcatctga tatgcagagt tagtcaacag acactggcat caattacaaa 60
   atcactgctg tttctgtgat tcaagctgtc aacacaataa aatcgaaatt cattgattcc 120
   atctctggtc cagatgttaa acgtttataa aaccggaaat gtcctaacaa ctctgtaatg 180
   gcaaattaaa ttgtgtgtct tttttgtttt gtctttctac ctgatgtgta ttcaagcgct 240
   ataacacgta tttccttgac aaaaatagtg acagtgaatt cacactaata aatgttcata 300
   ggttaaagtc tgcactgaca ttttctcatc aatcactggt atgtaagtta tcagtgactg 360
   acagctaggt ggactgcccc taggacttct gtttcaccag agcaggaatc aagtggtgag 420
   gcactgaatc gctgtacagg ctgaagacct ccttattaga gttgaacttc aaagtaactt 480
   gttttaaaaa atgtgaatta ctgtaaaata atctattttg gattcatgtg ttttccaggt 540
   ggata 545
<210> 943
   <211> 414
   <212> DNA
   <213> Homo sapiens
   <400> 943
   gggctgatca ggttgggtta tgcaagaatc tcccatgctg aactgagtga ttcagaaatt 60
   cagatggcaa aatttaggat ccctgatgac cccactaatt atagagacaa ccagaaagtg 120
   gtcatagacc acagagaagt ttctgagaaa attcatttta atcccagatt tggatcctac 180
   aaagaaggac acaattatga aaacaaccat aattttcata tgaatactcc caaatacttt 240
   ttatgaaaca tttaaaacaa gaagttattg gctgggaaaa tctaagaaaa aaagtatgta 300
   agataaaaag aagagattaa tgaaagtggg aaaatacaca tgaagaacct caacttaaaa 360
   aacacatggt atctatgcag tgggaaatta cctccatttg taaactatgt tgct 414
<210> 944
   <211> 163
   <212> DNA
   <213> Homo sapiens
   <400> 944
   gaaaagtagc tctaatcaag tgatatttct gggatatatc acttcagcac ctggctccag 60
   agattatcta cagctcactg aacatggcaa tgtgaaggat atcgacagca ctgatcatga 120
   cagatggtgt gaatacatta tgtatcgagg gctgatcagg ttg 163
<210> 945
   <211> 553
   <212> DNA
   <213> Homo sapiens
   <400> 945
   atttctccgg aagctgagcc agtctcctgg tctagcccag gttgccagaa cgcttggcat 60
   tgcagagtgc tagagccagt ggagaacttg ccaacttgat tgttttacag cagaggaaag 120
   aggatcacag agggaaaatg attcacccaa agtcacacag caagttcatg gctgagctga 180
   gaccaggatt aagcttcctg actcccagtt caccatgaaa agggttctgg caacaggttc 240
   aagctggaga atccttcaaa atgctacacc cacattctct ccaactcttc atctccctga 300
   tcttccagac aaactacctg gatgttgccc ttaaaccatt tctagctgtt aaccctatcc 360
   agaaaaatga ttgagtgata gctgagaagt ggaaagtgtg ggatttttgg caggtgctct   420
   ctttcctccg ccccccgcgc catcctttct cttcctcctc tctgtaatgg tatgtccagc   480
   ctcactctcc ctccctggtg ctgtatgcgt tccccctgtt agctacattt gtgatcacat 540
   acccttcttt taa 553
<210> 946
   <211> 560
   <212> DNA
   <213> Homo sapiens
   <400> 946
   gagtgcagta gcacgatctc ggctctcacc gcaacctccg tctcctgggt tcaagcgatt 60
   ctcctgcctc agcctcctaa gtatctggga ttacaggcat gtgccaccac acctgggtga 120
   tttttgtatt tttagtagag acggggtttc accatgttgg tcaggctggt ctcaaactcc 180
   tgacctagtg atccaccctc ctcggcctcc caaagtgctg ggattacagg catgagccac 240
   cacagctggc ccccttctgt tttatgtttg gtttttgaga aggaatgaag tgggaaccaa 300
   attaggtaat tttgggtaat ctgtctctaa aatattagct aaaaacaaag ctctatgtaa 360
   agtaataaag tataattgcc atataaattt caaaattcaa ctggctttta tgcaaagaaa 420
   caggttagga cacctaggtt ccaattcatt cacattcttg gttccagata aaatcaactg 480
   tttatatcaa tttctaatgg atttgctttt ctttttatat ggattccttt aaaacttatt 540
   ccagatgtag ttccttccaa 560
<210> 947
   <211> 288
   <212> DNA
   <213> Homo sapiens
   <400> 947
   ggctgaaagg attttacatt tattcaaagt caaaagggaa aagaaatcca agaactacag 60
   aagagcagtt gaagtgattt atgcttgatt tctaaatgca acttatgttt atacataatt 120
   taaaactcaa agaaagcatg cttatacaat catgtgcaac tttaaacttt aagaactctg 180
   gatgaataca tggtggcaac agtccatgac acctgaaaac atcatttgtg gagtggcgta 240
   gagttcagtg ttcgcagtcg catattacaa ccatgtttca cacagccc 288
<210> 948
   <211> 513
   <212> DNA
   <213> Homo sapiens
   <400> 948
   tttttatctc cacacgcagt atgaagataa aattacatag tattacctag acatagacag 60
   tattacctag gtagatgcac tgctcacctg cacccttccc agctctcatt tttgttaggt 120
   gatttgggat agggatagtg ttttggggta tggggggagt gtttctgacc tgctttgcag 180
   acgtgcctcc gcacctcagc agtttggggt gtggccccag ggcggttctt ggatgtaaaa 240
   gatgtggcca tctagcctcg taacttcact gtcacctgtg tcccataggg tgccttctga 300
   atactgttat tagaataagt ttgttgcaga acgtgaccct gcgtgcaaac atgtaccgtg 360
   gcctggtata tgatagagat tgatattaat gtaccatgta tgttaatgtg aatctgtggg 420
   caggatactt ttccatggca ggaaatatcc aagctgttga aactggctat gttttaatat 480
   gcctcattgt gcctttactg ttgtgtggac tgc 513
<210> 949
   <211> 284
   <212> DNA
   <213> Homo sapiens
   <400> 949
   ctttatcatc cccacaaaca ttttgaaact ggaatatttg tcttcagaaa atggaaacaa 60
   gactataaat gataagccct gtccctagca ccacctctcc tgtgtgtgga atagaggccc 120
   ctcgtgctac caacacttac cctgtgttta aaaagatctt gtaccaagcc aacggcgttc 180
   ctggctctcc tgcccacagg atgaacattt tcggcttcct taggagtttt gccctaccgt 240
   attccaaagc gtgtgctggt ttctcatatt gtctgtaggc tcac 284
<210> 950
   <211> 511
   <212> DNA
   <213> Homo sapiens
   <400> 950
   gggacttaac atttcacgtt gtatcttact tgcagtgaat gcaagggtta cttttctctg 60
   gggacctccc ccatcaccca ggttcctact ctgggctccc gattcccatg gctcccaaac 120
   catgccgcat ggtttggtta atgaaaccca gtagctaacc ccactgtgct tccacatgcc 180
   tggcctaaaa tgggtgatat acaggtctta tatccccata tggaatttat ccatcaacca 240
   cataaaaaca aacagtgcct tctgccctct gcccagatgt gtccagcacg ttctcaaagt 300
   ttccacatta gcactcccta aggacgctgg gagcctgtca gtttatgatc tgacctaggt 360
   cccccctttc ttctgtcccc tgtttttaag tccggatttt tacagaagga actgtctcca 420
   gacagctcat caaggaacca agcaaaggcc agatagcctg acagataggc tagtggtaat 480
   tgtgtatatg ggcgggacgt gtgtgtcatt a 511
<210> 951
   <211> 316
   <212> DNA
   <213> Homo sapiens
   <400> 951
   cctctgtcct caaatgtcca aaatgttgga ggacctctgt tcatatccca cgcctgggct 60
   cttgccagca gtggagttac tgtagaggga tgtcccaagc ttgttttcca atcagtgtta 120
   agctgtttga aactctcctg tgtctgtgtt ttgtttgtgc gtgtgtgtga gagcacatca 180
   gtgtgtgcag gctgtgtttc cccatttctc tcctcccttc agacccatca ttgagaacaa 240
   atgtaagaaa tcccttccca ccaccctccc tgcctcccag gccctctgcg ggggaaacaa 300
   gatcacccag catcct 316
<210> 952
   <211> 149
   <212> DNA
   <213> Homo sapiens
<220>
   <221> misc_feature
   <222> (55)..(55)
   <223> n is a, c, g, or t
   <400> 952
   atattttgta tcatcgtgcc tatagccgct gccaccgtgt ataaatcctg gtgtntgctc 60
   cttatcctgg acatgaatgt attgtacact gacgcgtccc cactcctgta cagctgcttt 120
   gtttctttgc aatgcattgt atggcttta 149
<210> 953
   <211> 475
   <212> DNA
   <213> Homo sapiens
   <400> 953
   cttggtgtcc tggtgtgaat agacaagaag ctgtactata tgttgctctc tcagtggcaa 60
   caatgaagtt tttgcaattc tagaacttgg attttttttt aacaaaagtc ccaaaacacc 120
   aaaaatgtaa acaagataag agattaatat tgtagtgatg taatttaatt aaagttatat 180
   tttgggttaa ttttaacaac tgaagtctta ttgttgaaac ttattttcaa caaaactgtg 240
   cagttaaatt tgtatacgta ttcacatact gaaagatgaa ccgttaaaat agcacttaat 300
   tttgtgtttc ttcaatatgt cttgatatac tttgtgcaat taatattaca catgtaagtt 360
   gtatggcagt ttacagaact caatgacttg tcatgaggtt ttcatatgag ctacacattg 420
   tgtacattga ttgtttttta tttttacata aatccattct gtcattttca acttt 475
<210> 954
   <211> 402
   <212> DNA
   <213> Homo sapiens
   <400> 954
   aaagtcagtc cattttcaag ttttggtctt cagagacaaa agaacgtccc agccacctga 60
   ttttgatggt gaggtaactc taagttgaat tcaggctagt gttgcagtat agctttggca 120
   tgttcatgag tgagcaccca gaatgtgttg aaccaacccc cacccctaac tactgactat 180
   gactgcagtg ggtttttatg gggaaaaaaa gtgtgaaaag caaaaagaaa ggaacagaga 240
   ttttttatca cctttattgt aagacagtcc atttatgaat tgagtataaa cacatacaaa 300
   gtaacaagag attcctaaga aacgcaaatc cttgagtttc acgcacttca tgttcaacca 360
   tttgctgtaa tccagaggca gcctgtgaat cattctcatg cc 402
<210> 955
   <211> 523
   <212> DNA
   <213> Homo sapiens
<220>
   <221> misc_feature
   <222> (29)..(29)
   <223> n is a, c, g, or t
   <400> 955
   atccgacttg aatattcctg gacttacana atgccaaggg ggtgactgga agttgtggat 60
   atcagggtat aaattatatc cgtgagttgg gggagggaag accagaattc ccttgaattg 120
   tgtattgatg caatataagc ataaaagatc accttgtatt ctctttacct tctaaaagcc 180
   attattatga tgttagaaga agaggaagaa attcaggtac agaaaacatg tttaaatagc 240
   ctaaatgatg gtgcttggtg agtcttggtt ctaaaggtac caaacaagga agccaaagtt 300
   ttcaaactgc tgcatacttt gacaaggaaa atctatattt gtcttccgat caacatttat 360
   gacctaagtc aggtaatata cctggtttac ttctttagca tttttatgca gacagtctgt 420
   tatgcactgt ggtttcagat gtgcaataat ttgtacaatg gtttattccc aagtatgcct 480
   taagcagaac aaatgtgttt ttctatatag ttccttgcct taa 523
<210> 956
   <211> 491
   <212> DNA
   <213> Homo sapiens
<220>
   <221> misc_feature
   <222> (332)..(332)
   <223> n is a, c, g, or t
   <220>
   <221> misc_feature
   <222> (365)..(365)
   <223> n is a, c, g, or t
   <400> 956
   cccaggcctg tcactttgag aggggcaaaa ctgagagggg cttttcctag agaaagagaa 60
   caaggagctt gccaggcttc atgtagccga cacacgtctc aggattttaa gtccacattg 120
   gcctcacact accagggcca atgcccaaaa taaggagttc caatttgggg ccaaatgagg 180
   aaggacacag actctgccct gggatctcct gtgctagcgg ccaatgacaa atccagtcat 240
   tggccaccag ccacctctgc agtggggacc acactagcag ccctgactcc acactcctcc 300
   tggggaccca agaggcagtg ttgctgtctg cntgtccacc ttggaatctg gctgaactgg 360
   ctggnaggac caagactgcg gctggggtgg gcagggaagg gaagccgggg gctgctgtga 420
   gggatcttgg agcttccctg tagcccacct tccccttgct tcatgtttgt agaggaacct 480
   tgtgccggcc a 491
<210> 957
   <211> 253
   <212> DNA
   <213> Homo sapiens
   <400> 957
   gtaaatagtt aaccttcagt agtctattaa ggcattaata cttctctgga catgcgcgtt 60
   tgagggtgga ggggtcctgt aaggtgcttc atcgtctgtg attactgctt gggatgtgtt 120
   ctttggcagc ttgtgagatt actttaccta gtgtttataa agtaggaagt taagtgaatc 180
   atagattaga atttaatact cttatggaaa taatttttta acatcttaat tgacaatggc 240
   gtttttttat aca 253
<210> 958
   <211> 480
   <212> DNA
   <213> Homo sapiens
<220>
   <221> misc_feature
   <222> (57)..(57)
   <223> n is a, c, g, or t
   <220>
   <221> misc_feature
   <222> (65)..(65)
   <223> n is a, c, g, or t
   <400> 958
   gtaggctcag cgatagtggt cctcttacag agaaacgggg agcaggacga cgggggngct 60
   ggggntggcg ggggagggtg cccacaaaaa gaatcaggac ttgtactggg aaaaaaaccc 120
   ctaaattaat tatatttctt ggacattccc tttcctaaca tcctgaggct taaaaccctg 180
   atgcaaactt ctcctttcag tggttggaga aattggccga gttcaaccat tcactgcaat 240
   gcctattcca aactttaaat ctatctattg caaaacctga aggactgtag ttagcgggga 300
   tgatgttaag tgtggccaag cgcacggcgg caagttttca agcactgagt ttctattcca 360
   agatcataga cttactaaag agagtgacaa atgcttcctt aatgtcttct ataccagaat 420
   gtaaatattt ttgtgttttg tgttaatttg ttagaattct aacacactat atacttccaa 480
<210> 959
   <211> 323
   <212> DNA
   <213> Homo sapiens
   <400> 959
   tcgactctgc tgctcatggg aagaacagaa ttgctcctgc atgcaactaa ttcaataaaa 60
   ctgtcttgtg agctgatcgc ttggagggtc ctctttttat gttgagttgc tgcttcccgg 120
   catgccttca ttttgctatg gggggcaggc aggggggatg gaaaataagt agaaacaaaa 180
   aagcagtggc taagatggta tagggactgt cataccagtg aagaataaaa gggtgaagaa 240
   taaaagggat atgatgacaa ggttgatcca cttcaagaat tgcttgcttt caggaagaga 300
   gatgtgtttc aacaagccaa cta 323
<210> 960
   <211> 533
   <212> DNA
   <213> Homo sapiens
   <400> 960
   gagccctaat tgatatgtat acagaaggta tggcagattt gaatgaaatg atccttcttc 60
   tgcccttatg tcgacctgag gaaaaagatg ccaagattgc cttgatcaaa gagaaaacaa 120
   aaagtcgcta tttccctgcc ttcgaaaaag tgttacagag ccatggacaa gactaccttg 180
   ttggcaacaa gctgagccgg gctgacatta gcctggtgga acttctctac tatgtggaag 240
   agcttgactc cagccttatc tccaacttcc ctctgctgaa ggccctgaaa accagaatca 300
   gcaacctgcc cacggtgaag aagtttctac agcctggcag cccaaggaag cctcccgcag 360
   atgcaaaagc tttagaagaa gccagaaaga ttttcaggtt ttaataaagc agccatggag 420
   gctaagaaca tgcaagacca atattctaaa gttttgcaac aatgaagtgc tttacttaag 480
   tgttgattgt gcctgttgta aagctaatga accctttcca attatatgct aat 533
<210> 961
   <211> 472
   <212> DNA
   <213> Homo sapiens
   <400> 961
   ccggcccagg ctcactgggc cagtgggagg ctggacatca gcaacaagac ctatgagact 60
   gtcgccagcc tgggagcagc cacccctcag ggcgagagtg aggactgtcc cccgcccttg 120
   ccagtcaaaa actcttctcg gactttggtc caagggtgtg caagacatgc cagtggagat 180
   cgttctgagc aaagaaagaa gggagagtaa tagaattggg agggcagaga cttaagggtt 240
   ctgcttccca gccctagaaa ttctatcatt gctcagcccc aatgagaaag cagatacacc 300
   taagccatca tcaaccacta acatctcaac ttgccagttg ctgggtgctg ggccctggca 360
   ggaatgggcc aagccaagca ggggagacta gagagcacca atggccaaca cagctgcctg 420
   gctggggagg ctgtgctgtt tcccctggag acctgactgg tctgtggttc cc 472
<210> 962
   <211> 495
   <212> DNA
   <213> Homo sapiens
   <400> 962
   gccggtgaga tgctctatct gccggctctg tggttccacc acgtccagca gtcccagggc 60
   tgcatcgcag tgaatttctg gtatgacatg gaatacgacc tcaagtatag ttacttccag 120
   ctgctcgact ccctcaccaa ggcttcaggc cttgactgat ggagcactgg tgaacaccac 180
   caagcacgcc tcgggggacg gagccagccc ccccctggcc aggtcgagag agcctggagt 240
   gtgcatgctg gctgctggcc ccgggtccag catggcttga gatcagcttt ggaggatctt 300
   ggaatgtggt cataaggact caaggtgcca ggcaggtctg ggtgagggtt ctcaggaagt 360
   tgccacacag gtgagcagag tggggatcag gtgcagcggc acctctcccc agcgctgtga 420
   tgttgggcga gtcactgcgt ctcgggcatt ggtgtcctgt cagtaaagag ataataatgg 480
   ctgtacctcg cgggg 495
<210> 963
   <211> 120
   <212> DNA
   <213> Homo sapiens
<220>
   <221> misc_feature
   <222> (43)..(43)
   <223> n is a, c, g, or t
   <400> 963
   cctttccgtt tctgtctatg atgtaggctt ctgaggagaa ccnagaagct tggctttagt 60
   ggtagaatga cagaacttag ggatcccttg caggctagaa caaagttctg acccttagac 120
<210> 964
   <211> 494
   <212> DNA
   <213> Homo sapiens
<220>
   <221> misc_feature
   <222> (335)..(335)
   <223> n is a, c, g, or t
   <400> 964
   gacctcttga agcccaatta ttgcctcaat ccagaaaagt ttacttctct ttatctgtgc 60
   tttactgaca gaagggcaag tcttctctcg ttttttgcag ataaaatttt agatgtgttg 120
   cattcattgg gtttctatga gatgtggttt tatcagacaa ttttttcttt tatttcacaa 180
   ttactttaat atctgtaaaa taaagaatta ttttaattca ttttcccagt cccaaaagtt 240
   aaatacaggc cacttacttc tttaaccaaa tgatatagtt tggctctgtg tccccaccca 300
   aatctcatgt caaattgtaa tccccgcatg tcagnggagg gacctggtgg gaggtgattg 360
   gatcatgggg agggatttcc cccttgctgt tctgttgata gtgaacgagt tctcacgaaa 420
   tctgatggtt taaaagtgca gcacttctcc ctttgctctc tctctcctgc tgtgccatgg 480
   taagacgtgc cttg 494
<210> 965
   <211> 324
   <212> DNA
   <213> Homo sapiens
   <400> 965
   tgattttaaa attggcctcc tcaaagttta gcgtcttgca taatgatgat gtacgtctct 60
   ggcatattac attttccttt gtatatcatt attgaggtta tttgtctgat atgacccaaa 120
   gaggcaaaac tcagcacagt cctttctgca gtattctaaa ggtcatcaaa cttcagccta 180
   gtgagtctgc ttgtttgatt tggccggaca ttttaagcat ggcagaagtg gtacaagaaa 240
   tcatggtatt aagttgaaac cacacccctt agaaaaatcc ttctattaat tcaaataatt 300
   tgacgatgct tatgcggttt ctga 324
<210> 966
   <211> 478
   <212> DNA
   <213> Homo sapiens
   <400> 966
   ttcacaaact tttatactct ttctgtatat acattttttt tctttaaaaa acaactatgg 60
   atcagaatag caacatttag aacacttttt gttatcagtc aatattttta gatagttaga 120
   acctggtcct aagcctaaaa gtgggcttga ttctgcagta aatcttttac aactgcctcg 180
   acacacataa acctttttaa aaatagacac tccccgaagt cttttgtttg tatggtcaca 240
   cactgatgct tagatgttcc agtaatctaa tatggccaca gtagtcttga tgaccaaagt 300
   cctttttttc catctttaga aaactacatg ggaacaaaca gatcgaacag ttttgaagct 360
   actgtgtgtg tgaatgaaca ctcttgcttt attccagaat gctgtacatc tattttggat 420
   tgtatattgt ggttgtgtat ttacgctttg attcatagta acttcttatg gaattgat 478
<210> 967
   <211> 44
   <212> DNA
   <213> Homo sapiens
   <400> 967
   gaaagcatgt ctgctgggtg tgaccatgtt tcctctcaat aaag 44
<210> 968
   <211> 65
   <212> DNA
   <213> Homo sapiens
   <400> 968
   ggaaagcatg tctgctgggt gtgaccatgt ttcctctcaa taaagttccc ctgtgacact 60
   caaaa 65
<210> 969
   <211> 494
   <212> DNA
   <213> Homo sapiens
<220>
   <221> misc_feature
   <222> (33)..(33)
   <223> n is a, c, g, or t
   <220>
   <221> misc_feature
   <222> (35)..(35)
   <223> n is a, c, g, or t
   <220>
   <221> misc_feature
   <222> (45)..(54)
   <223> n is a, c, g, or t
   <220>
   <221> misc_feature
   <222> (168)..(168)
   <223> n is a, c, g, or t
   <220>
   <221> misc_feature
   <222> (203)..(257)
   <223> n is a, c, g, or t
   <220>
   <221> misc_feature
   <222> (304)..(304)
   <223> n is a, c, g, or t
   <220>
   <221> misc_feature
   <222> (306)..(306)
   <223> n is a, c, g, or t
   <220>
   <221> misc_feature
   <222> (348)..(362)
   <223> n is a, c, g, or t
   <220>
   <221> misc_feature
   <222> (427)..(427)
   <223> n is a, c, g, or t
   <400> 969
   gaagaagggg ccatcacagg atgccacccc tgncntgggt tgggnnnnnn nnnncacgac 60
   cagccccttc ctgggtattt attctctatt tattggggat aggagaagag gcatcctgcc 120
   tgggtgggac agccccttca gccccttctc ccctccccgc ctggccangg cagggccacc 180
   ccactctacc tccttagctt tcnnnnnnnn nnnnnnnnnn nnnnnnnnnn nnnnnnnnnn 240
   nnnnnnnnnn nnnnnnnaga gctgacggga ggccccagct ctgaggggag ggggtccgtg 300
   gtanangcct ggggccggta gaggctcccc agggctccct tatgtccnnn nnnnnnnnnn 360
   nngggtgtgg atgtaattag ctctgggggg cagttgggta gatgggtggg ggctcctggt 420
   ggccttntgc tgcccaggcc acagccgcct ttgggttcca tcttgctaat aaacactggc 480
   tctgggacta gaaa 494
<210> 970
   <211> 332
   <212> DNA
   <213> Homo sapiens
<220>
   <221> misc_feature
   <222> (229)..(252)
   <223> n is a, c, g, or t
   <400> 970
   gaaacccagg tgctggacca gggccctcag ggaggggacc ctgcggctag agtgggctag 60
   gccctggctt tgcccgtcag atttgaacga atgtgtgtcc cttgagccca aggagagcgg 120
   caggaggggt gggaccaggc tgggaggaca gagccagcag ctgccatgcc ctcctgctcc 180
   ccccacccca gccctagccc tttagccttt caccctgtgc tctggaaann nnnnnnnnnn 240
   nnnnnnnnnn nnaggaggag caaaaatgag ccagcaccag cgccttggct ttgtgttagc 300
   atttcctcct gaagtgttct gttggcaata aa 332
<210> 971
   <211> 279
   <212> DNA
   <213> Homo sapiens
   <400> 971
   cttctacagg cttttgggaa gtagggtgga tgtgggtagg gctgggagga gggggccaca 60
   gcttaggttt ggagctctgg atgtacatac ataagtagga gcagtgggac gtgtttctgt 120
   cataatgcag gcatgaaggg tggagtgaag tcaggtcata agtttcatgt ttgcttttgt 180
   tttgttttgt ttttaatgta tgtagcagat gttacagtct tagggatccg ggatgggaga 240
   ccccacttta gaaagggtcg tcactccttt aatcctcta 279
<210> 972
   <211> 145
   <212> DNA
   <213> Homo sapiens
   <400> 972
   ctgaacgggc gactgtgtct tgactacctt tcaaaaccag cactgtgtgg gaatgtccgc 60
   caggcagagc tcggagcctc attgagacag gggagagaga aagacaaaga ggggaccttc 120
   ttccagatgc cttcccagtt gtaac 145
<210> 973
   <211> 499
   <212> DNA
   <213> Homo sapiens
<220>
   <221> misc_feature
   <222> (200)..(204)
   <223> n is a, c, g, or t
   <220>
   <221> misc_feature
   <222> (230)..(230)
   <223> n is a, c, g, or t
   <220>
   <221> misc_feature
   <222> (235)..(235)
   <223> n is a, c, g, or t
   <220>
   <221> misc_feature
   <222> (239)..(239)
   <223> n is a, c, g, or t
   <220>
   <221> misc_feature
   <222> (357)..(357)
   <223> n is a, c, g, or t
   <400> 973
   agacgagtgc tgagccaaga acctcctaga ggctgtccct ggacctggag ctgcaggcat 60
   cagagaacca gccctgctca cgccatgccc gcccccgcct tccctcttcc ctcttccctc 120
   tccctgccca gccctccctt ccttcctctg ccggcaaggc agggacccac agtggctgcc 180
   tgcctccggg agggaaggan nnnnagggag ggtgggtggg tgggaggggn ccttncctnc 240
   cagggaatgt gactctccca ggccccagaa tagctcctgg acccaagccc aaggcccagc 300
   ctgggacaag gctccgaggg tcggctggcc ggagctattt ttacctcccg cctcccntgc 360
   tggtcccccc acctgacgtc ttgctgcaga gtctgacact ggattccccc ccctcacccc 420
   gcccctggtc ccactcctgc ccccgcccta cctccgcccc accccatcat ctgtggacac 480
   tggagtctgg aataaatgc 499
<210> 974
   <211> 419
   <212> DNA
   <213> Homo sapiens
<220>
   <221> misc_feature
   <222> (26)..(29)
   <223> n is a, c, g, or t
   <220>
   <221> misc_feature
   <222> (44)..(58)
   <223> n is a, c, g, or t
   <220>
   <221> misc_feature
   <222> (63)..(139)
   <223> n is a, c, g, or t
   <400> 974
   ttgctgaaga gcaagcagag ggtccnnnnc gcctgctgta caannnnnnn nnnnnnnnca 60
   tcnnnnnnnn nnnnnnnnnn nnnnnnnnnn nnnnnnnnnn nnnnnnnnnn nnnnnnnnnn 120
   nnnnnnnnnn nnnnnnnnna ggcctctccc tctgtcagtt ccagaacttc tccctccatg 180
   acccactcta tgggaaactc ttcagcacct acctgcgccc cccacacacc tctcgaggca 240
   cctcccagac accaaatgcc tcatccccag gcaaccccac tgctctggcc aatgggactg 300
   tgcaagcacc caagcagaag ggagactgag tgcctcagcc tctcaccccc tcctcctcag 360
   ggcagcgcta ggggcctccc ctatgcctca gccccatctc tgctcctgtt tgaattttg 419
<210> 975
   <211> 427
   <212> DNA
   <213> Homo sapiens
<220>
   <221> misc_feature
   <222> (64)..(64)
   <223> n is a, c, g, or t
   <220>
   <221> misc_feature
   <222> (101)..(101)
   <223> n is a, c, g, or t
   <220>
   <221> misc_feature
   <222> (120)..(121)
   <223> n is a, c, g, or t
   <220>
   <221> misc_feature
   <222> (272)..(326)
   <223> n is a, c, g, or t
   <400> 975
   cgcatcaagg gcataagtta ttgtgaacgt ttttgccaat cactgctcaa cagccctgct 60
   agantttgta tgatgctgaa ttattatgca gactaattcc ncccagttga gacacaccan 120
   ncttgttcac ttgtatttat tgaaactgtg gattcttgcc cgtgctgtcc cttgtattta 180
   ctttaagcac tgatcactta tcattcattc ggtatggttt tccctgtccc ttgtacacat 240
   tctggtatga atttgtaaaa ataacctgct annnnnnnnn nnnnnnnnnn nnnnnnnnnn 300
   nnnnnnnnnn nnnnnnnnnn nnnnnncgtg cccaactgag gaacaggaga agaaatcacc 360
   aatttgggct ctcagagcta agacacactt attgattctg ttgcacattt tgcactggtt 420
   tatggcg 427
<210> 976
   <211> 457
   <212> DNA
   <213> Homo sapiens
<220>
   <221> misc_feature
   <222> (64)..(95)
   <223> n is a, c, g, or t
   <220>
   <221> misc_feature
   <222> (104)..(104)
   <223> n is a, c, g, or t
   <220>
   <221> misc_feature
   <222> (226)..(226)
   <223> n is a, c, g, or t
   <220>
   <221> misc_feature
   <222> (344)..(344)
   <223> n is a, c, g, or t
   <220>
   <221> misc_feature
   <222> (387)..(402)
   <223> n is a, c, g, or t
   <400> 976
   acagacttgg caagggaccc cctggttctg agccagtagc tgccatctgg aaattcctct 60
   tttnnnnnnn nnnnnnnnnn nnnnnnnnnn nnnnnctccc aggnacccgc tgaatttctg 120
   aggccttgct taaagctcag aagtggttta ggcatttgga aaatctggtt cacatcataa 180
   agaacttgat ttgaaatgtt ttctatagaa acaagtgcta agtgtnaccg tattatactt 240
   gatgttggtc atttctcagt cctatttctc agttctatta ttttagaacc tagtcagttc 300
   tttaagatta taactggtcc tacattaaaa taatgcttct cgangtcaga ttttacctgt 360
   ttgctgctga gaacatctct gcctaannnn nnnnnnnnnn nncttcagtt caacatgctt 420
   ccttagcttt tcatagttgt ctgacatttc catgaaa 457
<210> 977
   <211> 493
   <212> DNA
   <213> Homo sapiens
<220>
   <221> misc_feature
   <222> (28)..(28)
   <223> n is a, c, g, or t
   <220>
   <221> misc_feature
   <222> (44)..(44)
   <223> n is a, c, g, or t
   <220>
   <221> misc_feature
   <222> (53)..(53)
   <223> n is a, c, g, or t
   <220>
   <221> misc_feature
   <222> (73)..(74)
   <223> n is a, c, g, or t
   <220>
   <221> misc_feature
   <222> (88)..(88)
   <223> n is a, c, g, or t
   <220>
   <221> misc_feature
   <222> (95)..(96)
   <223> n is a, c, g, or t
   <220>
   <221> misc_feature
   <222> (98)..(98)
   <223> n is a, c, g, or t
   <220>
   <221> misc_feature
   <222> (108)..(123)
   <223> n is a, c, g, or t
   <220>
   <221> misc_feature
   <222> (351)..(351)
   <223> n is a, c, g, or t
   <220>
   <221> misc_feature
   <222> (364)..(378)
   <223> n is a, c, g, or t
   <400> 977
   gcgcagcttt tctcgctgca gagggagnag ctgcgggcgg tgancccgag ganggggcac 60
   gtgtgtacag ccnngtcacc gtgcagcnct cgctnntnga ggacaaannn nnnnnnnnnn 120
   nnntggaggc agtgatggag aagcaaaaga agaaggtgga aggcgaggtg gaaatggagg 180
   tcatttgacc tgccaggcgc ccttcgcaaa gagtgacgag gccccgtggg agaacggact 240
   cctcagactc tccccaatag cggaagtcga tcttctgaag gatggccaat ctgctccggc 300
   cctggtcttc ccccatcccg gtggacagac ttaacgatcc ttgctgcagt ncctccggag 360
   aggnnnnnnn nnnnnnnnga gtggggaggg cgtggagaca gtctacggaa agcgctagca 420
   gacccccgag agggtgcagt ggagccctga gcattgtaat atgcggccca gcctataaac 480
   agcctccgtg ctt 493
<210> 978
   <211> 1536
   <212> DNA
   <213> Homo sapiens
   <400> 978
   gtgacgcgag gctctgcgga gaccaggagt cagactgtag gacgacctcg ggtcccacgt 60
   gtccccggta ctcgccggcc ggagcccccg gcttcccggg gccgggggac cttagcggca 120
   cccacacaca gcctactttc caagcggagc catgtctggt aacggcaatg cggctgcaac 180
   ggcggaagaa aacagcccaa agatgagagt gattcgcgtg ggtacccgca agagccagct 240
   tgctcgcata cagacggaca gtgtggtggc aacattgaaa gcctcgtacc ctggcctgca 300
   gtttgaaatc attgctatgt ccaccacagg ggacaagatt cttgatactg cactctctaa 360
   gattggagag aaaagcctgt ttaccaagga gcttgaacat gccctggaga agaatgaagt 420
   ggacctggtt gttcactcct tgaaggacct gcccactgtg cttcctcctg gcttcaccat 480
   cggagccatc tgcaagcggg aaaaccctca tgatgctgtt gtctttcacc caaaatttgt 540
   tgggaagacc ctagaaaccc tgccagagaa gagtgtggtg ggaaccagct ccctgcgaag 600
   agcagcccag ctgcagagaa agttcccgca tctggagttc aggagtattc ggggaaacct 660
   caacacccgg cttcggaagc tggacgagca gcaggagttc agtgccatca tcctggcaac 720
   agctggcctg cagcgcatgg gctggcacaa ccgggtgggg cagatcctgc accctgagga 780
   atgcatgtat gctgtgggcc agggggcctt gggcgtggaa gtgcgagcca aggaccagga 840
   catcttggat ctggtgggtg tgctgcacga tcccgagact ctgcttcgct gcatcgctga 900
   aagggccttc ctgaggcacc tggaaggagg ctgcagtgtg ccagtagccg tgcatacagc 960
   tatgaaggat gggcaactgt acctgactgg aggagtctgg agtctagacg gctcagatag 1020
   catacaagag accatgcagg ctaccatcca tgtccctgcc cagcatgaag atggccctga 1080
   ggatgaccca cagttggtag gcatcactgc tcgtaacatt ccacgagggc cccagttggc 1140
   tgcccagaac ttgggcatca gcctggccaa cttgttgctg agcaaaggag ccaaaaacat 1200
   cctggatgtt gcacggcagc ttaacgatgc ccattaactg gtttgtgggg cacagatgcc 1260
   tgggttgctg ctgtccagtg cctacatccc gggcctcagt gccccattct cactgctatc 1320
   tggggagtga ttaccccggg agactgaact gcagggttca agccttccag ggatttgcct 1380
   caccttgggg ccttgatgac tgccttgcct cctcagtatg tgggggcttc atctctttag 1440
   agaagtccaa gcaacagcct ttgaatgtaa ccaatcctac taataaacca gttctgaagg 1500
   taaaaaaaaa aaaaaaaaaa aaaaaaaaaa aaaaaa 1536
<210> 979
   <211> 1524
   <212> DNA
   <213> Homo sapiens
   <400> 979
   agcagacaga ggactctcat taaggaaggt gtcctgtgcc ctgaccctac aagatgccaa 60
   gagaagatgc tcacttcatc tatggttacc ccaagaaggg gcacggccac tcttacacca 120
   cggctgaaga ggccgctggg atcggcatcc tgacagtgat cctgggagtc ttactgctca 180
   tcggctgttg gtattgtaga agacgaaatg gatacagagc cttgatggat aaaagtcttc 240
   atgttggcac tcaatgtgcc ttaacaagaa gatgcccaca agaagggttt gatcatcggg 300
   acagcaaagt gtctcttcaa gagaaaaact gtgaacctgt ggttcccaat gctccacctg 360
   cttatgagaa actctctgca gaacagtcac caccacctta ttcaccttaa gagccagcga 420
   gacacctgag acatgctgaa attatttctc tcacactttt gcttgaattt aatacagaca 480
   tctaatgttc tcctttggaa tggtgtagga aaaatgcaag ccatctctaa taataagtca 540
   gtgttaaaat tttagtaggt ccgctagcag tactaatcat gtgaggaaat gatgagaaat 600
   attaaattgg gaaaactcca tcaataaatg ttgcaatgca tgatactatc tgtgccagag 660
   gtaatgttag taaatccatg gtgttatttt ctgagagaca gaattcaagt gggtattctg 720
   gggccatcca atttctcttt acttgaaatt tggctaataa caaactagtc aggttttcga 780
   accttgaccg acatgaactg tacacagaat tgttccagta ctatggagtg ctcacaaagg 840
   atacttttac aggttaagac aaagggttga ctggcctatt tatctgatca agaacatgtc 900
   agcaatgtct ctttgtgctc taaaattcta ttatactaca ataatatatt gtaaagatcc 960
   tatagctctt tttttttgag atggagtttc gcttttgttg cccaggctgg agtgcaatgg 1020
   cgcgatcttg gctcaccata acctccgcct cccaggttca agcaattctc ctgccttagc 1080
   ctcctgagta gctgggatta caggcgtgcg ccactatgcc tgactaattt tgtagtttta 1140
   gtagagacgg ggtttctcca tgttggtcag gctggtctca aactcctgac ctcaggtgat 1200
   ctgcccgcct cagcctccca aagtgctgga attacaggcg tgagccacca cgcctggctg 1260
   gatcctatat cttaggtaag acatataacg cagtctaatt acatttcact tcaaggctca 1320
   atgctattct aactaatgac aagtattttc tactaaacca gaaattggta gaaggattta 1380
   aataagtaaa agctactatg tactgcctta gtgctgatgc ctgtgtactg ccttaaatgt 1440
   acctatggca atttagctct cttgggttcc caaatccctc tcacaagaat gtgcagaaga 1500
   aatcataaag gatcagagat tctg 1524
<210> 980
   <211> 2026
   <212> DNA
   <213> Homo sapiens
   <400> 980
   ctcgagatgg atctggtgct aaaaagatgc cttcttcatt tggctgtgat aggtgctttg 60
   ctggctgtgg gggctacaaa agtacccaga aaccaggact ggcttggtgt ctcaaggcaa 120
   ctcagaacca aagcctggaa caggcagctg tatccagagt ggacagaagc ccagagactt 180
   gactgctgga gaggtggtca agtgtccctc aaggtcagta atgatgggcc tacactgatt 240
   ggtgcaaatg cctccttctc tattgccttg aacttccctg gaagccaaaa ggtattgcca 300
   gatgggcagg ttatctgggt caacaatacc atcatcaatg ggagccaggt gtggggagga 360
   cagccagtgt atccccagga aactgacgat gcctgcatct tccctgatgg tggaccttgc 420
   ccatctggct cttggtctca gaagagaagc tttgtttatg tctggaagac ctggggccaa 480
   tactggcaag ttctaggggg cccagtgtct gggctgagca ttgggacagg cagggcaatg 540
   ctgggcacac acaccatgga agtgactgtc taccatcgcc ggggatcccg gagctatgtg 600
   cctcttgctc attccagctc agccttcacc attactgacc aggtgccttt ctccgtgagc 660
   gtgtcccagt tgcgggcctt ggatggaggg aacaagcact tcctgagaaa tcagcctctg 720
   acctttgccc tccagctcca tgaccccagt ggctatctgg ctgaagctga cctctcctac 780
   acctgggact ttggagacag tagtggaacc ctgatctctc gggcacttgt ggtcactcat 840
   acttacctgg agcctggccc agtcactgcc caggtggtcc tgcaggctgc cattcctctc 900
   acctcctgtg gctcctcccc agttccaggc accacagatg ggcacaggcc aactgcagag 960
   gcccctaaca ccacagctgg ccaagtgcct actacagaag ttgtgggtac tacacctggt 1020
   caggcgccaa ctgcagagcc ctctggaacc acatctgtgc aggtgccaac cactgaagtc 1080
   ataagcactg cacctgtgca gatgccaact gcagagagca caggtatgac acctgagaag 1140
   gtgccagttt cagaggtcat gggtaccaca ctggcagaga tgtcaactcc agaggctaca 1200
   ggtatgacac ctgcagaggt atcaattgtg gtgctttctg gaaccacagc tgcacaggta 1260
   acaactacag agtgggtgga gaccacagct agagagctac ctatccctga gcctgaaggt 1320
   ccagatgcca gctcaatcat gtctacggaa agtattacag gttccctggg ccccctgctg 1380
   gatggtacag ccaccttaag gctggtgaag agacaagtcc ccctggattg tgttctgtat 1440
   cgatatggtt ccttttccgt caccctggac attgtccagg gtattgaaag tgccgagatc 1500
   ctgcaggctg tgccgtccgg tgagggggat gcatttgagc tgactgtgtc ctgccaaggc 1560
   gggctgccca aggaagcctg catggagatc tcatcgccag ggtgccagcc ccctgcccag 1620
   cggctgtgcc agcctgtgct acccagccca gcctgccagc tggttctgca ccagatactg 1680
   aagggtggct cggggacata ctgcctcaat gtgtctctgg ctgataccaa cagcctggca 1740
   gtggtcagca cccagcttat catgcctggt caagaagcag gggccttggg gcaggttccg 1800
   ctgatcgtgg gcatcttgct ggtgttgatg gctgtggtcc ttgcatctct gatatatagg 1860
   cgcagactta tgaagcaaga cttctccgta ccccagttgc cacatagcag cagtcactgg 1920
   ctgcgtctac cccgcatctt ctgctcttgt cccattggtg agaatagccc cctcctcagt 1980
   gggcagcagg tctgagtact ctcatatgat gctgtgattg cggccg 2026
<210> 981
   <211> 4204
   <212> DNA
   <213> Homo sapiens
   <400> 981
   acgcaggcag tgatgtcacc cagaccacac cccttccccc aatgccactt cagggggtac 60
   tcagagtcag agacttggtc tgaggggagc agaagcaatc tgcagaggat ggcggtccag 120
   gctcagccag gcatcaactt caggaccctg agggatgacc gaaggccccg cccacccacc 180
   cccaactccc ccgaccccac caggatctac agcctcagga cccccgtccc aatccttacc 240
   ccttgcccca tcaccatctt catgcttacc tccaccccca tccgatcccc atccaggcag 300
   aatccagttc cacccctgcc cggaacccag ggtagtaccg ttgccaggat gtgacgccac 360
   tgacttgcgc attggaggtc agaagaccgc gagattctcg ccctgagcaa cgagcgacgg 420
   cctgacgtcg gcggagggaa gccggcccag gctcggtgag gaggcaaggt aagacgctga 480
   gggaggactg aggcgggcct cacctcagac agagggcctc aaataatcca gtgctgcctc 540
   tgctgccggg cctgggccac cccgcagggg aagacttcca ggctgggtcg ccactacctc 600
   accccgccga cccccgccgc tttagccacg gggaactctg gggacagagc ttaatgtggc 660
   cagggcaggg ctggttagaa gaggtcaggg cccacgctgt ggcaggaatc aaggtcagga 720
   ccccgagagg gaactgaggg cagcctaacc accaccctca ccaccattcc cgtcccccaa 780
   cacccaaccc cacccccatc ccccattccc atccccaccc ccacccctat cctggcagaa 840
   tccgggcttt gcccctggta tcaagtcacg gaagctccgg gaatggcggc caggcacgtg 900
   agtcctgagg ttcacatcta cggctaaggg agggaagggg ttcggtatcg cgagtatggc 960
   cgttgggagg cagcgaaagg gcccaggcct cctggaagac agtggagtcc tgaggggacc 1020
   cagcatgcca ggacaggggg cccactgtac ccctgtctca aaccgaggca ccttttcatt 1080
   cggctacggg aatcctaggg atgcagaccc acttcagcag ggggttgggg cccagccctg 1140
   cgaggagtca tggggaggaa gaagagggag gactgagggg accttggagt ccagatcagt 1200
   ggcaaccttg ggctggggga tgctgggcac agtggccaaa tgtgctctgt gctcattgcg 1260
   ccttcagggt gaccagagag ttgagggctg tggtctgaag agtgggactt caggtcagca 1320
   gagggaggaa tcccaggatc tgcagggccc aaggtgtacc cccaaggggc ccctatgtgg 1380
   tggacagatg cagtggtcct aggatctgcc aagcatccag gtgaagagac tgagggagga 1440
   ttgagggtac ccctgggaca gaatgcggac tgggggcccc ataaaaatct gccctgctcc 1500
   tgctgttacc tcagagagcc tgggcagggc tgtcagctga ggtccctcca ttatcctagg 1560
   atcactgatg tcagggaagg ggaagccttg gtctgagggg gctgcactca gggcagtaga 1620
   gggaggctct cagaccctac taggagtgga ggtgaggacc aagcagtctc ctcacccagg 1680
   gtacatggac ttcaataaat ttggacatct ctcgttgtcc tttccgggag gacctgggaa 1740
   tgtatggcca gatgtgggtc ccctcatgtt tttctgtacc atatcaggta tgtgagttct 1800
   tgacatgaga gattctcagg ccagcagaag ggagggatta ggccctataa ggagaaaggt 1860
   gagggccctg agtgagcaca gaggggatcc tccaccccag tagagtgggg acctcacaga 1920
   gtctggccaa ccctcctgac agttctggga atccgtggct gcgtttgctg tctgcacatt 1980
   gggggcccgt ggattcctct cccaggaatc aggagctcca ggaacaaggc agtgaggact 2040
   tggtctgagg cagtgtcctc aggtcacaga gtagaggggg ctcagatagt gccaacggtg 2100
   aaggtttgcc ttggattcaa accaagggcc ccacctgccc cagaacacat ggactccaga 2160
   gcgcctggcc tcaccctcaa tactttcagt cctgcagcct cagcatgcgc tggccggatg 2220
   taccctgagg tgccctctca cttcctcctt caggttctga ggggacaggc tgacctggag 2280
   gaccagaggc ccccggagga gcactgaagg agaagatctg taagtaagcc tttgttagag 2340
   cctccaaggt tccattcagt actcagctga ggtctctcac atgctccctc tctccccagg 2400
   ccagtgggtc tccattgccc agctcctgcc cacactcccg cctgttgccc tgaccagagt 2460
   catcatgcct cttgagcaga ggagtcagca ctgcaagcct gaagaaggcc ttgaggcccg 2520
   aggagaggcc ctgggcctgg tgggtgcgca ggctcctgct actgaggagc aggaggctgc 2580
   ctcctcctct tctactctag ttgaagtcac cctgggggag gtgcctgctg ccgagtcacc 2640
   agatcctccc cagagtcctc agggagcctc cagcctcccc actaccatga actaccctct 2700
   ctggagccaa tcctatgagg actccagcaa ccaagaagag gaggggccaa gcaccttccc 2760
   tgacctggag tccgagttcc aagcagcact cagtaggaag gtggccgagt tggttcattt 2820
   tctgctcctc aagtatcgag ccagggagcc ggtcacaaag gcagaaatgc tggggagtgt 2880
   cgtcggaaat tggcagtatt tctttcctgt gatcttcagc aaagcttcca gttccttgca 2940
   gctggtcttt ggcatcgagc tgatggaagt ggaccccatc ggccacttgt acatctttgc 3000
   cacctgcctg ggcctctcct acgatggcct gctgggtgac aatcagatca tgcccaaggc 3060
   aggcctcctg ataatcgtcc tggccataat cgcaagagag ggcgactgtg cccctgagga 3120
   gaaaatctgg gaggagctga gtgtgttaga ggtgtttgag gggagggaag acagtatctt 3180
   gggggatccc aagaagctgc tcacccaaca tttcgtgcag gaaaactacc tggagtaccg 3240
   gcaggtcccc ggcagtgatc ctgcatgtta tgaattcctg tggggtccaa gggccctcgt 3300
   tgaaaccagc tatgtgaaag tcctgcacca tatggtaaag atcagtggag gacctcacat 3360
   ttcctaccca cccctgcatg agtgggtttt gagagagggg gaagagtgag tctgagcacg 3420
   agttgcagcc agggccagtg ggagggggtc tgggccagtg caccttccgg ggccgcatcc 3480
   cttagtttcc actgcctcct gtgacgtgag gcccattctt cactctttga agcgagcagt 3540
   cagcattctt agtagtgggt ttctgttctg ttggatgact ttgagattat tctttgtttc 3600
   ctgttggagt tgttcaaatg ttccttttaa cggatggttg aatgagcgtc agcatccagg 3660
   tttatgaatg acagtagtca cacatagtgc tgtttatata gtttaggagt aagagtcttg 3720
   ttttttactc aaattgggaa atccattcca ttttgtgaat tgtgacataa taatagcagt 3780
   ggtaaaagta tttgcttaaa attgtgagcg aattagcaat aacatacatg agataactca 3840
   agaaatcaaa agatagttga ttcttgcctt gtacctcaat ctattctgta aaattaaaca 3900
   aatatgcaaa ccaggatttc cttgacttct ttgagaatgc aagcgaaatt aaatctgaat 3960
   aaataattct tcctcttcac tggctcgttt cttttccgtt cactcagcat ctgctctgtg 4020
   ggaggccctg ggttagtagt ggggatgcta aggtaagcca gactcacgcc tacccatagg 4080
   gctgtagagc ctaggacctg cagtcatata attaaggtgg tgagaagtcc tgtaagatgt 4140
   agaggaaatg taagagaggg gtgagggtgt ggcgctccgg gtgagagtag tggagtgtca 4200
   gtgc 4204
<210> 982
   <211> 23
   <212> DNA
   <213> Homo sapiens
   <400> 982
   tgtgtctctg gctgatacca aca 23
<210> 983
   <211> 23
   <212> DNA
   <213> Homo sapiens
   <400> 983
   ttcttgacca ggcatgataa gct 23
<210> 984
   <211> 15
   <212> DNA
   <213> Homo sapiens
   <400> 984
   ctggcagtgg tcagc 15
<210> 985
   <211> 22
   <212> DNA
   <213> Homo sapiens
   <400> 985
   ctgcttcgct gcatcgctga aa 22
<210> 986
   <211> 22
   <212> DNA
   <213> Homo sapiens
   <400> 986
   cagactcctc cagtcaggta ca 22
<210> 987
   <211> 30
   <212> DNA
   <213> Homo sapiens
   <400> 987
   cctgaggcac ctggaaggag gctgcagtgt 30
<210> 988
   <211> 2384
   <212> DNA
   <213> Homo sapiens
   <400> 988
   tattgagttc ttcaaacatt gtagcctctt tatggtctct gagaaataac taccttaaac 60
   ccataatctt taatacttcc taaactttct taataagaga agctctattc ctgacactac 120
   ctctcatttg caaggtcaaa tcatcattag ttttgtagtc tattaactgg gtttgcttag 180
   gtcaggcatt attattacta accttattgt taatattcta accataagaa ttaaactatt 240
   aatggtgaat agagtttttc actttaacat aggcctatcc cactggtggg atacgagcca 300
   attcgaaaga aaagtcagtc atgtgctttt cagaggatga aagcttaaga taaagactaa 360
   aagtgtttga tgctggaggt gggagtggta ttatataggt ctcagccaag acatgtgata 420
   atcactgtag tagtagctgg aaagagaaat ctgtgactcc aattagccag ttcctgcaga 480
   ccttgtgagg actagaggaa gaatgctcct ggctgttttg tactgcctgc tgtggagttt 540
   ccagacctcc gctggccatt tccctagagc ctgtgtctcc tctaagaacc tgatggagaa 600
   ggaatgctgt ccaccgtgga gcggggacag gagtccctgt ggccagcttt caggcagagg 660
   ttcctgtcag aatatccttc tgtccaatgc accacttggg cctcaatttc ccttcacagg 720
   ggtggatgac cgggagtcgt ggccttccgt cttttataat aggacctgcc agtgctctgg 780
   caacttcatg ggattcaact gtggaaactg caagtttggc ttttggggac caaactgcac 840
   agagagacga ctcttggtga gaagaaacat cttcgatttg agtgccccag agaaggacaa 900
   attttttgcc tacctcactt tagcaaagca taccatcagc tcagactatg tcatccccat 960
   agggacctat ggccaaatga aaaatggatc aacacccatg tttaacgaca tcaatattta 1020
   tgacctcttt gtctggatgc attattatgt gtcaatggat gcactgcttg ggggatctga 1080
   aatctggaga gacattgatt ttgcccatga agcaccagct tttctgcctt ggcatagact 1140
   cttcttgttg cggtgggaac aagaaatcca gaagctgaca ggagatgaaa acttcactat 1200
   tccatattgg gactggcggg atgcagaaaa gtgtgacatt tgcacagatg agtacatggg 1260
   aggtcagcac cccacaaatc ctaacttact cagcccagca tcattcttct cctcttggca 1320
   gattgtctgt agccgattgg aggagtacaa cagccatcag tctttatgca atggaacgcc 1380
   cgagggacct ttacggcgta atcctggaaa ccatgacaaa tccagaaccc caaggctccc 1440
   ctcttcagct gatgtagaat tttgcctgag tttgacccaa tatgaatctg gttccatgga 1500
   taaagctgcc aatttcagct ttagaaatac actggaagga tttgctagtc cacttactgg 1560
   gatagcggat gcctctcaaa gcagcatgca caatgccttg cacatctata tgaatggaac 1620
   aatgtcccag gtacagggat ctgccaacga tcctatcttc cttcttcacc atgcatttgt 1680
   tgacagtatt tttgagcagt ggctccgaag gcaccgtcct cttcaagaag tttatccaga 1740
   agccaatgca cccattggac ataaccggga atcctacatg gttcctttta taccactgta 1800
   cagaaatggt gatttcttta tttcatccaa agatctgggc tatgactata gctatctaca 1860
   agattcagac ccagactctt ttcaagacta cattaagtcc tatttggaac aagcgagtcg 1920
   gatctggtca tggctccttg gggcggcgat ggtaggggcc gtcctcactg ccctgctggc 1980
   agggcttgtg agcttgctgt gtcgtcacaa gagaaagcag cttcctgaag aaaagcagcc 2040
   actcctcatg gagaaagagg attaccacag cttgtatcag agccatttat aaaaggctta 2100
   ggcaatagag tagggccaaa aagcctgacc tcactctaac tcaaagtaat gtccaggttc 2160
   ccagagaata tctgctggta tttttctgta aagaccattt gcaaaattgt aacctaatac 2220
   aaagtgtagc cttcttccaa ctcaggtaga acacacctgt ctttgtcttg ctgttttcac 2280
   tcagcccttt taacattttc ccctaagccc atatgtctaa ggaaaggatg ctatttggta 2340
   atgaggaact gttatttgta tgtgaattaa agtgctctta tttt 2384
<210> 989
   <211> 1204
   <212> DNA
   <213> Homo sapiens
   <400> 989
   cggaacgagg gcaacctgca cagccatgcc cgggcaagaa ctcaggacgg tgaatggctc 60
   tcagatgctc ctggtgttgc tggtgctctc gtggctgccg catgggggcg ccctgtctct 120
   ggccgaggcg agccgcgcaa gtttcccggg accctcagag ttgcactccg aagactccag 180
   attccgagag ttgcggaaac gctacgagga cctgctaacc aggctgcggg ccaaccagag 240
   ctgggaagat tcgaacaccg acctcgtccc ggcccctgca gtccggatac tcacgccaga 300
   agtgcggctg ggatccggcg gccacctgca cctgcgtatc tctcgggccg cccttcccga 360
   ggggctcccc gaggcctccc gccttcaccg ggctctgttc cggctgtccc cgacggcgtc 420
   aaggtcgtgg gacgtgacac gaccgctgcg gcgtcagctc agccttgcaa gaccccaagc 480
   gcccgcgctg cacctgcgac tgtcgccgcc gccgtcgcag tcggaccaac tgctggcaga 540
   atcttcgtcc gcacggcccc agctggagtt gcacttgcgg ccgcaagccg ccagggggcg 600
   ccgcagagcg cgtgcgcgca acggggacga ctgtccgctc gggcccgggc gttgctgccg 660
   tctgcacacg gtccgcgcgt cgctggaaga cctgggctgg gccgattggg tgctgtcgcc 720
   acgggaggtg caagtgacca tgtgcatcgg cgcgtgcccg agccagttcc gggcggcaaa 780
   catgcacgcg cagatcaaga cgagcctgca ccgcctgaag cccgacacgg agccagcgcc 840
   ctgctgcgtg cccgccagct acaatcccat ggtgctcatt caaaagaccg acaccggggt 900
   gtcgctccag acctatgatg acttgttagc caaagactgc cactgcatat gagcagtcct 960
   ggtccttcca ctgtgcacct gcgcggggga ggcgacctca gttgtcctgc cctgtggaat 1020
   gggctcaagg ttcctgagac acccgattcc tgcccaaaca gctgtattta tataagtctg 1080
   ttatttatta ttaatttatt ggggtgacct tcttggggac tcgggggctg gtctgatgga 1140
   actgtgtatt tatttaaaac tctggtgata aaaataaagc tgtctgaact gttaaaaaaa 1200
   aaaa 1204
<210> 990
   <211> 29
   <212> DNA
   <213> Homo sapiens

## Claims

1. A method of identifying a melanoma comprising the steps of
(a) obtaining a tissue sample; and
(b) measuring the expression levels in the sample of genes encoding mRNA corresponding to
PLAB (SEQ ID NO: 1) and L1CAM (SEQ ID NO: 2); or
PLAB, L1 CAM and NTRK3 (SEQ ID NO: 3)
wherein the gene expression levels above pre-determined cut-off levels are indicative of the presence of a melanoma in the sample, and wherein said predetermined cut-off levels are the highest score in:
primary normal samples; and/or
benign nevi samples.

## Patentansprüche

1. Verfahren zum Identifizieren eines Melanoms, welches die Schritte umfassst
(a) Erhalten einer Gewebeprobe; und
(b) Messen der Expressionsniveaus in der Probe von Genen, die mRNA kodieren, entsprechend zu
PLAB (SEQ ID NO: 1) und L1 CAM (SEQ ID NO:2); oder
PLAB, L1 CAM und NTRK3 (SEQ ID NO: 3),
wobei die Genexpressionsniveaus oberhalb vorbestimmter Ausschlussniveaus das Vorhandensein eines Melanoms in der Probe anzeigen und wobei die vorbestimmten Ausschlussniveaus die höchste Bewertung in:
primären normalen Proben; und/oder gutartigen Nävi-Proben sind.

## Revendications

1. Méthode d'identification d'un mélanome comprenant les étapes consistant à
(a) obtenir un échantillon tissulaire ; et
(b) mesurer les taux d'expression dans l'échantillon de gènes codant pour l'ARNm correspondant à PLAB (SÉQ ID NO : 1) et L1CAM (SÉQ ID NO : 2) ; ou PLAB, L1CAM et NTRK3 (SÉQ ID NO : 3)
dans laquelle les taux d'expression génique supérieurs à des taux seuils prédéterminés sont indicatifs de la présence d'un mélanome dans l'échantillon, et dans laquelle lesdits taux seuils prédéterminés sont le score le plus élevé dans :
■ des échantillons normaux primaires ; et/ou
■ des échantillons de nævi bénins.
